# EUROPEAN PATENT APPLICATION

(11) **EP 4 796 557 A1**
(43) Date of publication of application: **26.08.2026**
(21) Application number: 24879101.4
(22) Date of filing: 18.10.2024
(51) Int. Cl.: C07K 7/06, A61K 47/65, C07K 16/00

(54) **POLYPEPTIDE, BIOACTIVE CONJUGATE, AND PREPARATION METHOD THEREFOR AND USE THEREOF**

(30) Priority: 19.10.2023 CN 202311358300
(71) Applicant: Unovel (Shanghai) Biopharma Co., Ltd., Shanghai 201114 (CN)
(72) Inventor: JIN, Mingzhi, Shanghai 201114 (CN); WANG, Liang, Shanghai 201114 (CN); LI, Xueqing, Shanghai 201114 (CN); SHEN, Dongguo, Shanghai 201114 (CN); ZHOU, Qing, Shanghai 201114 (CN); YUAN, Mingxu, Shanghai 201114 (CN); WANG, Ying, Shanghai 201114 (CN)
(74) Representative: Gassner, Birgitta
(86) International application number: PCT/CN2024/125665
(87) International publication number: WO 2025/082461

(57) **Abstract**

Provided are a bioactive conjugate, and a preparation method therefor and the use thereof. Specifically, the present invention relates to a bioactive conjugate using a linker of a polypeptide unit (-P1-P2-), and the use thereof in the field of disease treatment, including, but not limited to, the use thereof in the treatment of tumor diseases. In addition, also provided in the present invention are a linker-drug, a linker and a polypeptide fragment used for the bioactive conjugate, and a preparation method therefor and the use thereof.

## Description

### TECHNICAL FIELD

The present application relates to the field of biomedicines, and specifically relates to a polypeptide, a linker, a linker-payload, a bioactive conjugate, preparation methods therefor, and uses thereof in the prevention and/or treatment of diseases.

### BACKGROUND

Abnormal cellular activity can lead to various diseases in body, such as cancer, autoimmune diseases, and neurodegenerative diseases. Among them, cancer is characterized by abnormal cell proliferation. Intervention against abnormal cell proliferation is a conventional method for the treatment of cancer, and using chemical small molecules for the treatment of cancer is a widely used means currently. However, the clinical use of many cytotoxic drug molecules is limited due to the problems such as lacking of targeting selectivity, undesirable pharmacokinetic properties, or excessive toxicity, thus giving rise to the development of bioactive conjugates. Bioactive conjugates have played a significant role in improving targeting ability and addressing undesirable properties of drugs *per se.* Among others, antibody-drug conjugates (ADCs) have emerged as outstanding representatives of bioactive conjugates in tumor therapy. As of September 2023, a total of 13 ADCs have been applied in clinical treatment worldwide, namely: Mylotarg^{®} (targeting CD33), Adcetris^{®} (targeting CD30), Kadcyla^{®} (targeting Her2), Besponsa^{®} (targeting CD33), Polivy^{®} (targeting CD79b), Padcev^{®} (targeting Nectin-4), Enhertu^{®} (targeting Her2), Trodelvy^{®} (targeting Trop2), Zynlonta^{®} (targeting CD19), Tivdak^{®} (targeting TF), Elahere^{®} (targeting FRα), Akalux^{®} (targeting EGFR), and Aidixi^{®} (targeting Her2). There were as many as 249 newly initiated clinical trials related to ADCs in 2022 alone. The ADCs for clinical trials not only cover the targets of marketed drugs, but also involve multiple novel potential ADC targets, such as B7-H3, B7-H4, ROR1, 5T4, ITGB6, and LIV-1.

An ADC is composed of three parts: an antibody, a linker, and a payload, which also includes the linkage of the antibody to the linker and the linkage of the linker to the payload. The functional part of the ADC is mainly the payload. Its mechanism of action is to enrich the drug on the surface of tumor cells by means of the specific binding of antibody to antigen, and then to release the drug at the tumor site through specific mechanisms. The releasing mechanisms include: releasing the payload via the tumor tissue-specific environment outside tumor cells, or utilizing the endocytosis effect of tumor cells, whereby ADC is endocytosed into lysosomes and the active payload molecule is released under the action of various enzymes in the lysosome to exert its biological function, such as inducing apoptosis, thereby achieving anti-tumor effects. Therefore, the key to ADC druggability lies in, on one hand, maintaining the stability of ADC in the general environment of the body as much as possible, such as stability in plasma circulation, and on the other hand, enabling ADC to release efficiently and rapidly upon reaching the disease site.

With respect to the stability in the body environment, the selection of the ADC linker and the selection of the linkage of the linker to the antibody often play a key role. Since the link of an antibody to a linker usually needs to be carried out in a solution mainly consisting of aqueous phases, and selective modes of reaction are relatively limited. For example, in the marketed ADCs such as Mylotarg^{®}, Besponsa^{®}, Kadcyla^{®}, Akalux^{®}, and Elahere^{®}, the amino group of the lysine residue on the antibody surface reacts with the activated ester on the linker to achieve antibody extension. Due to possessing good nucleophilicity, the amino group on the lysine residue can also react with various functional groups, such as o-phthalaldehyde. As for other marketed ADCs, the interchain disulfide bonds between the antibody light and heavy chains or heavy and heavy chains are reduced to obtain free sulfhydry groups on cysteine residues, and then the free sulfhydry groups are subjected to Michael addition with maleimide to achieve antibody extension. Since thiol sulfhydry groups have better nucleophilicity, there are more functional groups capable of reacting with sulfhydry groups in the reaction systems mainly consisting of aqueous phases. Therefore, there are more diverse means for extending antibodies via free sulfhydry groups. In addition, other methods can also achieve antibody extension to the linker. For example, amino acid point mutations are utilized to engineer on the antibody framework to result in cysteine or non-natural amino acids residues, and then the sulfhydry group on the engineered cysteine residue or the keto or azido group on the non-natural amino acid residue is utilized to react with a functional group on the linker. Alternatively, the antibody is subjected to polypeptide extending first, and then, a functional group on the extended polypeptide reacts with the linker. Alternativelys, the polypeptide is glycosylated, and then a functional group in the engineered glycosyl, typically a keto, azido, or alkynyl, is used to react with the linker. Alternativelys, enzymatic catalysis is utilized to perform enzymatic link of the antibody or the polypeptide-extended antibody to a corresponding enzyme substrate linker.

The selection of the linker structure often balances the stability of ADC in the environment and the release at the tumor site. A stable linker is one of options. For example, a linker with stable properties is selected for Kadcyla^{®}, and such linker can ensure the stability of ADC in the environment. However, the drawback is that the payload release efficiency is affected by ADCs with such linkers, and the released payload typically retains a portion of the linker. Hence, on one hand, the payload selection is limited; on the other hand, since the released payload often lacks a bystander effect, the application scope of the ADC drug is also restricted. In contrast, these issues are partially addressed by the use of linkers responsive to lysosomal enzymes. Since lysosomal enzymes are essentially absent extracellularly, ADCs with such linkers can still maintain considerable stability in the body environment, and can be efficiently released in a lysosome after undergoing endocytosis and completing lysosomal transport, thereby exerting the biological function of the payload. At the same time, this type of release mechanism usually enables the payload to be released in its intact structure. Since the payload often has cell-penetrating ability, ADCs prepared with such linkers generally exhibit a bystander effect, thereby exerting their anti-tumor efficacy better. A relatively-typical example of ADCs with such linkers is Enhertu^{®}. Under the premise of using the same antibody as Kadcyla^{®}, Enhertu^{®} demonstrates better therapeutic effect and a broader applicable population of patients than Kadcyla^{®}. However, such linkers also have shortcomings, namely, high dependence on endocytosis and lysosomal transport. Although the payload can be efficiently released after the ADC enters the lysosome, due to the influence of endocytosis efficiency and lysosomal transport efficiency, the actual payload release efficiency of such ADCs after reaching tumor tissue is not high. For antigenic targets with inherently low endocytosis efficiency, such linkers are essentially inapplicable. If a linker independent of endocytosis can be identified, the payload release efficiency can be further improved. In this regard, corresponding attempts are made on Trodelvy^{®} from Gilead. The CL2A-SN38 linker-payload used in Trodelvy^{®} can release the payload SN38 in the extracellular environment to exert anti-tumor activity. However, to achieve extracellular release, this linker sacrifices the stability of the ADC in the body environment. The half-life of Trodelvy^{®} in plasma is less than 24 hours, resulting in a significant discrepancy in pharmacokinetic properties between the ADC and a naked antibody. The pharmacokinetic deficiency can only be compensated by increasing the frequency of clinical injections, which impacts the patient experience of Trodelvy^{®} to some extent. The hematotoxicity caused by the instability of Trodelvy^{®} in plasma has also become the main clinical dose-limiting factor of Trodelvy^{®}.

Therefore, the development of linkers that can maintain relative stability in plasma and efficiently release payloads in the tumor microenvironment and/or lysosomes is of great significance to further improve the therapeutic effect of bioactive conjugates and to enhance patient medication experience, etc.

In addition, Tissue Factor (TF), also known as coagulation factor III, is a single-pass transmembrane protein. TF is expressed at certain levels in vascular wall cells, somatic cells, and others, to exert the function of hemostasis. Studies have shown that TF is highly expressed in various malignancies, such as cervical cancer, ovarian cancer, prostate cancer, breast cancer, non-small cell lung cancer, colon cancer, and pancreatic cancer. Moreover, high expression of TF is associated with poor prognosis of multiple tumors. Since TF itself lacks an intracellular domain and does not play a role in intracellular signal transduction, the development of monoclonal antibody drugs with TF as a target is not a favorable strategy. However, the high endocytosis efficiency of TF protein after antibody binding offers a promising avenue for the development of ADC drugs targeting TF. The marketed drug Tivdak^{®} is a TF-targeting ADC, approved for the indication of refractory or recurrent cervical cancer. The challenge in developing TF-targeting ADC drugs, as compared to the development of conventional ADC drugs, lies in balancing the antibody's binding ability to the TF antigen and the effect of the anti-TF antibody *per se* on coagulation.

### SUMMARY OF THE INVENTION

To improve the therapeutic effect of antibody-drug conjugates (ADCs) or other ligand-drug conjugates, reduce drug toxic and side effects, and to improvethe therapeutic window, it is a very important work to discover linkers that can maintain relative stability in plasma, and also can efficiently release payloads in the tumor microenvironment and/or lysosomes. The present application designs a series of polypeptides that are responsive to tumor tissue-specific MMP-2 and/or MMP-9, as well as to Cathepsin B in tumor cells, and spacer structures for connecting polypeptides with antibodies and payloads.

**In one aspect, the present application provides a polypeptide or polypeptide fragment having a structure represented by -P1-P2-, wherein polypeptides P1 and P2 are enzyme-responsive, respectively.**

In some embodiments, P1 comprises a polypeptide responsive to matrix metalloproteinase 2 (MMP-2) or matrix metalloproteinase 9 (MMP-9) or cathepsin B, and P2 comprises a polypeptide responsive to cathepsin B or matrix metalloproteinase 2 (MMP-2), or matrix metalloproteinase 9 (MMP-9).

In some embodiments, P1 comprises a polypeptide structure of -X1-P-X2-X3-Z-, and P2 comprises a polypeptide structure of -X1'-X2'-X3'-X4'-; wherein P is a proline residue; X1, X2, X3, Z, X1', X2', X3', and X4' each represent absence, an amino acid, or an amino acid derivative, and at least two of X1', X2', X3', and X4' are a single amino acid or a single amino acid derivative.

In some embodiments, P1 and P2 are responsive to matrix metalloproteinase 2 (MMP-2) and/or matrix metalloproteinase 9 (MMP-9), and Cathepsin B.

In some embodiments, X1 represents absence, a single amino acid, or a single amino acid derivative, X2, X3, and Z each represent a single amino acid or a single amino acid derivative, X1' and X2' each represent a single amino acid or a single amino acid derivative, and X3' and X4' each represent absence or a single amino acid or a single amino acid derivative.

In some embodiments, X1 is selected from absence, Gly (G), Val (V), His (H), Ser (S), and Ile (I); X2 is selected from Ala (A), Ser (S), Leu (L), Arg (R), Gln (Q), Val (V), and Cit; X3 is selected from Gly (G), Ser (S), Pro (P), Asn (N), and Ala (A); and Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylomithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylornithine (Orn(Et2)), and N,N-diethyllysine (Lys(Et2)).

In some embodiments, X1' is selected from Val (V), Als (A), Leu (L), Ile (I), Met (M), Phe (F), Trp (W), Pro (P), Tyr (Y), Gly (G), and Glu (E); X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Orn(Et2); X3' is selected from absence, Phe (F), Gly (G), Lys (K), Lys(Me2), Lys(Et2), His (H), Ala (A), Arg (R), Cit, Orn, Orn(Me2), and Orn(Et2); and X4' is selected from absence, Gly (G), and Phe (F).

In some embodiments, X1 is selected from absence, Gly (G), Val (V), His (H), Ser (S), and Ile (I); X2 is selected from Ala (A), Ser (S), Leu (L), Arg (R), Gln (Q), Val (V), and Cit; X3 is selected from Gly (G), Ser (S), Pro (P), Asn (N), and Ala (A); Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylornithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylornithine (Orn(Et2)), and N,N-diethyllysine (Lys(Et2)); X1' is selected from Val (V), Als (A), Leu (L), Ile (I), Met (M), Phe (F), Trp (W), Pro (P), Tyr (Y), Gly (G), and Glu (E); X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Orn(Et2); X3' is selected from absence, Phe (F), Gly (G), Lys (K), Lys(Me2), Lys(Et2), His (H), Ala (A), Arg (R), Cit, Orn, Orn(Me2), and Orn(Et2); and X4' is selected from absence, Gly (G), and Phe (F).

In some preferred embodiments, X1 is absent, or is Gly (G), Ser (S), or His (H); X2 is Ala (A), Leu (L), Arg (R), Ser (S), Leu (L), or Cit; X3 is Gly (G), Ser (S), Ala (A), Asn (N), or Pro (P); and Z is Leu (L) or Dap(Me2).

In some preferred embodiments, X1' is Val (V) or Gly (G), X2' is Gly (G) or Cit, and at least one of X3' and X4' is absent, or is Phe (F) or Gly (G). In some preferred embodiments, both X3' and X4' are absent. In some preferred embodiments, X3' is Phe (F), and X4' is Gly (G).

In some embodiments, as for the polypeptide or polypeptide fragment, (-X1'-X2'-X3'-X4'-) in P2 comprises a fragment of -Val-Cit- (V-Cit), -Ala-Lys- (AK), or -Gly-Gly-Phe-Gly- (GGFG). In some preferred embodiments, -X1'-X2'-X3'-X4'- is -V-Cit- or -GGFG-.

In some embodiments, any of the polypeptides or polypeptide fragments described herein is selected from:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
-Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50);
-Gly-Pro-Arg-Gly-Dap(Me2)-Val-Cit- (SEQ ID NO: 51);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67); and
-Pro-Ala-Ser-Leu-Val-Cit- (SEQ ID NO: 68);
or a variant having one or two mismatches with respect to any sequence of SEQ ID NOs:1-51 and 60-68.

In some embodiments, the polypeptide or the polypeptide fragment is selected from:
-GPLSLVCit- (SEQ ID NO: 4);
-GPASLVCit- (SEQ ID NO: 5);
-GPAGLVCit- (SEQ ID NO: 6);
-GPRGLVCit- (SEQ ID NO: 10);
-GPSALVCit- (SEQ ID NO: 12);
-GPLGLGGFG- (SEQ ID NO: 20);
-GPRGLGGFG- (SEQ ID NO: 22);
-GPAALGGFG- (SEQ ID NO: 23);
-PRGLVCit- (SEQ ID NO: 25);
-PRSLVCit- (SEQ ID NO: 26);
-PRNLVCit- (SEQ ID NO: 27);
-PRPLVCit- (SEQ ID NO: 28);
-PCitGLVCit- (SEQ ID NO: 29);
-PCitPLVCit- (SEQ ID NO: 30);
-GPRSLVCit- (SEQ ID NO: 31);
-GPRPLVCit- (SEQ ID NO: 32);
-GPRNLVCit- (SEQ ID NO: 33);
-GPCitGLVCit- (SEQ ID NO: 34);
-GPCitPLVCit- (SEQ ID NO: 35);
-HPRGLVCit- (SEQ ID NO: 36);
-HPCitPLVCit- (SEQ ID NO: 41);
-SPRGLVCit- (SEQ ID NO: 42);
-SPRSLVCit- (SEQ ID NO: 43);
-SPRPLVCit- (SEQ ID NO: 44);
-SPRNLVCit- (SEQ ID NO: 45);
-SPCitGLVCit- (SEQ ID NO: 46);
-SPCitSLVCit- (SEQ ID NO: 47);
-SPCitPLVCit- (SEQ ID NO: 48);
-GPAPLVCit- (SEQ ID NO: 49);
-GPANLVCit- (SEQ ID NO: 50);
-GPRNDap(Me2)VCit- (SEQ ID NO: 60);
-PLSLVCit- (SEQ ID NO: 61);
-GPRNLGGFG- (SEQ ID NO: 62);
-PCitSLVCit- (SEQ ID NO: 63);
-GPCitNLVCit- (SEQ ID NO: 64);
-SPCitSDap(Me2)VCit- (SEQ ID NO: 65);
-PRSDap(Me2)VCit- (SEQ ID NO: 66);
-PCitNLVCit- (SEQ ID NO: 67); and
-PASLVCit- (SEQ ID NO: 68);
or a variant having one or two mismatches with respect to any of the above sequences.

In some embodiments, the polypeptide or the polypeptide fragment is selected from:
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Arg-Gly-Leu-Val-Cit- (SEQ ID NO: 10);
-Gly-Pro-Ser-Ala-Leu-Val-Cit- (SEQ ID NO: 12);
-Gly-Pro-Leu-Gly-Leu-Gly-Gly-Phe-Gly- (SEQ ID NO: 20);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Asn-Leu-Val-Cit- (SEQ ID NO: 50);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67); and
-Pro-Ala-Ser-Leu-Val-Cit- (SEQ ID NO: 68);
or a variant having one or two mismatches with respect to any of the above sequences.

In some embodiments, the polypeptide or the polypeptide fragment is one or more selected from:
-GPASLVCit- (SEQ ID NO: 5);
-GPAGLVCit- (SEQ ID NO: 6);
-GPRGLVCit- (SEQ ID NO: 10);
-GPSALVCit- (SEQ ID NO: 12);
-GPLGLGGFG- (SEQ ID NO: 20);
-GPRGLGGFG- (SEQ ID NO: 22);
-GPAALGGFG- (SEQ ID NO: 23);
-PRGLVCit- (SEQ ID NO: 25);
-PRSLVCit- (SEQ ID NO: 26);
-PRNLVCit- (SEQ ID NO: 27);
-PRPLVCit- (SEQ ID NO: 28);
-PCitGLVCit- (SEQ ID NO: 29);
-PCitPLVCit- (SEQ ID NO: 30);
-GPRSLVCit- (SEQ ID NO: 31);
-GPRPLVCit- (SEQ ID NO: 32);
-GPRNLVCit- (SEQ ID NO: 33);
-GPCitGLVCit- (SEQ ID NO: 34);
-GPCitPLVCit- (SEQ ID NO: 35);
-HPRGLVCit- (SEQ ID NO: 36);
-HPCitPLVCit- (SEQ ID NO: 41);
-SPRGLVCit- (SEQ ID NO: 42);
-SPRSLVCit- (SEQ ID NO: 43);
-SPRPLVCit- (SEQ ID NO: 44);
-SPRNLVCit- (SEQ ID NO: 45);
-SPCitGLVCit- (SEQ ID NO: 46);
-SPCitSLVCit- (SEQ ID NO: 47);
-SPCitPLVCit- (SEQ ID NO: 48);
-GPAPLVCit- (SEQ ID NO: 49);
-GPANLVCit- (SEQ ID NO: 50);
-GPRNDap(Me2)VCit- (SEQ ID NO: 60);
-PLSLVCit- (SEQ ID NO: 61);
-GPRNLGGFG- (SEQ ID NO: 62);
-PCitSLVCit- (SEQ ID NO: 63);
-GPCitNLVCit- (SEQ ID NO: 64);
-SPCitSDap(Me2)VCit- (SEQ ID NO: 65);
-PRSDap(Me2)VCit- (SEQ ID NO: 66);
-PCitNLVCit- (SEQ ID NO: 67); and
-PASLVCit- (SEQ ID NO: 68).

In some embodiments, the polypeptide or the polypeptide fragment is one or more selected from:
-GPAGLVCit- (SEQ ID NO: 6);
-GPRGLVCit- (SEQ ID NO: 10);
-PRGLVCit- (SEQ ID NO: 25);
-PRSLVCit- (SEQ ID NO: 26);
-PRNLVCit- (SEQ ID NO: 27);
-PRPLVCit- (SEQ ID NO: 28);
-PCitGLVCit- (SEQ ID NO: 29);
-PCitPLVCit- (SEQ ID NO: 30);
-GPRSLVCit- (SEQ ID NO: 31);
-GPRPLVCit- (SEQ ID NO: 32);
-GPRNLVCit- (SEQ ID NO: 33);
-GPCitGLVCit- (SEQ ID NO: 34);
-GPCitPLVCit- (SEQ ID NO: 35);
-HPRGLVCit- (SEQ ID NO: 36);
-SPRGLVCit- (SEQ ID NO: 42);
-SPRSLVCit- (SEQ ID NO: 43);
-SPRPLVCit- (SEQ ID NO: 44);
-SPRNLVCit- (SEQ ID NO: 45);
-SPCitSLVCit- (SEQ ID NO: 47);
-SPCitPLVCit- (SEQ ID NO: 48);
-GPANLVCit- (SEQ ID NO: 50);
-GPRNDap(Me2)VCit- (SEQ ID NO: 60);
-PLSLVCit- (SEQ ID NO: 61);
-GPRNLGGFG- (SEQ ID NO: 62);
-PCitSLVCit- (SEQ ID NO: 63);
-GPCitNLVCit- (SEQ ID NO: 64);
-SPCitSDap(Me2)VCit- (SEQ ID NO: 65);
-PRSDap(Me2)VCit- (SEQ ID NO: 66);
-PCitNLVCit- (SEQ ID NO: 67); and
-PASLVCit- (SEQ ID NO: 68).

**In another aspect, the present application provides a linker (also referred to as a "linker moiety"), comprising the above polypeptide or polypeptide fragment having the structure represented by -PI-P2-.**

In some embodiments, the linker has a general formula of C 1-La-L1-P 1-P2-L2,
wherein C1 is an adapter for connecting a bioligand unit;
La is absent or a tuning spacer;
L1 is absent or a spacer;
P1-P2 is the polypeptide or polypeptide fragment as described above;
L2 is absent or a spacer for connecting a payload with a specific biological function; and
positions of La and L1 are interchangeable.

In some embodiments, the bioligand unit comprises an antibody, antibody fragment, protein, polypeptide, polynucleotide, chemical small molecule, but is not limited thereto.

In some embodiments, in the linker, C1 is selected from: 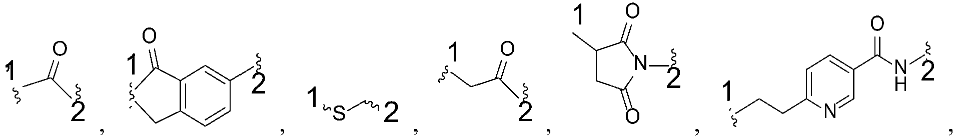 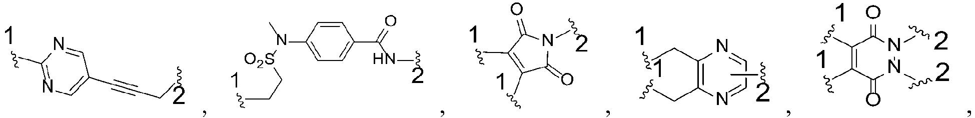 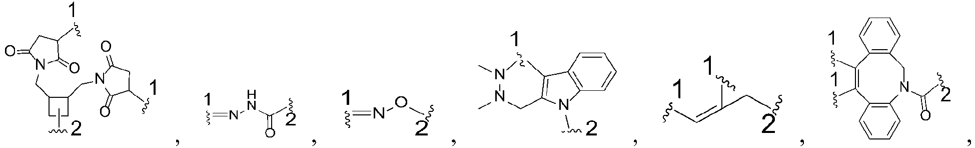 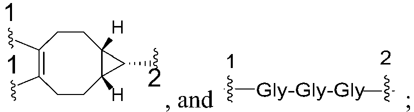 wherein 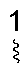 indicates a position for connection to the bioligand unit, and 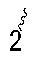 indicates a position for connection to La.

In some embodiments, C1 comprises a structure of -(succinimid-3-yl-N)-, -CH₂-C(=O)-, 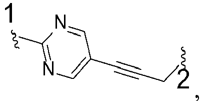 or -C(=O)-, wherein -(succinimid-3-yl-N)- has a structure of 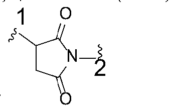 wherein 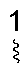 indicates a position for connection to the bioligand unit, and 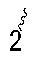 indicates a position for connection to La.

In some embodiments, C1 is -(succinimid-3-yl-N)-.

In some embodiments, La is absent or is selected from the following structure or a combination thereof:
R1-S-S-R2,
R1-(CH₂CH₂O)ₘ-R2,
R1-(CH₂CH₂O)ₘ-CH₂CH₂S-(CH₂CH₂O)ₙ-R2,
R1-(CH₂CH₂O)ₘ-CH₂CH₂NH-(CH₂CH₂O)ₙ-R2,
R1-(CH₂CH₂O)ₘ-CH₂CH₂(C=O)NH-(CH₂CH₂O)ₙ-R2, 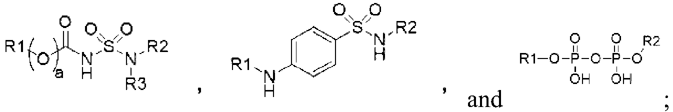
a is 0 or 1;
m is any integer from 1 to 20;
n is any integer from 1 to 20;
R3 is H, -C₁-C₁₀ alkylene-, -C₃-C₈ carbocyclyl-, -C₂-C₁₀ heteroaryl-, or -C₃-C₂₄ alkylheteroaryl-, wherein -C₁-C₁₀ alkylene-, -C₃-C₈ carbocyclyl-, -C₂-C₁₀ heteroaryl-, -C₃-C₂₄ alkylheteroaryl- can be substituted by one or more heteroatoms, and the heteroatom can be O, S, or NR³, wherein R³ is H or -C₁-C₄ alkylene; and
R1 is a structure for connection to C1, and R2 is a structure for connection to L1.

In some embodiments, R1 is a single bond or is selected from the following structures:
-X¹-C₁-C₁₀ alkylene-X²-, -X¹-C₁-C₁₀ heteroalkylene-X²-, -X¹-C₃-C₈ carbocyclyl-X²-, -X¹-arylene-X²-, -X¹-C₁-C₁₀ alkylene-arylene-X²-, -X¹-arylene-C₁-C₁₀ alkylene-X²-, -X¹-C₁-C₁₀ alkylene-(C₃-C₈ carbocyclyl)-X²-, -X¹-(C₃-C₈ carbocyclyl)-C₁-C₁₀ alkylene-X²-, -X¹-C₃-C₈ heterocyclyl-X²-, -X¹-C₁-C₁₀ alkylene-(C₃-C₈ heterocyclyl)-X²-, and -X¹-(C₃-C₈ heterocyclyl)-C₁-C₁₀ alkylene-X²-;
wherein X¹ is absent or is selected from O, S, NH, C(=O), NHC(=O), C(=O)NH, and NHC(=O)NH, and X² is absent or is selected from O, NH, S, C(=O), NHC(=O), C(=O)NH, and NHC(=O)NH.

When R1 is not a single bond, R1 is optionally substituted with a basic unit (BU). The basic unit is -(CH₂)ₓNH₂, -(CH₂)ₓNHRₘ, or -(CH₂)ₓN(Rₘ)₂; wherein x is any integer from 1 to 4; and each Rₘ is independently selected from C₁-C₆ alkyl and C₁-C₆ haloalkyl, or two Rₘ groups, in combination with the nitrogen to which they are attached, form a 4- to 6-membered heterocycloalkyl ring, or an azetidinyl, pyrrolidinyl, or piperidinyl group.

In some embodiments, R2 is a single bond or is selected from the following structures:
-X³-C₁-C₁₀ alkylene-X⁴-, -X³-C₁-C₁₀ heteroalkylene-X⁴-, -X³-C₃-C₈ carbocyclyl-X⁴-, -X³-arylene-X⁴-, -X³-C₁-C₁₀ alkylene-arylene-X⁴-, -X³-arylene-C₁-C₁₀ alkylene-X⁴-, -X³-C₁-C₁₀ alkylene-(-C₃-C₈ carbocyclyl)-X⁴-, -X³-(-C₃-C₈ carbocyclyl)-C₁-C₁₀ alkylene-X⁴-, -X³-C₃-C₈ heterocyclyl-X⁴-, -X³-C₁-C₁₀ alkylene-(-C₃-C₈ heterocyclyl)-X⁴-, and -X³-(-C₃-C₈ heterocyclyl)-C₁-C₁₀ alkylene-X⁴-;
wherein X³ is absent or is O, S, NH, C(=O), NHC(=O), C(=O)NH, or NHC(=O)NH, and X⁴ is absent or is selected from O, S, NH, C(=O), NHC(=O), C(=O)NH, or NHC(=O)NH.

When R2 is not a single bond, R2 is optionally substituted with a basic unit (BU). The basic unit is -(CH₂)ₓNH₂, -(CH₂)ₓNHRₘ, or -(CH₂)ₓN(Rₘ)₂; wherein x is any integer from 1 to 4; and each Rₘ is independently selected from C₁-C₆ alkyl and C₁-C₆ haloalkyl, or two Rₘ groups, in combination with the nitrogen to which they are attached, form a 4- to 6-membered heterocycloalkyl ring, or an azetidinyl, pyrrolidinyl, or piperidinyl group.

In some embodiments, La is absent or is selected from the following structures:
-C₁-C₁₀ alkylene-C(=O)- (for example, -C₂-C₈ alkylene-C(=O)-, -C₂-C₇ alkylene-C(=O)-, -C₂-C₆ alkylene-C(=O)-), -C₁-C₁₀ alkylene-C(=O)NH- (for example, -C₂-C₆ alkylene-C(=O)NH-, -C₂-C₅ alkylene-C(=O)NH-, -C₂-C₄ alkylene-C(=O)NH-),
-C₁-C₁₀ alkylene-C(=O)NH-(CH₂CH₂O)ₘ- (for example, -C₂-C₆ alkylene-C(=O)NH-(CH₂CH₂O)ₘ-, -C₂-C₅ alkylene-C(=O)NH-(CH₂CH₂O)ₘ-, -C₂-C₄ alkylene-C(=O)NH-(CH₂CH₂O)ₘ-),
-C₁-C₁₀ alkylene-C(=O)NH-(CH₂CH₂O)ₘ-C₁-C₁₀ alkylene-C(=O)- (for example, -C₂-C₆ alkylene-C(=O)NH-(CH₂CH₂Oₘ-C₂-C₆ alkylene-C(=O)-, -C₂-C₅ alkylene-C(=O)NH-(CH₂CH₂Oₘ-C₂-C₅ alkylene-C(=O)-, and -C₂-C₅ alkylene-C(=O)NH-(CH₂CH₂Oₘ-C₂-C₅ alkylene-C(=O)-),
m is any integer selected from 3 to 15, and preferably 5 to 10, 6 to 9, or 7 to 8.

In some embodiments, L1 is selected from the following structures:
-X⁵C₁-C₁₀ alkylene-X⁶-, -X⁵-C₁-C₁₀ heteroalkylene-X⁶-, -X⁵-C₃-C₈ carbocyclyl-X⁶-, -X⁵-arylene-X⁶-, -X⁵-heteroarylene-X⁶-, -X⁵-C₁-C₁₀ alkylene-arylene-X⁶-, -X⁵-C₁-C₁₀ alkylene-heteroarylene-X⁶-, -X⁵-arylene-C₁-C₁₀ alkylene-X⁶-, -X⁵-heteroarylene-C₁-C₁₀ alkylene-X⁶-, -X⁵-C₁-C₁₀ alkylene-(C₃-C₈ carbocyclyl)-X⁶-, -X⁵-(C₃-C₈ carbocyclyl)-C₁-C₁₀ alkylene-X⁶-, -X⁵-C₃-C₈ heterocyclyl-X⁶-, -X⁵-C₁-C₁₀ alkylene-(C₃-C₈ heterocyclyl)-X⁶-, -X⁵-(C₃-C₈ heterocyclyl)-C₁-C₁₀ alkylene-X⁶-, -X⁵-C₃-C₈ heterocyclyl-arylene-X⁶-, -X⁵-arylene-C₃-C₈ heterocyclyl-X⁶-, -X⁵-C₃-C₈ heterocyclyl-heteroarylene-X⁶-, and -X⁵-heteroarylene-C₃-C₈ heterocyclyl-X⁶-;
wherein X ⁵ is absent or is selected from O, S, NH, C(=O), NHC(=O), C(=O)NH, or NHC(=O)NH, and X ⁶ is absent or is selected from O, S, NH, C(=O), NHC(=O), C(=O)NH, and NHC(=O)NH; and
wherein any methylene unit of L1 can independently be substituted with -O-, -S-, -N(Rₙ)-, - CH(N(Rₙ)₂)-, -CH(P(Rₙ)₂)-, -C(N(Rₙ)₂)₂-, -C(ORₙ)(N(Rₙ)₂)-, -C(=O)-, -OC(=O)-, -C(=O)O-, - N(Rₙ)C(=O)-, -C(=O)N(Rₙ)-, -SO-, -SO₂-, -N(Rₙ)SO₂-, -SO₂N(Rₙ)-, -P(Rₙ)-, -P(=O)(Rₙ)-, - P(=O)(O)-, -C(=S)-, -C(=N-Rₙ)-, -S-S-, -N=N-, -N(Rₙ)-N=, =N-N(Rₙ)-, -C=N-, or -N=C-; wherein each Rₙ is independently selected from H, C₁-C₆ alkyl, C₃-C₈ carbocyclyl, and C₁-C₆ haloalkyl; or two Rₙ groups, in combination with the nitrogen or phosphorus to which they are attached, form a 4-to 6-membered heterocycloalkyl ring, or an azetidinyl, pyrrolidinyl, or piperidinyl group.

In some embodiments, L1 is absent or is -C₁-C₁₀ alkylene-C(=O)- (for example, -C₂-C₈ alkylene-C(=O)-, -C₂-C₇ alkylene-C(=O)-, -C₂-C₆ alkylene-C(=O)-, -C₂-C₅ alkylene-C(=O)-, or -C₂-C₄ alkylene-C(=O)-).

In some embodiments, C1-La-L1- is 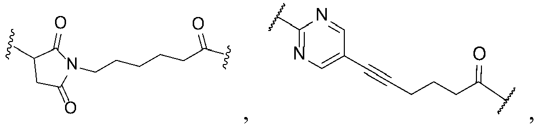 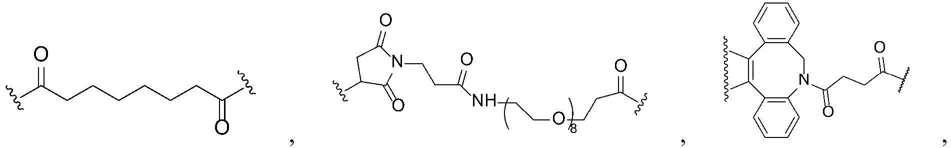 or 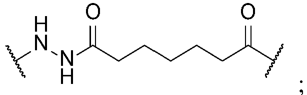 and preferably

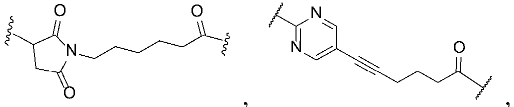

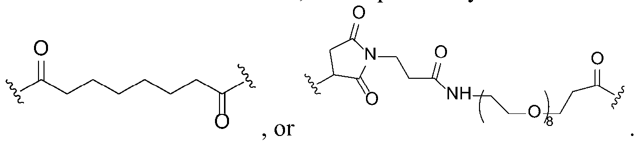

In some embodiments, L2 is absent or is selected from the following structures: 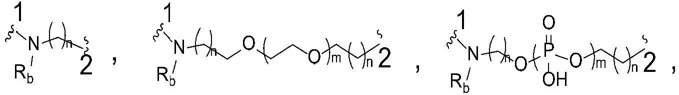 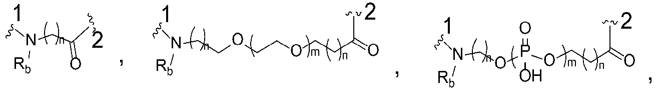 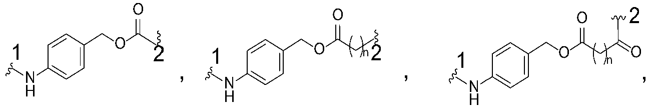 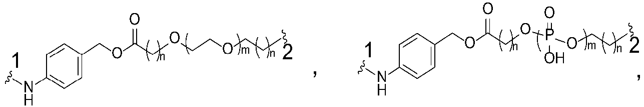 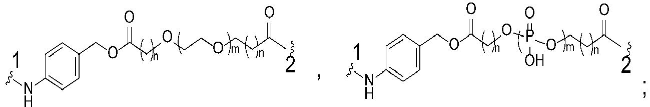
wherein 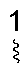 indicates a position for connection to the polypeptide, and 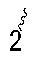 indicates a position for connection to a payload with a specific biological function;
m is any integer from 1 to 20; and
n is any integer from 1 to 6; and
R_{b} is independently selected from H, C₁-C₆ alkyl, C₃-C₈ carbocyclyl, C₁-C₆ haloalkyl, C₃-C₈ heterocyclyl, C₁-C₆ alkyl-C₃-C₈ heterocyclyl, substitutable aryl, substitutable heteroaryl, C₁-C₆ alkyl-substitutable aryl, and C₁-C₆ alkyl-substitutable heteroaryl.

Wherein, any methylene unit of L2 can be independently substituted with -CHX-, -C(X₂)-, -C₃-C₈ carbocyclyl-, -C₃-C₈ heterocyclyl-, -arylene-, -heteroarylene-, -O-, -S-, -N(Rₙ)-, -CH(N(Rₙ)₂)-, - CH(P(Rₙ)₂)-, -C(N(Rₙ)₂)₂-, -C(ORₙ)(N(Rₙ)₂)-, -C(=O)-, -OC(=O)-, -C(=O)O-, -N(Rₙ)C(=O)-, - C(=O)N(Rₙ)-, -SO-, -SO₂-, -N(Rₙ)SO₂-, -SO₂N(Rₙ)-, -P(Rₙ)-, -P(=O)(Rₙ)-, -P(=O)(O)-, -C(=S)-, - C(=N-Rₙ)-, -S-S-, -N=N-, -N(Rₙ)-N=, =N-N(Rₙ)-, -C=N-, or -N=C-; wherein X represents halogen or deuterium, and each Rₙ is independently selected from H, C₁-C₆ alkyl, C₃-C₈ cycloalkyl, and C₁-C₆ haloalkyl; or two Rₙ groups, in combination with the nitrogen or phosphorus to which they are attached, form a 4- to 6-membered heterocycloalkyl ring, or an azetidinyl, pyrrolidinyl, or piperidinyl group.

In some embodiments, L2 is absent or is selected from the following structures: 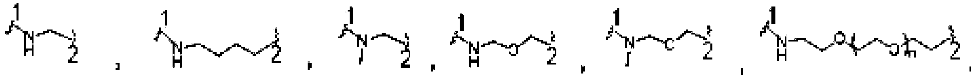 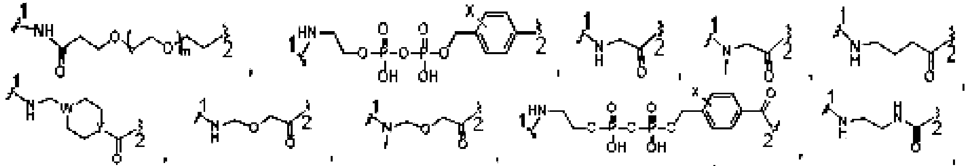 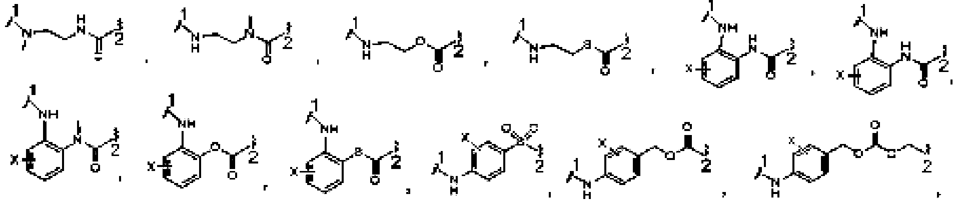 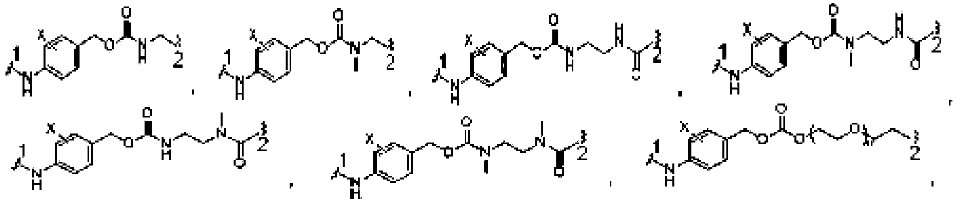 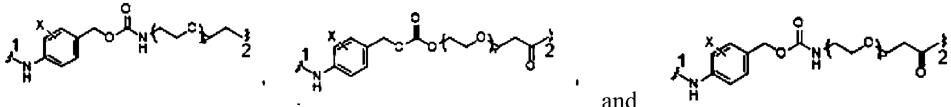
wherein 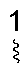 indicates a position for connection to the polypeptide, and 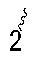 indicates a position for connection to a payload with a specific biological function;
W and Y are each independently N or CR^{Y}, and each R^{Y} is independently H or C₁-C₁₀ alkyl;
m is any integer from 0 to 20; and
X is halogen or deuterium.

In some embodiments, L2 is absent or selected from: 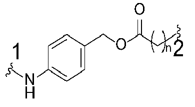
(for example, 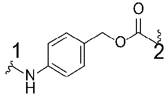 ), 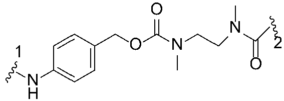
(for example, 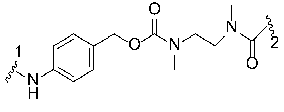 ), 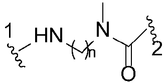
(for example, 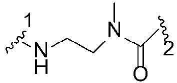 ); 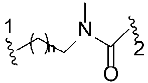
(for example, 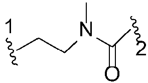 ), and
n is any integer selected from 1 to 6, 1-5, 1-4, or 1-3.

In some embodiments, L2 is absent or selected from -PAB- ( 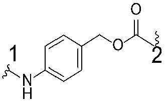 ), -PAB-DMEDA-( 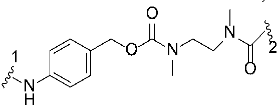 ), and -NMEDA- ( 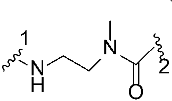 ).

In some embodiments, the linker is selected from the following structures:
-(succinimid-3-yl-N)-CH2-C(=O)-P1-P2-;
-(succinimid-3-yl-N)-CH2CH2-C(=O)-P 1-P2-;
-(succinimid-3-yl-N)-(CH2CH2O)₄-CH2CH2-C(=O)-P1-P2-;
-(succinimid-3-yl-N)-(CH2CH2O)₈-CH2CH2-C(=O)-P1-P2-;
-(succinimid-3-yl-N)-CH2CH2C(O)NH-(CH2CH2O)₄-CH2CH2-C(=O)-P1-P2-;
-(succinimid-3-yl-N)-CH2CH2C(O)NH-(CH2CH2O)₈-CH2CH2-C(=O)-P1-P2-;
-(succinimid-3-yl-N)-OC(O)-(CH2CH2O)₄-CH2CH2-C(=O)-P1-P2-;
-(succinimid-3-yl-N)-OC(O)-(CH2CH2O)₈-CH2CH2-C(=O)-P1-P2-;
-(succinimid-3-yl-N)-CH2CH2CH2CH2CH2-C(=O)-P1-P2-;
-(succinimid-3-yl-N)-CH2-C(=O)-P1-P2-PAB-;
-(succinimid-3-yl-N)-CH2CH2-C(=O)-P1-P2-PAB-;
-(succinimid-3-yl-N)-(CH2CH2O)₄-CH2CH2-C(=O)-P1-P2-PAB-;
-(succinimid-3-yl-N)-(CH2CH2O)₈-CH2CH2-C(=O)-P1-P2-PAB-;
-(succinimid-3-yl-N)-CH2CH2C(O)NH-(CH2CH2O)₄-CH2CH2-C(=O)-P1-P2-PAB-;
-(succinimid-3-yl-N)-CH2CH2C(O)NH-(CH2CH2O)₈-CH2CH2-C(=O)-P1-P2-PAB-;
-(succinimid-3-yl-N)-OC(O)-(CH2CH2O)₄-CH2CH2-C(=O)-P1-P2-PAB-;
-(succinimid-3-yl-N)-OC(O)-(CH2CH2O)₈-CH2CH2-C(=O)-P1-P2-PAB-;
-(succinimid-3-yl-N)-CH2CH2CH2CH2CH2-C(=O)-P1-P2-PAB-; 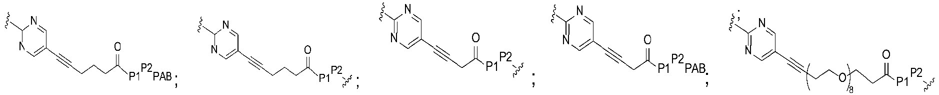 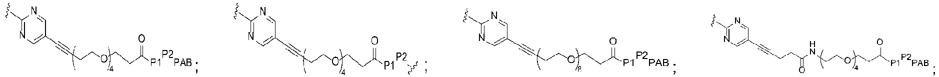 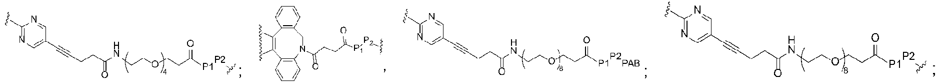 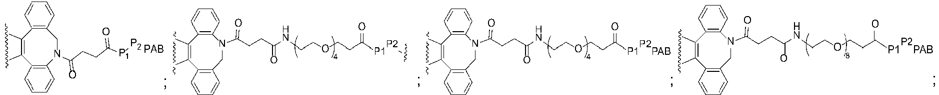 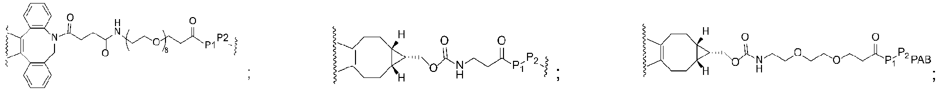 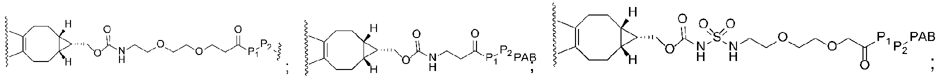 and 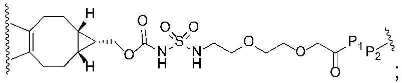
wherein:
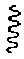 indicates a position for connection to the bioligand unit.
PAB represents the following structure: 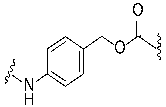
-P1-P2- represents the following structure:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50);
-Gly-Pro-Arg-Gly-Dap(Me2)-Val-Cit- (SEQ ID NO: 51);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67);
-Pro-Ala-Ser-Leu-Val-Cit- (SEQ ID NO: 68);
or a variant having one or two mismatches relative to any sequence of SEQ ID NOs:1-51 and 60-68.

In some embodiments, the linker is selected from:
-(succinimid-3-yl-N)-C1-C10 alkylene-C(=O)-P1-P2-;
-(succinimid-3-yl-N)-C1-C10 alkylene-C(=O)NH-P1-P2-;
-(succinimid-3-yl-N)-C1-C10 alkylene-C(=O)NH-(CH₂CH₂O)ₘ-P1-P2-;
-(succinimid-3-yl-N)-C1-C10 alkylene-C(=O)NH-(CH₂CH₂O)ₘ-C₁-C₁₀ alkylene-C(=O)-P1-P2-;
-(succinimid-3-yl-N)-C1-C10 alkylene-C(=O)-P1-P2-PAB-;
-(succinimid-3-yl-N)-C1-C10 alkylene-C(=O)NH-P1-P2-PAB-;
-(succinimid-3-yl-N)-C1-C10 alkylene-C(=O)NH-(CH₂CH₂O)ₘ-P1-P2-PAB-;
-(succinimid-3-yl-N)-C1-C10 alkylene-C(=O)NH-(CH₂CH₂O)ₘ-C₁-C₁₀ alkylene-C(=O)-P1-P2-PAB-;
-(succinimid-3-yl-N)-C1-C10 alkylene-C(=O)-P1-P2-PAB-DMEDA-;
-(succinimid-3-yl-N)-C1-C10 alkylene-C(=O)NH-P1-P2-PAB-DMEDA-;
-(succinimid-3-yl-N)-C1-C10 alkylene-C(=O)NH-(CH₂CH₂O)ₘ-P1-P2-PAB-DMEDA-;
-(succinimid-3-yl-N)-C1-C10 alkylene-C(=O)NH-(CH₂CH₂O)ₘ-C₁₋C₁₀ alkylene-C(=O)-P1-P2-PAB-DMEDA-;
-(succinimid-3-yl-N)-C1-C10 alkylene-C(=O)-P1-P2-NMEDA-;
-(succinimid-3-yl-N)-C1-C10 alkylene-C(=O)NH-P1-P2-NMEDA-;
-(succinimid-3-yl-N)-C1-C10 alkylene-C(=O)NH-(CH₂CH₂O)ₘ-P1-P2-NMEDA-;
-(succinimid-3-yl-N)-C1-C10 alkylene-C(=O)NH-(CH₂CH₂O)ₘ-C₁-C₁₀ alkylene-C(=O)-P1-P2-NMEDA-;
-(C=O)-C1-C10 alkylene-C(=O)-P1-P2-;
-(C=O)-C1-C10 alkylene-C(=O)-P1-P2-PAB-;
-(C=O)-C1-C10 alkylene-C(=O)NH-P1-P2-PAB-;
-(C=O)-C1-C10 alkylene-C(=O)-P1-P2-PAB-DMEDA-;
-(C=O)-C1-C10 alkylene-C(=O)NH-P1-P2-PAB-DMEDA-;
-(C=O)-C1-C10 alkylene-C(=O)-P1-P2-NMEDA-;
-(C=O)-C1-C10 alkylene-C(=O)NH-P1-P2-NMEDA-; 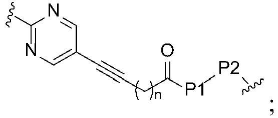 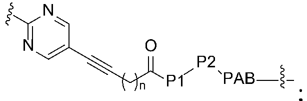 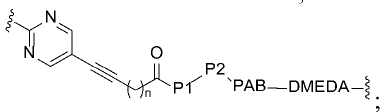 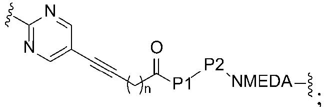 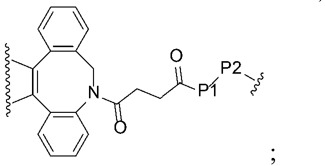 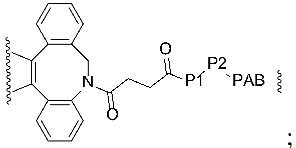 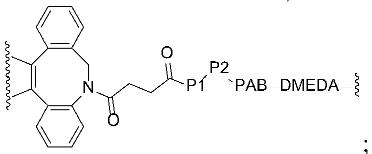 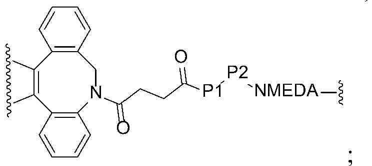 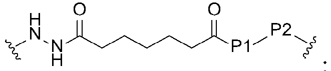 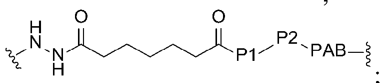 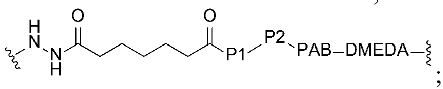 and 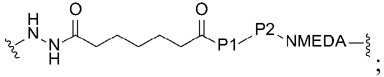
m is any integer selected from 3-15, preferably 5-10, 6-9, or 7-8; and
n is any integer selected from 1-10, preferably 2-6, 2-5, or 2-4.

In some embodiments, the linker is selected from the following structures:
-(succinimid-3-yl-N)-C5 alkylene-C(=O)-P1-P2-;
-(succinimid-3-yl-N)-C5 alkylene-C(=O)-P1-P2-PAB-;
-(succinimid-3-yl-N)-C5 alkylene-C(=O)-P1-P2-PAB-DMEDA-;
-(succinimid-3-yl-N)-C5 alkylene-C(=O)-P1-P2-NMEDA-; 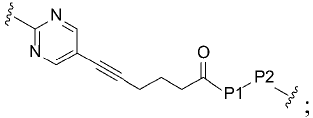 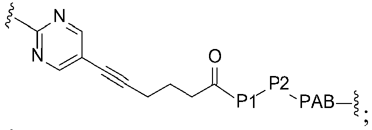 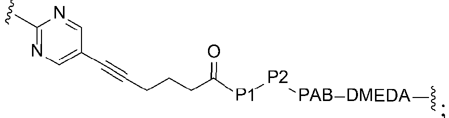 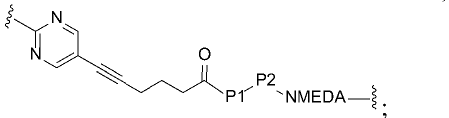
-(C=O)-C6 alkylene-C(=O)-P1-P2-;
-(C=O)-C6 alkylene-C(=O)-P1-P2-PAB-;
-(C=O)-C6 alkylene-C(=O)-P1-P2-PAB-DMEDA-;
-(C=O)-C6 alkylene-C(=O)-P1-P2-NMEDA-;
-(succinimid-3-yl-N)-C2 alkylene-C(=O)NH-(CH₂CH₂O)₈-C2 alkylene-C(=O)-P1-P2-;
-(succinimid-3-yl-N)-C2 alkylene-C(=O)NH-(CH₂CH₂O)₈-C2 alkylene-C(=O)-P1-P2-PAB-;
-(succinimid-3-yl-N)-C2 alkylene-C(=O)NH-(CH₂CH₂O)₈-C2 alkylene-C(=O)-P1-P2-PAB-DMEDA-;
-(succinimid-3-yl-N)-C2 alkylene-C(=O)NH-(CH₂CH₂O)₈-C2 alkylene-C(=O)-P1-P2-NMEDA-; 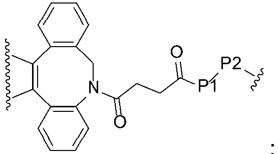 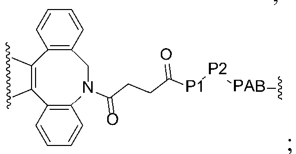 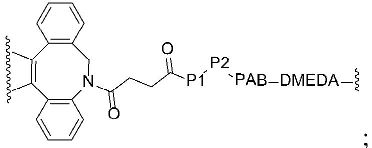 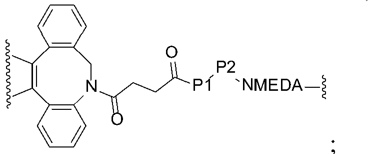 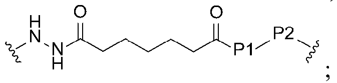 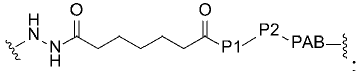 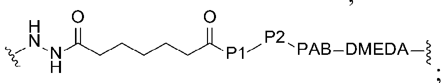 and

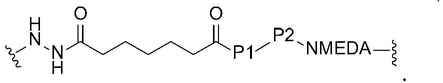

In some embodiments, the linker is one or more selected from:
-(succinimid-3-yl-N)-C5 alkylene-C(=O)-GPAGLVCit-PAB-;
-(succinimid-3-yl-N)-C5 alkylene-C(=O)-GPLGLGGFG-;
-(succinimid-3-yl-N)-C5-alkylene-C(=O)-GPASLVCit-PAB-;
-(succinimid-3-yl-N)-C5-alkylene-C(=O)-GPRGLVCit-PAB-;
-(succinimid-3-yl-N)-C5-alkylene-C(=O)-GPSALVCit-PAB-;
-(succinimid-3-yl-N)-C5-alkylene-C(=O)-GPRGLGGFG-;
-(succinimid-3-yl-N)-C5-alkylene-C(=O)-GPAALGGFG-;
-(succinimid-3-yl-N)-C5-alkylene-C(=O)-PRNLVCit-PAB-;
-(succinimid-3-yl-N)-C5-alkylene-C(=O)-GPRNLVCit-PAB-;
-(succinimid-3-yl-N)-C5-alkylene-C(=O)-GPRNDap(Me2)VCit-PAB-;
-(succinimid-3-yl-N)-C5-alkylene-C(=O)-SPRNLVCit-PAB-;
-(succinimid-3-yl-N)-C5-alkylene-C(=O)-GPLSLVCit-PAB-;
-(succinimid-3-yl-N)-C5-alkylene-C(=O)-PRGLVCit-PAB-;
-(succinimid-3-yl-N)-C5-alkylene-C(=O)-PRPLVCit-PAB-;
-(succinimid-3-yl-N)-C5-alkylene-C(=O)-GPRSLVCit-PAB-;
-(succinimid-3-yl-N)-C5-alkylene-C(=O)-GPRPLVCit-PAB-;
-(succinimid-3-yl-N)-C5-alkylene-C(=O)-GPCitPLVCit-PAB-;
-(succinimid-3-yl-N)-C5-alkylene-C(=O)-HPRGLVCit-PAB-;
-(succinimid-3-yl-N)-C5-alkylene-C(=O)-HPCitPLVCit-PAB-;
-(succinimid-3-yl-N)-C5-alkylene-C(=O)-SPCitGLVCit-PAB-;
-(succinimid-3-yl-N)-C5-alkylene-C(=O)-GPAPLVCit-PAB-;
-(succinimid-3-yl-N)-C5-alkylene-C(=O)-PLSLVCit-PAB-;
-(succinimid-3-yl-N)-C5-alkylene-C(=O)-SPCitSLVCit-PAB-; 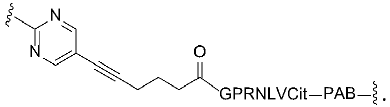 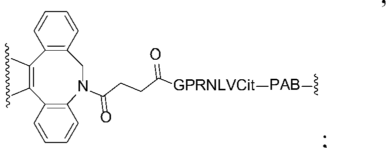
-(C=O)-C6-alkylene-C(=O)-GPRNLVCit-PAB-; 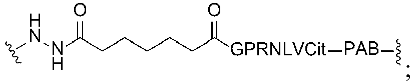
-(succinimid-3-yl-N)-C2-alkylene-C(=O)NH-(CH₂CH₂O)₈-C2-alkylene-C(=O)-GPRNLVCit-PAB-;
-(succinimid-3-yl-N)-C5-alkylene-C(=O)-GPRNLVCit-PAB-DMEDA-;
-(succinimid-3-yl-N)-C5-alkylene-C(=O)-GPRNLGGFG-;
-(succinimid-3-yl-N)-C5-alkylene-C(=O)-GPRNLGGFG-NMEDA-;
-(succinimid-3-yl-N)-C5-alkylene-C(=O)-PRSLVCit-PAB-;
-(succinimid-3-yl-N)-C5-alkylene-C(=O)-SPRSLVCit-PAB-;
-(succinimid-3-yl-N)-C5-alkylene-C(=O)-PCitSLVCit-PAB-;
-(succinimid-3-yl-N)-C5-alkylene-C(=O)-PCitPLVCit-PAB-;
-(succinimid-3-yl-N)-C5-alkylene-C(=O)-PCitGLVCit-PAB-;
-(succinimid-3-yl-N)-C5-alkylene-C(=O)-GPCitGLVCit-PAB-;
-(succinimid-3-yl-N)-C5-alkylene-C(=O)-SPRGLVCit-PAB-;
-(succinimid-3-yl-N)-C5-alkylene-C(=O)-SPRPLVCit-PAB-;
-(succinimid-3-yl-N)-C5-alkylene-C(=O)-SPCitPLVCit-PAB-;
-(succinimid-3-yl-N)-C5-alkylene-C(=O)-GPANLVCit-PAB-;
-(succinimid-3-yl-N)-C5-alkylene-C(=O)-GPCitNLVCit-PAB-;
-(succinimid-3-yl-N)-C5-alkylene-C(=O)-SPCitSDap(Me2)VCit-PAB-;
-(succinimid-3-yl-N)-C5-alkylene-C(=O)-PRSDap(Me2)VCit-PAB-;
-(succinimid-3-yl-N)-C5-alkylene-C(=O)-PCitNLVCit-PAB-; and
-(succinimid-3-yl-N)-C5-alkylene-C(=O)-PASLVCit-PAB-.

In some embodiments, the linker is selected from the following structures:
-(succinimid-3-yl-N)-CH₂CH₂CH₂CH₂CH₂-C(=O)-GPLGLGGFG-;
-(succinimid-3-yl-N)-CH₂CH₂CH₂CH₂CH₂-C(=O)-GPRGLGGFG-;
-(succinimid-3-yl-N)-CH₂CH₂CH₂CH₂CH₂-C(=O)-GPAALGGFG-;
-(succinimid-3-yl-N)-CH₂CH₂CH₂CH₂CH₂-C(=O)-GPAGLVCit-PAB-;
-(succinimid-3-yl-N)-CH₂CH₂CH₂CH₂CH₂-C(=O)-GPASLVCit-PAB-;
-(succinimid-3-yl-N)-CH₂CH₂CH₂CH₂CH₂-C(=O)-GPRGLVCit-PAB-;
-(succinimid-3-yl-N)-CH₂CH₂CH₂CH₂CH₂-C(=O)-GPSALVCit-PAB-;
-(succinimid-3-yl-N)-CH₂CH₂CH₂CH₂CH₂-C(=O)-PRNLVCit-PAB-;
-(succinimid-3-yl-N)-CH₂CH₂CH₂CH₂CH₂-C(=O)-GPRNLVCit-PAB-;
-(succinimid-3-yl-N)-CH₂CH₂CH₂CH₂CH₂-C(=O)-GPRGDap(Me2)VCit-PAB-;
-(succinimid-3-yl-N)-CH₂CH₂CH₂CH₂CH₂-C(=O)-SPRNLVCit-PAB-; and
-(succinimid-3-yl-N)-CH₂CH₂CH₂CH₂CH₂-C(=O)-GPLSLVCit-PAB-;
wherein PAB represents a structure of: 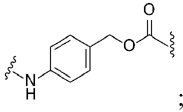 and
Dap(Me2) represents a structure of

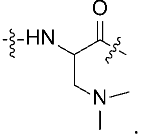

**In another aspect, the present application provides a linker-payload (also referred to as a "linker-payload intermediate"), comprising the above polypeptide or polypeptide fragment having the structure represented by -PI-P2-.**

In some embodiments, the linker-payload has a structure of:
C1'-La-L1-P1-P2-L2-D,
wherein C1' is a functional group that reacts with the bioligand unit;
La is absent or a tuning spacer;
L1 is absent or a spacer;
P1-P2 is a polypeptide or polypeptide fragment as described in any of the embodiments herein;
L2 is absent or a spacer for connecting a payload with a specific biological function;
D is a drug unit; and
positions of La and L1 are interchangeable.

In some embodiments, the tuning spacer La is the structural unit described in the preceding aspect.

In some embodiments, the spacer L1 is the spacer described in the preceding aspect.

In some embodiments, the spacer L2 is the spacer described in the preceding aspect.

In some embodiments, C1' is selected from functional groups that react with amino, sulfhydryl, keto, azido, or alkynyl.

In some embodiments, C1' is selected from enzyme-catalyzed reaction substrates.

In some embodiments, C1' is selected from the following structures: 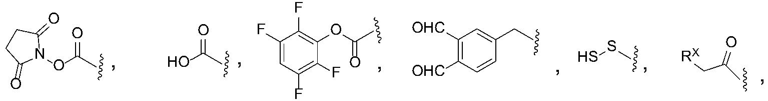 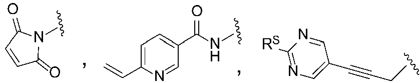 (e.g., 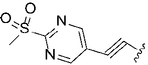 ), 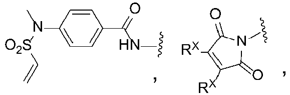 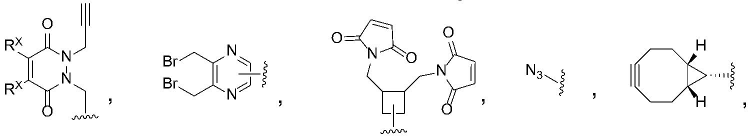 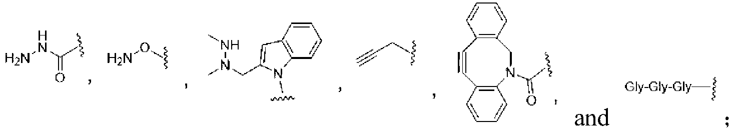 wherein,
R^{X} is selected from halogen, -SPh; R^{S} is selected from halogen, sulfonyl, a tertiary-amine group, a diazonium salt group, -OMs, MeSO₂-, and CF₃SO₃-; and
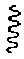 indicates a position for connection to La.

In some embodiments, C1' is selected from the following structures:

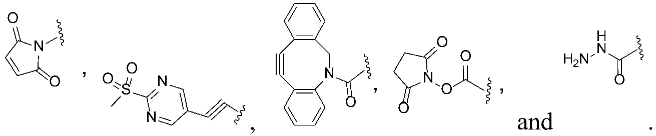

In some embodiments, C1'-La-L1- is MC- ( 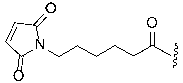 ), MeO2S-Pym-( 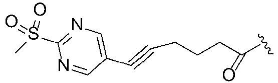 ), NHS- ( 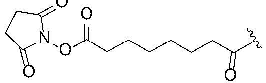 ), Mal-PEG8-( 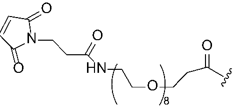 ), DBCO- ( 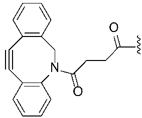 ), or Hydrazide- ( 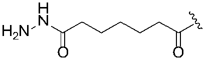 ); and preferably MC-, MeO2S-Pym-, NHS-, or Mal-PEG8-.

In some embodiments, the drug unit D is selected from immunomodulators, protein degrading agent, and cytotoxic agents.

In some embodiments, the drug unit D is selected from cytotoxic agents.

In some embodiments, the drug unit includes: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4]benzodi-azepines (PBDs), puromycins, rhizoxins, SN-38, taxanes, tubulysins, vincaalkaloids, tetrahydroisoquinoline alkaloids or derivatives thereof, protein degrading agents or derivatives thereof.

In some embodiments, the drug unit includes a DNA topoisomerase I inhibitor, a tubulin inhibitor, a DNA replication inhibitor, or a protein degrading agent.

In some embodiments, the drug unit includes a maytansinoid or derivatives thereof, a camptothecin or derivatives thereof, an auristatin or derivatives thereof, a tetrahydroisoquinoline alkaloid or derivatives thereof, a BTK protein degrading agent and derivatives thereof, or a GSPT1 protein degrading agent and derivatives thereof.

In some embodiments, the drug unit includes DM1, DM4, DX8951, MMAE, Dxd, SN38, Trabectedin (ET743), Lurbinectedin, CC885, or CC-90009.

In some embodiments, the linker-payload is selected from:
MC-P1-P2-D;
MC-P1-P2-PAB-D;
MC-P1-P2-PAB-DMEDA-D;
MC-P1-P2-NMEDA-D;
MeO2S-Pym-P1-P2-D;
MeO2S-Pym-P1-P2-PAB-D;
MeO2S-Pym-P1-P2-PAB-DMEDA-D;
MeO2S-Pym-P1-P2-NMEDA-D;
NHS-P1-P2-D;
NHS-P1-P2-PAB-D;
NHS-P1-P2-PAB-DMEDA-D;
NHS-P1-P2-NMEDA-D;
Mal-PEG8-P1-P2-D;
Mal-PEG8-P1-P2-PAB-D;
Mal-PEG8-P1-P2-PAB-DMEDA-D;
Mal-PEG8-P1-P2-NMEDA-D;
DBCO-P1-P2-D;
DBCO-P1-P2-PAB-D;
DBCO-P1-P2-PAB-DMEDA-D;
DBCO-P1-P2-NMEDA-D;
Hydrazide-P1-P2-D;
Hydrazide-P1-P2-PAB-D;
Hydrazide-P1-P2-PAB-DMEDA-D; and
Hydrazide-P1-P2-NMEDA-D_{∘}

In some preferred embodiments, the drug unit D includes DX8951, MMAE, Dxd, SN38, or Trabectedin (ET743).

In some embodiments, the linker-payload has a structure of:
(maleimid-N-yl)-CH2-C(=O)-P1-P2-D;
(maleimid-N-yl)-CH2CH2-C(=O)-P1-P2-D;
(maleimid-N-yl)-(CH2CH2O)₄-CH2CH2-C(=O)-P1-P2-D;
(maleimid-N-yl)-(CH2CH2O)₈-CH2CH2-C(=O)-P1-P2-D;
(maleimid-N-yl)-CH2CH2C(O)NH-(CH2CH2O)₄-CH2CH2-C(=O)-P1-P2-D;
(maleimid-N-yl)-CH2CH2C(O)NH-(CH2CH2O)₈-CH2CH2-C(=O)-P1-P2-D;
(maleimid-N-yl)-OC(O)-(CH2CH2O)₄-CH2CH2-C(=O)-P1-P2-D;
(maleimid-N-yl)-OC(O)-(CH2CH2O)₈-CH2CH2-C(=O)-P1-P2-D;
(maleimid-N-yl)-CH2CH2CH2CH2CH2-C(=O)-P1-P2-D;
(maleimid-N-yl)-CH2-C(=O)-P1-P2-PAB-D;
(maleimid-N-yl)-CH2CH2-C(=O)-P1-P2-PAB-D;
(maleimid-N-yl)-(CH2CH2O)₄-CH2CH2-C(=O)-P1-P2-PAB-D;
(maleimid-N-yl)-(CH2CH2O)₈-CH2CH2-C(=O)-P1-P2-PAB-D;
(maleimid-N-yl)-CH2CH2C(O)NH-(CH2CH2O)₄-CH2CH2-C(=O)-P1-P2-PAB-D;
(maleimid-N-yl)-CH2CH2C(O)NH-(CH2CH2O)₈-CH2CH2-C(=O)-P1-P2-PAB-D;
(maleimid-N-yl)-OC(O)-(CH2CH2O)₄-CH2CH2-C(=O)-P1-P2-PAB-D;
(maleimid-N-yl)-OC(O)-(CH2CH2O)₈-CH2CH2-C(=O)-P1-P2-PAB-D;
(maleimid-N-yl)-CH2CH2CH2CH2CH2-C(=O)-P1-P2-PAB-D;
(N-hydroxysuccinimide ester-1-yl)-CH2-C(=O)-P1-P2-D;
(N-hydroxysuccinimide ester-1-yl)-CH2CH2-C(=O)-P1-P2-D;
(N-hydroxysuccinimide ester-1-yl)-(CH2CH2O)₄-CH2CH2-C(=O)-P1-P2-D;
(N-hydroxysuccinimide ester-1-yl)-(CH2CH2O)₈-CH2CH2-C(=O)-P1-P2-D;
(N-hydroxysuccinimide ester-1-yl)-CH2CH2C(O)NH-(CH2CH2O)₄-CH2CH2-C(=O)-P1-P2-D;
(N-hydroxysuccinimide ester-1-yl)-CH2CH2C(O)NH-(CH2CH2O)₈-CH2CH2-C(=O)-P1-P2-D;
(N-hydroxysuccinimide ester-1-yl)-(1,4-cyclohexylene)-CH2-C(=O)-P1-P2-D;
(N-hydroxysuccinimide ester-1-yl)-(1,4-cyclohexylene)-CH2NH-(CH2CH2O)₄-CH2CH2-C(=O)-P1-P2-D;
(N-hydroxysuccinimide ester-1-yl)-(1,4-cyclohexylene)-CH2NH-(CH2CH2O)₈-CH2CH2-C(=O)-P1-P2-D;
(N-hydroxysuccinimide ester-1-yl)-(1,4-cyclohexylene)-CH2C(=O)NH-(CH2CH2O)₄-CH2CH2-C(=O)-P1-P2-D;
(N-hydroxysuccinimide ester-1-yl)-(1,4-cyclohexylene)-CH2C(=O)NH-(CH2CH2O)₈-CH2CH2-C(=O)-P1-P2-D;
(N-hydroxysuccinimide ester-1-yl)-(1,4-cyclohexylene)-CH2O-(CH2CH2O)₄-CH2CH2-C(=O)-P1-P2-D;
(N-hydroxysuccinimide ester-1-yl)-(1,4-cyclohexylene)-CH2O-(CH2CH2O)₈-CH2CH2-C(=O)-P1-P2-D;
(N-hydroxysuccinimide ester-1-yl)-CH2CH2CH2CH2CH2-C(=O)-P1-P2-D;
(N-hydroxysuccinimide ester-1-yl)-CH2-C(=O)-P1-P2-PAB-D;
(N-hydroxysuccinimide ester-1-yl)-CH2CH2-C(=O)-P1-P2-PAB-D;
(N-hydroxysuccinimide ester-1-yl)-(CH2CH2O)₄-CH2CH2-C(=O)-P1-P2-PAB-D;
(N-hydroxysuccinimide ester-1-yl)-(CH2CH2O)₈-CH2CH2-C(=O)-P1-P2-PAB-D;
(N-hydroxysuccinimide ester-1-yl)-CH2CH2C(O)NH-(CH2CH2O)₄-CH2CH2-C(=O)-P1-P2-PAB-D;
(N-hydroxysuccinimide ester-1-yl)-CH2CH2C(O)NH-(CH2CH2O)₈-CH2CH2-C(=O)-P1-P2-PAB-D;
(N-hydroxysuccinimide ester-1-yl)-(1,4-cyclohexylene)-CH2-C(=O)-P1-P2-PAB-D;
(N-hydroxysuccinimide ester-1-yl)-(1,4-cyclohexylene)-CH2NH-(CH2CH2O)₄-CH2CH2-C(=O)-P1-P2-PAB-D;
(N-hydroxysuccinimide ester-1-yl)-(1,4-cyclohexylene)-CH2NH-(CH2CH2O)₈-CH2CH2-C(=O)-P1-P2-PAB-D;
(N-hydroxysuccinimide ester-1-yl)-(1,4-cyclohexylene)-CH2C(=O)NH-(CH2CH2O)₄-CH2CH2-C(=O)-P1-P2-PAB-D;
(N-hydroxysuccinimide ester-1-yl)-(1,4-cyclohexylene)-CH2C(=O)NH-(CH2CH2O)₈-CH2CH2-C(=O)-P1-P2-PAB-D;
(N-hydroxysuccinimide ester-1-yl)-(1,4-cyclohexylene)-CH2O-(CH2CH2O)₄-CH2CH2-C(=O)-P1-P2-PAB-D;
(N-hydroxysuccinimide ester-1-yl)-(1,4-cyclohexylene)-CH2O-(CH2CH2O)₈-CH2CH2-C(=O)-P 1-P2-PAB-D;
(N-hydroxysuccinimide ester-1-yl)-CH2CH2CH2CH2CH2-C(=O)-P1-P2-PAB-D;
Gly-Gly-Gly-NH-CH2CH2CH2CH2CH2-C(=O)-P1-P2-D;
Gly-Gly-Gly-NH-CH2CH2CH2CH2CH2-C(=O)-P1-P2-PAB-D; 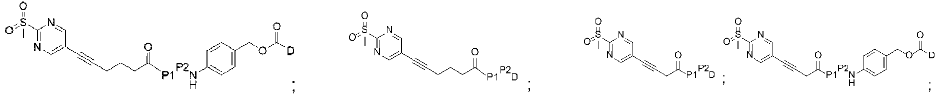 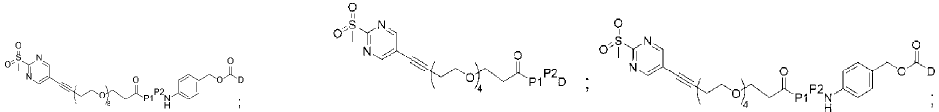 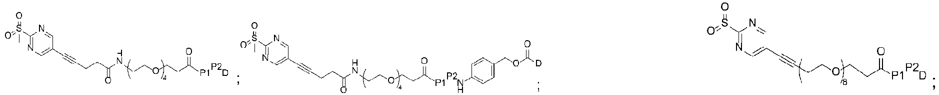 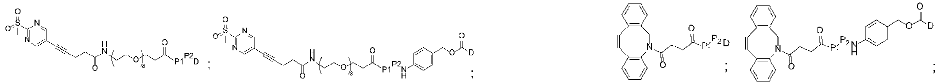 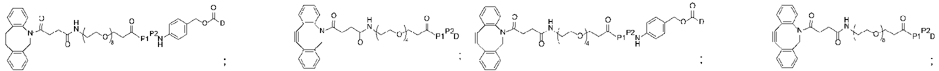 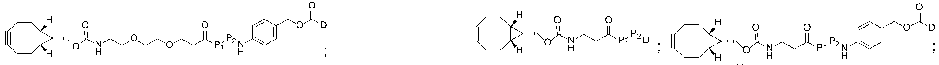 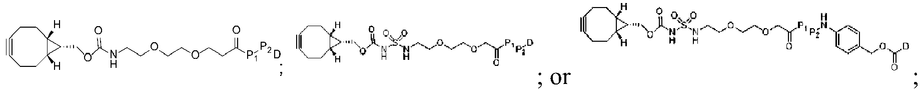
the (maleimid-N-yl)- has a structure of 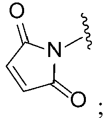 and
the (N-hydroxysuccinimide ester-1-yl)- has a structure of 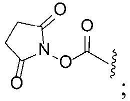
wherein 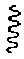 indicates a position for connection to La, L1, or P1-P2.

-P1-P2- represents the following structure:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50);
-Gly-Pro-Arg-Gly-Dap(Me2)-Val-Cit- (SEQ ID NO: 51);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67);
-Pro-Ala-Ser-Leu-Val-Cit- (SEQ ID NO: 68);
or a variant having one or two mismatches relative to any sequence of SEQ ID NOs:1-51 and 60-68.

D is camptothecin or a derivative thereof, auristatin or a derivative thereof, a tetrahydroisoquinoline alkaloid or a derivative thereof, a PROTAC protein degrading agent, or a molecular glue degrader. Preferably, D is DX8951, MMAE, Dxd, SN38, ET743, CC885, or CC-90009.

In some embodiments, the linker-payload is one or more selected from:
MC-GPAGLVCit-PAB-DX8951;
MC-GPAGLVCit-PAB-MMAE;
MC-GPAGLVCit-PAB-ET743;
MC-GPLGLGGFG-DX8951;
MC-GPASLVCit-PAB-DX8951;
MC-GPRGLVCit-PAB-DX8951;
MC-GPRGLVCit-PAB-MMAE;
MC-GPSALVCit-PAB-DX8951;
MC-GPRGLGGFG-DX8951;
MC-GPAALGGFG-DX8951;
MC-PRNLVCit-PAB-DX8951;
MC-PRNLVCit-PAB-MMAE;
MC-GPRNLVCit-PAB-DX8951;
MC-GPRNLVCit-PAB-MMAE;
MC-GPRNLVCit-PAB-ET743;
MC-GPRNDap(Me2)VCit-PAB-DX8951;
MC-GPRNDap(Me2)VCit-PAB-MMAE;
MC-SPRNLVCit-PAB-DX8951;
MC-SPRNLVCit-PAB-MMAE;
MC-GPLSLVCit-PAB-DX8951 ;
MC-GPLSLVCit-PAB-MMAE;
MC-PRGLVCit-PAB-DX8951;
MC-PRPLVCit-PAB-DX8951 ;
MC-GPRSLVCit-PAB-DX8951;
MC-GPRPLVCit-PAB-DX8951;
MC-GPCitPLVCit-PAB-DX8951 ;
MC-HPRGLVCit-PAB-DX8951;
MC-HPCitPLVCit-PAB-DX8951;
MC-SPCitGLVCit-PAB-DX8951 ;
MC-GPAPLVCit-PAB-DX895 1;
MC-PLSLVCit-PAB-DX8951;
MC-SPCitSLVCit-PAB-DX8951 ;
MC-SPCitSLVCit-PAB-MMAE;
MC-SPCitSLVCit-PAB-ET743;
MeO2S-Pym-GPRNLVCit-PAB-DX8951;
DBCO-GPRNLVCit-PAB-DX8951 ;
NHS-GPRNLVCit-PAB-DX8951;
Hydrazide-GPRNLVCit-PAB-DX8951 ;
Mal-PEG8-GPRNLVCit-PAB-DX8951 ;
MC-GPRNLVCit-PAB-DMEDA-SN38;
MC-GPRNLGGFG-Dxd;
MC-GPRNLGGFG-NMEDA-Dxd;
MC-PRSLVCit-PAB-DX8951;
MC-SPRSLVCit-PAB-DX8951
MC-PCitSLVCit-PAB-DX8951;
MC-PCitPLVCit-PAB-DX8951;
MC-PCitGLVCit-PAB-DX8951;
MC-GPCitGLVCit-PAB-DX8951;
MC-SPRGLVCit-PAB-DX8951;
MC-SPRPLVCit-PAB-DX8951;
MC-SPCitPLVCit-PAB-DX8951;
MC-GPANLVCit-PAB-DX8951 ;
MC-GPCitNLVCit-PAB-DX8951;
MC-SPCitSDap(Me2)VCit-PAB-DX8951;
MC-SPCitSDap(Me2)VCit-PAB-MMAE;
MC-PRSDap(Me2)VCit-PAB-DX8951;
MC-PRSDap(Me2)VCit-PAB-MMAE;
MC-PCitNLVCit-PAB-DX8951; and
MC-PASLVCit-PAB-DX8951.

In some embodiments, the linker-payload has a structure of:
MC-GPAGLVCit-PAB-DX8951;
MC-GPLGLGGFG-DX8951;
MC-GPASLVCit-PAB-DX8951;
MC-GPRGLVCit-PAB-DX8951;
MC-GPSALVCit-PAB-DX8951;
MC-GPRGLGGFG-DX8951;
MC-GPAALGGFG-DX8951;
MC-PRNLVCit-PAB-DX8951;
MC-GPRNLVCit-PAB-DX8951 ;
MC-GPRGDap(Me2)LVCit-PAB-DX8951;
MC-SPRNLVCit-PAB-DX8951;
MC-GPLSLVCit-PAB-DX8951;
MC-PRGLVCit-PAB-DX8951;
MC-PRPLVCit-PAB-DX8951;
MC-GPRSLVCit-PAB-DX8951;
MC-GPRPLVCit-PAB-DX8951 ;
MC-GPCitPLVCit-PAB-DX8951;
MC-HPRGLVCit-PAB-DX8951;
MC-HPCitPLVCit-PAB-DX8951;
MC-SPCitGLVCit-PAB-DX8951;
MC-GPAPLVCit-PAB-DX8951;
MC-PLSLVCit-PAB-DX8951;
MC-SPCitSLVCit-PAB-DX8951;
MC-PRSLVCit-PAB-DX8951;
MC-SPRSLVCit-PAB-DX8951;
MC-PCitSLVCit-PAB-DX8951 ;
MC-PCitPLVCit-PAB-DX8951;
MC-PCitGLVCit-PAB-DX8951;
MC-GPCitGLVCit-PAB-DX8951;
MC-SPRGLVCit-PAB-DX8951;
MC-SPRPLVCit-PAB-DX8951;
MC-SPCitPLVCit-PAB-DX8951;
MC-GPANLVCit-PAB-DX8951
MC-GPCitNLVCit-PAB-DX8951;
MC-SPCitSDap(Me2)VCit-PAB-DX8951;
MC-PRSDap(Me2)VCit-PAB-DX8951;
MC-GPAGLVCit-PAB-MMAE;
MC-GPRGLVCit-PAB-MMAE;
MC-PRNLVCit-PAB-MMAE;
MC-GPRNLVCit-PAB-MMAE;
MC-GPRGDap(Me2)LVCit-PAB-MMAE;
MC-SPRNLVCit-PAB-MMAE;
MC-GPLSLVCit-PAB-MMAE;
MC-SPCitSLVCit-PAB-MMAE
MC-SPCitSDap(Me2)VCit-PAB-MMAE
MC-SPCitSDap(Me2)VCit-PAB-MMAE
MC-GPAGLVCit-PAB-ET743;
MC-GPRNLVCit-PAB-ET743; or
MC-SPCitSLVCit-PAB-ET743;
MC- has a structure of 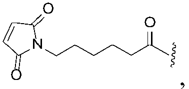
wherein 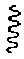 indicates a position for connection to the polypeptide,
DX8951 has a structure of 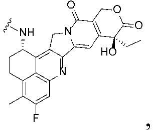
wherein 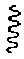 indicates a position of DX8951 connecting to the linker,
MMAE has a structure of 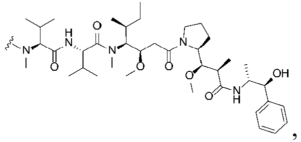
wherein 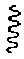 indicates a position of MMAE connecting to the linker, and
ET-743 has a structure of 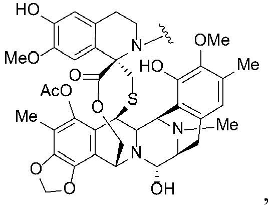
wherein 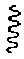 indicates a position of ET743 connecting to the linker.

**In another aspect, the present application provides a bioligand-drug conjugate, for example, an antibody-drug conjugate, wherein the conjugate comprises the above polypeptide or polypeptide fragment having the structure represented by -PI-P2-.**

In some embodiments, the conjugate has a general formula of: 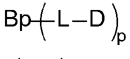 wherein,
Bp is a bioligand;
L is a linker comprising the polypeptide or polypeptide fragment having the structure represented by -P1-P2-;
D is a drug unit; and
p is any value from 1 to 20 (for example, any integer).

In some embodiments, the conjugate has a general formula of: 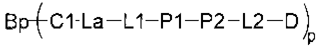 wherein,
Bp is a bioligand;
C1, La, L1, and L2 are as defined in any of the embodiments herein;
-P1-P2- unit is a polypeptide or polypeptide fragment as described in some embodiments herein;
D is a drug unit; and
p is any value from 1 to 20 (for example, any integer).

In some embodiments, the drug unit D is as defined in any of the preceding aspects.

In some embodiments, the bioligand targets a cell surface receptor or a tumor-associated antigen.

In some embodiments, the cell surface receptor or the tumor-associated antigen comprises EGFR, HER2, HER3, c-Met, claudin18.2, TROP-2, folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, IGF-1R, TNFR1, or GLP-1.

In some embodiments, the bioligand unit comprises an antibody, an antibody fragment, protein, polypeptide, polynucleotide, chemical small molecule, but is not limited thereto. In some embodiments, the bioligand comprises an antibody or an antigen-binding fragment thereof, a modified antibody or an antigen-binding fragment thereof, an oligonucleotide, a DNA fragment, a nucleic acid aptamer, a polypeptide, a nanoparticle with targeting ability, or a chemically-synthesized small molecule.

In some embodiments, the bioligand comprises an antibody or an antigen-binding fragment thereof, a modified antibody or an antigen-binding fragment thereof.

In some embodiments, the antibody includes a monoclonal antibody, a polyclonal antibody, a dimer, a polymer, a multispecific antibody, an intact antibody, an antibody fragment, a human antibody, a humanized antibody, or a chimeric antibody.

In some embodiments, the modified antibody includes a modified monoclonal antibody, a modified polyclonal antibody, a modified dimer, a modified polymer, a modified multispecific antibody, a modified intact antibody, a modified antibody fragment, a modified human antibody, a modified humanized antibody, or a modified chimeric antibody.

In some embodiments, the antigen-binding fragment includes Fab, Fab', Fv fragment, F(ab')2, scFv, di-scFv, and/or dAb.

In some embodiments, the modified antigen-binding fragment includes modified Fab, modified Fab', modified Fv fragment, modified F(ab')2, modified scFv, modified di-scFv, and/or modified dAb.

In some embodiments, the antibody is a monoclonal antibody or a modified monoclonal antibody.

In some embodiments, the antibody is a human antibody, a humanized antibody, or a chimeric antibody. Wherein the modified antibody is a modified human antibody, a modified humanized antibody, or a modified chimeric antibody.

In some embodiments, the modification method of the modified antibody is: amino acid point mutation, glycosylation modification, or polypeptide modification.

In some embodiments, the antibody or the antigen-binding fragment thereof, the modified antibody or the modified antigen-binding fragment thereof is: anti-EGFR, anti-HER2, anti-HER3, anti-c-Met, anti-claudin18.2, anti-TROP-2, anti-FRα, anti-ROR1, anti-ROR2, anti-BCMA, anti-PSMA, anti-CD19, anti-CD20, anti-CD22, anti-CD30, anti-CD33, anti-CD37, anti-CD46, anti-CD48, anti-CD56, anti-CD79b, anti-CD123, anti-CD138, anti-CD166, anti-CD276, anti-CEACAM5, anti-SDC1, anti-CD74, anti-CD70, anti-MUC1, anti-MUC16, anti-GUCY2C, anti-MSLN, anti-SCL34A2, anti-FOLH1, anti-TPBC, anti-PVRL4, anti-STEAP1, anti-SCL44A4, anti-NACM1, anti-EDNRB, anti-GPNMB, anti-FGFR, anti-SEZ6, anti-Nectin-4, anti-PD-L1, anti-TF, anti-B7-H3, anti-B7-H4, anti-LY6E, anti-LIV-1, anti-CDH4, anti-CDH6, anti-ITGB6, anti-GPC1, anti-GPC3, anti-ENPP3, anti-KAAG1, anti-FZD7, anti-SSTR2, anti-DLK1, anti-TMEFF1, anti-TM4SF1, anti-SLAMF7, anti-DLL3, anti-FLT3, anti-TNFRSF10B, anti-EPCAM, anti-AXL, anti-IL2RA, anti-5T4, anti-FAP, anti-ICAM-1, anti-IGF-1R, anti-TNFR1, or anti-GLP-1.

In some embodiments, the antibody or the antigen-binding fragment thereof, the modified antibody or the modified antigen-binding fragment thereof is an anti-Her2, anti-TF, anti-ROR1, or anti-GPC3 antibody or an antigen bindingfragment thereof.

In some embodiments, the antibody is Trastuzumab, Tisotumab, Zilovertamab, or Codrituzumab.

In some embodiments, the antibody-drug conjugate is selected from the following structures:
Ab-((succinimid-3-yl-N)-CH2-C(=O)-P1-P2-D)ₙ;
Ab-((succinimid-3-yl-N)-CH2CH2-C(=O)-P1-P2-D)ₙ;
Ab-((succinimid-3-yl-N)-(CH2CH2O)₄-CH2CH2-C(=O)-P1-P2-D)ₙ;
Ab-((succinimid-3-yl-N)-(CH2CH2O)₈-CH2CH2-C(=O)-P1-P2-D)ₙ;
Ab-((succinimid-3-yl-N)-CH2CH2C(O)NH-(CH2CH2O)₄-CH2CH2-C(=O)-P1-P2-D)ₙ;
Ab-((succinimid-3-yl-N)-CH2CH2C(O)NH-(CH2CH2O)₈-CH2CH2-C(=O)-P1-P2-D)ₙ;
Ab-((succinimid-3-yl-N)-OC(O)-(CH2CH2O)₄-CH2CH2-C(=O)-P1-P2-D)ₙ;
Ab-((succinimid-3-yl-N)-OC(O)-(CH2CH2O)₈-CH2CH2-C(=O)-P1-P2-D)ₙ;
Ab-((succinimid-3-yl-N)-CH2CH2CH2CH2CH2-C(=O)-P1-P2-D)ₙ;
Ab-((succinimid-3-yl-N)-CH2-C(=O)-P1-P2-PAB-D)ₙ;
Ab-((succinimid-3-yl-N)-CH2CH2-C(=O)-P1-P2-PAB-D)ₙ;
Ab-((succinimid-3-yl-N)-(CH2CH2O)₄-CH2CH2-C(=O)-P1-P2-PAB-D)ₙ;
Ab-((succinimid-3-yl-N)-(CH2CH2O)₈-CH2CH2-C(=O)-P1-P2-PAB-D)ₙ;
Ab-((succinimid-3-yl-N)-CH2CH2C(O)NH-(CH2CH2O)₄-CH2CH2-C(=O)-P1-P2-PAB-D)ₙ;
Ab-((succinimid-3-yl-N)-CH2CH2C(O)NH-(CH2CH2O)₈-CH2CH2-C(=O)-P1-P2-PAB-D)ₙ;
Ab-((succinimid-3-yl-N)-OC(O)-(CH2CH20)₄-CH2CH2-C(=O)-P1-P2-PAB-D)ₙ;
Ab-((succinimid-3-yl-N)-OC(O)-(CH2CH2O)₈-CH2CH2-C(=O)-P1-P2-PAB-D)ₙ;
Ab-((succinimid-3-yl-N)-CH2CH2CH2CH2CH2-C(=O)-P1-P2-PAB-D)ₙ; 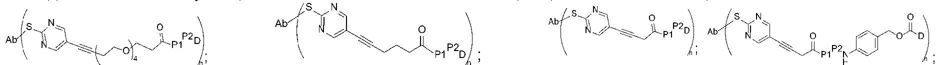 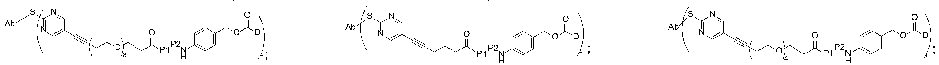 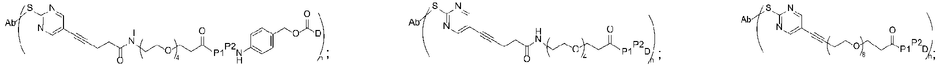 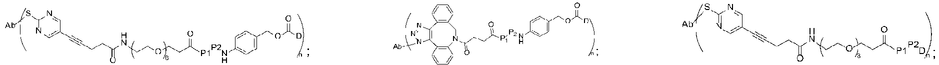 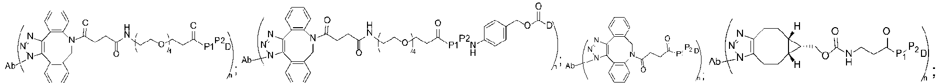 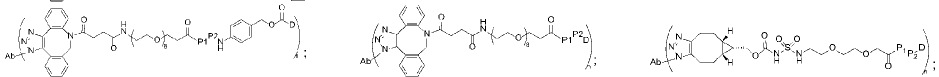 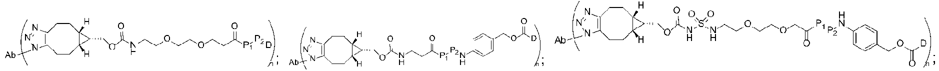 and 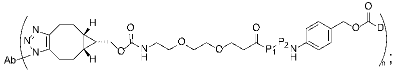
Ab represents a monoclonal antibody or an antigen binding fragment thereof, or a modified monoclonal antibody or an antigen binding fragment thereof, and preferably an anti-Her2, anti-TF, anti-ROR1, or anti-GPC3 antibody or an antigen binding fragment thereof;
PAB has a structure of: 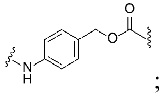 and
wherein the left 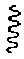 indicates a position for connection to P2, and the right 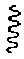 indicates a position for connection to the drug unit;
-P1-P2- represents the following structure:
   -His-Val-Leu-Asn-Leu-Val-Cit- (SEQ ID NO: 1);
   -Val-Pro-Leu-Ser-Leu-Val-Cit- (SEQ ID NO: 2);
   -Ile-Pro-Val-Ser-Leu-Val-Cit- (SEQ ID NO: 3);
   -Gly-Pro-Leu-Ser-Leu-Val-Cit- (SEQ ID NO: 4);
   -Gly-Pro-Ala-Ser-Leu-Val-Cit- (SEQ ID NO: 5);
   -Gly-Pro-Ala-Gly-Leu-Val-Cit- (SEQ ID NO: 6);
   -Gly-Pro-Gln-Gly-Leu-Val-Cit- (SEQ ID NO: 7);
   -Gly-Pro-Leu-Gly-Leu-Val-Cit- (SEQ ID NO: 8);
   -Gly-Pro-Ser-Gly-Leu-Val-Cit- (SEQ ID NO: 9);
   -Gly-Pro-Arg-Gly-Leu-Val-Cit- (SEQ ID NO: 10);
   -Gly-Pro-Ala-Ala-Leu-Val-Cit- (SEQ ID NO: 11);
   -Gly-Pro-Ser-Ala-Leu-Val-Cit- (SEQ ID NO: 12);
   -His-Val-Leu-Asn-Leu-Gly-Gly-Phe-Gly- (SEQ ID NO: 13);
   -Val-Pro-Leu-Ser-Leu-Gly-Gly-Phe-Gly- (SEQ ID NO: 14);
   - Ile-Pro-Val-Ser-Leu-Gly-Gly-Phe-Gly- (SEQ ID NO: 15);
   -Gly-Pro-Leu-Ser-Leu-Gly-Gly-Phe-Gly- (SEQ ID NO: 16);
   -Gly-Pro-Ala-Ser-Leu-Gly-Gly-Phe-Gly- (SEQ ID NO: 17);
   -Gly-Pro-Ala-Gly-Leu-Gly-Gly-Phe-Gly- (SEQ ID NO: 18);
   -Gly-Pro-Gln-Gly-Leu-Gly-Gly-Phe-Gly- (SEQ ID NO: 19);
   -Gly-Pro-Leu-Gly-Leu-Gly-Gly-Phe-Gly- (SEQ ID NO: 20);
   -Gly-Pro-Ser-Gly-Leu-Gly-Gly-Phe-Gly- (SEQ ID NO: 21);
   -Gly-Pro-Arg-Gly-Leu-Gly-Gly-Phe-Gly- (SEQ ID NO: 22);
   -Gly-Pro-Ala-Ala-Leu-Gly-Gly-Phe-Gly- (SEQ ID NO: 23);
   -Gly-Pro-Ser-Ala-Leu-Gly-Gly-Phe-Gly- (SEQ ID NO: 24);
   -Pro-Arg-Gly-Leu-Val-Cit- (SEQ ID NO: 25);
   -Pro-Arg-Ser-Leu-Val-Cit- (SEQ ID NO: 26);
   -Pro-Arg-Asn-Leu-Val-Cit- (SEQ ID NO: 27);
   -Pro-Arg-Pro-Leu-Val-Cit- (SEQ ID NO: 28);
   -Pro-Cit-Gly-Leu-Val-Cit- (SEQ ID NO: 29);
   -Pro-Cit-Pro-Leu-Val-Cit- (SEQ ID NO: 30);
   -Gly-Pro-Arg-Ser-Leu-Val-Cit- (SEQ ID NO: 31);
   -Gly-Pro-Arg-Pro-Leu-Val-Cit- (SEQ ID NO: 32);
   -Gly-Pro-Arg-Asn-Leu-Val-Cit- (SEQ ID NO: 33);
   -Gly-Pro-Cit-Gly-Leu-Val-Cit- (SEQ ID NO: 34);
   -Gly-Pro-Cit-Pro-Leu-Val-Cit- (SEQ ID NO: 35);
   -His-Pro-Arg-Gly-Leu-Val-Cit- (SEQ ID NO: 36);
   -His-Pro-Arg-Ser-Leu-Val-Cit- (SEQ ID NO: 37);
   -His-Pro-Arg-Pro-Leu-Val-Cit- (SEQ ID NO: 38);
   -His-Pro-Arg-Asn-Leu-Val-Cit- (SEQ ID NO: 39);
   -His-Pro-Cit-Gly-Leu-Val-Cit- (SEQ ID NO: 40);
   -His-Pro-Cit-Pro-Leu-Val-Cit- (SEQ ID NO: 41);
   -Ser-Pro-Arg-Gly-Leu-Val-Cit- (SEQ ID NO: 42);
   -Ser-Pro-Arg-Ser-Leu-Val-Cit- (SEQ ID NO: 43);
   -Ser-Pro-Arg-Pro-Leu-Val-Cit- (SEQ ID NO: 44);
   -Ser-Pro-Arg-Asn-Leu-Val-Cit- (SEQ ID NO: 45);
   -Ser-Pro-Cit-Gly-Leu-Val-Cit- (SEQ ID NO: 46);
   -Ser-Pro-Cit-Ser-Leu-Val-Cit- (SEQ ID NO: 47);
   -Ser-Pro-Cit-Pro-Leu-Val-Cit- (SEQ ID NO: 48);
   -Gly-Pro-Ala-Pro-Leu-Val-Cit- (SEQ ID NO: 49);
   -Gly-Pro-Ala-Asn-Leu-Val-Cit- (SEQ ID NO: 50);
   -Gly-Pro-Arg-Gly-Dap(Me2)-Val-Cit- (SEQ ID NO: 51);
   -Gly-Pro-Arg-Asn-Dap(Me2)-Val-Cit- (SEQ ID NO: 60);
   -Pro-Leu-Ser-Leu-Val-Cit- (SEQ ID NO: 61);
   -Gly-Pro-Arg-Asn-Leu-Gly-Gly-Phe-Gly- (SEQ ID NO: 62);
   -Pro-Cit-Ser-Leu-Val-Cit- (SEQ ID NO: 63);
   -Gly-Pro-Cit-Asn-Leu-Val-Cit- (SEQ ID NO: 64);
   -Ser-Pro-Cit-Ser-Dap(Me2)-Val-Cit- (SEQ ID NO: 65);
   -Pro-Arg-Ser-Dap(Me2)-Val-Cit- (SEQ ID NO: 66);
   -Pro-Cit-Asn-Leu-Val-Cit- (SEQ ID NO: 67); or
   -Pro-Ala-Ser-Leu-Val-Cit- (SEQ ID NO: 68);
   or a variant having one or two mismatches relative to any sequence of SEQ ID NOs:1-51 and 60-68;
   D is camptothecin or a derivative thereof, auristatin or a derivative thereof, a tetrahydroisoquinoline alkaloid or a derivative thereof, a protein degrading agent and a derivative thereof, and preferably DX8951, MMAE, Dxd, SN38, ET743, Lurbinectedin, CC885, or CC-90009;
   n represents an integer from 1 to 10.

In some embodiments, the antibody-drug conjugate is selected from: 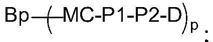 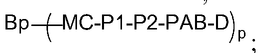 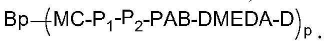 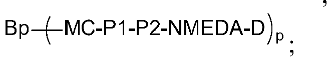 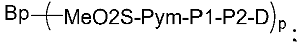 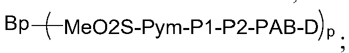 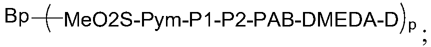 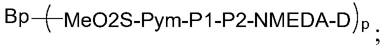 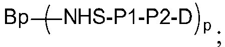 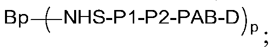 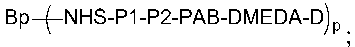 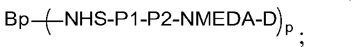 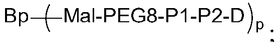 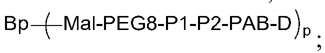 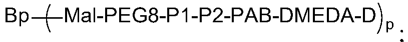 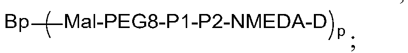 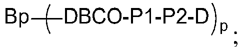 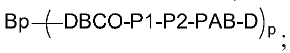 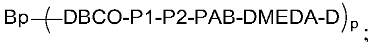 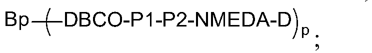 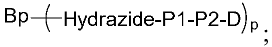 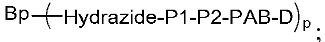 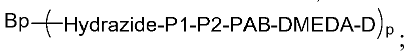 and

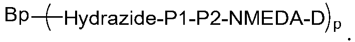

In some embodiments, the antibody-drug conjugate is selected from the following structures:
Ab-((succinimid-3-yl-N)-C5 alkylene-C(=O)-P1-P2-D)ₙ;
Ab-((succinimid-3-yl-N)-C5 alkylene-C(=O)-P1-P2-PAB-D)ₙ;
Ab-((succinimid-3-yl-N)-C5 alkylene-C(=O)-P1-P2-PAB-DMEDA-D)ₙ;
Ab-((succinimid-3-yl-N)-C5 alkylene-C(=O)-P1-P2-NMEDA-D)ₙ; 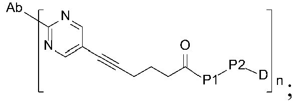 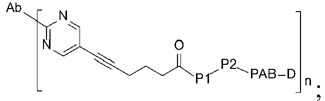 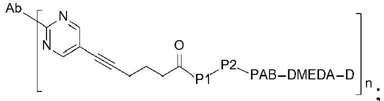 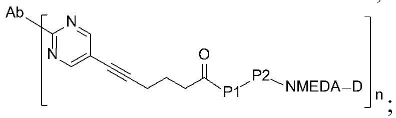
Ab-((C=O)-C6 alkylene-C(=O)-D)ₙ;
Ab-((C=O)-C6 alkylene-C(=O)-P1-P2-PAB-D)ₙ;
Ab-((C=O)-C6 alkylene-C(=O)-P1-P2-PAB-DMEDA-D)ₙ;
Ab-((C=O)-C6 alkylene-C(=O)-P1-P2-NMEDA-D)ₙ;
Ab-((succinimid-3-yl-N)-C2 alkylene-C(=O)NH-(CH₂CH₂O)₈-C2 alkylene-C(=O)-P1-P2-D)ₙ;
Ab-((succinimid-3-yl-N)-C2 alkylene-C(=O)NH-(CH₂CH₂O)₈-C2 alkylene-C(=O)-P1-P2-PAB-D)ₙ;
Ab-((succinimid-3-yl-N)-C2 alkylene-C(=O)NH-(CH₂CH₂O)₈-C2 alkylene-C(=O)-P1-P2-PAB-DMEDA-D)ₙ;
Ab-((succinimid-3-yl-N)-C2 alkylene-C(=O)NH-(CH₂CH₂O)₈-C2 alkylene-C(=O)-P1-P2-NMEDA-D)ₙ; 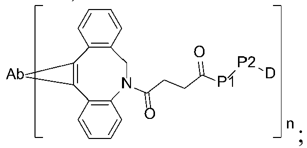 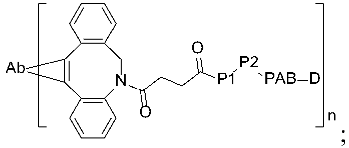 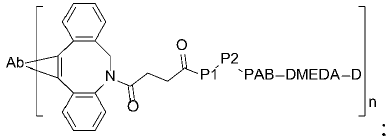 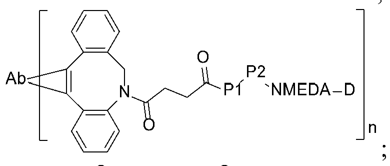 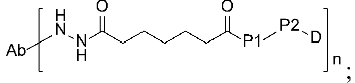 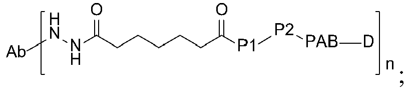 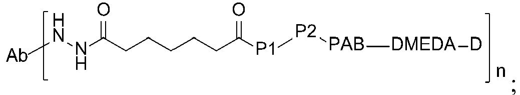 and 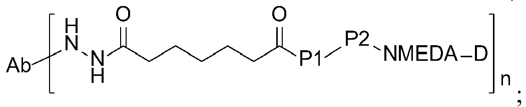
Ab represents a monoclonal antibody or an antigen-binding fragment thereof, or a modified monoclonal antibody or an antigen-binding fragment thereof, and preferably an anti-Her2, anti-TF, anti-ROR1, or anti-GPC3 antibody or an antigen-binding fragment thereof. D is camptothecin or a derivative thereof, auristatin or a derivative thereof, tetrahydroisoquinoline alkaloid or a derivative thereof, a protein degrading agent and a derivative thereof, and preferably DX8951, MMAE, Dxd, SN38, ET743, Lurbinectedin, CC885, or CC-90009; n represents any value selected from 1 to 10, and preferably 2 to 9 or 3 to 8 (for example, 2, 3, 4, 5, 6, 7, 8, or 9).

In some embodiments, the antibody-drug conjugate is selected from the following structures:
Ab-((succinimid-3-yl-N)-C5 alkylene-C(=O)-GPAGLVCit-PAB-D)ₙ;
Ab-((succinimid-3-yl-N)-C5 alkylene-C(=O)-GPLGLGGFG-D)ₙ;
Ab-((succinimid-3-yl-N)-C5 alkylene-C(=O)-GPASLVCit-PAB-D)ₙ;
Ab-((succinimid-3-yl-N)-C5 alkylene-C(=O)-GPRGLVCit-PAB-D)ₙ;
Ab-((succinimid-3-yl-N)-C5 alkylene-C(=O)-GPSALVCit-PAB-D)ₙ;
Ab-((succinimid-3-yl-N)-C5 alkylene-C(=O)-GPRGLGGFG-D)ₙ;
Ab-((succinimid-3-yl-N)-C5 alkylene-C(=O)-GPAALGGFG-D)ₙ;
Ab-((succinimid-3-yl-N)-C5 alkylene-C(=O)-PRNLVCit-PAB-D)ₙ;
Ab-((succinimid-3-yl-N)-C5 alkylene-C(=O)-GPRNLVCit-PAB-D)ₙ;
Ab-((succinimid-3-yl-N)-C5 alkylene-C(=O)-GPRNDap(Me2)VCit-PAB-D)ₙ;
Ab-((succinimid-3-yl-N)-C5 alkylene-C(=O)-SPRNLVCit-PAB-D)ₙ;
Ab-((succinimid-3-yl-N)-C5 alkylene-C(=O)-GPLSLVCit-PAB-D)ₙ;
Ab-((succinimid-3-yl-N)-C5 alkylene-C(=O)-PRGLVCit-PAB-D)ₙ;
Ab-((succinimid-3-yl-N)-C5 alkylene-C(=O)-PRPLVCit-PAB-D)ₙ;
Ab-((succinimid-3-yl-N)-C5 alkylene-C(=O)-GPRSLVCit-PAB-D)ₙ;
Ab-((succinimid-3-yl-N)-C5 alkylene-C(=O)-GPRPLVCit-PAB-D)ₙ;
Ab-((succinimid-3-yl-N)-C5 alkylene-C(=O)-GPCitPLVCit-PAB-D)ₙ;
Ab-((succinimid-3-yl-N)-C5 alkylene-C(=O)-HPRGLVCit-PAB-D)ₙ;
Ab-((succinimid-3-yl-N)-C5 alkylene-C(=O)-HPCitPLVCit-PAB-D)ₙ;
Ab-((succinimid-3-yl-N)-C5 alkylene-C(=O)-SPCitGLVCit-PAB-D)ₙ;
Ab-((succinimid-3-yl-N)-C5 alkylene-C(=O)-GPAPLVCit-PAB-D)ₙ;
Ab-((succinimid-3-yl-N)-C5 alkylene-C(=O)-PLSLVCit-PAB-D)ₙ;
Ab-((succinimid-3-yl-N)-C5 alkylene-C(=O)-SPCitSLVCit-PAB-D)ₙ; 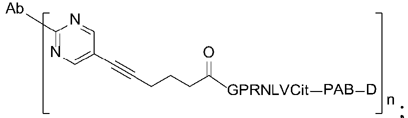 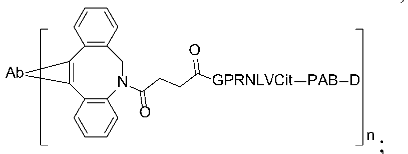
Ab-((C=O)-C6 alkylene-C(=O)-GPRNLVCit-PAB-D)ₙ; 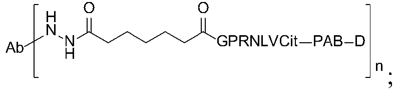
Ab-((succinimid-3-yl-N)-C2 alkylene-C(=O)NH-(CH₂CH₂O)₈-C2 alkylene-C(=O)-GPRNLVCit-PAB-D)ₙ;
Ab-((succinimid-3-yl-N)-C5 alkylene-C(=O)-GPRNLVCit-PAB-DMEDA-D)ₙ;
Ab-((succinimid-3-yl-N)-C5 alkylene-C(=O)-GPRNLGGFG-D)ₙ;
Ab-((succinimid-3-yl-N)-C5 alkylene-C(=O)-GPRNLGGFG-NMEDA-D)ₙ;
Ab-((succinimid-3-yl-N)-C5 alkylene-C(=O)-PRSLVCit-PAB-D)ₙ;
Ab-((succinimid-3-yl-N)-C5 alkylene-C(=O)-SPRSLVCit-PAB-D)ₙ;
Ab-((succinimid-3-yl-N)-C5 alkylene-C(=O)-PCitSLVCit-PAB-D)ₙ;
Ab-((succinimid-3-yl-N)-C5 alkylene-C(=O)-PCitPLVCit-PAB-D)ₙ;
Ab-((succinimid-3-yl-N)-C5 alkylene-C(=O)-PCitGLVCit-PAB-D)ₙ;
Ab-((succinimid-3-yl-N)-C5 alkylene-C(=O)-GPCitGLVCit-PAB-D)ₙ;
Ab-((succinimid-3-yl-N)-C5 alkylene-C(=O)-SPRGLVCit-PAB-D)ₙ;
Ab-((succinimid-3-yl-N)-C5 alkylene-C(=O)-SPRPLVCit-PAB-D)ₙ;
Ab-((succinimid-3-yl-N)-C5 alkylene-C(=O)-SPCitPLVCit-PAB-D)ₙ;
Ab-((succinimid-3-yl-N)-C5 alkylene-C(=O)-GPANLVCit-PAB-D)ₙ;
Ab-((succinimid-3-yl-N)-C5 alkylene-C(=O)-GPCitNLVCit-PAB-D)ₙ;
Ab-((succinimid-3-yl-N)-C5 alkylene-C(=O)-SPCitSDap(Me2)VCit-PAB-D)ₙ;
Ab-((succinimid-3-yl-N)-C5 alkylene-C(=O)-PRSDap(Me2)VCit-PAB-D)ₙ;
Ab-((succinimid-3-yl-N)-C5 alkylene-C(=O)-PCitNLVCit-PAB-D)ₙ; and
Ab-((succinimid-3-yl-N)-C5-alkylene-C(=O)-PASLVCit-PAB-D)ₙ;
Ab is an anti-Her2, anti-TF, anti-ROR1, or anti-GPC3 antibody or an antigen-binding fragment thereof; D is DX8951, MMAE, Dxd, SN38, or ET743; n represents any value selected from 2 to 9, and preferably 2 to 8 (for example, 2, 3, 4, 5, 6, 7, 8, or 9).

In some embodiments, the antibody-drug conjugate is selected from the following structures:
Ab-((succinimid-3-yl-N)-C5-alkylene-C(=O)-GPAGLVCit-PAB-DX8951)ₙ;
Ab-((succinimid-3-yl-N)-C5-alkylene-C(=O)-GPAGLVCit-PAB-MMAE)ₙ;
Ab-((succinimid-3-yl-N)-C5-alkylene-C(=O)-GPAGLVCit-PAB-ET743)ₙ;
Ab-((succinimid-3-yl-N)-C5-alkylene-C(=O)-GPLGLGGFG-DX8951)ₙ;
Ab-((succinimid-3-yl-N)-C5-alkylene-C(=O)-GPASLVCit-PAB-DX8951)ₙ;
Ab-((succinimid-3-yl-N)-C5-alkylene-C(=O)-GPRGLVCit-PAB-DX8951)ₙ;
Ab-((succinimid-3-yl-N)-C5-alkylene-C(=O)-GPRGLVCit-PAB-MMAE)ₙ;
Ab-((succinimid-3-yl-N)-C5-alkylene-C(=O)-GPSALVCit-PAB-DX8951)ₙ;
Ab-((succinimid-3-yl-N)-C5-alkylene-C(=O)-GPRGLGGFG-DX8951)ₙ;
Ab-((succinimid-3-yl-N)-C5 alkylene-C(=O)-GPAALGGFG-DX8951)ₙ;
Ab-((succinimid-3-yl-N)-C5-alkylene-C(=O)-PRNLVCit-PAB-DX8951)ₙ;
Ab-((succinimid-3-yl-N)-C5-alkylene-C(=O)-PRNLVCit-PAB-MMAE)ₙ;
Ab-((succinimid-3-yl-N)-C5-alkylene-C(=O)-GPRNLVCit-PAB-DX8951)ₙ;
Ab-((succinimid-3-yl-N)-C5-alkylene-C(=O)-GPRNLVCit-PAB-MMAE)ₙ;
Ab-((succinimid-3-yl-N)-C5-alkylene-C(=O)-GPRNLVCit-PAB-ET743)ₙ;
Ab-((succinimid-3-yl-N)-C5-alkylene-C(=O)-GPRNDap(Me2)VCit-PAB-DX8951)ₙ;
Ab-((succinimid-3-yl-N)-C5-alkylene-C(=O)-GPRNDap(Me2)VCit-PAB-MMAE)ₙ;
Ab-((succinimid-3-yl-N)-C5-alkylene-C(=O)-SPRNLVCit-PAB-DX8951)ₙ;
Ab-((succinimid-3-yl-N)-C5-alkylene-C(=O)-SPRNLVCit-PAB-MMAE)ₙ;
Ab-((succinimid-3-yl-N)-C5-alkylene-C(=O)-GPLSLVCit-PAB-DX8951)ₙ;
Ab-((succinimid-3-yl-N)-C5-alkylene-C(=O)-GPLSLVCit-PAB-MMAE)ₙ;
Ab-((succinimid-3-yl-N)-C5-alkylene-C(=O)-PRGLVCit-PAB-DX8951)ₙ;
Ab-((succinimid-3-yl-N)-C5-alkylene-C(=O)-PRPLVCit-PAB-DX8951)ₙ;
Ab-((succinimid-3-yl-N)-C5-alkylene-C(=O)-GPRSLVCit-PAB-DX8951)ₙ;
Ab-((succinimid-3-yl-N)-C5-alkylene-C(=O)-GPRPLVCit-PAB-DX8951)ₙ;
Ab-((succinimid-3-yl-N)-C5-alkylene-C(=O)-GPCitPLVCit-PAB-DX8951)ₙ;
Ab-((succinimid-3-yl-N)-C5-alkylene-C(=O)-HPRGLVCit-PAB-DX8951)ₙ;
Ab-((succinimid-3-yl-N)-C5-alkylene-C(=O)-HPCitPLVCit-PAB-DX8951)ₙ;
Ab-((succinimid-3-yl-N)-C5-alkylene-C(=O)-SPCitGLVCit-PAB-DX8951)ₙ;
Ab-((succinimid-3-yl-N)-C5-alkylene-C(=O)-GPAPLVCit-PAB-DX8951)ₙ;
Ab-((succinimid-3-yl-N)-C5-alkylene-C(=O)-PLSLVCit-PAB-DX8951)ₙ;
Ab-((succinimid-3-yl-N)-C5-alkylene-C(=O)-SPCitSLVCit-PAB-DX8951)ₙ;
Ab-((succinimid-3-yl-N)-C5-alkylene-C(=O)-SPCitSLVCit-PAB-MMAE)ₙ;
Ab-((succinimid-3-yl-N)-C5-alkylene-C(=O)-SPCitSLVCit-PAB-ET743)ₙ; 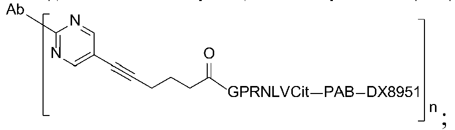 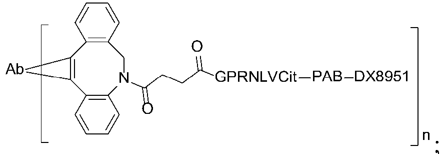
Ab-((C=O)-C6-alkylene-C(=O)-GPRNLVCit-PAB-DX8951)ₙ; 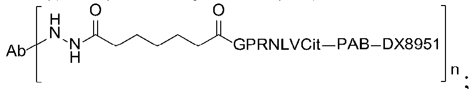
Ab-((succinimid-3-yl-N)-C2-alkylene-C(=O)NH-(CH₂CH₂O)₈-C2-alkylene-C(=O)-GPRNLVCit-PAB-DX8951)ₙ;
Ab-((succinimid-3-yl-N)-C5-alkylene-C(=O)-GPRNLVCit-PAB-DMEDA-SN38)ₙ;
Ab-((succinimid-3-yl-N)-C5-alkylene-C(=O)-GPRNLGGFG-Dxd)ₙ;
Ab-((succinimid-3-yl-N)-C5-alkylene-C(=O)-GPRNLGGFG-NMEDA-Dxd)ₙ;
Ab-((succinimid-3-yl-N)-C5-alkylene-C(=O)-PRSLVCit-PAB-DX8951)ₙ;
Ab-((succinimid-3-yl-N)-C5-alkylene-C(=O)-SPRSLVCit-PAB-DX8951)ₙ;
Ab-((succinimid-3-yl-N)-C5-alkylene-C(=O)-PCitSLVCit-PAB-DX8951)ₙ;
Ab-((succinimid-3-yl-N)-C5-alkylene-C(=O)-PCitPLVCit-PAB-DX8951)ₙ;
Ab-((succinimid-3-yl-N)-C5-alkylene-C(=O)-PCitGLVCit-PAB-DX8951)ₙ;
Ab-((succinimid-3-yl-N)-C5-alkylene-C(=O)-GPCitGLVCit-PAB-DX8951)ₙ;
Ab-((succinimid-3-yl-N)-C5-alkylene-C(=O)-SPRGLVCit-PAB-DX8951)ₙ;
Ab-((succinimid-3-yl-N)-C5-alkylene-C(=O)-SPRPLVCit-PAB-DX8951)ₙ;
Ab-((succinimid-3-yl-N)-C5-alkylene-C(=O)-SPCitPLVCit-PAB-DX8951)ₙ;
Ab-((succinimid-3-yl-N)-C5-alkylene-C(=O)-GPANLVCit-PAB-DX8951)ₙ;
Ab-((succinimid-3-yl-N)-C5-alkylene-C(=O)-GPCitNLVCit-PAB-DX8951)ₙ;
Ab-((succinimid-3-yl-N)-C5-alkylene-C(=O)-SPCitSDap(Me2)VCit-PAB-DX8951)ₙ;
Ab-((succinimid-3-yl-N)-C5-alkylene-C(=O)-SPCitSDap(Me2)VCit-PAB-MMAE)ₙ;
Ab-((succinimid-3-yl-N)-C5-alkylene-C(=O)-PRSDap(Me2)VCit-PAB-DX8951)ₙ;
Ab-((succinimid-3-yl-N)-C5-alkylene-C(=O)-PRSDap(Me2)VCit-PAB-MMAE)ₙ;
Ab-((succinimid-3-yl-N)-C5-alkylene-C(=O)-PCitNLVCit-PAB-DX8951)ₙ; and
Ab-((succinimid-3-yl-N)-C5-alkylene-C(=O)-PASLVCit-PAB-DX8951)ₙ;
Ab is an anti-Her2, anti-TF, anti-ROR1, or anti-GPC3 antibody or an antigen-binding fragment thereof; n represents any value selected from 2 to 9, and preferably from 2 to 8 (for example, 2, 3, 4, 5, 6, 7, 8, or 9).

In some embodiments, the antibody-drug conjugate is selected from the following structures:
anti-TF antibody-((succinimid-3-yl-N)-C5-alkylene-C(=O)-GPAGLVCit-PAB-DX8951)ₙ;
anti-GPC3 antibody-((succinimid-3-yl-N)-C5-alkylene-C(=O)-GPAGLVCit-PAB-DX8951)ₙ;
anti-Her2 antibody-((succinimid-3-yl-N)-C5-alkylene-C(=O)-GPAGLVCit-PAB-DX8951)ₙ;
anti-TF antibody-((succinimid-3-yl-N)-C5-alkylene-C(=O)-GPAGLVCit-PAB-MMAE)ₙ;
anti-GPC3 antibody-((succinimid-3-yl-N)-C5-alkylene-C(=O)-GPAGLVCit-PAB-MMAE)ₙ;
anti-GPC3 antibody-((succinimid-3-yl-N)-C5-alkylene-C(=O)-GPAGLVCit-PAB-ET743)ₙ;
anti-TF antibody-((succinimid-3-yl-N)-C5-alkylene-C(=O)-GPLGLGGFG-DX8951)ₙ;
anti-Her2 antibody-((succinimid-3-yl-N)-C5-alkylene-C(=O)-GPLGLGGFG-DX8951)ₙ;
anti-Her2 antibody-((succinimid-3-yl-N)-C5-alkylene-C(=O)-GPASLVCit-PAB-DX8951)ₙ;
anti-TF antibody-((succinimid-3-yl-N)-C5-alkylene-C(=O)-GPRGLVCit-PAB-DX8951)ₙ;
anti-GPC3 antibody-((succinimid-3-yl-N)-C5-alkylene-C(=O)-GPRGLVCit-PAB-DX8951)ₙ;
anti-Her2 antibody-((succinimid-3-yl-N)-C5-alkylene-C(=O)-GPRGLVCit-PAB-DX8951)ₙ;
anti-ROR1 antibody-((succinimid-3-yl-N)-C5-alkylene-C(=O)-GPRGLVCit-PAB-DX8951)ₙ;
anti-TF antibody-((succinimid-3-yl-N)-C5-alkylene-C(=O)-GPRGLVCit-PAB-MMAE)ₙ;
anti-GPC3 antibody-((succinimid-3-yl-N)-C5-alkylene-C(=O)-GPRGLVCit-PAB-MMAE)ₙ;
anti-Her2 antibody-((succinimid-3-yl-N)-C5-alkylene-C(=O)-GPSALVCit-PAB-DX8951)ₙ;
anti-Her2 antibody-((succinimid-3-yl-N)-C5 alkylene-C(=O)-GPRGLGGFG-DX8951)ₙ;
anti-Her2 antibody-((succinimid-3-yl-N)-C5 alkylene-C(=O)-GPAALGGFG-DX8951)ₙ;
anti-TF antibody-((succinimid-3-yl-N)-C5 alkylene-C(=O)-PRNLVCit-PAB-DX8951)ₙ;
anti-GPC3 antibody-((succinimid-3-yl-N)-C5 alkylene-C(=O)-PRNLVCit-PAB-DX8951)ₙ;
anti-Her2 antibody-((succinimid-3-yl-N)-C5 alkylene-C(=O)-PRNLVCit-PAB-DX8951)ₙ;
anti-TF antibody-((succinimid-3-yl-N)-C5 alkylene-C(=O)-PRNLVCit-PAB-MMAE)ₙ;
anti-GPC3 antibody-((succinimid-3-yl-N)-C5 alkylene-C(=O)-PRNLVCit-PAB-MMAE)ₙ;
anti-TF antibody-((succinimid-3-yl-N)-C5 alkylene-C(=O)-GPRNLVCit-PAB-DX8951)ₙ;
anti-GPC3 antibody-((succinimid-3-yl-N)-C5 alkylene-C(=O)-GPRNLVCit-PAB-DX8951)ₙ;
anti-Her2 antibody-((succinimid-3-yl-N)-C5 alkylene-C(=O)-GPRNLVCit-PAB-DX8951)ₙ;
anti-ROR1 antibody-((succinimid-3-yl-N)-C5 alkylene-C(=O)-GPRNLVCit-PAB-DX8951)ₙ;
anti-TF antibody-((succinimid-3-yl-N)-C5 alkylene-C(=O)-GPRNLVCit-PAB-MMAE)ₙ;
anti-GPC3 antibody-((succinimid-3-yl-N)-C5 alkylene-C(=O)-GPRNLVCit-PAB-MMAE)ₙ;
anti-TF antibody-((succinimid-3-yl-N)-C5 alkylene-C(=O)-GPRNDap(Me2)VCit-PAB-DX8951)ₙ;
anti-GPC3 antibody-((succinimid-3-yl-N)-C5 alkylene-C(=O)-GPRNDap(Me2)VCit-PAB-DX8951)ₙ;
anti-Her2 antibody-((succinimid-3-yl-N)-C5 alkylene-C(=O)-GPRNDap(Me2)VCit-PAB-DX8951)ₙ;
anti-TF antibody-((succinimid-3-yl-N)-C5 alkylene-C(=O)-GPRNDap(Me2)VCit-PAB-MMAE)ₙ;
anti-GPC3 antibody-((succinimid-3-yl-N)-C5 alkylene-C(=O)-GPRNDap(Me2)VCit-PAB-MMAE)ₙ;
anti-TF antibody-((succinimid-3-yl-N)-C5 alkylene-C(=O)-SPRNLVCit-PAB-DX8951)ₙ;
anti-GPC3 antibody-((succinimid-3-yl-N)-C5 alkylene-C(=O)-SPRNLVCit-PAB-DX8951)ₙ;
anti-Her2 antibody-((succinimid-3-yl-N)-C5-alkylene-C(=O)-SPRNLVCit-PAB-DX8951)ₙ;
anti-TF antibody-((succinimid-3-yl-N)-C5 alkylene-C(=O)-SPRNLVCit-PAB-MMAE)ₙ;
anti-GPC3 antibody-((succinimid-3-yl-N)-C5 alkylene-C(=O)-GPLSLVCit-PAB-DX8951)ₙ;
anti-GPC3 antibody-((succinimid-3-yl-N)-C5 alkylene-C(=O)-GPLSLVCit-PAB-MMAE)ₙ;
anti-TF antibody-((succinimid-3-yl-N)-C5 alkylene-C(=O)-PRGLVCit-PAB-DX8951)ₙ;
anti-TF antibody-((succinimid-3-yl-N)-C5 alkylene-C(=O)-PRPLVCit-PAB-DX8951)ₙ;
anti-TF antibody-((succinimid-3-yl-N)-C5 alkylene-C(=O)-GPRSLVCit-PAB-DX8951)ₙ;
anti-TF antibody-((succinimid-3-yl-N)-C5 alkylene-C(=O)-GPRPLVCit-PAB-DX8951)ₙ;
anti-TF antibody-((succinimid-3-yl-N)-C5 alkylene-C(=O)-GPCitPLVCit-PAB-DX8951)ₙ;
anti-TF antibody-((succinimid-3-yl-N)-C5 alkylene-C(=O)-HPRGLVCit-PAB-DX8951)ₙ;
anti-TF antibody -((succinimid-3-yl-N)-C5 alkylene-C(=O)-PLSLVCit-PAB-DX8951)ₙ;
anti-TF antibody-((succinimid-3-yl-N)-C5 alkylene-C(=O)-SPCitSLVCit-PAB-DX8951)ₙ;
anti-TF antibody-((succinimid-3-yl-N)-C5 alkylene-C(=O)-SPCitSLVCit-PAB-MMAE)ₙ; 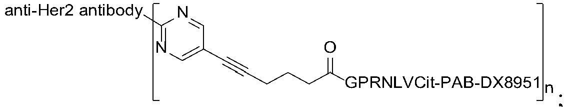
anti-Her2 antibody-((C=O)-C6 alkylene-C(=O)-GPRNLVCit-PAB-DX8951)ₙ;
anti-Her2 antibody-((succinimid-3-yl-N)-C2 alkylene-C(=O)NH-(CH₂CH₂O)₈-C2 alkylene-C(=O)-GPRNLVCit-PAB-DX8951)ₙ;
anti-Her2 antibody-((succinimid-3-yl-N)-C5 alkylene-C(=O)-GPRNLVCit-PAB-DMEDA-SN38)ₙ;
anti-Her2 antibody-((succinimid-3-yl-N)-C5 alkylene-C(=O)-GPRNLGGFG-Dxd)ₙ;
anti-Her2 antibody-((succinimid-3-yl-N)-C5 alkylene-C(=O)-GPRNLGGFG-NMEDA-Dxd)ₙ;
anti-TF antibody-((succinimid-3-yl-N)-C5 alkylene-C(=O)-PRSLVCit-PAB-DX8951)ₙ;
anti-TF antibody-((succinimid-3-yl-N)-C5 alkylene-C(=O)-SPRSLVCit-PAB-DX8951)ₙ;
anti-TF antibody-((succinimid-3-yl-N)-C5 alkylene-C(=O)-PCitSLVCit-PAB-DX8951)ₙ;
anti-TF antibody-((succinimid-3-yl-N)-C5 alkylene-C(=O)-PCitPLVCit-PAB-DX8951)ₙ;
anti-TF antibody-((succinimid-3-yl-N)-C5 alkylene-C(=O)-PCitGLVCit-PAB-DX8951)ₙ;
anti-TF antibody-((succinimid-3-yl-N)-C5 alkylene-C(=O)-GPCitGLVCit-PAB-DX8951)ₙ;
anti-TF antibody-((succinimid-3-yl-N)-C5 alkylene-C(=O)-SPRGLVCit-PAB-DX8951)ₙ;
anti-TF antibody-((succinimid-3-yl-N)-C5 alkylene-C(=O)-SPRPLVCit-PAB-DX8951)ₙ;
anti-TF antibody-((succinimid-3-yl-N)-C5 alkylene-C(=O)-SPCitPLVCit-PAB-DX8951)ₙ;
anti-TF antibody-((succinimid-3-yl-N)-C5 alkylene-C(=O)-GPANLVCit-PAB-DX8951)ₙ;
anti-TF antibody-((succinimid-3-yl-N)-C5 alkylene-C(=O)-GPCitNLVCit-PAB-DX8951)ₙ;
anti-TF antibody-((succinimid-3-yl-N)-C5 alkylene-C(=O)-SPCitSDap(Me2)VCit-PAB-DX8951)ₙ;
anti-TF antibody-((succinimid-3-yl-N)-C5 alkylene-C(=O)-SPCitSDap(Me2)VCit-PAB-MMAE)ₙ;
anti-TF antibody-((succinimid-3-yl-N)-C5 alkylene-C(=O)-PRSDap(Me2)VCit-PAB-DX8951)ₙ;
anti-TF antibody-((succinimid-3-yl-N)-C5 alkylene-C(=O)-PRSDap(Me2)VCit-PAB-MMAE)ₙ;
anti-TF antibody-((succinimid-3-yl-N)-C5 alkylene-C(=O)-PCitNLVCit-PAB-DX8951)ₙ; and
anti-TF antibody-((succinimid-3-yl-N)-C5 alkylene-C(=O)-PASLVCit-PAB-DX8951)ₙ;
n represents any value selected from 2 to 9, preferably from 2 to 8 (for example, 2, 3, 4, 5, 6, 7, 8, or 9); the anti-Her2, anti-TF, anti-ROR1, or anti-GPC3 antibody is as described herein.

In some embodiments, the anti-Her2, anti-TF, anti-ROR1, and anti-GPC3 antibodies are selected from Trastuzumab, Tisotumab, Zilovertamab, and Codrituzumab.

In some embodiments, the antibody-drug conjugate is selected from the following structures:
Ab-((succinimid-3-yl-N)-CH2CH2CH2CH2CH2-C(= O)-GPAGLUCit-PAB-DX895 1)n;
Ab-((succinimid-3-yl-N)-CH2CH2CH2CH2CH2-C(=O)-GPLGLGGFG-DX8951)n;
Ab-((succinimid-3-yl-N)-CH2CH2CH2CH2CH2-C(=O)-GPASLVCit-PAB-DX8951)n;
Ab-((succinimid-3-yl-N)-CH2CH2CH2CH2CH2-C(=O)-GPRGLUCit-PAB-DX8951)n;
Ab-((succinimid-3-yl-N)-CH2CH2CH2CH2CH2-C(=O)-GPSALVCit-PAB-DX8951)n;
Ab-((succinimid-3-yl-N)-CH2CH2CH2CH2CH2-C(=O)-GPRGLGGFG-DX8951)n;
Ab-((succinimid-3-yl-N)-CH2CH2CH2CH2CH2-C(=O)-GPAALGGFG-DX8951)n;
Ab-((succinimid-3-yl-N)-CH2CH2CH2CH2CH2-C(=O)-PRNLVCit-PAB-DX8951)n;
Ab-((succinimid-3-yl-N)-CH2CH2CH2CH2CH2-C(=O)-GPRNLVCit-PAB-DX8951)n;
Ab-((succinimid-3-yl-N)-CH2CH2CH2CH2CH2-C(=O)-GPRGDap(Me2)LVCit-PAB-DX8951)n;
Ab-((succinimid-3-yl-N)-CH2CH2CH2CH2CH2-C(=O)-SPRNLVCit-PAB-DX8951)n;
Ab-((succinimid-3-yl-N)-CH2CH2CH2CH2CH2-C(=O)-GPLSLVCit-PAB-DX8951)n;
Ab-((succinimid-3-yl-N)-CH2CH2CH2CH2CH2-C(=O)-GPAGLVCit-PAB-MMAE)n;
Ab-((succinimid-3-yl-N)-CH2CH2CH2CH2CH2-C(=O)-GPRGLVCit-PAB-MMAE)n;
Ab-((succinimid-3-yl-N)-CH2CH2CH2CH2CH2-C(=O)-PRNLVCit-PAB-MMAE)n;
Ab-((succinimid-3-yl-N)-CH2CH2CH2CH2CH2-C(=O)-GPRNLVCit-PAB-MMAE)n;
Ab-((succinimid-3-yl-N)-CH2CH2CH2CH2CH2-C(=O)-GPRGDap(Me2)LVCit-PAB-MMAE)n;
Ab-((succinimid-3-yl-N)-CH2CH2CH2CH2CH2-C(=O)-SPRNLVCit-PAB-MMAE)n;
Ab-((succinimid-3-yl-N)-CH2CH2CH2CH2CH2-C(=O)-GPLSLVCit-PAB-MMAE)n;
Ab-((succinimid-3-yl-N)-CH2CH2CH2CH2CH2-C(=O)-GPAGLVCit-PAB-ET743)n; and
Ab-((succinimid-3-yl-N)-CH2CH2CH2CH2CH2-C(=O)-GPRNLVCit-PAB-ET743)n;
Ab represents a monoclonal antibody or an antigen-binding fragment thereof, or a modified monoclonal antibody or an antigen-binding fragment thereof, and preferably an anti-Her2, anti-TF, anti-ROR1, anti-GPC3 antibody or an antigen-binding fragment thereof;
DX8951 has a structure of 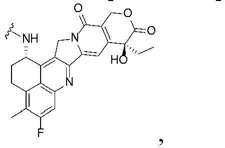
wherein 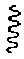 indicates a position of DX8951 connecting to the linker;
MMAE has a structure of 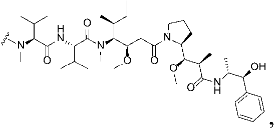
wherein 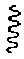 indicates a position of MMAE connecting to the linker;
ET-743 has a structure of 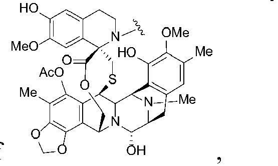
wherein 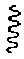 indicates a position of ET743 connecting to the linker; and n is an integer from 1 to 10. Preferably, n is 2, 4, 6, or 8.

**In** another aspect, the present application provides a monoclonal antibody.

An anti-Tissue Factor (TF) antibody or an antigen-binding fragment thereof, comprises:
(a) a VH comprising an amino acid sequence set forth in SEQ ID NO: 52 or 54, or an amino acid sequence having at least 80% identity to the amino acid sequence set forth in SEQ ID NO: 52 or 54, or an amino acid sequence obtained by substituting, deleting, or adding one or more amino acids in the amino acid sequence set forth in SEQ ID NO: 52 or 54 and having the same or similar function as the amino acid sequence set forth in SEQ ID NO: 52 or 54; and/or
(b) a VL comprising an amino acid sequence set forth in SEQ ID NO: 53 or 55, or an amino acid sequence having at least 80% identity to the amino acid sequence set forth in SEQ ID NO: 53 or 55, or an amino acid sequence obtained by substituting, deleting, or adding one or more amino acids in the amino acid sequence set forth in SEQ ID NO: 53 or 55 and having the same or similar function as the amino acid sequence set forth in SEQ ID NO: 53 or 55.

In some embodiments, the anti-TF antibody or antigen-binding fragment thereof comprises:
(1) a VH having an amino acid sequence set forth in SEQ ID NO: 52, and a VL having an amino acid sequence set forth in SEQ ID NO: 53; or
(2) a VH having an amino acid sequence set forth in SEQ ID NO: 54, and a VL having an amino acid sequence set forth in SEQ ID NO: 53; or
(3) a VH having an amino acid sequence set forth in SEQ ID NO: 54, and a VL having an amino acid sequence set forth in SEQ ID NO: 55; or
(4) a VH having an amino acid sequence set forth in SEQ ID NO: 52, and a VL having an amino acid sequence set forth in SEQ ID NO: 55.

In some embodiments, the anti-TF antibody or the antigen-binding fragment thereof comprises a light chain having an amino acid sequence set forth in SEQ ID NO: 56, or an amino acid sequence having at least 80% identity to the amino acid sequence set forth in SEQ ID NO: 56; a heavy chain having an amino acid sequence set forth in SEQ ID NO: 57, or an amino acid sequence having at least 80% identity to the amino acid sequence set forth in SEQ ID NO: 57.

In some embodiments, the anti-TF antibody or the antigen-binding fragment thereof comprises a light chain having an amino acid sequence set forth in SEQ ID NO: 56, or an amino acid sequence having at least 80% identity to the amino acid sequence set forth in SEQ ID NO: 56; a heavy chain having an amino acid sequence set forth in SEQ ID NO: 59, or an amino acid sequence having at least 80% identity to the amino acid sequence set forth in SEQ ID NO: 59.

In some embodiments, the anti-TF antibody or the antigen-binding fragment thereof comprises a light chain having an amino acid sequence set forth in SEQ ID NO: 58, or an amino acid sequence having at least 80% identity to the amino acid sequence set forth in SEQ ID NO: 58; a heavy chain having an amino acid sequence set forth in SEQ ID NO: 59, or an amino acid sequence having at least 80% identity to the amino acid sequence set forth in SEQ ID NO: 59.

In some embodiments, the TF is mammalian TF, and the antibody is preferably a murine antibody, simian antibody, rabbit antibody, chimeric antibody, humanized antibody, or human antibody; and/or
the antibody is an IgG antibody, preferably an IgG1, IgG2, IgG3, or IgG4 antibody, and more preferably an IgG1 antibody; and/or
the anti-TF antibody or the antigen-binding fragment thereof is selected from monoclonal antibodies, polyclonal antibodies, Fab fragments, Fab' fragments, F(ab')2 fragments, Fd fragments, Fv fragments, dAb fragments, isolated CDR regions, scFv, and nanobodies.

In some embodiments, the TF is mammalian TF, and the antibody is preferably a modified murine antibody, simian antibody, rabbit antibody, chimeric antibody, humanized antibody, or human antibody; and/or
the antibody is a modified IgG antibody, preferably a modified IgG1, IgG2, IgG3, or IgG4 antibody, and more preferably a modified IgG1 antibody; and/or
the anti-TF antibody or the antigen-binding fragment thereof is selected from modified monoclonal antibodies, polyclonal antibodies, Fab fragments, Fab' fragments, F(ab')2 fragments, Fd fragments, Fv fragments, dAb fragments, isolated CDR regions, scFv, and nanobodies.

In some embodiments, the modification method is amino acid point mutation, glycosylation modification, or polypeptide modification.

A nucleic acid molecule encodes the anti-TF antibody or the antigen-binding fragment thereof as described in some embodiments, or a heavy chain variable region thereof and/or a light chain variable region thereof.

A recombinant vector comprises the nucleic acid molecule as described in some embodiments.

A recombinant cell comprises the nucleic acid molecule as described in some embodiments or a recombinant vector comprising the nucleic acid molecule.

A method of preparing an anti-TF antibody or an antigen-binding fragment thereof, comprises culturing the recombinant cell as described in some embodiments.

Provided is use of the anti-TF antibody or the antigen-binding fragment thereof in some embodiments, the nucleic acid molecule in some embodiments, the recombinant vector in some embodiments, or the recombinant cell in some embodiments in the preparation of a medicament. Preferably, the medicament is a medicament for the prevention and/or treatment of cancer; and more preferably, the cancer is a TF-expressing cancer.

Provided is use of the anti-TF antibody or the antigen-binding fragment thereof in some embodiments, the nucleic acid molecule in some embodiments, the recombinant vector in some embodiments, or the recombinant cell in some embodiments in the preparation of a reagent for the diagnosis of cell proliferation-related diseases. Preferably, the disease is cancer; and more preferably, the cancer is a TF-expressing cancer.

**In another aspect, the present application provides preparation processes and uses of the polypeptide or the polypeptide fragment, the linker, the linker-payload, and the conjugate.**

A method of preparing the polypeptide or polypeptide fragment -P1-P2- as described in some embodiments, or a tautomer, a mesomer, an enantiomer, a diastereomer, or a mixed form thereof, or an acceptable salt thereof, or a precursor thereof.

A method of preparing the linker as described in some embodiments, or a tautomer, a mesomer, an enantiomer, a diastereomer, or a mixed form thereof, or an acceptable salt thereof, or a precursor thereof. The method comprises sequentially linking the polypeptide or the polypeptide fragment -P1-P2- as described in some embodiments with its preceding and succeeding units C1, La, L1, and L2. Wherein, C1, La, L1, and L2 are as defined in some embodiments.

A method of preparing the linker-payload as described in some embodiments, or a tautomer, a mesomer, an enantiomer, a diastereomer, or a mixed form thereof, or an acceptable salt thereof, or a precursor thereof, comprises sequentially linking the polypeptide or the polypeptide fragment -P1-P2- as described in some embodiments with its preceding and succeeding units C1', La, L1, L2, and D. Wherein, C1', La, L1, L2, and D are as defined in some embodiments.

A method of preparing the conjugate as described in some embodiments, or a tautomer, a mesomer, an enantiomer, a diastereomer, or a mixed form thereof, or an acceptable salt thereof, or a precursor thereof, comprises sequentially linking Bp, C1 or C1', La, L1, L2, P1, P2, D, or the precursors thereof,
wherein P1 and P2 are as defined in some embodiments;
C1, La, L1, and L2 are as defined in some embodiments;
C1' and D are as defined in some embodiments; and
Bp is as defined in some embodiments.

A method of preparing the conjugate as described in some embodiments, or a tautomer, a mesomer, an enantiomer, a diastereomer, or a mixed form thereof, or an acceptable salt thereof, or a precursor thereof, comprises performing coupling reaction between Bp and the linker-payload C1'-La-L1-P1-P2-L2-D in a suitable solvent and under suitable conditions. Wherein, C1'-La-L1-P1-P2-L2-D is as defined in some embodiments.

Preferably, a molar ratio of Bp to the linker-payload C1'-La-L1-P1-P2-L2-D is 1:(1-20), and more preferably is 1:4, 1:5, 1:6, 1:7, 1:8, 1:9, 1:10, 1:11, or 1:12.

Preferably, the coupling reaction is carried out in water or a buffered salt system and/or an organic solvent.

Preferably, the buffered salt system is selected from PBS, a sodium acetate buffer solution, a histidine-hydrochloride buffer solution, a histidine-acetate buffer solution, a citrate buffer solution, and the above buffer solutions added with sucrose. More preferably, the buffer solution is selected from PBS pH7.4, PBS pH7.2, PBS pH6.5, PBS pH6.0, histidine-acetate pH6.5, histidine-acetate pH6.0, histidine-acetate pH5.5, and combinations thereof with 5%-9% sucrose.

Preferably, the organic solvent is selected from N,N-dimethylformamide, N,N-dimethylacetamide, dimethyl sulfoxide, N-methylpyrrolidone, acetonitrile, methanol, ethanol, ethylene glycol, propylene glycol, and glycerol.

Preferably, the method further comprises a step of purifying the conjugation product. Preferably, purification is performed by using one or more of an ultrafiltration system, gel chromatography, affinity chromatography, hydrophobic chromatography, reversed phase chromatography, or ionexchange chromatography. More preferably, purification is performed by using one or more of an ultrafiltration tube, a tangential flow system, a desalting column, a gel column, a cation exchange column, an anion exchange column, or an affinity column.

A pharmaceutical composition comprises the polypeptide or the polypeptide fragment as described in some embodiments, the linker as described in any one of some embodiments, the linker-payload as described in any one of some embodiments, the conjugate as described in any one of some embodiments, the antibody or the antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, and optionally one or more pharmaceutical excipients. Preferably, the pharmaceutical composition further comprises a pharmaceutically-acceptable carrier and/or excipient

Provided is use of the polypeptide or the polypeptide fragment as described in any one of some embodiments, the linker as described in any one of some embodiments, the linker-payload as described in any one of some embodiments, the conjugate as described in any one of some embodiments, the antibody or the antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, in the preparation of a medicament for treating/preventing a disease or condition. Alternatively, the present application discloses use of the polypeptide or polypeptide fragment as described in any one of some embodiments, the linker as described in any one of some embodiments, the linker-payload as described in any one of some embodiments, the conjugate as described in any one of some embodiments, the antibody or the antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, in treating/preventing a disease or condition. Alternatively, the present application discloses the polypeptide or the polypeptide fragment as described in any one of some embodiments, the linker as described in any one of some embodiments, the linker-payload as described in any one of some embodiments, the conjugate as described in any one of some embodiments, the antibody or the antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, for use in treating/preventing a disease or condition.

Provided is use of the polypeptide or polypeptide fragment as described in any one of some embodiments, the linker as described in any one of some embodiments, the linker-payload as described in any one of some embodiments, the conjugate as described in any one of some embodiments, the antibody or the antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, in the preparation of a medicament for treating/preventing a disease associated with abnormal cellular activity. Alternatively, the present application discloses use of the polypeptide or the polypeptide fragment as described in any one of some embodiments, the linker as described in any one of some embodiments, the linker-payload as described in any one of some embodiments, the conjugate as described in any one of some embodiments, the antibody or antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, in treating/preventing a disease associated with abnormal cellular activity. Alternatively, the present application discloses the polypeptide or the polypeptide fragment as described in any one of some embodiments, the linker as described in any one of some embodiments, the linker-payload as described in any one of some embodiments, the conjugate as described in any one of some embodiments, the antibody or antigen-binding fragment thereof as described in any one of some embodiments, the nucleic acid molecule as described in some embodiments, the recombinant vector as described in some embodiments, the recombinant cell as described in some embodiments, and tautomers, mesomers, racemates, enantiomers, diastereomers, or available salts thereof, for use in treating/preventing a disease associated with abnormal cellular activity.

In some embodiments, the present application discloses use of the pharmaceutical composition as described herein in the preparation of a medicament for treating/preventing a disease or condition. Alternatively, the present application discloses use of the pharmaceutical composition as described in some embodiments in treating/preventing a disease or condition. Alternatively, the present application discloses the pharmaceutical composition as described in some embodiments for use in treating/preventing a disease or condition.

In some embodiments, the present application discloses use of the pharmaceutical composition as described herein in the preparation of a medicament for treating/preventing a disease associated with abnormal cellular activity. Alternatively, the present application discloses use of the pharmaceutical composition as described in some embodiments in treating/preventing a disease associated with abnormal cellular activity. Alternatively, the present application discloses the pharmaceutical composition as described in some embodiments for use in treating/preventing a disease associated with abnormal cellular activity.

In some embodiments, the disease or condition includes a tumor.

In some embodiments, the disease or condition includes a solid tumor and a hematological malignancy.

A method of treating a disease or condition (for example, a tumor) in a subject in need thereof, comprises administering to the subject an effective amount of the conjugate as described in any one of the embodiments. The effective amount herein may be determined by those skilled in the art based on the age, gender, body weight, health status, family medical history, disease progression, and the like of the subject. The conjugate described herein may be administered to a subject in conventional dosage forms via conventional routes of administration (for example, intramuscular injection, etc.). In some embodiments, the tumor is selected from tumors associated with the expression of the following proteins: EGFR, HER2, HER3, c-Met, claudin18.2, TROP-2, folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, or IGF-1R;
preferably, selected from tumors associated with Her2 expression;
preferably, selected from tumors associated with Trop2 expression;
preferably, selected from tumors associated with EGFR expression;
preferably, selected from tumors associated with FRα expression;
preferably, selected from tumors associated with TF expression;
preferably, selected from tumors associated with ROR1 expression; and
preferably, selected from tumors associated with GPC3 expression.

In some embodiments, the tumor is selected from esophageal cancer, brain cancer, lung cancer, epithelial cell carcinoma, bladder cancer, gastric cancer, ovarian cancer, pancreatic cancer, head and neck cancer, urothelial carcinoma, colon cancer, colorectal cancer, cervical cancer, endometrial cancer, rectal cancer, renal cancer, prostate cancer, melanoma, liver cancer, gallbladder cancer, cholangiocarcinoma, thyroid cancer, non-Hodgkin's lymphoma, multiple myeloma, and B-cell lymphoma.

Preferably, the cancer is selected from esophageal cancer, lung cancer, breast cancer, gastric cancer, urothelial carcinoma, cholangiocarcinoma, prostate cancer, colorectal cancer, cervical cancer, and ovarian cancer.

### Term definition

Unless otherwise defined, all the terms, symbols, and other scientific terms used herein are intended to have the meanings commonly understood by those skilled in the art. In certain instances, terms having commonly understood meanings are defined herein for clarity and/or ready reference, and the inclusion of such definitions herein is not to be construed as indicating a meaning different from that commonly understood in the art. The techniques and procedures described or referenced herein are generally well understood and routinely employed by those skilled in the art.

Where appropriate, unless otherwise specified, procedures involving the use of commercially available kits and reagents are generally carried out in accordance with the agreements and conditions defined by manufacturers. As used herein, unless otherwise explicitly indicated in the context, the singular forms "one", "a/an", and "the" include plural referents. Unless expressly stated otherwise, the terms "comprising", "such as", and the like are intended to express the inclusion without limiting. As used herein, the term "comprising" also specifically encompasses embodiments "consisting of the elements" and "consisting essentially of the elements ", unless expressly stated otherwise.

In the present application, the terms "antibody drug conjugate (antibody-drug conjugate)", "antibody drug conjugate (antibody-drug conjugate)", and "antibody drug conjugate (ADC)" are used interchangeably and generally refer to one or more therapeutic compounds (for example, Dxd) linked to one or more antibodies or antigen-binding fragments thereof, and are defined by the following general formula: Bp-(L-D)p, wherein Bp = antibody or antigen-binding fragment, L = linker, D = drug unit, and p = number of drug moieties per antibody or antigen-binding fragment, which may be any number from 1 to 10. In some embodiments, the linker L may include a cleavable moiety positioned between the antibody or antigen-binding fragment and the drug unit. Exemplary cleavable linkers are described and exemplified herein.

In the present application, the term "antibody" or "antigen-binding fragment thereof" is generally used in its broadest sense and includes certain types of immunoglobulin molecules comprising one or more antigen-binding domains that specifically bind to an antigen or epitope. Antibody specificity specifically covers monoclonal antibodies, polyclonal antibodies, dimers, polymers, multispecific antibodies (for example, bispecific antibodies), and antibody fragments such as Fab', Fab, F(ab')2, single domain antibodies (dAbs), Fv, scFv (single-chain Fv), linear antibodies, diabodies, and the like; so long as they exhibit the desired biological activity (Miller et al. (2003) Jour. of Immunology 170:4854-4861). The antibody may be a murine, human, humanized, chimeric antibody, or derived from other species. Without limitation, "antibody" typically may contain a protein consisting of at least two heavy chains (HC) and two light chains (LC) linked to each other by disulfide bonds, or an antigen-binding fragment thereof. Each heavy chain includes a heavy chain variable region (VH) and a heavy chain constant region. In certain natural IgG, IgD, and IgA antibodies, the heavy chain constant region includes three domains: CH1, CH2, and CH3. In certain natural antibodies, each light chain includes a light chain variable region (VL) and a light chain constant region. The light chain constant region includes one domain, CL. The VH and VL regions may be further subdivided into hypervariable regions, referred to as complementarity determining regions (CDRs), which alternates with relatively conserved regions known as framework regions (FRs). Each VH and VL include three CDRs and four framework regions (FRs), arranged from an amino terminus to a carboxy terminus in the following order: FR1, CDR1, FR2, CDR2, FR3, CDR3, and FR4. The variable domains of natural heavy and light chains each include four FR regions (HFR1, HFR2, HFR3, HFR4, LFR1, LFR2, LFR3, and LFR4); most of them are in a β-sheet configuration, are connected by three CDRs to form loop connections. Moreover, in some cases, part of the β-folded structure is formed. The CDRs within each chain are brought into close proximity by the FR regions and, together with the CDRs from the other chain, form the antigen binding site of the antibody. The constant region of an antibody may mediate the binding of immunoglobulins to host tissues or factors, including various cells of the immune system (e.g., effector cells) and the first component (Clq) of the classical complement system.

In the present application, the term "CDR" generally refers to one of the six hypervariable regions within the variable domain of an antibody that primarily contribute to antigen binding. One of the most commonly used definitions of the six CDRs is provided by Kabat E.A. et al., (1991) Sequences of proteins of immunological interest. NIH Publication 91-3242), Chothia et al., "Canonical Structures For the Hypervariable Regions of Immunoglobulins", J. Mol. Biol. 196:901 (1987); and MacCallum et al., Antibody-Antigen Interactions: Contact Analysis and Binding Site Topography, J. Mol. Biol. 262:732 (1996)). As used in the present application, the Kabat definition of CDRs may be applied to CDR1, CDR2, and CDR3 (CDR L1, CDR L2, CDR L3 or L1, L2, L3) of the light chain variable domain, as well as CDR1, CDR2, and CDR3 (CDR H1, CDR H2, CDR H3 or H1, H2, H3) of the heavy chain variable domain.

In the present application, the term "variable" generally refers to the fact that certain portions of the sequence of an antibody variable domain are highly diverse, thus forming the binding and specificity of various specific antibodies to the specific antigens thereof. However, the variability is not uniformly distributed throughout the entire variable region of the antibody. It is concentrated in three segments of the light and heavy chain variable regions, referred to as complementarity determining regions (CDRs) or hypervariable regions (HVRs). The more highly conserved portions in the variable domain are referred to as framework regions (FRs). In the art, the CDRs of an antibody may be defined by various methods, such as Kabat definition rule based on sequence variability (see Kabat et al., Protein Sequences of Immunology, 5th edition, National Institutes of Health, Bethesda, Maryland (1991)); Chothia definition rule based on the position of a structural ring region (see Al-Lazikani et al., J Mol Biol 273:927-48, 1997), and KABAT definition rule based on IMGT-ONTOLOGY concepts and IMGT Scientific chart rules.

In the present application, the term "monoclonal antibody" generally refers to an antibody obtained from a population of substantially homogeneous antibodies. That is, individual antibodies in the population are identical except a small number of possible natural mutations. Monoclonal antibodies typically exhibit high specificity for a single antigenic site. Furthermore, unlike conventional polyclonal antibody formulations (which typically have different antibodies directed against different epitopes), each monoclonal antibody is directed against a single epitope on the antigen. In addition to their specificity, the advantage of monoclonal antibodies is that they may be synthesized by hybridoma culture, free from contamination by other immunoglobulins. The modifier "monoclonal" refers to the feature of an antibody obtained from a population of substantially homogeneous antibodies and is not to be construed as requiring production of the antibody by any particular method. For example, the monoclonal antibodies used in the present application may be prepared in hybridoma cells or may be prepared by recombinant DNA methods.

In the present application, the term "chimeric antibody" generally refers to an antibody in which the variable region is derived from one species and the constant region is derived from another species. Typically, the variable region is derived from an antibody ("parent antibody") of an experimental animal, e.g., a rodent, and the constant region is derived from a human antibody, such that the resulting chimeric antibody is less likely to elicit an undesirable immune response in a human subject as compared to the parent (e.g., murine-derived) antibody.

In the present application, the term "humanized antibody" generally refers to an antibody in which some or all amino acids outside the CDR regions of a non-human antibody (e.g., a murine antibody) are replaced with the corresponding amino acids derived from a human immunoglobulin. In the CDR regions, minor additions, deletions, insertions, substitutions, or modifications of amino acids are also permissible, as long as they still retain the ability to bind specific antigens. The humanized antibody may optionally include at least a portion of a human immunoglobulin constant region. A "humanized antibody" retains the antigen specificity similar to that of the original antibody. A "humanized" form of a non-human (e.g., murine) antibody may minimally include a chimeric antibody containing sequences derived from a non-human immunoglobulin. In certain instances, CDR residues in a human immunoglobulin (receptor antibody) may be replaced with CDR residues from a non-human species (donor antibody) having the desired properties, affinity, and/or capability, such as a mouse, rat, rabbit, or a non-human primate. In certain instances, FR residues of a human immunoglobulin may be replaced with the corresponding non-human residues. Additionally, a humanized antibody may include amino acid modifications that are not present in the receptor antibody or the donor antibody. Such modifications may be made to further improve the performance of the antibody, such as binding affinity.

In the present application, for the term "fully-humanized antibody", "full-human antibody" or "completely-humanized antibody", also referred to as a "fully-humanized monoclonal antibody", both the variable and constant regions of these antibodies are of human origin, and immunogenicity and toxicity are eliminated. The development of monoclonal antibodies has gone through four stages: murine monoclonal antibodies, chimeric monoclonal antibodies, humanized monoclonal antibodies, and fully-humanized monoclonal antibodies, respectively. The antibody or ligand described in the present application may be a fully-humanized monoclonal antibody. Relevant technologies for the preparation of full-human antibodies may include: human hybridoma technology, EBV-transformed B lymphocyte technology, phage display technology, transgenic mouse antibody preparation technology, and single B cell antibody preparation technology, etc. The terms "full-length antibody", "intact antibody", and "complete antibody" are used interchangeably herein to refer to an antibody that has a structure substantially similar to that of a natural antibody and has a heavy chain containing an Fc region. For example, when referring to an IgG molecule, the "full-length antibody" is an antibody containing two heavy chains and two light chains.

In the present application, the term "Fc region" refers to the C-terminal region of an immunoglobulin heavy chain. In natural antibodies, the region interacts with Fc receptors and certain proteins of the complement system. The structures of the Fc regions in various immunoglobulins and the glycosylation sites contained therein are known in the art. J Allergy Clin Immunol, 2010, 125: S41-52, which is incorporated herein by reference in its entirety. The Fc region may be a naturally occurring Fc region or an Fc region modified as described elsewhere in the art or in the present disclosure.

In the present application, the term "antigen-binding domain or binding fragment" refers to a portion of an antibody that is capable of specifically binding to an antigen or epitope. An example of an antigen-binding domain is an antigen-binding domain formed by a VH-VL dimer of an antibody. Another example of an antigen-binding domain is an antigen-binding domain formed by diversification of certain loops of the tenth fibronectin type III domain of a connexin. Antigen-binding domains may be found in various situations, including antibodies and chimeric antigen receptors (CARs), for example, CARs derived from antibodies or antibody fragments (e.g., scFvs). In the present application, the term "ligand" generally refers to a macromolecular compound that may recognize and bind to an antigen or receptor associated with a target cell. The function of the ligand may deliver a drug to a target cell population that binds the ligand. These ligands include, but are not limited to, protein hormones, lectins, growth factors, antibodies, or other molecules capable of binding to cells, receptors, and/or antigens. In the present application, the ligand may be denoted as Ab, and the ligand antigen forms a linkage bond with the linker unit through a heteroatom on the ligand that may be an antibody or an antigen-binding fragment thereof. The antibody may be selected from chimeric antibodies, humanized antibodies, full-human antibodies, or murine antibodies; the antibody may be a monoclonal antibody. For example, the antibody may be an antibody targeting the following targets: HER2, HER3, B7H3, TROP2, Claudin 18.2, CD30, CD33, CD70, or EGFR. For example, the antibody may be an antibody targeting the following targets: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGREI, AG-7, AIF1, AKRIC1, AKRIC2, ASLG659, Axl, B7H3, BAFF-R, BCMA, BMPRIB, BNIP3, CIQA, CIQB, CA6, CADMI, CCD79b, CCL5, CCR5, CCR7, CDIle, CD123, CD138, CD142, CD147, CD166, CD19, CD19, CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDIIb, CEA, CEACAMS, c-Met, COL6A3, COL7AI, CRIPTO, CSFIR, CTSD, CTSS,CXCL11, CXCL10, DDIT4, DLL3, DLL4, DR5, E16, EFNA4, EGFR, EGFRvIII, EGLN, EGLN3, EMR2, ENPP3, EpCAM, EphA2, EphB2R, ETBR, FcRH2, FcRHI, FGFR2, FGFR3, FLT3, FOLR-a, GD2, GEDA, GPC-1, GPNMB, GPR20, GZMB, HER2, HER3, HLADOB, HMOX1, IFI6, IFNG, IGF-1R, IGFBP3, IL1ORA1, IL-13R, IL-2, IL20Ra, IL-3, IL-4, IL-6, IRTA2, KISSIR, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b,Napi3b, Nectin-4, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PSMA, PTK7, P-cadherin, RNF43, NaPi2b, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, Trop-2, TrpM4, Tyro7, UPKIB, VEGFA, WNT5A, epidermal growth factor, brevican, mesothelin, sodium-phosphate cotransporter 2B, claudin 18.2, endothelin receptor, mucins (such as mucin 1 and mucin 16), guanylyl cyclase C, integrin α4β7, integrin αvβ6, trophoblast glycoprotein, or tissue factor.

In the present application, the term "specifically bind", when referring to the interaction between a binding molecule (e.g., antibody) and its binding partner (e.g., antigen), generally refers that the interaction depends on the presence of a particular structure (e.g., an antigenic determinant or an epitope) on the binding partner. In other words, even when the binding partner is present in a mixture of other molecules or organisms, the antibody will still preferentially bind to or recognize the binding partner. The binding may be mediated by covalent or non-covalent interactions, or a combination thereof. In other words, the term "specifically bind" generally refers to immunospecific binding to an antigenic determinant or epitope, but not immunospecific binding to other antigenic determinants or epitopes. A binding molecule that immunospecifically binds to an antigen may bind to other peptides or polypeptides with lower affinity, as determined by radioimmunoassay (RIA), enzyme-linked immunosorbent assay (ELISA), BIACORE, or other assays known in the art. A binding molecule or fragment thereof that immunospecifically binds to an antigen may cross-react with related antigens carrying the same epitope. In certain instances, a binding molecule or fragment thereof that immunospecifically binds to an antigen does not cross-react with other antigens.

In the present application, the term "affinity" refers to the intensity of the sum of non-covalent interactions between a single binding site of a molecule (e.g., an antibody) and its binding partner (e.g., an antigen or epitope). Unless otherwise indicated, as used herein, the "affinity" refers to intrinsic binding affinity, which reflects the 1:1 interaction between members of a binding pair (e.g., an antibody and antigen or epitope). The affinity of a molecule X for its partner Y may be expressed by a dissociation equilibrium constant (K_{D}). The kinetic components affecting the dissociation equilibrium constant will be described in more detail below. Affinity may be measured by conventional methods known in the art, including the methods described herein, such as surface plasmon resonance (SPR) technology (e.g., BIACORE^{®}) or bio-layer interferometry (e.g., FORTEBIO).

In the present application, the term "K_{d}" (sec⁻¹) refers to a dissociation rate constant for a particular antibody-antigen interaction. This value is also referred to as K_{off} value.

In the present application, the term "kₐ" (M⁻¹ × sec⁻¹) refers to an association rate constant for a particular antibody-antigen interaction. This value is also referred to as kₒₙ value.

In the present application, the term "K_{D}" (M), as used herein, refers to a dissociation equilibrium constant for a particular antibody-antigen interaction. K_{D} = k_{d}/kₐ. In some embodiments, the affinity of an antibody is described by the K_{D} of the interaction between the antibody and its antigen. For clarity, as known in the art, a smaller K_{D} value indicates a higher affinity interaction, while a larger K_{D} value indicates a lower affinity interaction. If K_{D} of an antibody binding to antigen X is less than or equal to 5 × 10⁻⁸ M, the antibody is referred to as "specifically binding" to the antigen X; more ideally, the K_{D} is 1 × 10⁻⁸ M or less, more ideally 6 × 10⁻⁹ M or less, further ideally 3 × 10⁻⁹ M or less, and more ideally 2 × 10⁻⁹ M or less. The antibody may be chimeric, humanized, or preferably human antibody.

In the present application, the term "K_{A}" (M) refers to a binding equilibrium constant for a specific antibody-antigen interaction. K_{A} = kₐ/k_{d}.

In the present application, the term "linker", "adapter", "linker", "L" or "adapter structure", or "linker moiety" generally refers to a chemical structural fragment whose one end is for connection to a ligand and the other end is for connection to a cytotoxic drug, or that may be further linked to other linkers prior to connection to a cytotoxic drug. The direct or indirect connection to the ligand may refer that the group is directly connected to the ligand via covalent bonds or is connected to the ligand via a linker structure. The linker may be prone to or substantially resistant to acid-induced cleavage, peptidase-induced cleavage, light-based cleavage, esterase-induced cleavage, and/or disulfide bond cleavage under the condition of maintaining the activity of the compound or antibody.

In the present application, the term "polypeptide or a fragment thereof" refers to a peptide structural unit containing at least two covalently-attached amino acids. The term encompasses polypeptides, oligopeptides, and peptides. In some embodiments, two or more covalently-attached amino acids are joined by peptide bonds and may be composed of naturally occurring amino acids and peptide bonds. Alternatively, synthetic amino acids (e.g., homo-phenylalanine, citrulline, ornithine, and norleucine) or peptidomimetic structures, i.e., "peptide or protein analogs", e.g., peptoids, may be included. Peptoids are an exemplary class of peptide mimics; the side chains thereof are side-attached to the nitrogen atom of the peptide backbone, rather than the α-carbon (as in amino acids). As compared to peptides, peptoids have different hydrogen bonds and conformational features. Accordingly, peptoids may resist proteolysis or other physiological or storage conditions, and efficiently penetrate cell membranes. Such synthetic amino acids may be incorporated, especially when the antibody is synthesized *in vitro* by conventional methods well known in the art. In addition, any combination of peptidomimetic, synthetic, and naturally occurring residues/structures may be used. "Amino acids" also include imino acid residues, such as proline and hydroxyproline. The amino acid "R group" or "side chain" may be in the (L)- or (S)- configuration. In a particular embodiment, the amino acid is in the (L)- or (S)- configuration.

In the present application, the term "drug unit" or "D" generally refers to any compound having the desired biological activity and a reactive functional group, which may be used to incorporate the drug into the conjugate of the present application. In some embodiments, the drug unit represents a cytotoxic drug for cancer therapy; a protein or polypeptide with the desired biological activity, such as toxins being abrin, ricin A, *Pseudomonas* exotoxin, and diphtheria toxin; other suitable proteins, including tumor necrosis factors, α-interferon, β-interferon, nerve growth factors, platelet-derived growth factors, tissue plasminogen activators, and biological response modifiers, such as lymphokines, interleukin-1 (IL-1), interleukin-2 (IL-2), interleukin-6 (IL-6), granulocyte-macrophage colony-stimulating factor (GM-CSF), granulocyte colony-stimulating factor (G-CSF), or other growth factors.

In the present application, the term "cytotoxic drug" or "cytotoxic agent" generally refers to a toxic drug. The cytotoxic drug may refer to a substance that inhibits or prevents cell function and/or induces cell death or destruction. Cytotoxic drugs may kill tumor cells at sufficiently high concentrations. The "cytotoxic drug" may include cytotoxic drugs, including, but not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹², and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunomycin or other intercalating agents; growth inhibitors; enzymes and fragments thereof, such as nucleotide-degrading enzymes; antibiotics; toxins, such as small molecule toxins or enzyme-active toxins derived from bacteria, fungi, plants, or animals, including fragments and/or variants thereof; and various anti-tumor or anti-cancer agents as disclosed herein.

In the present application, the term "isotope-labeled compound" or "isotope-labeled derivative" generally refers to a currently disclosed compound in which one or more atoms are replaced by atoms having atomic masses or mass numbers different from those typically found in nature, including the respective pharmaceutical salts and prodrugs as described herein. Examples of isotopes that may be introduced into the currently disclosed compounds include isotopes of hydrogen, carbon, nitrogen, oxygen, phosphorus, fluorine, and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

In the present application, the term "pharmaceutically acceptable form" generally refers to forms of the compounds disclosed, including but not limited to, pharmaceutically acceptable salts, esters, hydrates, solvates, polymorphs, isomers, prodrugs, and isotope labeled derivatives thereof. In one embodiment, the "pharmaceutically acceptable form" includes, but is not limited to, pharmaceutically acceptable salts, esters, prodrugs, and isotope-labeled derivatives thereof. In some embodiments, the "pharmaceutically acceptable form" includes, but is not limited to, pharmaceutically acceptable isomers and stereoisomers, prodrugs, and isotope-labeled derivatives thereof.

The linker, linker-payload, and conjugate of the present application may include any pharmaceutically acceptable form thereof, such as pharmaceutically acceptable salts, esters, hydrates, solvates, polymorphs, isomers, prodrugs, or isotope-labeled derivatives. The above pharmaceutically acceptable forms are also within the scope of the present application.

In the present application, the term "pharmaceutically acceptable salt" generally refers to a pharmaceutically acceptable organic or inorganic salt of a compound (e.g., a drug, linker-payload, or conjugate). In some aspects, the compound may contain at least one amino group and, therefore, may form an acidic addition salt with the amino group. Exemplary salts include, but are not limited to, sulfates, trifluoroacetates, citrates, acetates, oxalates, chlorides, bromides, iodides, nitrates, bisulfates, phosphates, acid phosphates, isonicotinates, lactates, salicylates, citrates, tartrates, oleates, tannates, pantothenates, bitartrates, ascorbates, succinates, maleates, gentisates, fumarates, gluconates, glucuronates, saccharates, formates, benzoates, glutamates, methanesulfonates, ethanesulfonates, benzenesulfonates, p-toluenesulfonates, and pamoates (i.e., 1,1'-methylene-bis-(2-hydroxy-3-naphthoate)). Pharmaceutically acceptable salts may involve the introduction of another molecule such as an acetate ion, succinate ion, or other counterions. The counterion may be any organic or inorganic moiety that will stabilize the charge on the parent compound. Furthermore, pharmaceutically acceptable salts may have more than one charged atom in their structure. Given that multiple charged atoms are part of a pharmaceutically acceptable salt, there may be multiple counterions. Accordingly, a pharmaceutically acceptable salt may have one or more charged atoms and/or one or more counterions.

In the present application, the term "derivative" generally refers to a chemical compound or molecule prepared from a parent compound by one or more chemical reactions.

In the present application, the term "prodrug" generally refers to a compound with relatively low or no biological activity, which is converted *in vivo* to a more biologically active compound by a chemical or biological process (i.e., a chemical reaction or enzymatic biotransformation).

In the present application, the term "pharmaceutically acceptable carrier" generally refers to one or more nontoxic substances that do not interfere with the efficacy of the biological activity of active ingredients. Such formulations may typically contain salts, buffering agents, preservatives, compatible carriers, adjuvants, and optionally other therapeutic agents. Such pharmaceutically acceptable formulations may also typically contain compatible solid or liquid fillers, diluents, or encapsulating materials suitable for administration to human. Without limitations, pharmaceutically acceptable carriers may include liquids, such as water, saline, glycerol, and ethanol. Auxiliary substances, such as wetting agents or emulsifiers, and pH buffering substances, may also be present in these carriers.

In the present application, the term "methylene" generally refers to a residue derived from one carbon-atom group after removing two hydrogen atoms. Methylene may be substituted or unsubstituted, replaced or un-replaced.

The term "alkylene" generally refers to a saturated linear or branched aliphatic hydrocarbon alkyl, which is a residue derived by removing two hydrogen atoms from the same or two different carbon atoms of a parent alkyl. It may be a linear or branched group containing 1 to 20 carbon atoms, for example, alkylene containing 1 to 12 carbon atoms, and for example, containing 1 to 6 carbon atoms. Non-limiting examples of alkylene include, but are not limited to: methylene (-CH₂-), 1,1-ethylidene (-CH(CH₃)-), 1,2-ethylidene (-CH₂CH₂), 1,1-propylidene (-CH(CH₂CH₃)-), 1,2-propylidene (CH₂CH(CH₃)-), 1,3-propylidene (-CH₂CH₂CH₂-), 1,4-butylidene (-CH₂CH₂CH₂CH₂-), and 1,5-butylidene (-CH₂CH₂CH₂CH₂CH₂-), etc. Alkylene may be substituted or unsubstituted, replaced or un-replaced; for example, when being substituted, the substituent group may be substituted at any available point of attachment. Preferably, the substituent group is independently and optionally substituted by one or more substituents selected from alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, for example, it may be hydrogen, protium, deuterium, tritium, halogen, -NO₂, -CN, -OH, -SH, -NH₂, -C(O)H, -CO₂H, - C(O)C(O)H, -C(O)CH₂C(O)H, -S(O)H, -S(O)₂H, -C(O)NH₂, -SO₂NH₂, -OC(O)H, -N(H)SO₂H, or a C1-6 aliphatic group.

In the present application, the term "arylene" generally refers to a residue derived by removal of two hydrogen atoms from the same or two different carbon atoms of an aromatic ring.

The term "aromatic ring" may refer to a 6- to 14-membered all-carbon monocyclic or fused polycyclic ring (i.e., the ring sharing pairs of adjacent carbon atoms) having a conjugated π electron system, which may be 6- to 10- membered, for example, benzene and naphthalene. The aromatic ring may be fused to a heteroaryl, heterocyclyl, or cycloalkyl ring, wherein the ring linked to the parent structure is an aryl ring. The aryl may be substituted or unsubstituted; when substituted, the substituent is preferably one or more groups independently selected from the group of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaromatic ring" refers to a heteroaromatic system containing 1 to 4 heteroatoms and 5 to 14 ring atoms, wherein the heteroatom may be selected from the group of oxygen, sulfur, and nitrogen. The heteroaryl may be 5- to 10-membered, may be 5- or 6-membered, for example, furanyl, thienyl, pyridyl, pyrrolyl, N-alkylpyrrolyl, pyrimidinyl, pyrazinyl, imidazolyl, tetrazolyl, and the like. The heteroaryl ring may be fused to an aryl, heterocyclyl, or cycloalkyl ring, wherein the ring fused to the parent structure is a heteroaryl ring. The heteroarylene may be optionally substituted or unsubstituted; when substituted, the substituent is preferably one or more groups independently selected from the group of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaryl" refers to an aromatic group containing heteroatoms and having 5 or more ring atoms. For example, "C2-C10 heteroaryl" refers to a group containing 2-10 carbon atoms and the remaining number of heteroatoms among the ring atoms.

In the present application, the term "heterocyclylene" generally refers to a stable, non-aromatic 3- and 7-membered monocyclic structure, a fused 7- and 10-membered bicyclic heterocyclic structure, or a 6- and 10-membered bridged bicyclic heterocyclic structure. These cyclic structures may be saturated or partially saturated; and in addition to carbon atoms, these cyclic structures further contain one or more heteroatoms, wherein the heteroatoms may be selected from the group of oxygen, sulfur, and nitrogen; for example, containing 1-4 heteroatoms as defined above. When used to refer to an atom on a heterocyclic ring structure, the term "nitrogen" may include nitrogen that has undergone a substitution reaction. The heterocyclylene may be substituted or unsubstituted.

In the present application, the term "heteroatom" refers to an atom other than C and H or a group containing such an atom, for example, N, O, S, P, NR, and the like.

In the present application, the term "carbocyclylene" generally refers to a residue derived by removal of two hydrogen atoms from the same or two different carbon atoms of a carbocycle. The term "carbocycle" generally refers to a saturated or partially unsaturated monocyclic or polycyclic cyclic hydrocarbon. The carbocycle contains 3 to 20 carbon atoms, and may contain 3 to 12 carbon atoms, may contain 3 to 10 carbon atoms, or may contain 3 to 8 carbon atoms. Non-limiting examples of monocyclic carbocycles include cyclopropane, cyclobutane, cyclopentane, cyclopentene, cyclohexane, cyclohexene, cyclohexadiene, cycloheptane, cycloheptatriene, cyclooctane, and the like; polycyclic carbocycles may include spiro, fused, and bridged carbocycles. The carbocyclylene may be substituted or unsubstituted.

In the present application, the term "partially unsaturated" generally refers to a situation of a ring structure comprising at least one double or triple bond between ring atoms. The term "partially unsaturated" encompasses ring structures with multiple sites of unsaturation, but is not intended to include the aromatic ring or heteroaromatic ring as defined in the present application. The term "unsaturated" means that the moiety has one or more degrees of unsaturation.

In the present application, the term "halogen" refers to any halogen group, generally referring to fluorine (F), chlorine (Cl), bromine (Br), or iodine (I), for example, F or Cl.

In the present application, the term "haloalkyl" refers to an alkyl group substituted by one or more halogen atoms.

In the present application, the term "aliphatic group" generally refers to a linear, branched, or cyclic hydrocarbon containing 1-12 carbon atoms; these hydrocarbons may be fully saturated hydrocarbons or may contain one or more unsaturated units, but the unsaturated units are not aromatic groups. For example, suitable aliphatic groups may include substituted or unsubstituted linear, branched, or cyclic alkyl, alkenyl, alkynyl, and mixtures of these groups; for example, (cycloalkyl)alkyl, (cycloalkenyl)alkyl, or (cycloalkyl)alkenyl. For example, the aliphatic group contains 1-12, 1-8, 1-6, 1-4, or 1-3 carbon atoms.

In the present application, the term "alkyl" or "alkyl group" generally refers to a fully saturated linear, branched, or cyclic-hydrocarbon chain. In certain embodiments, alkyl may contain 1-8 carbon atoms ("C1-C8 alkyl"). In certain embodiments, alkyl may contain 1-6 carbon atoms ("C1-C6 alkyl"). In certain embodiments, alkyl contains 1-3 carbon atoms. In yet other embodiments, alkyl contains 2-3 carbon atoms, and in yet other embodiments, alkyl contains 1-2 carbon atoms.

In the present application, the term "alkylalkoxy" means alkyl substituted with an alkoxy group.

In the present application, the term "alkoxy" refers to alkyl as previously defined, the main carbon chain of which is connected by an oxygen ("alkoxy") atom.

In the present application, the term "alkylhydroxy" means alkyl substituted with a hydroxy group.

In the present application, the term "hydroxy" (hydroxy or hydroxyl) refers to -OH.

In the present application, the term "carbocycle" or "carbocyclyl" includes both aromatic groups (e.g., aryl) and non-aromatic groups (e.g., cycloalkyl). In certain embodiments, the carbocyclyl group contains 3-10 carbon atoms ("3- to 10-membered carbocycle"). In certain embodiments, the carbocyclyl group contains 3-8 carbon atoms ("3- to 8-membered carbocycle"). In certain embodiments, the carbocyclyl group contains 3-6 carbon atoms ("3- to 6-membered carbocycle"). In certain embodiments, the carbocyclyl group contains 3-5 carbon atoms ("3- to 5-membered carbocycle").

In the present application, the term "heterocycle, heterocyclyl, or heterocyclic" and "heterocyclyl group" means a monocyclic, bicyclic, or tricyclic heterocycle containing at least one heteroatom in the ring. The term "heterocycle", "heterocyclyl", or "heterocyclic", and "heterocyclyl group" encompasses heteroaryl. "Heteroaryl" refers to a cyclic moiety having one or more closed rings and having one or more heteroatoms (oxygen, nitrogen, or sulfur) in at least one such ring, wherein at least one of such rings is an aromatic ring, and wherein the ring or such rings may independently be fused and/or bridged. Examples include, but are not limited to, phenyl, thienyl, triazolyl, pyridyl, pyrimidinyl, pyridazinyl, and pyrazinyl.

In the present application, the term "PEG unit" generally refers to an organic moiety composed of repeating ethylene-oxy subunits (PEG or PEG subunits) and may be polydisperse, monodisperse, or discrete (i.e., having a discrete number of ethylene-oxy subunits). Polydisperse PEG is a heterogeneous mixture in terms of size and molecular weight, whereas monodisperse PEG is typically purified from such heterogeneous mixtures and thus possesses a single chain length and a single molecular weight. The PEG units of the present application may contain one or more polyethylene glycol chains, and each polyethylene glycol chain consists of one or more ethyleneoxy subunits covalently attached to each other. The polyethylene glycol chains may, for example, be linked together in a linear, branched, or star-shaped configuration.

In the present application, the term "bond (binding)" refers to the physical or chemical interaction between two substances or between combinations thereof. Bonds include ionic bonds, non-ionic bonds, hydrogen bonds, van der Waals bonds, hydrophobic interactions, and the like. Physical interactions (bonds) may be direct or indirect. Indirect physical interactions (bonds) are mediated or caused by the action of another protein or compound. A direct bond refers to an interaction that does not occur through or as a result of the action of another protein or compound, and essentially does not involve another intermediate.

In the present application, the term "optional" or "optionally" generally means that the event or circumstance subsequently described may, but need not, occur, and the description includes instances in which the event or circumstance occurs or does not occur. For example, "a heterocyclic group optionally substituted by alkyl" means that the alkyl may, but not necessarily, be present, and the description may include a situation in which the heterocyclic group is substituted by alkyl and a situation in which the heterocyclic group is not substituted by alkyl.

In the present application, the term "substituted" generally refers to one or more hydrogen atoms in a group, for example 5 atoms at most, for example, 1-3 hydrogen atoms are each independently substituted by a corresponding number of substituents. The substituents are only located in their possible chemical positions. Possible or impossible substitutions can be determined by those skilled in the art (either experimentally or theoretically) without undue efforts. For example, an amino or hydroxy with free hydrogen may be unstable when binds to a carbon atom with an unsaturated (e.g., olefinic) bond.

In the present application, the term 0 or more (e.g., 0 or at least 1, 0 or 1, 0) methylene units being "substituted" generally means that when the structure contains one or more methylene units, the one or more methylene units may be unsubstituted, or substituted by one or more groups other than methylene (e.g., -NHC(O)-, -C(O)NH-, -C(O)-, -OC(O)-, -C(O)O-, -NH-, -O-, -S-, -SO-, -SO₂-, -PH-, -P(-O)H-, -NHSO₂-, -SO₂NH-, -C(-S)-, -C(=NH)-, -N=N-, -C=N-, -N=C-, or -C(=N₂)-). One or more hydrogen atoms in a group, for example, 5 atoms at most, for example, 1-3 hydrogen atoms, are each independently substituted by a corresponding number of substituents. The substituents are only located in their possible chemical positions. Possible or impossible substitutions can be determined by those skilled in the art (either experimentally or theoretically) without undue efforts. For example, an amino or hydroxy with free hydrogen may be unstable when binds to a carbon atom with an unsaturated (e.g., olefinic) bond.

In the present application, the term "compound" generally refers to a substance having two or more different elements. For example, the compounds of the present application may be organic compounds, such as compounds having a molecular weight of 500 or less, compounds having a molecular weight of 1,000 or less, compounds having a molecular weight 1,000 or more, or compounds having a molecular weight of 10,000 or more, or100,000 or more. In the present application, the compounds may also refer to compounds linked by chemical bonds, for example, compounds in which one or more molecules having a molecular weight of 1000 or less are linked to a biomacromolecule by chemical bonds. The biomacromolecule may be a polysaccharide, protein, nucleic acid, polypeptide, or the like. For example, the compounds of the present application may include compounds in which a protein is linked to one or more molecules having a molecular weight of 1000 or less, compounds in which a protein is linked to one or more molecules having a molecular weight of 10,000 or less, or compounds in which a protein is linked to one or more molecules having a molecular weight of 100,000 or less. Unless otherwise specified, the structures described in the present application may also include compounds that differ only in the presence or absence of one or more isotopically enriched atoms. For example, except hydrogen atoms are substituted with deuterium or tritium, or carbon atoms are substituted with carbon-13 or carbon-14, compounds with remainders being consistent with the structures of the present application fall within the scope of the present application.

In the present application, the term "solvent", "solvent compound", or "buffer" generally refers to a pharmaceutically acceptable solvate formed by the ligand-drug conjugate of the present application and one or more solvent molecules. Non-limiting examples of the solvent molecules include water, ethanol, acetonitrile, isopropanol, DMSO, ethyl acetate, and the like.

In the present application, the term "drug loading" generally refers to the average number of the cytotoxic drug loaded onto each ligand, and may also be expressed as a ratio of the cytotoxic drug amount to antibody amount. The range of cytotoxic drug loading may be 0-12 linked on each ligand (Ab). In the embodiments of the present application, the drug loading is denoted as "n", which may exemplarily have a mean value of 1, 2, 3, 4, 5, 6, 7, 8, 9, or 10. The drug loading of each ADC molecule after conjugation may be characterized and identified by conventional methods such as UV/visible spectrophotometry, mass spectrometry, ELISA, and HPLC.

It should be understood by those skilled in the art that "substitution", "substituted", or "absent/absence" includes the following implicit limitations. That is, the substitution or absence is based on the permissible valence number of the substituted atoms and the substituents, and the substitution or absence results in a stable compound. For example, the stable compound does not spontaneously undergo transformations such as rearrangement, cyclization, or elimination. For the purposes of the present disclosure, heteroatoms such as nitrogen may have hydrogen substituents and/or any permissible substituent of the organic compounds described herein satisfying the valence number of the heteroatoms.

In the present application, the term "prevention and/or treatment" not only includes the prevention and/or treatment of diseases, but also generally includes preventing the onset of diseases, slowing down or reversing the progression of diseases, preventing or slowing down the onset of one or more symptoms associated with diseases, reducing and/or alleviating one or more symptoms associated with diseases, reducing the severity and/or duration of diseases and/or any associated symptoms thereof, and/or preventing the further increase in the severity of diseases and/or any associated symptoms thereof, preventing, reducing, or reversing any physiological damage caused by diseases, as well as any pharmacological effect that is generally beneficial to the patient being treated. The compositions of the present application form a viable therapeutic agent without the need to achieve complete healing or eradication of any symptom or manifestation of a disease. As recognized in the relevant art, a drug used as a therapeutic agent may reduce the severity of a given disease state but need not to eliminate every manifestation of the disease to be considered as a useful therapeutic agent. Similarly, a treatment of prophylactic administration constitutes a viable prophylactic agent without the need to completely and effectively prevent the onset of a condition. It is sufficient to merely reduce the impact of the disease in a subject (e.g., by reducing the number or severity of symptoms, or by enhancing the efficacy of another therapy, or by achieving another beneficial effect), or to reduce the likelihood of disease occurrence or progression.

In the present application, the term "administration" generally refers to the delivery of a protein (including an immunoglobulin) to a human or an animal in need thereof by any route known in the art. Pharmaceutical carriers and formulations or compositions are also well known in the art. Routes of administration may include: intravenous, intramuscular, intradermal, subcutaneous, transdermal, mucosal, intratumoral, or mucosal administrations.

In the present application, the term "contact" generally refers to bringing two or more different types of substances into contact with each other in any order, in any manner, and for any duration. When applied to cells, the term "contact" generally refers to a method by which the antibody or antigen-binding fragment thereof, drug, linker-payload, conjugate, and/or the pharmaceutical composition of the present application is delivered to or placed in direct proximity to a target cell. The delivery may be *in vitro* or *in vivo* and may involve the use of recombinant vector systems. "Contact" may occur *in vivo, ex vivo,* or *in vitro.*

In the present application, the term "effective amount" or "effective dose" generally refers to an amount sufficient to achieve or at least partially achieve the desired effect. A "therapeutically effective amount" or "therapeutically effective dose" of a drug or therapeutic agent is generally any amount of the drug which, when used alone or in combination with another therapeutic agent, facilitates the regression of diseases (as evidenced by reduction in the severity of disease symptoms, increased frequency and duration of disease-free periods, or prevention of an injury or disability caused by the disease). A "prophylactically effective amount" or "prophylactically effective dose" of a drug generally refers to an amount of the drug which, when administered alone or in combination with another therapeutic agent to a subject at risk of disease development or recurrence, inhibits the development or recurrence of the disease. The ability of a therapeutic or prophylactic agent to promote regression of diseases or inhibit development or recurrence of diseases may be assessed by various methods known to those skilled in the art. For example, the agent efficacy against human is predicted in human subjects during clinical trials or in animal model systems, or the activity of the agent is determined via *in vitro* assays.

In the present application, the term "tumor" generally refers to all neoplastic cell growth and proliferation, whether malignant or benign, and all pre-cancerous and cancerous cells and tissues. The terms "cancer", "carcinoma", "cell proliferative disorder", "proliferative disorder" and "tumor" as used herein are not mutually exclusive. In the present application, tumors may be solid or non-solid tumors.

In the present application, the term "subject" generally refers to a human or non-human animal (including mammals) in need of diagnosis, prognosis, amelioration, prevention, and/or treatment of a disease, such as human, non-human primates (apes, gibbons, gorillas, chimpanzees, orangutans, macaques), domestic animals (dogs and cats), farm animals (poultry such as chicken and ducks, horses, cattle, goats, sheep, and pigs), and laboratory animals (mice, rats, rabbits, guinea pigs). Human subjects include fetal, neonates, infant, juvenile, and adult subjects. Subjects may include animal disease models.

In the present application, the terms "comprise", "include", "have", "may", "contain", and variants thereof are generally intended to be open-ended transitional phrases, terms, or words that do not exclude the possibility of additional behaviors or structures. The term "consisting of... " generally indicates that no other components (or likewise, features, integers, steps, etc.) are present. Unless expressly stated otherwise, the singular forms such as "a", "an", and "the" in English, and "a", "an", and "said/the" in Chinese, generally include the plural forms of referents as well. Similarly, unless expressly stated otherwise, plural terms herein cover singular referents. Unless expressly stated otherwise, the word "or" herein is intended to include "and".

In the present application, the terms "host cell", "host cell line", and "host cell culture" are used interchangeably and refer to a cell into which exogenous nucleic acid has been introduced, as well as progenies of such cells. Host cells include "transformants" (or "transformed cells") and "transfectants" (or "transfected cells"), which all include both the originally transformed or transfected cells and progenies derived therefrom. Such progenies may not be identical in nucleic acid content to the parent cell and may contain mutations. Recombinant host cells include transfectomas, such as CHO cells, HEK293 cells, NS/0 cells, and lymphocytes. The term "transfectoma", as used herein, includes recombinant eukaryotic host cells expressing antibodies, such as CHO cells, NS/0 cells, HEK293 cells, plant cells, or fungi (including yeast cells).

In the present application, the term "vector" refers to a nucleic acid molecule capable of propagating another nucleic acid linked thereto. The term includes vectors as self-replicating nucleic acid structures, as well as vectors that are introduced into the genome of a host cell. Certain vectors are capable of directing the expression of nucleic acids operably linked thereto.

In the present application, the term "inhibit" or "inhibition" means reducing a measurable extent, and may include but without need for complete prevention or inhibition. As used herein, the terms "inhibit growth" and "inhibit proliferation" (for example, referring to cells, e.g., tumor cells) are intended to include any measurable reduction in cell growth, when contacted with an anti-TF antibody-drug conjugate, as compared to the growth of the same cells that are not in contact with the anti-target cell antibody-drug conjugate, such as at least about 10%, 20%, 30%, 40%, 50%, 60%, 70%, 80%, 90%, 99%, or 100% inhibition of cell growth in a cell culture. Such reduction in cell growth may occur via various mechanisms. These mechanisms may be exerted by the anti-target cell antibody and the drug alone or in combination. For example, antibody-dependent cell-mediated phagocytosis (ADCP), antibody-dependent cell-mediated cytotoxicity (ADCC), complement-dependent cytotoxicity (CDC), and/or cell inhibition or cell apoptosis or cell death caused by the cytotoxic drug.

In the present application, the term "bystander killing" or "bystander effect" refers to the killing of target-negative cells in the presence of target-positive cells, wherein the killing of target-negative cells is not observed in the absence of target-positive cells. Cell-cell contact or at least proximity between target-positive and target-negative cells may enable bystander killing. Such killing may be distinguished from "off-target killing". The off-target killing refers to indiscriminate killing of target-negative cells. "Off-target killing" may be observed in the absence of target-positive cells.

In the present application, the term "target cell" refers to a cell that expresses an antigen on its surface. The terms "target-negative" or "target antigen-negative" refer to cells or tissues lacking expression of the target antigen. The term "target-positive" or "target antigen-positive" refer to the presence of target antigen expression. For example, cells or cell lines without expressing the target antigen may be described as the target-negative, whereas cells or cell lines that express the target antigen may be described as the target-positive.

In the present application, the term "internalization" refers to the situation that an antibody or antigen-binding fragment, when bound to a cell, is able to traverse the cell lipid bilayer membrane into an internal compartment (i.e., "internalized"), preferably into a degradative compartment within the cell. For example, an internalized anti-TF antibody is an antibody that, after being bound to TF on the cell membrane, is capable of entering the cell.

In the present application, the terms "tissue factor", "TF", "CD142", "tissue factor antigen", "TF antigen", and "CD142 antigen" are used interchangeably herein and, unless otherwise indicated, include any variant, isoform, and species homologue of a human tissue factor naturally expressed by cells or expressed on cells transfected with tissue factor genes. The "anti-TF antibody" refers to an antibody as described above that specifically binds to an antigen tissue factor or tissue factor antigen. For example: Tisotumab, and anti-TF antibodies in patents CN111818943A, CN106938051B, CN110575547A, CN103119065A, WO2011157741, WO 2010066803, and US 9168314 B2. In some embodiments, the anti-TF antibody or the antigen-binding fragment thereof is selected from the murine chimeric antibody 1849 with anticoagulant effect in US9920133B2 or the murine chimeric antibody 1859 without anticoagulant effect in US9920133B2.

In the present application, the term "GPC3" refers to any naturally mature GPC3 produced by the processing of the GPC3 precursor protein in a cell. The term includes GPC3 from any vertebrate, including mammals such as primates (e.g., humans and *Cynomolgus macaques)* and rodents (e.g., mice and rats), unless otherwise indicated. The term also includes naturally occurring GPC3 variants, such as splice variants or allelic variants. Exemplary heavy chain and light chain amino acid sequences of the human GPC3 monoclonal antibody are set forth in SEQ ID No: 50 and SEQ ID No: 66 of the patent US10782300B2.

In the present application, the term "Her2", "human epidermal growth factor receptor 2", or "Her2/neu" refers to any natural form of human Her2. The term encompasses full-length Her2, as well as any form of human Her2 produced by cellular processing (e.g., patent CN103319599). The term also encompasses naturally occurring Her2 variants, including but not limited to splice variants, allelic variants, and isoforms. Her2 may be isolated from human, or may be produced by recombinant methods or by synthetic methods.

In the present application, the phrase, a certain structure being "optionally linked to other molecular moieties" generally refers that the structure is not linked to any other chemical structures, or the structure is linked to (e.g., linked via a chemical bond or an adapter structure) one or more other chemical structures different from the structure (for example, the ligand as described herein).

The pharmaceutical composition may be in the form of a sterile injection water or oily suspension for intramuscular and subcutaneous administration. The suspension may be formulated by known technologies using the suitable dispersing agent or the wetting agent and the suspending agent as described above. A sterile injectable preparation may also be a sterile injectable solution or suspension prepared in a non-toxic parenterally-acceptable diluent or solvent, for example, a solution prepared in 1,3-butanediol. Moreover, sterile fixed oil may conveniently be used as a solvent or a suspending medium. For example, any blending fixed oil, including synthetic mono- or diglycerides, may be used. In addition, fatty acids, e.g., oleic acid may also be used for preparing injections.

In the present application, the term "comprise" generally means including the features specified explicitly, but does not exclude other elements. The terms "or more" and "or less" generally refer to cases including the recited number.

In the present application, the term "about" generally refers to a variation within 0.5% to 10% above or below the specified value, for example, within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the term "about" generally means being approximately, in the region of, roughly, or around. When the term "about" is used to refer to a numerical range, the cutoff or specific value is intended to indicate that the recited value may differ from the enumerated value by up to 10%. Accordingly, the term "about" may be used to encompass a variation of ±10% or less, ±5% or less, ±1% or less, ±0.5% or less, or ±0.1% or less from a particular value.

In the present application, the polypeptide, linker fragment, linker, or ADC "responsive to matrix metalloproteinase 2, matrix metalloproteinase 9, or cathepsin B" means that the enzyme may effectively degrade the polypeptide, linker fragment, linker, or ADC.

### DETAILED DESCRIPTION OF THE INVENTION

### The polypeptide or fragments thereof

In a first aspect, the present application provides a polypeptide P1 or a polypeptide fragment - P1-P2-, wherein the polypeptide P1 and the polypeptide P2 are each responsive to an enzyme.

In some embodiments, P1 comprises a polypeptide responsive to matrix metalloproteinase 2 (MMP-2) or matrix metalloproteinase 9 (MMP-9) or cathepsin B. P2 comprises a polypeptide responsive to cathepsin B or matrix metalloproteinase 2 (MMP-2) or matrix metalloproteinase 9 (MMP-9).

In some embodiments, in -P1-P2-, P1 comprises the following polypeptide structure: -X1-P-X2-X3-Z-, and P2 comprises the following polypeptide structure: -X1'-X2'-X3'-X4'-; wherein P is a proline residue, X1, X2, X3, Z, X1', X2', X3', and X4' each represent absence, a single amino acid, or an amino acid derivative.

In some embodiments, X1 represents absence, a single amino acid, or a single amino acid derivative; X2, X3, and Z each represent a single amino acid or a single amino acid derivative; X1' and X2' each represent a single amino acid or a single amino acid derivative; and X3' and X4' each represent absence or a single amino acid or a single amino acid derivative.

In some embodiments, X1 is selected from absence, Gly (G), Val (V), His (H), Ser (S), and Ile (I); X2 is selected from Ala (A), Ser (S), Leu (L), Arg (R), Gln (Q), Val (V), and Cit; X3 is selected from Gly (G), Ser (S), Pro (P), Asn (N), and Ala (A); and Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylornithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylornithine (Orn(Et2)), and N,N-diethyllysine (Lys(Et2)).

For example, the polypeptide P1 may be -SPRGL-, -GPRGL-, -PRGL-, -PRNL-, or - GPRNDap(Me2)-.

In some embodiments, X1' is selected from Val (V), Als (A), Leu (L), Ile (I), Met (M), Phe (F), Trp (W), Pro (P), Tyr (Y), Gly (G), and Glu (E); X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Orn(Et2); X3' is selected from absence, Phe (F), Gly (G), Lys (K), Lys(Me2), Lys(Et2), His (H), Ala (A), Arg (R), Cit, Orn, Orn(Me2), and Orn(Et2); X4' is selected from absence, Gly (G), and Phe (F).

In some embodiments, X1 is selected from absence, Gly (G), Val (V), His (H), Ser (S), and Ile (I); X2 is selected from Ala (A), Ser (S), Leu (L), Arg (R), Gln (Q), Val (V), and Cit; X3 is selected from Gly (G), Ser (S), Pro (P), Asn (N), and Ala (A); Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylornithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylornithine (Orn(Et2)), and N,N-diethyllysine (Lys(Et2)); X1' is selected from Val (V), Als (A), Leu (L), Ile (I), Met (M), Phe (F), Trp (W), Pro (P), Tyr (Y), Gly (G), and Glu (E); X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Orn(Et2); X3' is selected from absence, Phe (F), Gly (G), Lys (K), Lys(Me2), Lys(Et2), His (H), Ala (A), Arg (R), Cit, Orn, Orn(Me2), and Orn(Et2); and X4' is selected from absence, Gly (G), and Phe (F).

In some embodiments, P2 is -X1'-X2'-X3'-X4'-, which is further selected from the following fragments: -Val-Cit-, -Ala-Lys-, or -Gly-Gly-Phe-Gly-.

Wherein, the polypeptide or the polypeptide fragment -P1-P2- is selected from the following structures:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50); and
-Gly-Pro-Arg-Gly-Dap(Me2)-Val-Cit- (SEQ ID NO: 51);
or a variant having one or two mismatches relative to any sequence of SEQ ID NOs:1-51.

In the present application, the mismatch refers to any mismatching between two sequences, including deletion, addition, and substitution of amino acids. For example, the mismatch may be a conservative amino acid substitution. Those skilled in the art will recognize that conservative amino acid substitution refers to the replacement of one amino acid with another amino acid having a similar structure or chemical property (for example, a similar side chain). Exemplary conservative substitutions in the art are described, for example, in Watson et al., Molecular Biology of the Gene, The Benjamin/Cummings Publishing Company, 4th edition (1987).

In some embodiments, a polypeptide or polypeptide fragment -P1-P2- is selected from the following structures:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50); and
-Gly-Pro-Arg-Gly-Dap(Me2)-Val-Cit- (SEQ ID NO: 51);
or a variant having one or two mismatches relative to any sequence of SEQ ID NOs:1-51.

### Linker

**In a second aspect,** the present application provides a novel linker structure, and the linker comprises a -P1-P2- unit. The -P1-P2- unit is the polypeptide or the polypeptide fragment as described in the first aspect. The linker has a general formula of: C1-La-L1-P1-P2-L2:
wherein C1 is an adapter for connecting a bioligand unit;
La is absent or a tuning spacer;
L1 is absent or a spacer;
P1-P2 is the polypeptide or the polypeptide fragment as described in the first aspect;
L2 is absent or a spacer for connecting a payload with a specific biological function; and
positions of La and L1 are interchangeable.

In some embodiments, the bioligand unit comprises an antibody, antibody fragment, protein, polypeptide, polynucleotide, or chemical small molecule.

In some embodiments, the adapter C1 is selected from the following structures: 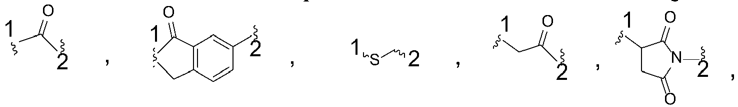 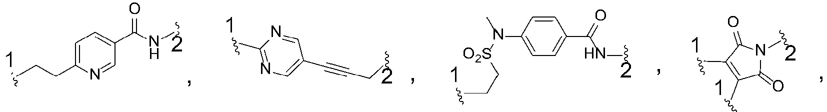 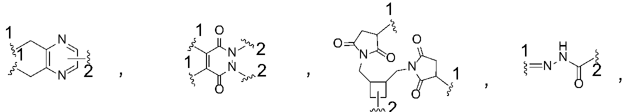 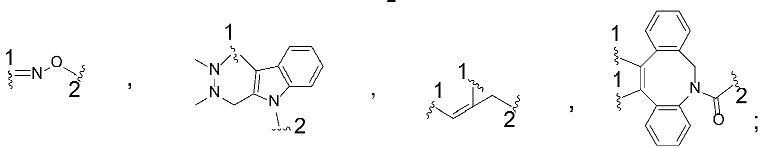 and 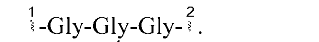 - 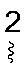 . wherein 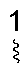 indicates a position for connection to the bioligand unit, and 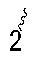 indicates a position for connection to La.

In some embodiments, C1 includes a structure of -(succinimid-3-yl-N)-, -CH₂-C(=O)-, or - C(=O)-.

In some embodiments, La may be absent.

In some embodiments, La is selected from the following structures or combinations thereof:
R1-S-S-R2,
R1-(CH₂CH₂O)ₘ-R2,
R1-(CH₂CH₂O)ₘ-CH₂CH₂S-(CH₂CH₂O)ₙ-R2,
R1-(CH₂CH₂O)ₘ-CH₂CH₂NH-(CH₂CH₂O)ₙ-R2,
R1-(CH₂CH₂O)ₘ-CH₂CH₂(C=O)NH-(CH₂CH₂O)ₙ-R2, 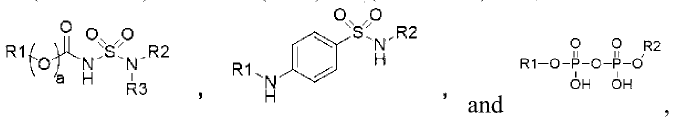
a is 0 or 1;
m is any integer from 1 to 20;
n is any integer from 1 to 20;
R3 is H, -C1-C10 alkylene-, -C3-C8 carbocyclyl-, -C2-C10 heteroaryl-, or -C3-C24 alkylheteroaryl-, wherein -C1-C10 alkylene-, -C3-C8 carbocyclyl-, -C2-C10 heteroaryl-, and -C3-C24 alkylheteroaryl- can be substituted by one or more heteroatoms, the heteroatoms can be O, S, or NR³, wherein R³ is H or -C1-C4 alkylene;
R1 is a structure for connection to C1, and R2 is a structure for connection to L1.

In some embodiments, R1 is a single bond or is selected from the following structures:
-X¹-C1-C10 alkylene-X²-, -X¹-C1-C10 heteroalkylene-X²-, -X¹-C3-C8 carbocyclyl-X²-, -X¹-arylene-X²-, -X¹-C1-C10 alkylene-arylene-X²-, -X¹-arylene-C1-C10 alkylene-X²-, -X¹-C1-C10 alkylene-(C3-C8 carbocyclyl)-X²-, -X¹-(C3-C8 carbocyclyl)-C1-C10 alkylene-X²-, -X¹-C3-C8 heterocyclyl-X²-, -X¹-C1-C10 alkylene-(C3-C8 heterocyclyl)-X²-, and -X¹-(C3-C8 heterocyclyl)-C1-C10 alkylene-X²-;
wherein X¹ is absent or selected from O, S, NH, C(=O), NHC(=O), C(=O)NH, and NHC(=O)NH, and X² is absent or selected from O, NH, S, C(=O), C(=O)NH, NHC(=O), and NHC(=O)NH;
when R1 is not a single bond, R1 is optionally substituted with a basic unit (BU), the basic unit is -(CH₂)ₓNH₂, -(CH₂)ₓNHRₘ, or -(CH₂)ₓN(Rₘ)₂; wherein x is any integer from 1 to 4; and each Rₘ is independently selected from C1-C6 alkyl and C1-C6 haloalkyl, or two Rₘ groups, in combination with the nitrogen to which they are attached, form a 4- to 6-membered heterocycloalkyl ring, or an azetidinyl, pyrrolidinyl, or piperidinyl group.

In some embodiments, R2 is a single bond or is selected from the following structures:
-X³-C1-C10 alkylene-X⁴-, -X³-C1-C10 heteroalkylene-X⁴-, -X³-C3-C8 carbocyclyl-X⁴-, -X³-arylene-X⁴-, -X³-C1-C10 alkylene-arylene-X⁴-, -X³-arylene-C1-C10 alkylene-X⁴-, -X³-C1-C10 alkylene-(C3-C8 carbocyclyl)-X⁴-, -X³-(C3-C8 carbocyclyl)-C1-C10 alkylene-X⁴-, -X³-C3-C8 heterocyclyl-X⁴-, -X³-C1-C10 alkylene-(C3-C8 heterocyclyl)-X⁴-, and -X³-(C3-C8 heterocyclyl)-C1-C10 alkylene-X⁴-;
wherein X³ is absent or O, S, NH, C(=O), NHC(=O), C(=O)NH, or NHC(=O)NH, and X ⁴ is absent or selected from O, NH, S, C(=O), C(=O)NH, NHC(=O), and NHC(=O)NH;
when R2 is not a single bond, R2 is optionally substituted with a basic unit (BU), the basic unit is -(CH₂)ₓNH₂, -(CH₂)ₓNHRₘ, or -(CH₂)ₓN(Rₘ)₂; wherein x is any integer from 1 to 4; and each Rₘ is independently selected from C1-C6 alkyl and C1-C6 haloalkyl, or two Rₘ groups, in combination with the nitrogen to which they are attached, form a 4- to 6-membered heterocycloalkyl ring, or an azetidinyl, pyrrolidinyl, or piperidinyl group.

For example, La may be the following structure: -CH₂CH₂C(O)NH-, -OC(O)-, or -OC(O)-(CH₂CH₂O)₄-.

In certain embodiments, L1 is selected from the following structures:
-X⁵-C1-C10 alkylene-X⁶-, -X⁵-C1-C10 heteroalkylene-X⁶-, -X⁵-C3-C8 carbocyclyl-X⁶-, -X⁵-arylene-X⁶-, -X⁵-heteroarylene-X⁶-, -X⁵-C1-C10 alkylene-arylene-X⁶-, -X⁵-C1-C10 alkylene-heteroarylene-X⁶-, -X⁵-arylene-C1-C10 alkylene-X⁶-, -X⁵-heteroarylene-C1-C10 alkylene-X⁶-, -X⁵-C1-C10 alkylene-(C3-C8 carbocyclyl)-X⁶-, -X⁵-(C3-C8 carbocyclyl)-C1-C10 alkylene-X⁶-, -X⁵-C3-C8 heterocyclyl-X⁶-, -X⁵-C1-C10 alkylene-(C3-C8 heterocyclyl)-X⁶-, -X⁵-(C3-C8 heterocyclyl)-C1-C10 alkylene-X⁶-, -X⁵-C3-C8 heterocyclyl-arylene-X⁶-, -X⁵-arylene-C3-C8 heterocyclyl-X⁶-, -X⁵-C3-C8 heterocyclyl-heteroarylene-X⁶-, and -X⁵-heteroarylene-C3-C8 heterocyclyl-X⁶-;
wherein X⁵ is absent or is selected from O, S, NH, C(=O), NHC(=O), C(=O)NH, and NHC(=O)NH, and X⁶ is absent or is selected from O, S, NH, C(=O), NHC(=O), C(=O)NH, and NHC(=O)NH; and
where any methylene unit of L1 can be independently substituted with -O-, -S-, -N(Rₙ)-, - CH(N(Rₙ)₂)-, -CH(P(Rₙ)₂)-, -C(N(Rₙ)₂)₂-, -C(ORₙ)(N(Rₙ)₂)-, -C(=O)-, -OC(=O)-, -C(=O)O-, - N(Rₙ)C(=O)-, -C(=O)N(Rₙ)-, -SO-, -SO₂-, -N(Rₙ)SO₂-, -SO₂N(Rₙ)-, -P(Rₙ)-, -P(=O)(Rₙ)-, - P(=O)(O)-, -C(=S)-, -C(=N-Rₙ)-, -S-S-, -N=N-, -N(Rₙ)-N=, =N-N(Rₙ)-, -C=N-, or -N=C-; each Rₙ is independently selected from H, C1-C6 alkyl, C3-C8 carbocyclyl, and C1-C6 haloalkyl, or two Rₙ groups, in combination with the nitrogen or phosphorus to which they are attached, form a 4- to 6-membered heterocycloalkyl ring, or an azetidinyl, pyrrolidinyl, or piperidinyl group.

In some embodiments, L2 is absent.

In some embodiments, L2 is selected from the following structures: 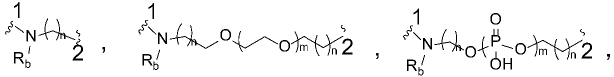 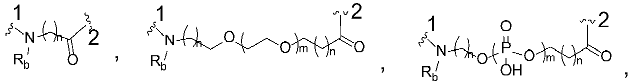 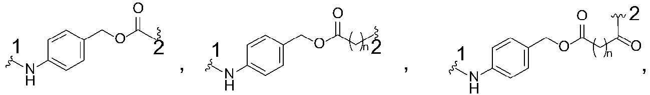 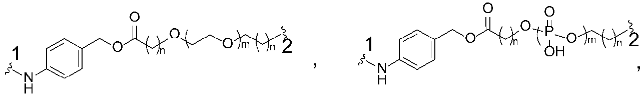 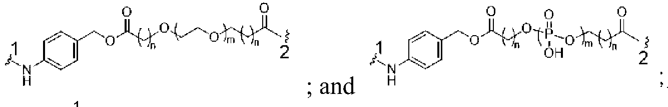
wherein 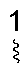 indicates a position for connection to the polypeptide or polypeptide fragment -P1-P2-, and 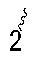 indicates a position for connection to a payload with specific biological function;
m is any integer from 1 to 20;
n is any integer from 1 to 6;
R_{b} is independently selected from H, C1-C6 alkyl, C3-C8 carbocyclyl, C1-C6 haloalkyl, C3-C8 heterocyclyl, C1-C6 alkyl-C3-C8 heterocyclyl, substitutable aryl, substitutable heteroaryl, C1-C6 alkyl-substitutable aryl, and C1-C6 alkyl-substitutable heteroaryl; and
wherein any methylene unit of L2 can independently be substituted with -CHX-, -C(X₂)-, -C3-C8 carbocyclyl-, -C3-C8 heterocyclyl-, -arylene-, -heteroarylene-, -O-, -S-, -N(Rₙ)-, -CH(N(Rₙ)₂)-, - CH(P(Rₙ)₂)-, -C(N(Rₙ)₂)₂-, -C(ORₙ)(N(Rₙ)₂)-, -C(=O)-, -OC(=O)-, -C(=O)O-, -N(Rₙ)C(=O)-, - C(=O)N(Rₙ)-, -SO-, -SO₂-, -N(Rₙ)SO₂-, -SO₂N(Rₙ)-, -P(Rₙ)-, -P(=O)(Rₙ)-, -P(=O)(O)-, -C(=S)-, - C(=N-Rₙ)-, -S-S-, -N=N-, -N(Rₙ)-N=, =N-N(Rₙ)-, -C=N-, or -N=C-; wherein X represents halogen or deuterium, and each Rₙ is independently selected from H, C1-C6 alkyl, C3-C8 cycloalkyl, and C1-C6 haloalkyl, or two Rₙ groups, in combination with the nitrogen or phosphorus to which they are attached, form a 4- to 6-membered heterocycloalkyl ring, or an azetidinyl, pyrrolidinyl, or piperidinyl group.

In some embodiments, L2 may be absent.

In some embodiments, L2 is selected from the following structures: 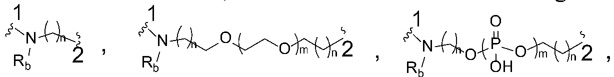 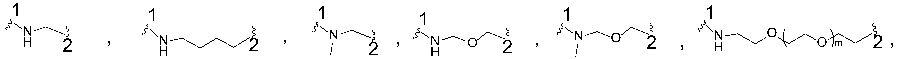 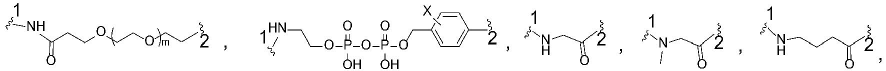 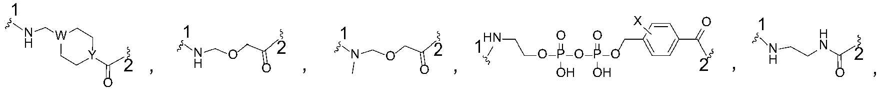 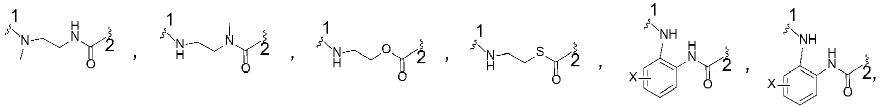 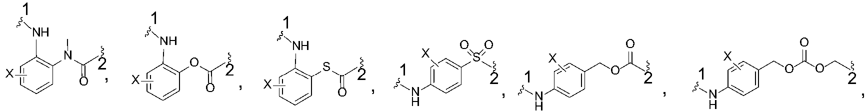 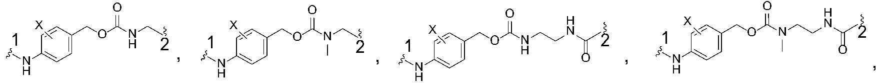 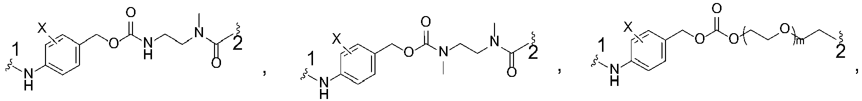 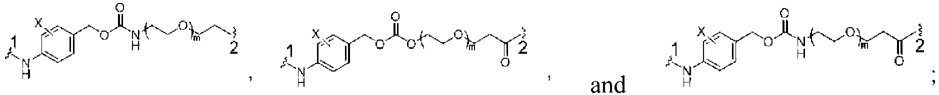 wherein 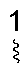 indicates a position for connection to the polypeptide or polypeptide fragment -P1-P2-, and 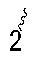 indicates a position for connection to a payload with specific biological function;
W and Y are each independently N or CR^{Y}, and each R^{Y} is independently H or C1-C10 alkyl;
m is any integer from 0 to 20; and
X is halogen or deuterium.

In some embodiments, L2 may be:

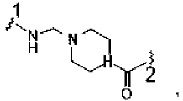

In some embodiments, the linker is selected from the following structures:
-(succinimid-3-yl-N)-CH2-C(=O)-P1-P2-;
-(succinimid-3-yl-N)-CH2CH2-C(=O)-P1-P2-;
-(succinimid-3-yl-N)-(CH2CH2O)₄-CH2CH2-C(=O)-P1-P2-;
-(succinimid-3-yl-N)-(CH2CH2O)₈-CH2CH2-C(=O)-P1-P2-;
-(succinimid-3-yl-N)-CH2CH2C(O)NH-(CH2CH2O)₄-CH2CH2-C(=O)-P1-P2-;
-(succinimid-3-yl-N)-CH2CH2C(O)NH-(CH2CH2O)₈-CH2CH2-C(=O)-P1-P2-;
-(succinimid-3-yl-N)-OC(O)-(CH2CH2O)₄-CH2CH2-C(=O)-P1-P2-;
-(succinimid-3-yl-N)-OC(O)-(CH2CH2O)₈-CH2CH2-C(=O)-P1-P2-;
-(succinimid-3-yl-N)-CH2CH2CH2CH2CH2-C(=O)-P1-P2-;
-(succinimid-3-yl-N)-CH2-C(=O)-P1-P2-PAB-;
-(succinimid-3-yl-N)-CH2CH2-C(=O)-P1-P2-PAB-;
-(succinimid-3-yl-N)-(CH2CH2O)₄-CH2CH2-C(=O)-P1-P2-PAB-;
-(succinimid-3-yl-N)-(CH2CH2O)₈-CH2CH2-C(=O)-P1-P2-PAB-;
-(succinimid-3-yl-N)-CH2CH2C(O)NH-(CH2CH2O)₄-CH2CH2-C(=O)-P1-P2-PAB-;
-(succinimid-3-yl-N)-CH2CH2C(O)NH-(CH2CH2O)₈-CH2CH2-C(=O)-P1-P2-PAB-;
-(succinimid-3-yl-N)-OC(O)-(CH2CH2O)₄-CH2CH2-C(=O)-P1-P2-PAB-;
-(succinimid-3-yl-N)-OC(O)-(CH2CH2O)₈-CH2CH2-C(=O)-P1-P2-PAB-;
-(succinimid-3-yl-N)-CH2CH2CH2CH2CH2-C(=O)-P1-P2-PAB-; 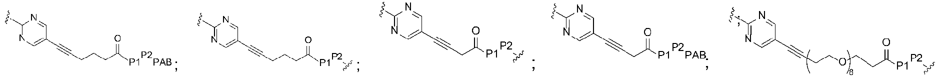 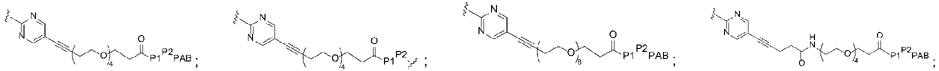 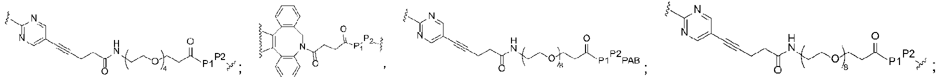 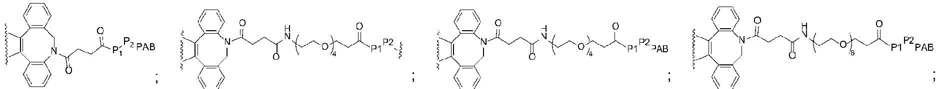 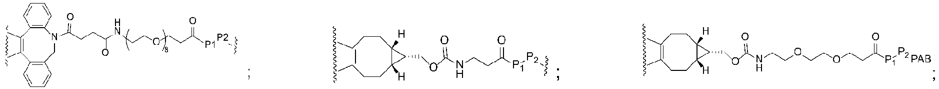 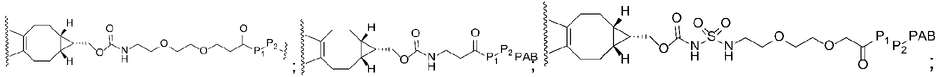 and 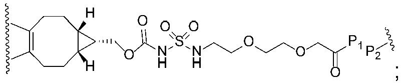
wherein 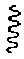 indicates a position for connection to a bioligand unit;
PAB represents the following structure: 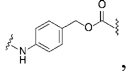
-P1-P2- represents the following structure:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50); or
-Gly-Pro-Arg-Gly-Dap(Me2)-Val-Cit- (SEQ ID NO: 51);
or, a variant having one or two mismatches relative to any sequence of SEQ ID NOs:1-51.

In some embodiments, the linker may be selected from:
-(succinimid-3-yl-N)-CH2CH2CH2CH2CH2-C(=O)-GPLGLGGFG-;
-(succinimid-3-yl-N)-CH2CH2CH2CH2CH2-C(=O)-GPRGLGGFG-;
-(succinimid-3-yl-N)-CH2CH2CH2CH2CH2-C(=O)-GPAALGGFG-;
-(succinimid-3-yl-N)-CH2CH2CH2CH2CH2-C(=O)-GPAGLVCit-PAB-;
-(succinimid-3-yl-N)-CH2CH2CH2CH2CH2-C(=O)-GPASLVCit-PAB-;
-(succinimid-3-yl-N)-CH2CH2CH2CH2CH2-C(=O)-GPRGLVCit-PAB-;
-(succinimid-3-yl-N)-CH2CH2CH2CH2CH2-C(=O)-GPSALVCit-PAB-;
-(succinimid-3-yl-N)-CH2CH2CH2CH2CH2-C(=O)-PRNLVCit-PAB-;
-(succinimid-3-yl-N)-CH2CH2CH2CH2CH2-C(=O)-GPRNLVCit-PAB-;
-(succinimid-3-yl-N)-CH2CH2CH2CH2CH2-C(=O)-GPRGDap(Me2)VCit-PAB-;
-(succinimid-3-yl-N)-CH2CH2CH2CH2CH2-C(=O)-SPRNLVCit-PAB-; and
-(succinimid-3-yl-N)-CH2CH2CH2CH2CH2-C(=O)-GPLSLVCit-PAB-;
wherein,
PAB represents a structure of

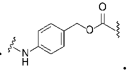

### Linker-payload

In a third aspect, the present application provides a linker-payload comprising the linker as described in the second aspect.

In some embodiments, the linker-payload has a structure of:
C1'-La-L1-P1-P2-L2-D
wherein C1' is a functional group that reacts with a bioligand unit;
La is absent or a tuning spacer;
L1 is absent or a spacer;
P1-P2 is the polypeptide or the polypeptide fragment as described in the first aspect;
L2 is absent or a spacer for connecting a payload with a specific biological function;
D is a drug unit; and
positions of La and L1 are interchangeable.

It should be noted that the bioligand comprises antibodies, antibody fragments, proteins, polypeptides, polynucleotides, and chemical small molecules. The functional group C1' may react with a functional group on the bioligand, thereby connecting the linker-payload to the bioligand. For example, when C1' is 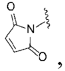 it may react with the side chain -SH of Cys of a monoclonal antibody after being reduced by a reducing agent, thereby connecting the linker-payload to the antibody.

In some embodiments, C1' is selected from functional groups that react with amino, sulfhydryl, keto, azido, or alkynyl groups.

In some embodiments, C1' is selected from enzyme-catalyzed reaction substrates. For example, Gly-Gly-Gly- may be recognized by a Sortase A enzyme.

In some embodiments, C1' is selected from the following structures: 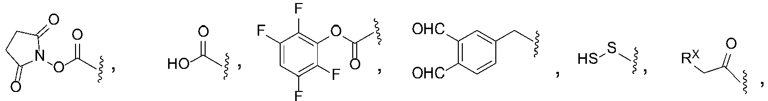 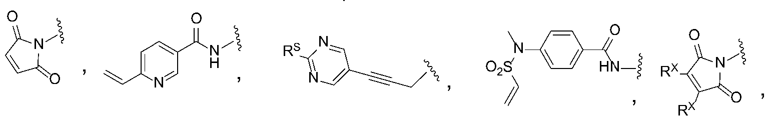 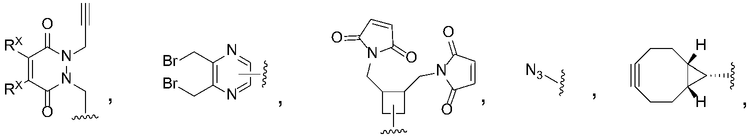 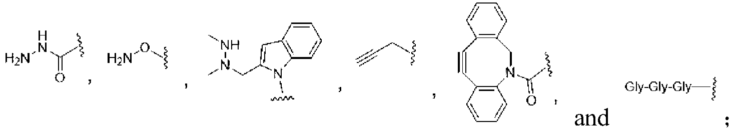 wherein,
R^{X} is selected from halogen and -SPh, R^{S} is selected from halogen, sulfonyl, a tertiary-amine salt, diazonium salt group, -OMs, MeSO₂-, and CF₃SO₃-;
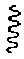 indicates a position for connection to La.

As disclosed herein, the drug unit refers to the moiety (fragment or group) well known in the art, which is capable of forming a bio-active drug (e.g., a small-molecule cytotoxic drug, including the group formed after loss of one atom or atomic group) or derivatives thereof (e.g., its precursor) after linker cleavage/degradation/enzymatic hydrolysis in tumor tissues or within tumor cells, in an antibody-drug conjugate (also referred to as antibody-drug conjugate, ADC). To avoid ambiguity, "drug" does not refer solely to "pharmaceuticals" approved by pharmaceutical regulatory authorities, but also includes any molecule with potential therapeutic bio-activity in clinical use, research and development, or academic studies.

In some embodiments, the drug unit D is selected from immunomodulators, protein degrading agents, and cytotoxic agents.

In some embodiments, the drug unit D is selected from cytotoxic agents.

In some embodiments, the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4]benzodi-azepines (PBDs), puromycins, rhizoxins, SN-38, taxanes, tubulysins, vincaalkaloids, tetrahydroisoquinoline alkaloids or derivatives thereof, protein degrading agents or derivatives thereof.

In some embodiments, the drug unit comprises a DNA topoisomerase I inhibitor, a tubulin inhibitor, a DNA replication inhibitor, or a protein degrading agent.

In some embodiments, the drug unit comprises a maytansinoid or derivatives thereof, a camptothecin or derivatives thereof, an auristatin or derivatives thereof, a tetrahydroisoquinoline alkaloid or derivatives thereof, a BTK protein degrading agent and derivatives thereof, or a GSPT1 protein degrading agent and derivatives thereof.

In some embodiments, the drug unit comprises DM1, DM4, DX8951, MMAE, Dxd, SN38, Trabectedin (ET743), Lurbinectedin, CC885, or CC-90009.

In some embodiments, the linker-payload has the following structure:
(maleimid-N-yl)-CH2-C(=O)-P1-P2-D;
(maleimid-N-yl)-CH2CH2-C(=O)-P1-P2-D;
(maleimid-N-yl)-(CH2CH2O)₄-CH2CH2-C(=O)-P1-P2-D;
(maleimid-N-yl)-(CH2CH2O)₈-CH2CH2-C(=O)-P1-P2-D;
(maleimid-N-yl)-CH2CH2C(O)NH-(CH2CH2O)₄-CH2CH2-C(=O)-P1-P2-D;
(maleimid-N-yl)-CH2CH2C(O)NH-(CH2CH2O)₈-CH2CH2-C(=O)-P1-P2-D;
(maleimid-N-yl)-OC(O)-(CH2CH2O)₄-CH2CH2-C(=O)-P1-P2-D;
(maleimid-N-yl)-OC(O)-(CH2CH2O)₈-CH2CH2-C(=O)-P1-P2-D;
(maleimid-N-yl)-CH2CH2CH2CH2CH2-C(=O)-P1-P2-D;
(maleimid-N-yl)-CH2-C(=O)-P1-P2-PAB-D;
(maleimid-N-yl)-CH2CH2-C(=O)-P1-P2-PAB-D;
(maleimid-N-yl)-(CH2CH2O)₄-CH2CH2-C(= O)-P1-P2-PAB-D;
(maleimid-N-yl)-(CH2CH2O)₈-CH2CH2-C(=O)-P1-P2-PAB-D;
(maleimid-N-yl)-CH2CH2C(O)NH-(CH2CH2O)₄-CH2CH2-C(=O)-P1-P2-PAB-D;
(maleimid-N-yl)-CH2CH2C(O)NH-(CH2CH2O)₈-CH2CH2-C(=O)-P1-P2-PAB-D;
(maleimid-N-yl)-OC(O)-(CH2CH2O)₄-CH2CH2-C(=O)-P1-P2-PAB-D;
(maleimid-N-yl)-OC(O)-(CH2CH2O)₈-CH2CH2-C(=O)-P1-P2-PAB-D;
(maleimid-N-yl)-CH2CH2CH2CH2CH2-C(=O)-P1-P2-PAB-D;
(N-hydroxysuccinimide ester-1-yl)-CH2-C(=O)-P1-P2-D;
(N-hydroxysuccinimide ester-1-yl)-CH2CH2-C(=O)-P1-P2-D;
(N-hydroxysuccinimide ester-1-yl)-(CH2CH2O)₄-CH2CH2-C(=O)-P1-P2-D;
(N-hydroxysuccinimide ester-1-yl)-(CH2CH2O)₈-CH2CH2-C(=O)-P1-P2-D;
(N-hydroxysuccinimide ester-1-yl)-CH2CH2C(O)NH-(CH2CH2O)₄-CH2CH2-C(=O)-P1-P2-D;
(N-hydroxysuccinimide ester-1-yl)-CH2CH2C(O)NH-(CH2CH2O)₈-CH2CH2-C(=O)-P1-P2-D;
(N-hydroxysuccinimide ester-1-yl)-(1,4-cyclohexyl)-CH2-C(=O)-P1-P2-D;
(N-hydroxysuccinimide ester-1-yl)-(1,4-cyclohexyl)-CH2NH-(CH2CH2O)₄-CH2CH2-C(=O)-P1-P2-D;
(N-hydroxysuccinimide ester-1-yl)-(1,4-cyclohexyl)-CH2NH-(CH2CH2O)₈-CH2CH2-C(=O)-P1-P2-D;
(N-hydroxysuccinimide ester-1-yl)-(1,4-cyclohexyl)-CH2C(=O)NH-(CH2CH2O)₄-CH2CH2-C(=O)-P1-P2-D;
(N-hydroxysuccinimide ester-1-yl)-(1,4-cyclohexyl)-CH2C(=O)NH-(CH2CH2O)₈-CH2CH2-C(=O)-P1-P2-D;
(N-hydroxysuccinimide ester-1-yl)-(1,4-cyclohexyl)-CH2O-(CH2CH2O)₄-CH2CH2-C(=O)-P1-P2-D;
(N-hydroxysuccinimide ester-1-yl)-(1,4-cyclohexyl)-CH2O-(CH2CH2O)₈-CH2CH2-C(=O)-P1-P2-D;
(N-hydroxysuccinimide ester-1-yl)-CH2CH2CH2CH2CH2-C(=O)-P1-P2-D;
(N-hydroxysuccinimide ester-1-yl)-CH2-C(=O)-P1-P2-PAB-D;
(N-hydroxysuccinimide ester-1-yl)-CH2CH2-C(=O)-P1-P2-PAB-D;
(N-hydroxysuccinimide ester-1-yl)-(CH2CH2O)₄-CH2CH2-C(=O)-P1-P2-PAB-D;
(N-hydroxysuccinimide ester-1-yl)-(CH2CH2O)₈-CH2CH2-C(=O)-P1-P2-PAB-D;
(N-hydroxysuccinimide ester-1-yl)-CH2CH2C(O)NH-(CH2CH20)₄-CH2CH2-C(=O)-P1-P2-PAB-D;
(N-hydroxysuccinimide ester-1-yl)-CH2CH2C(O)NH-(CH2CH2O)₈-CH2CH2-C(=O)-P1-P2-PAB-D;
(N-hydroxysuccinimide ester-1-yl)-(1,4-cyclohexyl)-CH2-C(=O)-P1-P2-PAB-D;
(N-hydroxysuccinimide ester-1-yl)-(1,4-cyclohexyl)-CH2NH-(CH2CH2O)₄-CH2CH2-C(=O)-P1-P2-PAB-D;
(N-hydroxysuccinimide ester-1-yl)-(1,4-cyclohexyl)-CH2NH-(CH2CH2O)₈-CH2CH2-C(=O)-P1-P2-PAB-D;
(N-hydroxysuccinimide ester-1-yl)-(1,4-cyclohexyl)-CH2C(=O)NH-(CH2CH2O)₄-CH2CH2-C(=O)-P1-P2-PAB-D;
(N-hydroxysuccinimide ester-1-yl)-(1,4-cyclohexyl)-CH2C(=O)NH-(CH2CH2O)₈-CH2CH2-C(=O)-P1-P2-PAB-D;
(N-hydroxysuccinimide ester-1-yl)-(1,4-cyclohexyl)-CH2O-(CH2CH2O)₄-CH2CH2-C(=O)-P1-P2-PAB-D;
(N-hydroxysuccinimide ester-1-yl)-(1,4-cyclohexyl)-CH2O-(CH2CH2O)₈-CH2CH2-C(=O)-P1-P2-PAB-D;
(N-hydroxysuccinimide ester-1-yl)-CH2CH2CH2CH2CH2-C(=O)-P1-P2-PAB-D;
Gly-Gly-Gly-NH-CH2CH2CH2CH2CH2-C(= O)-P1-P2-D;
Gly-Gly-Gly-NH-CH2CH2CH2CH2CH2-C(=O)-P1-P2-PAB-D; 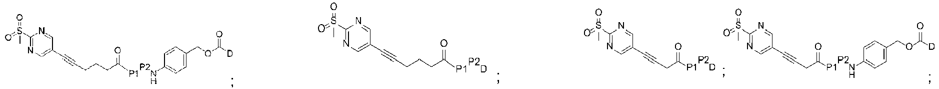 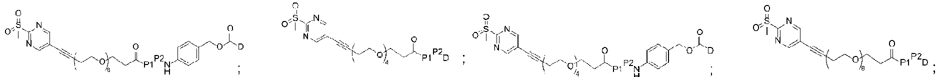 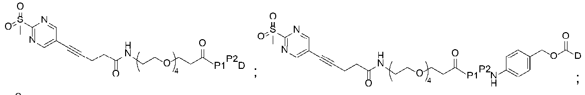 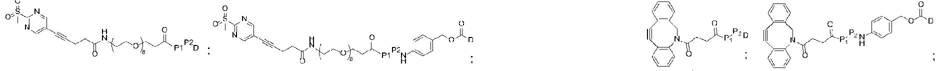 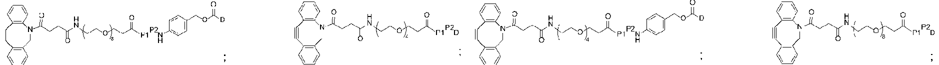 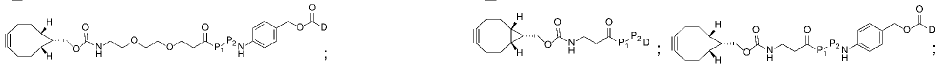 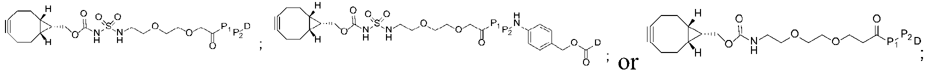
(N-hydroxysuccinimide ester-1-yl)- has a structure of 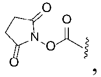
wherein 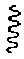 indicates a position for connection to the linker;
-P1-P2- represents the following structure:
   -His-Val-Leu-Asn-Leu-Val-Cit- (SEQ ID NO: 1);
   -Val-Pro-Leu-Ser-Leu-Val-Cit- (SEQ ID NO: 2);
   -Ile-Pro-Val-Ser-Leu-Val-Cit- (SEQ ID NO: 3);
   -Gly-Pro-Leu-Ser-Leu-Val-Cit- (SEQ ID NO: 4);
   -Gly-Pro-Ala-Ser-Leu-Val-Cit- (SEQ ID NO: 5);
   -Gly-Pro-Ala-Gly-Leu-Val-Cit- (SEQ ID NO: 6);
   -Gly-Pro-Gln-Gly-Leu-Val-Cit- (SEQ ID NO: 7);
   -Gly-Pro-Leu-Gly-Leu-Val-Cit- (SEQ ID NO: 8);
   -Gly-Pro-Ser-Gly-Leu-Val-Cit- (SEQ ID NO: 9);
   -Gly-Pro-Arg-Gly-Leu-Val-Cit- (SEQ ID NO: 10);
   -Gly-Pro-Ala-Ala-Leu-Val-Cit- (SEQ ID NO: 11);
   -Gly-Pro-Ser-Ala-Leu-Val-Cit- (SEQ ID NO: 12);
   -His-Val-Leu-Asn-Leu-Gly-Gly-Phe-Gly- (SEQ ID NO: 13);
   -Val-Pro-Leu-Ser-Leu-Gly-Gly-Phe-Gly- (SEQ ID NO: 14);
   - Ile-Pro-Val-Ser-Leu-Gly-Gly-Phe-Gly- (SEQ ID NO: 15);
   -Gly-Pro-Leu-Ser-Leu-Gly-Gly-Phe-Gly- (SEQ ID NO: 16);
   -Gly-Pro-Ala-Ser-Leu-Gly-Gly-Phe-Gly- (SEQ ID NO: 17);
   -Gly-Pro-Ala-Gly-Leu-Gly-Gly-Phe-Gly- (SEQ ID NO: 18);
   -Gly-Pro-Gln-Gly-Leu-Gly-Gly-Phe-Gly- (SEQ ID NO: 19);
   -Gly-Pro-Leu-Gly-Leu-Gly-Gly-Phe-Gly- (SEQ ID NO: 20);
   -Gly-Pro-Ser-Gly-Leu-Gly-Gly-Phe-Gly- (SEQ ID NO: 21);
   -Gly-Pro-Arg-Gly-Leu-Gly-Gly-Phe-Gly- (SEQ ID NO: 22);
   -Gly-Pro-Ala-Ala-Leu-Gly-Gly-Phe-Gly- (SEQ ID NO: 23);
   -Gly-Pro-Ser-Ala-Leu-Gly-Gly-Phe-Gly- (SEQ ID NO: 24);
   -Pro-Arg-Gly-Leu-Val-Cit- (SEQ ID NO: 25);
   -Pro-Arg-Ser-Leu-Val-Cit- (SEQ ID NO: 26);
   -Pro-Arg-Asn-Leu-Val-Cit- (SEQ ID NO: 27);
   -Pro-Arg-Pro-Leu-Val-Cit- (SEQ ID NO: 28);
   -Pro-Cit-Gly-Leu-Val-Cit- (SEQ ID NO: 29);
   -Pro-Cit-Pro-Leu-Val-Cit- (SEQ ID NO: 30);
   -Gly-Pro-Arg-Ser-Leu-Val-Cit- (SEQ ID NO: 31);
   -Gly-Pro-Arg-Pro-Leu-Val-Cit- (SEQ ID NO: 32);
   -Gly-Pro-Arg-Asn-Leu-Val-Cit- (SEQ ID NO: 33);
   -Gly-Pro-Cit-Gly-Leu-Val-Cit- (SEQ ID NO: 34);
   -Gly-Pro-Cit-Pro-Leu-Val-Cit- (SEQ ID NO: 35);
   -His-Pro-Arg-Gly-Leu-Val-Cit- (SEQ ID NO: 36);
   -His-Pro-Arg-Ser-Leu-Val-Cit- (SEQ ID NO: 37);
   -His-Pro-Arg-Pro-Leu-Val-Cit- (SEQ ID NO: 38);
   -His-Pro-Arg-Asn-Leu-Val-Cit- (SEQ ID NO: 39);
   -His-Pro-Cit-Gly-Leu-Val-Cit- (SEQ ID NO: 40);
   -His-Pro-Cit-Pro-Leu-Val-Cit- (SEQ ID NO: 41);
   -Ser-Pro-Arg-Gly-Leu-Val-Cit- (SEQ ID NO: 42);
   -Ser-Pro-Arg-Ser-Leu-Val-Cit- (SEQ ID NO: 43);
   -Ser-Pro-Arg-Pro-Leu-Val-Cit- (SEQ ID NO: 44);
   -Ser-Pro-Arg-Asn-Leu-Val-Cit- (SEQ ID NO: 45);
   -Ser-Pro-Cit-Gly-Leu-Val-Cit- (SEQ ID NO: 46);
   -Ser-Pro-Cit-Ser-Leu-Val-Cit- (SEQ ID NO: 47);
   -Ser-Pro-Cit-Pro-Leu-Val-Cit- (SEQ ID NO: 48);
   -Gly-Pro-Ala-Pro-Leu-Val-Cit- (SEQ ID NO: 49);
   -Gly-Pro-Ala-Asn-Leu-Val-Cit- (SEQ ID NO: 50);
   -Gly-Pro-Arg-Gly-Dap(Me2)-Val-Cit- (SEQ ID NO: 51);
   D is camptothecin or a derivative thereof, auristatin or a derivative thereof, a tetrahydroisoquinoline alkaloid or a derivative thereof, a PROTAC protein degrading agent, or a molecular-glue protein degrading agent. Preferably D is DX8951, MMAE, Dxd, SN38, ET743, CC885, or CC-90009.

In certain embodiments, the linker-payload has the following structure:
MC-GPAGLVCit-PAB-DX8951;
MC-GPLGLGGFG-DX8951;
MC-GPASLVCit-PAB-DX8951;
MC-GPRGLVCit-PAB-DX8951;
MC-GPSALVCit-PAB-DX8951;
MC-GPRGLGGFG-DX8951;
MC-GPAALGGFG-DX8951;
MC-PRNLVCit-PAB-DX8951;
MC-GPRNLVCit-PAB-DX8951;
MC-GPRGDap(Me2)LVCit-PAB-DX8951;
MC-SPRNLVCit-PAB-DX8951;
MC-GPLSLVCit-PAB-DX8951;
MC-PRGLVCit-PAB-DX8951;
MC-PRPLVCit-PAB-DX8951;
MC-GPRSLVCit-PAB-DX8951;
MC-GPRPLVCit-PAB-DX8951;
MC-GPCitPLVCit-PAB-DX8951 ;
MC-HPRGLVCit-PAB-DX8951;
MC-HPCitPLVCit-PAB-DX8951;
MC-SPCitGLVCit-PAB-DX8951;
MC-GPAPLVCit-PAB-DX8951;
MC-PLSLVCit-PAB-DX8951;
MC-SPCitSLVCit-PAB-DX8951;
MC-PRSLVCit-PAB-DX8951;
MC-SPRSLVCit-PAB-DX8951;
MC-PCitSLVCit-PAB-DX8951;
MC-PCitPLVCit-PAB-DX8951;
MC-PCitGLVCit-PAB-DX8951;
MC-GPCitGLVCit-PAB-DX8951;
MC-SPRGLVCit-PAB-DX8951;
MC-SPRPLVCit-PAB-DX8951;
MC-SPCitPLVCit-PAB-DX8951;
MC-GPANLVCit-PAB-DX8951
MC-GPCitNLVCit-PAB-DX8951;
MC-SPCitSDap(Me2)VCit-PAB-DX8951;
MC-PRSDap(Me2)VCit-PAB-DX8951;
MC-GPAGLVCit-PAB-MMAE;
MC-GPRGLVCit-PAB-MMAE;
MC-PRNLVCit-PAB-MMAE;
MC-GPRNLVCit-PAB-MMAE;
MC-GPRGDap(Me2)LVCit-PAB-MMAE;
MC-SPRNLVCit-PAB-MMAE;
MC-GPLSLVCit-PAB-MMAE;
MC-SPCitSLVCit-PAB-MMAE
MC-SPCitSDap(Me2)VCit-PAB-MMAE
MC-SPCitSDap(Me2)VCit-PAB-MMAE
MC-GPAGLVCit-PAB-ET743;
MC-GPRNLVCit-PAB-ET743;
MC-SPCitSLVCit-PAB-ET743;
MC- has a structure of 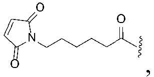
wherein 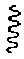 indicates a position for connection to the polypeptide,
DX8951 has a structure of 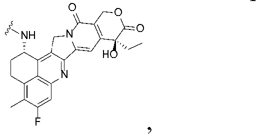
wherein 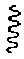 indicates a position for connection to DX8951,
MMAE has a structure of 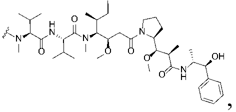
wherein 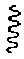 indicates a position for connection to MMAE,
ET-743 has a structure of 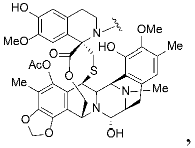
wherein 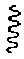 indicates a position for connection to ET743.

### Ab+Linker+Payload antibody-drug conjugate

**In a fourth aspect,** the present application discloses a conjugate comprising the polypeptide or polypeptide fragment -P1-P2- unit.

In some embodiments, the conjugate has a general formula of: 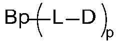 wherein,
Bp is a bioligand;
L is a linker comprising a -P1-P2- unit which is the polypeptide or the polypeptide fragment as described in the preceding two aspects;
D is a drug unit; and
p is any integer from 1 to 20.

In some embodiments, the conjugate has a general formula of: 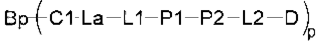 wherein,
Bp is a bioligand;
C1, La, L1, and L2 are as defined in the preceding two aspects;
-P1-P2- unit is the polypeptide or the polypeptide fragment as described in the preceding two aspects;
D is a drug unit; and
p is any integer from 1 to 20.

In some embodiments, the bioligand targets a cell surface receptor or a tumor-associated antigen.

In some embodiments, the cell surface receptor or tumor-associated antigen comprises EGFR, HER2, HER3, c-Met, claudin18.2, TROP-2, a folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, IGF-1R, TNFR1, or GLP-1.

In some embodiments, the bioligand comprises an antibody or an antigen-binding fragment thereof, a modified antibody or an antigen-binding fragment thereof, an oligonucleotide, a DNA fragment, a nucleic acid aptamer, a polypeptide, a nanoparticle with targeting ability, or a chemically-synthesized small molecule.

In some embodiments, the bioligand comprises an antibody or an antigen-binding fragment thereof, a modified antibody or an antigen-binding fragment thereof.

In some embodiments, the antibody comprises a monoclonal antibody, a polyclonal antibody, a dimer, a polymer, a multispecific antibody, an intact antibody, an antibody fragment, a human antibody, a humanized antibody, or a chimeric antibody.

In some embodiments, the modified antibody comprises a modified monoclonal antibody, a modified polyclonal antibody, a modified dimer, a modified polymer, a modified multispecific antibody, a modified intact antibody, a modified antibody fragment, a modified human antibody, a modified humanized antibody, or a modified chimeric antibody.

In some embodiments, the antigen-binding fragment comprises a Fab, a Fab', a Fv fragment, a F(ab')2, a scFv, a di-scFv, and/or a dAb.

In some embodiments, the modified antigen-binding fragment comprises a modified Fab, a modified Fab', a modified Fv fragment, a modified F(ab')2, a modified scFv, a modified di-scFv, and/or a modified dAb.

In some embodiments, the antibody is a monoclonal antibody or a modified monoclonal antibody.

In some embodiments, the antibody is a human antibody, a humanized antibody, or a chimeric antibody. The modified antibody is a modified human antibody, a modified humanized antibody, or a modified chimeric antibody.

In some embodiments, the modification method is amino acid point mutation, glycosylation modification, and polypeptide modification.

In some embodiments, the antibody or the antigen-binding fragment thereof, the modified antibody or the modified antigen-binding fragment thereof is anti-EGFR, anti-HER2, anti-HER3, anti-c-Met, anti-claudin18.2, anti-TROP-2, anti-FRα, anti-ROR1, anti-ROR2, anti-BCMA, anti-PSMA, anti-CD19, anti-CD20, anti-CD22, anti-CD30, anti-CD33, anti-CD37, anti-CD46, anti-CD48, anti-CD56, anti-CD79b, anti-CD123, anti-CD138, anti-CD166, anti-CD276, anti-CEACAM5, anti-SDC1, anti-CD74, anti-CD70, anti-MUC1, anti-MUC16, anti-GUCY2C, anti-MSLN, anti-SCL34A2, anti-FOLH1, anti-TPBC, anti-PVRL4, anti-STEAP1, anti-SCL44A4, anti-NACM1, anti-EDNRB, anti-GPNMB, anti-FGFR, anti-SEZ6, anti-Nectin-4, anti-PD-L1, anti-TF, anti-B7-H3, anti-B7-H4, anti-LY6E, anti-LIV-1, anti-CDH4, anti-CDH6, anti-ITGB6, anti-GPC1, anti-GPC3, anti-ENPP3, anti-KAAG1, anti-FZD7, anti-SSTR2, anti-DLK1, anti-TMEFF1, anti-TM4SF1, anti-SLAMF7, anti-DLL3, anti-FLT3, anti-TNFRSF10B, anti-EPCAM, anti-AXL, anti-IL2RA, anti-5T4, anti-FAP, anti-ICAM-1, anti-IGF-1R, anti-TNFR1, or anti-GLP-1.

In some embodiments, the antibody or the antigen-binding fragment thereof, the modified antibody or the modified antigen-binding fragment thereof is an anti-Her2, anti-TF, anti-ROR1, or anti-GPC3 antibody or an antigen binding fragment thereof.

In some embodiments, the antibody is Trastuzumab, Tisotumab, Zilovertamab, or Codrituzumab.

In some embodiments, the conjugate is an antibody-drug conjugate, wherein the antibody-drug conjugate is selected from the following structures:
Ab-((succinimid-3-yl-N)-CH2-C(=O)-P1-P2-D)ₙ;
Ab-((succinimid-3-yl-N)-CH2CH2-C(=O)-P1-P2-D)ₙ;
Ab-((succinimid-3-yl-N)-(CH2CH2O)₄-CH2CH2-C(=O)-P1-P2-D)ₙ;
Ab-((succinimid-3-yl-N)-(CH2CH2O)₈-CH2CH2-C(=O)-P1-P2-D)ₙ;
Ab-((succinimid-3-yl-N)-CH2CH2C(O)NH-(CH2CH2O)₄-CH2CH2-C(=O)-P1-P2-D)ₙ;
Ab-((succinimid-3-yl-N)-CH2CH2C(O)NH-(CH2CH2O)₈-CH2CH2-C(=O)-P1-P2-D)ₙ;
Ab-((succinimid-3-yl-N)-OC(O)-(CH2CH2O)₄-CH2CH2-C(= O)-P1-P2-D)ₙ;
Ab-((succinimid-3-yl-N)-OC(O)-(CH2CH2O)₈-CH2CH2-C(= O)-P1-P2-D)ₙ;
Ab-((succinimid-3-yl-N)-CH2CH2CH2CH2CH2-C(=O)-P1-P2-D)ₙ;
Ab-((succinimid-3-yl-N)-CH2-C(=O)-P1-P2-PAB-D)ₙ;
Ab-((succinimid-3-yl-N)-CH2CH2-C(=O)-P1-P2-PAB-D)ₙ;
Ab-((succinimid-3-yl-N)-(CH2CH2O)₄-CH2CH2-C(=O)-P1-P2-PAB-D)ₙ;
Ab-((succinimid-3-yl-N)-(CH2CH2O)₈-CH2CH2-C(= O)-P1-P2-PAB-D)ₙ;
Ab-((succinimid-3-yl-N)-CH2CH2C(O)NH-(CH2CH2O)₄-CH2CH2-C(=O)-P1-P2-PAB-D)ₙ;
Ab-((succinimid-3-yl-N)-CH2CH2C(O)NH-(CH2CH2O)₈-CH2CH2-C(=O)-P1-P2-PAB-D)ₙ;
Ab-((succinimid-3-yl-N)-OC(O)-(CH2CH2O)₄-CH2CH2-C(=O)-P1-P2-PAB-D)ₙ;
Ab-((succinimid-3-yl-N)-OC(O)-(CH2CH2O)₈-CH2CH2-C(=O)-P1-P2-PAB-D)ₙ;
Ab-((succinimid-3-yl-N)-CH2CH2CH2CH2CH2-C(=O)-P1-P2-PAB-D)ₙ; 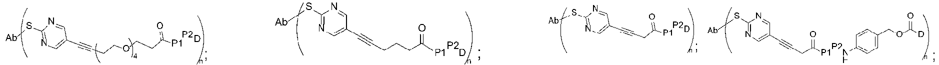 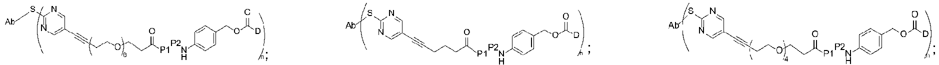 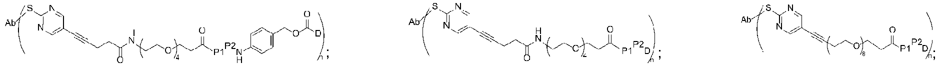 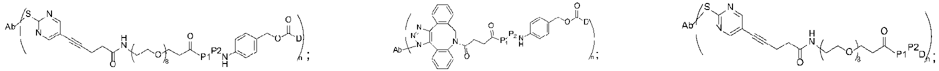 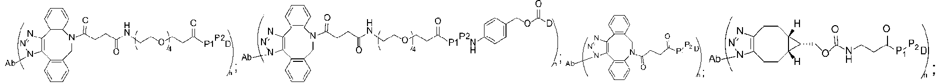 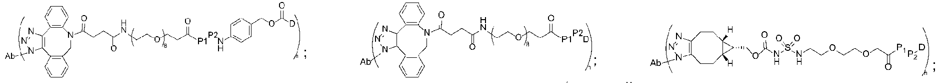 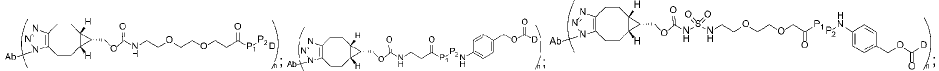 and 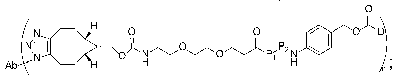 and
Ab represents a monoclonal antibody or an antigen-binding fragment thereof, or a modified monoclonal antibody or an antigen-binding fragment thereof, and preferably an anti-Her2, anti-TF, anti-ROR1, anti-GPC3 antibody or an antigen-binding fragment thereof;
-P1-P2- represents the following structure:
   -His-Val-Leu-Asn-Leu-Val-Cit- (SEQ ID NO: 1);
   -Val-Pro-Leu-Ser-Leu-Val-Cit- (SEQ ID NO: 2);
   -Ile-Pro-Val-Ser-Leu-Val-Cit- (SEQ ID NO: 3);
   -Gly-Pro-Leu-Ser-Leu-Val-Cit- (SEQ ID NO: 4);
   -Gly-Pro-Ala-Ser-Leu-Val-Cit- (SEQ ID NO: 5);
   -Gly-Pro-Ala-Gly-Leu-Val-Cit- (SEQ ID NO: 6);
   -Gly-Pro-Gln-Gly-Leu-Val-Cit- (SEQ ID NO: 7);
   -Gly-Pro-Leu-Gly-Leu-Val-Cit- (SEQ ID NO: 8);
   -Gly-Pro-Ser-Gly-Leu-Val-Cit- (SEQ ID NO: 9);
   -Gly-Pro-Arg-Gly-Leu-Val-Cit- (SEQ ID NO: 10);
   -Gly-Pro-Ala-Ala-Leu-Val-Cit- (SEQ ID NO: 11);
   -Gly-Pro-Ser-Ala-Leu-Val-Cit- (SEQ ID NO: 12);
   -His-Val-Leu-Asn-Leu-Gly-Gly-Phe-Gly- (SEQ ID NO: 13);
   -Val-Pro-Leu-Ser-Leu-Gly-Gly-Phe-Gly- (SEQ ID NO: 14);
   - Ile-Pro-Val-Ser-Leu-Gly-Gly-Phe-Gly- (SEQ ID NO: 15);
   -Gly-Pro-Leu-Ser-Leu-Gly-Gly-Phe-Gly- (SEQ ID NO: 16);
   -Gly-Pro-Ala-Ser-Leu-Gly-Gly-Phe-Gly- (SEQ ID NO: 17);
   -Gly-Pro-Ala-Gly-Leu-Gly-Gly-Phe-Gly- (SEQ ID NO: 18);
   -Gly-Pro-Gln-Gly-Leu-Gly-Gly-Phe-Gly- (SEQ ID NO: 19);
   -Gly-Pro-Leu-Gly-Leu-Gly-Gly-Phe-Gly- (SEQ ID NO: 20);
   -Gly-Pro-Ser-Gly-Leu-Gly-Gly-Phe-Gly- (SEQ ID NO: 21);
   -Gly-Pro-Arg-Gly-Leu-Gly-Gly-Phe-Gly- (SEQ ID NO: 22);
   -Gly-Pro-Ala-Ala-Leu-Gly-Gly-Phe-Gly- (SEQ ID NO: 23);
   -Gly-Pro-Ser-Ala-Leu-Gly-Gly-Phe-Gly- (SEQ ID NO: 24);
   -Pro-Arg-Gly-Leu-Val-Cit- (SEQ ID NO: 25);
   -Pro-Arg-Ser-Leu-Val-Cit- (SEQ ID NO: 26);
   -Pro-Arg-Asn-Leu-Val-Cit- (SEQ ID NO: 27);
   -Pro-Arg-Pro-Leu-Val-Cit- (SEQ ID NO: 28);
   -Pro-Cit-Gly-Leu-Val-Cit- (SEQ ID NO: 29);
   -Pro-Cit-Pro-Leu-Val-Cit- (SEQ ID NO: 30);
   -Gly-Pro-Arg-Ser-Leu-Val-Cit- (SEQ ID NO: 31);
   -Gly-Pro-Arg-Pro-Leu-Val-Cit- (SEQ ID NO: 32);
   -Gly-Pro-Arg-Asn-Leu-Val-Cit- (SEQ ID NO: 33);
   -Gly-Pro-Cit-Gly-Leu-Val-Cit- (SEQ ID NO: 34);
   -Gly-Pro-Cit-Pro-Leu-Val-Cit- (SEQ ID NO: 35);
   -His-Pro-Arg-Gly-Leu-Val-Cit- (SEQ ID NO: 36);
   -His-Pro-Arg-Ser-Leu-Val-Cit- (SEQ ID NO: 37);
   -His-Pro-Arg-Pro-Leu-Val-Cit- (SEQ ID NO: 38);
   -His-Pro-Arg-Asn-Leu-Val-Cit- (SEQ ID NO: 39);
   -His-Pro-Cit-Gly-Leu-Val-Cit- (SEQ ID NO: 40);
   -His-Pro-Cit-Pro-Leu-Val-Cit- (SEQ ID NO: 41);
   -Ser-Pro-Arg-Gly-Leu-Val-Cit- (SEQ ID NO: 42);
   -Ser-Pro-Arg-Ser-Leu-Val-Cit- (SEQ ID NO: 43);
   -Ser-Pro-Arg-Pro-Leu-Val-Cit- (SEQ ID NO: 44);
   -Ser-Pro-Arg-Asn-Leu-Val-Cit- (SEQ ID NO: 45);
   -Ser-Pro-Cit-Gly-Leu-Val-Cit- (SEQ ID NO: 46);
   -Ser-Pro-Cit-Ser-Leu-Val-Cit- (SEQ ID NO: 47);
   -Ser-Pro-Cit-Pro-Leu-Val-Cit- (SEQ ID NO: 48);
   -Gly-Pro-Ala-Pro-Leu-Val-Cit- (SEQ ID NO: 49);
   -Gly-Pro-Ala-Asn-Leu-Val-Cit- (SEQ ID NO: 50);
   -Gly-Pro-Arg-Gly-Dap(Me2)-Val-Cit- (SEQ ID NO: 51);
   or a variant having one or two mismatches with respect to any sequences of SEQ ID NOs:1-51;
   D is camptothecin or a derivative thereof, auristatin or a derivative thereof, a tetrahydroisoquinoline alkaloid or a derivative thereof, a protein degrading agent and a derivative thereof, and preferably DX8951, MMAE, Dxd, SN38, ET743, Lurbinectedin, CC885, or CC-90009;
   n represents an integer from 1 to 10.

In some embodiments, the antibody-drug conjugate is selected from the following structures:
Ab-((succinimid-3-yl-N)-CH2CH2CH2CH2CH2-C(=O)-GPAGLVCit-PAB-DX8951)n;
Ab-((succinimid-3-yl-N)-CH2CH2CH2CH2CH2-C(=O)-GPLGLGGFG-DX8951)n;
Ab-((succinimid-3-yl-N)-CH2CH2CH2CH2CH2-C(=O)-GPASLVCit-PAB-DX8951)n;
Ab-((succinimid-3-yl-N)-CH2CH2CH2CH2CH2-C(=O)-GPRGLVCit-PAB-DX8951)n;
Ab-((succinimid-3-yl-N)-CH2CH2CH2CH2CH2-C(=O)-GPSALVCit-PAB-DX8951)n;
Ab-((succinimid-3-yl-N)-CH2CH2CH2CH2CH2-C(=O)-GPRGLGGFG-DX8951)n;
Ab-((succinimid-3-yl-N)-CH2CH2CH2CH2CH2-C(=O)-GPAALGGFG-DX8951)n;
Ab-((succinimid-3-yl-N)-CH2CH2CH2CH2CH2-C(=O)-PRNLVCit-PAB-DX8951)n;
Ab-((succinimid-3-yl-N)-CH2CH2CH2CH2CH2-C(=O)-GPRNLVCit-PAB-DX8951)n;
Ab-((succinimid-3-yl-N)-CH2CH2CH2CH2CH2-C(=O)-GPRGDap(Me2)LVCit-PAB-DX8951)n;
Ab-((succinimid-3-yl-N)-CH2CH2CH2CH2CH2-C(=O)-SPRNLVCit-PAB-DX8951)n;
Ab-((succinimid-3-yl-N)-CH2CH2CH2CH2CH2-C(=O)-GPLSLVCit-PAB-DX8951)n;
Ab-((succinimid-3-yl-N)-CH2CH2CH2CH2CH2-C(=O)-GPAGLVCit-PAB-MMAE)n;
Ab-((succinimid-3-yl-N)-CH2CH2CH2CH2CH2-C(=O)-GPRGLVCit-PAB-MMAE)n;
Ab-((succinimid-3-yl-N)-CH2CH2CH2CH2CH2-C(=O)-PRNLVCit-PAB-MMAE)n;
Ab-((succinimid-3-yl-N)-CH2CH2CH2CH2CH2-C(=O)-GPRNLVCit-PAB-MMAE)n;
Ab-((succinimid-3-yl-N)-CH2CH2CH2CH2CH2-C(=O)-GPRGDap(Me2)LVCit-PAB-MMAE)n;
Ab-((succinimid-3-yl-N)-CH2CH2CH2CH2CH2-C(=O)-SPRNLVCit-PAB-MMAE)n;
Ab-((succinimid-3-yl-N)-CH2CH2CH2CH2CH2-C(=O)-GPLSLVCit-PAB-MMAE)n;
Ab-((succinimid-3-yl-N)-CH2CH2CH2CH2CH2-C(=O)-GPAGLVCit-PAB-ET743)n; and
Ab-((succinimid-3-yl-N)-CH2CH2CH2CH2CH2-C(=O)-GPRNLVCit-PAB-ET743)n;
Ab represents a monoclonal antibody or an antigen-binding fragment thereof, or a modified monoclonal antibody or an antigen-binding fragment thereof, and preferably anti-Her2, anti-TF, anti-ROR1, or anti-GPC3;
DX8951 has a structure of

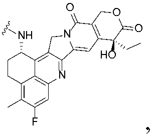

wherein 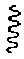 indicates a position for connection to DX8951,
MMAE has a structure of 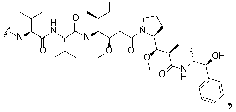
wherein 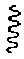 indicates a position for connection to MMAE;
ET-743 has a structure of 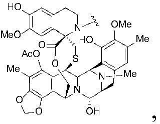
wherein 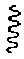 indicates a position for connection to ET743;
n is an integer from 1 to 10, and preferably, 2, 4, 6, or 8.

In some embodiments, the antibody or the antigen-binding fragment thereof is selected from Trastuzumab, Tisotumab, Zilovertamab, and Codrituzumab.

In some embodiments, the antibody-drug conjugate is selected from:
Ab1-((succinimid-3-yl-N)-CH2CH2CH2CH2CH2-C(=O)-GPAGLVCit-PAB-DX8951)n;
Ab1-((succinimid-3-yl-N)-CH2CH2CH2CH2CH2-C(=O)-GPLGLGGFG-PAB-DX8951)n;
Ab1-((succinimid-3-yl-N)-CH2CH2CH2CH2CH2-C(=O)-GPASLVCit-PAB-DX8951)n;
Ab1-((succinimid-3-yl-N)-CH2CH2CH2CH2CH2-C(=O)-GPRGLVCit-PAB-DX8951)n;
Ab1-((succinimid-3-yl-N)-CH2CH2CH2CH2CH2-C(=O)-GPSALVCit-PAB-DX8951)n;
Ab1-((succinimid-3-yl-N)-CH2CH2CH2CH2CH2-C(=O)-GPRGLGGFG-PAB-DX8951)n;
Ab1-((succinimid-3-yl-N)-CH2CH2CH2CH2CH2-C(=O)-GPAALGGFG-PAB-DX8951)n;
Ab1-((succinimid-3-yl-N)-CH2CH2CH2CH2CH2-C(=O)-PRNLVCit-PAB-DX8951)n;
Ab1-((succinimid-3-yl-N)-CH2CH2CH2CH2CH2-C(=O)-GPRNLVCit-PAB-DX8951)n;
Ab1-((succinimid-3-yl-N)-CH2CH2CH2CH2CH2-C(=O)-GPRGDap(Me2)LVCit-PAB-DX8951)n; and
Ab1-((succinimid-3-yl-N)-CH2CH2CH2CH2CH2-C(=O)-SPRNLVCit-PAB-DX8951)n;
the antibody Ab1 is Trastuzumab; Trastuzumab is an anti-Her2 monoclonal antibody, and
n is an integer from 1 to 10. Preferably, n is 2, 4, 6, or 8.

In some embodiments, the antibody-drug conjugate is selected from:
Ab2-((succinimid-3-yl-N)-CH2CH2CH2CH2CH2-C(=O)-GPAGLVCit-PAB-DX8951)n;
Ab2-((succinimid-3-yl-N)-CH2CH2CH2CH2CH2-C(=O)-GPLGLGGFG-PAB-DX8951)n;
Ab2-((succinimid-3-yl-N)-CH2CH2CH2CH2CH2-C(=O)-GPRGLVCit-PAB-DX8951)n;
Ab2-((succinimid-3-yl-N)-CH2CH2CH2CH2CH2-C(=O)-PRNLVCit-PAB-DX8951)n;
Ab2-((succinimid-3-yl-N)-CH2CH2CH2CH2CH2-C(=O)-GPRNLVCit-PAB-DX8951)n;
Ab2-((succinimid-3-yl-N)-CH2CH2CH2CH2CH2-C(=O)-GPRGDap(Me2)LVCit-PAB-DX8951)n;
Ab2-((succinimid-3-yl-N)-CH2CH2CH2CH2CH2-C(=O)-SPRNLVCit-PAB-DX8951)n.
Ab2-((succinimid-3-yl-N)-CH2CH2CH2CH2CH2-C(=O)-GPAGLVCit-PAB-MMAE)n;
Ab2-((succinimid-3-yl-N)-CH2CH2CH2CH2CH2-C(=O)-GPRGLVCit-PAB-MMAE)n;
Ab2-((succinimid-3-yl-N)-CH2CH2CH2CH2CH2-C(=O)-PRNLVCit-PAB-MMAE)n;
Ab2-((succinimid-3-yl-N)-CH2CH2CH2CH2CH2-C(=O)-GPRNLVCit-PAB-MMAE)n;
Ab2-((succinimid-3-yl-N)-CH2CH2CH2CH2CH2-C(=O)-GPRGDap(Me2)LVCit-PAB-MMAE)n; and
Ab2-((succinimid-3-yl-N)-CH2CH2CH2CH2CH2-C(=O)-SPRNLVCit-PAB-MMAE)n,
wherein Ab2 is an anti-TF antibody, which may be Tisotumab or a humanized anti-TF antibody, and n is an integer from 1 to 10. Preferably, n is 2, 4, 6, or 8.

In some embodiments, the antibody-drug conjugate is selected from:
Ab3-((succinimid-3-yl-N)-CH2CH2CH2CH2CH2-C(=O)-GPAGLVCit-PAB-DX8951)n;
Ab3-((succinimid-3-yl-N)-CH2CH2CH2CH2CH2-C(=O)-GPLGLGGFG-PAB-DX8951)n;
Ab3-((succinimid-3-yl-N)-CH2CH2CH2CH2CH2-C(=O)-GPASLVCit-PAB-DX8951)n;
Ab3-((succinimid-3-yl-N)-CH2CH2CH2CH2CH2-C(=O)-GPRGLVCit-PAB-DX8951)n;
Ab3-((succinimid-3-yl-N)-CH2CH2CH2CH2CH2-C(=O)-GPSALVCit-PAB-DX8951)n;
Ab3-((succinimid-3-yl-N)-CH2CH2CH2CH2CH2-C(=O)-GPRGLGGFG-PAB-DX8951)n;
Ab3-((succinimid-3-yl-N)-CH2CH2CH2CH2CH2-C(=O)-GPAALGGFG-PAB-DX8951)n;
Ab3-((succinimid-3-yl-N)-CH2CH2CH2CH2CH2-C(=O)-PRNLVCit-PAB-DX8951)n;
Ab3-((succinimid-3-y1-N)-CH2CH2CH2CH2CH2-C(=O)-GPRNLVCit-PAB-DX8951)n;
Ab3-((succinimid-3-yl-N)-CH2CH2CH2CH2CH2-C(=O)-GPRGDap(Me2)LVCit-PAB-DX8951)n;
Ab3-((succinimid-3-yl-N)-CH2CH2CH2CH2CH2-C(=O)-SPRNLVCit-PAB-DX8951)n;
Ab3-((succinimid-3-yl-N)-CH2CH2CH2CH2CH2-C(=O)-GPLSLVCit-PAB-DX8951)n;
Ab3-((succinimid-3-yl-N)-CH2CH2CH2CH2CH2-C(=O)-GPAGLVCit-PAB-MMAE)n;
Ab3-((succinimid-3-yl-N)-CH2CH2CH2CH2CH2-C(=O)-GPRGLVCit-PAB-MMAE)n;
Ab3-((succinimid-3-yl-N)-CH2CH2CH2CH2CH2-C(=O)-PRNLVCit-PAB-MMAE)n;
Ab3-((succinimid-3-yl-N)-CH2CH2CH2CH2CH2-C(=O)-GPRNLVCit-PAB-MMAE)n;
Ab3-((succinimid-3-yl-N)-CH2CH2CH2CH2CH2-C(=O)-GPRGDap(Me2)LVCit-PAB-MMAE)n;
Ab3-((succinimid-3-yl-N)-CH2CH2CH2CH2CH2-C(=O)-SPRNLVCit-PAB-MMAE)n;
Ab3-((succinimid-3-yl-N)-CH2CH2CH2CH2CH2-C(=O)-GPLSLVCit-PAB-MMAE)n; and
Ab3-((succinimid-3-yl-N)-CH2CH2CH2CH2CH2-C(=O)-GPAGLVCit-PAB-ET743)n;
wherein Ab3 is Codrituzumab, Codrituzumab is an anti-GPC3 antibody, and preferably, n is an integer from 1 to 10. Preferably, n is 2, 4, 6, or 8.

In some embodiments, the antibody-drug conjugate is:
Ab4-((succinimid-3-yl-N)-CH₂CH₂CH₂CH₂CH₂-C(=O)-GPAGLVCit-PAB-DX8951)n;
wherein Ab4 is Zilovertamab, and Zilovertamab is an anti-ROR1 antibody; and
n is an integer from 1 to 10. Preferably, n is 2, 4, 6, or 8.

### Bioligand

The antibody or antigen-binding fragment of the aforementioned bioligand can be prepared by various methods known in the art, for example, by a genetic-engineering recombinant technology. For example, DNA molecules encoding the heavy and light chain genes of the antibody in this disclosure are obtained by chemical synthesis or PCR amplification. The obtained DNA molecules are inserted into an expression vector, and then transfected into host cells. The transfected host cells are then cultured under specific conditions to express the antibody of this disclosure.

In some embodiments, the bioligand is an anti-Her2 monoclonal antibody, such as Trastuzumab or Pertuzumab.

In some embodiments of the present application, the bioligand is Trastuzumab, an anti-Her2 monoclonal antibody with an amino acid sequence known to those skilled in the art, and the exemplary sequence thereof may be referring to, for example, the patent CN103319599.

In some embodiments of the present application, the bioligand is an anti-GPC3 antibody recorded in U.S. Patent US10782300B2 (i.e., Codrituzumab), which may be obtained by screening through the disclosed vector design and construction, and a method for constructing an antibody library displaying antibodies.

The heavy chain and light chain amino acid sequences of the Codrituzumab monoclonal antibody may be referring to SEQ ID No: 50 and SEQ ID No: 66 in the Patent US10782300B2, respectively.

In some embodiments of the present application, the bioligand is an anti-ROR1 antibody set forth in SEQ ID No: 5 and SEQ ID No: 6 of the Patent US10335496B2.

In certain embodiments of the present application, the bioligand is an anti-TF antibody or an antigen-binding fragment thereof. In some embodiments, the anti-TF antibody includes all anti-TF antibodies known in the art, for example: Tisotumab, and anti-TF antibodies disclosed in patents CN111818943A, CN106938051B, CN110575547A, CN103119065A, WO2011157741, WO 2010066803, and US 9168314 B2. In some embodiments, the anti-TF antibody or the antigen-binding fragment thereof is selected from: the anticoagulant murine chimeric antibody 1849 in US9920133B2 or the non-anticoagulant murine chimeric antibody 1859 in US9920133B2. In some embodiments, the anti-TF antibody is the anti-TF antibody described in the following fifth aspect.

In a fifth aspect, the present application discloses an anti-Tissue Factor (TF) antibody or an antigen-binding fragment thereof, comprising:
(a) a VH comprising an amino acid sequence set forth in SEQ ID NO: 52 or 54, or an amino acid sequence having at least 80% identity to the amino acid sequence set forth in SEQ ID NO: 52 or 54, or an amino acid sequence obtained by substituting, deleting, or adding one or more amino acids to the amino acid sequence set forth in SEQ ID NO: 52 or 54 and having the same or similar function as the amino acid sequence set forth in SEQ ID NO: 52 or 54; and/or
(b) a VL comprising an amino acid sequence set forth in SEQ ID NO: 53 or 55, or an amino acid sequence having at least 80% identity to the amino acid sequence set forth in SEQ ID NO: 53 or 55, or an amino acid sequence obtained by substituting, deleting, or adding one or more amino acids to the amino acid sequence set forth in SEQ ID NO: 53 or 55 and having the same or similar function as the amino acid sequence set forth in SEQ ID NO: 53 or 55.

In some embodiments, the anti-TF antibody or the antigen-binding fragment thereof is characterized in that the anti-TF antibody or the antigen-binding fragment thereof comprises:
(1) a VH having an amino acid sequence set forth in SEQ ID NO: 52, and a VL having an amino acid sequence set forth in SEQ ID NO: 53; or
(2) a VH having an amino acid sequence set forth in SEQ ID NO: 54, and a VL having an amino acid sequence set forth in SEQ ID NO: 53; or
(3) a VH having an amino acid sequence set forth in SEQ ID NO: 54, and a VL having an amino acid sequence set forth in SEQ ID NO: 55;
(4) a VH having an amino acid sequence set forth in SEQ ID NO: 52, and a VL having an amino acid sequence set forth in SEQ ID NO: 55.

In some embodiments, the anti-TF antibody or the antigen-binding fragment thereof comprises a light chain having an amino acid sequence set forth in SEQ ID NO: 56, or an amino acid sequence having at least 80% identity to the amino acid sequence set forth in SEQ ID NO: 56; a heavy chain having an amino acid sequence set forth in SEQ ID NO: 57, or an amino acid sequence having at least 80% identity to the amino acid sequence set forth in SEQ ID NO: 57.

In some embodiments, the anti-TF antibody or the antigen-binding fragment thereof comprisees a light chain having an amino acid sequence set forth in SEQ ID NO: 56, or an amino acid sequence having at least 80% identity to the amino acid sequence set forth in SEQ ID NO: 56; a heavy chain having an amino acid sequence set forth in SEQ ID NO: 59, or an amino acid sequence having at least 80% identity to the amino acid sequence set forth in SEQ ID NO: 59.

In some embodiments, the anti-TF antibody or the antigen-binding fragment thereof comprisees a light chain having an amino acid sequence set forth in SEQ ID NO: 58, or an amino acid sequence having at least 80% identity to the amino acid sequence set forth in SEQ ID NO: 58; a heavy chain having an amino acid sequence set forth in SEQ ID NO: 59, or an amino acid sequence having at least 80% identity to the amino acid sequence set forth in SEQ ID NO: 59.

In some embodiments, the TF is mammalian TF, and the antibody is preferably a murine antibody, simian antibody, rabbit antibody, chimeric antibody, humanized antibody, or human antibody; and/or
the antibody is an IgG antibody, preferably an IgG1, IgG2, IgG3, or IgG4 antibody, and more preferably an IgG1 antibody; and/or
the anti-TF antibody or the antigen-binding fragment thereof is selected from monoclonal antibodies, polyclonal antibodies, Fab fragments, Fab' fragments, F(ab')2 fragments, Fd fragments, Fv fragments, dAb fragments, isolated CDR regions, scFv, and nanobodies.

In some embodiments, the TF is a mammalian TF, and the antibody is preferably a modified murine antibody, simian antibody, rabbit antibody, chimeric antibody, humanized antibody, or human antibody; and/or

In some embodiments, the antibody is a modified IgG antibody, preferably a modified IgG1, IgG2, IgG3, or IgG4 antibody, and more preferably a modified IgG1 antibody; and/or;
in some embodiments, the anti-TF antibody or the antigen-binding fragment thereof is selected from modified monoclonal antibodies, polyclonal antibodies, Fab fragments, Fab' fragments, F(ab')2 fragments, Fd fragments, Fv fragments, dAb fragments, isolated CDR regions, scFv, and nanobodies.

In some embodiments, the modification method is amino acid point mutation, glycosylation modification, or polypeptide modification.

The antigen-binding fragment of the present application may be obtained by hydrolyzing an intact antibody molecule (see Morimoio et al., J. Biochem. Biophys. Methods 24:107-117 (1992) and Brennan et al, Science 229:81 (1985)). In addition, these antigen-binding fragments may also be directly produced by recombinant host cells (see Verma et al. J. Immunol. Methods. 216:165-181 (1998); Little et al. Immunol. Today, 21:364-370 (2000)). For example, Fab' fragments may be directly obtained from host cells; Fab fragments may be chemically conjugated to form F(ab')2 fragments (Carter et al, Biotechnology, 10: 163-167 (1992)), Additionally, Fv, Fab, or F(ab')2 fragments may also be directly isolated from recombinant host cell culture fluid. Those skilled in the art is fully aware of other technologies for preparing these antigen-binding fragments.

**In a sixth aspect,** provided is a nucleic acid molecule, encoding an anti-TF antibody or an antigen-binding fragment thereof, or a heavy chain variable region and/or light chain variable region thereof. According to the codon degeneracy known in the art, in certain embodiments, the nucleotide sequence may be substituted based on the codon degeneracy.

In certain embodiments, the nucleotide sequence is codon-optimized.

In certain embodiments, the isolated nucleic acid molecule of the present disclosure comprises: (i) a first nucleic acid and a second nucleic acid encoding a heavy variable region and a light chain variable region of the antibody or the antigen-binding fragment thereof of the present disclosure, respectively, or (ii) a first nucleic acid and a second nucleic acid encoding a heavy chain variable region and a heavy chain constant region, and a light chain variable region and a light chain constant region of the antibody or the antigen-binding fragment thereof of the present disclosure, respectively, or (iii) a first nucleic acid and a second nucleic acid encoding a heavy chain and a light chain of the antibody or the antigen-binding fragment thereof of the present disclosure, respectively.

In certain embodiments, the first nucleic acid and the second nucleic acid comprise a degenerate sequence or substantially the same sequence of any of the first nucleic acid and the second nucleic acid as described in (i)-(iii) above. In certain embodiments, the degenerate sequence or substantially the same sequence refers to a sequence having at least about 85%, 90%, 95%, 99% or higher sequence identity to the nucleic acid molecules described in (i)-(iii), or a sequence having one or more nucleotide substitutions, or a sequence differing by no more than 3, 6, 15, 30, or 45 nucleotides.

**In a seventh aspect,** provided is a vector (for example, a cloning vector or an expression vector), comprising the nucleic acid molecule of the present application.

In certain embodiments, the vector of the present disclosure is, for example, a plasmid, cosmid, bacteriophage, lentivirus, or the like.

In certain embodiments, the vector is capable of expressing the antibody or the antigen-binding fragment thereof of the present disclosure in a subject (e.g., a mammal, such as human).

**In an eighth aspect,** the present application provides a recombinant cell, comprising the nucleic acid molecule as described above in the claims or a recombinant vector comprising the nucleic acid molecule.

The host cell may be a eukaryotic cell (for example, a mammalian cell, insect cell, yeast cell) or a prokaryotic cell (for example, *Escherichia coli).* Suitable eukaryotic cells include, but are not limited to, NS0 cells, Vero cells, Hda cells, COS cells, CHO cells, HEK293 cells, BHK cells, and MDCKII cells. Suitable insect cells include, but are not limited to, Sf9^{®} cells. In certain embodiments, the host cell of the present application is a mammalian cell, e.g., CHO (for example, CHOK1, CHO-S, CHODXBil, CHODG44).

**In a ninth aspect,** the present application provides a method of preparing an anti-TF antibody or an antigen-binding fragment thereof, comprising culturing the recombinant cell as described.

**In a tenth aspect,** the present application provides use of the anti-TF antibody or the antigen-binding fragment thereof, the nucleic acid molecule, the recombinant vector, or the recombinant cell in the preparation of a medicament. Preferably, the medicament is a medicament for the prevention and/or treatment of a cancer; and more preferably, the cancer is a TF-expressing cancer.

In some embodiments, provided is use of the anti-TF antibody or the antigen-binding fragment thereof, the nucleic acid molecule, the recombinant vector, or the recombinant cell in the preparation of a reagent for diagnosing a cell proliferation-related disease. Preferably, the disease is a cancer; and more preferably, the cancer is a TF-expressing cancer.

### Preparation process

**In an eleventh aspect,** the present application provides a method of preparing the polypeptide or the polypeptide fragment -P1-P2-, or a tautomer, a mesomer, an enantiomer, a diastereomer, or a mixed form thereof, or a pharmaceutically acceptable salt thereof, or a precursor thereof.

**In a twelfth aspect,** the present application provides a method of preparing the linker or a tautomer, a mesomer, an enantiomer, a diastereomer, or a mixed form thereof, or a pharmaceutically acceptable salt thereof, or a precursor thereof.

In some embodiments, the method comprises sequentially linking the polypeptide or the polypeptide fragment -P1-P2- with its preceding and succeeding units C1, La, L1, and L2. C1, La, L1, and L2 are as defined in the present application.

**In a thirteenth aspect,** the present application provides a method of preparing the linker-payload or a tautomer, a mesomer, an enantiomer, a diastereomer, or a mixed form thereof, or a pharmaceutically acceptable salt thereof, or a precursor thereof.

In some embodiments, the method comprises sequentially linking the above polypeptide or the polypeptide fragment -P1-P2- with its preceding and succeeding units C1', La, L1, L2, and D. C1', La, L1, L2, and D are as defined in the present application.

**In a fourteenth aspect,** the present application provides a method of preparing the conjugate or a tautomer, a mesomer, an enantiomer, a diastereomer, or a mixed form thereof, or a pharmaceutically acceptable salt thereof, or a precursor thereof.

In some embodiments, the method comprises sequentially linking Bp, C1 or C1', La, L1, L2, P1, P2, D, or a precursor thereof.

P1 and P2 are as defined above.

C1, La, L1, and L2 are as defined above.

C1' and D are as defined above.

Bp is as defined above.

**In a fifteenth aspect,** the present application provides a method of preparing the conjugate or a tautomer, a mesomer, an enantiomer, a diastereomer, or a mixed form thereof, or a pharmaceutically acceptable salt thereof, or a precursor thereof.

In some embodiments, the method comprises performing a coupling reaction on Bp and the linker-payload C1'-La-L1-P1-P2-L2-D in a suitable solvent and under a suitable condition. C1'-La-L1-P1-P2-L2-D is as defined above.

Preferably, the molar ratio of Bp to the linker-payload C1'-La-L1-P1-P2-L2-D is 1:(1-20).

In some embodiments, the molar ratio of Bp to the linker-payload C1'-La-L1-P1-P2-L2-D may be 1:4, 1:5, 1:6, 1:7, 1:8, 1:9, 1:10, 1:11, or 1:12.

In some embodiments, preferably, the coupling reaction is carried out in water or a buffered salt system and/or an organic solvent.

In some embodiments, preferably, the buffered salt system is selected from PBS, a sodium acetate buffer solution, a histidine-hydrochloride buffer solution, a histidine-acetate buffer solution, a citrate buffer solution, and the above buffer solutions added with sucrose. More preferably, the buffer solution is selected from PBS pH7.4, PBS pH7.2, PBS pH6.5, PBS pH6.0, histidine-acetate pH6.5, histidine-acetate pH6.0, histidine-acetate pH5.5, and combinations thereof with 5%-9% sucrose.

In some embodiments, the selected buffer system is histidine-acetate pH5.5.

In some embodiments, the selected buffer system is PBS pH6.0.

In some embodiments, the organic solvent is selected from N,N-dimethylformamide, N,N-dimethylacetamide, dimethyl sulfoxide, N-methylpyrrolidone, acetonitrile, methanol, ethanol, ethylene glycol, propylene glycol, and glycerol.

Preferably, the method further comprises a step of purifying the conjugation product. Preferably, purification is performed by using one or more of an ultrafiltration system, gel chromatography, affinity chromatography, hydrophobic chromatography, reversed phase chromatography, or ionexchange chromatography. More preferably, purification is performed by using one or more of an ultrafiltration tube, a tangential flow system, a desalting column, a gel column, a cation exchange column, an anion exchange column, or an affinity column.

**In a sixteenth aspect,** the present application provides a pharmaceutical composition, comprising the polypeptide or the polypeptide fragment, the linker, the linker-payload, the conjugate, the antibody or the antigen-binding fragment thereof, the nucleic acid molecule, the recombinant vector, the recombinant cell, and the tautomers, mesomers, racemates, enantiomers, diastereomers, or available salts thereof disclosed above, as well as optionally one or more pharmaceutical excipients.

In some embodiments, preferably, the composition further comprises a pharmaceutically-acceptable carrier and/or excipient; for example, Tween 80, poloxamer 188, sucrose, trehalose, and the like.

**In a seventeenth aspect,** the present application provides use of the polypeptide or the polypeptide fragment, the linker, the linker-payload, the conjugate, the antibody or the antigen-binding fragment thereof, the nucleic acid molecule, the recombinant vector, the recombinant cell, and the tautomers, mesomers, racemates, enantiomers, diastereomers, or available salts thereof disclosed above in the preparation of a medicament for the treatment or prevention of a disease or disorder.

**In an eighteenth aspect,** the present application provides use of any one of the polypeptides or polypeptide fragments, any one of the linkers, any one of the linker-payloads, conjugates, antibodies or antigen-binding fragments thereof, nucleic acid molecules, recombinant vectors, recombinant cells, as well as the tautomers, mesomers, racemates, enantiomers, diastereomers, or available salts thereof in the preparation of a medicament for the treatment/prevention of diseases associated with abnormal cellular activity.

**In a nineteenth aspect,** the present application provides use of any one of the polypeptides or polypeptide fragments, linkers, linker-payloads, conjugates, antibodies or antigen-binding fragments thereof, nucleic acid molecules, recombinant vectors, recombinant cells, as well their tautomers, mesomers, racemates, enantiomers, diastereomers, or available salts thereof in the preparation of a medicament for the treatment/prevention of diseases associated with abnormal cellular activity.

In some embodiments, the medicament is used for treating or preventing a disease or a disorder.

In some embodiments, the medicament is used for treating or preventing diseases associated with abnormal cellular activity.

In some embodiments, the disease or condition comprises a tumor.

In some embodiments, the disease or condition comprises a solid tumor and a hematological malignancy.

**In a twentieth aspect,** the present application provides a method of treating a disease or disorder in a subject in need thereof, comprising administering to the subject an effective amount of the conjugate.

In some embodiments, the tumor is selected from tumors associated with the expression of the following proteins: EGFR, HER2, HER3, c-Met, claudin18.2, TROP-2, folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, and IGF-1R;
preferably, it is selected from tumors associated with Her2 expression;
preferably, it is selected from tumors associated with Trop2 expression;
preferably, it is selected from tumors associated with EGFR expression;
preferably, it is selected from tumors associated with FRα expression;
preferably, it is selected from tumors associated with TF expression;
preferably, it is selected from tumors associated with ROR1 expression; and
preferably, it is selected from tumors associated with GPC3 expression.

In some embodiments, the tumor or the cancer is selected from esophageal cancer, brain cancer, lung cancer, epithelial cell carcinoma, bladder cancer, gastric cancer, ovarian cancer, pancreatic cancer, head and neck cancer, urothelial carcinoma, colon cancer, colorectal cancer, cervical cancer, endometrial cancer, rectal cancer, renal cancer, prostate cancer, melanoma, liver cancer, gallbladder cancer, cholangiocarcinoma, thyroid cancer, non-Hodgkin's lymphoma, multiple myeloma, and B-cell lymphoma..

Preferably, the tumor or the cancer is selected from esophageal cancer, lung cancer, breast cancer, gastric cancer, urothelial carcinoma, cholangiocarcinoma, prostate cancer, colorectal cancer, cervical cancer, and ovarian cancer.

In some embodiments, the conjugate may be administered by any conventional route known in the art, for example, parenteral, intravenous, intramuscular, intra-arterial, intrathecal, intracapsular, intraorbital, intracardiac, intradermal, intraperitoneal, intratracheal, subcutaneous, subepidermal, intra-articular, subcapsular, subarachnoid, intraspinal, epidural, or intrasternal administration.

### Beneficial effects of invention

The present application designs a series of polypeptides that are responsive to tumor tissue-specific MMP-2 and/or MMP-9, as well as to Cathepsin B in tumor cells, and designs spacer structures for linking polypeptides with antibodies and payloads. Hence, the present application can achieve at least one of the following technical effects.
1. The polypeptide linker can be hydrolyzed by both enzymes MMP-2/MMP-9 and Cathepsin B;
2. The ADC prepared with the polypeptide linker can be lysed by the enzyme MMP-2/MMP-9 or Cathepsin B to release the drug;
3. The ADC prepared with the polypeptide linker preferably exhibits better hydrophilicity as compared to ADCs of the same type;
4. The ADC prepared with the polypeptide linker preferably exhibits better stability in culture medium and plasma as compared to ADCs of the same type;
5. The ADC prepared with the polypeptide linker preferably exhibits better cell killing activity as compared to ADCs of the same type; and
6. The ADC prepared with the polypeptide linker preferably exhibits a better tumor inhibition effect in CDX models as compared to ADCs of the same type.

### BRIEF DESCRIPTION OF THE DRAWINGS

FIG.1 shows test results of endocytosis of the anti-TF antibody of the present application in A431 cells over 4 h;
FIG. 2 shows the growth inhibition effect of the h1849-H2L1-ADC-01 of the present application on a human prostate cancer DU145 xenograft model;
FIG. 3 shows the growth inhibition effect of the h1849-H3L3-ADC-01 of the present application on the human prostate cancer DU145 xenograft model;
FIG. 4 shows the growth inhibition effect of the h1849-H3L3-ADC-04 of the present application on the human prostate cancer DU145 xenograft model;
FIG. 5 shows the growth inhibition effect of the h1849-H3L3-ADC-05 of the present application on a human cervical cancer Ca Ski xenograft model; and
FIG. 6 shows the growth inhibition effect of the GC33-ADC of the present application on a human liver cancer HepG2 xenograft model.

### DETAILED DESCRIPTION OF THE EMBODIMENTS

The present application will be further illustrated by the following description of detailed embodiments, which is not a limitation to the present disclosure. Various amendments or improvements may be made by those skilled in the art based on the teachings of the present application without departing from the basic spirit and scope of the present application. Reagents or instruments used herein unspecified with manufacturers are all conventional products that are commercially available.

### Sequences and specific information thereof

| **SEQ ID NO** | **Sequence name** | | **Amino acid sequence** |
|---|---|---|---|
| 1 | | 1-P2 | -His-Val-Leu-Asn-Leu-Val-Cit- |
| 2 | | 1-P3 | -Val-Pro-Leu-Ser-Leu-Val-Cit- |
| 3 | | 1-P4 | -Ile-Pro-Val-Ser-Leu-Val-Cit- |
| 4 | | 1-P5 | -Gly-Pro-Leu-Ser-Leu-Val-Cit- |
| 5 | | 1-P6 | -Gly-Pro-Ala-Ser-Leu-Val-Cit- |
| 6 | | 1-P7 | -Gly-Pro-Ala-Gly-Leu-Val-Cit- |
| 7 | | 1-P8 | -Gly-Pro-Gln-Gly-Leu-Val-Cit- |
| 8 | | 1-P9 | -Gly-Pro-Leu-Gly-Leu-Val-Cit- |
| 9 | | 1-P10 | -Gly-Pro-Ser-Gly-Leu-Val-Cit- |
| 10 | | 1-P11 | -Gly-Pro-Arg-Gly-Leu-Val-Cit- |
| 11 | | 1-P12 | -Gly-Pro-Ala-Ala-Leu-Val-Cit- |
| 12 | | 1-P13 | -Gly-Pro-Ser-Ala-Leu-Val-Cit- |
| 13 | | 1-P14 | -His-Val-Leu-Asn-Leu-Gly-Gly-Phe-Gly- |
| 14 | | 1-P15 | -Val-Pro-Leu-Ser-Leu-Gly-Gly-Phe-Gly- |
| 15 | | 1-P16 | -Ile-Pro-Val-Ser-Leu-Gly-Gly-Phe-Gly- |
| 16 | | 1-P17 | -Gly-Pro-Leu-Ser-Leu-Gly-Gly-Phe-Gly- |
| 17 | | 1-P18 | -Gly-Pro-Ala-Ser-Leu-Gly-Gly-Phe-Gly- |
| 18 | | 1-P19 | -Gly-Pro-Ala-Gly-Leu-Gly-Gly-Phe-Gly- |
| 19 | | 1-P20 | -Gly-Pro-Gln-Gly-Leu-Gly-Gly-Phe-Gly- |
| 20 | | 1-P21 | -Gly-Pro-Leu-Gly-Leu-Gly-Gly-Phe-Gly- |
| 21 | | 1-P22 | -Gly-Pro-Ser-Gly-Leu-Gly-Gly-Phe-Gly- |
| 22 | | 1-P23 | -Gly-Pro-Arg-Gly-Leu-Gly-Gly-Phe-Gly- |
| 23 | | 1-P24 | -Gly-Pro-Ala-Ala-Leu-Gly-Gly-Phe-Gly- |
| 24 | | 1-P25 | -Gly-Pro-Ser-Ala-Leu-Gly-Gly-Phe-Gly- |
| 25 | | 2-P1 | -Pro-Arg-Gly-Leu-Val-Cit- |
| 26 | | 2-P2 | -Pro-Arg-Ser-Leu-Val-Cit- |
| 27 | | 2-P3 | -Pro-Arg-Asn-Leu-Val-Cit- |
| 28 | | 2-P4 | -Pro-Arg-Pro-Leu-Val-Cit- |
| 29 | | 2-P5 | -Pro-Cit-Gly-Leu-Val-Cit- |
| 30 | | 2-P6 | -Pro-Cit-Pro-Leu-Val-Cit- |
| 31 | | 2-P7 | -Gly-Pro-Arg-Ser-Leu-Val-Cit- |
| 32 | | 2-P8 | -Gly-Pro-Arg-Pro-Leu-Val-Cit- |
| 33 | | 2-P9 | -Gly-Pro-Arg-Asn-Leu-Val-Cit- |
| 34 | | 2-P10 | -Gly-Pro-Cit-Gly-Leu-Val-Cit- |
| 35 | | 2-P11 | -Gly-Pro-Cit-Pro-Leu-Val-Cit- |
| 36 | | 2-P12 | -His-Pro-Arg-Gly-Leu-Val-Cit- |
| 37 | | 2-P13 | -His-Pro-Arg-Ser-Leu-Val-Cit- |
| 38 | | 2-P14 | -His-Pro-Arg-Pro-Leu-Val-Cit- |
| 39 | | 2-P15 | -His-Pro-Arg-Asn-Leu-Val-Cit- |
| 40 | | 2-P16 | -His-Pro-Cit-Gly-Leu-Val-Cit- |
| 41 | | 2-P17 | -His-Pro-Cit-Pro-Leu-Val-Cit- |
| 42 | | 2-P18 | -Ser-Pro-Arg-Gly-Leu-Val-Cit- |
| 43 | | 2-P19 | -Ser-Pro-Arg-Ser-Leu-Val-Cit- |
| 44 | | 2-P20 | -Ser-Pro-Arg-Pro-Leu-Val-Cit- |
| 45 | | 2-P21 | -Ser-Pro-Arg-Asn-Leu-Val-Cit- |
| 46 | | 2-P22 | -Ser-Pro-Cit-Gly-Leu-Val-Cit- |
| 47 | | 2-P23 | -Ser-Pro-Cit-Ser-Leu-Val-Cit- |
| 48 | | 2-P24 | -Ser-Pro-Cit-Pro-Leu-Val-Cit- |
| 49 | | 2-P25 | -Gly-Pro-Ala-Pro-Leu-Val-Cit- |
| 50 | | 2-P26 | -Gly-Pro-Ala-Asn-Leu-Val-Cit- |
| 51 | | 2-P27 | -Gly-Pro-Arg-Gly-Dap(Me2)-Val-Cit- |
| 52 | h1849-H2L1 VH | | EVQLVESGGGVVQPGRSLRLSCAASGFTFSDYNMAWIRQAPGK GLEWVAAIIYDGTRTYYRDSVKGRFTISRDNSKSTLYLQMDSLR AEDTAVYYCATGDSYTNFAYWGQGTLVTVSS |
| 53 | h1849-H2L1 VL | | EIVLTQSPTFLSASVGDRVTITCRASSSLSYMHWYQQKPGTSPKL LIYETSKLQSGVPSRFSGSGSGTSFTLTIDSLQPEDFATYYCQQGN SYPRTFGGGTKVEIK |
| 54 | h1849-H3L3 VH | | EVQLVESGGGVVQPGRSLRLSCAASGFTFSDYNMAWIRQAPGK GLEWVAAIIYDGTRTYYRDSVKGRFTISRDNSKSTLYLQMNSLR AEDTAVYYCATGDSYTNFAYWGQGTLVTVSS |
| 55 | h1849-H3L3 VL | | EIVLTQSPTSLSASVGDRVTITCRASSSLSYMHWYQQKPGTSPKL LIYETSKLESGVPSRFSGSGSGTDFTLTIDSLQPEDFATYYCQQGN SYPRTFGGGTKVEIK |
| 56 | h1849-H2L1 LC | | EIVLTQSPTFLSASVGDRVTITCRASSSLSYMHWYQQKPGTSPKL LIYETSKLQSGVPSRFSGSGSGTSFTLTIDSLQPEDFATYYCQQGN SYPRTFGGGTKVEIKRTVAAPSVFIFPPSDEQLKSGTASVVCLLNN FYPREAKVQWKVDNALQSGNSQESVTEQDSKDSTYSLSSTLTLS KADYEKHKVYACEVTHQGLSSPVTKSFNRGEC |
| 57 | h1849-H2L1 HC | | EVQLVESGGGVVQPGRSLRLSCAASGFTFSDYNMAWIRQAPGK GLEWVAAIIYDGTRTYYRDSVKGRFTISRDNSKSTLYLQMDSLR AEDTAVYYCATGDSYTNFAYWGQGTLVTVSSASTKGPSVFPLAP SSKSTSGGTAALGCLVKDYFPEPVTVSWNSGALTSGVHTFPAVL QSSGLYSLSSVVTVPSSSLGTQTYICNVNHKPSNTKVDKKVEPKS CDKTHTCPPCPAPELLGGPSVFLFPPKPKDTLMISRTPEVTCVVV DVSHEDPEVKFNWYVDGVEVHNAKTKPREEQYNSTYRVVSVL TVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPGK |
| 58 | h1849-H3L3 LC | | EIVLTQSPTSLSASVGDRVTITCRASSSLSYMHWYQQKPGTSPKL LIYETSKLESGVPSRFSGSGSGTDFTLTIDSLQPEDFATYYCQQGN SYPRTFGGGTKVEIKRTVAAPSVFIFPPSDEQLKSGTASVVCLLNN FYPREAKVQWKVDNALQSGNSQESVTEQDSKDSTYSLSSTLTLS KADYEKHKVYACEVTHQGLSSPVTKSFNRGEC |
| 59 | h1849-H3L3 HC | | EVQLVESGGGVVQPGRSLRLSCAASGFTFSDYNMAWIRQAPGK GLEWVAAIIYDGTRTYYRDSVKGRFTISRDNSKSTLYLQMNSLR AEDTAVYYCATGDSYTNFAYWGQGTLVTVSSASTKGPSVFPLAP SSKSTSGGTAALGCLVKDYFPEPVTVSWNSGALTSGVHTFPAVL QSSGLYSLSSVVTVPSSSLGTQTYICNVNHKPSNTKVDKKVEPKS CDKTHTCPPCPAPELLGGPSVFLFPPKPKDTLMISRTPEVTCVVV DVSHEDPEVKFNWYVDGVEVHNAKTKPREEQYNSTYRVVSVL TVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPGK |
| 60 | | | -Gly-Pro-Arg-Asn-Dap(Me2)-Val-Cit- |
| 61 | | | -Pro-Leu-Ser-Leu-Val-Cit- |
| 62 | | | -Gly-Pro-Arg-Asn-Leu-Gly-Gly-Phe-Gly- |
| 63 | | | -Pro-Cit-Ser-Leu-Val-Cit- |
| 64 | | | -Gly-Pro-Cit-Asn-Leu-Val-Cit- |
| 65 | | | -Ser-Pro-Cit-Ser-Dap(Me2)-Val-Cit- |
| 66 | | | -Pro-Arg-Ser-Dap(Me2)-Val-Cit- |
| 67 | | | -Pro-Cit-Asn-Leu-Val-Cit- |
| 68 | | | -Pro-Ala-Ser-Leu-Val-Cit- |

### Example 1 Synthesis of polypeptide fragments, linkers, and linker-payloads

### Preparation scheme

The structures of the compounds described in the following examples are determined by nuclear magnetic resonance (¹HNMR) or mass spectrometry (MS). A Bruker AVANCE NEO 400MHz nuclear magnetic resonance spectrometer was used as a determining instrument of the nuclear magnetic resonance (¹H NMR). The solvents for determination were deuterated methanol (CD₃OD), deuterated chloroform (CDCl₃), or hexadeuterated dimethyl sulfoxide (DMSO-d₆); and tetramethylsilane (TMS) was used as an internal standard.

Abbreviations used in the NMR spectra in the examples are shown below. s: singlet, d: doublet, t: triplet, q: quartet, dd: double doublet, qd: quartet doublet, ddd: double double doublet, ddt: double double triplet, dddd: double double double doublet, m: multiplet, br: broad, J: coupling constant, Hz: hertz, and DMSO-*d₆*: deuterated dimethyl sulfoxide. *δ* values are represented by ppm.

A SHIMADZU LCMS-2020 mass spectrometer was used as a determining instrument of MS.

### I. Synthesis of polypeptide fragments and bioactive molecules

### 1.1. Synthesis of polypeptide fragments

### List of polypeptides:

| Polypeptide name | Polypeptide abbreviation | Polypeptide name | Polypeptide abbreviation | Polypeptide name | Polypeptide abbreviation |
|---|---|---|---|---|---|
| 1-P2 | HVLNLVCit | 1-P19 | GPAGLGGFG | 2-P11 | GPCitPLVCit |
| 1-P3 | VPLSLVCit | 1-P20 | GPQGLGGFG | 2-P12 | HPRGLVCit |
| 1-P4 | IPVSLVCit | 1-P21 | GPLGLGGFG | 2-P13 | HPRSLVCit |
| 1-P5 | GPLSLVCit | 1-P22 | GPSGLGGFG | 2-P14 | HPRPLVCit |
| 1-P6 | GPASLVCit | 1-P23 | GPRGLGGFG | 2-P15 | HPRNLVCit |
| 1-P7 | GPAGLVCit | 1-P24 | GPAALGGFG | 2-P16 | HPCitGLVCit |
| 1-P8 | GPQGLVCit | 1-P25 | GPSALGGFG | 2-P17 | HPCitPLVCit |
| 1-P9 | GPLGLVCit | 2-P1 | PRGLVCit | 2-P18 | SPRGLVCit |
| 1-P10 | GPSGLVCit | 2-P2 | PRSLVCit | 2-P19 | SPRSLVCit |
| 1-P11 | GPRGLVCit | 2-P3 | PRNLVCit | 2-P20 | SPRPLVCit |
| 1-P12 | GPAALVCit | 2-P4 | PRPLVCit | 2-P21 | SPRNLVCit |
| 1-P13 | GPSALVCit | 2-P5 | PCitGLVCit | 2-P22 | SPCitGLVCit |
| 1-P14 | HVLNLGGFG | 2-P6 | PCitPLVCit | 2-P23 | SPCitSLVCit |
| 1-P15 | VPLSLGGFG | 2-P7 | GPRSLVCit | 2-P24 | SPCitPLVCit |
| 1-P16 | IPVSLGGFG | 2-P8 | GPRPLVCit | 2-P25 | GPAPLVCit |
| 1-P17 | GPLSLGGFG | 2-P9 | GPRNLVCit | 2-P26 | GPANLVCit |
| 1-P18 | GPASLGGFG | 2-P10 | GPCitGLVCit | 2-P27 | GPRGDap(Me2)VCit |

### 1.1.1: Synthesis of 2-P1

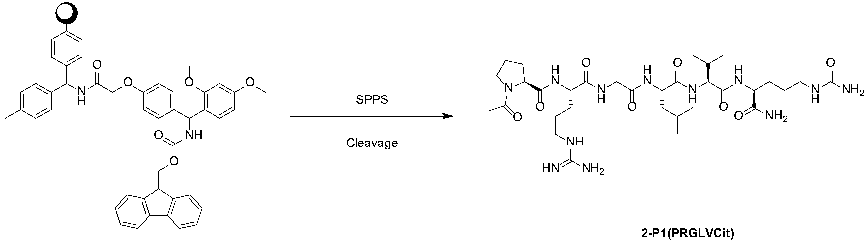

This polypeptide was synthesized by standard Fmoc chemistry method.
1) Resin preparation: DMF was added to a container containing a Rink Amide MBHA resin (concentration: 0.3 mmol/g, 0.1 mmol, 0.30 g) for swelling for 2 h.
2) Deprotection: 20% piperidine DMF (10.0 mL) was added, stirred under N₂ at 25°C for 15 min, washed with DMF (10.0 mL × 5), and filtered to obtain the resin.
3) Coupling: a solution of HBTU (2.85 eq.) and Fmoc-Cit-OH (3.00 eq.) in DMF (10.0 mL) was added to the resin, followed by the addition of DIEA (6.00 eq.), stirred under N₂ at 25°C for 30 min, and the resin was washed with DMF (10.0 mL × 5).
4) The above steps 2 to 3 were repeated to couple amino acids sequentially using the materials and reagents listed in #1-#6 below.

| **#** | **Materials** | **Coupling reagents** |
|---|---|---|
| 1 | Fmoc-Cit-OH (3.00 eq.) | HBTU (2.85 eq.) and DIEA (6.00 eq.) |
| 2 | Fmoc-Val-OH (3.00 eq.) | HBTU (2.85 eq.) and DIEA (6.00 eq.) |
| 3 | Fmoc-Leu-OH (3.00 eq.) | HBTU (2.85 eq.) and DIEA (6.00 eq.) |
| 4 | Fmoc-Gly-OH (3.00 eq.) | HBTU (2.85 eq) and DIEA (6.00 eq.) |
| 5 | Fmoc-Arg(Pbf)-OH (3.00 eq.) | HBTU (2.85 eq) and DIEA (6.00 eq.) |
| 6 | Fmoc-Pro-OH (3.00 eq.) | HBTU (2.85 eq) and DIEA (6.00 eq.) |

5) 5% Ac₂O, 10% NMM, and DMF were added and stirred at room temperature for 30 min.

Polypeptide cleavage and purification:
1) Lysis solution (10.0 mL, 90.0% TFA/2.50% TIS/2.0% H₂O/5.0% DTT) was added to a resin flask at room temperature and stirred for 2.5 h.
2) The peptide was precipitated with cold isopropyl ether (50 mL). Filtration was performed and a filter cake was collected. The filter cake was washed with isopropyl ether (50 mL × 2) and vacuum-dried for 2 h to obtain a crude peptide (0.20 g).
3) The crude peptide was purified by high performance liquid chromatography (HPLC) (purification conditions are shown in the table below) to obtain a final product 2-P1 (PRGLVCit) (7.4 mg, 9.97 µmol, yield: 9.97%, purity: 99.5%, TFA), a white solid.
   ESI-MS (m/z): 739.4 [M+H];

### Purification conditions:

| | |
|---|---|
| **Dissolution conditions** | Dissolve in 10% ACN-20% TFA |
| **Instrument** | Gilson GX-281 |
| **Mobile phase** | A: H₂O (0.075% TFA in H₂O) |
| | B: CH₃CN |
| **Gradient** | 6-30-40 min, Retention time: 33 min |
| **Chromatographic column** | luna, 10 µm, c18, 100A, 25 mm+Gemin, 5 µm, c18, 110A |
| **Flow rate** | 20 mL/Min |
| **Wavelength** | 220/254 nm |
| **Sample injection temperature** | 30°C |

Other polypeptides were synthesized by the above method using the corresponding materials and reagents, as shown in the table below:

| No. | Polypeptide name | Polypeptide abbreviation | Raw material | HPLC analysis method | HPLC retention time/min | ESI-MS (m/z) |
|---|---|---|---|---|---|---|
| 1.1.2 | 1-P2 | HVLNLVCit | Fmoc-Cit-OH, Fmoc-Val-OH, Fmoc-Leu-OH, Fmoc-Asn-OH, Fmoc-Leu-OH, Fmoc-Val-OH, Fmoc-His(Boc)-OH | 20-50-10 min | 4.163 | 892.7 |
| 1.1.3 | 1-P3 | VPLSLVCit | Fmoc-Cit-OH, Fmoc-Val-OH, Fmoc-Leu-OH, Fmoc-Ser(tBu)-OH, Fmoc-Leu-OH, Fmoc-Pro-OH, Fmoc-Val-OH | 20-50-10 min | 6.301 | 825.6 |
| 1.1.4 | 1-P4 | IPVSLVCit | Fmoc-Cit-OH, Fmoc-Val-OH, Fmoc-Leu-OH, Fmoc-Ser(tBu)-OH, Fmoc-Val-OH, Fmoc-Pro-OH, Fmoc-Ile-OH | 20-50-10 min | 5.782 | 825.6 |
| 1.1.5 | 1-P5 | GPLSLVCit | Fmoc-Cit-OH, Fmoc-Val-OH, Fmoc-Leu-OH, Fmoc-Ser(tBu)-OH, Fmoc-Leu-OH, Fmoc-Pro-OH, Fmoc-Gly-OH | 20-50-10 min | 5.721 | 783.6 |
| 1.1.6 | 1-P6 | GPASLVCit | Fmoc-Cit-OH, Fmoc-Val-OH, Fmoc-Leu-OH, Fmoc-Ser(tBu)-OH, Fmoc-Ala-OH, Fmoc-Pro-OH, Fmoc-Gly-OH | 15-45-10 min | 4.216 | 741.6 |
| 1.1.7 | 1-P7 | GPAGLVCit | Fmoc-Cit-OH, Fmoc-Val-OH, Fmoc-Leu-OH, Fmoc-Gly-OH, Fmoc-Ala-OH, Fmoc-Pro-OH, Fmoc-Gly-OH | 10-40-10 min | 5.999 | 711.5 |
| 1.1.8 | 1-P8 | GPQGLVCit | Fmoc-Cit-OH, Fmoc-Val-OH, Fmoc-Leu-OH, Fmoc-Gly-OH, Fmoc-Gln-OH, Fmoc-Pro-OH, Fmoc-Gly-OH | 5-35-10 min | 7.221 | 768.4 |
| 1.1.9 | 1-P9 | GPLGLVCit | Fmoc-Cit-OH, Fmoc-Val-OH, Fmoc-Leu-OH, Fmoc-Gly-OH, Fmoc-Leu-OH, Fmoc-Pro-OH, Fmoc-Gly-OH | 20-50-10 min | 4.764 | 753.5 |
| 1.1.1 0 | 1-P10 | GPSGLVCit | Fmoc-Cit-OH, Fmoc-Val-OH, Fmoc-Leu-OH, Fmoc-Gly-OH, Fmoc-Ser(tBu)-OH, Fmoc-Pro-OH, Fmoc-Gly-OH | 5-35-10 min | 7.039 | 727.5 |
| 1.1.1 1 | 1-P11 | GPRGLVCit | Fmoc-Cit-OH, Fmoc-Val-OH, Fmoc-Leu-OH, Fmoc-Gly-OH, Fmoc-Arg(Boc)₂-OH, Fmoc-Pro-OH, Fmoc-Gly-OH | 5-35-10 min | 7.202 | 796.5 |
| 1.1.1 2 | 1-P12 | GPAALVCit | Fmoc-Cit-OH, Fmoc-Val-OH, Fmoc-Leu-OH, Fmoc-Ala-OH, Fmoc-Ala-OH, Fmoc-Pro-OH, Fmoc-Gly-OH | 15-45-10 min | 4.746 | 725.6 |
| 1.1.1 3 | 1-P13 | GPSALVCit | Fmoc-Cit-OH, Fmoc-Val-OH, Fmoc-Leu-OH, Fmoc-Ala-OH, Fmoc-Ser(tBu)-OH, Fmoc-Pro-OH, Fmoc-Gly-OH | 10-40-10 min | 5.829 | 741.5 |
| 1.1.1 4 | 1-P14 | HVLNLGGFG | Fmoc-Gly-OH, Fmoc-Phe-OH, Fmoc-Gly-Gly-OH, Fmoc-Leu-OH, Fmoc-Asn-OH, Fmoc-Leu-OH, Fmoc-Val-OH, Fmoc-His(Boc)-OH | 20-50-10 min | 5.383 | 954.6 |
| 1.1.1 5 | 1-P15 | VPLSLGGFG | Fmoc-Gly-OH, Fmoc-Phe-OH, Fmoc-Gly-Gly-OH, Fmoc-Leu-OH, Fmoc-Ser(tBu)-OH, Fmoc-Leu-OH, Fmoc-Pro-OH , Fmoc-Val-OH | 25-55-10 min | 5.422 | 887.7 |
| 1.1.1 6 | 1-P16 | IPVSLGGFG | Fmoc-Gly-OH, Fmoc-Phe-OH, Fmoc-Gly-Gly-OH, Fmoc-Leu-OH, Fmoc-Ser(tBu)-OH, Fmoc-Val-OH, Fmoc-Pro-OH, Fmoc-Ile-OH | 25-55-10 min | 4.985 | 887.6 |
| 1.1.1 7 | 1-P17 | GPLSLGGFG | Fmoc-Gly-OH, Fmoc-Phe-OH, Fmoc-Gly-Gly-OH, Fmoc-Leu-OH, Fmoc-Ser(tBu)-OH, Fmoc-Leu-OH, Fmoc-Pro-OH , Fmoc-Gly-OH | 25-55-10 min | 4.162 | 845.5 |
| 1.1.1 8 | 1-P18 | GPASLGGFG | Fmoc-Gly-OH, Fmoc-Phe-OH, Fmoc-Gly-Gly-OH, Fmoc-Leu-OH, Fmoc-Ser(tBu)-OH, Fmoc-Ala-OH , Fmoc-Pro-OH, Fmoc-Gly-OH | 10-40-10 min | 7.493 | 803.5 |
| 1.1.1 9 | 1-P19 | GPAGLGGFG | Fmoc-Gly-OH, Fmoc-Phe-OH, Fmoc-Gly-Gly-OH, Fmoc-Leu-OH, Fmoc-Gly-OH, Fmoc-Ala-OH, Fmoc-Pro-OH, Fmoc-Gly-OH | 20-50-10 min | 3.604 | 773.5 |
| 1.1.2 0 | 1-P20 | GPQGLGGFG | Fmoc-Gly-OH, Fmoc-Phe-OH, Fmoc-Gly-Gly-OH, Fmoc-Leu-OH, Fmoc-Gly-OH, Fmoc-Gln-OH, Fmoc-Pro-OH, Fmoc-Gly-OH | 15-45-10 min | 5.026 | 830.6 |
| 1.1.2 1 | 1-P21 | GPLGLGGFG | Fmoc-Gly-OH, Fmoc-Phe-OH, Fmoc-Gly-Gly-OH, Fmoc-Leu-OH, Fmoc-Gly-OH, Fmoc-Leu-OH, Fmoc-Pro-OH, Fmoc-Gly-OH | 25-55-10 min | 4.164 | 815.5 |
| 1.1.2 2 | 1-P22 | GPSGLGGFG | Fmoc-Gly-OH, Fmoc-Phe-OH, Fmoc-Gly-Gly-OH, Fmoc-Leu-OH, Fmoc-Gly-OH, Fmoc-Ser(tBu)-OH, Fmoc-Pro-OH, Fmoc-Gly-OH | 15-45-10 min | 5.071 | 789.5 |
| 1.1.2 3 | 1-P23 | GPRGLGGFG | Fmoc-Gly-OH, Fmoc-Phe-OH, Fmoc-Gly-Gly-OH, Fmoc-Leu-OH, Fmoc-Gly-OH, Fmoc-Arg(Boc)₂-OH, Fmoc-Pro-OH, Fmoc-Gly-OH | 15-45-10 min | 5.078 | 858.6 |
| 1.1.2 4 | 1-P24 | GPAALGGFG | Fmoc-Gly-OH, Fmoc-Phe-OH, Fmoc-Gly-Gly-OH, Fmoc-Leu-OH, Fmoc-Ala-OH, Fmoc-Ala-OH, Fmoc-Pro-OH, Fmoc-Gly-OH | 20-50-10 min | 4.085 | 787.5 |
| 1.1.2 5 | 1-P25 | GPSALGGFG | Fmoc-Gly-OH, Fmoc-Phe-OH, Fmoc-Gly-Gly-OH, Fmoc-Leu-OH, Fmoc-Ala-OH, Fmoc-Ser(tBu)-OH, Fmoc-Pro-OH, Fmoc-Gly-OH | 20-50-10 min | 3.505 | 803.5 |
| 1.1.2 6 | 2-P2 | PRSLVCit | Fmoc-Cit-OH, Fmoc-Val-OH, Fmoc-Leu-OH, Fmoc-Ser(tBu)-OH, Fmoc-Arg(Boc)₂-OH, Fmoc-Pro-OH | 10-40-10 min | 5.655 | 769.6 |
| 1.1.2 7 | 2-P3 | PRNLVCit | Fmoc-Cit-OH, Fmoc-Val-OH, Fmoc-Leu-OH, Fmoc-Gly-OH, Fmoc-Asn-OH, Fmoc-Arg(Boc)₂-OH, Fmoc-Pro-OH | 10-40-10 min | 5.452 | 796.7 |
| 1.1.2 8 | 2-P4 | PRPLVCit | Fmoc-Cit-OH, Fmoc-Val-OH, Fmoc-Leu-OH, Fmoc-Pro-OH, Fmoc-Arg(Boc)₂-OH, Fmoc-Pro-OH | 10-40-10 min | 5.598 | 779.7 |
| 1.1.2 9 | 2-P5 | PCitGLVCit | Fmoc-Cit-OH, Fmoc-Val-OH, Fmoc-Leu-OH, Fmoc-Gly-OH, Fmoc-Cit-OH, Fmoc-Pro-OH | 10-40-10 min | 6.096 | 740.6 |
| 1.1.3 0 | 2-P6 | PCitPLVCit | Fmoc-Cit-OH, Fmoc-Val-OH, Fmoc-Leu-OH, Fmoc-Pro-OH, Fmoc-Cit-OH, Fmoc-Pro-OH, | 10-40-10 min | 5.524 | 780.6 |
| 1.1.3 1 | 2-P7 | GPRSLVCit | Fmoc-Cit-OH, Fmoc-Val-OH, Fmoc-Leu-OH, Fmoc-Ser(tBu)-OH, Fmoc-Arg(Boc)₂-OH, Fmoc-Pro-OH, Fmoc-Gly-OH | 10-40-10 min | 5.641 | 826.7 |
| 1.1.3 2 | 2-P8 | GPRPLVCit | Fmoc-Cit-OH, Fmoc-Val-OH, Fmoc-Leu-OH, Fmoc-Pro-OH, Fmoc-Arg(Boc)₂-OH, Fmoc-Pro-OH, Fmoc-Gly-OH | 5-35-10 min | 7.292 | 836.7 |
| 1.1.3 3 | 2-P9 | GPRNLVCit | Fmoc-Cit-OH, Fmoc-Val-OH, Fmoc-Leu-OH, Fmoc-Asn-OH, Fmoc-Arg(Boc)₂-OH, Fmoc-Pro-OH, Fmoc-Gly-OH | 10-40-10 min | 5.301 | 853.7 |
| 1.1.3 4 | 2-P10 | GPCitGLVCit | Fmoc-Cit-OH, Fmoc-Val-OH, Fmoc-Leu-OH, Fmoc-Gly-OH, Fmoc-Cit-OH, Fmoc-Pro-OH, Fmoc-Gly-OH | 10-40-10 min | 5.268 | 797.6 |
| 1.1.3 5 | 2-P11 | GPCitPLVCit | Fmoc-Cit-OH, Fmoc-Val-OH, Fmoc-Leu-OH, Fmoc-Pro-OH, Fmoc-Cit-OH, Fmoc-Pro-OH, Fmoc-Gly-OH | 5-35-10 min | 7.048 | 837.7 |
| 1.1.3 6 | 2-P12 | HPRGLVCit | Fmoc-Cit-OH, Fmoc-Val-OH, Fmoc-Leu-OH, Fmoc-Gly-OH, Fmoc-Arg(Boc)₂-OH, Fmoc-Pro-OH, Fmoc-His(Boc)-OH | 5-35-10 min | 6.745 | 876.7 |
| 1.1.3 7 | 2-P13 | HPRSLVCit | Fmoc-Cit-OH, Fmoc-Val-OH, Fmoc-Leu-OH, Fmoc-Ser(tBu)-OH, Fmoc-Arg(Boc)₂-OH, Fmoc-Pro-OH, Fmoc-His(Boc)-OH | 5-35-10 min | 6.724 | 906.7 |
| 1.1.3 8 | 2-P14 | HPRPLVCit | Fmoc-Cit-OH, Fmoc-Val-OH, Fmoc-Leu-OH, Fmoc-Pro-OH, Fmoc-Arg(Boc)₂-OH, Fmoc-Pro-OH, Fmoc-His(Boc)-OH | 5-35-10 min | 6.852 | 916.7 |
| 1.1.3 9 | 2-P15 | HPRNLVCit | Fmoc-Cit-OH, Fmoc-Val-OH, Fmoc-Leu-OH, Fmoc-Asn-OH, Fmoc-Arg(Boc)₂-OH, Fmoc-Pro-OH, Fmoc-His(Boc)-OH | 5-35-10 min | 6.55 | 933.7 |
| 1.1.4 0 | 2-P16 | HPCitGLVCit | Fmoc-Cit-OH, Fmoc-Val-OH, Fmoc-Leu-OH, Fmoc-Gly-OH, Fmoc-Cit-OH, Fmoc-Pro-OH, Fmoc-His(Boc)-OH | 10-40-10 min | 4.71 | 877.7 |
| 1.1.4 1 | 2-P17 | HPCitPLVCit | Fmoc-Cit-OH, Fmoc-Val-OH, Fmoc-Leu-OH, Fmoc-Pro-OH, Fmoc-Cit-OH, Fmoc-Pro-OH, Fmoc-His(Boc)-OH | 10-40-10 min | 4.66 | 917.8 |
| 1.1.4 2 | 2-P18 | SPRGLVCit | Fmoc-Cit-OH, Fmoc-Val-OH, Fmoc-Leu-OH, Fmoc-Gly-OH, Fmoc-Arg(Boc)₂-OH, Fmoc-Pro-OH, Fmoc-Ser(tBu)-OH | 10-40-10 min | 5.229 | 826.7 |
| 1.1.4 3 | 2-P19 | SPRSLVCit | Fmoc-Cit-OH, Fmoc-Val-OH, Fmoc-Leu-OH, Fmoc-Ser(tBu)-OH, Fmoc-Arg(Boc)₂-OH, Fmoc-Pro-OH, Fmoc-Ser(tBu)-OH | 10-40-10 min | 5.347 | 856.8 |
| 1.1.4 4 | 2-P20 | SPRPLVCit | Fmoc-Cit-OH, Fmoc-Val-OH, Fmoc-Leu-OH, Fmoc-Pro-OH, Fmoc-Arg(Boc)₂-OH, Fmoc-Pro-OH, Fmoc-Ser(tBu)-OH | 10-40-10 min | 4.926 | 866.7 |
| 1.1.4 5 | 2-P21 | SPRNLVCit | Fmoc-Cit-OH, Fmoc-Val-OH, Fmoc-Leu-OH, Fmoc-Asn-OH, Fmoc-Arg(Boc)₂-OH, Fmoc-Pro-OH, Fmoc-Ser(tBu)-OH | 10-40-10 min | 5.149 | 883.8 |
| 1.1.4 6 | 2-P22 | SPCitGLVCit | Fmoc-Cit-OH, Fmoc-Val-OH, Fmoc-Leu-OH, Fmoc-Gly-OH, Fmoc-Cit-OH, Fmoc-Pro-OH, Fmoc-Ser(tBu)-OH | 10-40-10 min | 5.123 | 827.7 |
| 1.1.4 7 | 2-P23 | SPCitSLVCit | Fmoc-Cit-OH, Fmoc-Val-OH, Fmoc-Leu-OH, Fmoc-Ser(tBu)-OH, Fmoc-Cit-OH, Fmoc-Pro-OH, Fmoc-Ser(tBu)-OH | 10-40-10 min | 5.602 | 857.8 |
| 1.1.4 8 | 2-P24 | SPCitPLVCit | Fmoc-Cit-OH, Fmoc-Val-OH, Fmoc-Leu-OH, Fmoc-Pro-OH, Fmoc-Cit-OH, Fmoc-Pro-OH, Fmoc-Ser(tBu)-OH | 10-40-10 min | 4.878 | 867.7 |
| 1.1.4 9 | 2-P25 | GPAPLVCit | Fmoc-Cit-OH, Fmoc-Val-OH, Fmoc-Leu-OH, Fmoc-Pro-OH, Fmoc-Ala-OH, Fmoc-Pro-OH, Fmoc-Gly-OH | 10-40-10 min | 5.701 | 751.7 |
| 1.1.5 0 | 2-P26 | GPANLVCit | Fmoc-Cit-OH, Fmoc-Val-OH, Fmoc-Leu-OH, Fmoc-Asn-OH, Fmoc-Ala-OH, Fmoc-Pro-OH, Fmoc-Gly-OH | 5-35-10 min | 7.699 | 768.6 |
| 1.1.5 1 | 2-P27 | GPRGDap(Me 2)Vcit | Fmoc-Cit-OH, Fmoc-Val-OH, Fmoc-Dap(Me)₂-OH, Fmoc-Gly-OH, Fmoc-Arg(Boc)₂-OH, Fmoc-Pro-OH, Fmoc-Gly-OH | 0-30-10 min | 6.239 | 797.7 |

### 1.2. Synthesis of linkers

### 1.2.1. Synthesis of L005

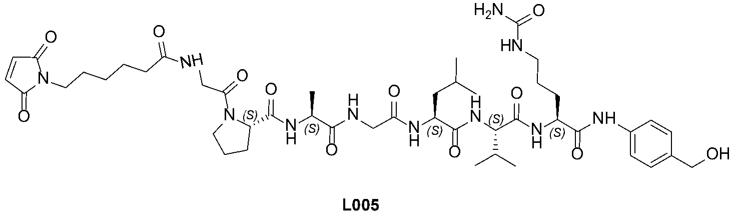

### 1.2.1.1: Synthesis of (S)-2-((S)-2-(S)-2-[(S)-1-(6-(2,5-dioxo-2,5-dihydro-1H-pyrrol-1-yl)hexanoyl)glycyl)pyrrolidin-2-carboxamido)propanamido)acetamido)-4-methylpentanamido)-3-methylbutanamido)-5-ureidovaleric acid (1.2.1.1)

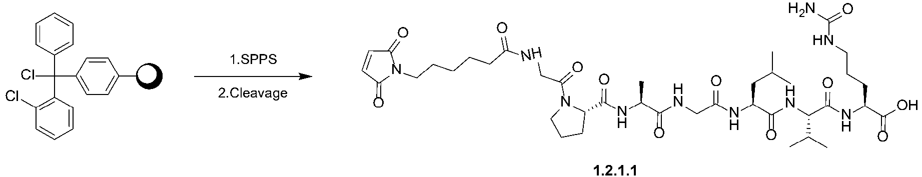

This polypeptide was synthesized using standard Fmoc chemistry.
1) Under nitrogen bubbling, dichloromethane was added to a container containing a CTC resin (2.0 mmol, 0.83 mmol/g, 2.4 g) and Fmoc-Cit-OH (0.8 g, 2.0 mmol/g, 1.0 eq.).
2) Diisopropylethylamine (6.0 eq.) was added dropwise and mixed for 2 h.
3) Methanol (2.4 mL) was added and mixed for 0.5 h.
4) The mixture was drained and washed with DMF 5 times.
5) 20% piperidine/DMF was added and reacted for 0.5 h.
6) The resulting product was drained and washed with DMF 5 times.
7) Fmoc-amino acid solution was added and mixed for 30 s, and then the corresponding coupling reagent was added. The reaction was continued under nitrogen bubbling for 1 h.
8) Steps 4 to 7 were repeated for the next amino acid coupling.

| # | Materials | Coupling reagents |
|---|---|---|
| 1 | Fmoc-Cit-OH (1.0 eq.) | DIEA (6.0 eq.) |
| 2 | Fmoc-Val-OH (3.0 eq.) | HBTU (2.85 eq.) and DIEA (6.0 eq.) |
| 3 | Fmoc-Leu-OH (1.0 eq.) | HBTU (2.85 eq.) and DIEA (6.0 eq.) |
| 4 | Fmoc-Gly-OH (3.0 eq.) | HBTU (2.85 eq.) and DIEA (6.0 eq.) |
| 5 | Fmoc-Ala-OH (3.0 eq.) | HBTU (2.85 eq.) and DIEA (6.0 eq.) |
| 6 | Fmoc-Pro-OH (3.0 eq.) | HBTU (2.85 eq.) and DIEA (6.0 eq.) |
| 7 | Fmoc-Gly-OH (3.0 eq.) | HBTU (2.85 eq.) and DIEA (6.0 eq.) |
| 8 | 6-Maleimidocaproic acid (2.0 eq.) | DIC (2.0 eq.) and HOAT (4.0 eq.) |

DMF containing 20% piperidine was used for Fmoc deprotection for 30 min. The coupling reaction was monitored by a ninhydrin test, and the resin was washed with N,N-dimethylformamide 5 times.

Polypeptide cleavage and purification:
1) A cleavage buffer (20% HFIP/DCM) was added to a flask containing a side chain-protected peptide at room temperature and stirred for 0.5 hour × 3.
2) Filtrate was collected by filtration.
3) The solvent was removed under vacuum.
4) The crude peptide was purified by preparative HPLC (A: 0.075% TFA in H₂O, B: ACN) to obtain a white solid compound 1.2.1.1 (125 mg, 99.2%).

ESI-MS (m/z): 863.4 [M+H].

### 1.2.1.2: Synthesis of (S)-1-((6-(2,5-dioxo-2,5-dihydro-1H-pyrrol-1-yl)hexanoyl)glycyl)-N-((S)-1-((2-(((S)-1-(S)-1-[(S)-1]((4-(hydroxymethyl)phenyl)amino)-1-oxo-5-ureidopent-2-yl)amino)-3-methyl-1-oxobut-2-yl)amino)-4-methyl-1-oxopentan-2-yl)amino)-2-oxoethyl)amino)-1-oxopropan-2-yl)pyrrolidin-2-carboxamide (L005)

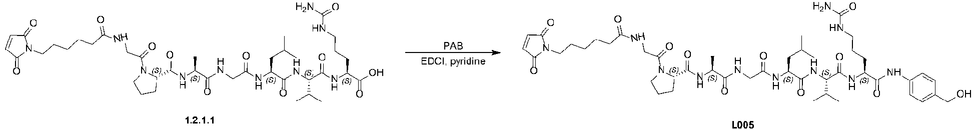

EDCI (44.4 mg, 231 µmol) was added to a pyridine (1 mL) solution of compound 1.2.1.1 (50.0 mg, 57.9 µmol) and (4-aminophenyl)methanol (7.14 mg, 57.9 µmol). The mixture was stirred at 20°C for 1 h. LC-MS monitored completion of the reaction. The reaction solution was added dropwise to isopropyl ether (10 mL × 1) to obtain a solid. The residue was purified by preparative HPLC to obtain a pink solid compound L005 (16.0 mg, 14.8 µmol, yield: 25.7%, purity: 90.1%).

ESI-MS (m/z): 968.3 [M+H]⁺.

### 1.2.2. Synthesis of L006

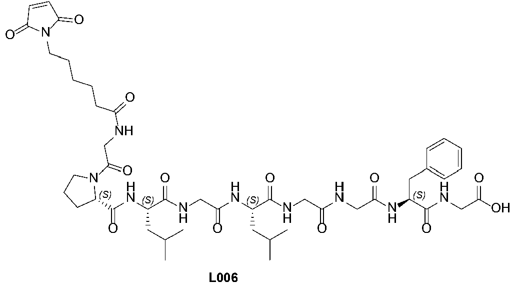

### 1.2.2.1 Synthesis of (6-(2,5-dioxo-2,5-dihydro-1H-pyrrol-1-yl)hexanoyl)glycyl-L-prolyl-L-leucylglycyl-L-leucylglycylglycyl-L-phenylalanyl-glycine (L006)

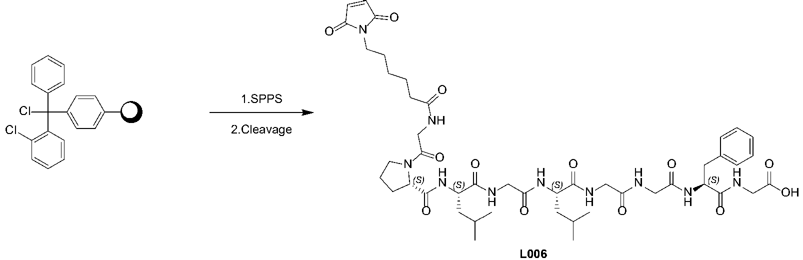

This polypeptide was synthesized by standard Fmoc chemistry method.
1) Dichloromethane was added to a container containing a CTC resin (1.0 mmol, 0.83 mmol/g, 1.2 g) and Fmoc-Gly-OH (0.3 g, 1.0 mmol, 1.0 eq.) under nitrogen bubbling.
2) Diisopropylethylamine (6.0 eq.) was added stepwise and mixed for 2 h.
3) Methanol (1.2 mL) was added and stirred for 0.5 h.
4) The resulting product was drained and washed with DMF 5 times.
5) 20% piperidine/DMF was added and reacted for 0.5 hours.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Gly-OH (1.0 eq.) | DIEA (6.0 eq.) |
| 2 | Fmoc-Phe-OH (3.0 eq.) | HBTU (2.85 eq.) and DIEA (6.0 eq.) |
| 3 | Fmoc-Gly-OH (1.0 eq.) | HBTU (2.85 eq.) and DIEA (6.0 eq.) |
| 4 | Fmoc-Gly-OH (3.0 eq.) | HBTU (2.85 eq.) and DIEA (6.0 eq.) |
| 5 | Fmoc-Leu-OH (3.0 eq.) | HBTU (2.85 eq.) and DIEA (6.0 eq.) |
| 6 | Fmoc-Gly-OH (3.0 eq.) | HBTU (2.85 eq.) and DIEA (6.0 eq.) |
| 7 | Fmoc-Leu-OH (3.0 eq.) | HBTU (2.85 eq.) and DIEA (6.0 eq.) |
| 8 | Fmoc-Pro-OH (3.0 eq.) | HBTU (2.85 eq.) and DIEA (6.0 eq.) |
| 9 | Fmoc-Gly-OH (3.0 eq.) | HBTU (2.85 eq.) and DIEA (6.0 eq.) |
| 10 | 6-Maleimidocaproic acid (2.0 eq.) | DIC (2.0 eq.) and HOAT (4.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) A cleavage buffer (20% HFIP/DCM) was added to a flask containing a side chain-protected peptide, and stirred at room temperature for 0.5 hours × 3.
2) Filtrate was collected by filtration.
3) The solvent was removed under vacuum.
4) The crude peptide was purified by HPLC (A: 0.075% TFA in H₂O, B: ACN) to obtain a white solid compound L006 (134 mg, 99.5%).

ESI-MS (m/z): 967.5 [M+H]⁺.

### 1.2.3. Synthesis of L011

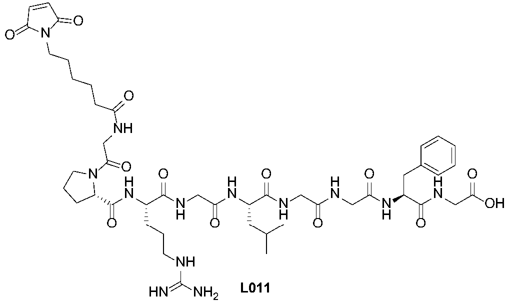

### 1.2.3.1 Synthesis of (6-(2,5-dioxo-2,5-dihydro-1H-pyrrol-1-yl)hexanoyl)glycyl-L-prolyl-L-arginylglycyl-L-leucylglycylglycyl-L-phenylalanyl-glycine (L011)

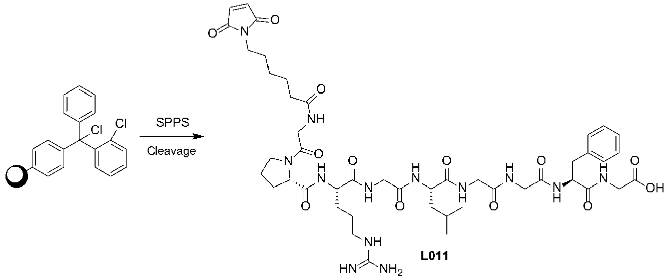

This polypeptide was synthesized by standard Fmoc chemistry method.
1) Dichloromethane was added to a container containing a CTC resin (1.0 mmol, 0.83 mmol/g, 1.2 g) and Fmoc-Gly-OH (1.0 mmol, 1.0 eq.) under nitrogen bubbling.
2) Diisopropylethylamine (6.0 eq.) was added stepwise and mixed for 2 h.
3) Methanol (1.3 mL) was added and stirred for 0.5 h.
4) The resulting product was drained and washed with DMF 3 times.
5) 20% piperidine/DMF was added and reacted for 30 min.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Gly-OH (1.0 eq.) | DIEA (6.0 eq.) |
| 2 | Fmoc-Phe-OH (3.0 eq.) | HBTU (2.85 eq.) and DIEA (6.0 eq.) |
| 3 | Fmoc-Gly-OH (3.0 eq.) | HBTU (2.85 eq.) and DIEA (6.0 eq.) |
| 4 | Fmoc-Gly-OH (3.0 eq.) | HBTU (2.85 eq.) and DIEA (6.0 eq.) |
| 5 | Fmoc-Leu-OH (3.0 eq.) | HBTU (2.85 eq.) and DIEA (6.0 eq.) |
| 6 | Fmoc-Gly-OH (3.0 eq.) | HBTU (2.85 eq.) and DIEA (6.0 eq.) |
| 7 | Fmoc-Arg(Boc)₂-OH (3.0 eq.) | HATU (2.85 eq.) and DIEA (6.0 eq.) |
| 8 | Fmoc-Pro-OH (3.0 eq.) | HBTU (2.85 eq.) and DIEA (6.0 eq.) |
| 9 | Fmoc-Gly-OH (3.0 eq.) | HBTU (2.85 eq.) and DIEA (6.0 eq.) |
| 10 | 6-(2,5-dioxo-2,5-dihydro-1H-pyrrol-1-vl)hexanoic acid (2.0 eq.) | HOAT (2.0 eq.) and DIC (2.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) The resulting peptide resin was washed with methanol 3 times and dried under vacuum.
2) A cleavage buffer (50% TFA/DCM) was added to the peptide resin and stirred for 1 h.
3) DCM and TFA were concentrated under reduced pressure.
4) Vacuum drying was performed for 2 h to obtain the crude peptide (1.0 g).
5) The crude peptide was purified by HPLC (A: 0.075% TFA in H₂O, B: ACN) to obtain a white solid compound L011 (100 mg, purity: 99.1%).

ESI-MS (m/z): 1010.4 [M+H]⁺.

### 1.2.4. Synthesis of L012

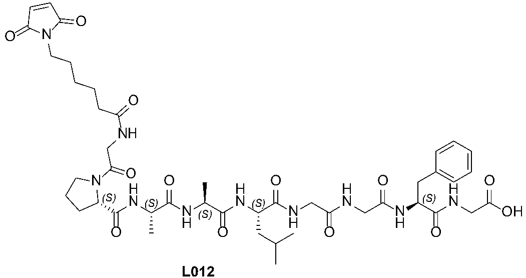

### 1.2.4.1 Synthesis of (6-(2,5-dioxo-2,5-dihydro-1H-pyrrol-1-yl)hexanoyl)glycyl-L-prolyl-L-alanyl-glycyl-L-leucylglycylglycyl-L-phenylalanyl-glycine (L012)

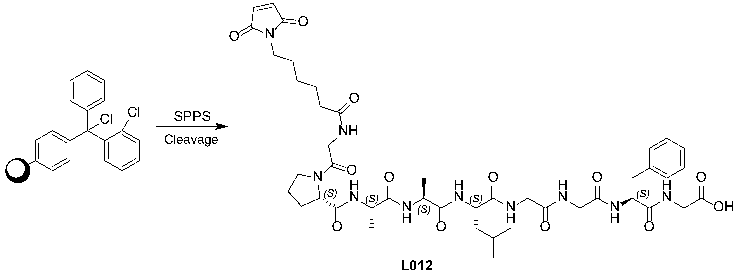

This polypeptide was synthesized by standard Fmoc chemistry method.
1) DCM was added to a container containing a CTC resin (1.0 mmol, 0.83 mmol/g, 1.2 g) and Fmoc-Gly-OH (1.0 mmol, 1.0 eq.) under N₂ bubbling.
2) Diisopropylethylamine (6.0 eq.) was added stepwise and mixed for 2 h.
3) Methanol (1.3 mL) was added and stirred for 0.5 h.
4) The resulting product was drained and washed with DMF 3 times.
5) 20% piperidine/DMF was added and reacted for 30 min.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Gly-OH (1.0 eq.) | DIEA (6.0 eq.) |
| 2 | Fmoc-Phe-OH (3.0 eq.) | HBTU (2.85 eq.) and DIEA (6.0 eq.) |
| 3 | Fmoc-Gly-OH (3.0 eq.) | HBTU (2.85 eq.) and DIEA (6.0 eq.) |
| 4 | Fmoc-Gly-OH (3.0 eq.) | HBTU (2.85 eq.) and DIEA (6.0 eq.) |
| 5 | Fmoc-Leu-OH (3.0 eq.) | HBTU (2.85 eq.) and DIEA (6.0 eq.) |
| 6 | Fmoc-Ala-OH (3.0 eq.) | HBTU (2.85 eq.) and DIEA (6.0 eq.) |
| 7 | Fmoc-Ala-OH (3.0 eq.) | HBTU (2.85 eq.) and DIEA (6.0 eq.) |
| 8 | Fmoc-Pro-OH (3.0 eq.) | HBTU (2.85 eq.) and DIEA (6.0 eq.) |
| 9 | Fmoc-Gly-OH (3.0 eq.) | HBTU (2.85 eq.) and DIEA (6.0 eq.) |
| 10 | 6-(2,5-dioxo-2,5-dihydro-1H-pyrrol-1-yl)hexanoic acid (2.0 eq.) | HOAT (2.0 eq.) and DIC (2.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) The resulting peptide resin was washed with methanol 3 times and dried under vacuum.
2) A cleavage buffer (20% HFIP/DCM) was added to the peptide resin and stirred for 30 min × 3 times.
3) DCM was concentrated under reduced pressure.
4) Vacuum drying was performed for 2 h to obtain the crude peptide (0.9 g).
5) The crude peptide was purified by HPLC (A: 0.075% TFA in H₂O, B: ACN) to obtain a white solid compound L012 (70 mg, 99.8%).

ESI-MS (m/z): 939.4 [M+H]⁺.

Other linkers can be synthesized by the above method using the corresponding raw materials.

### 1.3. Synthesis of linker fragments

### 1.3.1 Synthesis of (S)-2-((S)-4-amino-2-((S)-2-(S)-1-((6-(2,5-dioxo-2,5-dihydro-1H-pyrrol-1-yl)hexanoyl)glycyl)pyrrolidin-2-carboxamido)-5-guanidinopentanamido)-4-oxobutanamido)-3-(dimethylamino)propanoic acid (1.3.1)

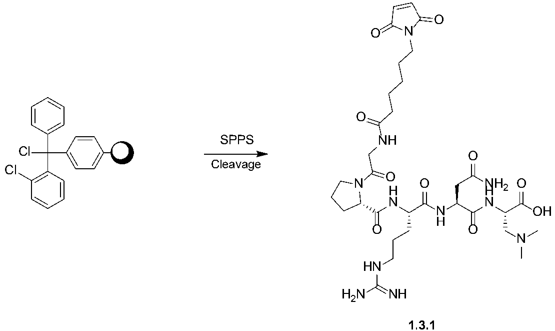

This polypeptide was synthesized by standard Fmoc chemistry method.
1) Under nitrogen bubbling, DCM was added to a container containing a CTC resin (1.0 mmol, 1.6 g, Sub=0.64 mmol/g) and (S)-2-((((9H-fluoren-9-yl)methoxy)carbonyl)amino)-3-(dimethylamino)propanoic acid (0.35 g, 1.0 mmol, 1 eq.).
2) DIEA (4.0 eq.) was added dropwise and mixed for 2 h.
3) Methanol (1.5 mL) was added and mixed for 30 min.
4) The mixture was drained and washed with DMF 5 times.
5) 20% piperidine/DMF was added for reaction for 30 min.
6) The resulting product was drained and washed with DMF 3 times.
7) Fmoc amino acid solution was added and mixed for 30 s, and then the corresponding coupling reagent was added, and subjected to N₂ bubbling for about 1 h.
8) Steps 5 to 7 were repeated for the next amino acid coupling.

| # | Materials | Coupling reagents |
|---|---|---|
| 1 | (S)-2-((((9H-fluoren-9-yl)methoxy)carbonyl)amino)-3-(dimethylamino)propanoic acid (1.0 *eq.*) | DIEA (4.0 *eq.*) |
| 2 | Fmoc-Asn(Trt)-OH (3.0 *eq*.) | HATU (1.90 *eq*.) and DIEA (4.0 *eq*.) |
| 3 | Fmoc-Arg(Pbf)-OH (3.0 *eq.)* | HATU (2.85 *eq.*) and DIEA (6.0 *eq.*) |
| 4 | Fmoc-Pro-OH (3.0 *eq.*) | HATU (2.85 *eq*.) and DIEA (6.0 *eq.*) |
| 5 | Fmoc-Gly-OH (3.0 *eq.*) | HATU (2.85 *eq*.) and DIEA (6.0 *eq.*) |
| 6 | MAL (2.0 *eq*.) | DIC (2.0 *eq*.) and HOAT (4 *eq.*) |

Fmoc deprotection was performed using DMF solution containing 20% piperidine for 30 min. The coupling reaction was monitored by the ninhydrin test, and the resin was washed with DMF for 5 times.

Polypeptide cleavage and purification:
1) The above obtained peptide resin was washed with MeOH three times and dried under vacuum.
2) A cleavage buffer (20% HFIP/DCM) was added to the peptide resin, stirred for 0.5 h three times.
3) DCM was concentrated under reduced pressure.
4) The peptide was dried under vacuum for 2 hours to obtain a compound 1.3.1 (55 mg, 87.79%).

ESI-MS (m/z): 750.3[M+H]⁺.

Other linker fragments were synthesized using the above method with the following raw materials, as shown in the table below:

| Linker fragment | Linker fragment composition | Raw material | HPLC analysis method | HPLC retention time | ESI-MS(m/z) |
|---|---|---|---|---|---|
| 1.3.2 | MC-PRNL | Fmoc-Leu-OH, Fmoc-Asn(Trt)-OH, Fmoc-Arg(Pbf)-OH, Fmoc-Pro-OH, 6-maleimidohexanoic acid N-hydroxylsuccinimide ester | 10-80-20 min | 7.966 | 692.5 |
| 1.3.3 | MC-GPRNL | Fmoc-Leu-OH, Fmoc-Asn(Trt)-OH, Fmoc-Arg(Pbf)-OH, Fmoc-Pro-OH, Fmoc-Gly-OH, 6-maleimidohexanoic acid N-hydroxylsuccinimide ester | 10-80-6 min | 1.196 | 749.4 |
| 1.3.4 | MC-SPRNL | Fmoc-Leu-OH, Fmoc-Asn(Trt)-OH, Fmoc-Arg(Pbf)-OH, Fmoc-Pro-OH, Fmoc-Ser(tBu)-OH, 6-maleimidohexanoic acid N-hydroxylsuccinimide ester | 0-60-20 min | 11.307 | 779.5 |
| 1.3.5 | MC-GPLSL | Fmoc-Leu-OH, Fmoc-Ser(tBu)-OH, Fmoc-Leu-OH, Fmoc-Pro-OH, Fmoc-Gly-OH, 6-maleimidohexanoic acid N-hydroxylsuccinimide ester | 10-80-15 min | 8.122 | 679.3 |
| 1.3.6 | MC-PRGL | Fmoc-Leu-OH, Fmoc-Gly-OH, Fmoc-Arg(Pbf)-OH, Fmoc-Pro-OH, 6-maleimidohexanoic acid N-hydroxylsuccinimide ester | 10-80-25 min | 8.074 | 635.3 |
| 1.3.7 | MC-PRPL | Fmoc-Leu-OH, Fmoc-Pro-OH, Fmoc-Arg(Pbf)-OH, Fmoc-Pro-OH, 6-maleimidohexanoic acid N-hydroxylsuccinimide ester | 10-80-25 min | 8.033 | 675.3 |
| 1.3.8 | MC-GPRSL | Fmoc-Leu-OH, Fmoc-Ser(tBu)-OH, Fmoc-Arg(Pbf)-OH, Fmoc-Pro-OH, Fmoc-Gly-OH, 6-maleimidohexanoic acid N-hydroxylsuccinimide ester | 10-80-25 min | 7.172 | 722.3 |
| 1.3.9 | MC-GPRPL | Fmoc-Leu-OH, Fmoc-Pro-OH, Fmoc-Arg(Pbf)-OH, Fmoc-Pro-OH, Fmoc-Gly-OH, 6-maleimidohexanoic acid N-hvdroxvlsuccinimide ester | 10-80-15 min | 6.571 | 732.3 |
| 1.3.10 | MC-GPCitPL | Fmoc-Leu-OH, Fmoc-Pro-OH, Fmoc-Cit-OH, Fmoc-Pro-OH, Fmoc-Gly-OH, 6-maleimidohexanoic acid N-hvdroxvlsuccinimide ester | 10-80-15 min | 6.653 | 733.3 |
| 1.3.11 | MC-HPRGL | Fmoc-Leu-OH, Fmoc-Gly-OH, Fmoc-Arg(Pbf)-OH, Fmoc-Pro-OH, Fmoc-His(Trt)-OH, 6-maleimidohexanoic acid N-hydroxylsuccinimide ester | 40-100-20 min | 7.168 | 1266.7 |
| 1.3.12 | MC-HPCitPL | Fmoc-Leu-OH, Fmoc-Pro-OH, Fmoc-Cit-OH, Fmoc-Pro-OH, Fmoc-His(Trt)-OH, 6-maleimidohexanoic acid N-hydroxylsuccinimide ester | 30-60-20 min | 10.052 | 1055.2 |
| 1.3.13 | MC-SPCitGL | Fmoc-Leu-OH, Fmoc-Gly-OH, Fmoc-Cit-OH, Fmoc-Pro-OH, Fmoc-Ser(tBu)-OH, 6-maleimidohexanoic acid N-hydroxylsuccinimide ester | 10-80-25 min | 8.223 | 723.2 |
| 1.3.14 | MC-GPAPL | Fmoc-Leu-OH, Fmoc-Pro-OH, Fmoc-Ala-OH, Fmoc-Pro-OH. Fmoc-Gly-OH, 6-maleimidohexanoic acid N-hydroxylsuccinimide ester | 10-80-15 min | 7.116 | 647.3 |
| 1.3.15 | MC-PLSL | Fmoc-Leu-OH, Fmoc-Ser(tBu)-OH, Fmoc-Leu-OH, Fmoc-Pro-OH, 6-maleimidohexanoic acid N-hydroxylsuccinimide ester | 35-65-20 min | 7.039 | 622.4 |
| 1.3.16 | 6-(2-(methylsulf onyl)pyrimi din-5-yl)-5-hexynoic acid - GPRNL | Fmoc-Leu-OH, Fmoc-Asn(Trt)-OH, Fmoc-Arg(Pbf)-OH, Fmoc-Pro-OH, Fmoc-Gly-OH, 6-(2-(methylsulfonyl)pyrimidin-5-yl)-5-hexynoic acid | 15-45-20 min | 8.482 | 806.5 |
| 1.3.17 | Fmoc-GPRNL | Fmoc-Leu-OH, Fmoc-Asn(Trt)-OH, Fmoc-Arg(Pbf)-OH, Fmoc-Pro-OH, Fmoc-Gly-OH | 10-80-20 min | 10.948 | 778.5 |
| 1.3.18 | 1-tert-Butyl 7-(2,5-dioxopyrroli din-1-yl) heptanedioa te GPRNL | Fmoc-Leu-OH, Fmoc-Asn(Trt)-OH, Fmoc-Arg(Pbf)-OH, Fmoc-Pro-OH, Fmoc-Gly-OH, 1-tert-Butyl 7-(2,5-dioxopyrrolidin-1-yl) heptanedioate | 40-100-20 min | 13.947 | 1248.8 |
| 1.3.19 | MC-PRSL | Fmoc-Leu-OH, Fmoc-Ser(tBu)-OH, Fmoc-Arg(Pbf)-OH, Fmoc-Pro-OH, 6-maleimidohexanoic acid N-hydroxylsuccinimide ester | 10-80-25 min | 7.925 | 665.3 |
| 1.3.20 | MC-SPRSL | Fmoc-Leu-OH, Fmoc-Ser(tBu)-OH, Fmoc-Arg(Pbf)-OH, Fmoc-Pro-OH, Fmoc-Ser(tBu)-OH, 6-maleimidohexanoic acid N-hydroxylsuccinimide ester | 0-60-20 min | 11.401 | 752.4 |
| 1.3.21 | MC-PCitSL | Fmoc-Leu-OH, Fmoc-Ser(tBu)-OH, Fmoc-Cit-OH, Fmoc-Pro-OH, 6-maleimidohexanoic acid N-hydroxylsuccinimide ester | 0-60-20 min | 13.332 | 666.4 |
| 1.3.22 | MC-PCitPL | Fmoc-Leu-OH, Fmoc-Pro-OH, Fmoc-Cit-OH, Fmoc-Pro-OH, 6-maleimidohexanoic acid N-hvdroxvlsuccinimide ester | 0-60-20 min | 13.451 | 676.4 |
| 1.3.23 | MC-PCitGL | Fmoc-Leu-OH, Fmoc-Gly-OH, Fmoc-Cit-OH, Fmoc-Pro-OH, 6-maleimidohexanoic acid N-hydroxylsuccinimide ester | 0-60-20 min | 13.500 | 636.3 |
| 1.3.24 | MC-GPCitGL | Fmoc-Leu-OH, Fmoc-Gly-OH, Fmoc-Cit-OH, Fmoc-Pro-OH, Fmoc-Gly-OH, 6-maleimidohexanoic acid N-hydroxylsuccinimide ester | 0-60-20 min | 12.629 | 693.3 |
| 1.3.25 | MC-SPRGL | Fmoc-Leu-OH, Fmoc-Gly-OH, Fmoc-Arg(Pbf)-OH, Fmoc-Pro-OH, Fmoc-Ser(tBu)-OH, 6-maleimidohexanoic acid N-hvdroxvlsuccinimide ester | 0-60-20 min | 11.593 | 722.4 |
| 1.3.26 | MC-SPRPL | Fmoc-Leu-OH, Fmoc-Pro-OH, Fmoc-Arg(Pbf)-OH, Fmoc-Pro-OH, Fmoc-Ser(tBu)-OH, 6-maleimidohexanoic acid N-hydroxylsuccinimide ester | 0-60-20 min | 11.474 | 762.5 |
| 1.3.27 | MC-SPCitPL | Fmoc-Leu-OH, Fmoc-Pro-OH, Fmoc-Cit-OH, Fmoc-Pro-OH, Fmoc-Ser(tBu)-OH, 6-maleimidohexanoic acid N-hydroxylsuccinimide ester | 0-60-20 min | 12.512 | 763.4 |
| 1.3.28 | MC-GPANL | Fmoc-Leu-OH, Fmoc-Asn(Trt)-OH, Fmoc-Ala-OH, Fmoc-Pro-OH, Fmoc-Gly-OH, 6-maleimidohexanoic acid N-hydroxylsuccinimide ester | 0-60-20 min | 12.932 | 664.4 |
| 1.3.29 | MC-GPCitNL | Fmoc-Leu-OH, Fmoc-Asn(Trt)-OH, Fmoc-Cit-OH, Fmoc-Pro-OH, Fmoc-Gly-OH, 6-maleimidohexanoic acid N-hvdroxvlsuccinimide ester | 20-50-20 min | 6.581 | 750.5 |
| 1.3.30 | MC-SPCitS | Fmoc-Ser(tBu)-OH, Fmoc-Cit-OH, Fmoc-Pro-OH, Fmoc-Ser(tBu)-OH, 6-maleimidohexanoic acid N-hydroxylsuccinimide ester | 0-60-20 min | 9.470 | 640.4 |
| 1.3.31 | MC-PRS | Fmoc-Ser(tBu)-OH, Fmoc-Arg(Pbf)-OH, Fmoc-Pro-OH, 6-maleimidohexanoic acid N-hydroxylsuccinimide ester | 0-60-20 min | 9.598 | 552.4 |
| 1.3.32 | MC-PCitNL | Fmoc-Leu-OH, Fmoc-Asn(Trt)-OH, Fmoc-Cit-OH, Fmoc-Pro-OH, 6-maleimidohexanoic acid N-hydroxylsuccinimide ester | 0-60-20 min | 12.629 | 693.3 |
| 1.3.33 | MC-PASL | Fmoc-Leu-OH, Fmoc-Ser(tBu)-OH, Fmoc-Ala-OH, Fmoc-Pro-OH, 6-maleimidohexanoic acid N-hydroxylsuccinimide ester | 25-55-20 min | 7.374 | 580.4 |

### 1.4. Synthesis of linker-payload intermediates

### List of linker-payloads:

| Compound No. | Intermediate composition | Compound structure |
|---|---|---|
| L005-P005 | MC-GPAGLVCit-PAB-DX8951 | 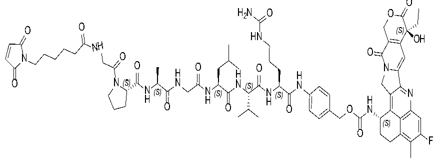 |
| L005-P002 | MC-GPAGLVCit-PAB-MMAE | 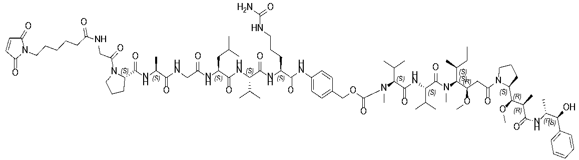 |
| L005-P003 | MC-GPAGLVCit-PAB-ET743 | 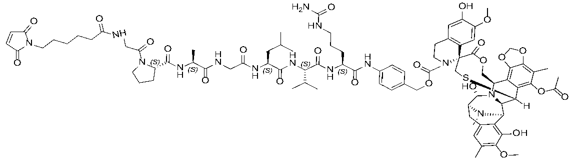 |
| L006-P005 | MC-GPLGLGGFG-DX8951 | 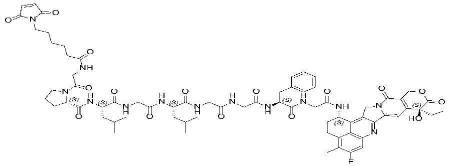 |
| L008-P005 | MC-GPASLVCit-PAB-DX8951 | 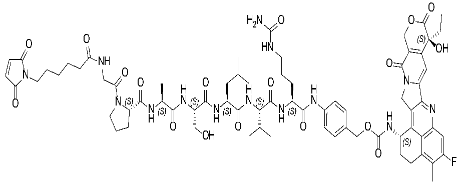 |
| L009-P005 | MC-GPRGLVCit-PAB-DX8951 | 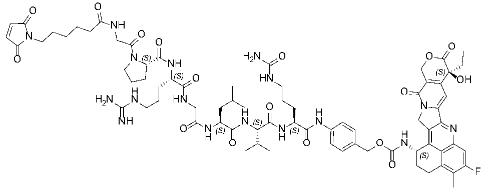 |
| L009-P002 | MC-GPRGLVCit-PAB-MMAE | 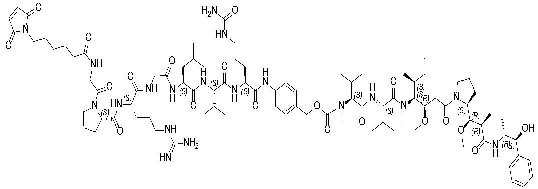 |
| L010-P005 | MC-GPSALVCit-PAB-DX8951 | 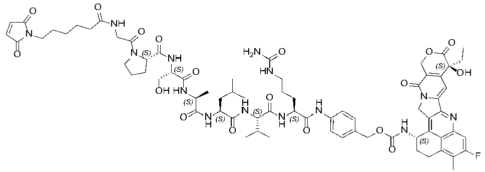 |
| L011-P005 | MC-GPRGLGGFG-DX8951 | 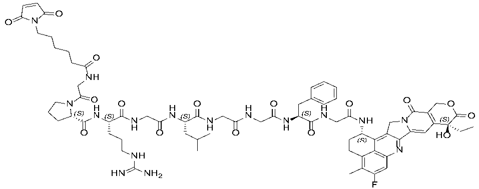 |
| L012-P005 | MC-GPAALGGFG-DX8951 | 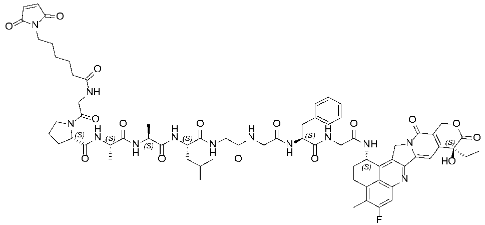 |
| L013-P005 | MC-PRNLVCit-PAB-DX8951 | 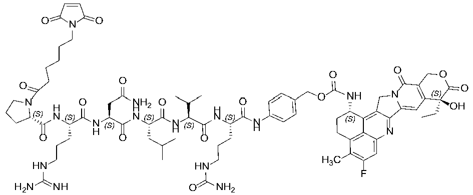 |
| L013-P002 | MC-PRNLVCit-PAB-MMAE | 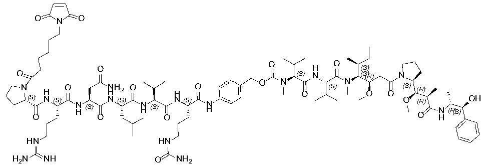 |
| L015-P005 | MC-GPRNLVCit-PAB-DX8951 | 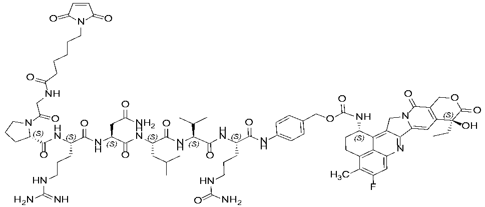 |
| L015-P002 | MC-GPRNLVCit-PAB-MMAE | 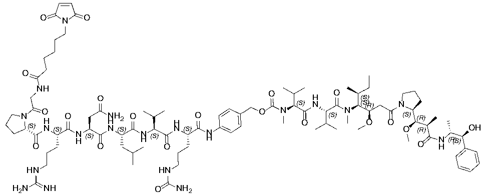 |
| L015-P003 | MC-GPRNLVCit-PAB-ET743 | 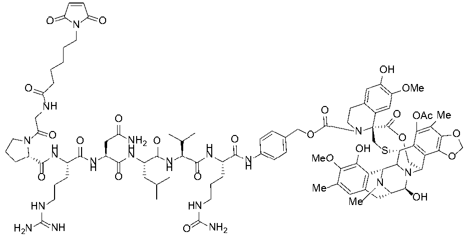 |
| L016-P005 | MC-GPRNDap(Me2)VCit-PAB-DX8951 | 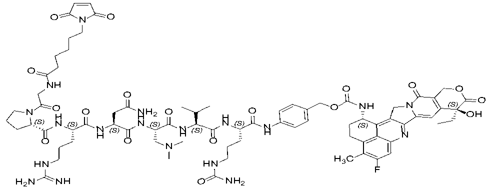 |
| L016-P002 | MC-GPRNDap(Me2)VCit-PAB-MMAE | 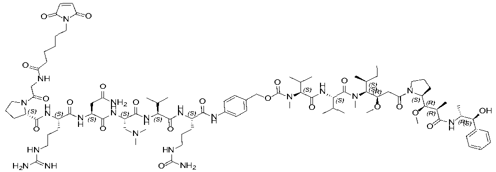 |
| L017-P005 | MC-SPRNLVCit-PAB-DX8951 | 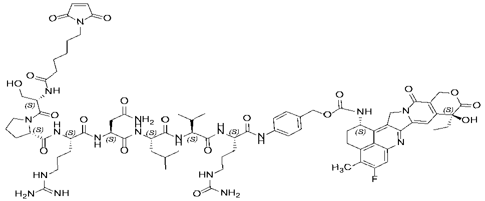 |
| L017-P002 | MC-SPRNLVCit-PAB-MMAE | 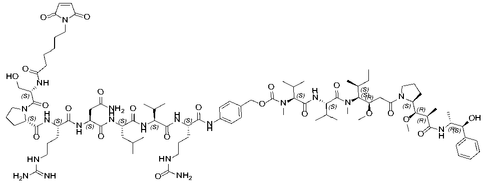 |
| L021-P005 | MC-GPLSLVCit-PAB-DX8951 | 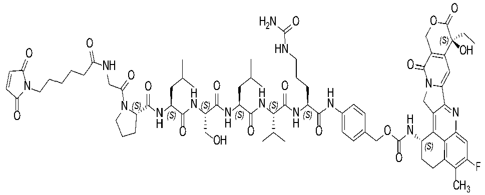 |
| L021-P002 | MC-GPLSLVCit-PAB-MMAE | 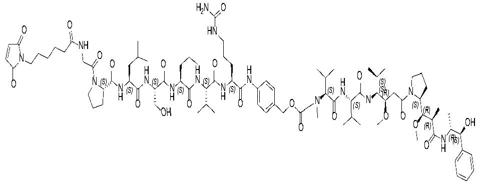 |
| L027-P005 | MC-PRGLVCit-PAB-DX8951 | 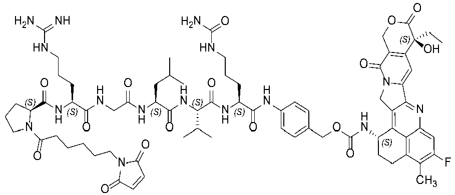 |
| L028-P005 | MC-PRPLVCit-PAB-DX8951 | 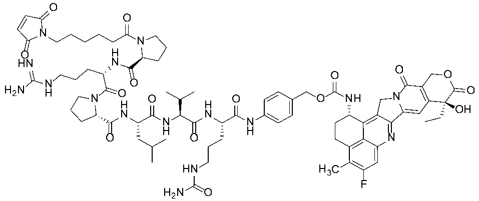 |
| L029-P005 | MC-GPRSLVCit-PAB-DX8951 | 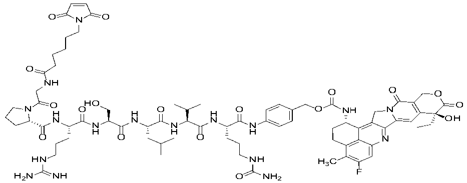 |
| L030-P005 | MC-GPRPLVCit-PAB-DX8951 | 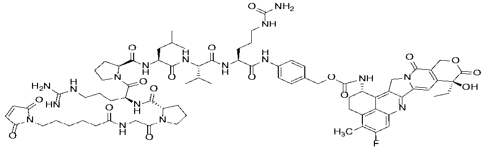 |
| L031-P005 | MC-GPCitPLVCit-PAB-DX8951 | 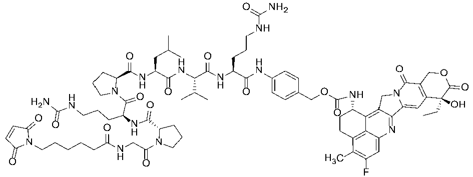 |
| L032-P005 | MC-HPRGLVCit-PAB-DX8951 | 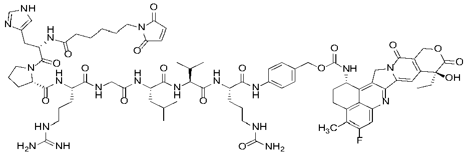 |
| L033-P005 | MC-HPCitPLVCit-PAB-DX8951 | 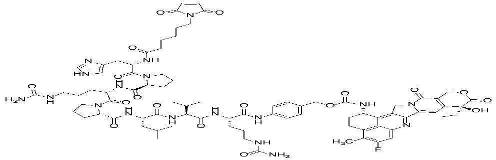 |
| L034-P005 | MC-SPCitGLVCit-PAB-DX8951 | 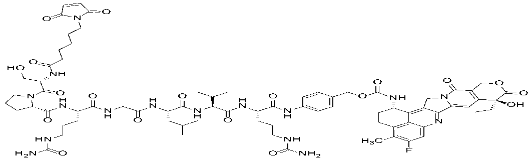 |
| L035-P005 | MC-GPAPLVCit-PAB-DX8951 | 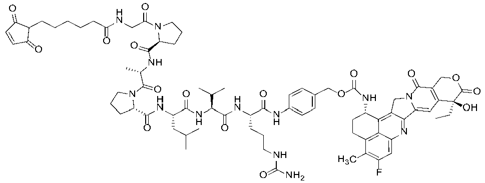 |
| L037-P005 | MC-PLSLVCit-PAB-DX8951 | 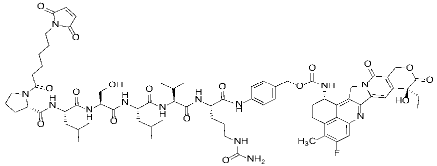 |
| L038-P005 | MC-SPCitSLVCit-PAB-DX8951 | 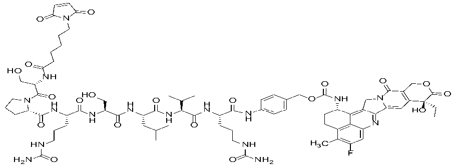 |
| L038-P002 | MC-SPCitSLVCit-PAB-MMAE | 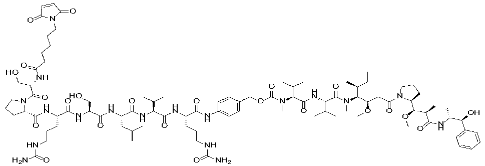 |
| L038-P003 | MC-SPCitSLVCit-PAB-ET743 | 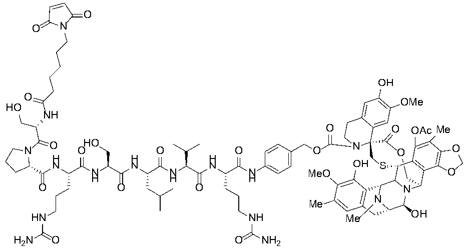 |
| L039-P005 | MeO2S-Pym-GPRNLVCit-PAB-DX8951 | 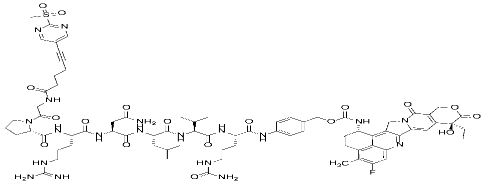 |
| L040-P005 | DBCO-GPRNLVCit-PAB-DX8951 | 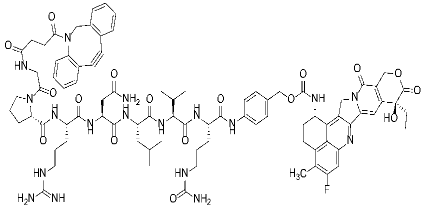 |
| L041-P005 | NHS-GPRNLVCit-PAB-DX8951 | 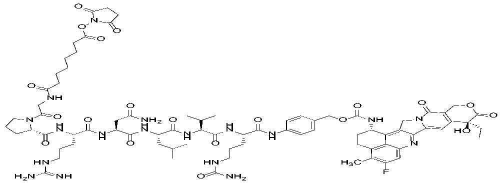 |
| L042-P005 | Hydrazide-GPRNLVCit-PAB-DX8951 | 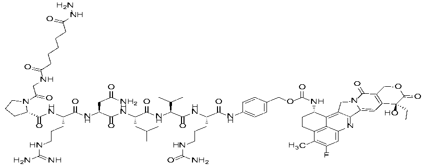 |
| L043-P005 | Mal-PEG8-GPRNLVCit-PAB-DX8951 | 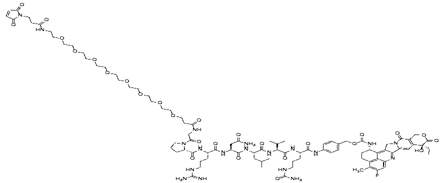 |
| L045-P006 | MC-GPRNLVCit-PAB-DMEDA-SN38 | 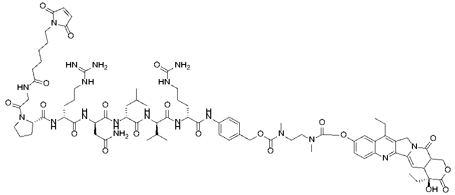 |
| L047-P001 | MC-GPRNLGGFG-Dxd | 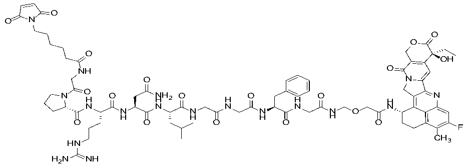 |
| L048-P001 | MC-GPRNLGGFG-NMEDA-Dxd | 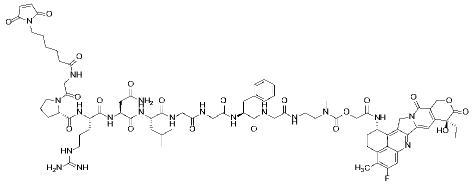 |
| L049-P005 | MC-PRSLVCit-PAB-DX8951 | 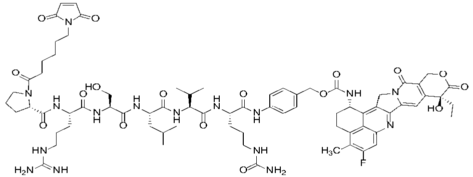 |
| L050-P005 | MC-SPRSLVCit-PAB-DX8951 | 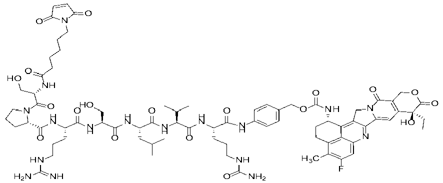 |
| L051-P005 | MC-PCitSLVCit-PAB-DX8951 | 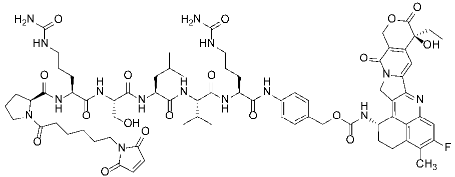 |
| L052-P005 | MC-PCitPLVCit-PAB-DX8951 | 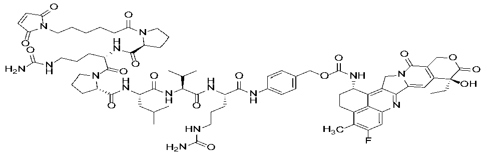 |
| L053-P005 | MC-PCitGLVCit-PAB-DX8951 | 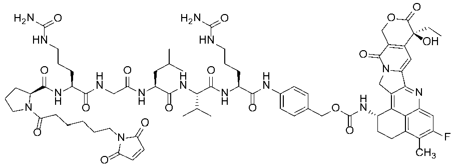 |
| L054-P005 | MC-GPCitGLVCit-PAB-DX8951 | 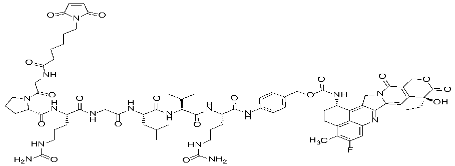 |
| L055-P005 | MC-SPRGLVCit-PAB-DX8951 | 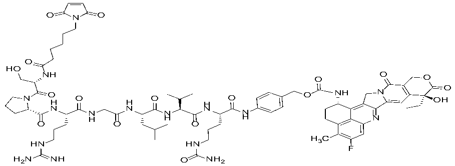 |
| L056-P005 | MC-SPRPLVCit-PAB-DX8951 | 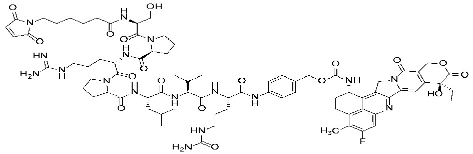 |
| L057-P005 | MC-SPCitPLVCit-PAB-DX8951 | 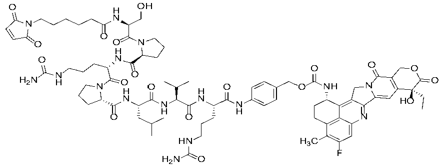 |
| L058-P005 | MC-GPANLVCit-PAB-DX8951 | 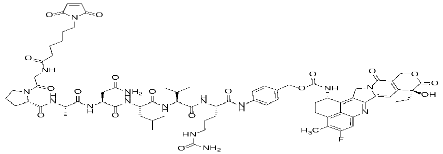 |
| L059-P005 | MC-GPCitNLVCit-PAB-DX8951 | 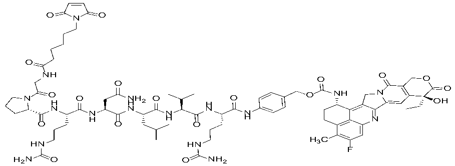 |
| L060-P005 | MC-SPCitSDap(Me2)VCit-PAB-DX8951 | 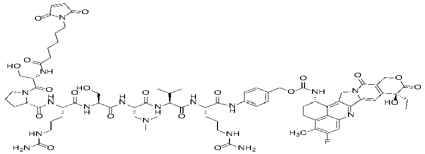 |
| L060-P002 | MC-SPCitSDap(Me2)VCit-PAB-MMAE | 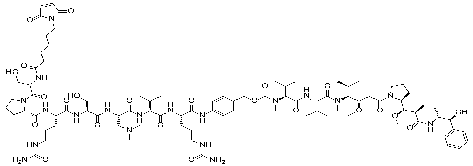 |
| L062-P005 | MC-PRSDap(Me2)VCit-PAB-DX8951 | 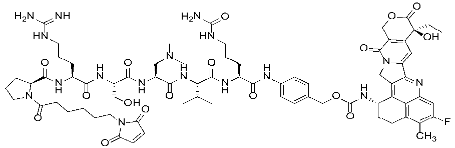 |
| L062-P002 | MC-PRSDap(Me2)VCit-PAB-MMAE | 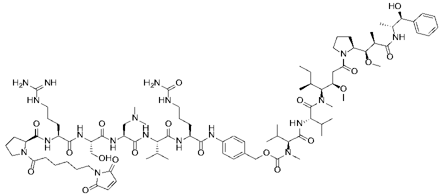 |
| L064-P005 | MC-PCitNLVCit-PAB-DX8951 | 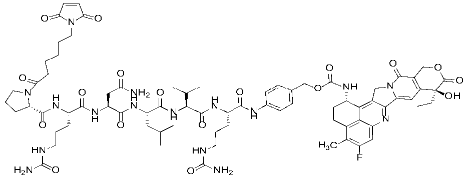 |
| L065-P005 | MC-PASLVCit-PAB-DX8951 | 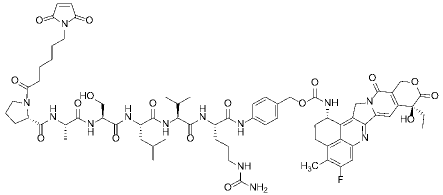 |

### 1.4.1. Synthesis of L005-P005

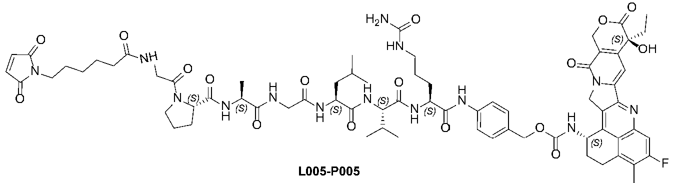

### 1.4.1.1: Synthesis of 4-((3S,9S,12S,15S)-1-((S)-1-((6-(2,5-dioxo-2,5-dihydro-1H-pyrrolidin-1-yl)hexanoyl)pyrrolidin-2-yl)-9-isobutyl-12-isopropyl-3-methyl-1,4,7,10,13-pentaoxo-15-(3-ureidopropyl)-2,5,8,11,14-pentaazahexadecan-16-amino)benzyl(4-nitrophenyl)carbonate (1.4.1.1)

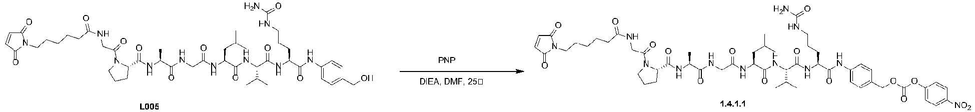

DIEA (8.54 mg, 66.1 µmol, 11.5 µL) was added to a DMF (200 µL) solution of compound L005 (16.0 mg, 16.5 µmol) and bis(4-nitrophenyl)carbonate (15.1 mg, 49.6 µmol). Stirring was carried out at 25°C for 4 h. LC-MS monitored completion of the reaction. The reaction solution was dropped into isopropyl ether (5 mL × 1) to obtain a solid. The crude product compound 1.4.1.1 (16.0 mg) was a red solid, which was used directly in the next step without further purification.

ESI-MS (m/z): 1133.3 [M+H]⁺.

### 1.4.1.2: Synthesis of 4-((3S,9S,12S,15S)-1-((S)-1-)((6-(2,5-dioxo-2,5-dihydro-1H-pyrrol-1-yl)hexanoyl)glycyl)pyrrolidin-2-yl)-9-isobutyl-12-isopropyl-3-methyl-1,4,7,10,13-pentaoxo-15-(3-ureidopropyl)-2,5,8,11,14-pentaazahexadecan-16-amino)benzyl(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L005-P005)

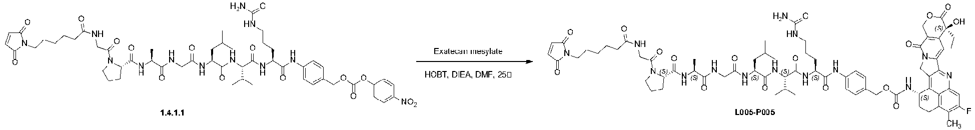

Compound 1.4.1.1 (16.0 mg) and Exatecan mesylate(6.00 mg, 11.3 µmol) were dissolved in DMF (200 µL), and then HOBt (2.29 mg, 16.9 µmol) and DIEA (7.30 mg, 56.5 µmol, 9.84 µL) were added thereto, respectively. Stirring was carried out at 25°C for 2 h. LC-MS monitored completion of the reaction. Preparation and purification were performed by HPLC. A yellow solid compound L005-P005 (6.30 mg, 4.10 µmol, yield: 30.9%, purity: 99.0%) was obtained. ESI-MS (m/z): 1430.3 [M+H]⁺.

### 1.4.2 Synthesis of L005-P002

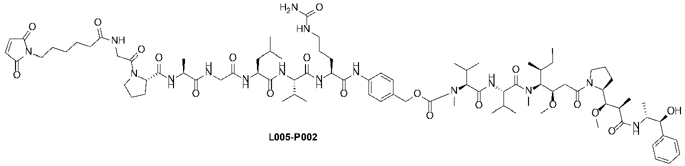

### 1.4.2.1: Synthesis of ethyl (3-ureidopropyl)-2,5,8,11,14-pentaazahexadecan-16-amido)benzyl(((S)-1-(((3R,4S,5S)-1-(S)-2-(1R,2R)-3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl(methyl)carbamate (L005-P002)

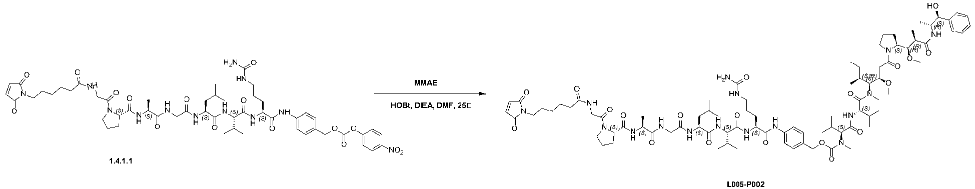

HOBt (5.44 mg, 40.2 µmol) and DIEA (8.67 mg, 67.1 µmol) were added to a DMF (400 µL) solution of compound 1.4.1.1 (38 mg, 33.5 µmol) and MMAE (24.1 mg, 33.4 µmol). The mixture was stirred at 25°C for 8 h. LC-MS monitored completion of the reaction. Preparation and purification were performed by HPLC to obtain a yellow solid L005-P002 (25 mg, 14.6 µmol, yield: 43.5%).

ESI-MS (m/z): 1712.6 [M+H]⁺.

### 1.4.3 Synthesis of L005-P003

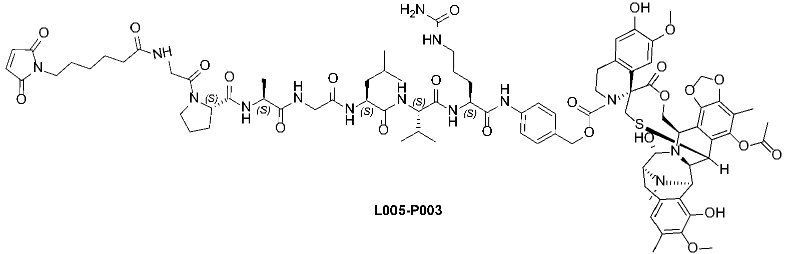

### 1.4.3.1: Synthesis of L005-P003

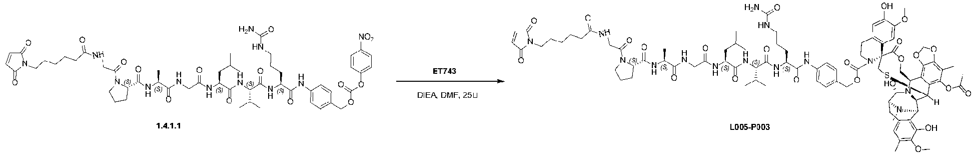

DIEA (12.7 mg, 17.1 µL, 298.4 µmol) was added to a DMF (0.5 mL) solution of compound 1.4.1.1 (111 mg, 98.4 µmol) and ET743 (15 mg, 19.7 µmol), then degassed and purged with N₂ three times, and the mixture was stirred under N₂ atmosphere at 25°C for 0.5 h. LC-MS monitored completion of the reaction. The mixture was added to stirring cold water (10 mL) and stirred at 25°C for 5 min. The residue solution was subjected to preparative purification by HPLC to obtain a white solid L005-P003 (26 mg, yield: 71.6%). ESI-MS (m/z): 1737.8 [M-H₂O]⁺.

### 1.4.4 Synthesis of L006-P005

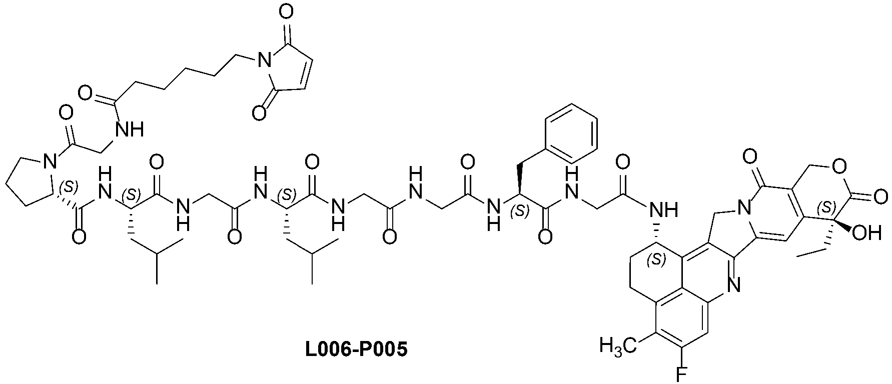

### 1.4.4.1: Synthesis of (S)-1-((6-(2,5-dioxo-2,5-dihydro-1H-pyrrol-1-yl)hexanoyl)glycyl)-N-((S) -1-(((4S,13S)-13-(1S,9S)-9-ethyl-5-fluoro-9-hydroxy-4-methyl-10,13,15-hexahydro-1H,12H-benzo [de]pyrano[3',4':6,7]indolizino[1,2-b]quinolin-1-yl)amino)-2-oxoethyl)carbamoyl)-4-isobutyl-2,5,8, 11-tetraoxo-14-phenyl-3,6,9,12-tetraazatetradecyl)amino)-4-methyl-1-oxopentan-2-yl)pyrrolidine-2 -carboxamide (L006-P005)

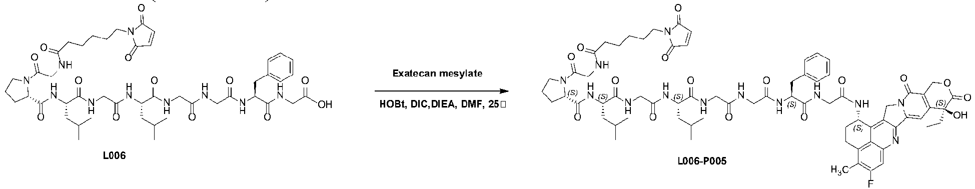

Compound L006 (30.0 mg, 31.0 µmol) and exatecan mesylate (13.2 mg, 24.8 µmol) were dissolved in DMF (300 µL), and then HOBt (10.1 mg, 74.4 µmol), DIC (15.6 mg, 124 µmol, 19.2 µL), and DIEA (20.1 mg, 155 µmol, 27.0 µL) were added thereto, respectively. Stirring was carried out at 25°C for 3 h. LC-MS monitored completion of the reaction. Preparation and purification was performed by HPLC. A yellow solid L006-P005 (13.0 mg, 9.39 µmol, yield: 30.2%) was obtained. ESI-MS (m/z): 1384.3 [M+H]⁺.

### 1.4.5 Synthesis of L008-P005

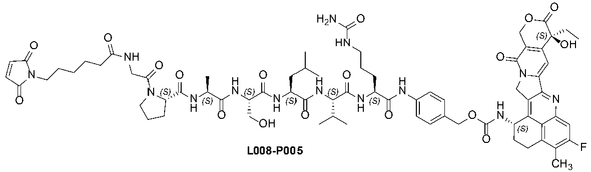

### 1.4.5.1 Synthesis of (6-(2,5-dioxo-2,5-dihydro-1H-pyrrol-1-yl)hexanoyl)glycyl-L-prolyl-L-alanyl-L-seryl-L-leucine (1.4.5.1)

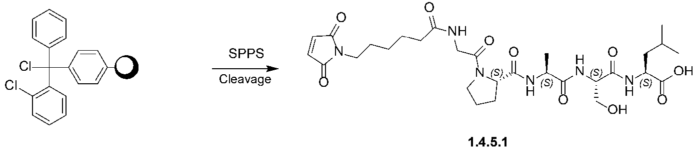

This polypeptide was synthesized by standard Fmoc chemistry method.
1) DCM was added to a container containing a CTC resin (2.0 mmol, 0.83 mmol/g, 2.4 g) and Fmoc-Leu-OH (2.0 mmol, 1.0 eq.) under N₂ bubbling.
2) DIEA (6.0 eq.) was added stepwise, and mixed for 2 h.
3) MeOH (2.5 mL) was added and stirred for 0.5 h.
4) The resulting product was drained and washed with DMF 3 times.
5) 20% piperidine/DMF was added and reacted for 30 min.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Leu-OH (1.0 eq.) | DIEA (6.0 eq.) |
| 2 | Fmoc-Ser(tBu)-OH (3.0 eq.) | HBTU (2.85 eq.) and DIEA (6.0 eq.) |
| 3 | Fmoc-Ala-OH (3.0 eq.) | HBTU (2.85 eq.) and DIEA (6.0 eq.) |
| 4 | Fmoc-Pro-OH (3.0 eq.) | HBTU (2.85 eq.) and DIEA (6.0 eq.) |
| 5 | Fmoc-Gly-OH (3.0 eq.) | HBTU (2.85 eq.) and DIEA (6.0 eq.) |
| 6 | 6-(2,5-dioxo-2,5-dihydro-1H-pyrrol-1-yl)hexanoic acid (2.0 eq.) | HOAT (2.0 eq.) and DIC (2.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) The resulting peptide resin was washed with methanol 3 times and dried under vacuum.
2) A cleavage buffer (50% TFA/DCM) was added to the peptide resin and stirred for 1 h.
3) DCM and TFA were concentrated under reduced pressure.
4) Vacuum drying was performed for 2 h to obtain the crude peptide (1.2 g).
5) The crude peptide was purified by prep-HPLC (A: 0.075% TFA in H₂O, B: ACN) to obtain a white solid compound 1.4.5.1 (110 mg, 99.8%).

ESI-MS (m/z): 637.3 [M+H]⁺.

### 1.4.5.2 Synthesis of (9H-fluoren-9-yl)methyl((S)-1-((S)-1-((4-(hydroxymethyl)phenyl)amino)-1-oxo-5-ureidopentan-2-yl)amino)-3-methyl-1-oxobutan-2-yl)carbamate (1.4.5.2)

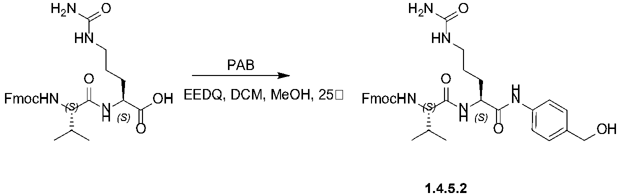

EEDQ (19.9 g, 80.5 mmol, 2.0 eq.) and PAB (9.92 g, 80.5 mmol, 2.0 eq.) were added to a solution of compound Fmoc-Val-Cit-OH (20.0 g, 40.3 mmol, 1.0 eq.) in DCM (200 mL) and MeOH (50.0 mL). The mixture was stirred at 25°C for 16 h. LC-MS monitored completion of the reaction. The reaction product was filtered, and the filtrate was added to 2,000 mL EtOAc/MTBE (8/1) and stirred for 10 min twice, filtered, and the filtrate was washed with EtOAc to obtain a white solid compound 1.4.5.2 (19.8 g, crude product). ESI-MS (m/z): 602.3 [M+H]⁺.

### 1.4.5.3 Synthesis of (9H-fluoren-9-yl)methyl((S)-3-methyl-1-(((S)-1-((4-(((4-nitrophenoxy)carbonyl)oxy)methyl)phenyl)amino)-1-oxo-5-ureidopentan-2-yl)amino)-1-oxobutan-2-yl)carbamate (1.4.5.3)

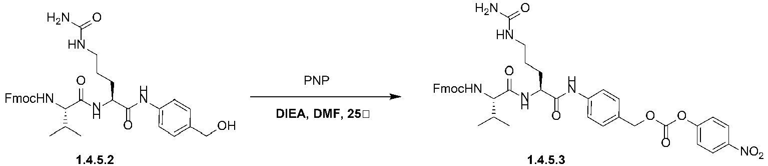

DIEA (8.59 g, 66.4 mmol, 11.5 mL, 2.0 eq.) was added to a DMF (200 mL) solution of compound 1.4.5.2 (20.0 g, 33.2 mmol, 1.0 eq.) and PNP (20.2 g, 66.4 mmol, 2.0 eq.). The mixture was stirred at 25°C for 3 h. LC-MS monitored completion of the reaction. The reaction mixture was dropped into stirring ice MTBE and filtered. The solid obtained by filtration was dissolved in DCM, then poured into MTBE/EtOAc (1:1), stirred for 30 min, then filtered, and concentrated under reduced pressure to obtain a white solid compound 1.4.5.3 (21 g, crude product). ESI-MS (m/z): 767.4 [M+H]⁺.

### 1.4.5.4 Synthesis of (9H-fluoren-9-yl)methyl((S)-1-((S)-1-(4-(1S,9S)-9-ethyl-5-fluoro-9-hydroxy-4-methyl-10,13-dioxo-2,3,9,10,13,15-hexahydro-1H,12H-benzo[d]pyrano[3',4':6,7]indolizino[1,2-b]quinolin-1-yl)carbamoyl)oxy)methyl)phenyl)amino)-1-oxo-5-ureidopentan-2-yl)amino)-3-methyl-1-oxobutan-2-yl)carbamate (1.4.5.4)

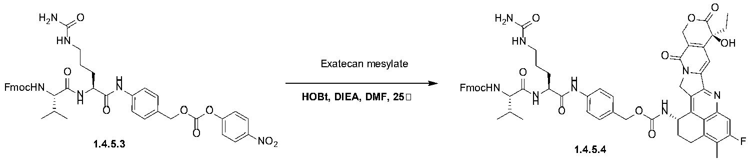

Compound 1.4.5.3 (3.00 g, 3.91 mmol, 1.0 eq.), exatecan mesylate (1.87 g, 3.52 mmol, 0.8 eq.), and HOBt (635 mg, 4.70 mmol, 1.2 eq.) were dissolved in DMF (30 mL), and DIEA (1.01 g, 7.83 mmol, 1.29 mL, 2.0 eq.) was added to the mixture. The mixture was stirred under N₂ atmosphere at 25°C for 3.0 h. LC-MS monitored completion of the reaction. The mixture was ground with isopropyl ether (300 mL) to obtain a brown solid compound 1.4.5.4 (5.60 g, crude product). ESI-MS (m/z):1063.4 [M+H]⁺.

### 1.4.5.5 Synthesis of 4-(((S)-2-[(S)-2-amino-3-methylbutanamido]-5-ureidopentanamido)benzyl ((1S,9S)-9-ethyl-5-fluoro-9-hydroxy-4-methyl-10,13-dioxo-2,3,9,10,13,15-hexahydro-1H,12H-ben zo[de]pyrano[3',4':6,7]indolizino[1,2-b]quinolin-1-yl)carbamate (1.4.5.5)

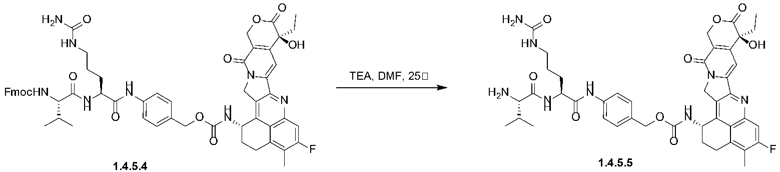

TEA (10 mL) was added to a mixture of compound 1.4.5.4 (5.60 g, crude product) in DMF (40 mL). The mixture was stirred at 25°C for 16 h. LC-MS monitored completion of the reaction. The reaction mixture was purified by preparative HPLC to obtain a yellow solid compound 1.4.5.5 (2.75 g, 3.27 mmol, yield: 83.5%). ESI-MS (m/z):841.6 [M+H]⁺.

### 1.4.5.6 Synthesis of 4-((3S,6S,9S,12S,15S)-1-((S)-1-)((6-(2,5-dioxo-2,5-dihydro-1H-pyrrol-1-yl)hexanoyl)glycyl)pyrrolidin-2-yl)-6-(hydroxymethyl)-9-isobutyl-12-isopropyl-3-methyl-1,4,7,10,13-pentaoxo-15-(3-ureidopropyl)-2,5,8,11,14-pentaazahexadecan)benzyl(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L008-P005)

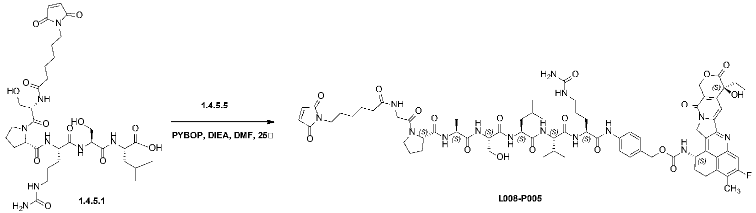

Compound 1.4.5.1 (30.0 mg, 47.1 µmol), compound 1.4.5.5 (39.6 mg, 47.1 µmol), PYBOP (49.0 mg, 94.2 µmol), and DIEA (32.8 µL, 188 µmol) were added to DMF (0.5 mL), respectively. Stirring was carried out at 25°C for 1 h. LC-MS monitored completion of the reaction. The crude product was purified by HPLC to obtain a yellow solid compound L008-P005 (8.0 mg, yield: 11.6%). ESI-MS (m/z): 1459.4 [M+H]⁺.

### 1.4.6 Synthesis of L009-P005

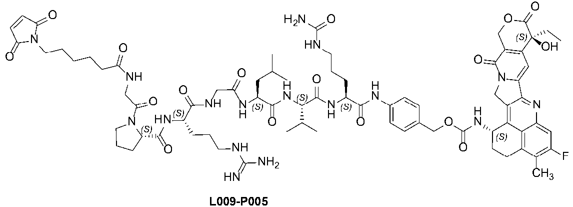

### 1.4.6.1 Synthesis of (6-(2,5-dioxo-2,5-dihydro-1H-pyrrol-1-yl)hexanoyl)glycyl-L-prolyl-L-arginylglycyl-L leucine (1.4.6.1)

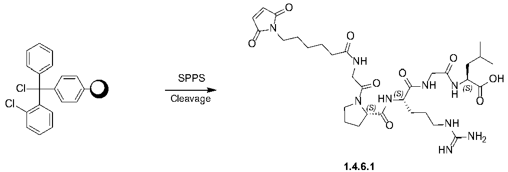

This polypeptide was synthesized by standard Fmoc chemistry method.
1) Dichloromethane was added to a container containing a CTC resin (2.0 mmol, 0.83 mmol/g, 2.4 g) and Fmoc-Leu-OH (2.0 mmol, 1.0 eq.) under nitrogen bubbling.
2) Diisopropylethylamine (6.0 eq.) was added stepwise and mixed for 2 h.
3) Methanol (2.5 mL) was added and stirred for 0.5 hours.
4) The stirred solution was drained and washed with DMF three times.
5) 20% piperidine/DMF was added and reacted for 30 min.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Leu-OH (1.0 eq.) | DIEA (6.0 eq.) |
| 2 | Fmoc-Gly-OH (3.0 eq.) | HBTU (2.85 eq.) and DIEA (6.0 eq.) |
| 3 | Fmoc-Arg(Boc)₂-OH (3.0 eq.) | HATU (2.85 eq.) and DIEA (6.0 eq.) |
| 4 | Fmoc-Pro-OH (3.0 eq.) | HBTU (2.85 eq.) and DIEA (6.0 eq.) |
| 5 | Fmoc-Gly-OH (3.0 eq.) | HBTU (2.85 eq.) and DIEA (6.0 eq.) |
| 6 | 6-(2,5-dioxo-2,5-dihydro-1H-pyrrol-1-yl)hexanoic acid (2.0 eq.) | HOAT (2.0 eq.) and DIC (2.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) The above obtained polypeptide resin was washed three times with methanol and dried under vacuum.
2) A cleavage buffer (50% TFA/DCM) was added to the polypeptide resin and stirred for 1 h.
3) DCM and TFA were concentrated under reduced pressure.
4) Vacuum drying was performed for 2 hours to obtain the crude peptide (1.4 g).
5) The crude peptide was purified by HPLC (A: 0.075% TFA in H₂O, B: ACN) to obtain a white solid compound 1.4.6.1 (120 mg, 99.0%).

ESI-MS (m/z): 692.4 [M+H]⁺.

### 1.4.6.2 Synthesis of 4-((6S,12S,15S,18S)-1-amino-6-((S)-1-((6-(2,5-dioxo-2,5-dihydro-1H-pyr rol-1-yl)hexanoyl)glycyl)pyrrolidin-2-carboxamido)-1-imino-12-isobutyl-15-isopropyl-7,10,13,6-te traoxo-18-(3-ureidopropyl)-2,8,11,14,17-pentaazanonan-19-amino)benzyl(((1S,9S)-9-ethyl-5-fluoro -9-hydroxy-4-methyl-10,13-dioxo-2,3,9,10,13,15-hexahydro-1H,12H-benzo[de]pyrano[3',4':6,7]ind olizino[1,2-b]quinolin-1-yl)carbamate (L009-P005)

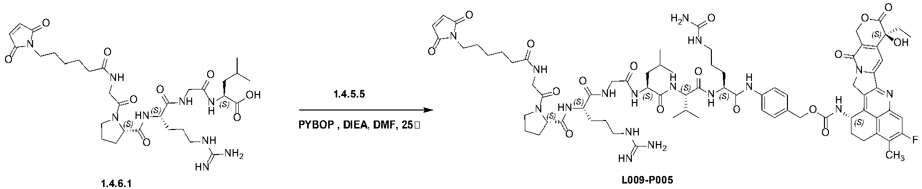

Compound 1.4.6.1 (30.0 mg, 43.4 µmol), compound 1.4.5.5 (36.5 mg, 43.4 µmol), PYBOP (45.1 mg, 86.7 µmol), and DIEA (30.2 µL, 173 µmol) were added to DMF (0.5 mL), respectively. Stirring was carried out at 25°C for 1 h. LC-MS monitored completion of the reaction. The crude product was purified by HPLC. A yellow solid compound L009-P005 (7.0 mg, yield: 10.6%) was obtained. ESI-MS (m/z): 1515.4 [M+H]⁺.

### 1.4.7 Synthesis of L009-P002

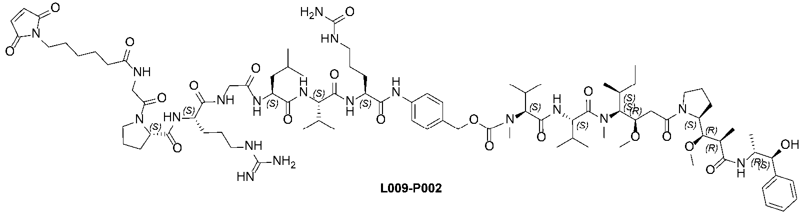

### 1.4.7.1 Synthesis of 4-((6S,12S,15S,18S)-1-amino-6-((S)-1-(6-(2,5-dioxo-2,5-dihydro-1H-pyrrol-1-yl) hexanoyl)glycyl)pyrrolidine-2-carboxamido)-1-imino-12-isobutyl-15-isopropyl-7,10,13,16-tetraoxo-18-(3-ureidopropyl)-2,8,11,14,17-pentaazanonadecan-19-amido)benzyl(1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (L009-P002)

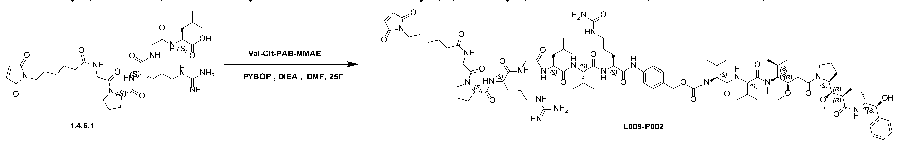

PYBOP (45.1 mg, 86.7 µmol) and DIEA (30.2 µL, 173 µmol) were added to a DMF (0.3 mL) solution of compound 1.4.6.1 (30.0 mg, 43.4 µmol) and compound Val-Cit-PAB-MMAE (48.7 mg, 43.4 µmol). The mixture was stirred at 25°C for 1.5 h. LC-MS monitored completion of the reaction. The product was purified by preparative HPLC to obtain a white solid compound L009-P002 (24.0 mg, yield: 30.8%).

ESI-MS (m/z): 1798.6 [M+H]⁺; ¹H NMR: (400 MHz, DMSO-*d₆*) *δ*: 10.02 (br s, 1 H), 8.23-8.40 (m, 1 H), 8.04-8.14 (m, 2 H), 7.86-8.01 (m, 4 H), 7.78-7.85 (m, 1 H), 7.54-7.60 (m, 2 H), 7.38 (br t, J=5.75 Hz, 1 H), 7.23-7.35 (m, 7 H), 7.13-7.22 (m, 2 H), 7.00 (s, 2 H), 5.98 (br s, 1 H), 5.38-5.48 (m, 1 H), 4.91-5.15 (m, 3 H), 4.67-4.79 (m, 1 H), 4.48 (br d, J=5.75 Hz, 1 H), 4.43 (br d, *J* =6.63 Hz, 1 H), 4.30-4.40 (m, 4 H), 4.20-4.29 (m, 2 H), 4.16 (br t, *J* =7.75 Hz, 1 H), 3.95-4.03 (m, 2 H), 3.92 (br d, *J* =5.63 Hz, 1 H), 3.78-3.87 (m, 2 H), 3.69-3.77 (m, 3 H), 3.29-3.37 (m, 7 H), 3.23 (d, *J=6.13* Hz, 4 H), 3.19 (d, J=10.13 Hz, 3 H), 3.11 (br s, 2 H), 3.05-3.10 (m, 2 H), 2.97 (br s, 1 H), 2.86 (br d, *J* =14.76 Hz, 3 H), 2.41 (br d, *J* =15.63 Hz, 1 H), 2.26 (br dd, *J=16.01,* 9.26 Hz, 1 H), 2.08-2.15 (m, 4 H), 1.94-2.05 (m, 4 H), 1.69-1.78 (m, 4 H), 1.51-1.62 (m, 5 H), 1.43-1.51 (m, 10 H), 1.23 (br s, 4 H), 0.96-1.06 (m, 7 H), 0.74-0.89 (m, 33 H).

### 1.4.8 Synthesis of L010-P005

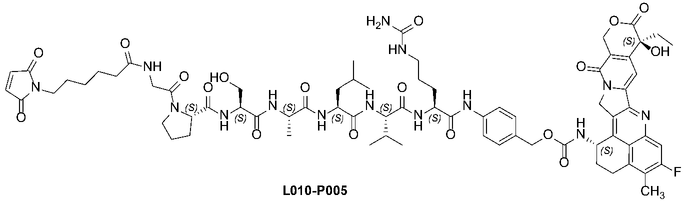

### 1.4.8.1 Synthesis of (6-(2,5-dioxo-2,5-dihydro-1H-pyrrol-1-yl)hexanoyl)glycyl-L-prolyl-L-seryl-L-alanyl-L-leucine (1.4.8.1)

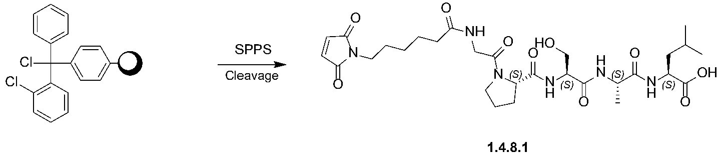

This polypeptide was synthesized by standard Fmoc chemistry method.
1) Dichloromethane was added to a container containing a CTC resin (2.0 mmol, 0.83 mmol/g, 2.4 g) and Fmoc-Leu-OH (2.0 mmol, 1.0 eq.) under nitrogen bubbling.
2) Diisopropylethylamine (6.0 eq.) was added stepwise and mixed for 2 h.
3) Methanol (2.5 mL) was added and stirred for 0.5 h.
4) The resulting product was drained and washed with DMF 3 times.
5) 20% piperidine/DMF was added and reacted for 30 min.
6) The resulting product was drained and washed with DMF 5 times.
7) Fmoc-amino acid solution was added and mixed for 30 s, and then the corresponding coupling reagent was added. The reaction was continued for 1 h under nitrogen bubbling.
8) Steps 4 to 7 were repeated for the next amino acid coupling.

| # | Materials | Coupling reagents |
|---|---|---|
| 1 | Fmoc-Leu-OH (1.0 eq.) | DIEA (6.0 eq.) |
| 2 | Fmoc-Ala-OH (3.0 eq.) | HBTU (2.85 eq.) and DIEA (6.0 eq.) |
| 3 | Fmoc-Ser(tBu)-OH (3.0 eq.) | HBTU (2.85 eq.) and DIEA (6.0 eq.) |
| 4 | Fmoc-Pro-OH (3.0 eq.) | HBTU (2.85 eq.) and DIEA (6.0 eq.) |
| 5 | Fmoc-Gly-OH (3.0 eq.) | HBTU (2.85 eq.) and DIEA (6.0 eq.) |
| 6 | 6-(2,5-dioxo-2,5-dihydro-1H-pyrrol-1-yl)hexanoic acid (2.0 eq.) | HOAT (2.0 eq.) and DIC (2.0 eq.) |

DMF containing 20% piperidine was used for Fmoc deprotection for 30 min. The coupling reaction was monitored by a ninhydrin test, and the resin was washed with DMF 5 times.

Polypeptide cleavage and purification:
1) The resulting peptide resin was washed with methanol 3 times and dried under vacuum.
2) A cleavage buffer (50% TFA/DCM) was added to the peptide resin and stirred for 1 h.
3) DCM and TFA were concentrated under reduced pressure.
4) Vacuum drying was performed for 2 h to obtain the crude peptide (1.2 g).
5) The crude peptide was purified by HPLC (A: 0.075% TFA in H₂O, B: ACN) to obtain a white solid compound 1.4.8.1 (90 mg, 99.6%).

ESI-MS (m/z): 637.3 [M+H]⁺.

### 1.4.8.2 Synthesis of 4-((3S,6S,9S,12S,15S)-1-((S)-1-)((6-(2,5-dioxo-2,5-dihydro-1H-pyrrol-1-y 1)hexanoyl)glycyl)pyrrolidin-2-yl)-3-(hydroxymethyl)-9-isobutyl-12-isopropyl-6-methyl-1,4,7,10,1 3-pentaoxo-15-(3-ureidopropyl)-2,5,8,11,14-pentaazahexadecanyl)benzyl(((1S,9S)-9-ethyl-5-fluoro -9-hydroxy-4-methyl-10,13-dioxo-2,3,9,10,13,15-hexahydro-1H,12H-benzo[de]pyrano[3',4':6,7]ind olizino[1,2-b]quinolin-1-yl)carbamate (L010-P005)

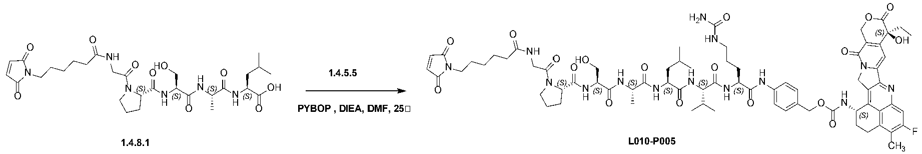

Compound 1.4.8.1 (30.0 mg, 47.1 µmol) and compound 1.4.5.5 (39.6 mg, 47.1 µmol), PYBOP (49.0 mg, 94.2 µmol) and DIEA (32.8 µL, 188 µmol) were added to DMF (0.5 mL), respectively. Stirring was carried out at 25°C for 1 h. LC-MS monitored completion of the reaction. The crude product was purified by HPLC. A yellow solid compound L010-P005 (9.0 mg, yield: 13.1%) was obtained.

ESI-MS (m/z): 1460.2 [M+H]⁺.

### 1.4.9 Synthesis of L011-P005

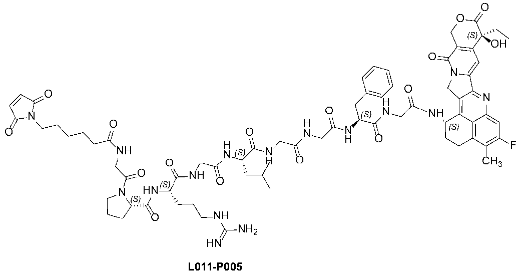

### 1.4.9.1 Synthesis of (S)-1-((6-(2,5-dioxo-2,5-dihydro-1H-pyrrol-1-yl)hexanoyl)glycyl)-N-((S)-1-((4S,13S)-13-(2-((1S,9S)-9-ethyl-5-fluoro-9-hydroxy-4-methyl-10,13-dioxo-2,3,9,10,13,15-hexah ydro-1H,12H-benzo[d]pyrano[3',4':6,7]indolizino[1,2-b]quinolin-1-yl)amino)-2-oxoethyl)carbamoy l)-4-isobutyl-2,5,8,11-tetraoxo-14-phenyl-3,6,9,12-tetraazatetradecyl)amino)-5-guanidino-1-oxopen tan-2-yl)pyrrolidin-2-carboxamide (L011-P005)

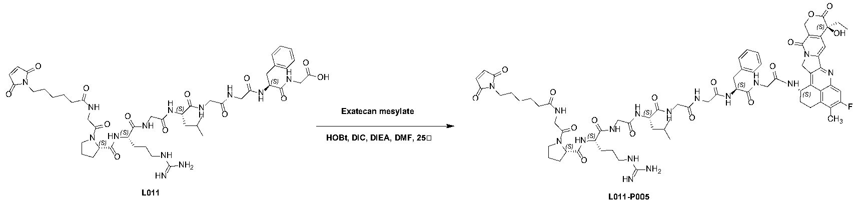

Compound L011 (30.0 mg, 29.7 µmol) and exatecan mesylate (12.6 mg, 23.8 µmol) were dissolved in DMF (300 µL), and then HOBt (9.63 mg, 71.3 µmol), DIC (18.4 µL, 119 µmol), and DIEA (20.7 µL, 118.80 µmol) were added thereto, respectively. Stirring was carried out at 25°C for 8 h. LC-MS monitored completion of the reaction. The reaction mixture was acidified with acetic acid. The product was purified by HPLC. A yellow solid compound L011-P005 (6.0 mg, yield: 14.2%) was obtained. ESI-MS (m/z): 1428.4 [M+H]⁺.

### 1.4.10 Synthesis of L012-P005

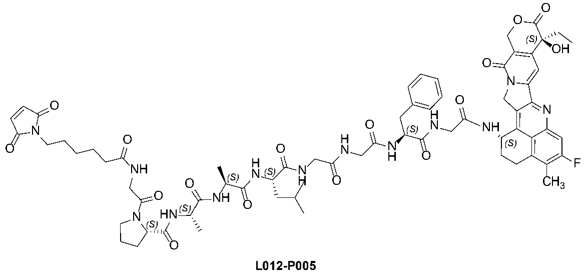

### 1.4.10.1 Synthesis of (S)-1-((6-(2,5-dioxo-2,5-dihydro-1H-pyrrol-1-yl)hexanoyl)glycyl)-N-((S) -1-(((S)-1-(((S)-1-((2-((2-(((S)-1-((2-(((1 S,9S)-9-ethyl-5-fluoro-9-hydroxy-4-methyl-1 0, 13-dioxo-2, 3,9,10,13,15-hexahydro-1H,12H-benzo[en]pyrano[3',4':6,7]indolizino[1,2-b]quinolin-1-yl)amino)-2 -oxoethyl)amino)-1-oxo-3-phenylprop-2-yl)amino)-2-oxoethyl)amino)-2-oxyethyl(amino)-4-methyl -1-oxopent-2-yl)amino)-1-oxoprop-2-yl)amino)-1-oxopropan-2-yl)pyrrolidin-2-carboxamide (L012-P005)

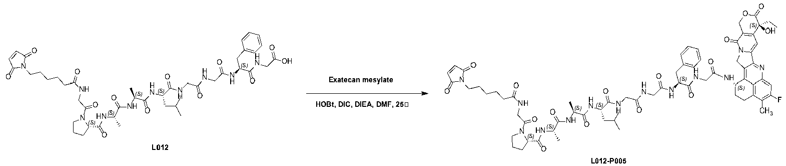

Compound L012 (30.0 mg, 32.0 µmol) and exatecan mesylate (13.6 mg, 25.6 µmol) were dissolved in DMF (300 µL), and then HOBt (10.4 mg, 76.7 µmol), DIC (19.8 µL, 128 µmol), and DIEA (22.3 µL, 128 µmol) were added thereto, respectively. Stirring was carried out at 25°C for 8 h. LC-MS monitored completion of the reaction. The reaction mixture was acidified with acetic acid. The product was purified by HPLC. A yellow solid compound L012-P005 (8.0 mg, yield: 18.5%) was obtained. ESI-MS (m/z): 1357.4 [M+H]⁺.

### 1.4.11 Synthesis of L013-P005

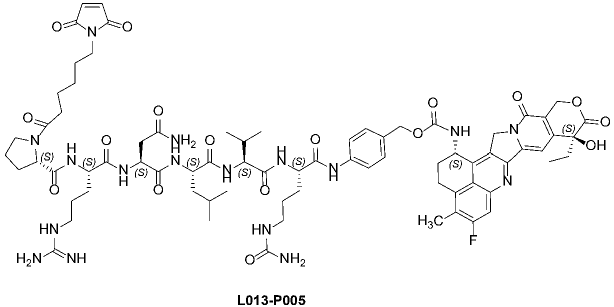

### 1.4.11.1 Synthesis of 4-((6S,9S,12S,15S,18S)-1-amino-9-(2-amino-2-oxoethyl)-6-((S)-1-(6-(2, 5-dioxo-2,5-dihydro-1H-pyrrol-1-yl)hexanoyl)pyrrolidin-2-carboxamido)-1-imino-12-isobutyl-15-i sopropyl-7,10,13,16-tetraoxo-18-(3-ureidopropyl)-2,8,11,14,17-pentaazanonadecan-19-amido)benz yl((1S,9S)-9-ethyl-5-fluoro-9-hydroxy-4-methyl-10,13-dioxo-2,3,9,10,13,15-hexahydro-1H,12H-be nzo[de]pyrano[3',4':6,7]indolizino[1,2-b]quinolin-1-yl)carbamate (L013-P005)

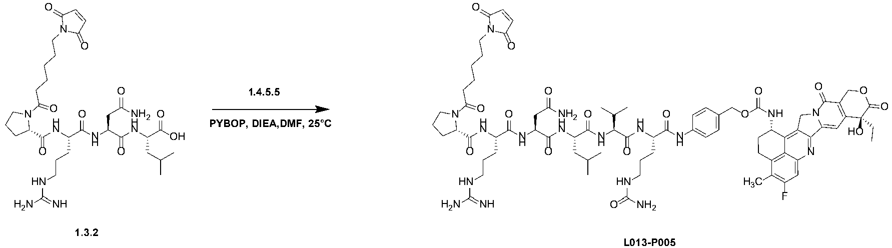

Compound 1.3.2 (30.0 mg, 43.4 µmol, 1 eq.) and compound 1.4.5.5 (29.2 mg, 34.7 µmol, 0.8 eq.) were dissolved in DMF (0.5 mL), and then PYBOP (45.1 mg, 86.7 µmol) and DIEA (30.2 µL, 173 µmol, 4 eq.) were added thereto. The mixture was stirred at 25°C for 1 h. LC-MS monitored completion of the reaction. TFA was added to the reaction mixture to adjust the pH value. The product was purified by HPLC. A yellow solid compound L013-P005 (10.7 mg, 6.57 µmol, yield: 15.1%) was obtained. ESI-MS (m/z): 1514.9 [M+H]⁺.

### 1.4.12 Synthesis of L013-P002

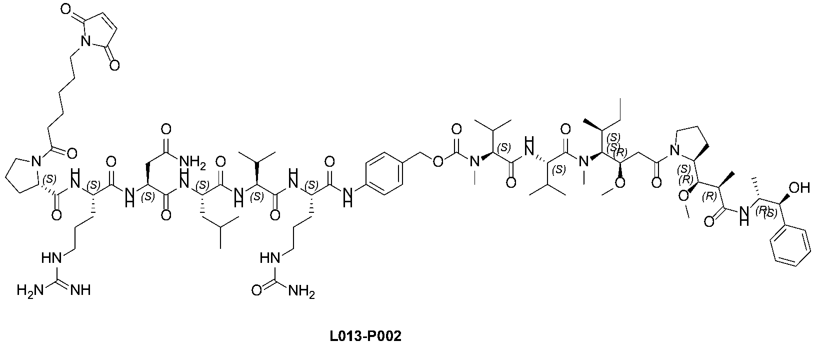

### 1.4.12.1 Synthesis of 4-((6S,9S,12S,15S,18S)-1-amino-9-(2-amino-2-oxoethyl)-6-((S)-1-(6-(2,5-dioxo-2,5-dihydro-1H-pyrrol-1-yl)hexanoyl)pyrrolidin-2-carboxamido)-1-imino-12-isobutyl-15-isopropyl-7,10,13,16-tetraoxo-18-(3-ureidopropyl)-2,8,11,14,17-pentaazanonadecan-19-amido)benzyl(1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)-3-methyl-1-oxobut-2-yl(methyl)carbamate (L013-P002)

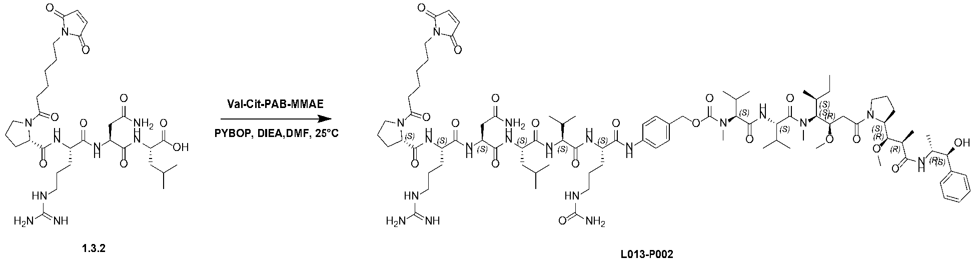

Compound 1.3.2 (30.0 mg, 43.4 µmol, 1 eq.) and compound Val-Cit-PAB-MMAE (34.1 mg, 30.4 µmol, 0.7 eq.) were dissolved in DMF (0.5 mL), and then PYBOP (45.1 mg, 86.7 µmol, 2 eq.) and DIEA (30.2 µL, 173 µmol, 4 eq.) were added to the resulting solution. The mixture was stirred at 25°C for 1 h. LC-MS monitored completion of the reaction. TFA was added to the reaction mixture to adjust the pH value. The residue was purified by preparative HPLC. A white solid compound L013-P002 (10.7 mg, 5.60 µmol, yield: 12.9%) was obtained. ESI-MS (m/z): 1798.6 [M+H]⁺.

### 1.4.13 Synthesis of L015-P005

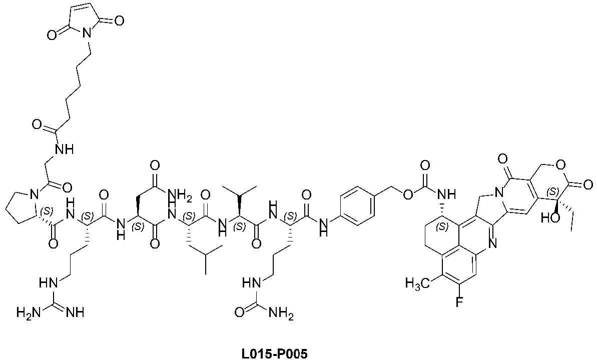

### 1.4.13.1 Synthesis of 4-((6S,9S,12S,15S,18S)-1-amino-9-(2-amino-2-oxyethyl)-6-((S)-1-(6-(2, 5-dioxo-2,5-dihydro-1H-pyrrol-1-yl)hexanoyl)glyceryl)pyrrolidin-2-carboxamido)-1-imino-12-isob utyl-15-isopropyl-7,10,13,16-tetraoxo-18-(3-ureidopropyl)-2,8,11,14,17-pentaazanonadecan-19-ami do)benzyl((1S,9S)-9-ethyl-5-fluoro-9-hydroxy-4-methyl-10,13-dioxo-2,3,9,10,13,15-hexahydro-1H, 12H-benzo[de]pyrano[3',4':6,7]indolizino[1,2-b]quinolin-1-yl)carbamate (L015-P005)

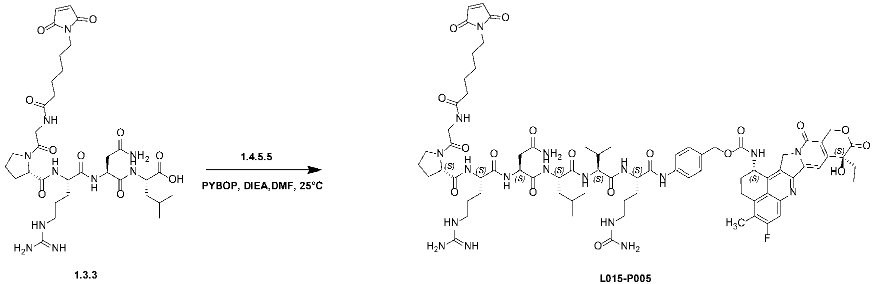

Compound 1.3.3 (35.0 mg, 46.7 µmol, 1 eq.) and compound 1.4.5.5 (39.3 mg, 46.7 µmol, 1 eq.) were dissolved in DMF (0.5 mL), and then PYBOP (48.7 mg, 93.5 µmol, 2 eq.), and DIEA (32.6 µL, 187 µmol, 4 eq.) were added to the resulting solution, and the mixture was stirred at 25°C for 1 h. LC-MS monitored completion of the reaction. TFA was added to the reaction mixture to adjust the pH value. The residue was purified by preparative HPLC. A yellow solid compound L015-P005 (10.4 mg, 6.17 µmol, yield: 13.2%) was obtained. ESI-MS (m/z): 1572.7 [M +H]⁺.

### 1.4.14 Synthesis of L015-P002

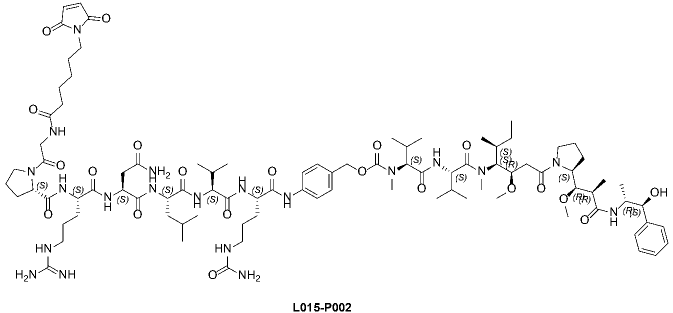

### 1.4.14.1 Synthesis of 4-((6S,9S,12S,15S,18S)-1-amino-9-(2-amino-2-oxyethyl)-6-((S)-1-(6-(2,5-dioxo-2,5-dihydro-1H-pyrrol-1-yl)hexanoyl)glyceryl)pyrrolidin-2-carboxamido)-1-imino-12-isobutyl-15-isopropyl-7,10,13,16-tetraoxo-18-(3-ureidopropyl)-2,8,11,14,17-pentaazanonadecan-19-amido)benzyl-2-((1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)-3-methyl-1-oxobut-2-yl(methyl)carbamate (L015-P002)

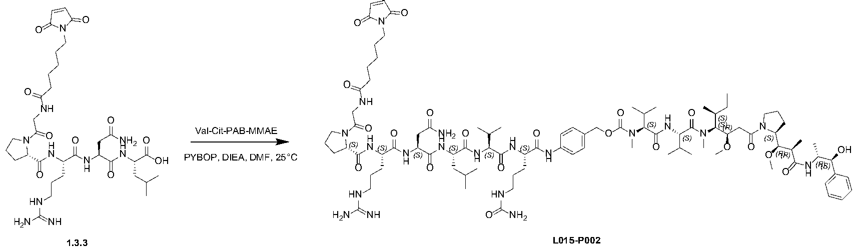

Compound 1.3.3 (35.0 mg, 46.7 µmol, 1 eq.) and compound Val-Cit-PAB-MMAE (42.0 mg, 37.4 µmol, 0.8 eq.) were dissolved in DMF (0.5 mL), and then PYBOP (48.7 mg, 93.5 µmol, 2 eq.) and DIEA (24.4 µL, 140 µmol, 3 eq.) were added to the resulting solution, and the mixture was stirred at 25°C for 1 h. LC-MS monitored completion of the reaction. TFA was added to the reaction mixture to adjust the pH value. The residue was purified by preparative HPLC to obtain a white solid compound L015-P002 (11.4 mg, 5.79 µmol, yield: 12.4%, TFA). ESI-MS (m/z): 1855.1[M+H]⁺.

### 1.4.15 Synthesis of L015-P003

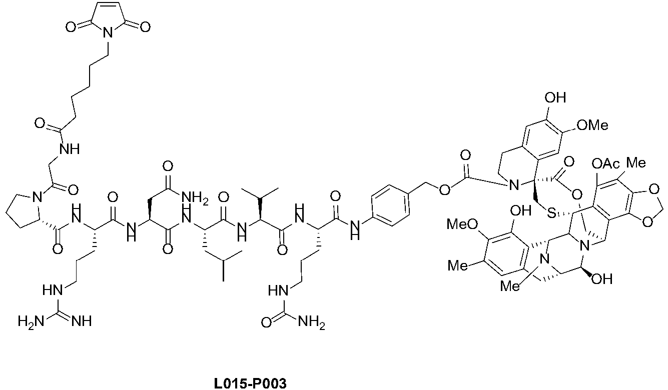

### 1.4.15.1 Synthesis of Fmoc-Val-Cit-PAB-ET743 (1.4.15.1)

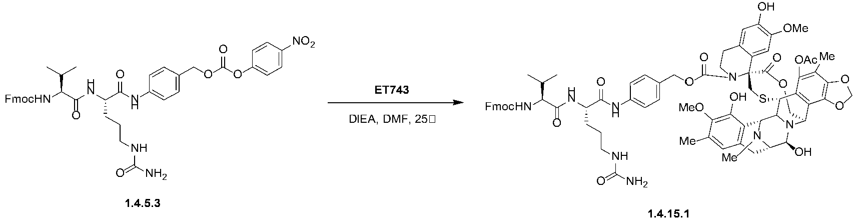

Compound 1.4.5.3 (101 mg, 131 µmol, 2.00 eq.) and ET743 (P003) (50.0 mg, 65.6 µmol, 1.00 eq.) were dissolved in DMF (100 µL), and DIEA (45.7 µL, 263 µmol, 4.00 eq.) was added to the resulting solution. The mixture was stirred at 25°C for 8 h. LC-MS monitored completion of the reaction. The reaction mixture was quenched with H₂O (1.00 mL) and purified by preparative HPLC to obtain a white solid compound 1.4.15.1 (50.0 mg, 36.0 µmol, yield: 54.8%). ESI-MS (m/z): 1372.7[M-H₂O+H]⁺.

### 1.4.15.2 Synthesis of Val-Cit-PAB-ET743 (1.4.15.2)

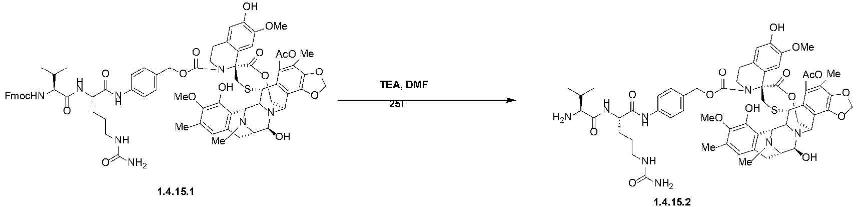

Compound 1.4.15.1 (50.0 mg, 36.0 µmol, 1.00 eq.) was dissolved in DMF (400 µL), and TEA (100 µL) was added to the resulting solution. The mixture was stirred at 25°C for 6 h. LC-MS monitored completion of the reaction. The reaction mixture was filtered to remove insoluble solids and purified by prep-HPLC to obtain a white solid compound 1.4.15.2 (33.0 mg, 28.3 µmol, yield: 78.6%). ESI-MS (m/z): 1150.8 [M-H₂O+H]⁺.

### 1.4.15.3 Synthesis of L015-P003

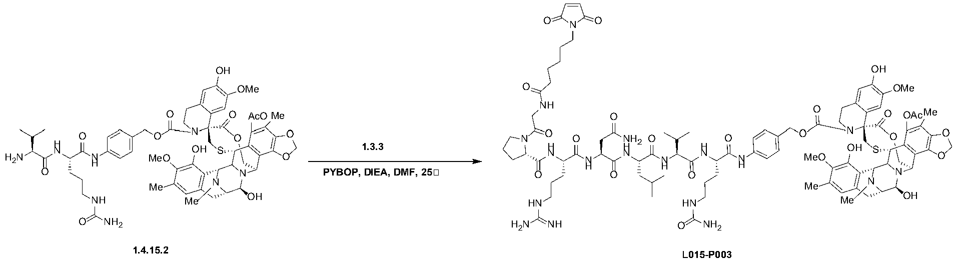

Compound 1.4.15.2 (33.0 mg, 28.3 µmol, 1.00 eq.) was dissolved in DMF (500 µL), and then PYBOP (22.1 mg, 42.4 µmol, 1.50 eq.), DIEA (19.7 µL, 113 µmol, 4.00 eq.), and compound 1.3.3 (25.4 mg, 33.9 µmol, 1.20 eq.) were added to the resulting solution. The mixture was stirred at 25°C for 6 h. LC-MS monitored completion of the reaction. The reaction mixture was filtered to remove insoluble solids and purified by prep-HPLC to obtain a white solid compound L015-P003 (8.60 mg, 4.53 µmol, yield: 16.0%). ESI-MS (m/z): 1899.1 [M+H]⁺.

### 1.4.16 Synthesis of L016-P005

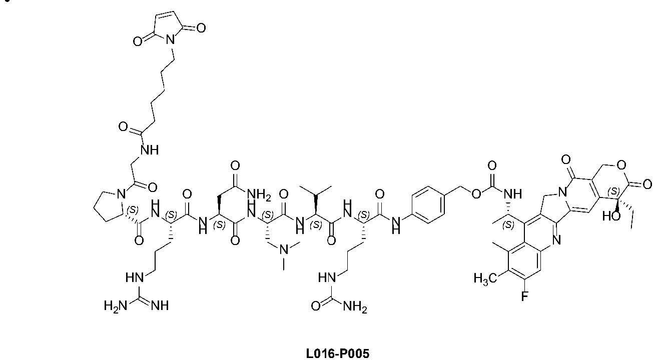

### 1.4.16.1 Synthesis of 4-((6S,9S,12S,15S,18S)-1-amino-9-(2-amino-2-oxyethyl)-12-((dimethyla mino)methyl)-6-((S)-1-(6-(2,5-dioxo-2,5-dihydro-1H-pyrrol-1-yl)hexanoyl)glyceryl)pyrrolidin-2-ca rboxamido)-1-imino-15-isopropyl-7,10,13,16-tetraoxo-18-(3-ureidopropyl)-2,8,11,14,17-pentaazan onadecan-19-amido)benzyl((1S,9S)-9-ethyl-5-fluoro-9-hydroxy-4-methyl-10,13-dioxo-2,3,3,9,10,1 3,15-hexahydro-1H,12H-benzo[de]pyrano[3',4':6,7]indolizino[1,2-b]quinolin-1-yl)carbamate (L016 -P005)

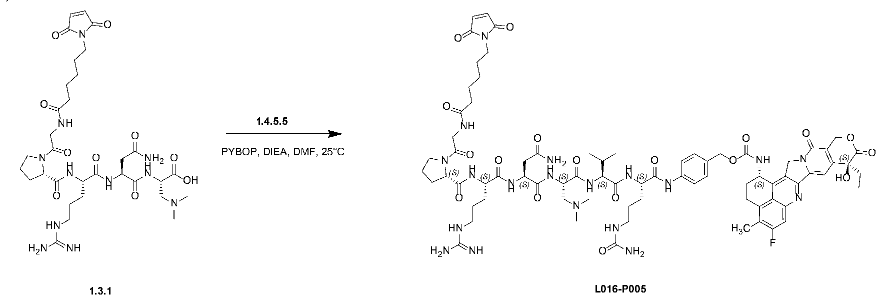

Compound 1.3.1 (15.3 mg, 20.4 µmol, 1 eq.) and compound 1.4.5.5 (15.4 mg, 18.4 µmol, 0.9 eq.) were dissolved in DMF (0.3 mL), and then PYBOP (21.2 mg, 40.8 µmol, 2 eq.) and DIEA (14.2 µL, 81.6 µmol, 4 eq.) were added to the solution. The mixture was stirred at 25°C for 0.5 h. LC-MS monitored completion of the reaction. TFA was added to the reaction mixture to adjust the pH value. The residue was purified by preparative HPLC. A yellow solid compound L016-P005 (6.60 mg, 3.81 µmol, yield: 18.7%, TFA) was obtained. ESI-MS (m/z): 1573.7[M+H]⁺.

### 1.4.17 Synthesis of L016-P002

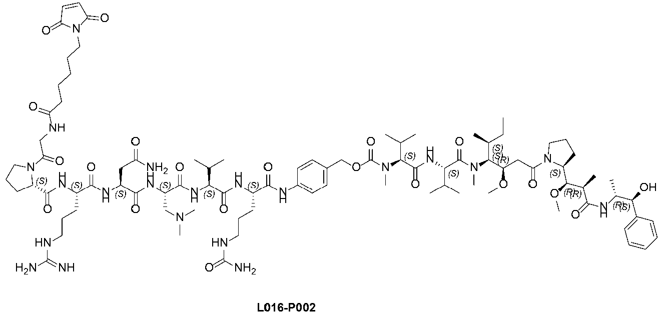

### 1.3.17.1 Synthesis of 4-((6S,9S,12S,15S,18S)-1-amino-9-(2-amino-2-oxyethyl)-12-((dimethyla mino)methyl)-6-((S)-1-(6-(2,5-dioxo-2,5-dihydro-1H-pyrrol-1-yl)hexanoyl)glyceryl)pyrrolidin-2-ca rboxamido)-1-imino-15-isopropyl-7,10,13,16-tetraoxo-18-(3-ureidopropyl)-2,8,11,14,17-pentaazan onadecan-19-amido)benzyl-1-((S)-2-((1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)a mino)-3-methyl-1-oxobut-2-yl)amino)-3-methyl-1-oxobut-2-yl(methyl)carbamate (L016-P002)

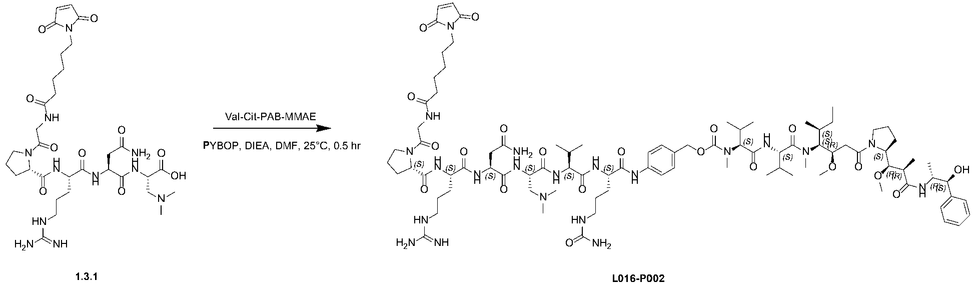

Compound 1.3.1 (15.0 mg, 20.0 µmol, 1 eq.) and compound Val-Cit-PAB-MMAE (15.7 mg, 14.0 µmol, 0.7 eq.) were dissolved in DMF (0.3 mL), and then PYBOP (20.8 mg, 40.0 µmol, 2 eq.) and DIEA (13.9 µL, 80.0 µmol, 4 eq.) were added to the resulting solution. The mixture was stirred at 25°C for 0.5 h. LC-MS monitored completion of the reaction. TFA was added to the reaction mixture to adjust the pH value. The residue was purified by preparative HPLC to obtain a white solid compound L016-P002 (6.00mg, 2.94 µmol, yield: 14.7%, TFA). ESI-MS (m/z): 1856.1[M+H]⁺.

### 1.4.18 Synthesis of L017-P005

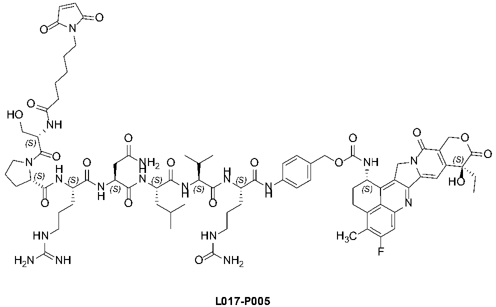

### 1.4.18.1 Synthesis of 4-((6S,9S,12S,15S,18S)-1-amino-9-(2-amino-2-oxoethyl)-6-((S)-1-(6-(2, 5-dioxo-2,5-dihydro-1H-pyrrol-1-yl)hexanoyl)-L-seryl)pyrrolidin-2-carboxamido)-1-imino-12-isob utyl-15-isopropyl-7,10,13,16-tetraoxo-18-(3-ureidopropyl)-2,8,11,14,17-pentaazanonadecan-19-ami do)benzyl((1S,9S)-9-ethyl-5-fluoro-9-hydroxy-4-methyl-10,13-dioxo-2,3,9,10,13,15-hexahydro-1H, 12H-benzo[de]pyrano[3',4':6,7]indolizino[1,2-b]quinolin-1-yl)carbamate (L017-P005)

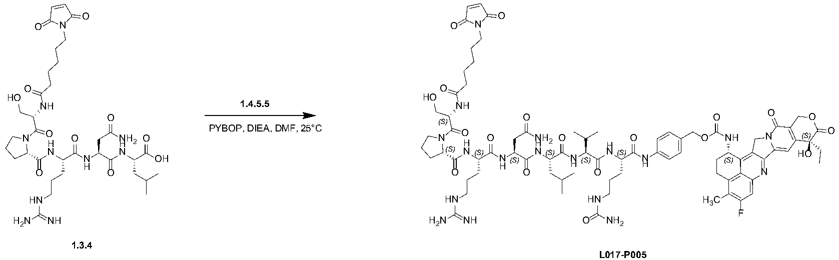

Compound 1.3.4 (30.0 mg, 38.5 µmol, 1 eq.) and compound 1.4.5.5 (32.4 mg, 38.5 µmol, 1 eq.) were dissolved in DMF (0.5 mL), and then PYBOP (40.1 mg, 77.0 µmol, 2 eq.) and DIEA (20.1 µL, 116 µmol, 3 eq.) were added to the resulting solution. The mixture was stirred at 25°C for 1 h. LC-MS monitored completion of the reaction. TFA was added to the reaction mixture to adjust the pH value. The residue was purified by preparative HPLC. A yellow solid compound L017-P005 (11.0mg, 6.11 µmol, yield: 15.9%, TFA) was obtained. ESI-MS (m/z): 1602.5[M+H]⁺.

### 1.4.19 Synthesis of L017-P002

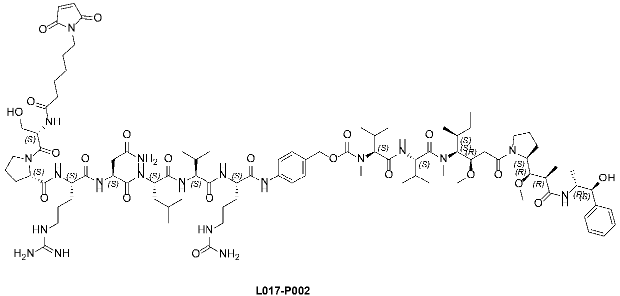

### 1.4.19.1 Synthesis of 4-((6S,9S,12S,15S,18S)-1-amino-9-(2-amino-2-oxyethyl)-6-((S)-1-(6-(2,5-dioxo-2,5-dihydro-1H-pyrrol-1-yl)hexanoyl)-L-acyl)pyrrolidin-2-carboxamido)-1-imino-12-isobutyl-15-isopropyl-7,10,13,16-tetraoxo-18-(3-ureidopropyl)-2,8,11,14,17-pentaazanonadecan-19-amido)benzyl(S)-2-((1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)-3-methyl-1-oxobutan-2-yl(methyl)carbamate (L017-P002)

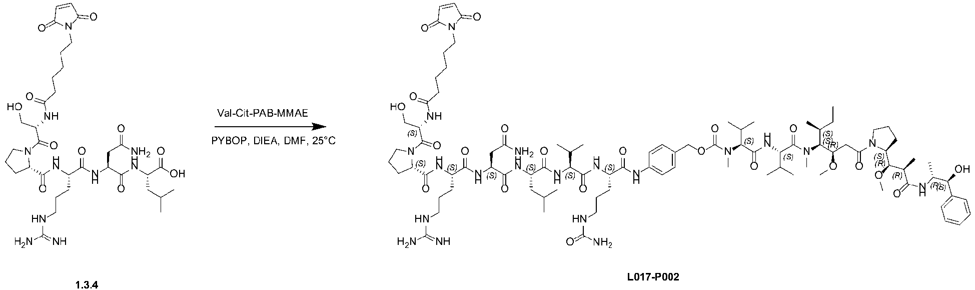

Compound 1.3.4 (30.0 mg, 38.5 µmol, 1 eq.) and compound Val-Cit-PAB-MMAE (34.6 mg, 30.8 µmol, 0.8 eq.) were dissolved in DMF (0.5 mL), and then PYBOP (40.1 mg, 77.0 µmol, 2 eq.) and DIEA (26.8 µL, 154 µmol, 4 eq.) were added to the resulting solution. The mixture was stirred at 25°C for 1 h. LC-MS monitored completion of the reaction. TFA was added to the reaction mixture to adjust the pH value. The residue was purified by preparative HPLC. A white solid compound L017-P002 (10.5 mg, 5.25 µmol, yield: 13.6%, TFA) was obtained. ESI-MS (m/z): 1885.3[M+H]⁺.

### 1.4.20 Synthesis of L021-P005

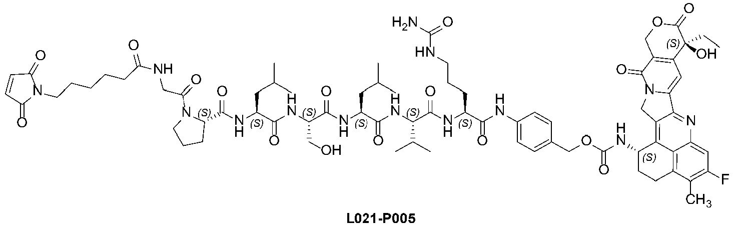

### 1.4.20.1 Synthesis of 4-((3S,6S,9S,12S,15S)-1-((S)-1-(6-(2,5-dioxo-2,5-dihydro-1H-pyrrol-1-y l)hexanoyl)glyceryl)pyrrolidin-2-yl)-6-(hydroxymethyl)-3,9-diisobutyl-12-isopropyl-1,4,7,10,13-pe ntaoxo-15-(3-ureidopropyl)-2,5,8,11,14-pentaazahexadecan-16-amido)benzyl((1S,9S)-9-ethyl-5-flu oro-9-hydroxy-4-methyl-10,13-dioxo-2,3,9,10,13,15-hexahydro-1H,12H-benzo[de]pyrano[3',4':6,7] indolizino[1,2-b]quinolin-1-yl)carbamate (L021-P005)

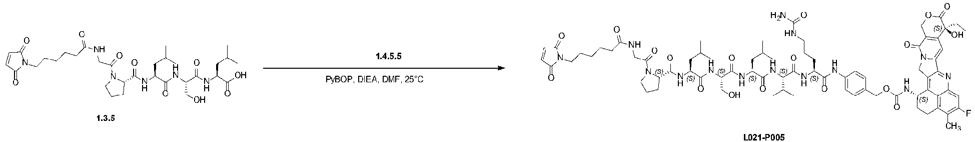

Compound 1.3.5 (55.0 mg, 81.0 µmol, 1.00 eq.) and compound 1.4.5.5 (68.1 mg, 81.1 µmol, 1.00 eq.) were dissolved in DMF (0.50 mL), and then PYBOP (84.3 mg, 162 µmol, 2.00 eq.) and DIEA (41.9 mg, 324 µmol, 56.5 µL, 4.00 eq.) were added to the resulting solution. The mixture was stirred at 25°C for 1 h. LC-MS monitored completion of the reaction. The residue was purified by preparative HPLC to obtain a yellow solid L021-P005 (25.0 mg, 16.4 µmol, yield: 20.3%, TFA). ESI-MS (m/z): 1502.8[M+H]⁺.

### 1.4.21 Synthesis of L021-P002

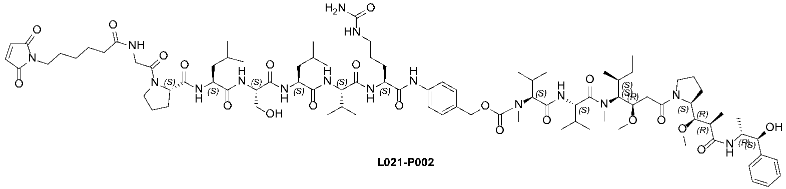

### 1. 4.21.1 Synthesis of 4-((3S,6S,9S,12S,15S)-1-((S)-1-((6-(2,5-dioxo-2,5-dihydro-1H-pyrrol-1-yl)hexyl)glycyl)pyrrolidin-2-yl)-6-(hydroxymethyl)-3,9-diisobutyl-12-isopropyl-1,4,7,10,13-pentao xo-15-(3-ureidopropyl)-2,5,8,11,14-pentaazahexadecan-16-amido)benzyl((S)-1-((S)-1-((3R,4S,5S)-1-((S)-2-((1R,2R)-3-((1S,2R)-1-hydroxy-1-phenylpropan-2-yl)amino)-1-methoxy-2-methyl-3-oxop ropyl)pyrrolidin-1-yl)-3-methoxy-5-methyl-1-oxohept-4-yl)(methyl)amino)-3-methyl-1-oxobutan-2 -yl)amino)-3-methyl-1-oxobutan-2-yl(methyl)carbamate (L021-P002)

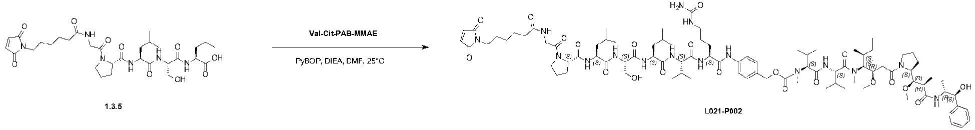

PYBOP (76.7 mg, 147 µmol, 2.00 eq.) and DIEA (38.1 mg, 295 µmol, 51.3 µL, 4.00 eq.) were added to a solution of compound 1.3.5 (50.0 mg, 73.7 µmol, 1.00 eq.) and compound Val-Cit-PAB-MMAE (82.8 mg, 73.7 µmol, 1.00 eq.) in DMF (0.50 mL). The mixture was stirred at 25°C for 1 hour. LC-MS monitored completion of the reaction. The mixture was purified by preparative HPLC to obtain a white solid L021-P002 (44.0 mg, 24.3 µmol, yield: 33.0%, TFA). ESI-MS (m/z): 1784.2[M+H]⁺.

### 1.4.22 Synthesis of L027-P005

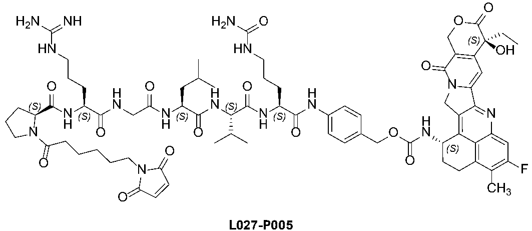

### 1.4.22.1 Synthesis of 4-((6S,12S,15S,18S)-1-amino-6-((S)-1-(6-(2,5-dioxo-2,5-dihydro-1H-pyr rol-1-yl)hexanoyl)pyrrolidin-2-carboxamido)-1-imino-12-isobutyl-15-isopropyl-7,10,13,16-tetraoxo -18-(3-ureidopropyl)-2,8,11,14,17-pentaazanonadecan-19-amido)benzyl((1S,9S)-9-ethyl-5-fluoro-9 -hydroxy-4-methyl-10,13-dioxo-2,3,9,10,13,15-hexahydro-1H,12H-benzo[de]pyrano[3',4':6,7]indol izino[1,2-b]quinolin-1-yl)carbamate (L027-P005)

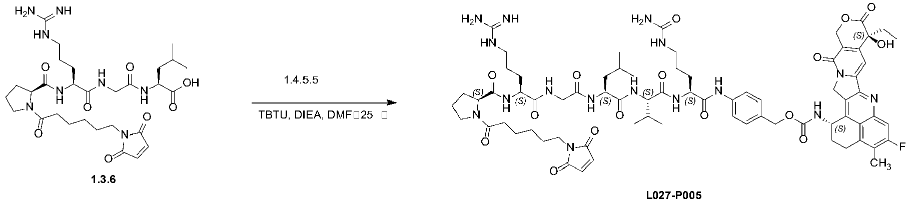

Compound 1.4.5.5 (100 mg, 118 µmol, 1.0 eq.) and compound 1.3.6 (75.4 mg, 118 µmol, 1.0 eq.) were dissolved in DMF (0.20 mL), and then TBTU (45.8 mg, 142 µmol, 1.2 eq.) and DIEA (46.1 mg, 356 µmol, 62.1 µL, 3.0 eq.) were added to the resulting solution. The mixture was stirred at 25°C for 1.0 h. LC-MS monitored completion of the reaction. The mixture was purified by preparative HPLC twice to obtain a yellow solid L027-P005 (45.0 mg, 30.8 µmol, yield: 25.9%). ESI-MS (m/z): 1458.6[M+H]⁺.

### 1.4.23 Synthesis of L028-P005

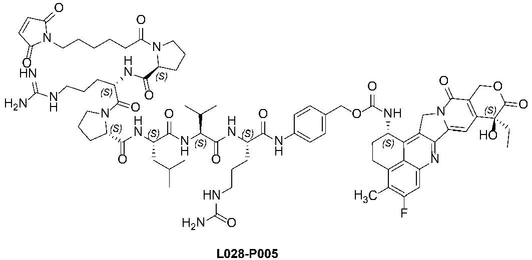

### 1.4.23.1 Synthesis of 4-((S)-2-((S)-2-((S)-2-((S)-1-((6-(2,5-dioxo-2,5-dihydro-1H-pyrrol-1-yl)h exyl)-L-prolyl-L-arginyl)pyrrolidin-2-carboxamido)-4-methylpentaamido)-3-methylbutanamido)-5-ureidopentanamido)benzyl((1S,9S)-9-ethyl-5-fluoro-9-hydroxy-4-methyl-10,13-dioxo-2,3,9,10,13-dioxohexahydro-1H,12H-benzo[de]pyrano[3',4':6,7]indeno[1,2-b]quinolin-1-yl)carbamate (L028-P0 05)

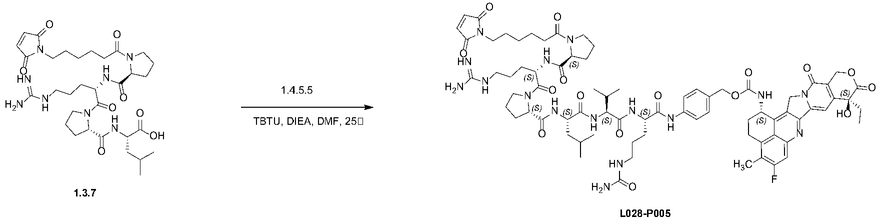

Compound 1.4.5.5 (100 mg, 118 µmol, 1.0 eq.) and compound 1.3.7 (80.2 mg, 118 µmol, 1.0 eq.) were dissolved in DMF (0.20 mL), and then TBTU (45.8 mg, 142 µmol, 1.2 eq.) and DIEA (46.1 mg, 356 µmol, 62.1 µL, 3.0 eq.) were added to the resulting solution. The mixture was stirred at 25°C for 1.0 h. LC-MS monitored completion of the reaction. The mixture was purified by preparative HPLC to obtain a yellow solid L028-P005 (36.0 mg, 24.0 µmol, yield: 20.2%). ESI-MS (m/z): 1498.0 [M+H]⁺.

### 1.4.24 Synthesis of L029-P005

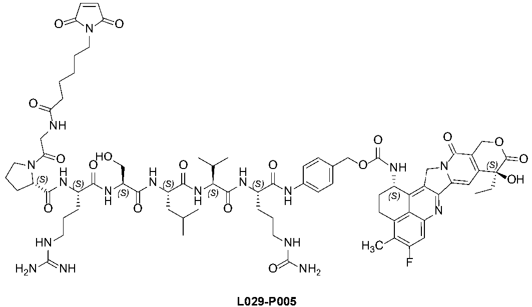

### 1.4.24.1 Synthesis of 4-((6S,9S,12S,15S,18S)-1-amino-6-((S)-1-(6-(2,5-dioxo-2,5-dihydro-1H-pyrrol-1-yl)hexanoyl)glyceryl)pyrrolidin-2-carboxamido-9-(hydroxymethyl)-1-imino-12-isobutyl-1 5-isopropyl-7,10,13,16-tetraoxo-18-(3-ureidopropyl)-2,8,11,14,17-pentaazanonadecan-19-amido)be nzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate L029-P005

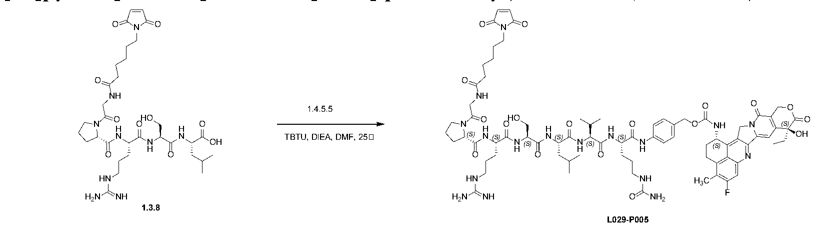

Compound 1.4.5.5 (100 mg, 118 µmol, 1.0 eq.) and compound 1.3.8 (85.8 mg, 118 µmol, 1.0 eq.) were dissolved in DMF (0.20 mL), and then TBTU (45.8 mg, 142 µmol, 1.2 eq.) and DIEA (46.1 mg, 356 µmol, 62.1 µL, 3.0 eq.) were added to the resulting solution. The mixture was stirred at 25°C for 1.0 h. LC-MS monitored completion of the reaction. The mixture was purified by preparative HPLC to obtain a yellow solid L029-P005 (65.0 mg, 42.0 µmol, yield: 35.3%). ESI-MS (m/z): 1545.5 [M+H]⁺.

### 1.4.25 Synthesis of L030-P005

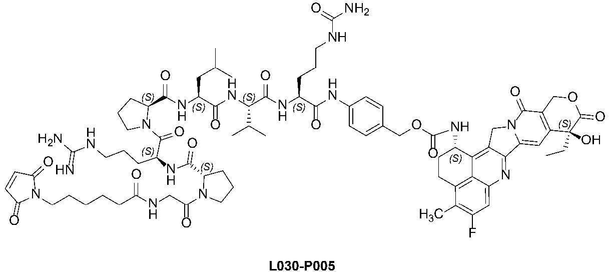

### 1.4.25.1 Synthesis of 4-((S)-2-((S)-2-((S)-2-((S)-1-((6-(2,5-dioxo-2,5-dihydro-1H-pyrrol-1-yl)h exanoyl)glycyl-L-prolyl-L-arginyl)pyrrolidin-2-carboxamido)-4-methylpentaamido)-3-methylbutyl amido)-5-ureidopentaamido)benzyl((1S,9S)-9-ethyl-5-fluoro-9-hydroxy-4-methyl-10,13-dioxo-2,3, 9,10,13-dioxo-15-hexahydro-1H,12H-benzo[de]pyrano[3',4':6,7]indolizino[1,2-b]quinolin-1-yl)carb amate (L030-P005)

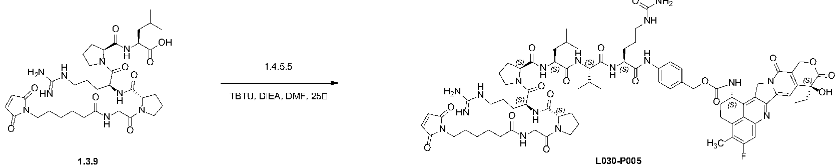

TBTU (27.4 mg, 85.6 µmol, 1.2 eq.) and DIEA (18.4 mg, 142 µmol, 24.8 µL, 2.0 eq.) were added to a solution of compound 1.3.9 (47.0 mg, 64.2 µmol, 0.9 eq.) and compound 1.4.5.5 (60.0 mg, 71.33 µmol, 1.0 eq.) in DMF (1.00 mL). The mixture was stirred at 25°C for 1.0 h. LC-MS monitored completion of the reaction. The mixture was purified by preparative HPLC twice to obtain a yellow solid L030-P005 (24.0 mg, 15.4 µmol, yield: 21.6%). ESI-MS (m/z): 1554.7 [M+H]⁺.

### 1.4.26 Synthesis of L031-P005

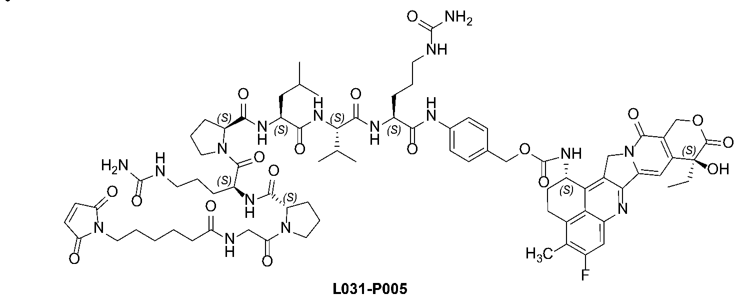

### 1.4.26.1 Synthesis of 4-((S)-2-((S)-2-((S)-2-((S)-1-((S)-2-((S)-1-((6-(2,5-dioxo-2,5-dihydro-1H -pyrrol-1-yl)hexanoyl)glycyl)pyrrolidin-2-carboxamido)-5-ureidopentanoyl)pyrrolidin-2-carboxami do)-4-methylpentanamido)-3-methylbutanamido)-5-ureidopentanamido)benzyl((1S,9S)-9-ethyl-5-fl uoro-9-hydroxy)-4-methyl-10,13-dioxo-2,3,9,10,13,15-hexahydro-1H,12H-benzo[de]pyrano[3',4':6, 7]indolo[1,2-b]quinolin-1-yl)carbamate (L031-P005)

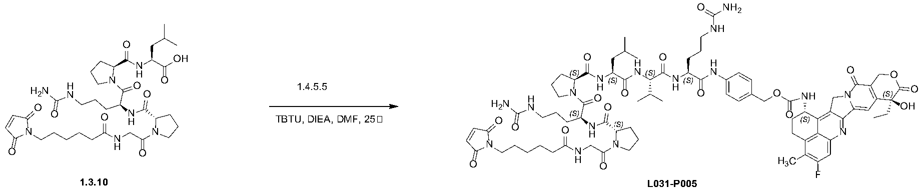

Compound 1.4.5.5 (60.0 mg, 71.3 µmol, 1.0 eq.) and compound 1.3.10 (47.0 mg, 64.2 µmol, 0.9 eq.) were dissolved in DMF (1.00 mL), and then TBTU (25.2 mg, 78.4 µmol, 1.1 eq.) and DIEA (18.4 mg, 142 µmol, 24.8 µL, 2.0 eq.) were added to the resulting solution. The mixture was stirred at 25°C for 1.0 h. LC-MS monitored completion of the reaction. The mixture was purified by preparative HPLC to obtain a yellow solid L031-P005 (14.0 mg, 9.00 µmol, yield: 12.6%). ESI-MS (m/z): 1556.7 [M+H]⁺.

### 1.4.27 Synthesis of L032-P005

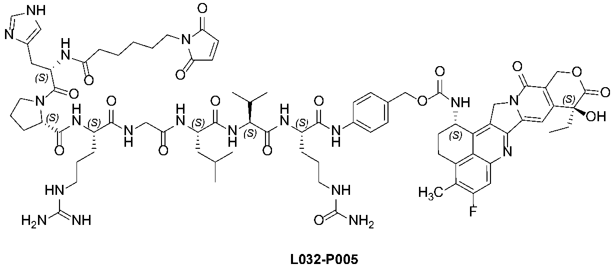

### 1.4.27.1 Synthesis of 4-((6S,12S,15S,18S)-6-((S)-1-(Na-(6-(2,5-dioxo-2,5-dihydro-1H-pyrrol-1 -yl)hexanoyl)-Nt-triphenyl-L-histidyl)pyrrolidin-2-carboxamido)-1-imino-12-isobutyl-15-isopropyl -7,10,13,16-tetraoxo-1-((2,2,4,6,7-pentamethyl-2,3-dihydrobenzofuran)-5-sulfonamido)-18-(3-ureid opropyl)-2,8,11,14,17-pentaazanonan-19-amido)benzyl((1S,9S)-9-ethyl-5-fluoro-9-hydroxy-4-meth yl-10,13-dioxo-2,3,9,10,13,15-hexahydro-1H,12H-benzo[de]pyrano[3',4':6,7]indolizino[1,2-b]quino lin-1-yl)carbamate (1.4.27.1)

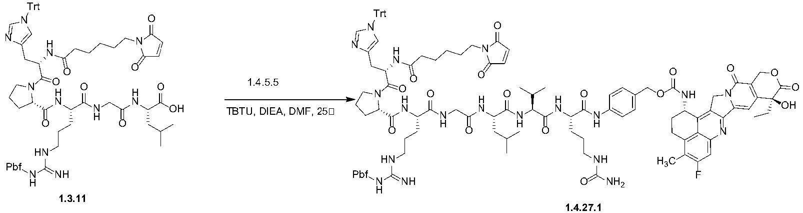

Compound 1.3.11 (225 mg, 178 µmol, 1.5 eq.), compound 1.4.5.5 (100 mg, 119 µmol, 1.0 eq.), and TBTU (49.6 mg, 155 µmol, 1.3 eq.) were dissolved in DMF (3.0 mL), and then DIEA (61.5 mg, 476 µmol, 78.7 µL, 4.0 eq.) was added to the resulting solution. The mixture was stirred at 25°C for 1.0 h. LC-MS monitored completion of the reaction. The mixture was purified by preparative HPLC to obtain a yellow solid 1.4.27.1 (53.0 mg, 25.4 µmol, yield: 21.3%). ESI-MS (m/z): 1045.4 [M/2+H]⁺.

### 1.4.27.2 Synthesis of 4-((6S,12S,15S,18S)-1-amino-6-((S)-1-((6-(2,5-dioxo-2,5-dihydro-1H-py rrol-1-yl)hexyl)-L-histidyl)pyrrolidin-2-carboxamido)-1-imino-12-isobutyl-15-isopropyl-7,10,13,16 -tetraoxo-18-(3-ureidopropyl)-2,8,11,14,17-pentaazanonan-19-amido)benzyl((1S,9S)-9-ethyl)-5-flu oro-9-hydroxy-4-methyl-10,13-dioxo-2,3,9,10,13,15-hexahydro-1H,12H-benzo[de]pyrano[3',4':6,7] indolo[1,2-b]quinolin-1-yl)carbamate (L032-P005)

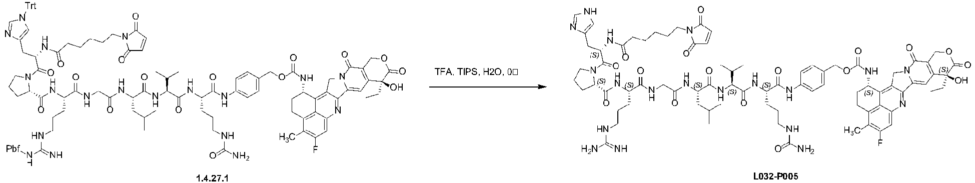

Compound 1.4.27.1 (53.0 mg, 25.4 µmol, 1.0 eq.) was cooled in a solution of TFA (1.46 g, 12.8 mmol, 950 µL, 504 eq.) and H₂O (25.0 µL) to 0°C, and then triisopropylsilane (19.3 mg, 122 µmol, 25.0 µL, 4.8 eq.) was added thereto. The mixture was stirred at 0°C for 2.0 h. LC-MS monitored completion of the reaction. The mixture was purified by preparative HPLC to obtain a yellow solid L032-P005 (10.0 mg, 6.21 µmol, yield: 24.5%). ESI-MS (m/z):1595.4 [M+H]⁺.

### 1.4.28 Synthesis of L033-P005

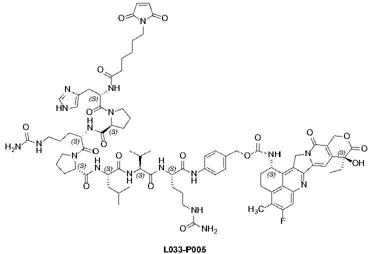

### 1.4.28.1 Synthesis of 4-((S)-2-((S)-2-((S)-2-((S)-1-((S)-2-((S)-1-(Na-(6-(2,5-dioxo-2,5-dihydro -1H-pyrrol-1-yl)hexanoyl)-Nt-trialkyl-L-histidyl)pyrrolidin-2-carboxamido)-5-ureidopentanoyl)pyrr olidin-2-carboxamido)-4-methylpentanamido)-3-methylbutylamido)-5-ureidopentanamido)benzyl ((1S,9S)-9-ethyl-5-fluoro-9-hydroxy-4-methyl-10,13-dioxo-2,3,9,10,13,15-hexahydro-1H,12H-ben zo[de]pyrano[3',4':6,7]indolizino[1,2-b]quinolin-1-yl)carbamate (1.4.28.1)

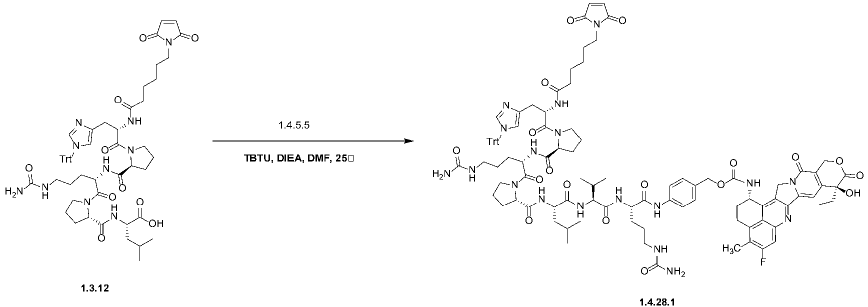

Compound 1.3.12 (60.0 mg, 56.8 µmol, 1.0 eq.) and compound 1.4.5.5 (57.4 mg, 68.2 µmol, 1.2 eq.) were dissolved in DMF (1.00 mL), and then TBTU (21.9 mg, 68.2 µmol, 1.2 eq.) and DIEA (22.1 mg, 170.6 µmol, 0.9 mL, 3.0 eq.) were added to the resulting solution. The mixture was stirred at 25°C for 3.0 h. LC-MS monitored completion of the reaction. The mixture was purified by preparative HPLC to obtain a yellow solid 1.4.28.1 (39.0 mg, 20.7 µmol, yield: 36.5%). ESI-MS (m/z):939.8 [M/2+H]⁺.

### 1.4.28.2 Synthesis of 4-((S)-2-((S)-2-((S)-2-((S)-1-((S)-2-((S)-1-((6-(2,5-dioxo-2,5-dihydro-1H -pyrrol-1-yl)hexanoyl)-L-histidyl)pyrrolidin-2-carboxamido)-5-ureidopentanoyl)pyrrolidin-2-carbo xamido)-4-methylpentanamido)-3-methylbutanamido)-5-ureidopentanamido)benzyl((1S,9S)-9-ethy 1-5-fluoro-9-hydroxy-4-methyl-10,13-dioxo-2,3,9,10,13,15-hexahydro-1H,12H-benzo[de]pyrano[3', 4':6,7]indolo[1,2-b]quinolin-1-yl)carbamate (L033-P005)

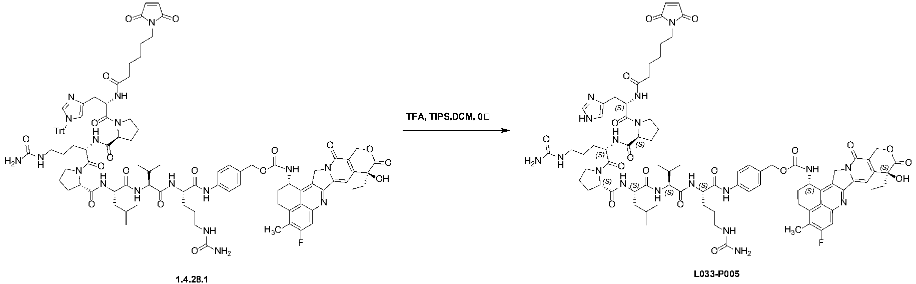

TFA (0.20 mL) and triisopropylsilane (0.05 mL) were added to a solution of compound 1.4.28.1 (39.0 mg, 20.7 µmol, 1.0 eq.) in DCM (0.80 mL). The mixture was stirred at 25°C for 0.5 h. LC-MS monitored completion of the reaction. The mixture was purified by preparative HPLC to obtain a yellow solid L033-P005 (18.9 mg, 11.5 µmol, yield: 55.6%). ESI-MS (m/z): 1635.9[M+H]⁺.

### 1.4.29 Synthesis of L034-P005

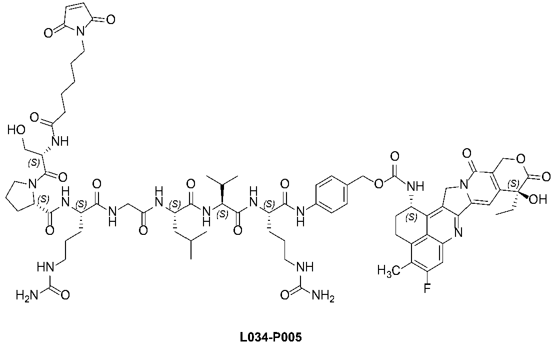

### 1.4.29.1 Synthesis of 4-((6S,12S,15S,18S)-1-amino-6-((S)-1-((6-(2,5-dioxo-2,5-dihydro-1H-py rrol-1-yl)hexanoyl)-L-seryl)pyrrolidin-2-carboxamido)-12-isobutyl-15-isopropyl-1,7,10,13,16-pent aoxo-18-(3-ureidopropyl)-2,8,11,14,17-pentaazanonan-19-amido)benzyl((1S,9S)-9-ethyl-5-fluoro-2, 3,9,10,13,15-hexahydro-1H,12H-benzo[de]pyrano[3',4':6,7]indolo[1,2-b]quinolin-1-yl)carbamate (L 034-P005)

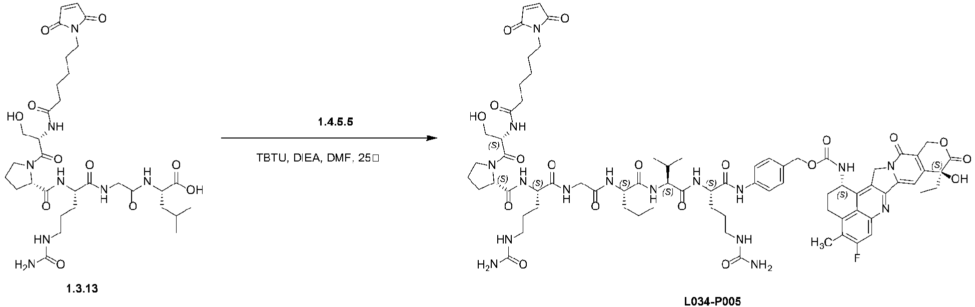

HOBt (19.3 mg, 143 µmol, 1.5 eq.), EDCI (27.4 mg, 143 µmol, 1.5 eq.), and DIEA (36.9 mg, 285 µmol, 49.7 µL, 3.0 eq.) were added to a solution of compound 1.4.5.5 (80.0 mg, 95.1 µmol, 1.0 eq.) and compound 1.3.13 (55.0 mg, 76.1 µmol, 0.8 eq.) in DMF (2.00 mL). The mixture was stirred at 25°C for 1.0 h. LC-MS monitored completion of the reaction. The mixture was purified by HPLC to obtain a yellow solid L034-P005 (20.0 mg, 36.4 µmol, yield: 38.3%). ESI-MS (m/z): 1546.3[M+H]⁺.

### 1.4.30 Synthesis of L035-P005

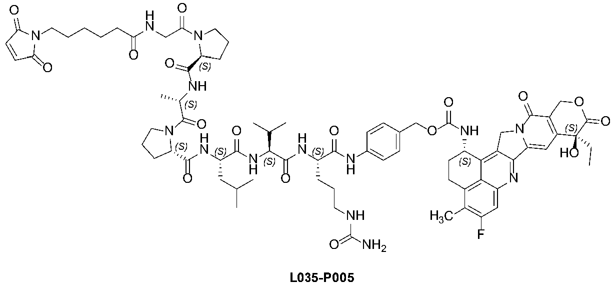

### 1.4.30.1 Synthesis of 4-((S)-2-((S)-2-((S)-2-((S)-1-((6-(2,5-dioxo-2,5-dihydro-1H-pyrrol-1-yl)h exyl)glycyl-L-prolyl-L-alanyl)pyrrolidin-2-carboxamido)-4-methylpentaamido)-3-methylbutylamin o)-5-ureidopentaamido)benzyl((1S,9S)-9-ethyl-5-fluoro-9-hydroxy-4-methyl-10,13-dioxo-2,3,9,10, 13-dioxo,15-hexahydro-1H,12H-benzo[de]pyrano[3',4':6,7]indolizino[1,2-b]quinolin-1-yl)carbamat e (L035-P005)

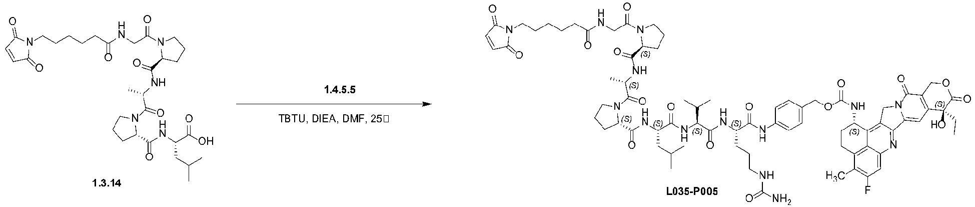

Compound 1.3.14 (55.0 mg, 85.0 µmol, 0.9 eq.) and compound 1.4.5.5 (79.5 mg, 94.5 µmol, 1.0 eq.) were dissolved in DMF (1.00 mL), and then TBTU (36.4 mg, 113 µmol, 1.2 eq.) and DIEA (22.1 mg, 171 µmol, 46.9 µL, 3.0 eq.) were added to the resulting solution. The mixture was stirred at 25°C for 1.0 h. LC-MS monitored completion of the reaction. The mixture was purified by preparative HPLC to obtain a yellow solid **L035-P005** (53.0 mg, 36.1 µmol, yield: 42.4%). ESI-MS (m/z):1469.5[M+H]⁺.

### 1.4.31 Synthesis of L037-P005

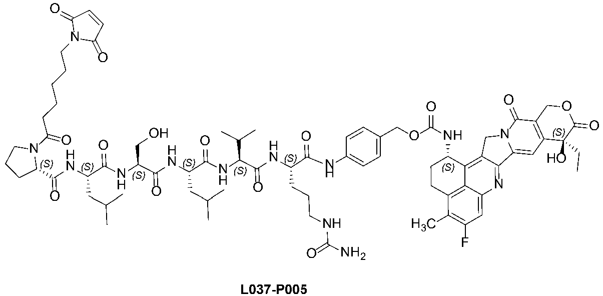

### 1.4.31.1 Synthesis of 4-((3S,6S,9S,12S,15S)-1-((S)-1-(6-(2,5-dioxo-2,5-dihydro-1H-pyrrol-1-y l)hexanoyl)pyrrolidin-2-yl)-6-(hydroxymethyl)-3,9-diisobutyl-12-isopropyl-1,4,7,10,13-pentaoxo-1 5-(3-ureidopropyl)-2,5,8,11,14-pentaazahexadecan-16-amido)benzyl((1S,9S)-9-ethyl-5-fluoro-9)-hy droxy-4-methyl-10,13-dioxo-2,3,9,10,13,15-hexahydro-1H,12H-benzo[de]pyrano[3',4':6,7]indolo[1, 2-b]quinolin-1-yl)carbamate (L037-P005)

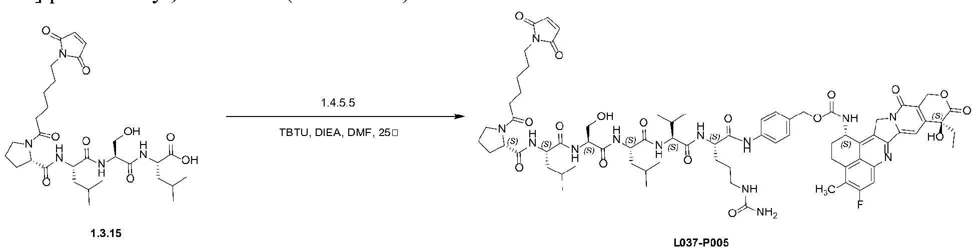

Compound 1.3.15 (19.9 mg, 32.1 µmol, 0.9 eq.) and compound 1.4.5.5 (30.0 mg, 35.6 µmol, 1.0 eq.) were dissolved in DMF (1.00 mL), and then TBTU (13.7 mg, 42.8 µmol, 1.2 eq.) and DIEA (13.8 mg, 107 µmol, 18.6 µL, 3.0 eq.) were added to the resulting solution. The mixture was stirred at 25°C for 1.0 h. LC-MS monitored completion of the reaction. The reaction mixture was purified by prep-HPLC to obtain a yellow solid L037-P005 (29.0 mg, 20.0 µmol, yield: 56.2%).

ESI-MS (m/z):1445.0[M+H]⁺; ¹H NMR: (400 MHz, DMSO-*d*₆) *δ*: 9.96 (br d, J=19.64 Hz, 1 H), 8.03-8.11 (m, 2 H), 8.00 (br d, J=8.13 Hz, 1 H), 7.93 (br dd, J=7.25, 5.50 Hz, 1 H), 7.77 (d, *J*=11.01 Hz, 1 H), 7.67-7.73 (m, 1 H), 7.58-7.65 (m, 3 H), 7.36 (d, J=8.63 Hz, 2 H), 7.31 (s, 1 H), 6.99 (d, *J*=1.38 Hz, 2 H), 6.42-6.61 (m, 1 H), 5.99 (br d, *J=*4.50 Hz, 1 H), 5.44 (s, 2 H), 5.28 (br d, *J*=3.50 Hz, 3 H), 5.07 (s, 2 H), 4.28-4.40 (m, 3 H), 4.12-4.27 (m, 3 H), 3.55-3.60 (m, 2 H), 3.37 (br d, *J*=5.75 Hz, 3 H), 3.20-3.25 (m, 1 H), 2.98-3.17 (m, 3 H), 2.88-2.96 (m, 1 H), 2.37 (s, 3 H), 2.22-2.27 (m, 1 H), 2.07-2.20 (m, 3 H), 1.94-2.04 (m, 2 H), 1.79-1.94 (m, 5 H), 1.65-1.77 (m, 2 H), 1.56-1.65 (m, 3 H), 1.39-1.53 (m, 10 H), 1.31-1.39 (m, 1 H), 1.19-1.26 (m, 1 H), 1.09-1.18 (m, 1 H), 0.84-0.88 (m, 12 H), 0.79-0.83 (m, 9 H).

### 1.4.32 Synthesis of L038-P005

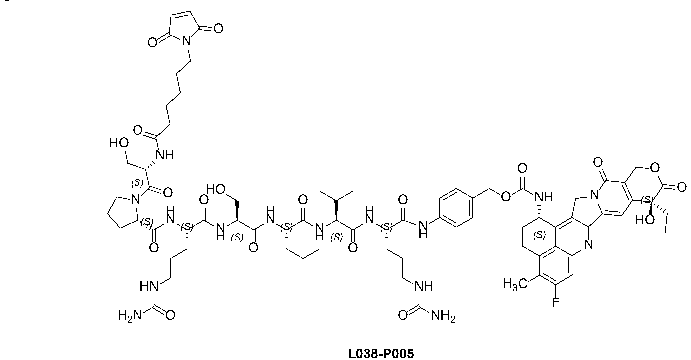

### 1.4.32.1 Synthesis of ((S)-2-((S)-1-((6-(2,5-dioxo-2,5-dihydro-1H-pyrrol-1-yl)hexanoyl)-L-seryl)pyrrolidin-2-carboxamido)-5-ureidopentanoyl)-L-seryl-L-leucine (1.4.32.1)

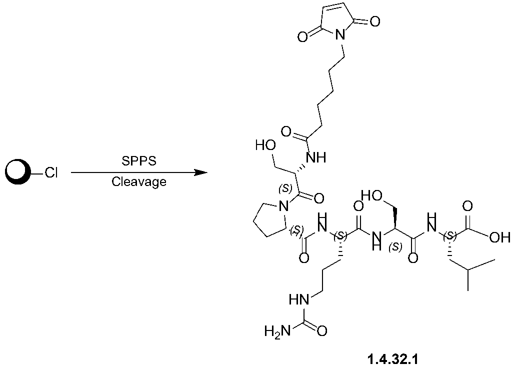

This polypeptide was synthesized using standard Fmoc chemistry method:
1) Resin preparation: In a container containing 2-CTC resin (1.50 mmol, 3.00 g, Sub = 0.50 mmol/g), Fmoc-Leu-OH (0.53 g, 1.50 mmol, 1.00 eq.) was added to DCM (30.0 mL), and subjected to N₂ bubbling, then DIEA (4.00 eq.) was added dropwise and mixed for 2 h. MeOH (3.0 mL) was then added to the N₂-bubbled resin for 30 min. The resin was washed with DMF (50 mL × 5). Then a solution of DMF (50 mL) containing 20% piperidine was added, and the mixture was bubbled with N₂ at 20°C for 30 min. The mixture was filtered to obtain the resin. Prior to the next step, the resin was washed with DMF (50 mL × 5).
2) Coupling: A solution of Fmoc-Ser(tBu)-OH (1.72 g, 3.00 eq.), HATU (1.62 g, 2.85 eq.) in DMF (20 mL) was added to the resin by bubbling N₂. Then DIEA (6.00 eq.) was added dropwise to the mixture, and the mixture was bubbled with N₂ at 20°C for 30 min. The coupling reaction was monitored by a ninhydrin method; if colorless, the coupling was complete. The resin was then washed with DMF (50 mL × 5).
3) Deprotection: a solution of DMF (50 mL) containing 20% piperidine was added to the resin, and the mixture was bubbled with N₂ at 20°C for 30 min. The deprotection reaction was monitored by the ninhydrin method; if the reaction turned blue or brownish red, the deprotection reaction was complete. The resin was then washed with DMF (50 mL × 5).
4) Steps 2) to 3) were repeated for the next amino acid coupling.
5) After the final positioning, the resin was washed with DMF (50 mL × 5) and MeOH (50 mL × 3), then dried under vacuum.

List of amino acids and corresponding reagents used for SPPS:

| # | **Materials** | **Coupling reagents** |
|---|---|---|
| 1 | Fmoc-Leu-OH (3.00 equiv.) | HATU (2.85 equiv.) and DIEA (6.00 equiv.) |
| 2 | Fmoc-Ser(tBu)-OH (3.00 equiv.) | HATU (2.85 equiv.) and DIEA (6.00 equiv.) |
| 3 | Fmoc-Cit-OH (3.00 equiv.) | HATU (2.85 equiv.) and DIEA (6.00 equiv.) |
| 4 | Fmoc-Pro-OH (3.00 equiv.) | HATU (2.85 equiv.) and DIEA (6.00 equiv.) |
| 5 | Fmoc-Ser(tBu)-OH (3.00 equiv.) | HATU (2.85 equiv.) and DIEA (6.00 equiv.) |
| 6 | 6-(2,5-dioxo-2,5-dihydro-1H-pyrrol-1-yl)hexanoic acid (3.00 equiv.) | DIEA (6.00 equiv.) |

Polypeptide cleavage, cyclization, and purification:
1) At room temperature, the cleavage mixture (TFA/3-MPA/Tis/H₂O, v/v/v, 90.0/5.0/2.5/2.5, 450 mL) was added to a flask containing the side chain-protected peptide and stirred for 2 h.
2) After filtration, the filtrate was precipitated with cold isopropyl ether and centrifuged (3,000 rpm for 3 min).
3) The residue was washed twice with isopropyl ether.
4) The crude peptide was dried under vacuum for 2 h.
5) The crude peptide was purified by prep-HPLC (A: 0.075% TFA-H₂O, B: ACN) to obtain a white solid compound **1.4.32.1** (890 mg, yield: 79.3%, purity: 99.67%).

### Purification conditions:

| | | | |
|---|---|---|---|
| Instrument | AUNO LC-2000 | | |
| Chromatographic column | Welch Ultimate XB-C18, 50*250 mm, 7µm | | |
| Wavelength | 220 nm &254nm | | |
| Column oven temperature | Ambient | | |
| Flow rate | 60.0 mL/min | | |
| Injection volume | 5 mL | | |
| Diluent | ACN/H₂O=1/6 | | |
| Mobile phase A | 0.1% TFA in water | | |
| Mobile phase B | ACN | | |

| | Time (min) | A% | B% |
|---|---|---|---|
| Gradient program | 0 | 86 | 14 |
| | 3 | 86 | 14 |
| | 43 | 56 | 44 |
| | 43.1 | 10 | 90 |
| | 48 | 10 | 90 |
| | 48.1 | 90 | 10 |
| | 53 | 90 | 10 |
| Retention time | 28.1 min | | |

ESI-MS (m/z):753.5[M+H]⁺.

### 1.4.32.2 Synthesis of 4-((6S,9S,12S,15S,18S)-1-amino-6-((S)-1-((6-(2,5-dioxo-2,5-dihydro-1H-pyrrol-1-yl)hexanoyl)-L-seryl)pyrrolidin-2-carboxamido)-9-(hydroxymethyl)-12-isobutyl-15-isopropyl-1,7,10,13,16-pentaoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L038-P005)

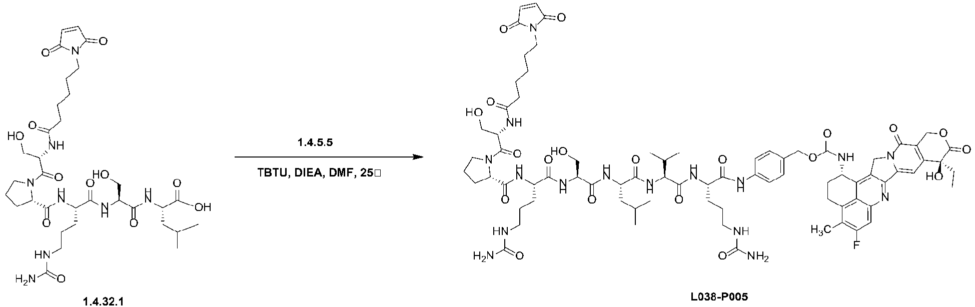

Compound 1.4.32.1 (890 mg, 1.18 mmol, 0.9 eq.) and compound 1.4.5.5 (1.1 g, 1.31 mmol, 1.0 eq.) were dissolved in DMF (20 mL), and then TBTU (506 mg, 1.57 mmol, 1.2 eq.) and DIEA (424 mg, 3.28 mmol, 543 µL, 2.5 eq.) were added to the resulting solution. The mixture was stirred at 25°C for 1.0 h. LC-MS monitored completion of the reaction. The mixture was purified by preparative HPLC to obtain a yellow solid L038-P005 (750 mg, 1.09 mmol, yield: 26.5%). ESI-MS (m/z):1575.7[M+H]⁺.

### 1.4.33 Synthesis of L038-P002

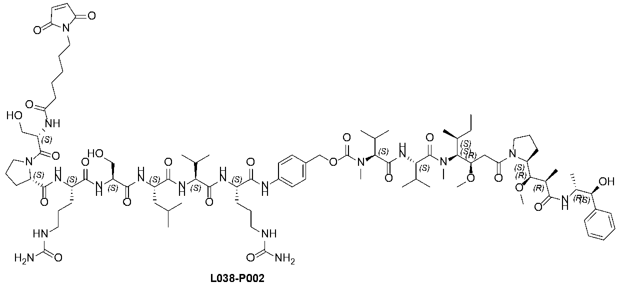

### 1.4.33.1 Synthesis of 4-((6S,9S,12S,15S,18S)-1-amino-6-((S)-1-((6-(2,5-dioxo-2,5-dihydro-1H-pyrrol-1-yl)hexanoyl)-L-seryl)pyrrolidin-2-carboxamido)-9-(hydroxymethyl)-12-isobutyl-15-isopropyl-1,7,10,13,16-pentaoxo-18-(3-ureidopropyl)-2,8,11,14,17-pentaazanonan-19-amido)benzyl((S)-1-((S)-1-((3R,4S,SS)-1-((S)-2-((1R,2R)-3-((1 S,2R)-1-hydroxy-1-phenylpropan-2-yl)amino)-1-methoxy-2-methyl-3-oxopropyl)pyrrolidin-1-yl)-3-methoxy-5-methyl-1-oxohept-4-yl)(methyl)amino)-3-methyl-1-oxobutan-2-yl)amino)-3-methyl-1-oxobutan-2-yl)(methyl)carbamate (L038-P002)

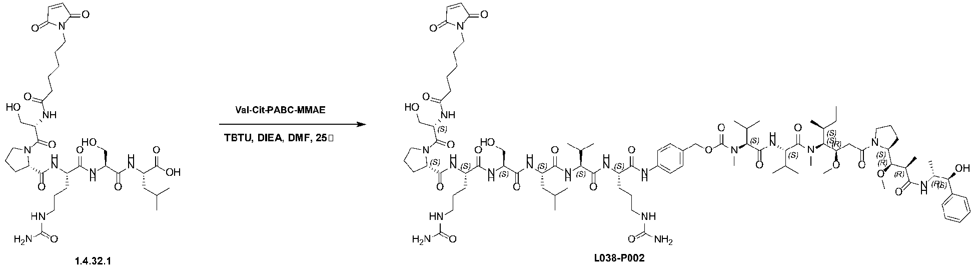

Compound 1.4.32.1 (20.0 mg, 26.6 µmol, 1.0 eq.), compound Val-Cit-PABC-MMAE (29.8 mg, 26.6 µmol, 1.0 eq.), and TBTU (10.2 mg, 31.9 µmol, 1.2 eq.) were dissolved in DMF (0.5 mL), and then DIEA (10.3 mg, 79.7 µmol, 13.2 µL, 3.0 eq.) was added to the resulting solution. The mixture was stirred at 25°C for 1.0 h. LC-MS monitored completion of the reaction. The mixture was purified by preparative HPLC to obtain a yellow solid L038-P002 (28.0 mg, 15.1 µmol, yield: 56.7%). ESI-MS (m/z):930.1[M/2+H]⁺.

### 1.4.34 Synthesis of L038-P003

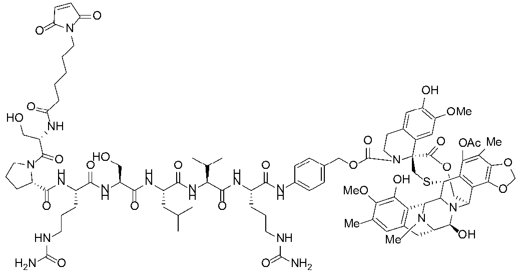

### 1.4.34.1 Synthesis of Fmoc-Val-Cit-PAB-ET743 (1.4.34.1)

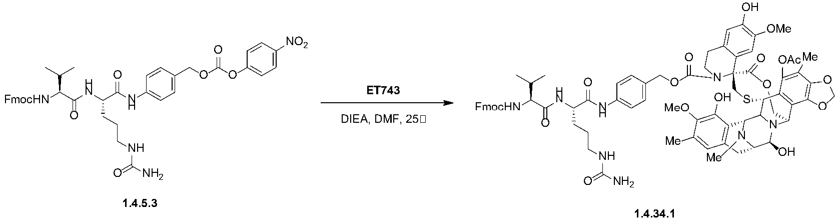

Compound 1.4.5.3 (65.0 mg, 84.8 µmol, 1.0 eq.) and compound ET743 (P003) (32.5 mg, 42.4 µmol, 0.5 eq.) were dissolved in DMF (0.50 mL), and then DIEA (21.9 mg, 170 µmol, 28.0 µL, 2.0 eq.) was added to the resulting solution. The mixture was stirred at 25°C for 2.0 h. LC-MS monitored completion of the reaction. The mixture was purified by preparative HPLC to obtain a white solid compound 1.4.34.1 (10.0 mg, 7.20 µmol, yield: 8.49%). ESI-MS (m/z):1371.7[M-OH]⁺.

### 1.4.34.2 Synthesis of Val-Cit-PAB-ET743 (1.4.34.2)

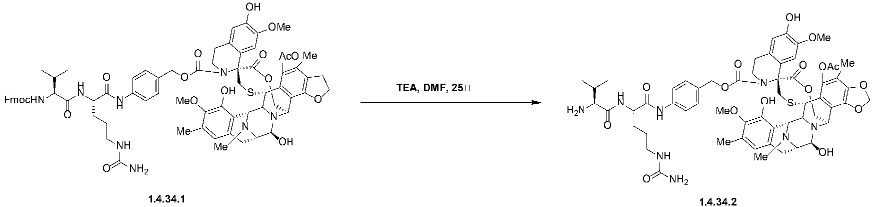

Compound 1.4.34.1 (10.0 mg, 7.20 µmol, 1.0 eq.) was dissolved in DMF (0.08 mL), and then TEA (72.7 mg, 718 µmol, 100 µL, 99.8 eq.) was added to the resulting solution. The mixture was stirred at 25°C for 4.0 h. LC-MS monitored completion of the reaction. The mixture was dropped into cold isopropyl ether (25 mL) and centrifuged to obtain a brown oily compound 1.4.34.2 (6.80 mg, crude product). ESI-MS (m/z):1149.7[M-OH]⁺.

### 1.4.34.3 Synthesis of L038-P003

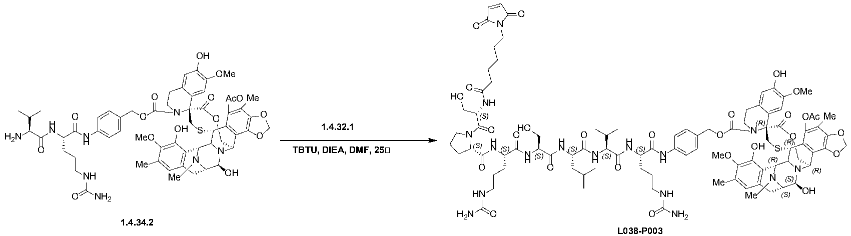

Compound 1.4.34.2 (6.80 mg, 5.83µmol, 1.0 eq.) and compound 1.4.32.1 (4.40 mg, 5.83 µmol, 1.0 eq.) were dissolved in DMF (0.10 mL), and then TBTU (2.24 mg, 6.99 µmol, 1.2 eq.) and DIEA (2.27 mg, 17.6 µmol, 2.88 µL, 3.0 eq.) were added to the resulting solution. The mixture was stirred at 25°C for 1.0 h. LC-MS monitored completion of the reaction. The mixture was purified by preparative HPLC to obtain a yellow solid L038-P003 (2.70 mg, 1.42 µmol, yield: 24.4%). ESI-MS (m/z): 1885.1[M-OH]⁺.

### 1.4.35 Synthesis of L039-P005

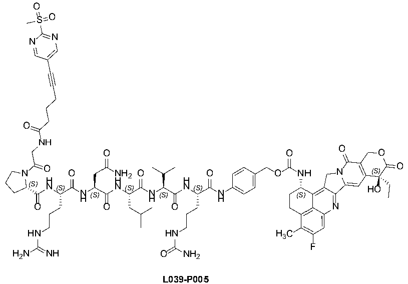

### 1.4.35.1 Synthesis of 4-((6S,9S,12S,15S,18S)-1-amino-9-(2-amino-2-oxyethyl)-1-imino-12-isobutyl-15-isopropyl-6-((S)-1-((6-(2-(methylsulfonyl)pyrimidin-5-yl)hex-5-ynoyl)glycyl)pyrrolidin-2-carboxamido)-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamate (L039-P005)

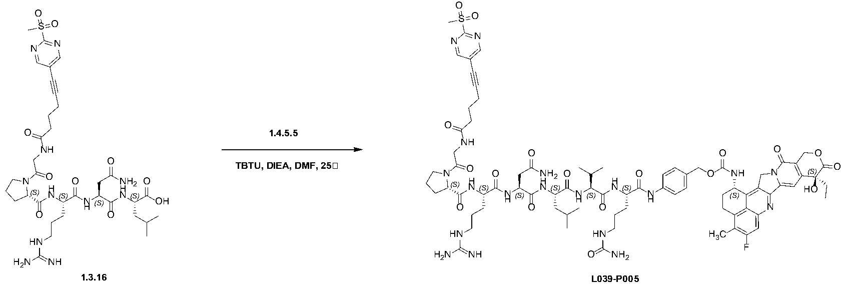

Compound 1.3.16 (17.2 mg, 21.4µmol, 0.9 eq.) and compound 1.4.5.5 (20.0 mg, 23.7 µmol, 1.0 eq.) were dissolved in DMF (0.20 mL), and then TBTU (9.16 mg, 28.5 µmol, 1.2 eq.) and DIEA (6.15 mg, 47.5 µmol, 8.29 µL, 2.0 eq.) were added to the resulting solution. The mixture was stirred at 25°C for 1.0 h. LC-MS monitored completion of the reaction. The mixture was purified by preparative HPLC to obtain a yellow solid L039-P005 (8.40 mg, 5.16 µmol, yield: 21.6%). ESI-MS (m/z):1629.1[M+H]⁺.

### 1.4.36 Synthesis of L040-P005

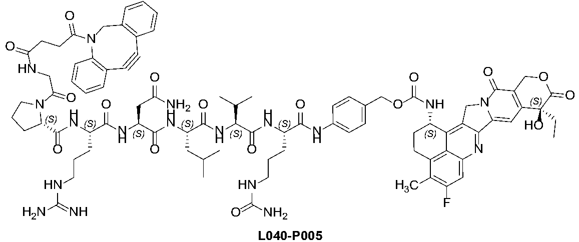

### 1.4.36.1 Synthesis of (9H-fluoren-9-yl)methyl (2-((S)-2-((6S,9S,12S,15S,18S)-1,23-diamino-15-(2-amino-2-oxyethyl)-6-((4-((((((((((1S,9S)-9-ethyl-5-fluoro-9-hydroxy-4-methyl-10,13-dioxo-2,3,9,10,13,15-hexahydro-1H,12H-benzo[3',4:6,7]indolo[1,2-b]quinolin-1-yl)carbamoyl)oxy)methyl)phenyl)carbamoyl)-23-imino-12-isobutyl-9-isopropyl-1,8,11,14,17-pentaoxo-2,7,10,13,16,22-hexaazatrichloro-18-yl)carbamoyl)pyrrolidin-1-yl)-2-oxyethyl)carbamate (1.4.36.1)

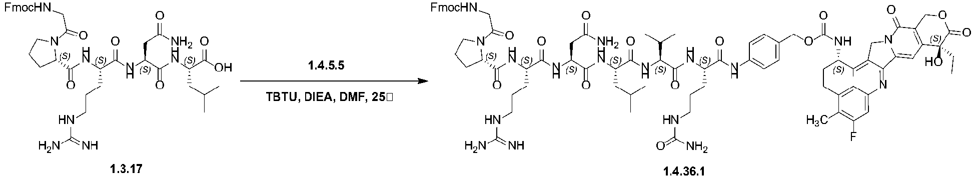

Compound 1.3.17 (92.5 mg, 118 µmol, 1.0 eq.) and compound 1.4.5.5 (100 mg, 118 µmol, 1.0 eq.) were dissolved in DMF (2.00 mL), and then TBTU (45.8 mg, 142 µmol, 1.2 eq.) and DIEA (46.1 mg, 356 µmol, 62.1 µL, 3.0 eq.) were added to the solution. The mixture was stirred at 25°C for 1.0 h. LC-MS monitored completion of the reaction. The mixture was purified by preparative HPLC to obtain a yellow solid compound 1.4.36.1 (97.0 mg, 59.7 µmol, yield: 50.2%). ESI-MS (m/z):1601.0[M+H]⁺.

### 1.4.36.2 Synthesis of 4-((6S,9S,12S,15S,18S)-1-amino-9-(2-amino-2-oxyethyl)-6-((S)-1-glycylpyrrolidin-2-carboxamido)-1-imino-12-isobutyl-15-isopropyl-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)carbamate (1.4.36.2)

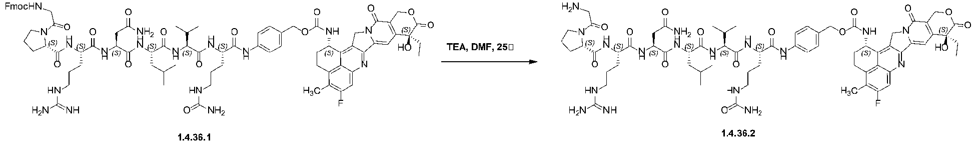

TEA (145 mg, 1.44 mmol, 0.20 mL, 30.6 eq.) was added to a solution of compound 1.4.36.1 (75.0 mg, 46.8 µmol, 1.0 eq.) in DMF (0.60 mL). The mixture was stirred at 25°C for 5.0 h. LC-MS monitored completion of the reaction. The mixture was purified by preparative HPLC to obtain a yellow solid compound 1.3.36.2 (26.0 mg, 18.6 µmol, yield: 39.8%). ESI-MS (m/z):1378.8[M+H]⁺.

### 1.4.36.3 Synthesis of L040-P005

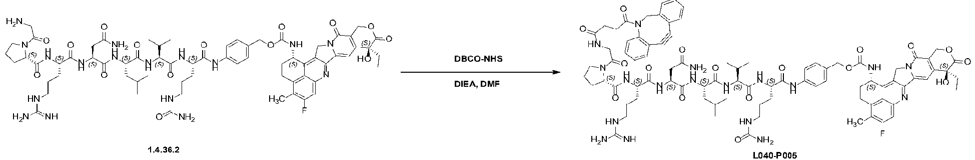

DIEA (1.21 mg, 9.37 µmol, 1.63 µL, 1.0 eq.) was added to a solution of compound 1.4.36.2 (12.9 mg, 9.37 µmol, 1.0 eq.) and DBCO-NHS (3.77 mg, 9.37 µmol, 1.0 eq.) in DMF (0.20 mL). LC-MS monitored completion of the reaction. The mixture was purified by preparative HPLC to obtain a white solid L040-P005 (6.00 mg, 3.54 µmol, yield: 37.8%).

ESI-MS (m/z):1666.0[M+H]⁺.

### 1.4.37 Synthesis of L041-P005

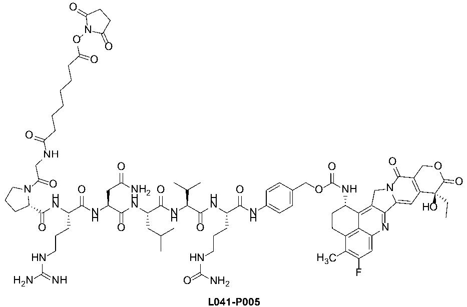

### 1.4.37.1 Synthesis of 2,5-dioxopyrrolidin-1-yl 8-((2-((S)-2-((6S,9S,12S,15S,18S)-1,23-diamino-15-(2-amino-2-oxoethyl)-6-((4-(((((((1S,95)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamoyl)oxy)methyl)phenyl)carbamoyl)-23-imino-12-isobutyl-9-isopropyl-1,8,11,14,17-pentaoxo-2,7,10,13,16,22-hexaazatrichloro-18-yl)carbamoyl)pyrrolidin-1-yl)-2-oxoethyl)amino)-8-oxooctanoate (L041-P005)

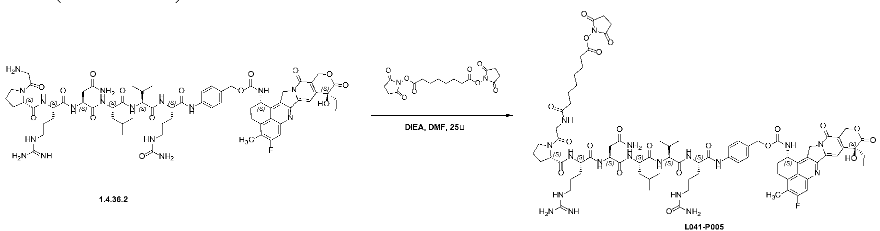

A solution of compound 1.4.36.2 (12.9 mg, 9.37 µmol, 1.0 eq.) and DIEA (1.21 mg, 9.37 µmol, 1.63 µL, 1.0 eq.) in DMF (0.20 mL) was added to a solution of compound DSS (17.2 mg, 46.8 µmol, 5.0 eq.) in DMF (0.20 mL). The mixture was stirred at 25°C for 1.0 h. LC-MS monitored completion of the reaction. The mixture was purified by preparative HPLC to obtain a white solid L041-P005 (5.00 mg, 3.05 µmol, yield: 32.5%). ESI-MS (m/z):1632.1[M+H]⁺.

### 1.4.38 Synthesis of L042-P005

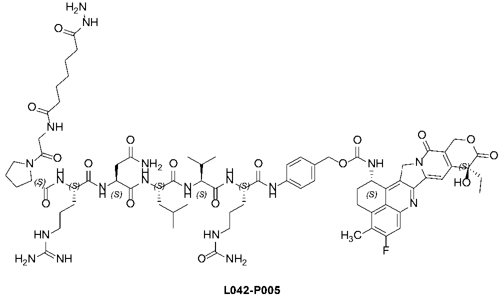

### 1.4.38.1 Synthesis of tert-butyl 7-((2-((S)-2-((6S,9S,12S,15S,18S)-1-amino-6-((4-((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)carbamoyl)oxy)phenyl)carbamoyl)-23-amino-12-isobutyl-9-isopropyl-1,8,11,14,17-pentaoxo-15-(2-oxo-2-(triphenylamino)ethyl)-23-((2,2,4,6,7-pentamethyl-2,3-dihydrobenzofuran)-5-sulfonamido)-2,7,10,13,16,22-hexaazatrichloro-18-yl)carbamoyl)pyrrolidin-1-yl)-2-oxoethyl)amino)-7-oxoheptanoate (1.4.38.1)

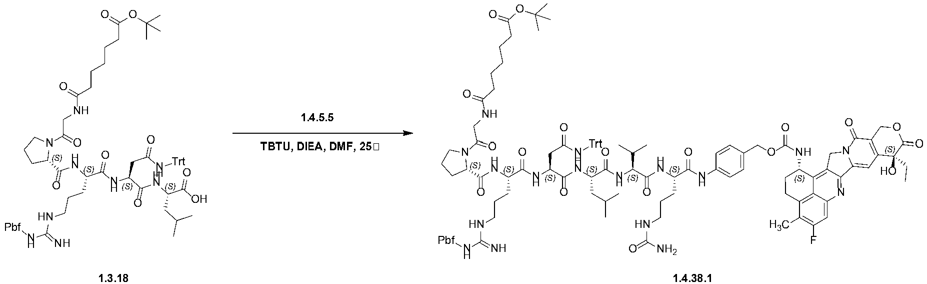

TBTU (50.4 mg, 156 µmol, 1.2 eq.) and DIEA (42.2 mg, 327 µmol, 56.9 µL, 2.5 eq.) were added to a solution of compound 1.3.18 (146 mg, 117 µmol, 0.9 eq.) and compound 1.4.5.5 (110 mg, 130 µmol, 1.0 eq.) in DMF (0.20 mL). The mixture was stirred at 25°C for 1.0 h. LC-MS monitored completion of the reaction. The mixture was purified by preparative HPLC to obtain a yellow solid compound 1.4.38.1 (182 mg, 87.86 µmol, yield: 67.17%). ESI-MS (m/z):1036.6[M/2+H]⁺.

### 1.4.38.2 Synthesis of 7-((2-((S)-2-((6S,9S,12S,15S,18S)-1,23-diamino-15-(2-amino-2-oxoethyl)-6-((4-((((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamoyl)oxy)(methyl)phenyl)carbamoyl)-23-imino-12-isobutyl-9-isopropyl-1,8,11,14,17-pentaoxo-2,7,10,13,16,22-hexaazatrichloro-18-yl)carbamoyl)pyrrolidin-1-yl)-2-oxoethyl)amino)-7-oxoheptanoic acid (1.4.38.2)

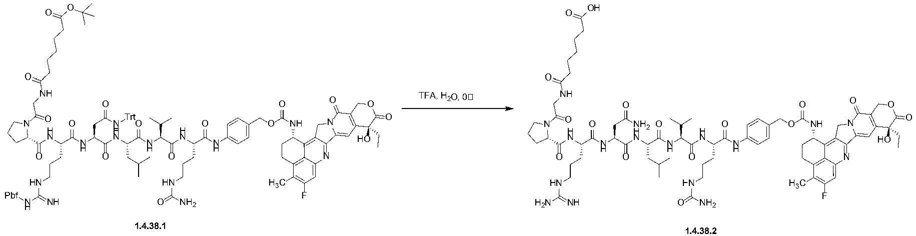

A solution of TFA (1.46 g, 12.7 mmol, 0.95 mL) in H₂O (0.025 mL) was cooled to 0°C, and compound 1.4.38.1 (111 mg, 53.5 µmol, 1.0 eq.) was cooled to 0°C in an ice bath, then the two were mixed. Then triisopropylsilane (19.2 mg, 121 µmol, 0.025 mL) was added to the mixture under stirring. The mixture was stirred at 0°C for 4.0 h. LC-MS monitored completion of the reaction. The mixture was dropped into isopropyl ether (10 mL) and filtered to obtain residue. The residue was purified by preparative HPLC to obtain a yellow solid compound 1.4.38.2 (40.0 mg, 26.3 µmol, yield: 49.0%). ESI-MS (m/z):1520.9[M+H]⁺.

### 1.4.38.3 Synthesis of tert-butyl 2-(7-((2-((S)-2-((6S,9S,12S,15S,18S)-1,23-diamino-15-(2-amino-2-oxoethyl)-6-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamate(formyl)oxy)methyl)phenyl)carbamoyl)-23-imino-12-isobutyl-9-isopropyl-1,8,11,14,17-pentaoxo-2,7,10,13,16,22-hexaazatrichloro-18-yl)carbamoyl)pyrrolidin-1-yl)-2-oxoethyl)amino)-7-oxoheptanoyl)hydrazino-1-carboxylate (1.4.38.3)

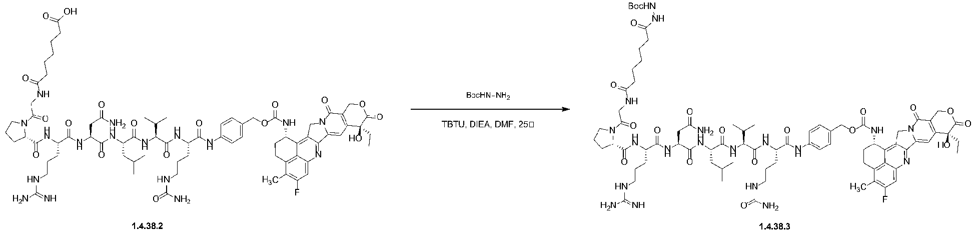

TBTU (12.6 mg, 39.4 µmol, 1.5 eq.) and DIEA (10.2 mg, 78.9 µmol, 13.7 µL, 3.0 eq.) were added to a solution of compound 1.4.38.2 (40.0 mg, 26.3 µmol, 1.0 eq.) and compound tert-butyl carbazate (5.21 mg, 39.4 µmol, 1.5 eq.) in DMF (0.50 mL). The mixture was stirred at 25°C for 1.0 h. LC-MS monitored completion of the reaction. The mixture was dropped into cold isopropyl ether (5.0 mL) and centrifuged to obtain a brown oily compound 1.4.38.3 (42.0 mg, crude product). ESI-MS (m/z):1635.8[M+H]⁺.

### 1.4.38.4 Synthesis of 4-((6S,9S,12S,15S,18S)-1-amino-9-(2-amino-2-oxyethyl)-6-((S)-1-((7-hydrazino-7-oxoheptanoyl)glycidyl)pyrrolidin-2-carboxamido)-l-imino-12-isobuty1-15-isopropyl-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L042-P005)

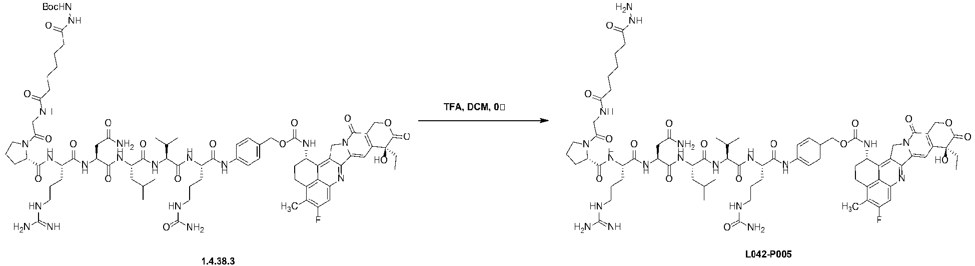

TFA (614 mg, 5.38 mmol, 0.4 mL) was added to a solution of compound 1.4.38.3 (33.0 mg, 20.1 µmol, 1.0 eq.) in DCM (0.60 mL) at 0°C. The mixture was stirred at 0°C for 0.5 h. LC-MS monitored completion of the reaction. The mixture was dropped into isopropyl ether (10 mL) and filtered to obtain residue. The residue was purified by preparative HPLC to obtain a yellow solid L042-P005 (19.0 mg, 12.3 µmol, yield: 61.3%). ESI-MS (m/z):1535.0[M+H]⁺.

### 1.4.39 Synthesis of L043-P005

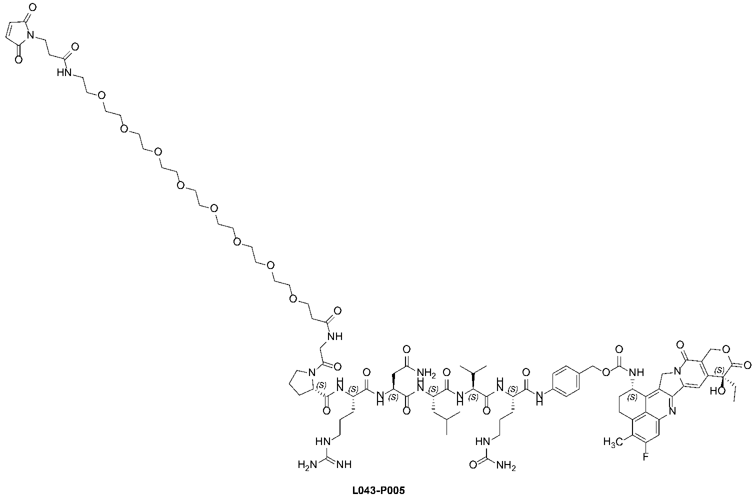

### 1.4.39.1 Synthesis of 4-((6S,9S,12S,15S,18S)-1-amino-9-(2-amino-2-oxyethyl)-6-((S)-1-(34-(2,5-dioxo-2,5-dihydro-1H-pyrrol-1-yl)-4,32-dioxo-7,10,13,16,19,22,25,28-octaoxo-3,31-diazatetraazacyclopentyl)pyrrolidin-2-carboxamido)-1-imino-12-isobutyl-15-isopropyl-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamate (L043-P005)

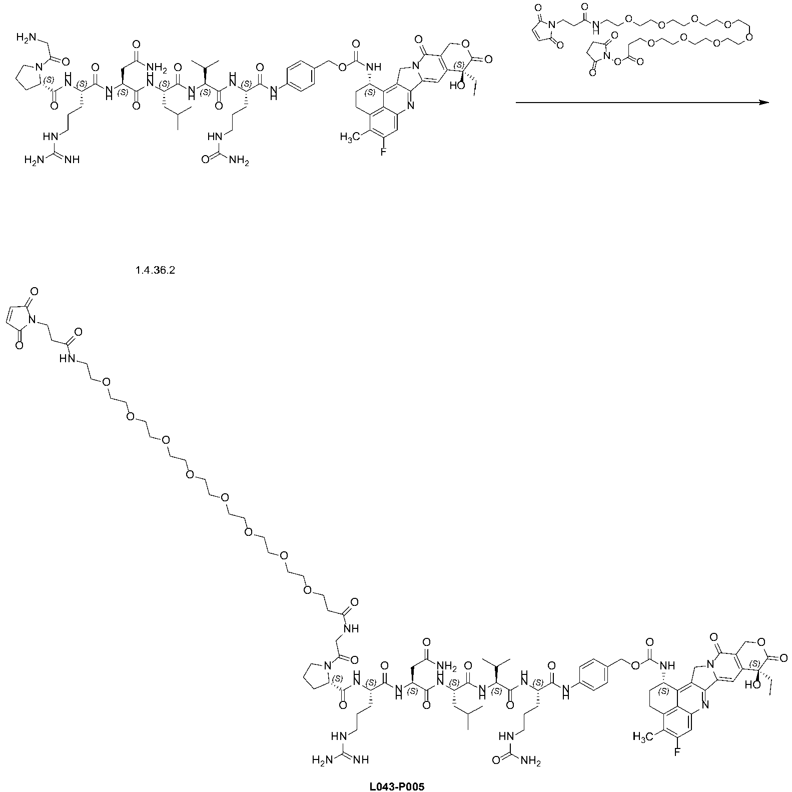

DIEA (4.88 mg, 37.7 µmol, 6.57 µL, 2.0 eq.) was added to a solution of compound 1.4.36.2 (26.0 mg, 18.8 µmol, 1.0 eq.) and compound Mal-amido-PEG8-NHS (13.0 mg, 18.8 µmol, 1.0 eq.) in DMF (0.50 mL). The mixture was stirred at 25°C for 1.0 h. LC-MS monitored completion of the reaction. The mixture was purified by preparative HPLC to obtain a white solid L043-P005 (13.0 mg, 6.39 µmol, yield: 33.8%). ESI-MS (m/z):977.4[M/2+H]⁺.

### 1.4.40 Synthesis of L045-P006

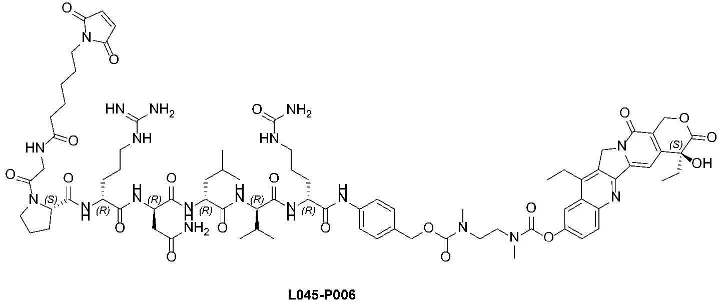

### 1.4.40.1 Synthesis of tert-butyl (2-((chlorocarbonyl)(methyl)amino)ethyl)(methyl)carbamate (1.4.40.1)

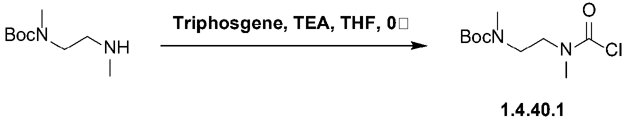

TEA (2.15 mg, 21.2 mmol, 2.0 eq.) was added to a THF (20 mL) solution of compound N-Boc-N,N'-dimethylethylamine (2.0 g, 10.6 mmol, 1.0 eq.) and triphosgene (3.15 g, 10.6 mmol, 1.0 eq.). The mixture was stirred at 0°C for 3.0 h. The mixture was filtered, and the filtrate was concentrated under reduced pressure to obtain a crude product. The crude product was purified by column chromatography (SiO₂, PE/EA=100/1 to 0/1) to obtain a light yellow oil compound 1.4.40.1 (1.90 g, 7.85 mmol, yield: 73.9%). ¹H NMR: (400 MHz, CHLOROFORM-*d*): δ ppm 3.43-3.58 (m, 2 H), 3.35-3.41 (m, 2 H), 2.98-3.15 (m, 3 H), 2.78-2.87 (m, 3 H), 1.40 (br s, 9 H).

### 1.4.40.2 Synthesis of (S)-tert-butyl(4,11-diethyl-4-hydroxy-3,14-dioxo-3,4,12,14-tetrahydro-1H-pyrano[3',4':6,7]indolo[1,2-b]quinolin-9-yl)ethane-1,2-diylbis(methylcarbamate) (1.4.40.2)

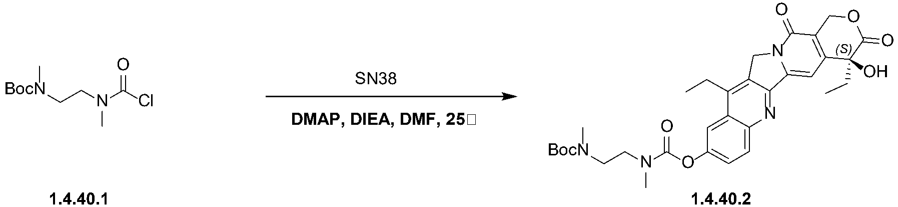

DIEA (515 mg, 3.99 mmol, 659 µL, 5.0 eq.) and DMAP (97.5 mg, 797 µmol, 1.0 eq.) were added to a DMF (2.00 mL) solution of compound SN38 (313 mg, 797 µmol, 1.0 eq.). Compound 1.4.40.1 (200 mg, 798 µmol, 1.0 eq.) was then added to the mixture in an ice bath. The mixture was stirred at 25°C for 3.0 h. LC-MS monitored completion of the reaction. The mixture was quenched with water (10 mL) and extracted with ethyl acetate (10 mL × 3). The organic layers were combined and washed with saline water, dried over Na₂SO4, and concentrated under reduced pressure to obtain a yellow solid compound 1.4.40.2 (570 mg, crude product). ESI-MS (m/z):607.2[M+H]⁺.

### 1.4.40.3 Synthesis of (S)-4,11-diethyl-4-hydroxy-3,14-dioxo-3,4,12,14-tetrahydro-1H-pyrano[3',4':6,7]indolo[1,2-b]quinolin-9-ylmethyl(2-(methylamino)ethyl)carbamate (1.4.40.3)

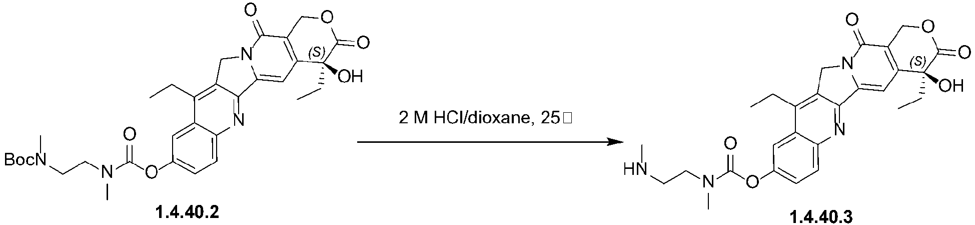

A mixture of compound 1.4.40.2 (400 mg, 659 µmol, 1.0 eq.) in 2 M HCl/dioxane (4.00 mL) was stirred at 25°C for 2.0 h. LC-MS monitored completion of the reaction. The reaction mixture was concentrated under reduced pressure to obtain a yellow solid compound 1.4.40.3 (423 mg, crude product). ESI-MS (m/z):507.0[M+H]⁺.

### 1.4.40.4 Synthesis of 4-((R)-2-((R)-2-((((9H-fluoren-9-yl)methoxy)carbonyl)-3-methylbutylamino)-5-ureidopentanamido)benzyl)((S)-4,11-diethyl-4-hydroxy-3,14-dioxo-3,4,12,14-tetrahydro-1H-pyrano[3',4':6,7]indolizino[1,2-b]quinolin-9-yl)ethane-1,2-diacylbis(methylcarbamate) (1.4.40.4)

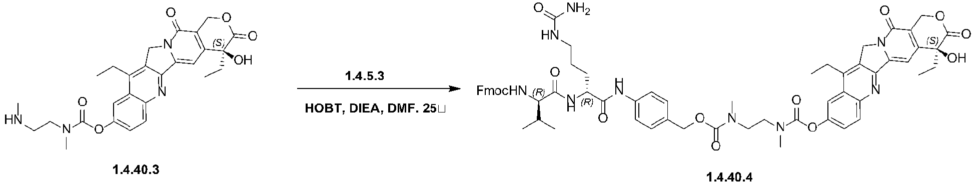

HOBt (64.0 mg, 473 µmol, 1.2 eq.) and DIEA (102 mg, 790 µmol, 138 µL, 2.0 eq.) were added to a mixture of compound 1.4.40.3 (200 mg, 394 µmol, 1.0 eq.) and compound 1.4.5.3 (303 mg, 395 µmol, 1.0 eq.) in DMF (2.0 mL). The mixture was stirred at 25°C for 1.0 h. LC-MS monitored completion of the reaction. The mixture was ground with IPE (20 mL) at 0°C for 10 min to obtain a yellow oily compound 1.4.40.4 (500 mg, crude product). ESI-MS (m/z):1134.5[M+H]⁺.

### 1.4.40.5 Synthesis of 4-((R)-2-((R)-2-amino-3-methylbutylamino)-5-ureidopentaamino)benzyl((S)-4,11-diethyl-4-hydroxy-3,14-dioxo-3,4,12,14-tetrahydro-1H-pyrano[3',4':6,7]indolizino[1,2-b]quinolin-9-yl)ethane-1,2-bis(methylcarbamate) (1.4.40.5)

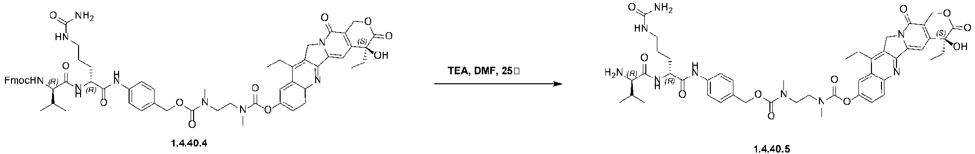

The mixture of compound 1.4.40.4 (500 mg, 441 µmol, 1.0 eq.) in TEA (1.00 mL) and DMF (4.00 mL) was stirred at 25°C for 6.0 h. LC-MS monitored completion of the reaction. The mixture was purified by preparative HPLC to obtain a yellow solid compound 1.4.40.5 (181 mg, 188 µmol, yield: 42.6%). ESI-MS (m/z):912.4[M+H]⁺.

### 1.4.40.6 Synthesis of 4-((6R,9R,12R,15R,18R)-1-amino-9-(2-amino-2-oxyethyl)-6-((S)-1-((6-(2,5-dioxo-2,5-dihydro-1H-pyrrol-1-yl)hexanoyl)glycyl)pyrrolidin-2-carboxamido)-1-imino-12-isobutyl-15-isopropyl-7,10,13,16-tetraoxo-18-(3-ureidopropyl)-2,8,11,14,17-pentaazanonan-19-amido)benzyl((S)-4,11-diethyl-4-hydroxy-3,14-dioxo-3,4,12,14-tetrahydro-1H-pyrano[3',4':6,7]indolo[1,2-b]quinolin-9-yl)ethane-1,2-diacylbis(methylcarbamate) (L045-P006)

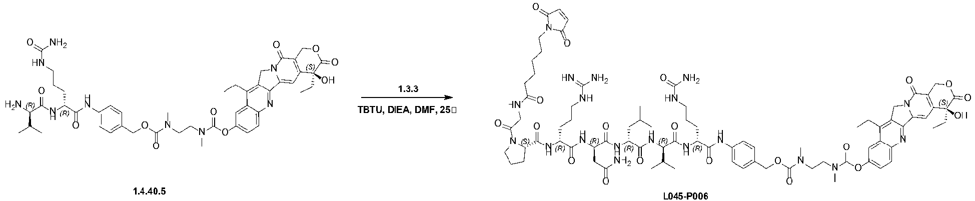

A mixture of compound 1.4.40.5 (30.0 mg, 33.8 µmol, 1.0 eq.), compound 1.3.3 (26.0 mg, 33.8 µmol, 1.0 eq.), and TBTU (13.0 mg, 40.5 µmol, 1.2 eq.) in DMF (0.20 mL) was added to DIEA (13.1 mg, 101 µmol, 17.7 µL, 3.0 eq.), and then the mixture was stirred under N₂ atmosphere at 25°C for 1.0 h. LC-MS monitored completion of the reaction. The mixture was purified by prep-HPLC to obtain a white solid compound L045-P006 (12.6 mg, 6.93 µmol, yield: 20.6%). ESI-MS (m/z):822.3[M/2+H]⁺.

### 1.4.41 Synthesis of L047-P001

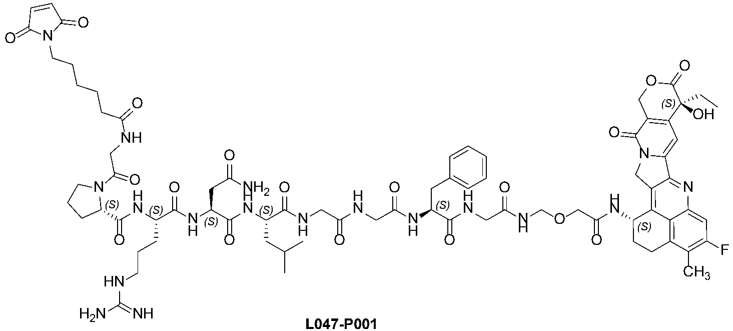

### 1.4.41.1 Synthesis of (S)-2-((S)-2-((S)-1-((6-(2,5-dioxo-2,5-dihydro-1H-pyrrol-1-yl)hexanoyl)glycyl)pyrrolidin-2-carboxamido)-5-guanidopentanamido)-N1-((S)-1-((2-((S)-1-((2-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)amino)-2-oxoethoxy)methyl)amino)-2-oxyethyl)amino)-1-oxo-3-phenylpropan-2-yl)amino)-2-oxyethyl)amino)-2-oxyethyl)amino)-4-methyl-1-oxopentan-2-yl)butanediamide (L047-P001)

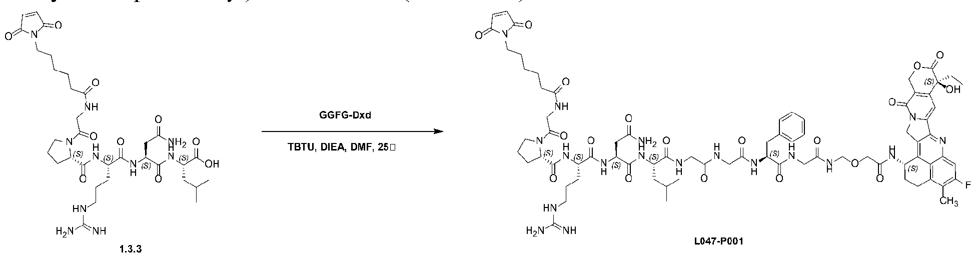

TBTU (12.9 mg, 40.2 µmol, 1.2 eq.) and DIEA (12.9 mg, 100 µmol, 16.6 mL, 3.0 eq.) were added to a solution of compound 1.3.3 (25.0 mg, 33.4 µmol, 1.0 eq.) and compound GGFG-Dxd (GGFG-P001, commercially available) (28.1 mg, 33.4 µmol, 1.0 eq.) in DMF (0.5 mL). The mixture was stirred at 25°C for 1.0 h. LC-MS monitored completion of the reaction. The mixture was purified by preparative HPLC to obtain a yellow solid L047-P001 (24.0 mg, 15.3 µmol, yield: 45.7%). ESI-MS (m/z):1571.7[M+H]⁺.

### 1.4.42 Synthesis of L048-P001

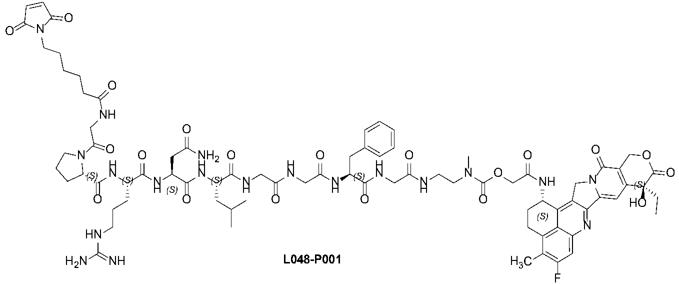

### 1.4.42.1 Synthesis of 2-(tert-butoxy)-N-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)acetamide (1.4.42.1)

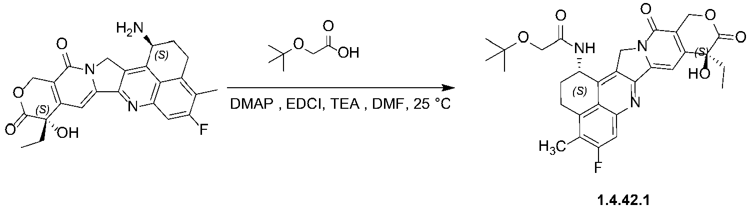

DMAP (345 mg, 2.82 mmol, 0.5 eq.) and TEA (856 mg, 8.47 mmol, 1.18 mL, 1.5 eq.) were added to a solution of compound Exatecan (3.00 g, 5.64 mmol, 1.0 eq.) and compound 2-(tert-butoxy)acetic acid (896 mg, 6.77 mmol, 1.2 eq.) in DMF (1.0 mL), followed by the addition of EDCI (2.16 g, 11.3 mmol, 2.0 eq.), and then the mixture was stirred at 25°C for 1.0 h. LC-MS monitored completion of the reaction. The reaction mixture was dropped into a stirred solution of isopropyl ether (200 mL × 2), and a translucent white precipitate gradually formed during the addition. The precipitate was dried under reduced pressure to obtain a gray solid compound 1.4.42.1 (5.00 g, crude product). ESI-MS (m/z):550.2[M+H]⁺.

### 1.4.42.2 Synthesis of N-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide (1.4.42.2)

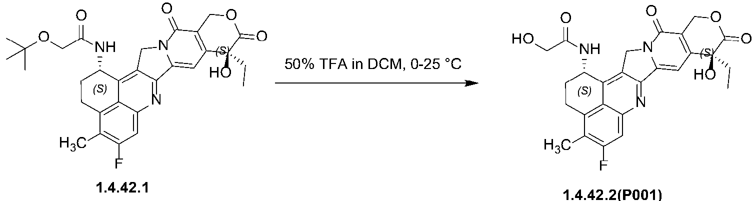

TFA (38.4 g, 336 mmol, 25 mL, 37 eq.) was added to a solution of compound 1.4.42.1 (5.00 g, 9.10 mmol, 1.0 eq.) in DCM (25.0 mL). The mixture was stirred at 0-25°C for 2.0 h. LC-MS monitored completion of the reaction. The mixture was ground with isopropyl ether (200 mL × 2) at 0-5°C for 5 min, and concentrated under reduced pressure to obtain a residue. The residue was purified by preparative HPLC to obtain a yellow solid compound 1.4.42.2 (P001) (1.30 g, 2.61 mmol, yield: 28.6%). ESI-MS (m/z):494.1[M+H]⁺.

### 1.4.42.3 Synthesis of tert-butyl (2-(chlorocarbonyl)(methyl)amino)ethyl)carbamate (1.4.42.3)

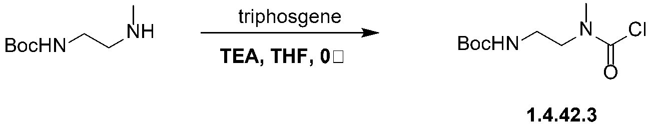

TEA (1.74 g, 17.2 mmol, 2.40 mL, 3.0 eq.) and compound tert-butyl 2-(methylamino)ethylcarbamate (1.00 g, 5.74 mmol, 1.0 eq.) were added to a solution of triphosgene (1.70 g, 5.75 mmol, 1.0 eq.) in THF (10 mL). The mixture was stirred at 0°C for 0.5 h. TLC (PE:EA=1:1) showed that compound 1-68 (Rf=0.20) was completely consumed and a new spot (Rf=0.50) was formed. The mixture was concentrated under reduced pressure to obtain a crude product. The crude product was purified by silica-gel column chromatography (SiO₂, PE/EA=100/1 to 0/1) to obtain a colorless oily compound 1.4.42.3 (998 mg, 4.22 mmol, yield: 73.5%).

¹H NMR: (400 MHz, CHLOROFORM-d): δ ppm 4.79 (br s, 1 H), 3.55-3.65 (m, 1 H), 3.49-3.53 (m, 1 H), 3.34 (br s, 2 H), 3.16 (s, 2 H), 3.07 (s, 1 H), 1.43 (s, 9 H).

### 1.4.42.4 Synthesis of 2-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)amino)-2-oxyethyl(2-((tert-butoxycarbonyl)amino)ethyl)(methyl)carbamate (1.4.42.4)

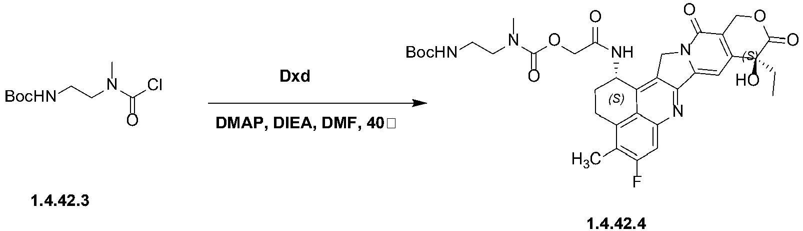

DIEA (258 mg, 2.0 mmol, 330 µL, 5.0 eq.) was added to a solution of 1.4.42.2 (Dxd) (197 mg, 399 µmol, 1.0 eq.) and DMAP (49.0 mg, 401 µmol, 1.0 eq.) in DMF (2.00 mL). Compound 1.4.42.3 (189 mg, 798 µmol, 2.0 eq.) was then added to the mixture in an ice bath. The mixture was stirred at 40°C for 16 h. LC-MS monitored completion of the reaction. The mixture was purified by preparative HPLC to obtain a yellow solid compound 1.4.42.4 (71.0 mg, 102 µmol, yield: 25.6%). ESI-MS (m/z):694.4[M+H]⁺.

### 1.4.42.5 Synthesis of 2-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indeno[1,2-b]quinolin-1-yl)amino)-2-oxyethyl(2-aminoethyl)(methyl)carbamate (1.4.42.5)

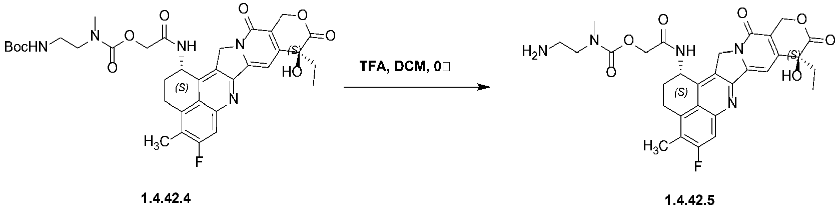

TFA (10.1 mg, 87.6 µmol, 6.53 µL, 1.0 eq.) was added to a mixture of compound 1.4.42.4 (61.0 mg, 87.6 µmol, 1.0 eq.) in DCM (825 µL). The mixture was stirred at 0°C for 1.0 h. LC-MS monitored completion of the reaction. The mixture was dropped into isopropyl ether (10.0 mL) to obtain a residue. The residue was purified by preparative HPLC to obtain a yellow solid compound 1.4.42.5 (25.0 mg, 42.1 µmol, yield: 47.9%). ESI-MS (m/z):594.2[M+H]⁺.

### 1.4.42.6 Synthesis of 2-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-oxyethyl((6S,9S,12S,21S)-1-amino-9-(2-amino-2-oxyethyl)-21-benzyl-6-((S)-1-((6-(2,5-dioxo)-2,5-dihydro-1H-pyrrol-1-yl)hexanoyl)glycyl)pyrrolidin-2-carboxamido)-1-imino-12-isobutyl-7,10,13,16,19,22,25-heptaoxo-2,8,11,14,17,20,23,26-octaazaoctacosan-28-yl)(methyl)carbamate (L048-P001)

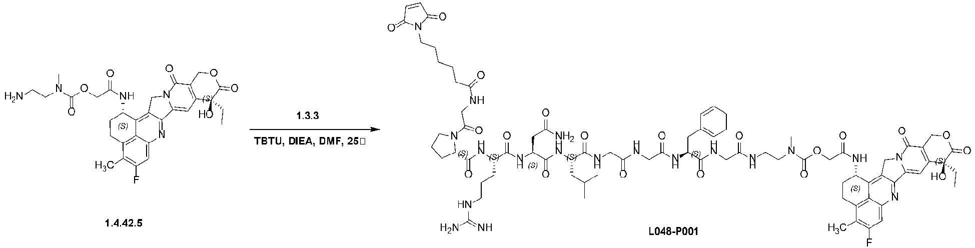

TBTU (16.2 mg, 50.5 µmol, 1.2 eq.) and DIEA (16.3 mg, 126 µmol, 20.8 µL, 3.0 eq.) were added to a solution of compound 1.4.42.5 (25.0 mg, 42.1 µmol, 1.0 eq.) and compound 1.3.3 (45.0 mg, 42.1 µmol, 1.0 eq.) in DMF (0.50 mL). The mixture was stirred at 25°C for 1.0 h. LC-MS monitored completion of the reaction. The mixture was purified by preparative HPLC to obtain a yellow solid compound L048-P001 (15.0 mg, 9.13 µmol, yield: 21.7%). ESI-MS (m/z):1643.0[M+H]⁺.

### 1.4.43 Synthesis of L049-P005

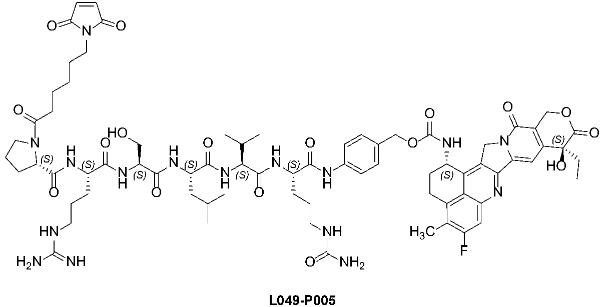

### 1.4.43.1 Synthesis of 4-((6S,9S,12S,15S,18S)-1-amino-6-((S)-1-(6-(2,5-dioxo-2,5-dihydro-1H-pyrrol-1-yl)hexyl)pyrrolidin-2-carboxamido)-9-(hydroxymethyl)-1-imino-12-isobutyl-15-isopropyl-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamate (L049-P005)

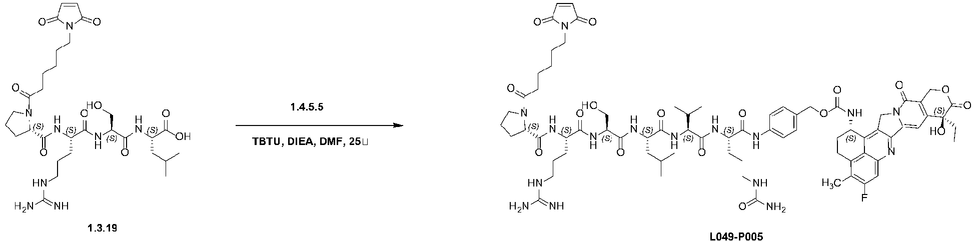

TBTU (29.7 mg, 92.7 µmol, 1.2 eq.) and DIEA (19.9 mg, 154 µmol, 26.9 µL, 2.0 eq.) were added to a solution of compound 1.4.5.5 (65.0 mg, 77.3 µmol, 1.0 eq.) and compound 1.3.19 (46.2 mg, 69.5 µmol, 0.9 eq.) in DMF (1.10 mL). The mixture was stirred at 25°C for 1.0 h. LC-MS monitored completion of the reaction. The mixture was purified by preparative HPLC to obtain a yellow solid compound L049-P005 (45.0 mg, 30.2 µmol, yield: 39.1%). ESI-MS (m/z):1488.8[M+H]⁺.

### 1.4.44 Synthesis of L050-P005

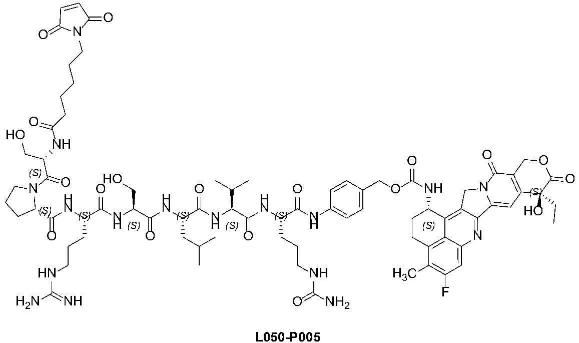

### 1.4.44.1 Synthesis of 4-((6S,9S,12S,15S,18S)-1-amino-6-((S)-1-((6-(2,5-dioxo-2,5-dihydro-1H-pyrrol-1-yl)hexanoyl)-L-seryl)pyrrolidin-2-carboxamido)-9-(hydroxymethyl)-1-imino-12-isobutyl-15-isopropyl-7,10,13,16-tetraoxo-18-(3-ureidopropyl)-2,8,11,14,17-pentaazanonan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolo[1,2-b]quinolin-1-yl)carbamate (L050-P005)

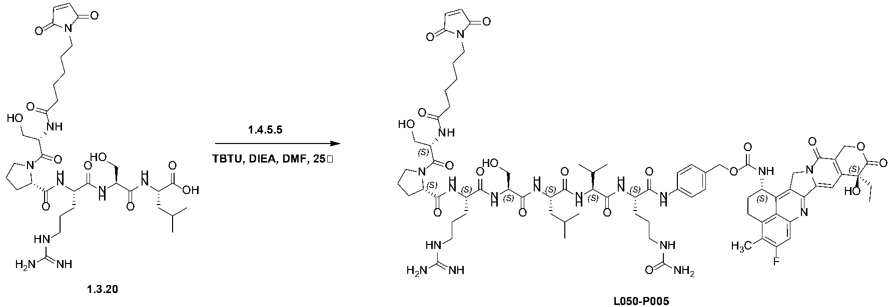

DIEA (10.3 mg, 79.8 µmol, 13.2 µL, 3.0 eq.) was added to a solution of compound 1.3.20 (20.0 mg, 26.6 µmol, 1.0 eq.), compound 1.4.5.5 (24.0 mg, 29.3 µmol, 1.1 eq.), and TBTU (10.3 mg, 31.9 µmol, 1.2 eq.) in DMF (0.50 mL). The mixture was stirred at 25°C for 1.0 h. LC-MS monitored completion of the reaction. The mixture was purified by preparative HPLC to obtain a yellow solid compound L050-P005 (22.0 mg, 14.0 µmol, yield: 52.5%). ESI-MS (m/z):1574.8[M+H]⁺.

### 1.4.45 Synthesis of L051-P005

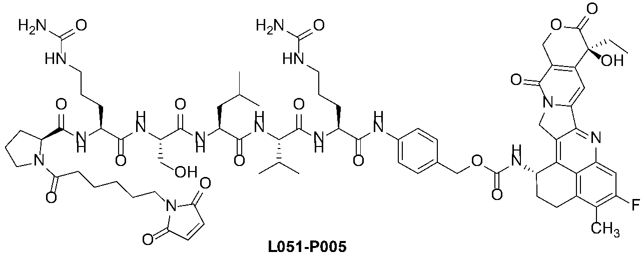

### 1.4.45.1 Synthesis of 4-((6S,9S,12S,15S,18S)-1-amino-6-((S)-1-(6-(2,5-dioxo-2,5-dihydro-1H-pyrrol-1-yl)hexanoyl)pyrrolidin-2-carboxamido)-9-(hydroxymethyl)-12-isobutyl-15-isopropyl-1,7,10,13,16-pentaoxo-18-(3-ureidopropyl)-2,8,11,14,17-pentaazanonadecan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L051-P005)

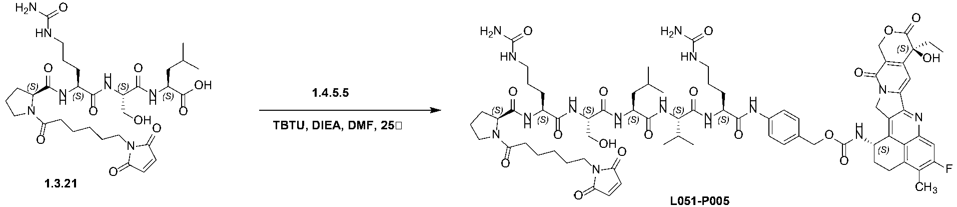

DIEA (11.6 mg, 90.1 µmol, 14.9 µL, 3.0 eq.) was added to a solution of compound 1.3.21 (20.0 mg, 30.0 µmol, 1.0 eq.), compound 1.4.5.5 (27.8 mg, 33.3 µmol, 1.1 eq.), and TBTU (11.6 mg, 36.1 µmol, 1.2 eq.) in DMF (0.50 mL). The mixture was stirred at 25°C for 1.0 h. LC-MS monitored completion of the reaction. The mixture was purified by preparative HPLC to obtain a yellow solid compound L051-P005 (12.0 mg, 7.75 µmol, yield: 25.7%). ESI-MS (m/z):1489.9[M+H]⁺.

### 1.4.46 Synthesis of L052-P005

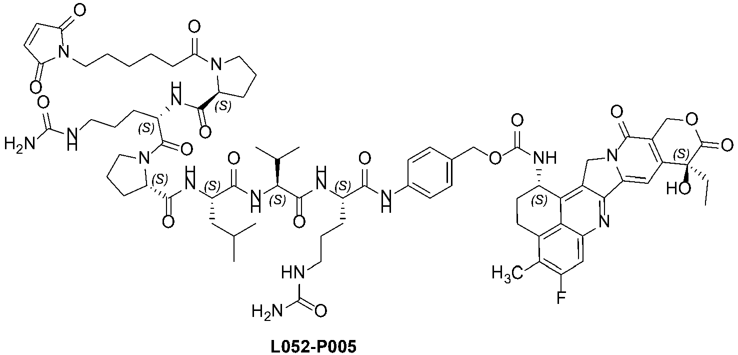

### 1.4.46.1 Synthesis of 4-(S)-2-(S)-2-(S)-2,2-(S)-2(S)-1-((S)-2-((S)-1-((S)-1-(6-(2,5-dioxo-2,5-dihydro-1H-pyrrol-1-yl)hexanoyl)pyrrolidin-2-carboxamido)-5-ureidopentanoyl)pyrrolidinyl-2-carboxamide)-4-methylpentanamido)-3-methylbutanamido)-5-ureidopentanamidobenzyl((1S,95)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate(L052-P005)

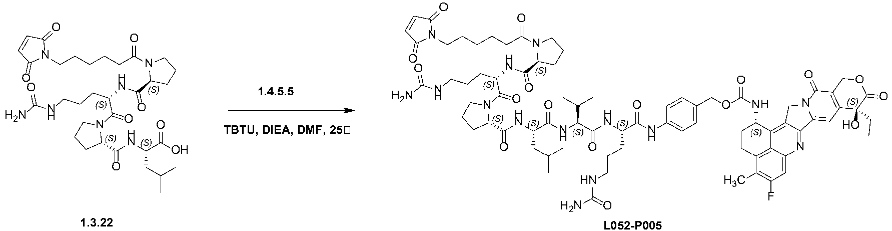

DIEA (11.5 mg, 88.8 µmol, 14.7 µL, 3.0 eq.) was added to a solution of compound 1.3.22 (20.0 mg, 29.6 µmol, 1.0 eq.), compound 1.4.5.5 (27.4 mg, 32.6 µmol, 1.1 eq.), and TBTU (11.4 mg, 35.5 µmol, 1.2 eq.) in DMF (0.50 mL). The mixture was stirred at 25°C for 1.0 h. LC-MS monitored completion of the reaction. The mixture was purified by preparative HPLC to obtain a yellow solid compound L052-P005 (24.0 mg, 15.8 µmol, yield: 53.4%). ESI-MS (m/z):750.3[M/2+H]⁺.

### 1.4.47 Synthesis of L053-P005

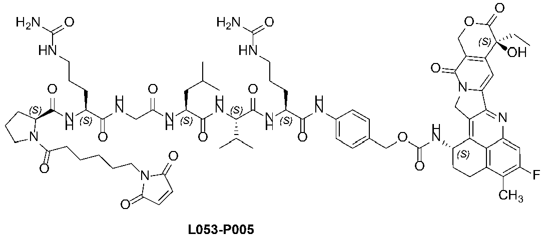

### 1.4.47.1 Synthesis of 4-((2S,5S,8S,14S)-14-((S)-1-(6-(2,5-dioxo-2,5-dihydro-1H-pyrrol-1-yl)hexanoyl)pyrrolidin-2-carboxamido)-8-isobutyl-5-isopropyl-4,7,10,13,19-pentaoxo-2-(3-ureidopropyl)-3,6,9,12,18-pentaazaeicosanamino)benzyl((1S,9S)-9-ethyl-5-fluoro-9-hydroxy-4-methyl-10,13-dioxo-2,3,9,10,13,15-hexahydro-1H,12H-benzopyrano[3',4':6,7]indolizino[1,2-b]quinolin-1-yl)carbamate (L053-P005)

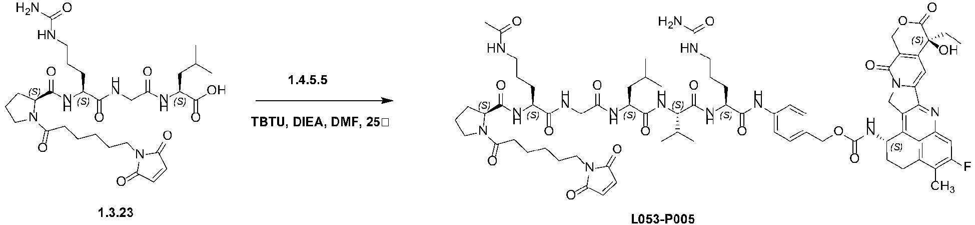

DIEA (12.2 mg, 94.4 µmol, 15.6 µL, 3.0 eq.) was added to a solution of compound 1.3.23 (20.0 mg, 31.5 µmol, 1.0 eq.), compound 1.4.5.5 (29.1 mg, 34.6 µmol, 1.1 eq.), and TBTU (12.1 mg, 37.8 µmol, 1.2 eq.) in DMF (0.50 mL). The mixture was stirred at 25°C for 1.0 h. LC-MS monitored completion of the reaction. The mixture was purified by preparative HPLC to obtain a yellow solid compound L053-P005 (18.0 mg, 11.9 µmol, yield: 37.9%). ESI-MS (m/z):1458.8[M+H]⁺.

### 1.4.48 Synthesis of L054-P005

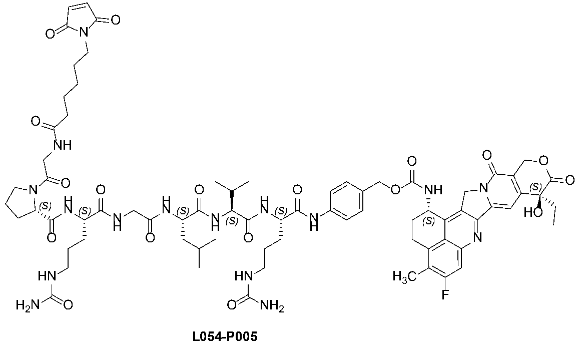

### 1.4.48.1 Synthesis of 4-((6S,12S,15S,18S)-1-amino-6-((S)-1-(6-(2,5-dioxo-2,5-dihydro-1H-pyrrol-1-yl)hexanoyl)glyceryl)pyrrolidin-2-carboxamido)-12-isobutyl-15-isopropyl-1,7,10,13,16-pentaoxo-18-(3-ureidopropyl)-2,8,11,14,17-pentaazanonadecan-19-amido)benzy1((1S,9S)-9-ethyl-5-fluoro-9-hydroxy-4-methyl-10,13-dioxo-2,3,9,10,13,15-hexahydro-1H,12H-benzopyrano[3',4':6,7]indolizino[1,2-b]quinolin-1-yl)carbamate (L054-P005)

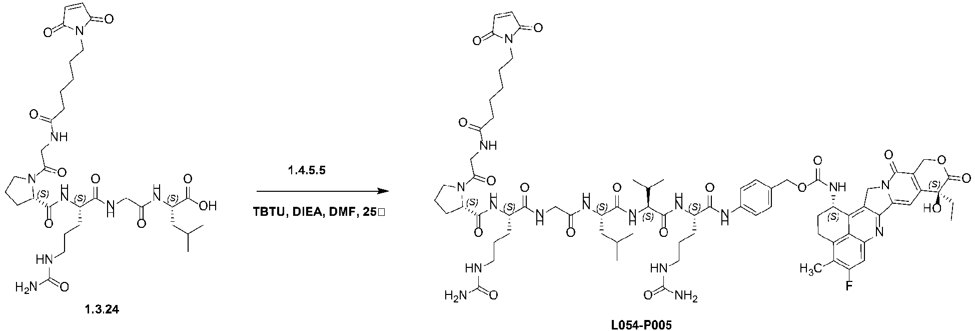

DIEA (11.2 mg, 86.6 µmol, 14.3 µL, 3.0 eq.) was added to a DMF (0.50 mL) solution of compound 1.3.24 (20.0 mg, 28.9 µmol, 1.0 eq.), compound 1.4.5.5 (26.7 mg, 31.8 µmol, 1.1 eq.), and TBTU (11.1 mg, 34.6 µmol, 1.2 eq.). The mixture was stirred at 25°C for 1.0 h. LC-MS monitored completion of the reaction. The mixture was purified by preparative HPLC to obtain a yellow solid compound L054-P005 (29.0 mg, 18.4 µmol, yield: 63.9%). ESI-MS (m/z):1515.9[M+H]⁺.

### 1.4.49 Synthesis of L055-P005

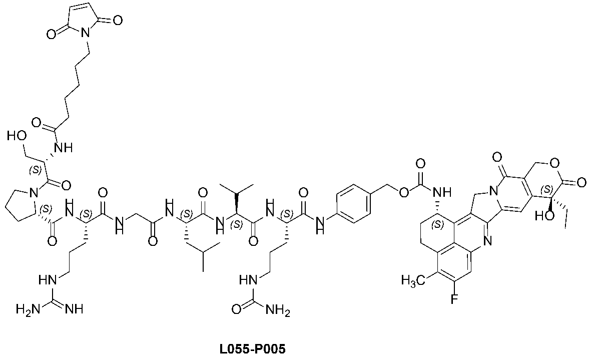

### 1.4.49.1 Synthesis of 4-((6S,12S,15S,18S)-1-amino-6-((S)-1-(6-(2,5-dioxo-2,5-dihydro-1H-pyrrol-1-yl)hexanoyl)-L-seryl)pyrrolidin-2-carboxamido)-1-imino-12-isobutyl-15-isopropyl-7,10,13,16-tetraoxo-18-(3-ureidopropyl)-2,8,11,14,17-pentaazanonadecan-19-amido)benzyl((1S,9S)-9-ethyl-5-fluoro-9-hydroxy-4-methyl-10,13-dioxo-2,3,9,10,13,13,15-hexahydro-1H,12H-benzopyrano[3',4':6,7]indolizino[1,2-b]quinolin-1-yl)carbamate (L055-P005)

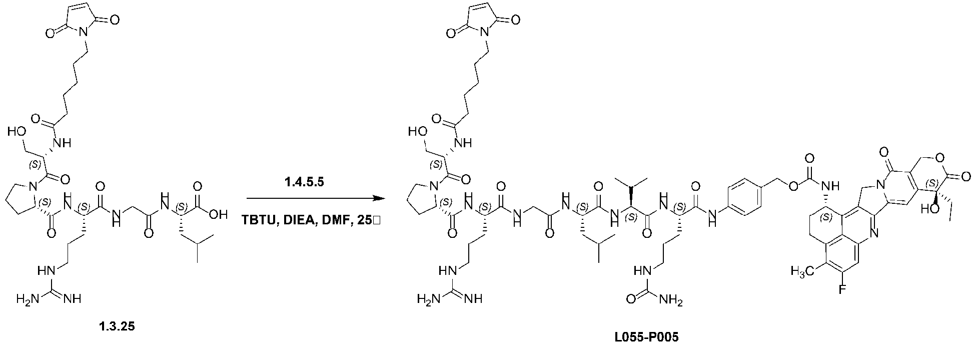

DIEA (10.7 mg, 83.1 µmol, 13.7 µL, 3.0 eq.) was added to a DMF (0.50 mL) solution of compound 1.3.25 (20.0 mg, 27.7 µmol, 1.0 eq.), compound 1.4.5.5 (25.6 mg, 30.5 µmol, 1.1 eq.), and TBTU (10.7 mg, 33.3 µmol, 1.2 eq.). The mixture was stirred at 25°C for 1.0 h. LC-MS monitored completion of the reaction. The mixture was purified by preparative HPLC to obtain a yellow solid compound L055-P005 (15.0 mg, 9.69 µmol, yield: 34.9%). ESI-MS (m/z):1544.9[M+H]⁺.

### 1.4.50 Synthesis of L056-P005

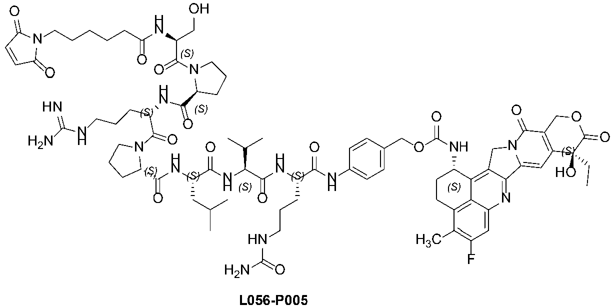

### 1.4.50.1 Synthesis of 4-(S)-2-((S)-2-(S)-2-((S)-1-(6-(2,5-dioxo-2,5-dihydro-1H-pyrrol-1-yl)hexanoyl)-L-seryl-L-prolyl-L-arginyl)pyrrolidin-2-carboxamido)-4-methylpentanamido)-3-methylbutanamido)-5-ureidopentanamidobenzyl((1S,9S)-9-ethyl-5-fluoro-9-hydroxy-4-methyl-10,13-dioxo-2,3,9,10,13,15-hexahydro-1H,12H-benzopyrano[3',4':6,7]indolizino[1,2-b]quinolin-1-yl)carbamate (L056-P005)

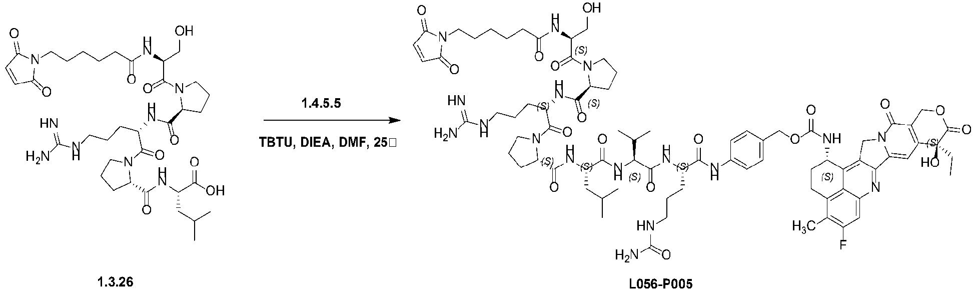

DIEA (10.2 mg, 78.8 µmol, 13.0 µL, 3.0 eq.) was added to a DMF (0.50 mL) solution of compound 1.3.26 (20.0 mg, 26.3 µmol, 1.0 eq.), compound 1.4.5.5 (24.3 mg, 28.9 µmol, 1.1 eq.), and TBTU (10.1 mg, 31.5 µmol, 1.2 eq.). The mixture was stirred at 25°C for 1.0 h. LC-MS monitored completion of the reaction. The mixture was purified by preparative HPLC to obtain a yellow solid compound L056-P005 (17.0 mg, 10.5 µmol, yield: 40.0%). ESI-MS (m/z):1584.9[M+H]⁺.

### 1.4.51 Synthesis of L057-P005

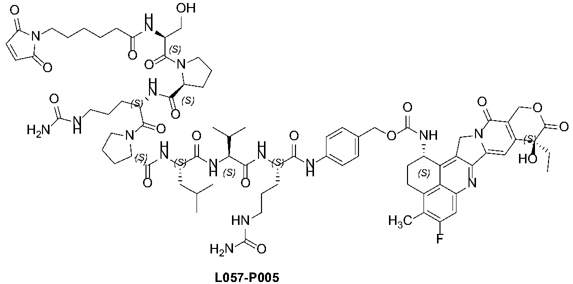

### 1.4.51.1 Synthesis of 4-((S)-2-(S)-2-(S)-2,2-(S)-1-(S)-2(S)-1,1-(S)-1-(6-(2,5-dioxo-2,5-dihydro -1H-pyrrol-1-yl)hexanoyl)-L-seryl)pyrrolidin-2-carboxamido)-5-ureidopentanoyl)pyrrolidin-2-carb oxamido(4-methylpentanamido)-3-methylbutanamido)-5-ureidopentanamido(1S,9S)-9-ethyl-5-fluor o-9-hydroxy-4-methyl-10,13-dioxo-2,3,9,10,13,15-hexahydro-1H,12H-benzo[de]pyrano[3',4':6,7]in dolizino[1,2-b]quinolin-1-yl)carbamate (L057-P005)

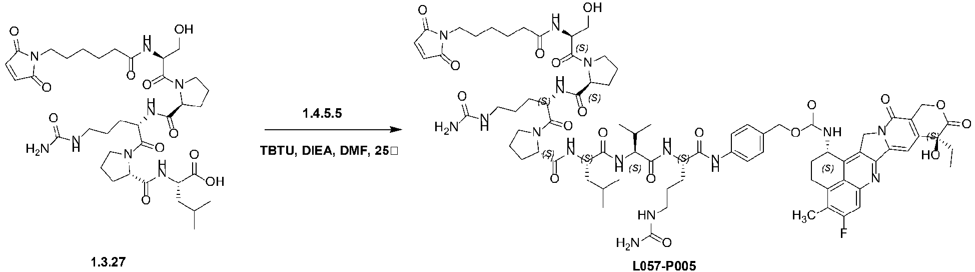

DIEA (10.2 mg, 78.7 µmol, 13.0 µL, 3.0 eq.) was added to a DMF (0.5 mL) solution of compound 1.3.27 (20.0 mg, 26.2 µmol, 1.0 eq.), compound 1.4.5.5 (24.3 mg, 28.8 µmol, 1.1 eq.), and TBTU (10.1 mg, 31.5 µmol, 1.2 eq.). The mixture was stirred at 25°C for 1.0 h. LC-MS monitored completion of the reaction. The mixture was purified by preparative HPLC twice to obtain a yellow solid compound L057-P005 (5.30 mg, 3.34 µmol, yield: 12.7%). ESI-MS (m/z):793.8[M/2+H]⁺.

### 1.4.52 Synthesis of L058-P005

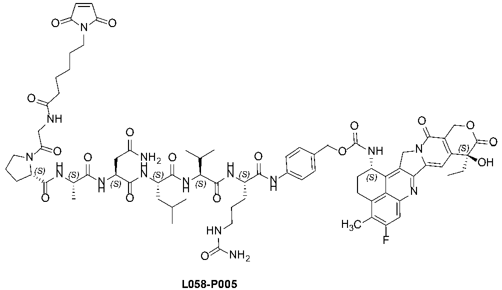

### 1.4.52.1 Synthesis of 4-((3S,6S,9S,12S,15S)-6-(2-amino-2-oxyethyl)-1-((S)-1-(6-(2,5-dioxo-2,5-dihydro-1H-pyrrol-1-yl)hexanoyl)glyceryl)pyrrolidin-2-yl)-9-isobutyl-12-isopropyl-3-methyl-1,4,7,10,13-pentaoxo-15-(3-ureidopropyl)-2,5,8,11,14-pentaazahexadecan-16-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L058-P005)

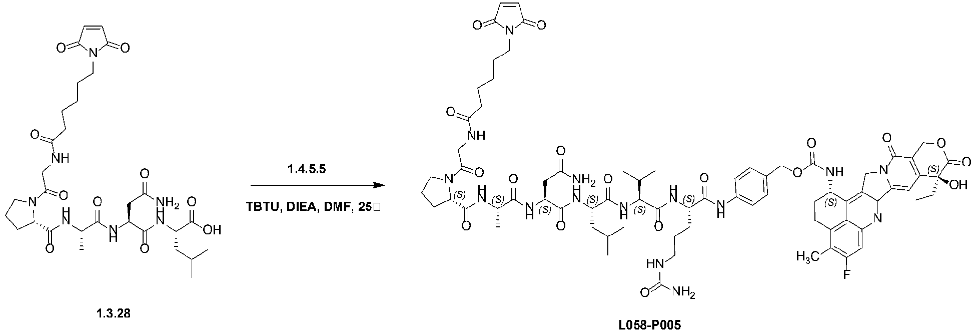

DIEA (11.7 mg, 90.4 µmol, 14.9 µL, 3.0 eq.) was added to a DMF (0.50 mL) solution of compound 1.3.28 (20.0 mg, 30.1 µmol, 1.0 eq.), compound 1.4.5.5 (27.8 mg, 33.3 µmol, 1.1 eq.), and TBTU (11.6 mg, 36.2 µmol, 1.2 eq.). The mixture was stirred at 25°C for 1.0 h. LC-MS monitored completion of the reaction. The mixture was purified by preparative HPLC to obtain a yellow solid L058-P005 (22.0 mg, 14.5 µmol, yield: 48.0%). ESI-MS (m/z):1486.8[M+H]⁺.

### 1.4.53 Synthesis of L059-P005

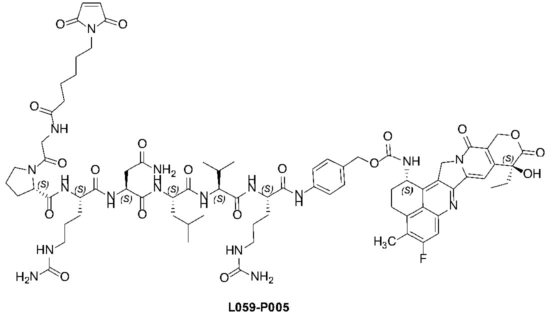

### 1.4.53.1 Synthesis of 4-((6S,9S,12S,15S,18S)-1-amino-9-(2-amino-2-oxyethyl)-6-((S)-1-(6-(2, 5-dioxo-2,5-dihydro-1H-pyrrol-1-yl)hexanoyl)glyceryl)pyrrolidin-2-carboxamido)-12-isobutyl-15-i sopropyl-1,7,10,13,16-pentaoxo-18-(3-ureidopropyl)-2,8,11,14,17-pentaazanonadecan-19-amido)be nzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L059-P005)

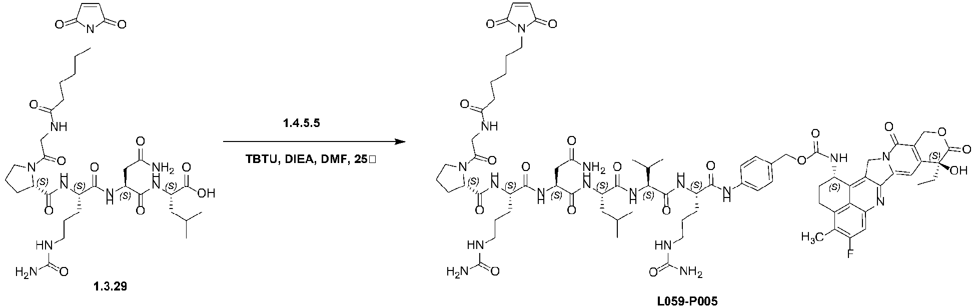

TBTU (31.4 mg, 97.8 µmol, 1.2 eq.) and DIEA (42.1 mg, 326 µmol, 54.0 µL, 4.0 eq.) were added to a DMF (1.00 mL) solution of compound 1.3.29 (55.0 mg, 73.4 µmol, 0.9 eq.) and compound 1.4.5.5 (68.5 mg, 81.5 µmol, 1.0 eq.). The mixture was stirred at 25°C for 1.0 h. LC-MS monitored completion of the reaction. The mixture was purified by preparative HPLC to obtain a yellow solid L059-P005 (62.0 mg, 38.2 µmol, yield: 52.0%). ESI-MS (m/z):1572.8[M+H]⁺.

### 1.4.54 Synthesis of L060-P005

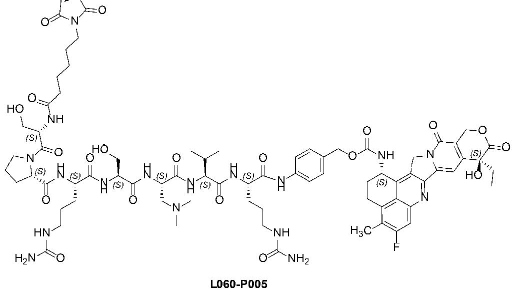

### 1.4.54.1 Synthesis of (9H-fluoren-9-yl)methyl((4S,7S,10S)-15-amino-10-((4-(18S,9S)-9-ethyl-5 -fluoro-9-hydroxy-4-methyl-10,13-dioxo-2,3,9,10,13,15-hexahydro-1H,12H-benzopyrano[3',4':6,7] indolizino[1,2-b]quinolin-1-yl)carbamoyl)oxy)methyl)phenyl)carbamoyl)-7-isopropyl-2-methyl-5,8, 15-trioxo-2,6,9,14-tetraazapentadecan-4-yl)carbamate (1.4.54.1)

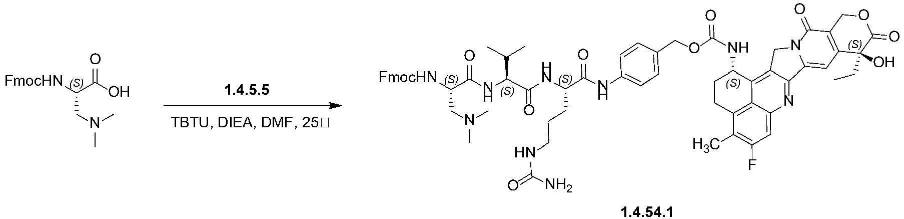

DIEA (87.5 mg, 677 µmol, 112 µL, 3.0 eq.) was added to a DMF (2.50 mL) solution of compound FMOC-AZA-L-LEUCINE (80.0 mg, 226 µmol, 1.0 eq.), compound 1.4.5.5 (190 mg, 226 µmol, 1.0 eq.), and TBTU (87.0 mg, 271 µmol, 1.2 eq.). The mixture was stirred at 25°C for 1.0 h. LC-MS monitored completion of the reaction. The mixture was dropped into cold isopropyl ether (25 mL) and centrifuged to obtain a brown oily compound 1.4.54.1 (285 mg, crude product). ESI-MS (m/z):1177.6[M+H]⁺.

### 1.4.54.2 Synthesis of 4-(S)-2-((S)-2-(S)-3-(dimethylamino)propanamido)-3-methylbutanamido)-5-ureidopentanamido)benzyl((1S,9S)-9-ethyl-5-fluoro-9-hydroxy-4-methyl-10,13-dioxo-2,3,9,10,13,15-hexahydro-1H,12H-benzopyrano[3',4':6,7]indolizino[1,2-b]quinolin-1-yl)carbamate (1.4.54.2)

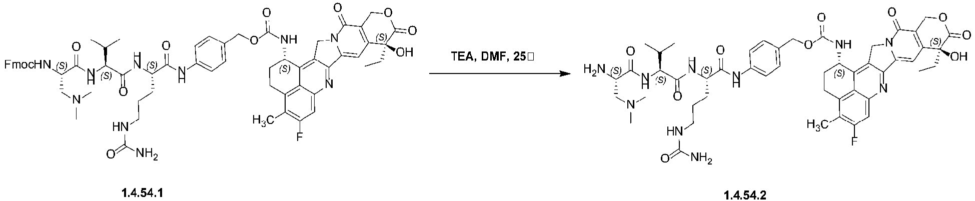

TEA (313 mg, 3.09 mmol, 0.43 mL, 12.8 eq.) was added to a DMF (1.70 mL) solution of compound 1.4.54.1 (285 mg, 242 µmol, 1.0 eq.). The mixture was stirred at 25°C for 16 h. LC-MS monitored completion of the reaction. The mixture was dropped into cold isopropyl ether (25.0 mL) and centrifuged to obtain a yellow solid compound 1.4.54.2 (95.0 mg, 99.5 µmol, yield: 41.1%). ESI-MS (m/z):955.5[M+H]⁺.

### 1.4.54.3 Synthesis of 4-((6S,9S,12S,15S,18S)-1-amino-12-(dimethylamino)methyl)-6-((S)-1-(6 -(2,5-dioxo-2,5-dihydro-1H-pyrrol-1-yl)hexanoyl)-L-seryl)pyrrolidin-2-carboxamido)-9-(hydroxym ethyl)-15-isopropyl-1,7,10,13,16-pentaoxo-18-(3-ureidopropyl)-2,8,11,14,17-pentaazanonadecan-1 9-amido)benzyl((1S,9S)-9-ethyl-5-fluoro-9-hydroxy-4-methyl-10,13-dioxo-2,3,9,10,13,15-hexahyd ro-1H,12H-benzo[de]pyrano[3',4':6,7]indolizino[1,2-b]quinolin-1-yl)carbamate (L060-P005)

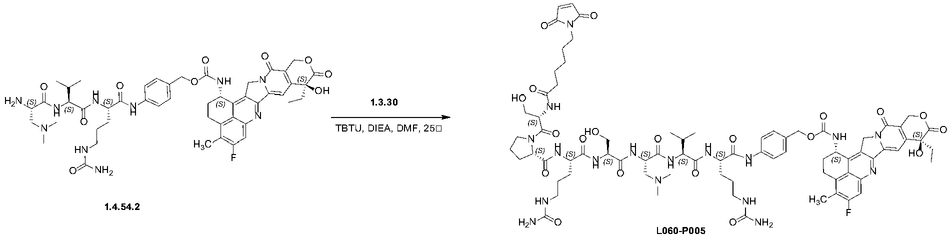

DIEA (6.09 mg, 47.1 µmol, 7.79 µL, 3.0 eq.) was added to a DMF (0.25 mL) solution of compound 1.4.54.2 (15.0 mg, 15.7 µmol, 1.0 eq.), compound 1.3.30 (10.0 mg, 15.70 µmol, 1 eq.), and TBTU (6.05 mg, 18.8 µmol, 1.2 eq.). The mixture was stirred at 25°C for 1.0 h. LC-MS monitored completion of the reaction. The mixture was purified by preparative HPLC to obtain a yellow solid compound L060-P005 (8.00 mg, 5.07 µmol, yield: 32.3%). ESI-MS (m/z):1577.1[M+H]⁺.

### 1.4.55 Synthesis of L060-P002

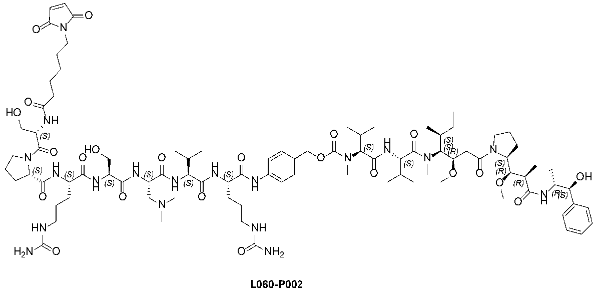

### 1.4.55.1 Synthesis of 4-((5S,8S,11S)-5-((dimethylamino)methyl)-1-(9H-fluoren-9-yl)-8-isopropyl-3,6,9-trioxo-11-(3-ureidopropyl)-2-oxo-4,7,10-triazadodecan-12-amido)benzyl((S)-1-((3R,4S,5S)-1-(1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)3-methyl-1-oxobut-2-yl(methyl)carbamate (1.4.55.1)

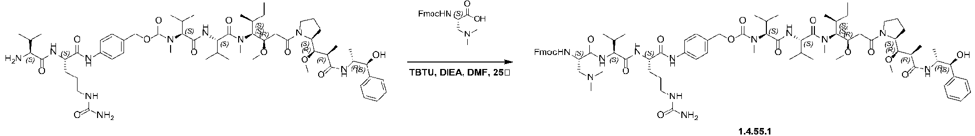

DIEA (87.5 mg, 677 µmol, 112 µL, 3.0 eq.) was added to a DMF (3.0 mL) solution of compound Val-Cit-PAB-MMAE (254 mg, 226 µmol, 1.0 eq.), compound FMOC-AZA-L-LEUCINE (80.0 mg, 226 µmol, 1.0 eq.), and TBTU (87.0 mg, 271 µmol, 1.2 eq.). The mixture was stirred at 25°C for 1.0 h. LC-MS monitored completion of the reaction. The mixture was dropped into isopropyl ether (30.0 mL) and centrifuged to obtain a brown oily compound 1.4.55.1 (361 mg, crude product). ESI-MS (m/z):1459.9[M+H]⁺.

### 1.4.55.2 Synthesis of 4-(S)-2-(S)-2-(S)-3-(methylbutanamido)-5-ureidopentanamido)benzyl(S)-1-((S)-1-((3R,4S,5S)-1-(S)-2-(1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)-3-methyl-1-oxobut-2-yl)(methyl)carbamate (1.4.55.2)

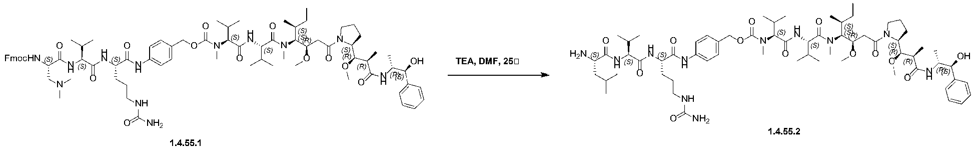

TEA (750 mg, 7.42 mmol, 1.03 mL, 30 eq.) was added to a solution of compound 1.4.55.1 (361 mg, 248 µmol, 1.0 eq.) in DMF (4.10 mL). The mixture was stirred at 25°C for 6.0 h. LC-MS monitored completion of the reaction. The mixture was purified by preparative HPLC to obtain a white solid compound 1.4.55.2 (143 mg, 116 µmol, yield: 48.2%). ESI-MS (m/z):1237.8[M+H]⁺.

### 1.4.55.3 Synthesis of 4-((6S,9S,12S,15S,18S)-1-amino-12-(dimethylamino)methyl)-6-((S)-1-((6-(2,5-dioxo-2,5-dihydro-1H-pyrrol-1-yl)hexanoyl)-L-seryl)pyrrolidin-2-carboxamido)-9-(hydroxymethyl)-15-isopropyl-1,7,10,13,16-pentaoxo-18-(3-ureidopropyl)-2,8,11,14,17-pentaazanonadecan-19-amido)benzyl((S)-2-((1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobut-2-yl)amino)-3-methyl-1-oxobutan-2-yl)(methyl)carbamate (L060-P002)

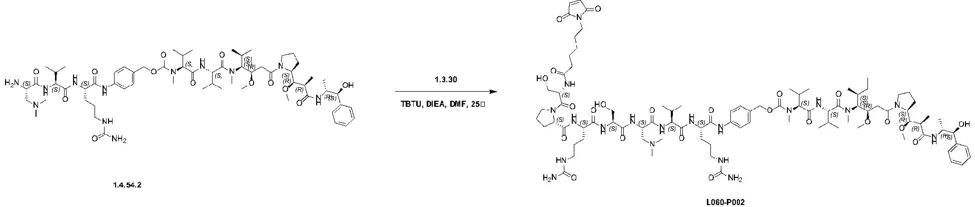

DIEA (6.23 mg, 48.5 µmol, 8.01 µL, 3.0 eq.) was added to a solution of compound 1.4.55.2 (20.0 mg, 16.2 µmol, 1.0 eq.), compound 1.3.30 (11.0 mg, 1620 µmol, 1.0 eq.) and TBTU (6.23 mg, 19.4 µmol, 1.2 eq.) in DMF (0.30 mL). The mixture was stirred at 25°C for 1.0 h. LC-MS monitored completion of the reaction. The mixture was purified by preparative HPLC to obtain a yellow solid compound L060-P002 (12.0 mg, 6.45 µmol, yield: 39.9%). ESI-MS (m/z): 930.5[M/2+H]⁺.

### 1.4.56 Synthesis of L062-P005

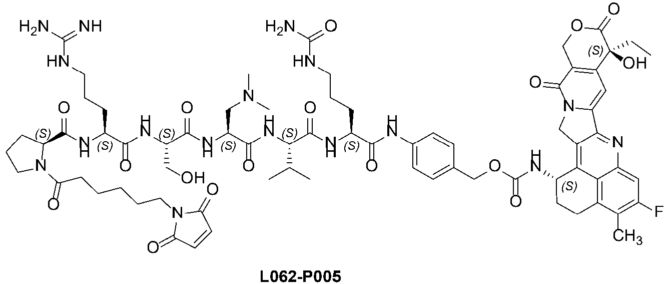

### 1.4.56.1 Synthesis of 4-((6S,9S,12S,15S,18S)-1-amino-12-(dimethylamino)methyl)-6-((S)-1-(6 -(2,5-dioxo-2,5-dihydro-1H-pyrrol-1-yl)hexanoyl)pyrrolidin-2-carboxamido)-9-(hydroxymethyl)-1-imino-15-isopropyl-7,10,13,16-tetraoxo-18-(3-ureidopropyl)-2,8,11,14,17-pentaazanonadecan-19-a mido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L062-P005)

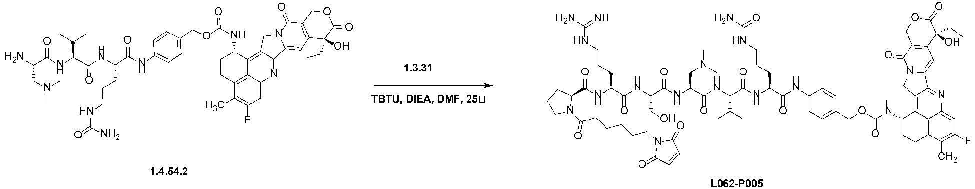

DIEA (13.5 mg, 105 µmol, 17.3 µL, 4.0 eq.) was added to a solution of compound 1.4.54.2 (25.0 mg, 26.2 µmol, 1.0 eq.), compound 1.3.31 (15.0 mg, 26.2 µmol, 1.0 eq.) and TBTU (10.1 mg, 31.4 µmol, 1.2 eq.) in DMF (0.40 mL). The mixture was stirred at 25°C for 1.0 h. LC-MS monitored completion of the reaction. The mixture was purified by preparative HPLC to obtain a yellow solid compound L062-P005 (10.0mg, 6.72 µmol, yield: 25.7%). ESI-MS (m/z):1488.8[M+H]⁺.

### 1.4.57 Synthesis of L062-P002

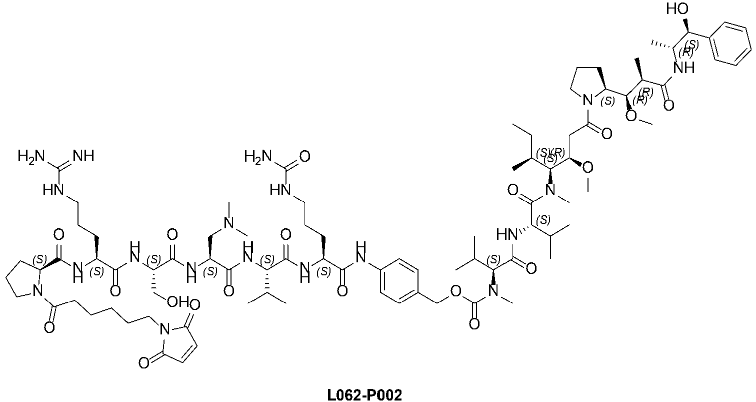

### 1.4.57.1 Synthesis of 4-((6S,9S,12S,15S,18S)-1-amino-12-(((dimethylamino)methyl)-6-((S)-1-(6-(2,5-dioxo-2,5-dihydro-1H-pyrrol-1-yl)hexanoyl)pyrrolidin-2-carboxamido)-9-(hydroxymethyl)-1-imino-15-isopropyl-7,10,13,16-tetraoxo-18-(3-ureidopropyl)-2,8,11,14,17-pentaazanonadecan-19-amido)benzyl((((S)-1-((3R,4S,5S)-1-((S)-2-((1R,2R)-3-((1S,2R)-1-hydroxy-1-phenylprop-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (L062-P002)

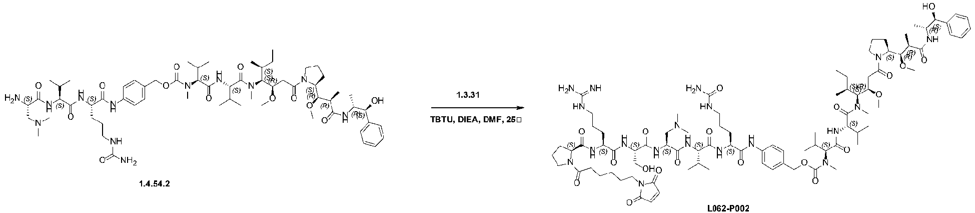

DIEA (15.6 mg, 121 µmol, 20.0µL, 5.0 eq.) was added to a solution of compound 1.4.54.2 (30.0 mg, 24.2 µmol, 1.0 eq.), compound 1.3.31 (28.0 mg, 48.2 µmol, 2.0 eq.), and TBTU (9.34 mg, 29.1 µmol, 1.2 eq.) in DMF (0.40 mL). The mixture was stirred at 25°C for 2.0 h. LC-MS monitored completion of the reaction. The mixture was purified by preparative HPLC to obtain a yellow solid L062-P002 (8.00 mg, 4.52 µmol, yield: 18.6%). ESI-MS (m/z):1771.3[M+H]⁺.

### 1.4.58 Synthesis of L064-P005

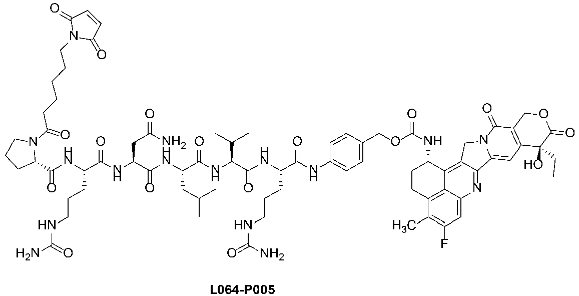

### 1.4.58.1 Synthesis of 4-((6S,9S,12S,15S,18S)-1-amino-9-(2-amino-2-oxyethyl)-6-((S)-1-(6-(2,5-dioxo-2,5-dihydro-1H-pyrrol-1-yl)hexanoyl)pyrrolidin-2-carboxamido)-12-isobutyl-15-isopropyl-1,7,10,13,16-pentaoxo-18-(3-ureidopropyl)-2,8,11,14,17-pentaazanonadecan-19-amido)benzyl((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (L064-P005)

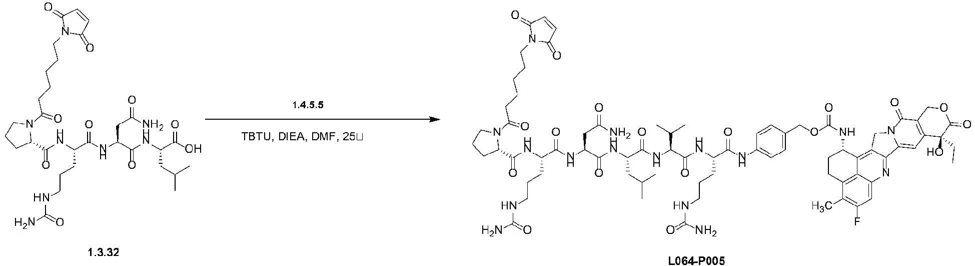

TBTU (44.4 mg, 138 µmol, 1.2 eq.) and DIPEA (59.7 mg, 461 µmol, 76.3 µL, 4.0 eq.) were added to a DMF (80.0 µL) solution of compound 1.3.32 (80.0 mg, 115 µmol, 1.0 eq.) and compound 1.4.5.5 (116 mg, 138 µmol, 1.2 eq.). The resulting mixture was stirred at 25°C for 1.0 h. LC-MS monitored completion of the reaction. The reaction mixture was purified by preparative HPLC under TFA conditions to obtain 106 mg of the desired product with a purity of 88.9%. The mixture was purified by preparative HPLC to obtain a yellow solid L064-P005 (8.10 mg, yield: 4.43%). SI-MS (m/z):1516.0[M+H]⁺.

### 1.4.59 Synthesis of L065-P005

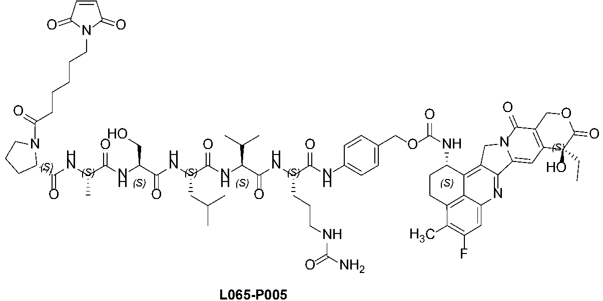

### 1.4.58.1 Synthesis of 4-((3S,6S,9S,9S,12S,15S,15S)-1-((S)-1-(6-(2,5-dioxo-2,5-dihydro-2,5-di hydroxy-2,5-dihydro-1-dihydro-1-dihydro-1H-pyrrolidin-2-yl-pyrrolidin-2-yl-pyrrolidin-6-(hydrox ymethyl)-6-(hydroxymethyl)-9-isobutyl-12-isopropyl-12-isopropyl ester-3-methyl-1,4,7,10,10,13-p entazolazo-15-(3-ureidoethylpropoxy-2,5,8,8,8,11,11,11,4-pentazolethyl-5-fluoro-9-hydroxy-4-met hyl-10,13-dioxo-2,3,9,10,13,15-hexahydro-1H,12H-benzo[de]pyrano[3',4':6,7]indolizino[1,2-b]quin olin-1-yl)carbamate (L065-P005)

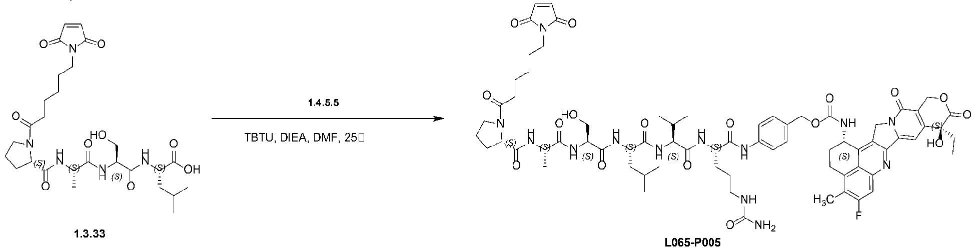

TBTU (9.97 mg, 31.0 µmol, 1.2 eq.) and DIPEA (10.0 mg, 77.6 µmol, 12.8 µL, 3.0 eq.) were added to a DMF (300 µL) solution of compound 1.3.33 (15.0 mg, 25.8 µmol, 1.0 eq.) and compound 1.4.5.5 (21.7 mg, 1 eq.). The mixture was stirred at 25°C for 2.0 h. LC-MS monitored completion of the reaction. The reactant was filtered, and the filtrate was purified by preparative HPLC to obtain a yellow solid L065-P005 (8.00 mg, yield: 22.3%). ESI-MS (m/z):1402.5[M+H]⁺.

### Example 2

### 2.1 Humanization and expression of the anti-TF antibody

Referring to the amino acid sequence information of the heavy and light chains of the murine anti-TF antibody 1849 disclosed in Patent US9920133B2, a homology model of the antibody was obtained by modeling. The framework amino acids within 5Å of the CDRs were analyzed, and these amino acid sites typically affect the conformation of the CDRs or antigen binding activity. Humanized Germline was obtained by IMGT analysis. The selected humanized Germline framework was spliced with CDRs of the antibody, and the framework region sequence of the designed humanized antibody was aligned with that of the original antibody. Through analyzing the homology modeling results of the parental antibody, and on the basis of maintaining antibody activity and reducing heterogenicity, amino acids similar to human antibody surface residues were selected for substitution, while CDR sequences of the original antibody were retained. Back mutation was performed in the framework region after humanization to obtain the humanized antibody sequence. Five heavy chains with high humanization scores and five light chains with high humanization scores were selected for gene synthesis. The heavy chains were subcloned into a pcDNA3.4-IgG1 expression vector, and the light chains were subcloned into a pcDNA3.4-IgKc expression vector. After sequence verification of the vectors, endotoxin-free plasmids were prepared. The plasmids were then co-transfected into 293F cells for expression. The cells were cultured in a 37°C, 5% CO₂ incubator for 5-7 consecutive days, and centrifuged to collect culture supernatant, and then the culture supernatant was filtered through a 0.45 µm filter membrane. Supernatant was collected and the antibody was purified using a Protein A affinity column. The light chain sequence of the 1849-H3L3 antibody finally obtained was set forth in SEQ ID NO. 58, and the heavy chain sequence was set forth in SEQ ID NO. 59. The light chain sequence of the h1849-H2L1 antibody was set forth in SEQ ID NO. 56, and the heavy chain sequence was set forth in SEQ ID NO. 57.

Meanwhile, the chimeric antibody 1849-Chimeric (VH1VLI1) was prepared according to the information disclosed in Patent US9920133B2.

### 2.2Affinity measurement of the anti-TF antibody

The anti-TF antibody was tested by ELISA assay with the following specific experimental procedures: the antigen protein TF was diluted by sterile CBS to a final concentration of 1 µg/mL to prepare an antigen diluent. A new 96-well plate was taken, and added with the antigen diluent at 100 µL/well and coated overnight at 4°C. The antigen coating solution was removed, and the plate was washed three times with PBST (containing 0.5% Tween). A blocking buffer (3% BSA) was added at 200 µL/well and blocked at 37°C for 2 h. After removing the blocking buffer, the plate was washed three times with PBST. 100 µL, 10 µL, and 1 µL of humanized antibody expression supernatant or purified humanized antibodies (initial concentration: 1 µg/mL, 5-fold serial dilution for 7 concentration points) were added and incubated at room temperature for 1 h. PBS was added to the control wells. The liquid in the wells was removed, and the plate was washed three times with PBST. 100 µL of HRP-Protein A (diluted at 1:10000) was added and incubated at room temperature for 1 h. After removing the liquid within the well, the plate was washed three times with PBST. TMB developer was added at 100 µL/well and incubated at room temperature in the dark for 10 min. The stop buffer was added at 50 µL/well, and the OD value in each well was read using a microplate reader. The EC50 value was calculated based on the raw data.

The results are shown in Table 1 below. After humanization, the affinity of the humanized anti-TF antibody was improved, as compared to the chimeric antibody 1849-Chimeric (VH1VL1).

**Table 1 Test results of the anti-TF antibody affinity**

| **Antibody No.** | **EC50 (µg/mL)** |
|---|---|
| h1849-H2L1 | 0.01553 |
| h1849-H3L3 | 0.01909 |
| 1849-Chimeric (VH1VL1) | 0.04817 |

### 2.3 Cellular affinity detection of the anti-TF antibody

The binding activity of the humanized TF antibody to MDA-MB-231 cells was detected by a FACS method with the following specific experimental procedure: MDA-MB-231 cells were thawn and adjusted to the logarithmic growth phase. The cells were divided into several aliquots, 3 × 10⁵ cells per aliquot. 100 µL of humanized antibody (initial concentration: 10 µg/mL, 10-fold serial dilution for 7 concentration points) was added thereto, respectively, mixed thoroughly, and incubated at room temperature for 1 h. The cells were centrifuged at 800 × g under room temperature for 5 min, the supernatant containing the antibody was removed, and the cells were washed three times with PBS. 100 µL of Alexa Fluor^{®} 488 anti-human IgG (diluted at 1:1000) was added thereto, mixed thoroughly, and incubated at room temperature in the dark for 45 min. The cells were centrifuged at 800 × g under room temperature for 5 min, the supernatant containing the secondary antibody was removed, and the cells were washed three times with PBS. The cells were resuspended in 200 µL PBS and analyzed by flow cytometry. The EC50 value was calculated based on the fluorescence value.

The results are shown in Table 2. Compared with the chimeric antibody, the affinity of the humanized anti-TF antibody is not only retained, but also improved.

**Table 2 Test results of anti-TF antibody cellular affinity**

| **Antibody No.** | **EC50 (µg/mL)** |
|---|---|
| h1849-H2L1 | 1.13 |
| h1849-H3L3 | 2.297 |
| 1849-Chimeric (VH1VL1) | 8.44 |

### 2.4 Dynamic affinity detection of the anti-TF antibody

Fortibio is a commonly used instrument for dynamic affinity detection, which is used to detect the dynamic affinity between the anti-TF antibody and the TF protein. The method is briefly described as follows: 1849-Chimeric and humanized antibodies (h1849-H2L1, h1849-H3L3) were serially diluted with PBST. A ProA biosensor was pre-wetted with PBST buffer before use. The anti-TF antibody was diluted with PBST and then immobilized on the ProA sensor. The sensor immobilized with the antibody was equilibrated in PBST buffer for 60 s to obtain a baseline, then transferred to PBST for association for 60 s, and then dissociated in PBST for 180 s. After one analysis cycle, the sensor was regenerated with 10 mM Gly Phl.5. Data analysis was performed using a 1:1 model to determine an association rate constant Kₒₙ and a dissociation rate constant K_{off}, then the constants were used to calculate the equilibrium dissociation constant KD.

As shown in Table 3, the association rate constant of the humanized anti-TF antibody had no significant change, but the dissociation rate constants decreased to some extent, especially for h1849-H3L3, it showed almost no dissociation after binding.

**Table 3 Test results of dynamic affinity of the anti-TF antibody**

| **Antibody No.** | **kₒₙ (1/Ms)** | **k_{off} (1/s)** | **KD (M)** |
|---|---|---|---|
| h1849-H2L1 | 3.92E+05 | 1.69E-04 | 4.30E-10 |
| h1849-H3L3 | 3.24E+05 | <1.0E-7 | ND |
| 1849-Chimeric (VH1VL1) | 2.33E+05 | 2.86E-04 | 1.23E-09 |

### 2.5 Detection of endocytosis of the humanized antibody

The strength of endocytosis against the anti-TF antibody by MDA-MB-231 cells was detected by FACS with the following specific experimental procedure: target cells MDA-MB-231 were prepared, with 4 × 10⁵ cells per well. 100 µL of 10 µg/mL purified antibody was added thereto and incubated at 4°C for 30 min. It was centrifuged at 4°C, 800 × g for 5 min, the supernatant containing the antibody was removed, and the cells were washed three times with pre-cooled PBS. The cells were divided equally into two groups and incubated at 4°C and 37°C for 2 h and 4 h, respectively. After incubation, ice-bath PBS was immediately added to terminate endocytosis, and it was centrifuged at 4°C, 800 × g for 5 min and resuspended in 100 µL pre-cooled PBS. Alexa Fluor^{®} 488 anti-human IgG was immediately added thereto and incubated at 4°C for 30 min. The cells were centrifuged at 4°C, 800 × g for 5 min, the supernatant containing the antibody was removed, and the cells were washed three times with pre-cooled PBS. The cells were resuspended in 200 µL pre-cooled PBS and immediately analyzed by flow cytometry. Calculation of internalization level of cell surface-bound antibodies: MFI% at time point of tx = MFI of the sample incubated at 37°C/MFI of the control sample incubated at 4°C. Internalization percentage at time point of tx = 100% - MFI% at time point of tx.

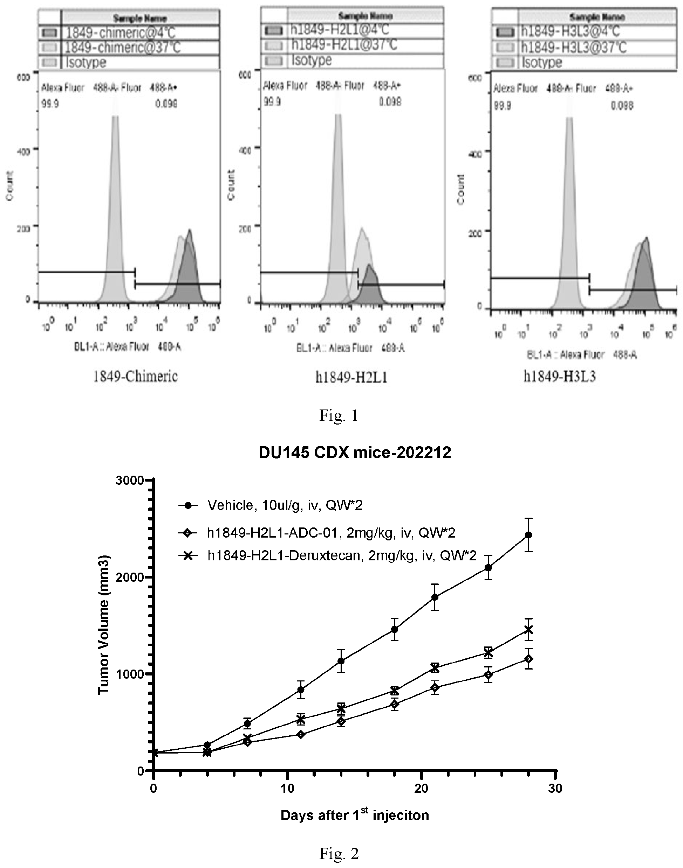

The results are shown in FIG. 1. Similar to the chimeric antibody 1849-Chimeric (VH1VL1), h1849-H2L1 and h1849-H3L3 may be effectively endocytosed by cells.

### 2.6 Effect of the anti-TF antibody on coagulation function

The effect of a candidate anti-TF antibody on coagulation function was measured using the SYSMEX CS2100i detection platform. The anti-TF antibody (Tisotumab and h1849-H3L3) was diluted with a histidine-acetate buffer to 4, 2, 0.4, 0.04, and 0.004 mg/mL, and added to plasma to achieve final concentrations of 100 µg/mL, 50 µg/mL, 25 µg/mL, 10 µg/mL, 5 µg/mL, 1 µg/mL, 0.5 µg/mL, and 0.1 µg/mL, respectively. After incubation on the instrument for 190 s, PT and TT clotting times were measured using Thromborel^{®} S Reagent and Dade^{®} Thrombin Reagent, respectively. The mean value was automatically calculated by the instrument.

According to the results of Table 4, in the present application, the plasma prothrombin time (PT) of samples added with the antibody Tisotumab was prolonged, indicating that the antibody Tisotumab affected the extrinsic coagulation pathway. In contrast, the plasma PT of samples added with the antibody h1849-H3L3 showed almost no change, indicating that the antibody h1849-H3L3 had little effect on the extrinsic coagulation pathway. The thrombin time (TT) of all antibody-added samples remained unchanged, indicating that all antibodies had no effect on the common coagulation pathway.

**Table 4 Test results of anti-TF antibody coagulation function**

| **Test item** | | | **PT** | | | **TT** | | |
|---|---|---|---|---|---|---|---|---|
| Sample | Final sample concentration | **No.** | Test 1 | Test 2 | Mean | Test 1 | Test 2 | Mean |
| Tisotumab | 100 µg/ml | **1** | 17.4 | 17.8 | 17.6 | 19.4 | 19.4 | 19.4 |
| | 50 µg/ml | **2** | 16.2 | 16.5 | 16.4 | 19.0 | 19.8 | 19.4 |
| | 25 µg/ml | **3** | 15.2 | 15.5 | 15.4 | 19.3 | 19.2 | 19.3 |
| | 10 µg/ml | **4** | 15.0 | 15.1 | 15.1 | 19.3 | 19.1 | 19.2 |
| | 5 µg/ml | **5** | 14.7 | 14.8 | 14.8 | 18.9 | 19.3 | 19.1 |
| | 1 µg/ml | **6** | 14.5 | 14.6 | 14.6 | 18.9 | 19.4 | 19.2 |
| | 0.5 µg/ml | **7** | 14.5 | 14.4 | 14.5 | 19.3 | 19.4 | 19.4 |
| | 0.1 µg/ml | **8** | 14.4 | 14.5 | 14.5 | 19.2 | 19.2 | 19.2 |
| h1849-H3L3 | 100 µg/ml | **9** | 14.4 | 14.5 | 14.5 | 19.5 | 19.7 | 19.6 |
| | 50 µg/ml | **10** | 14.9 | 15.1 | 15.0 | 19.3 | 19.1 | 19.2 |
| | 25 µg/ml | **11** | 14.7 | 14.8 | 14.8 | 19.3 | 19.4 | 19.4 |
| | 10 µg/ml | **12** | 14.3 | 14.7 | 14.5 | 19.3 | 19.6 | 19.5 |
| | 5 µg/ml | **13** | 14.3 | 14.3 | 14.3 | 19.0 | 19.3 | 19.2 |
| | 1 µg/ml | **14** | 14.2 | 14.5 | 14.4 | 19.2 | 19.1 | 19.2 |
| | 0.5 µg/ml | **15** | 14.3 | 14.4 | 14.4 | 19.1 | 19.4 | 19.3 |
| | 0.1 µg/ml | **16** | 14.3 | 14.3 | 14.3 | 18.6 | 19.3 | 19.0 |
| Buffer | | **17** | 14.2 | 14.3 | 14.3 | 19.7 | 19.6 | 19.7 |
| Untreated plasma | | **18** | 14.0 | 14.1 | 14.1 | 19.3 | 19.7 | 19.5 |

### Example 3 Preparation and physicochemical property analysis of ADC

### 3.1 Methods of physicochemical property analysis of ADC

### 3.1.1 HIC-HPLC method for determining DAR

The DAR value of the conjugated sample was determined by HIC-HPLC:

| **HPLC parameters** | | | | |
|---|---|---|---|---|
| Instrument | Agilent 1260-series high performance liquid chromatography | | | |
| Chromatographic column | TSKgel Butyl-NPR 2.5 µm 4.6 mm×3.5 cm | | | |
| Column temperature | 25°C | | | |
| Mobile phase | A: 1.5M (NH4)2SO4, 50 mM KPi, pH7.0 | | | |
| | B: 50 mM KPi, 25% IPA, pH7.0 | | | |
| Flow rate | 0.5 mL/min | | | |
| Sample box temperature | 25°C | | | |
| Injection volume | 10 µl, >30 µg | | | |
| Detection wavelength | 280 nm, 248 nm, 214 nm | | | |

| | Time (min) | A% | B% | Flow (mL/min) |
|---|---|---|---|---|
| Mobile phase gradient | 0 | 100 | 0 | 0.6 |
| | 2 | 100 | 0 | 0.6 |
| | 15 | 0 | 100 | 0.6 |
| | 16 | 0 | 100 | 0.6 |
| | 17 | 100 | 0 | 0.6 |
| | 20 | 100 | 0 | 0.6 |

| | | | | |
|---|---|---|---|---|
| Sample treatment: the sample was diluted to 2.5 mg/mL with ultrapure water, and injected at 10 µL. | | | | |

The average drug-to-antibody ratio was calculated by the HIC-HPLC formula: $HIC DAR = 2 \times \frac{A \left(DAR2\right)}{A \left(total\right)} + 4 \times \frac{A \left(DAR4\right)}{A \left(total\right)} + 6 \times \frac{A \left(DAR6\right)}{A \left(total\right)} + 8 \times \frac{A \left(DAR8\right)}{A \left(total\right)}$ wherein A(total) represents a total peak area, and A(DAR2), A(DAR4), A(DAR6), and A(DAR8) represent the peak areas generated by antibody-drug conjugates with a linking number of 2, 4, 6, and 8, respectively.

### 3.1.2 PLRP-HPLC method for determining DAR

The DAR value of the conjugated sample was determined by PLRP-HPLC:

| **HPLC parameters** | | | | |
|---|---|---|---|---|
| Instrument | Agilent 1260-series high performance liquid chromatography | | | |
| Chromatographic column | PLRP-S 1000Å 8 µm 2.1 mm×15 cm | | | |
| Column temperature | 75°C | | | |
| Mobile phase | A: Pure water containing 0.05% trifluoroacetic acid | | | |
| | B: Acetonitrile containing 0.05% trifluoroacetic acid | | | |
| Flow rate | 0.8 mL/min | | | |
| Sample box temperature | 10°C | | | |
| Injection volume | 20µl | | | |
| Detection wavelength | 280 nm, 248 nm, 214 nm | | | |

| | Time (min) | A% | B% | Flow (mL/min) |
|---|---|---|---|---|
| Mobile phase gradient | 0 | 75 | 25 | 0.8 |
| | 3 | 75 | 25 | 0.8 |
| | 28 | 50 | 50 | 0.8 |
| | 30 | 5 | 95 | 0.8 |
| | 32 | 5 | 95 | 0.8 |
| | 33 | 75 | 25 | 0.8 |
| | 40 | 75 | 25 | 0.8 |

| | | | | |
|---|---|---|---|---|
| Sample treatment: 20 µL (100 µg) of antibody-drug conjugate was taken, and added with 5 µL of 1 M Tris-HCl (pH 8.0) solution and 75 µL of 6 M guanidine hydrochloride solution, mixed well, then added with 2 µL of 0.5 M TCP solution, and incubated at 37°C for 15 min such that the antibody-drug conjugate is reduced thoroughly. The resulting solution was directly used for PLRP-HPLC test. | | | | |

The average drug-to-antibody ratio was calculated by PLRP-HPLC formula: $PR DAR = 2 \times \left[\frac{A \left({LC}_{1}\right)}{A \left({LC}_{total}\right)}+\frac{A \left({HC}_{1}\right)}{A \left({HC}_{total}\right)}+2\times \frac{A \left({HC}_{2}\right)}{A \left({HC}_{total}\right)}+3\times \frac{A \left({HC}_{3}\right)}{A \left({HC}_{total}\right)}\right]$

After being reduced, the ADC was analyzed by PLRP-HPLC, and theoretically, the following components may appear: the antibody light chain (LC₀), the antibody light chain conjugated with 1 linker-payload (LC₁), and the antibody heavy chain (HC₀), the antibody heavy chain conjugated with 1 linker-payload (HC₁), the antibody heavy chain conjugated with 2 linker-payloads (HC₂), and the antibody heavy chain conjugated with 3 linker-payloads (HC₃). In the formula, A represents the peak area, LCₜₒₜₐₗ represents all the light chains, and HCₜₒₜₐₗ represents all the heavy chains.

### 3.1.3 Mass spectrometry method for determining DAR

The DAR value of the conjugated sample was determined by mass spectrometry:

| **LC-MS parameters** | | | | |
|---|---|---|---|---|
| Instrument | Agilent 1260-6230 LC/TOF | | | |
| Chromatographic column | PLRP-S 1000Å 8 µm 2.1 mm×50 mm | | | |
| Column temperature | 75°C | | | |
| Mobile phase | A: Pure water containing 0.05%FA | | | |
| | B: Acetonitrile containing 0.05%FA | | | |
| Flow rate | 0.5 mL/min | | | |
| Sample box temperature | 10°C | | | |
| Injection volume | 4 µl, 2 µg | | | |
| Detection wavelength | 280 nm | | | |
| Gas temp | 350°C | | | |
| Drying Gas | 13 1/min | | | |
| Nebulizer | 45 psi | | | |
| Sheath Gas Temp | 350°C | | | |
| Sheath Gas Flow | 11 1/min | | | |
| m/z | 300~3200 | | | |

| | Time (min) | A% | B% | Flow (mL/min) |
|---|---|---|---|---|
| Mobile phase gradient | 0 | 75 | 25 | 0.8 |
| | 1 | 75 | 25 | 0.8 |
| | 9 | 55 | 45 | 0.8 |
| | 10 | 10 | 90 | 0.8 |
| | 11 | 10 | 90 | 0.8 |
| | 11.1 | 75 | 25 | 0.8 |
| | 15 | 75 | 25 | 0.8 |

| | | | | |
|---|---|---|---|---|
| Sample treatment: 50 µg of sample was added with 2 µL of 0.5 M TCEP, and added with ultrapure water to 100 µL to be diluted to a concentration of about 0.5 mg/mL, then mixed well, and reduced at 37°C for 30 min. | | | | |

The average drug-to-antibody ratio was calculated by PLRP-MS formula: $MS DAR = 2 \times \left[\frac{A \left({LC}_{1}\right)}{A \left({LC}_{total}\right)}+\frac{A \left({HC}_{1}\right)}{A \left({HC}_{total}\right)}+2\times \frac{A \left({HC}_{2}\right)}{A \left({HC}_{total}\right)}+3\times \frac{A \left({HC}_{3}\right)}{A \left({HC}_{total}\right)}\right]$

After being reduced, the ADC was analyzed by LC-MS, and theoretically, the following components may appear: the antibody light chain (LC₀), the antibody light chain conjugated with 1 linker-payload (LC₁), and the antibody heavy chain (HC₀), the antibody heavy chain conjugated with 1 linker-payload (HC₁), the antibody heavy chain conjugated with 2 linker-payloads (HC₂), and the antibody heavy chain conjugated with 3 linker-payloads (HC₃). In the formula, A represents the peak area, LCₜₒₜₐₗ represents all the light chains, and HCₜₒₜₐₗ represents all the heavy chains.

### 3.2 Preparation of ADC

### 3.2.1 Preparation of h1849-H3L3-ADC

### 3.2.1.1 Preparation of h1849-H3L3-ADC-01 (DAR8)

The antibody h1849-H3L3 (mAb) was placed in a 20 mM histidine-acetate buffer (pH 5.5), and a 5 mM tris(2-carboxyethyl)phosphine (TCEP) stock solution was added thereto at a TCEP/mAb ratio of 6. The mixed solution was placed at 37°C for reaction for 2 h. After 2 h, N,N-dimethylacetamide (DMA) and a solution of 15 mg/mL compound L005-P005 dissolved in DMA was added to the mixed solution. The final DMA content was 10%, and the ratio of drug DX8951 to mAb was 14. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate h1849-H3L3-ADC-01 of L005-P005 and the antibody h1849-H3L3 was obtained, and the DAR value determined by the PLRP method was 8.03.

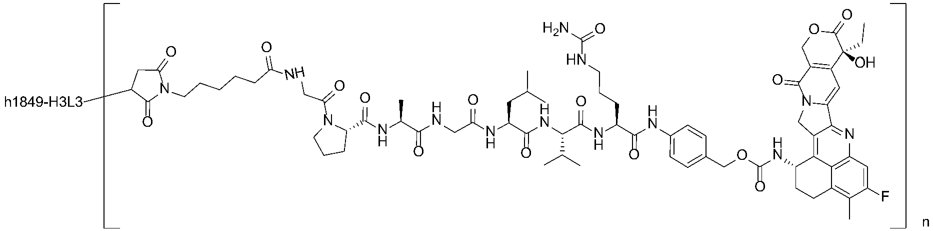

### 3.2.1.2 Preparation of h1849-H3L3-ADC-02 (DAR8)

Compound L005-P005 in 3.2.1.1 was replaced by compound L006-P005 to obtain a conjugate h1849-H3L3-ADC-02 of L006-P005 and the antibody h1849-H3L3. The DAR value determined by the PLRP method was 6.76.

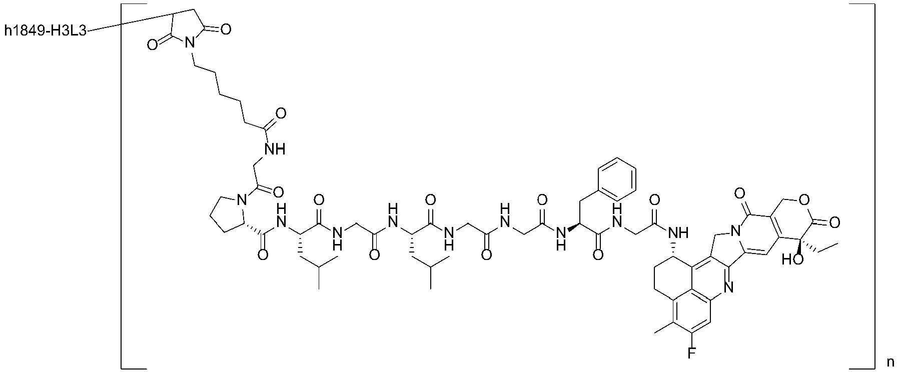

### 3.2.1.3 Preparation of h1849-H3L3-ADC-03 (DAR8)

Compound L005-P005 in 3.2.1.1 was replaced by compound L009-P005 to obtain a conjugate h1849-H3L3-ADC-03 of L009-P005 and the antibody h1849-H3L3. The DAR value determined by the PLRP method was 8.00.

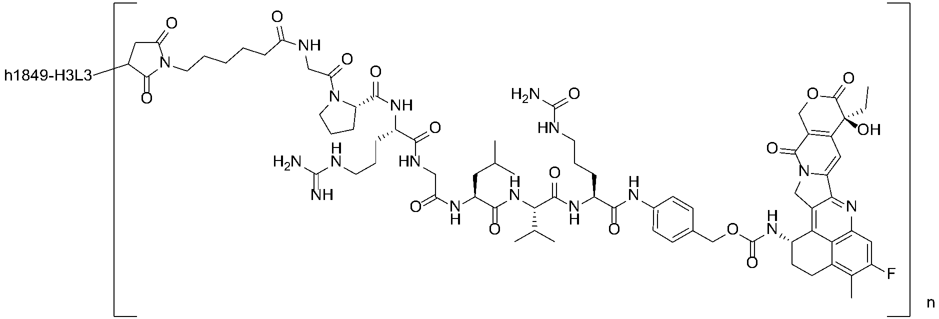

### 3.2.1.4 Preparation of h1849-H3L3-ADC-04 (DAR8)

Compound L005-P005 in 3.2.1.1 was replaced by compound L013-P005 to obtain a conjugate h1849-H3L3-ADC-04 of L013-P005 and the antibody h1849-H3L3. The DAR value determined by the PLRP method was 7.30.

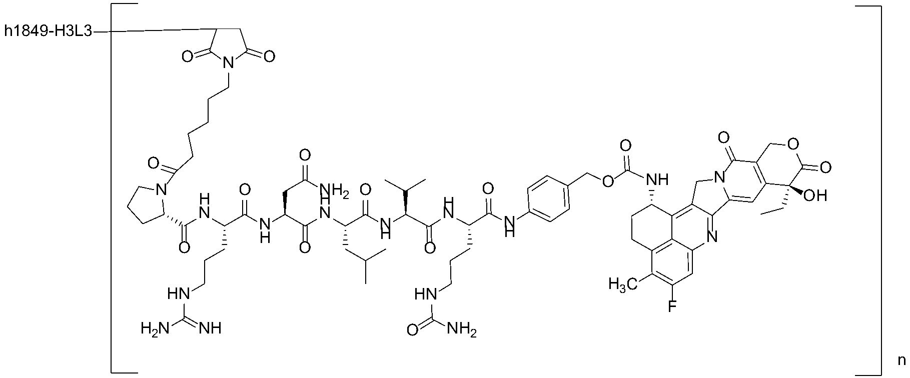

### 3.2.1.5 Preparation of h1849-H3L3-ADC-05 (DAR8)

Compound L005-P005 in 3.2.1.1 was replaced by compound L015-P005 to obtain a conjugate h1849-H3L3-ADC-05 of L015-P005 and the antibody h1849-H3L3. The DAR value determined by the PLRP method was 7.54.

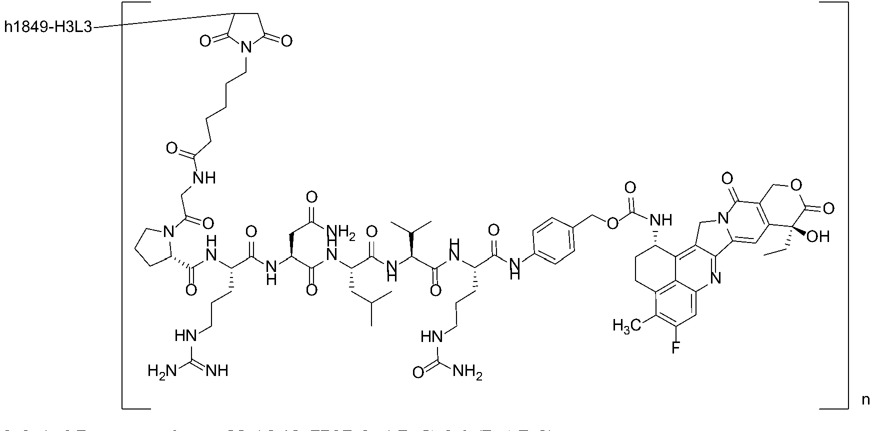

### 3.2.1.6 Preparation of h1849-H3L3-ADC-06 (DAR8)

Compound L005-P005 in 3.2.1.1 was replaced by compound L016-P005 to obtain a conjugate h1849-H3L3-ADC-06 of L016-P005 and the antibody h1849-H3L3. The DAR value determined by the PLRP method was 7.32.

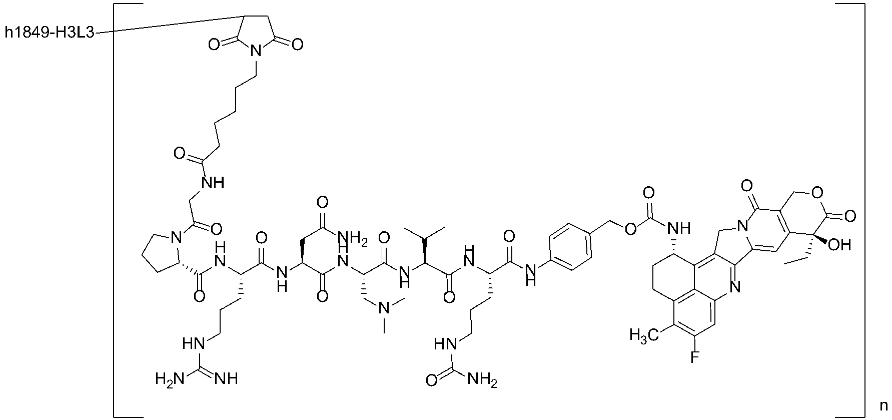

### 3.2.1.7 Preparation of h1849-H3L3-ADC-07 (DAR8)

Compound L005-P005 in 3.2.1.1 was replaced by compound L017-P005 to obtain a conjugate h1849-H3L3-ADC-07 of L017-P005 and the antibody h1849-H3L3. The DAR value determined by the PLRP method was 8.08.

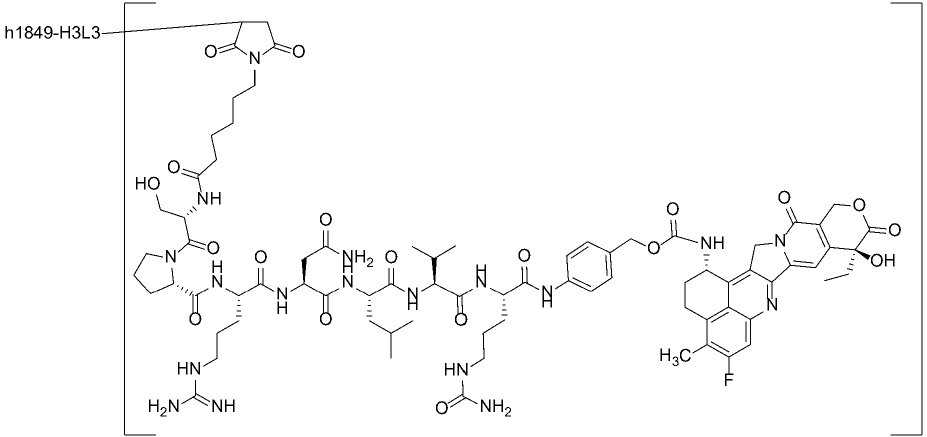

### 3.2.1.8 Preparation of h1849-H3L3-ADC-08 (DAR8)

Compound L005-P005 in 3.2.1.1 was replaced by compound L027-P005 to obtain a conjugate h1849-H3L3-ADC-08 of L027-P005 and the antibody h1849-H3L3. The DAR value determined by the PLRP method was 7.96.

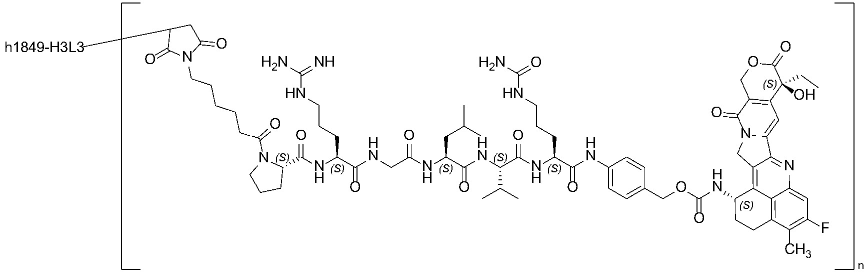

### 3.2.1.9 Preparation of h1849-H3L3-ADC-09 (DAR8)

Compound L005-P005 in 3.2.1.1 was replaced by compound L028-P005 to obtain a conjugate h1849-H3L3-ADC-09 of L028-P005 and the antibody h1849-H3L3. The DAR value determined by the PLRP method was 8.05.

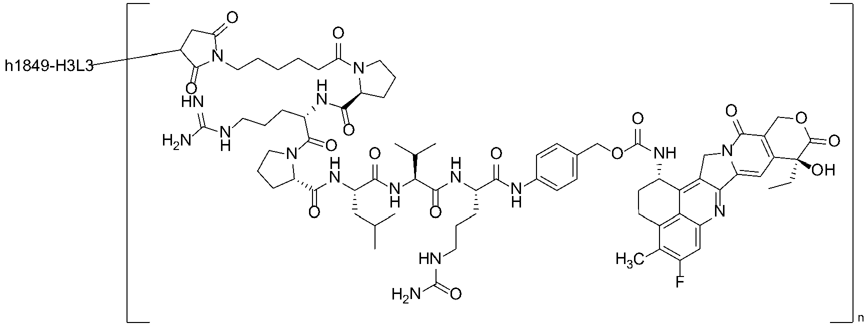

### 3.2.1.10 Preparation of h1849-H3L3-ADC-10 (DAR8)

Compound L005-P005 in 3.2.1.1 was replaced by compound L029-P005 to obtain a conjugate h1849-H3L3-ADC-10 of L029-P005 and the antibody h1849-H3L3. The DAR value determined by the PLRP method was 8.08.

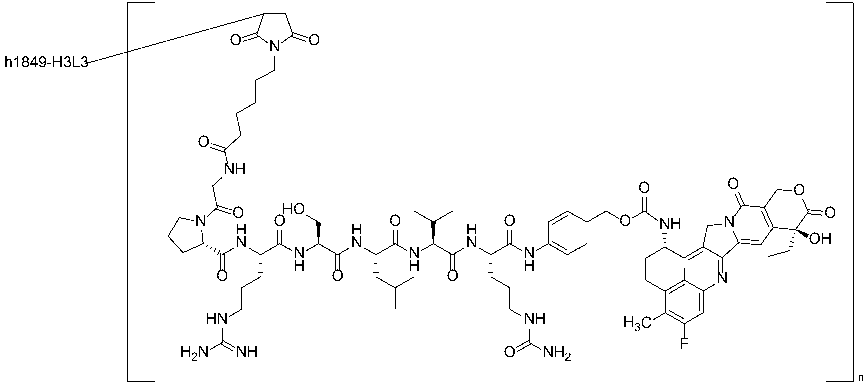

### 3.2.1.11 Preparation of h1849-H3L3-ADC-11 (DAR8)

Compound L005-P005 in 3.2.1.1 was replaced by compound L030-P005 to obtain a conjugate h1849-H3L3-ADC-11 of L030-P005 and the antibody h1849-H3L3. The DAR value determined by the PLRP method was 8.08.

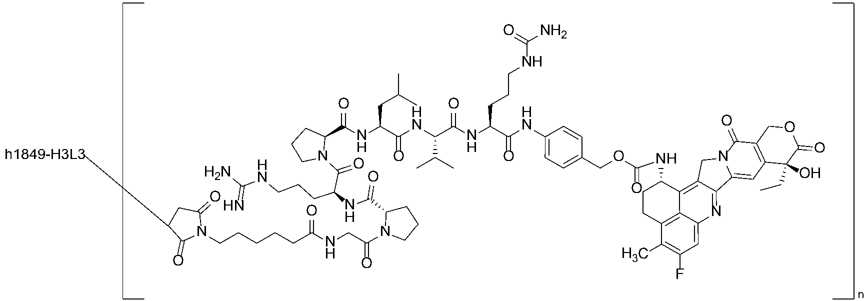

### 3.2.1.12 Preparation of h1849-H3L3-ADC-12 (DAR8)

Compound L005-P005 in 3.2.1.1 was replaced by compound L031-P005 to obtain a conjugate h1849-H3L3-ADC-12 of L031-P005 and the antibody h1849-H3L3. The DAR value determined by the PLRP method was 7.61.

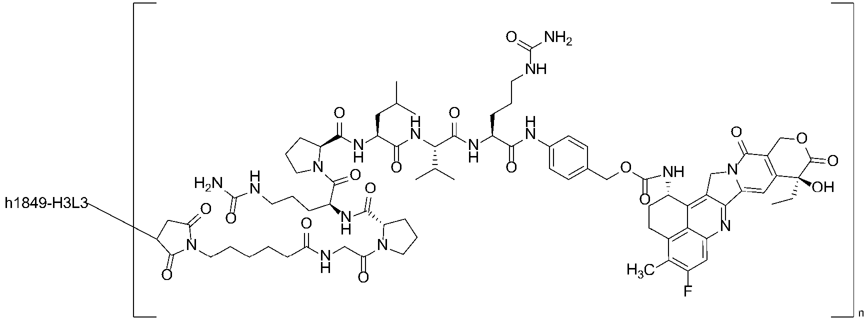

### 3.2.1.13 Preparation of h1849-H3L3-ADC13 (DAR8)

Compound L005-P005 in 3.2.1.1 was replaced by compound L032-P005 to obtain a conjugate h1849-H3L3-ADC-13 of L032-P005 and the antibody h1849-H3L3. The DAR value determined by the PLRP method was 7.53.

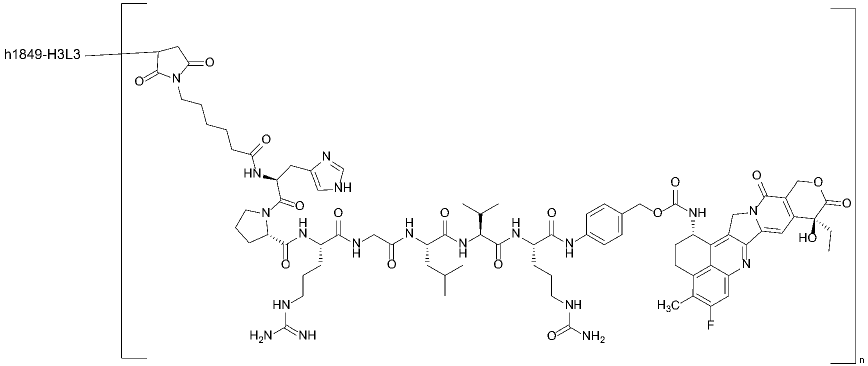

### 3.2.1.14 Preparation of h1849-H3L3-ADC-14 (DAR8)

Compound L005-P005 in 3.2.1.1 was replaced by compound L037-P005 to obtain a conjugate h1849-H3L3-ADC-14 of L037-P005 and the antibody h1849-H3L3. The DAR value determined by the PLRP method was 7.55.

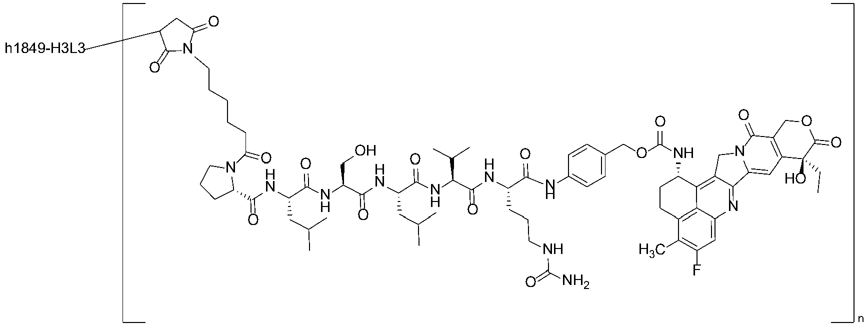

### 3.2.1.15 Preparation of h1849-H3L3-ADC-15 (DAR8)

Compound L005-P005 in 3.2.1.1 was replaced by compound L038-P005 to obtain a conjugate h1849-H3L3-ADC-15 of L038-P005 and the antibody h1849-H3L3. The DAR value determined by the PLRP method was 8.04.

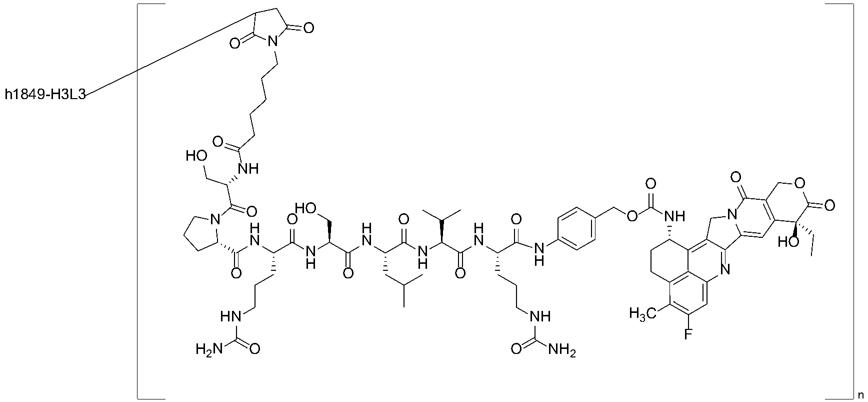

### 3.2.1.16 Preparation of h1849-H3L3-ADC-16 (DAR8)

Compound L005-P005 in 3.2.1.1 was replaced by compound L049-P005 to obtain a conjugate h1849-H3L3-ADC-16 of L049-P005 and the antibody h1849-H3L3. The DAR value determined by the PLRP method was 8.25.

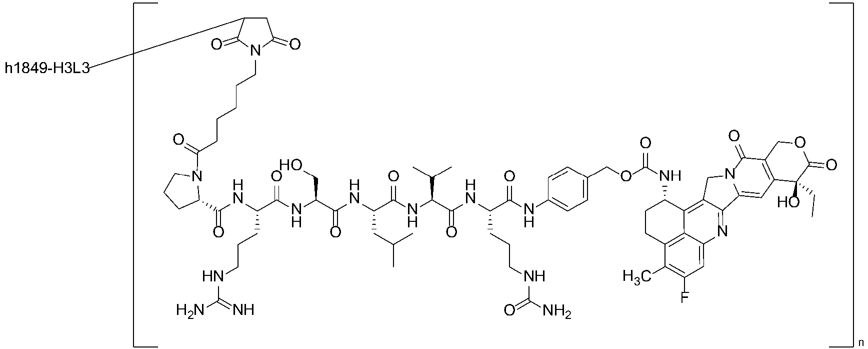

### 3.2.1.17 h1849-H3L3-ADC-17 (DAR8)

Compound L005-P005 in 3.2.1.1 was replaced by compound L050-P005 to obtain a conjugate h1849-H3L3-ADC-17 of L050-P005 and the antibody h1849-H3L3. The DAR value determined by the PLRP method was 7.98.

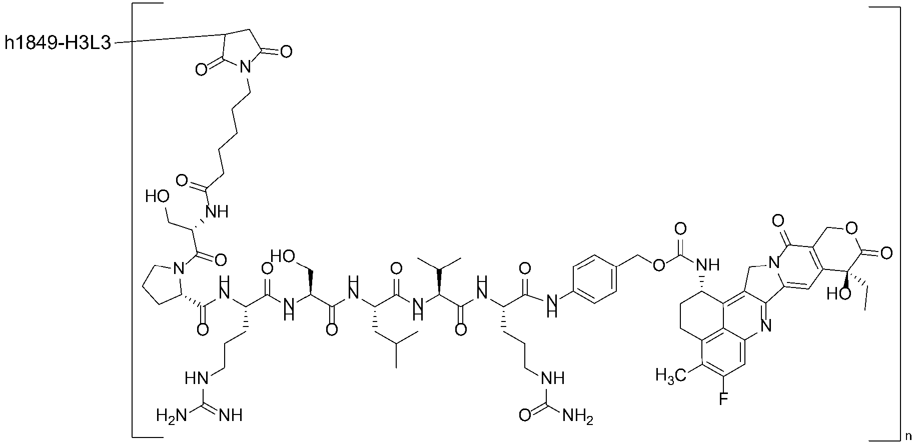

### 3.2.1.18 h1849-H3L3-ADC-18 (DAR8)

Compound L005-P005 in 3.2.1.1 was replaced by compound L051-P005 to obtain a conjugate h1849-H3L3-ADC-18 of L051-P005 and the antibody h1849-H3L3. The DAR value determined by the PLRP method was 7.64.

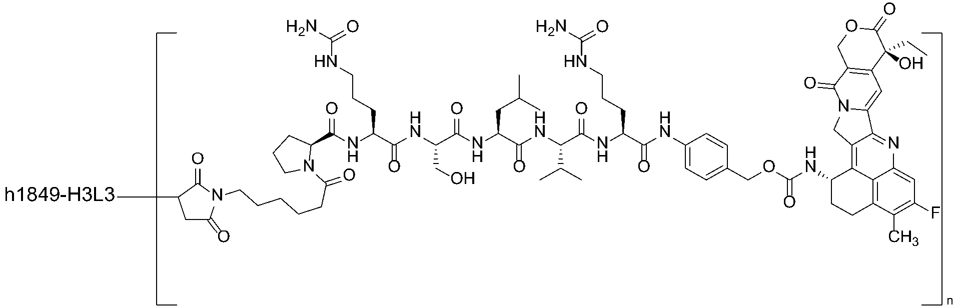

### 3.2.1.19 h1849-H3L3-ADC-19 (DAR8)

Compound L005-P005 in 3.2.1.1 was replaced by compound L052-P005 to obtain a conjugate h1849-H3L3-ADC-19 of L052-P005 and the antibody h1849-H3L3. The DAR value determined by the PLRP method was 7.74.

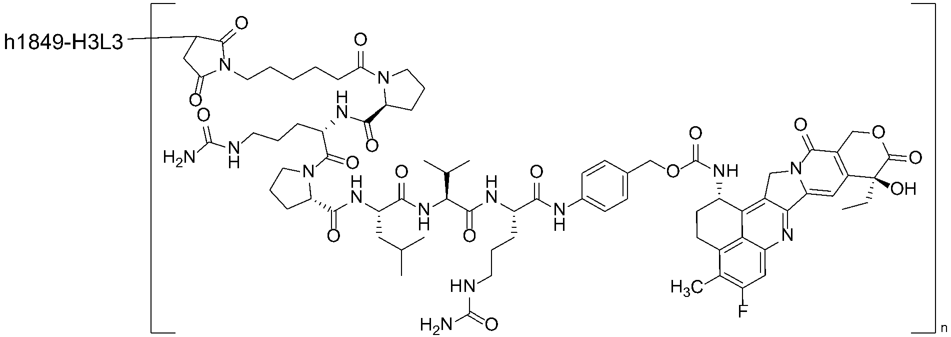

### 3.2.1.20 h1849-H3L3-ADC-20 (DAR8)

Compound L005-P005 in 3.2.1.1 was replaced by compound L053-P005 to obtain a conjugate h1849-H3L3-ADC-20 of L053-P005 and the antibody h1849-H3L3. The DAR value determined by the PLRP method was 8.06.

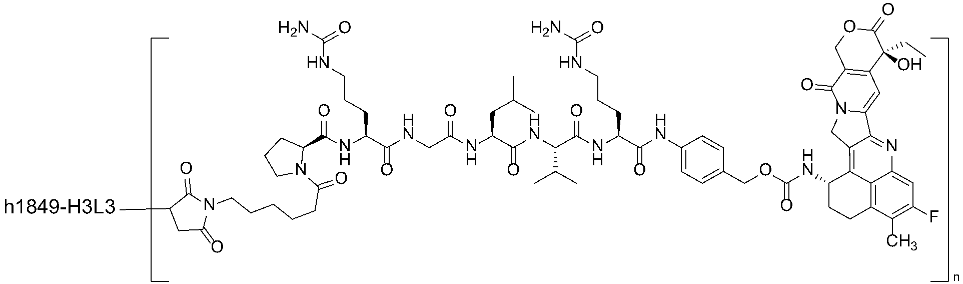

### 3.2.1.21 h1849-H3L3-ADC-21 (DAR8)

Compound L005-P005 in 3.2.1.1 was replaced by compound L054-P005 to obtain a conjugate h1849-H3L3-ADC-21 of L054-P005 and the antibody h1849-H3L3. The DAR value determined by the PLRP method was 7.78.

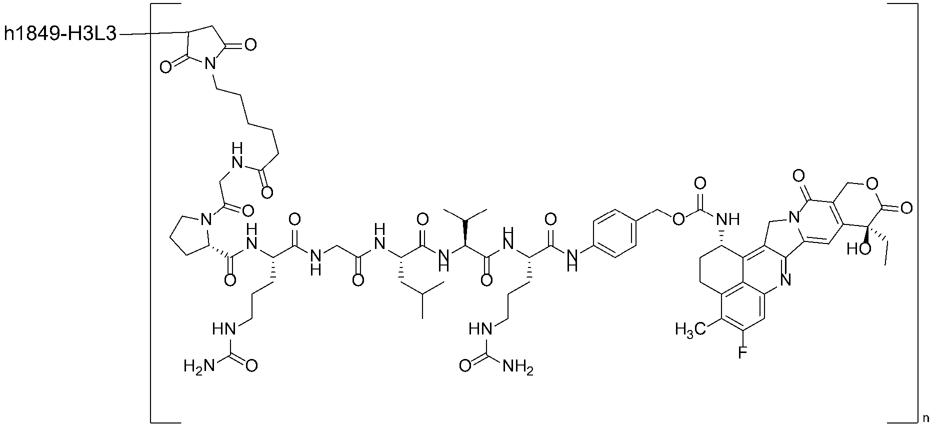

### 3.2.1.22 h1849-H3L3-ADC-22 (DAR8)

Compound L005-P005 in 3.2.1.1 was replaced by compound L055-P005 to obtain a conjugate h1849-H3L3-ADC-22 of L055-P005 and the antibody h1849-H3L3. The DAR value determined by the PLRP method was 7.8.

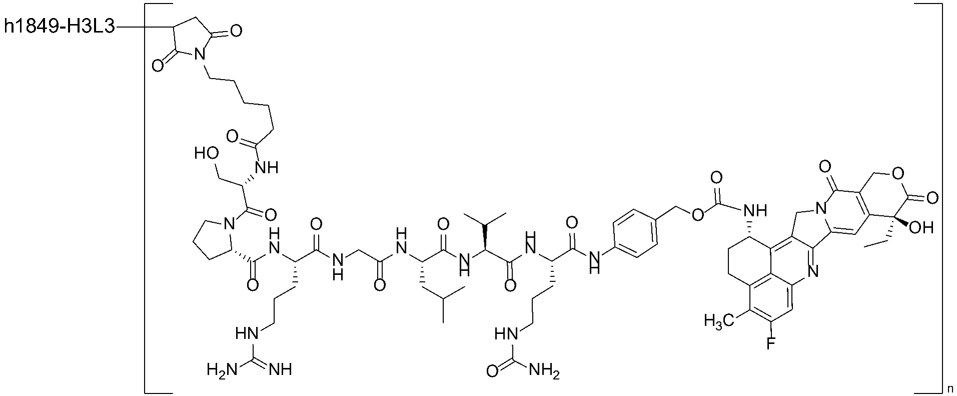

### 3.2.1.23 h1849-H3L3-ADC-23 (DAR8)

Compound L005-P005 in 3.2.1.1 was replaced by compound L056-P005 to obtain a conjugate h1849-H3L3-ADC-23 of L056-P005 and the antibody h1849-H3L3. The DAR value determined by the PLRP method was 7.81.

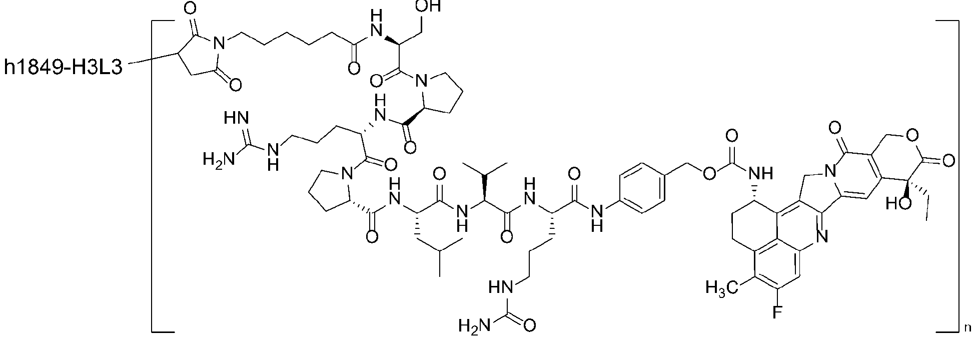

### 3.2.1.24 h1849-H3L3-ADC-24 (DAR8)

Compound L005-P005 in 3.2.1.1 was replaced by compound L057-P005 to obtain a conjugate h1849-H3L3-ADC-24 of L057-P005 and the antibody h1849-H3L3. The DAR value determined by the PLRP method was 7.76.

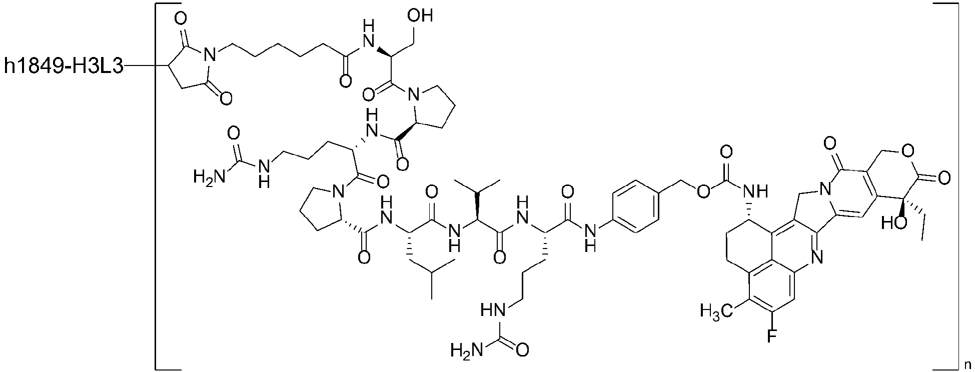

### 3.2.1.25 h1849-H3L3-ADC-25 (DAR8)

Compound L005-P005 in 3.2.1.1 was replaced by compound L058-P005 to obtain a conjugate h1849-H3L3-ADC-25 of L058-P005 and the antibody h1849-H3L3. The DAR value determined by the PLRP method was 7.79.

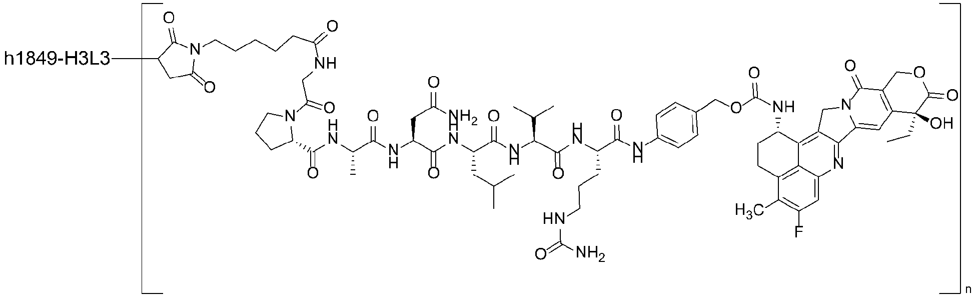

### 3.2.1.26 h1849-H3L3-ADC-26 (DAR8)

Compound L005-P005 in 3.2.1.1 was replaced by compound L059-P005 to obtain a conjugate h1849-H3L3-ADC-26 of L059-P005 and the antibody h1849-H3L3. The DAR value determined by the PLRP method was 7.83.

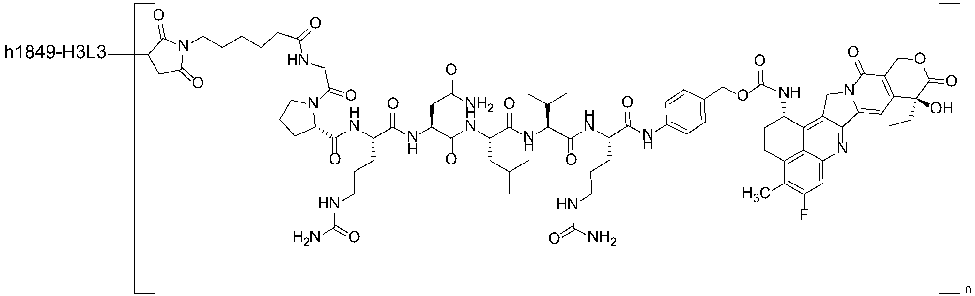

### 3.2.1.27 h1849-H3L3-ADC-27 (DAR8)

Compound L005-P005 in 3.2.1.1 was replaced by compound L060-P005 to obtain a conjugate h1849-H3L3-ADC-27 of L060-P005 and the antibody h1849-H3L3. The DAR value determined by the PLRP method was 7.81.

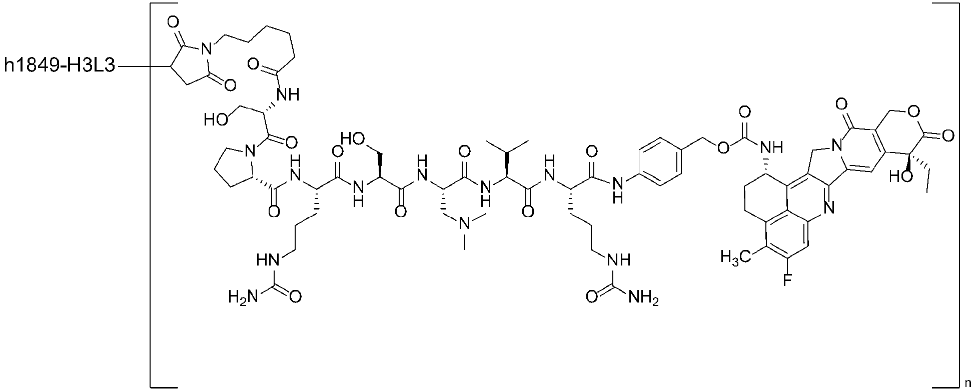

### 3.2.1.28 h1849-H3L3-ADC-28 (DAR8)

Compound L005-P005 in 3.2.1.1 was replaced by compound L062-P005 to obtain a conjugate h1849-H3L3-ADC-28 of L062-P005 and the antibody h1849-H3L3. The DAR value determined by the PLRP method was 7.74.

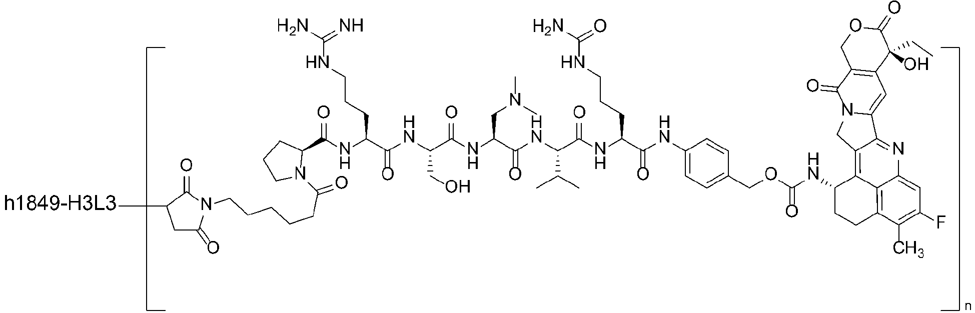

### 3.2.1.29 h1849-H3L3-ADC-29 (DAR8)

Compound L005-P005 in 3.2.1.1 was replaced by compound L064-P005 to obtain a conjugate h1849-H3L3-ADC-29 of L064-P005 and the antibody h1849-H3L3. The DAR value determined by the PLRP method was 7.52.

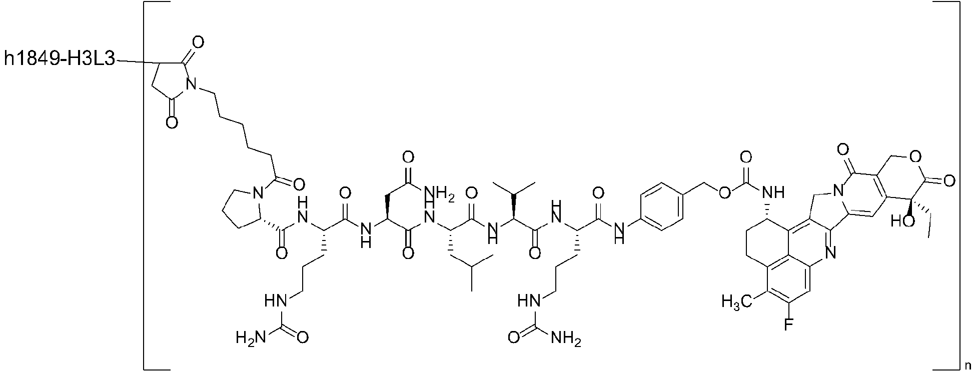

### 3.2.1.30 h1849-H3L3-ADC-30 (DAR8)

Compound L005-P005 in 3.2.1.1 was replaced by compound L065-P005 to obtain a conjugate h1849-H3L3-ADC-30 of L065-P005 and the antibody h1849-H3L3. The DAR value determined by the PLRP method was 7.89.

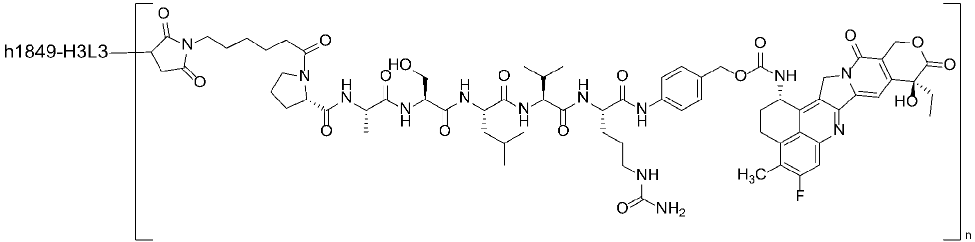

### 3.2.1.31 Preparation of h1849-H3L3-Deruxtecan (DAR8)

Compound L005-P005 in 3.2.1.1 was replaced by compound Deruxtecan (CAS: 1599440-13-7) to obtain a conjugate h1849-H3L3-Deruxtecan of Deruxtecan and the antibody h1849-H3L3. The DAR value determined by PLRP was 8.08.

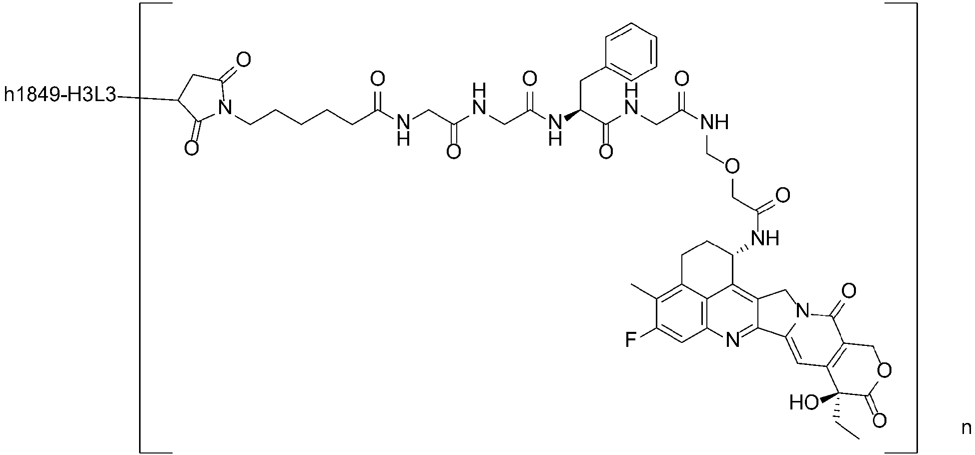

### 3.2.1.32 Preparation of h1849-H3L3-ADC-31 (DAR4)

The antibody h1849-H3L3 was placed in 20 mM histidine-acetate buffer (pH 5.5), and 5 mM tris(2-carboxyethyl)phosphine (TCEP) stock solution was added thereto at a TCEP/mAb ratio of 2.5. The mixed solution was placed at 37°C for reaction for 2 h. After 2 h, N,N-dimethylacetamide (DMA) and a solution of 15 mg/mL compound L005-P002 dissolved in DMA was added to the mixed solution. The final DMA content was 10%, and the Drug/mAb ratio was 7. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate h1849-H3L3-ADC-31 of L005-P002 and the antibody h1849-H3L3 was obtained, and the DAR value determined by the HIC method was 3.98.

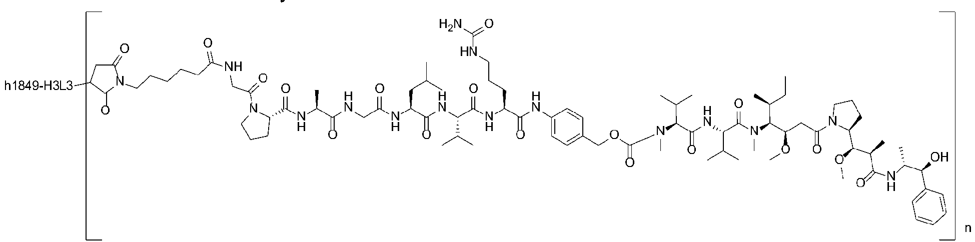

### 3.2.1.33 Preparation of h1849-H3L3-ADC-32 (DAR4)

Compound L005-P002 in 3.2.1.32 was replaced by compound L009-P002 to obtain a conjugate h1849-H3L3-ADC-32 of L009-P002 and the antibody h1849-H3L3. The DAR value determined by the HIC method was 3.20.

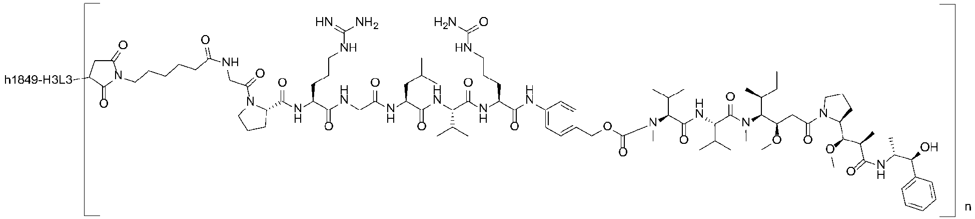

### 3.2.1.34 Preparation of h1849-H3L3-ADC-33 (DAR4)

Compound L005-P002 in 3.2.1.32 was replaced by compound L013-P002 to obtain a conjugate h1849-H3L3-ADC-33 of L013-P002 and the antibody h1849-H3L3. The DAR value determined by the HIC method was 4.10.

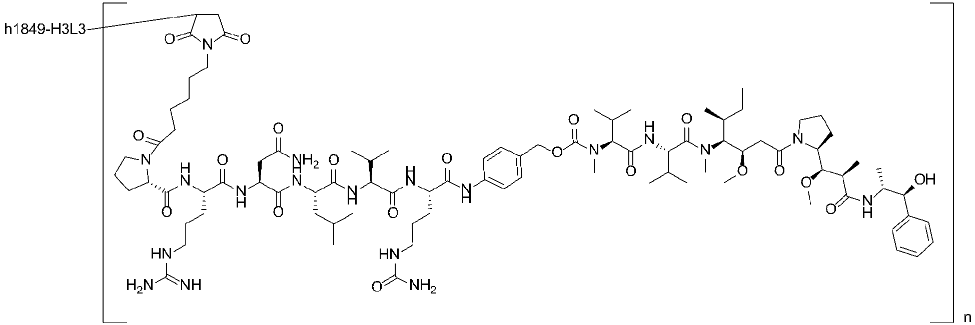

### 3.2.1.35 Preparation of h1849-H3L3-ADC-34 (DAR4)

Compound L005-P002 in 3.2.1.32 was replaced by compound L015-P002 to obtain a conjugate h1849-H3L3-ADC-34 of L015-P002 and the antibody h1849-H3L3. The DAR value determined by the HIC method was 3.92.

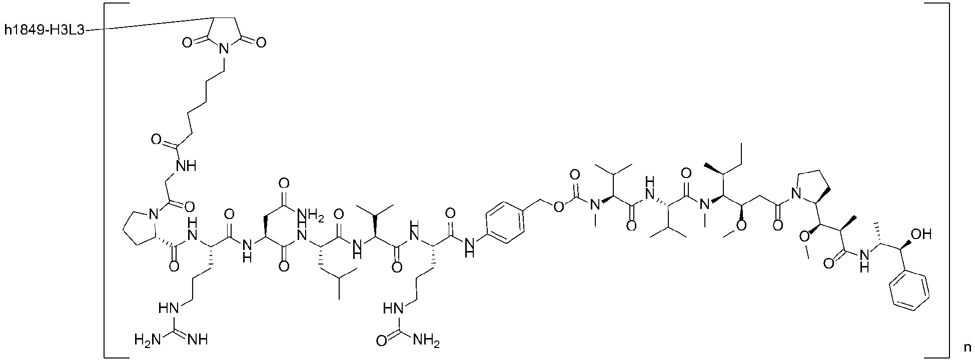

### 3.2.1.36 Preparation of h1849-H3L3-ADC-35 (DAR4)

Compound L005-P002 in 3.2.1.32 was replaced by compound L016-P002 to obtain a conjugate h1849-H3L3-ADC-35 of L016-P002 and the antibody h1849-H3L3. The DAR value determined by the HIC method was 3.93.

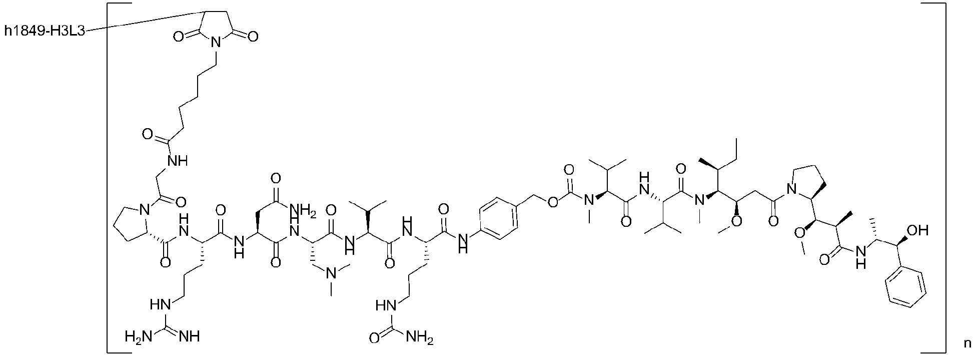

### 3.2.1.37 Preparation of h1849-H3L3-ADC-36 (DAR4)

Compound L005-P002 in 3.2.1.32 was replaced by compound L017-P002 to obtain a conjugate h1849-H3L3-ADC-36 of L017-P002 and the antibody h1849-H3L3. The DAR value determined by the RLRPmethod was 3.89.

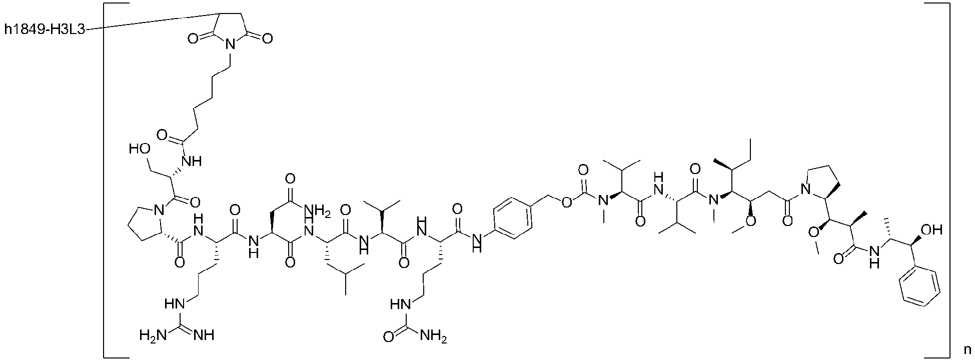

### 3.2.1.38 h1849-H3L3-ADC-37 (DAR4)

The antibody h1849-H3L3 was placed in 25 mM histidine-hydrochloride buffer (pH 5.5), and 5 mM tris(2-carboxyethyl)phosphine (TCEP) stock solution was added thereto at a TCEP/mAb ratio of 2.6. The mixed solution was placed at 37°C for reaction for 2 h. After 2 h, N,N-dimethylacetamide (DMA) and a solution of 15 mg/mL compound L038-P002 dissolved in DMA was added to the mixed solution. The final DMA content was 10%, and the Drug/mAb ratio was 7. The solution was continuously placed at 22°C for 2 h to perform the coupling reaction sufficiently. After completion of the reaction, a ZebaTM centrifugal desalting column with 40 KD MWCO was used for one-step purification, and the conjugated product was exchanged into 25 mM histidine-hydrochloride(pH 5.5) buffer for storage. The conjugate h1849-H3L3-ADC-37 of L038-P002 and the antibody h1849-H3L3 was obtained, and the DAR value determined by the PLRP method was 3.94.

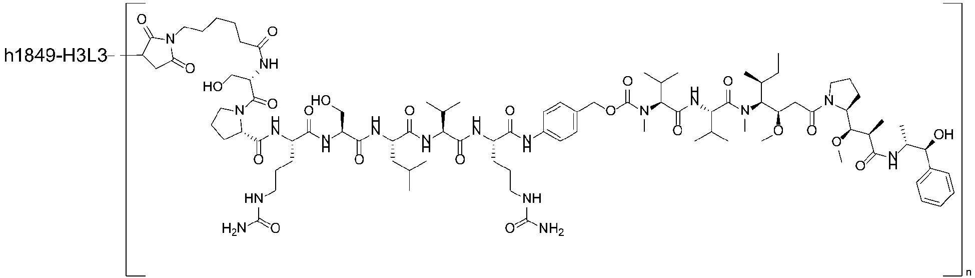

### 3.2.1.39 h1849-H3L3-ADC-38 (DAR4)

Compound L038-P002 in 3.2.1.38 was replaced by compound L060-P002 to obtain a conjugate h1849-H3L3-ADC-38 of L060-P002 and the antibody h1849-H3L3. The DAR value determined by the PLRP method was 3.73.

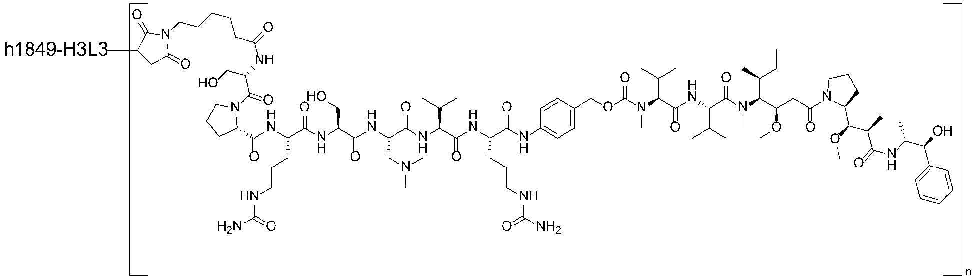

### 3.2.1.40 h1849-H3L3-ADC-39 (DAR4)

The antibody h1849-H3L3 was placed in 25 mM histidine-hydrochloride buffer (pH 5.5), and 5 mM tris(2-carboxyethyl)phosphine (TCEP) stock solution was added thereto at a TCEP/mAb ratio of 2.6. The mixed solution was placed at 37°C for reaction for 2 h. After 2 h, N,N-dimethylacetamide (DMA) and a solution of 15 mg/mL compound L062-P002 dissolved in DMA was added to the mixed solution. The final DMA content was 10%, and the Drug/mAb ratio was 8. The solution was continuously placed at 22°C for 4 h to perform the coupling reaction sufficiently. After completion of the reaction, a ZebaTM centrifugal desalting column with 40 KD MWCO was used for one-step purification, and the conjugated product was exchanged into 25 mM histidine-hydrochloride (pH 5.5) buffer for storage. The conjugate h1849-H3L3-ADC-39 of L062-P002 and the antibody h1849-H3L3 was obtained, and the DAR value determined by the PLRP method was 3.56.

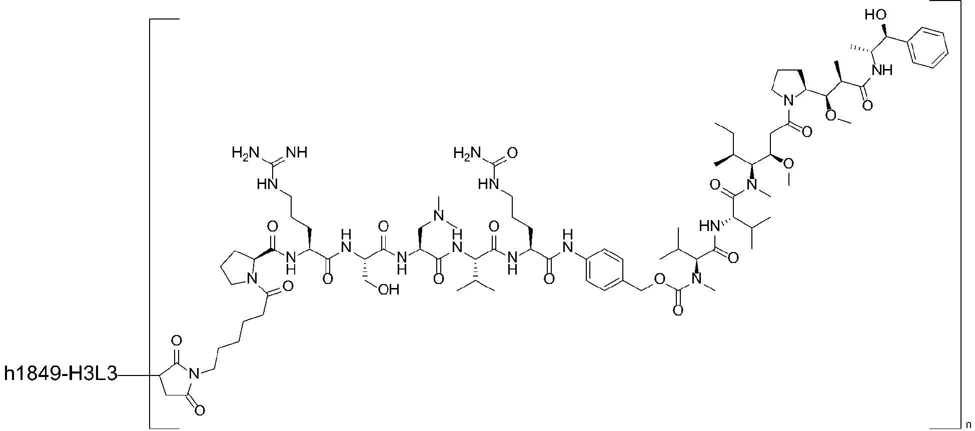

### 3.2.1.41 Preparation of h1849-H3L3-ADC-40 (DAR8)

Compound L062-P002 in 3.2.1.40 was replaced by compound L016-P002 to obtain a conjugate h1849-H3L3-ADC-40 of L016-P002 and the antibody h1849-H3L3. The DAR value determined by the PLRP method was 8.08.

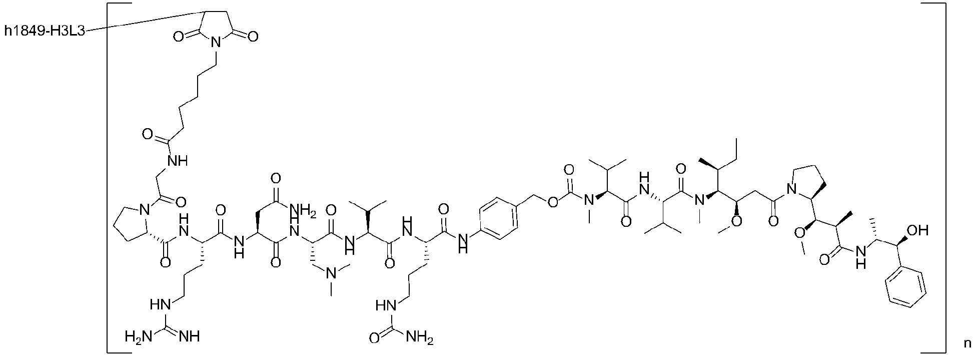

### 3.2.1.42 Preparation of h1849-H3L3-vcMMAE (DAR4)

Compound L005-P002 in 3.2.1.32 was replaced by compound vcMMAE (CAS No.: 646502-53-6) to obtain a conjugate h1849-H3L3-vcMMAE of vcMMAE and the antibody h1849-H3L3. The DAR value determined by HIC was 4.0.

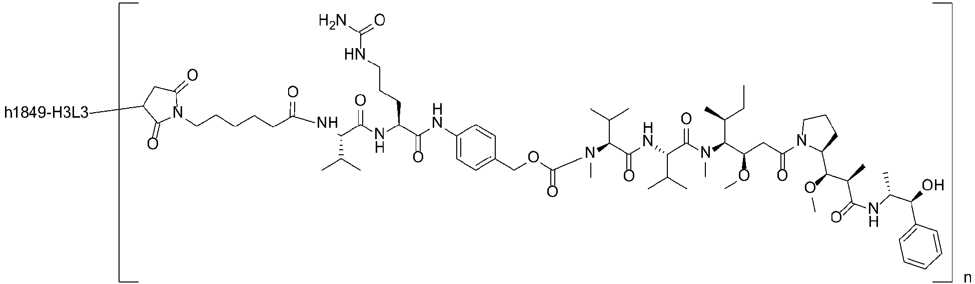

### 3.2.2 Preparation of GC33-ADC

### 3.2.2.1 Preparation of GC33-ADC-01 (DAR8)

The antibody Codrituzumab (GC33; anti-GPC3 antibody) was placed in 20 mM histidine-acetate buffer (pH 5.5), and 5 mM tris(2-carboxyethyl)phosphine (TCEP) stock solution was added thereto at a TCEP/mAb ratio of 6. The mixed solution was placed at 37°C for reaction for 2 h. After 2 h, N,N-dimethylacetamide (DMA) and a solution of 15 mg/mL compound L005-P005 dissolved in DMA was added to the mixed solution. The final DMA content was 10%, and the Drug/mAb ratio was 14. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate GC33-ADC-01 of L005-P005 and the antibody GC33 was obtained, and the DAR value determined by the PLRP method was 8.00.

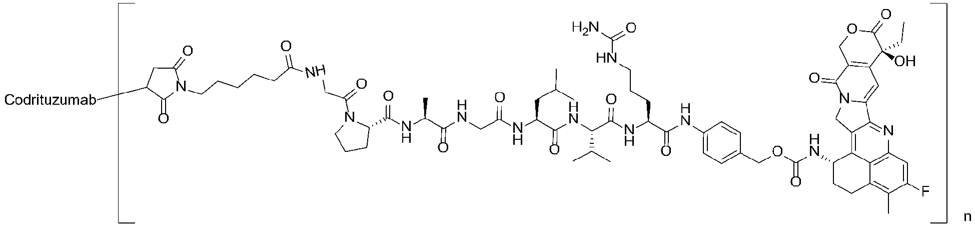

### 3.2.2.2 Preparation of GC33-ADC-02 (DAR8)

Compound L005-P005 in 3.2.2.1 was replaced by compound L009-P005 to obtain a conjugate GC33-ADC-02 of L009-P005 and the antibody GC33. The DAR value determined by the PLRP method was 8.00.

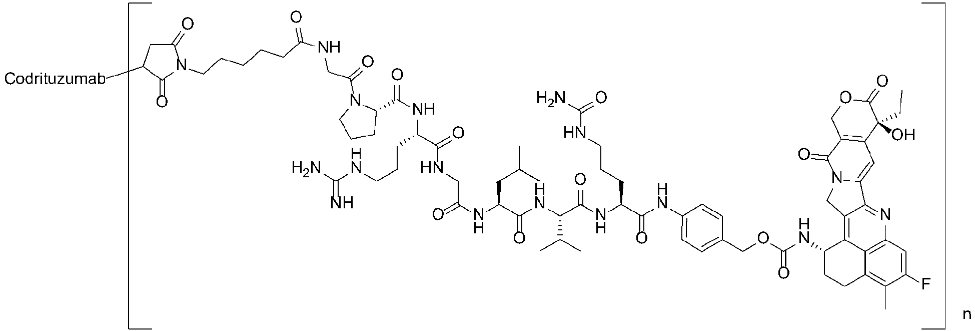

### 3.2.2.3 Preparation of GC33-ADC-03 (DAR8)

Compound L005-P005 in 3.2.2.1 was replaced by compound L013-P005 to obtain a conjugate GC33-ADC-03 of L013-P005 and the antibody GC33. The DAR value determined by the PLRP method was 8.12.

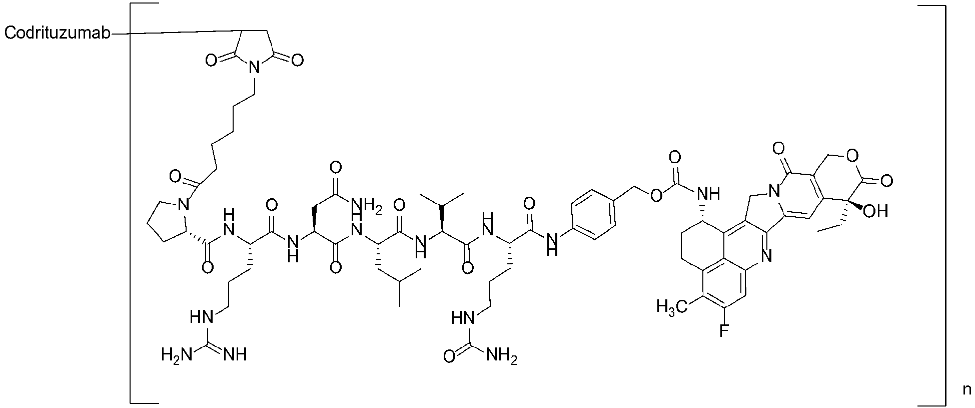

### 3.2.2.4 Preparation of GC33-ADC-04 (DAR8)

Compound L005-P005 in 3.2.2.1 was replaced by compound L015-P005 to obtain a conjugate GC33-ADC-04 of L015-P005 and the antibody GC33. The DAR value determined by the PLRP method was 8.08.

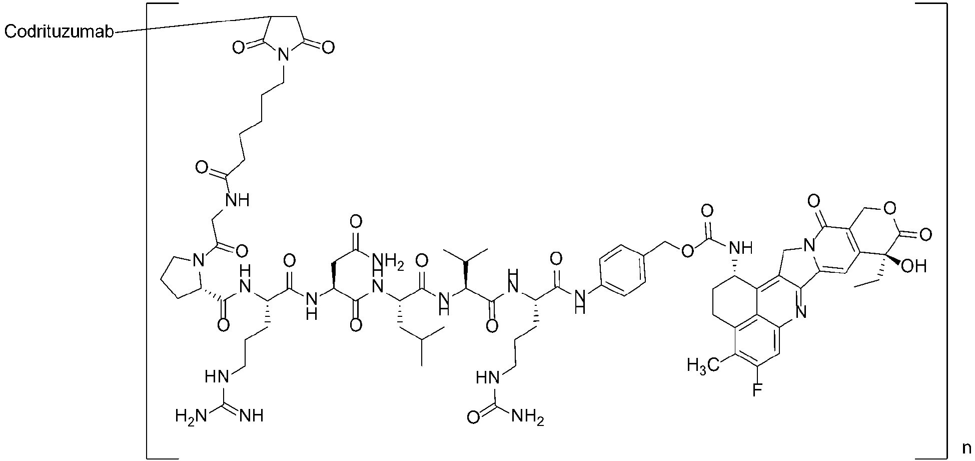

### 3.2.2.5 Preparation of GC33-ADC-05 (DAR8)

Compound L005-P005 in 3.2.2.1 was replaced by compound L016-P005 to obtain a conjugate GC33-ADC-05 of L016-P005 and the antibody GC33. The DAR value determined by the PLRP method was 6.99.

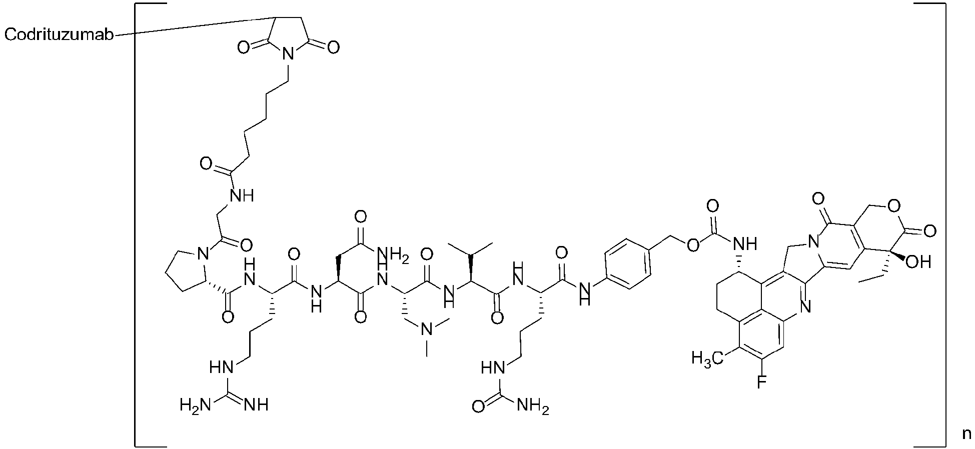

### 3.2.2.6 Preparation of GC33-ADC-06 (DAR8)

Compound L005-P005 in 3.2.2.1 was replaced by compound L017-P005 to obtain a conjugate GC33-ADC-06 of L017-P005 and the antibody GC33. The DAR value determined by the PLRP method was 8.03.

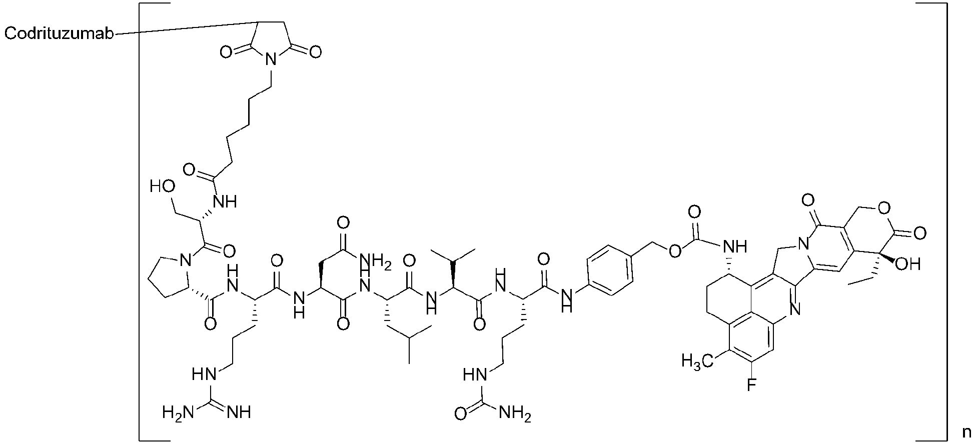

### 3.2.2.7 Preparation of GC33-ADC-07 (DAR8)

Compound L005-P005 in 3.2.2.1 was replaced by compound L021-P005 to obtain a conjugate GC33-ADC-07 of L021-P005 and the antibody GC33. The DAR value determined by the PLRP method was 8.00.

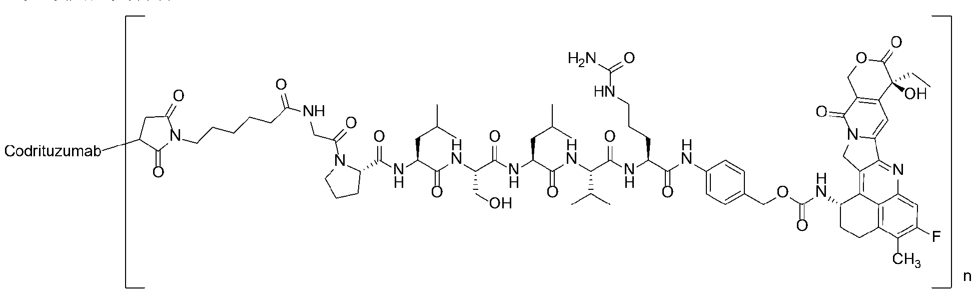

### 3.2.2.8 Preparation of GC33-Deruxtecan (DAR8)

Compound L005-P005 in 3.2.2.1 was replaced by compound Deruxtecan (CAS: 1599440-13-7) to obtain a conjugate GC33-Deruxtecan of Deruxtecan and the antibody GC33. The DAR value determined by the PLRP method was 8.00.

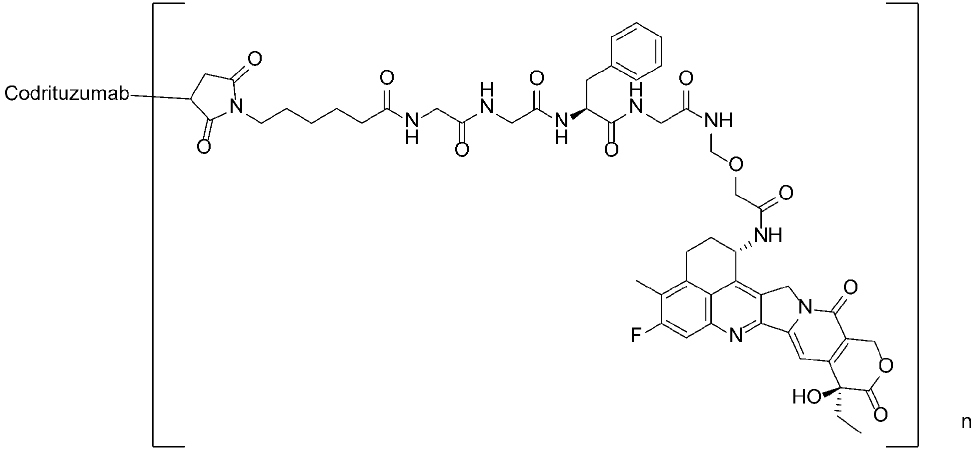

### 3.2.2.9 Preparation of GC33-ADC-08 (DAR4)

The antibody Codrituzumab (GC33) was placed in 20 mM histidine-acetate buffer (pH 5.5), and 5 mM tris(2-carboxyethyl)phosphine (TCEP) stock solution was added thereto at a TCEP/mAb ratio of 2.5. The mixed solution was placed at 37°C for reaction for 2 h. After 2 h, N,N-dimethylacetamide (DMA) and a solution of 15 mg/mL compound L005-P002 dissolved in DMA was added to the mixed solution. The final DMA content was 10%, and the Drug/mAb ratio was 7. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate GC33-ADC-08 of L005-P002 and the antibody GC33 was obtained, and the DAR value determined by the HIC method was 4.36.

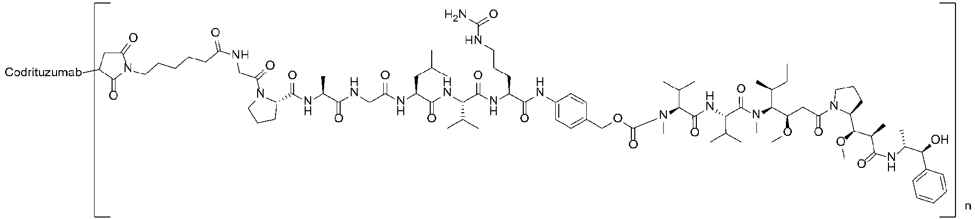

### 3.2.2.10 Preparation of GC33-ADC-09 (DAR4)

Compound L005-P002 in 3.2.2.9 was replaced by compound L009-P002 to obtain a conjugate GC33-ADC-09 of L009-P002 and the antibody GC33. The DAR value determined by the HIC method was 3.92.

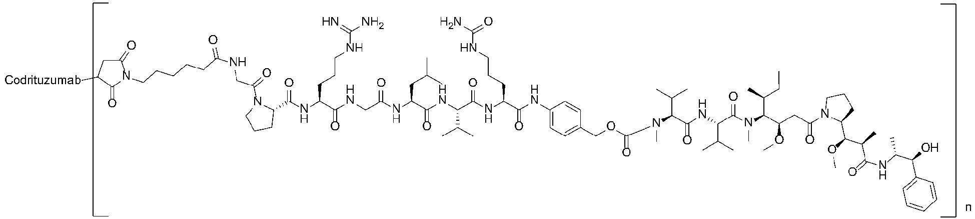

### 3.2.2.11 Preparation of GC33-ADC-10 (DAR4)

Compound L005-P002 in 3.2.2.9 was replaced by compound L013-P002 to obtain a conjugate GC33-ADC-10 of L013-P002 and the antibody GC33. The DAR value determined by the HIC method was 4.06.

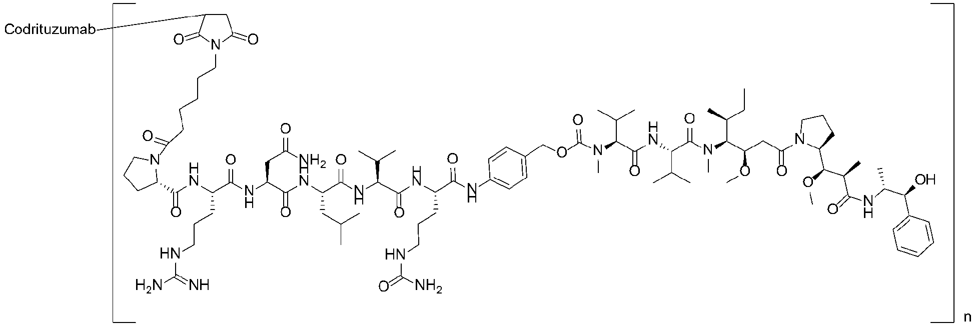

### 3.2.2.12 Preparation of GC33-ADC-11 (DAR4)

Compound L005-P002 in 3.2.2.9 was replaced by compound L015-P002 to obtain a conjugate GC33-ADC-11 of L015-P002 and the antibody GC33. The DAR value determined by the HIC method was 4.00.

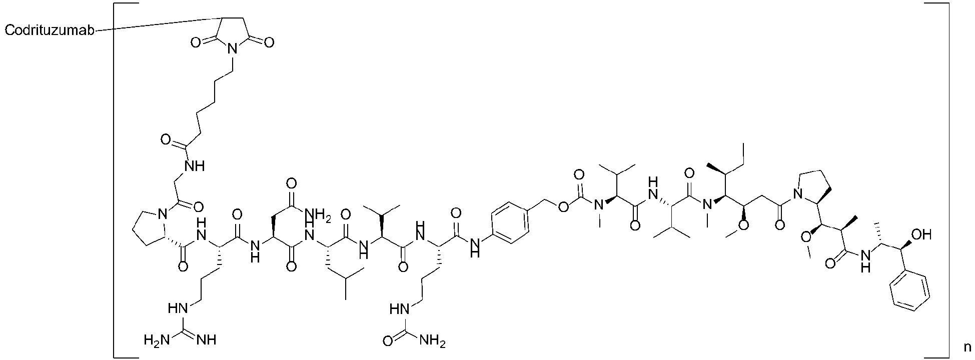

### 3.2.2.13 Preparation of GC33-ADC-12 (DAR4)

Compound L005-P002 in 3.2.2.9 was replaced by compound L016-P002 to obtain a conjugate GC33-ADC-12 of L016-P002 and the antibody GC33. The DAR value determined by the HIC method was 3.89.

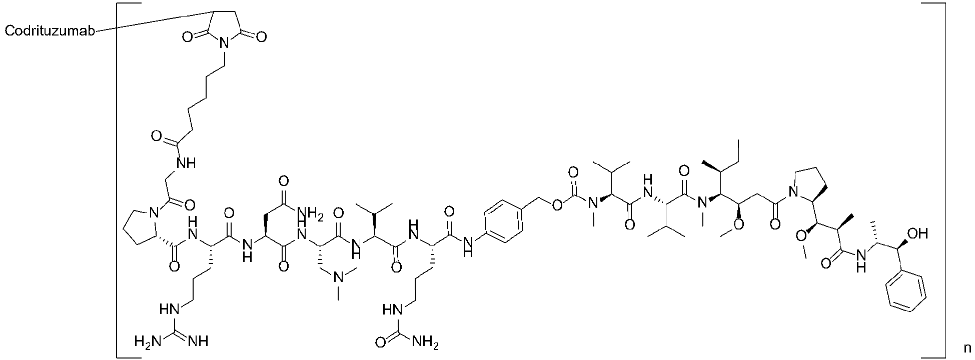

### 3.2.2.14 Preparation of GC33-ADC-13 (DAR4)

Compound L005-P002 in 3.2.2.9 was replaced by compound L017-P002 to obtain a conjugate GC33-ADC-13 of L017-P002 and the antibody GC33. The DAR value determined by the HIC method was 4.15.

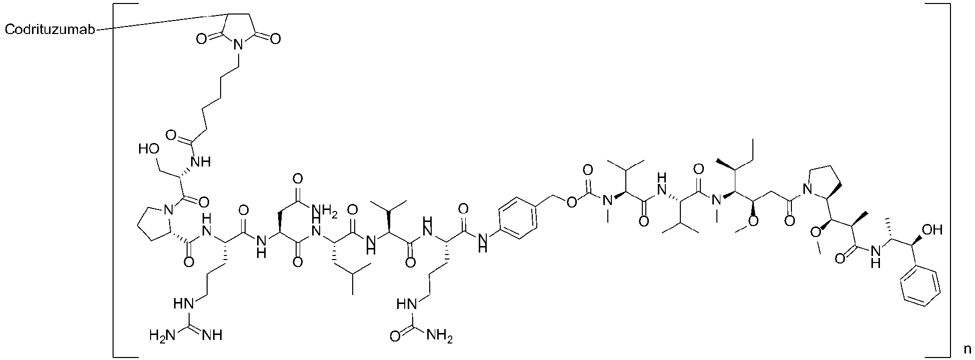

### 3.2.2.15 Preparation of GC33-ADC-14 (DAR4)

Compound L005-P002 in 3.2.2.9 was replaced by compound L021-P002 to obtain a conjugate GC33-ADC-14 of L021-P002 and the antibody GC33. The DAR value determined by the HIC method was 4.65.

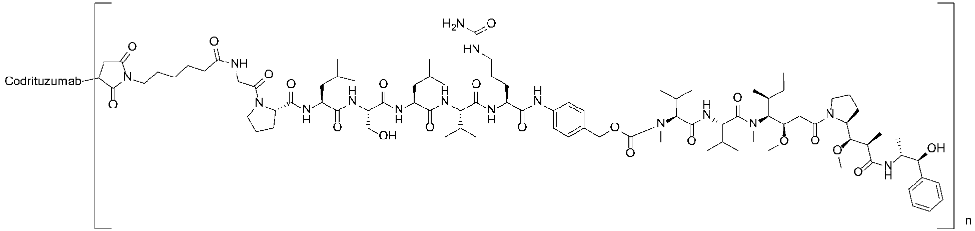

### 3.2.2.16 Preparation of GC33-ADC-15 (DAR4)

Compound L005-P002 in 3.2.2.9 was replaced by compound L005-P003 to obtain a conjugate GC33-ADC-15 of L005-P003 and the antibody GC33. The DAR value determined by the HIC method was 3.81.

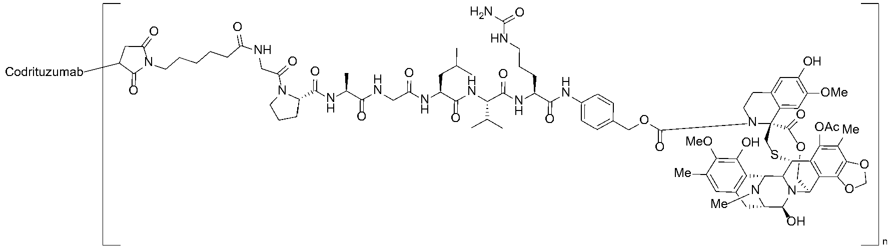

### 3.2.3 Preparation of anti-Her2-ADC

### 3.2.3.1 Preparation of anti-Her2-ADC-01 (DAR8)

The antibody Trastuzumab (i.e., Herceptin, anti-Her2 monoclonal antibody) was exchanged into a 20 mM histidine-acetate buffer (pH 5.5), and 5 mM tris(2-carboxyethyl)phosphine (TCEP) stock solution was added thereto at a TCEP/mAb ratio of 6. The mixed solution was placed at 37°C for reaction for 2 h. After 2 h, N,N-dimethylacetamide (DMA) and a solution of 15 mg/mL compound L005-P005 dissolved in DMA was added to the mixed solution. The final DMA content was 10%, and the Drug/mAb ratio was 14. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate anti-Her2-ADC-01 of L005-P005 and the antibody anti-Her2 was obtained, and the DAR value determined by the PLRP method was 8.19.

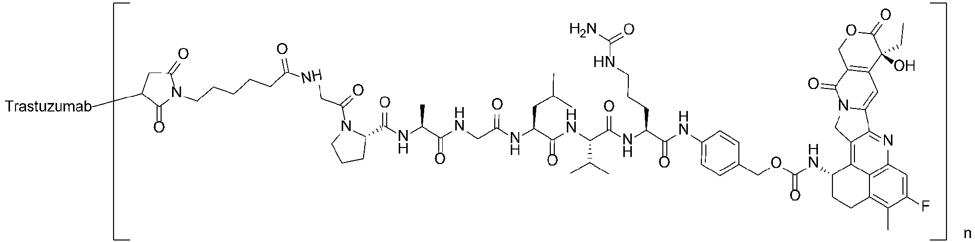

### 3.2.3.2 Preparation of anti-Her2-ADC-02 (DAR8)

Compound L005-P005 in 3.2.3.1 was replaced by compound L006-P005 to obtain a conjugate anti-Her2-ADC-02 of L006-P005 and the anti-Her2 antibody. The DAR value determined by the PLRP method was 8.00.

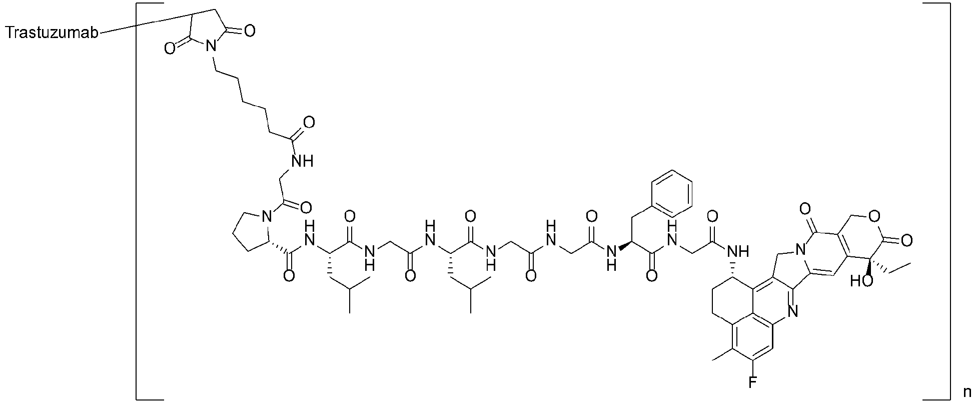

### 3.2.3.3 Preparation of anti-Her2-ADC-03 (DAR8)

Compound L005-P005 in 3.2.3.1 was replaced by compound L008-P005 to obtain a conjugate anti-Her2-ADC-03 of L008-P005 and the anti-Her2 antibody. The DAR value determined by the PLRP method was 8.00.

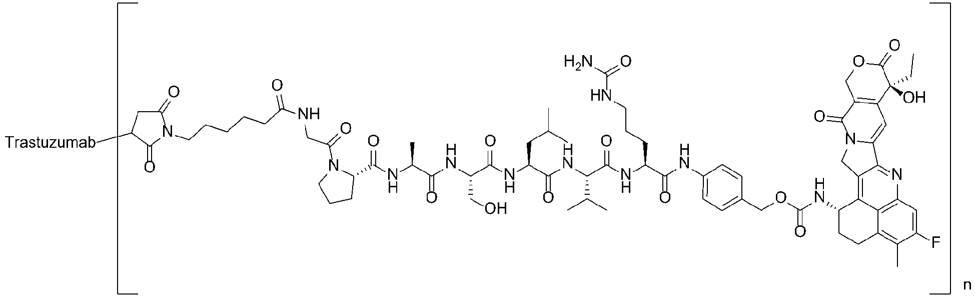

### 3.2.3.4 Preparation of anti-Her2-ADC-04 (DAR8)

Compound L005-P005 in 3.2.3.1 was replaced by compound L009-P005 to obtain a conjugate anti-Her2-ADC-04 of L009-P005 and the anti-Her2 antibody. The DAR value determined by the PLRP method was 7.98.

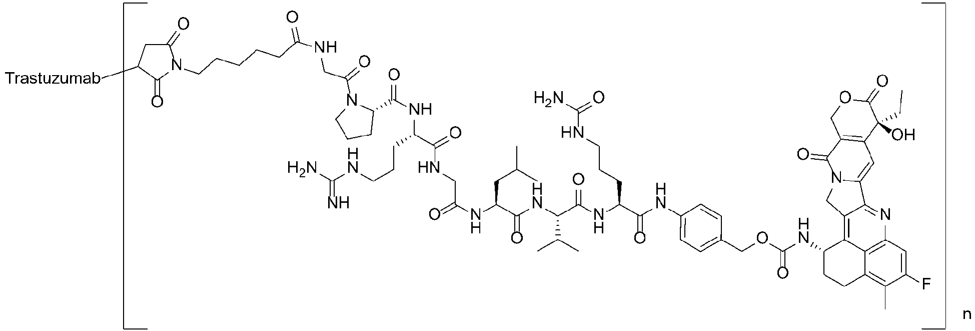

### 3.2.3.5 Preparation of anti-Her2-ADC-05 (DAR8)

Compound L005-P005 in 3.2.3.1 was replaced by compound L010-P005 to obtain a conjugate anti-Her2-ADC-05 of L010-P005 and the anti-Her2 antibody. The DAR value determined by the PLRP method was 7.98.

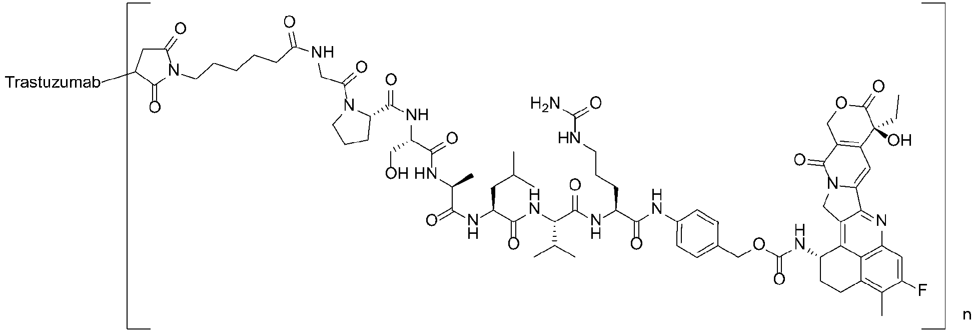

### 3.2.3.6 Preparation of anti-Her2-ADC-06 (DAR8)

Compound L005-P005 in 3.2.3.1 was replaced by compound L011-P005 to obtain a conjugate anti-Her2-ADC-06 of L011-P005 and the anti-Her2 antibody. The DAR value determined by the PLRP method was 7.94.

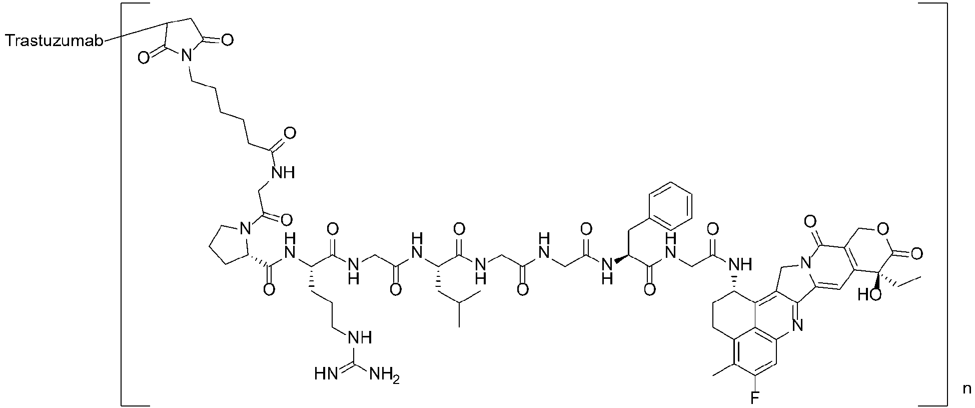

### 3.2.3.7 Preparation of anti-Her2-ADC-07 (DAR8)

Compound L005-P005 in 3.2.3.1 was replaced by compound L012-P005 to obtain a conjugate anti-Her2-ADC-07 of L012-P005 and the anti-Her2 antibody. The DAR value determined by the PLRP method was 8.00.

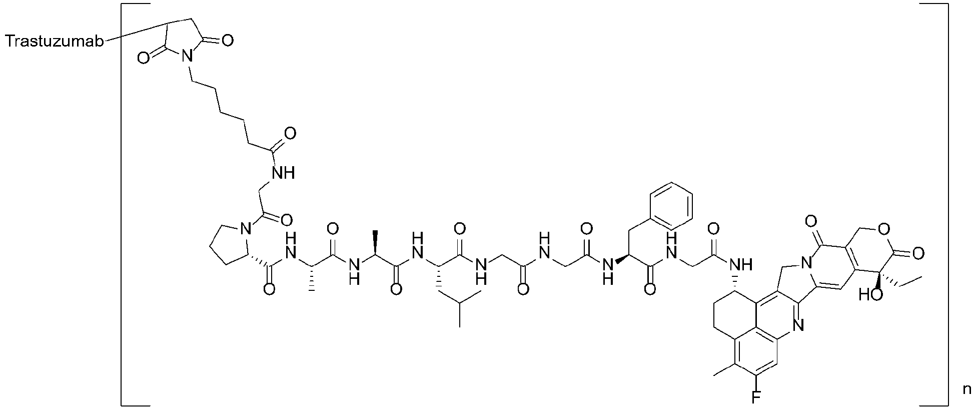

### 3.2.3.8 Preparation of anti-Her2-ADC-08 (DAR8)

Compound L005-P005 in 3.2.3.1 was replaced by compound L013-P005 to obtain a conjugate anti-Her2-ADC-08 of L013-P005 and the anti-Her2 antibody. The DAR value determined by mass spectrometry was 6.68.

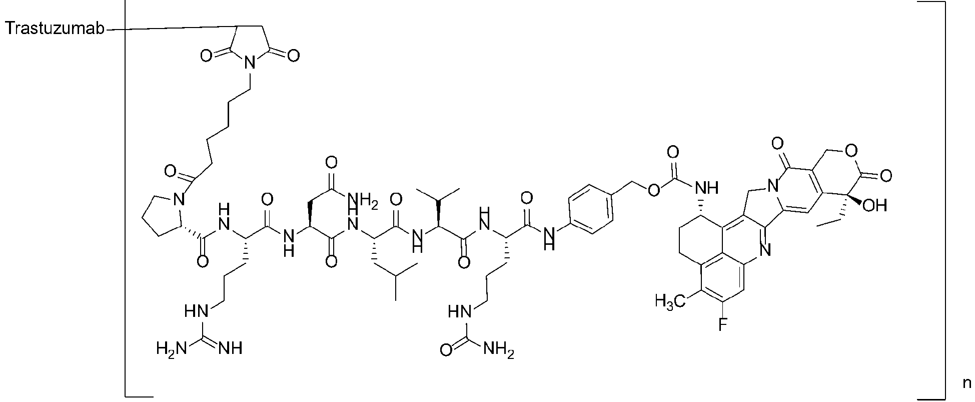

### 3.2.3.9 Preparation of anti-Her2-ADC-09 (DAR8)

Compound L005-P005 in 3.2.3.1 was replaced by compound L015-P005 to obtain a conjugate anti-Her2-ADC-09 of L015-P005 and the anti-Her2 antibody. The DAR value determined by mass spectrometry was 8.17.

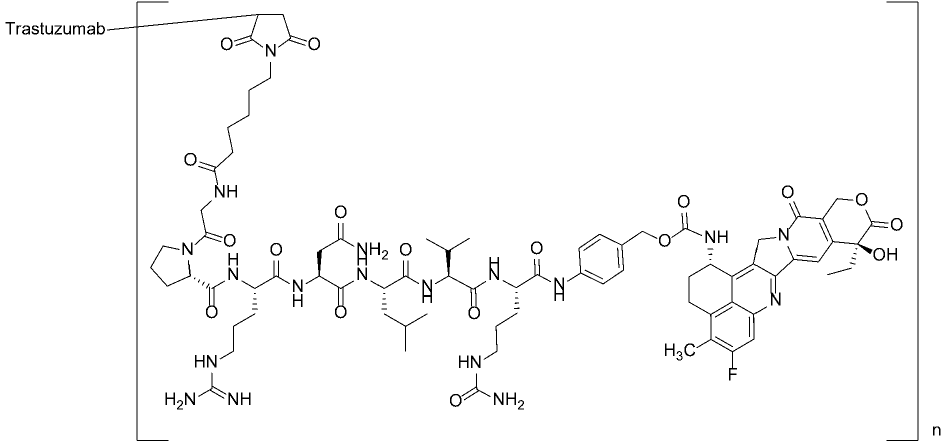

### 3.2.3.10 Preparation of anti-Her2-ADC-10 (DAR8)

Compound L005-P005 in 3.2.3.1 was replaced by compound L016-P005 to obtain a conjugate anti-Her2-ADC-10 of L016-P005 and the anti-Her2 antibody. The DAR value determined by mass spectrometry was 7.44.

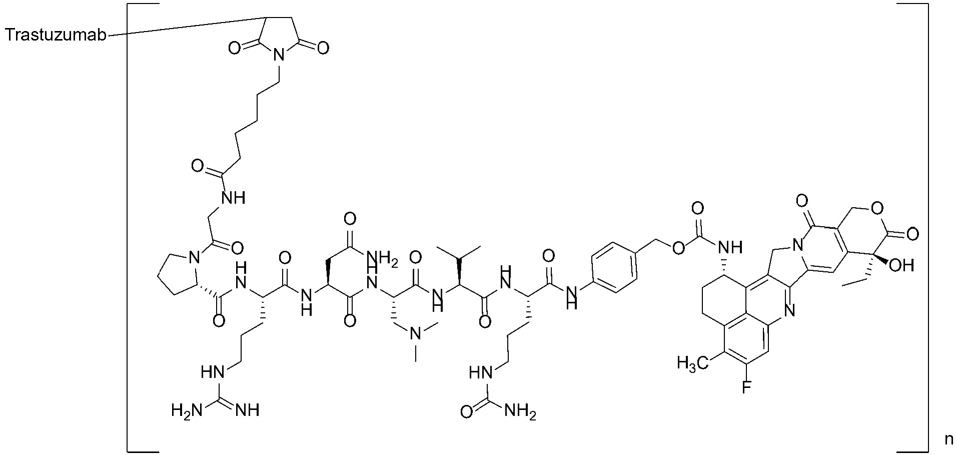

### 3.2.3.11 Preparation of anti-Her2-ADC-11 (DAR8)

Compound L005-P005 in 3.2.3.1 was replaced by compound L017-P005 to obtain a conjugate anti-Her2-ADC-11 of L017-P005 and the anti-Her2 antibody. The DAR value determined by mass spectrometry was 7.82.

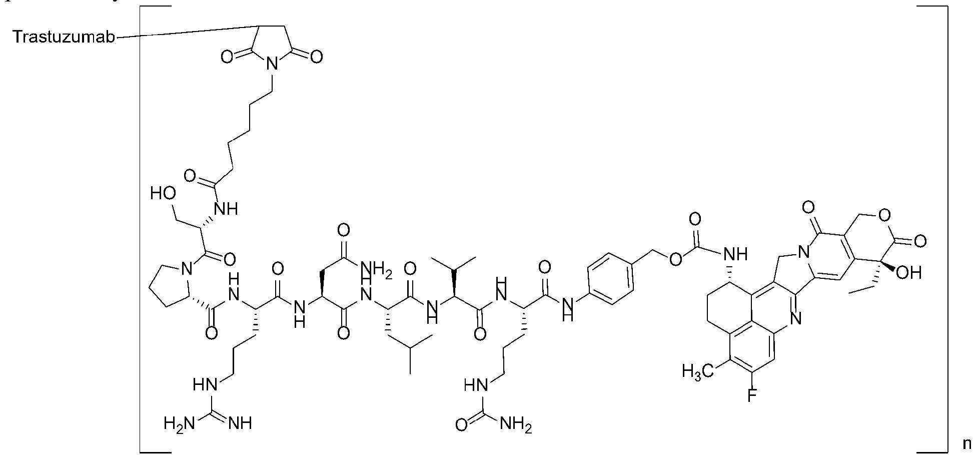

### 3.2.3.12 Preparation of anti-Her2-ADC-12 (DAR8)

Compound L005-P005 in 3.2.3.1 was replaced by compound L039-P005 to obtain a conjugate anti-Her2-ADC-12 of L039-P005 and the anti-Her2 antibody. The DAR value determined by mass spectrometry was 7.98.

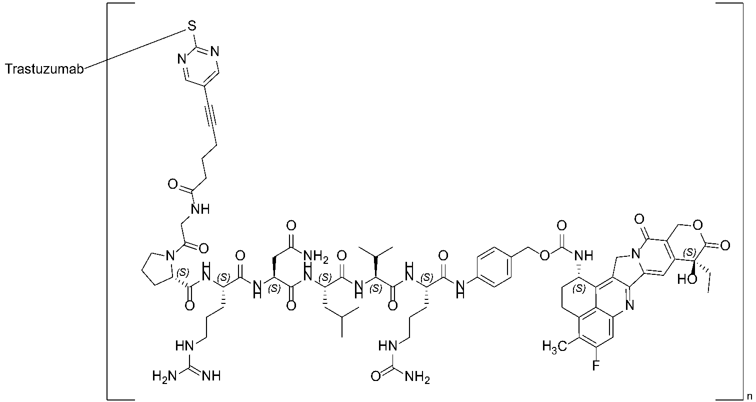

### 3.2.3.13 Preparation of anti-Her2-ADC-13 (DAR8)

Compound L005-P005 in 3.2.3.1 was replaced by compound L041-P005 to obtain a conjugate anti-Her2-ADC-13 of L041-P005 and the anti-Her2 antibody. The DAR value determined by mass spectrometry was 6.3.

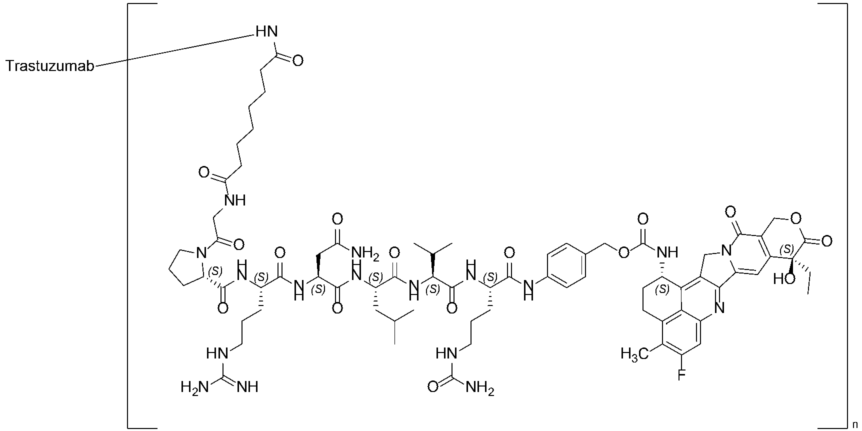

### 3.2.3.14 Preparation of anti-Her2-ADC-14 (DAR8)

Compound L005-P005 in 3.2.3.1 was replaced by compound L043-P005 to obtain a conjugate anti-Her2-ADC-14 of L043-P005 and the anti-Her2 antibody. The DAR value determined by mass spectrometry was 8.0.

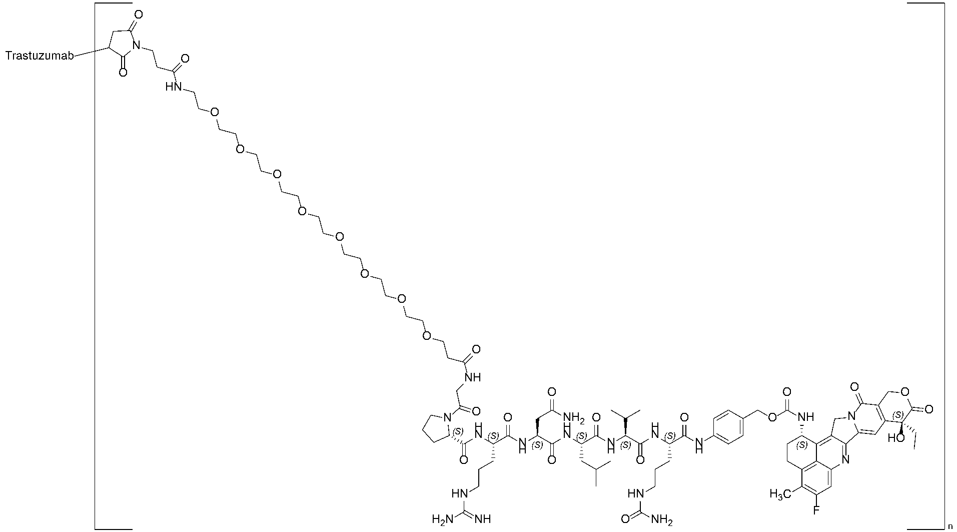

### 3.2.3.15 Preparation of anti-Her2-ADC-15 (DAR8)

Compound L005-P005 in 3.2.3.1 was replaced by compound L045-P006 to obtain a conjugate anti-Her2-ADC-15 of L045-P006 and the anti-Her2 antibody. The DAR value determined by mass spectrometry was 8.0.

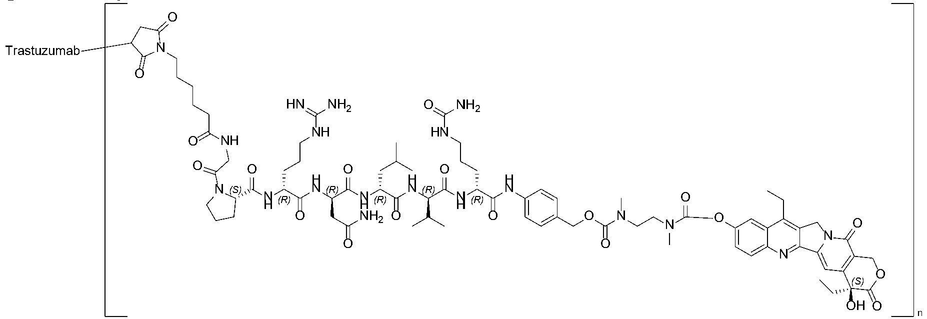

### 3.2.3.16 Preparation of anti-Her2-ADC-16 (DAR8)

Compound L005-P005 in 3.2.3.1 was replaced by compound L047-P001 to obtain a conjugate anti-Her2-ADC-16 of L047-P001 and the anti-Her2 antibody. The DAR value determined by mass spectrometry was 7.84.

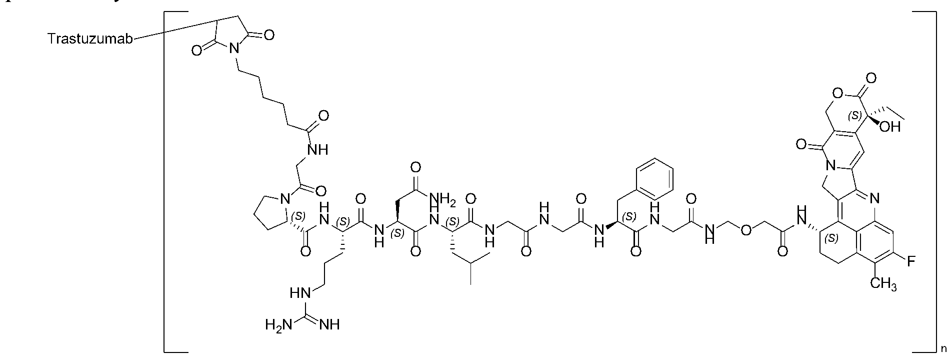

### 3.2.3.17 Preparation of anti-Her2-ADC-17 (DAR8)

Compound L005-P005 in 3.2.3.1 was replaced by compound L048-P001 to obtain a conjugate anti-Her2-ADC-17 of L048-P001 and the anti-Her2 antibody. The DAR value determined by mass spectrometry was 7.95.

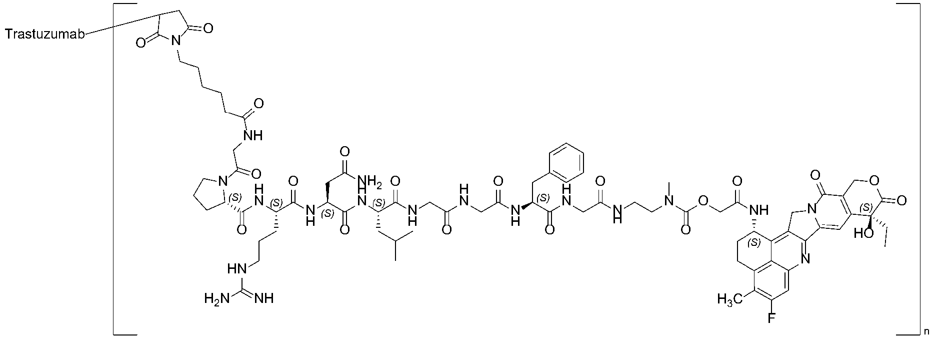

### 3.2.3.18 Preparation of anti-Her2-Deruxtecan (DAR8)

Compound L005-P005 in 3.2.3.1 was replaced by Deruxtecan (CAS No: 1599440-13-7) to obtain a conjugate anti-Her2-Deruxtecan of compound Deruxtecan and the anti-Her2 antibody. The DAR value determined by mass spectrometry was 8.00.

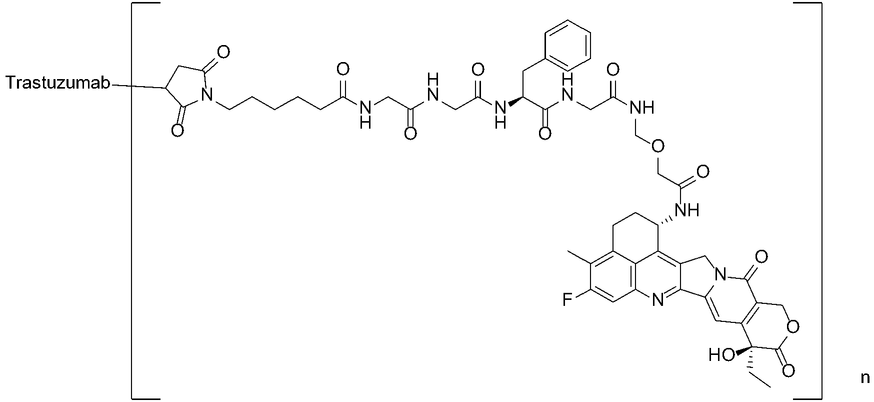

### 3.2.4 Preparation of anti-ROR1-ADC

### 3.2.4.1 Preparation of anti-ROR1-ADC-01 (DAR8)

The antibody Zilovertamab was placed in 20 mM histidine-acetate buffer (pH 5.5), and 5 mM tris(2-carboxyethyl)phosphine (TCEP) stock solution was added thereto at a TCEP/mAb ratio of 6. The mixed solution was placed at 37°C for reaction for 2 h. After 2 h, N,N-dimethylacetamide (DMA) and a solution of 15 mg/mL compound L009-P005 dissolved in DMA was added to the mixed solution. The final DMA content was 10%, and the Drug/mAb ratio was 14. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate anti-ROR1-ADC-01 of L009-P005 and the anti-ROR1 antibody was obtained, and the DAR value determined by the PLRP method was 8.02.

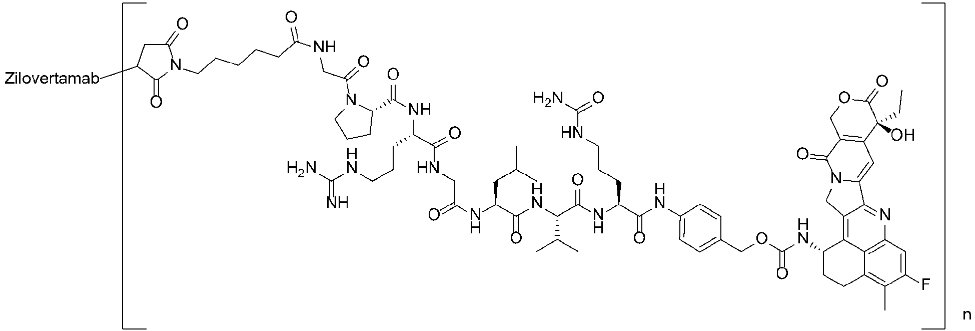

### 3.2.4.2 Preparation of anti-ROR1-ADC-02 (DAR8)

Compound L009-P005 in 3.2.4.1 was replaced by compound L015-P005 to obtain a conjugate anti-ROR1-ADC-02 of L015-P005 and the anti-ROR1 antibody. The DAR value determined by PLRP was 7.95.

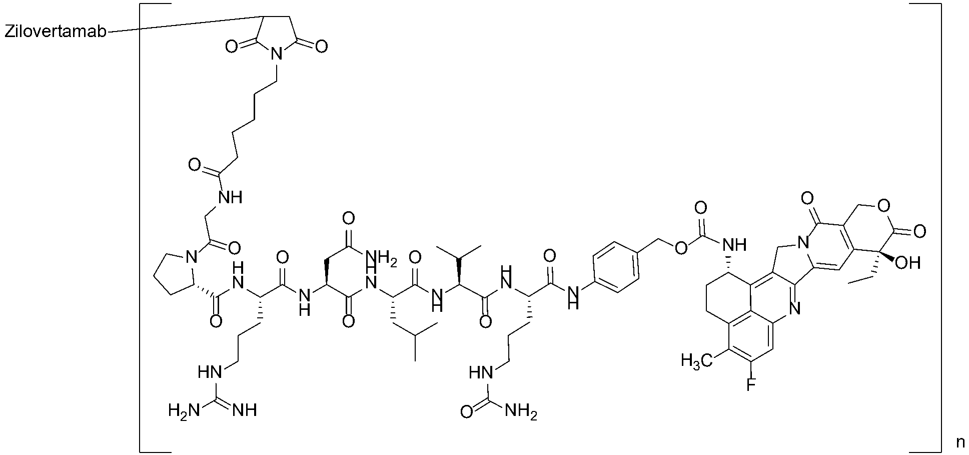

### 3.2.5 Preparation of h1849-H2L1-ADC

### 3.2.5.1 Preparation of h1849-H2L1-ADC-01 (DAR8)

The antibody h1849-H2L1 was placed in 20 mM histidine-acetate buffer (pH 5.5), and 5 mM tris(2-carboxyethyl)phosphine (TCEP) stock solution was added thereto at a TCEP/mAb ratio of 6. The mixed solution was placed at 37°C for reaction for 2 h. After 2 h, N,N-dimethylacetamide (DMA) and a solution of 15 mg/mL compound L005-P005 dissolved in DMA was added to the mixed solution. The final DMA content was 10%, and the Drug/mAb ratio was 14. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate h1849-H2L1-ADC-01 of L005-P005 and the antibody h1849-H2L1 was obtained, and the DAR value determined by the PLRP method was 7.81.

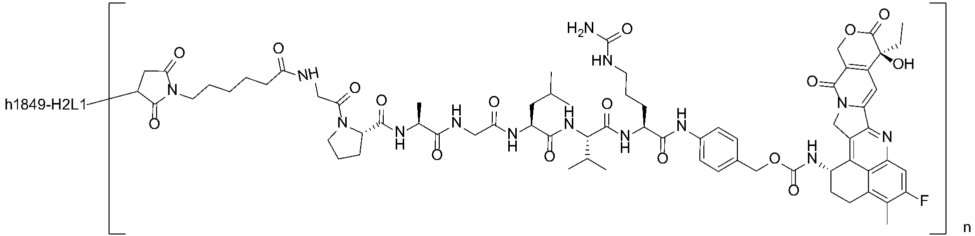

### 3.2.5.2 Preparation of h1849-H2L1-Deruxtecan (DAR8)

Compound L005-P005 in 3.2.5.1 was replaced by compound Deruxtecan (CAS No: 1599440-13-7) to obtain a conjugate h1849-H2L1-Deruxtecan of Deruxtecan and the antibody h1849-H2L1. The DAR value determined by the PLRP method was 6.15.

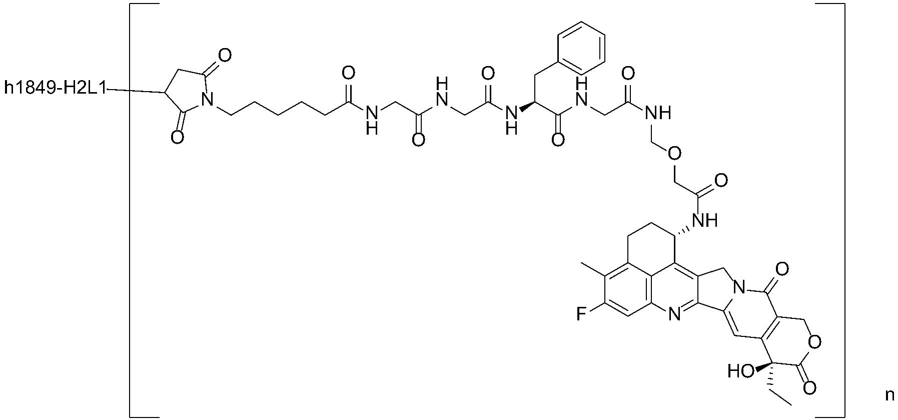

### 3.2.6 Preparation of Tisotumab-ADC

### 3.2.6.1 Preparation of Tisotumab-vcMMAE (DAR4)

The antibody Tisotumab was placed in 20 mM histidine-acetate buffer (pH 5.5), and 5 mM tris(2-carboxyethyl)phosphine (TCEP) stock solution was added thereto at a TCEP/mAb ratio of 2.5. The mixed solution was placed at 37°C for reaction for 2 h. After 2 h, N,N-dimethylacetamide (DMA) and a solution of 15 mg/mL compound vcMMAE (CAS No.: 646502-53-6) dissolved in DMA was added to the mixed solution. The final DMA content was 10%, and the Drug/mAb ratio was 7. The solution was continuously placed at 22°C for 1 h to perform the coupling reaction sufficiently. After completion of the reaction, a ZebaTM centrifugal desalting column with 40 KD MWCO was used for one-step purification, and the conjugated product was exchanged into 20 mM histidine-acetate (pH 5.5) solution for storage. The conjugate Tisotumab-vcMMAE of vcMMAE and the antibody Tisotumab was obtained, and the DAR value determined by the PLRP method was 4.00.

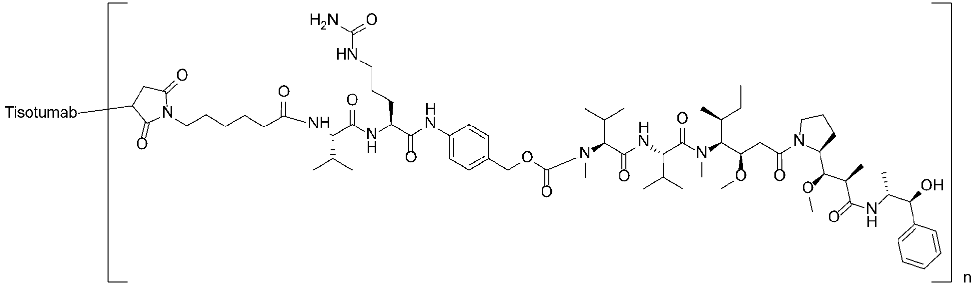

### Example 4 Enzymolysis assay of polypeptides and linker-payloads by matrix metalloproteinase MMP and cathepsin B

Matrix metalloproteinase is an extracellular enzyme capable of exerting protein degradation effect under the assistance of cofactors such as Ca ions and Zn ions. It is known currently that it is highly expressed in various tumor microenvironments. Therefore, if the linker may be efficiently degraded by this enzyme, it can be expected that ADC drugs containing the linker may achieve degradation and release at tumor sites, thereby specifically killing tumor cells through the bystander effect.

Cathepsin B is closely related to tumors and other diseases, and it is highly expressed in many tumor cells. It is a specific intracellular enzyme capable of achieving specific and efficient intracellular enzymatic cleavage. As a known linker lyase for ADC drugs, if the linker may be efficiently degraded by this enzyme, it can also be expected that the ADC drug containing the linker may achieve the desired therapeutic effect on tumors.

### 4.1 Enzymolysis assay of polypeptide enzymolysis by MMP2 and MMP9

First, MMP2 or MMP9 enzyme solution having a final concentration of 200 µg/mL was mixed with 4 mM APMA at a ratio of 1:1, placed in a 37°C water bath and incubated for 24 h to activate the MMP2 or MMP9 enzyme solution. Subsequently, the test polypeptide having a final concentration of 100 µmol was incubated with the activated MMP2 or MMP9 enzyme solution in a 37°C water bath for 4 h. After the reaction was complete, an equal volume of acetonitrile solution was added thereto, followed by RP-HPLC analysis.

### RP-HPLC detection method:

| | | | | |
|---|---|---|---|---|
| Instrument | Agilent 1260 high performance liquid chromatography | | | |
| Chromatographic column | Agilent ZORBAX SB-C18, 4.6 x 100 mm, 2.7 µm or equivalent column | | | |
| Column temperature | 30°C | | | |
| Mobile phase | Phase A: Pure water containing 0.05% trifluoroacetic acid | | | |
| | Phase B: Acetonitrile containing 0.05% trifluoroacetic acid | | | |
| Flow rate | 1 mL/min | | | |
| Sample box temperature | 10°C | | | |
| Injection volume | 20 µL | | | |
| Detection wavelength | 280 nm, 248 nm, 214 nm | | | |

| | Time (min) | A% | B% | Flow (mL/min) |
|---|---|---|---|---|
| Mobile phase gradient | 0 | 90.0 | 10.0 | 1.0 |
| | 10.00 | 50.0 | 50.0 | 1.0 |
| | 15.00 | 5.0 | 95.0 | 1.0 |
| | 18.00 | 5.0 | 95.0 | 1.0 |
| | 18.10 | 90.0 | 10.0 | 1.0 |
| | 20.00 | 90.0 | 10.0 | 1.0 |

The enzymolysis efficiency of the polypeptide was calculated based on the peak areas of the target peak before and after enzymolysis. The formula is as follows: $Enzymolysis efficiency = \frac{Peak area after enzymolysis}{Peak area before enzymolysis} \times 100 %$

The enzymolysis efficiencies of the polypeptides are shown in Tables 5 and 6. The results demonstrate that the polypeptide fragments of the present application all responded to enzymes MMP2 and MMP9 and could be enzymatically cleaved by MMP2 and MMP9 to different extents.

**Table 5 Enzymolysis results of polypeptide enzymolysis by MMP2**

| Polypeptide name | Sequence | Enzymolysis efficiency (%) |
|---|---|---|
| 1-P2 | HVLNLVCit | 14.28 |
| 1-P3 | VPLSLVCit | 49.85 |
| 1-P4 | IPVSLVCit | 21.87 |
| 1-P5 | GPLSLVCit | 52.03 |
| 1-P6 | GPASLVCit | 86.72 |
| 1-P7 | GPAGLVCit | 90.29 |
| 1-P8 | GPQGLVCit | 54.71 |
| 1-P9 | GPLGLVCit | 81.75 |
| 1-P10 | GPSGLVCit | 75.95 |
| 1-P11 | GPRGLVCit | 86.82 |
| 1-P12 | GPAALVCit | 38.94 |
| 1-P13 | GPSALVCit | 90.13 |
| 1-P14 | HVLNLGGFG | 11.78 |
| 1-P15 | VPLSLGGFG | 40.90 |
| 1-P16 | IPVSLGGFG | 13.02 |
| 1-P17 | GPLSLGGFG | 37.27 |
| 1-P18 | GPASLGGFG | 68.38 |
| 1-P19 | GPAGLGGFG | 89.82 |
| 1-P20 | GPQGLGGFG | 51.36 |
| 1-P21 | GPLGLGGFG | 90.43 |
| 1-P22 | GPSGLGGFG | 82.72 |
| 1-P23 | GPRGLGGFG | 89.54 |
| 1-P24 | GPAALGGFG | 96.89 |
| 1-P25 | GPSALGGFG | 83.12 |
| 2-P1 | PRGLVCit | 80.31 |
| 2-P2 | PRSLVCit | 53.20 |
| 2-P3 | PRNLVCit | 66.36 |
| 2-P4 | PRPLVCit | 46.14 |
| 2-P5 | PCitGLVCit | 61.03 |
| 2-P6 | PCitPLVCit | 36.21 |
| 2-P7 | GPRSLVCit | 62.64 |
| 2-P8 | GPRPLVCit | 55.07 |
| 2-P9 | GPRNLVCit | 77.00 |
| 2-P10 | GPCitGLVCit | 71.24 |
| 2-P11 | GPCitPLVCit | 40.95 |
| 2-P12 | HPRGLVCit | 77.58 |
| 2-P13 | HPRSLVCit | 57.42 |
| 2-P14 | HPRPLVCit | 54.65 |
| 2-P15 | HPRNLVCit | 63.27 |
| 2-P16 | HPCitGLVCit | 53.54 |
| 2-P17 | HPCitPLVCit | 42.87 |
| 2-P18 | SPRGLVCit | 86.03 |
| 2-P19 | SPRSLVCit | 55.76 |
| 2-P20 | SPRPLVCit | 52.52 |
| 2-P21 | SPRNLVCit | 70.46 |
| 2-P22 | SPCitGLVCit | 55.25 |
| 2-P23 | SPCitSLVCit | 33.63 |
| 2-P24 | SPCitPLVCit | 45.97 |
| 2-P25 | GPAPLVCit | 85.08 |
| 2-P26 | GPANLVCit | 100.00 |
| 2-P27 | GPRGDap(Me2)VCit | 9.00 |

**Table 6 Enzymolysis results of polypeptide enzymolysis by MMP9**

| Polypeptide name | Sequence | Enzymolysis efficiency (%) |
|---|---|---|
| 1-P2 | HVLNLVCit | ND |
| 1-P3 | VPLSLVCit | 51.44 |
| 1-P4 | IPVSLVCit | 18.84 |
| 1-P5 | GPLSLVCit | 54.80 |
| 1-P6 | GPASLVCit | 28.79 |
| 1-P7 | GPAGLVCit | 25.55 |
| 1-P8 | GPQGLVCit | 29.30 |
| 1-P9 | GPLGLVCit | 68.34 |
| 1-P10 | GPSGLVCit | 27.93 |
| 1-P11 | GPRGLVCit | 81.51 |
| 1-P12 | GPAALVCit | 20.93 |
| 1-P13 | GPSALVCit | 45.08 |
| 1-P14 | HVLNLGGFG | 4.86 |
| 1-P15 | VPLSLGGFG | 33.25 |
| 1-P16 | IPVSLGGFG | 13.30 |
| 1-P17 | GPLSLGGFG | 27.26 |
| 1-P18 | GPASLGGFG | 9.10 |
| 1-P19 | GPAGLGGFG | 29.28 |
| 1-P20 | GPQGLGGFG | 32.62 |
| 1-P21 | GPLGLGGFG | 75.60 |
| 1-P22 | GPSGLGGFG | 37.81 |
| 1-P23 | GPRGLGGFG | 84.66 |
| 1-P24 | GPAALGGFG | 37.13 |
| 1-P25 | GPSALGGFG | 38.53 |
| 2-P1 | PRGLVCit | 93.67 |
| 2-P2 | PRSLVCit | 79.34 |
| 2-P3 | PRNLVCit | 97.39 |
| 2-P4 | PRPLVCit | 61.01 |
| 2-P5 | PCitGLVCit | 68.91 |
| 2-P6 | PCitPLVCit | 36.08 |
| 2-P7 | GPRSLVCit | 77.95 |
| 2-P8 | GPRPLVCit | 62.58 |
| 2-P9 | GPRNLVCit | 100.00 |
| 2-P10 | GPCitGLVCit | 80.34 |
| 2-P11 | GPCitPLVCit | 45.82 |
| 2-P12 | HPRGLVCit | 95.73 |
| 2-P13 | HPRSLVCit | 81.06 |
| 2-P14 | HPRPLVCit | 58.33 |
| 2-P15 | HPRNLVCit | 94.69 |
| 2-P16 | HPCitGLVCit | 64.81 |
| 2-P17 | HPCitPLVCit | 48.98 |
| 2-P18 | SPRGLVCit | 93.84 |
| 2-P19 | SPRSLVCit | 79.26 |
| 2-P20 | SPRPLVCit | 57.87 |
| 2-P21 | SPRNLVCit | 95.84 |
| 2-P22 | SPCitGLVCit | 73.74 |
| 2-P23 | SPCitSLVCit | 59.39 |
| 2-P24 | SPCitPLVCit | 38.72 |
| 2-P25 | GPAPLVCit | 45.37 |
| 2-P26 | GPANLVCit | 84.35 |
| 2-P27 | GPRGDap(Me2)VCit | 10.53 |

### 4.2 Enzymolysis assay of the linker-payload enzymolysis by MMP9

MMP9 having a final concentration of 200 µg/mL was mixed with 4 mM APMA at a ratio of 1:1 and placed in a 37°C water bath and incubated for 24 h to activate the MMP9 enzyme solution. Subsequently, the linker-payload having a final concentration of 100 µmol was incubated with the activated MMP9 solution having a final concentration of 0.2 µg/mL in a 37°C water bath for 24 h. After the reaction was complete, LC-MS analysis was performed.

LC-MS results (Table 7) show that all the linker-payloads could be enzymatically cleaved by MMP9 under these conditions, thereby releasing the active molecules LVCit-PAB-MMAE or LVCit-PAB-DX8951.

**Table 7 Enzymolysis results of the linker-payload enzymolysis by MMP9**

| Sample | MMP9 enzymolys is efficiency (%) | Measured MS molecular weight of the linker-payload before enzymolysis | Structure of the linker-payload before enzymolysis | Measured MS molecular weight of the linker-payload after enzymolysis | Structure of the linker-payload after enzymolysis |
|---|---|---|---|---|---|
| L013-P005 | 15.18 | 1514.7624 | MC-PRNLVCit-PAB-DX8951 | 954.4862 | LVCit-PAB-DX8951 |
| L015-P005 | 100 | 1571.8171 | MC-GPRNLVCit-PAB-DX8951 | 954.4879 | LVCit-PAB-DX8951 |
| L015-P002 | 100 | 1854.1312 | MC-GPRNLVCit-PAB-MMAE | 1236.8258 | LVCit-PAB-MMAE |
| L017-P005 | 100 | 1601.7969 | MC-SPRNLVCit-PAB-DX8951 | 954.4819 | LVCit-PAB-DX8951 |
| L027-P005 | 100 | 1457.6980 | MC-PRGLVCit-PAB-DX8951 | 954.4497 | LVCit-PAB-DX8951 |
| L028-P005 | 56.86 | 1497.7393 | MC-PRPLVCit-PAB-DX8951 | 954.4548 | LVCit-PAB-DX8951 |
| L029-P005 | 20.57 | 1544.7489 | MC-GPRSLVCit-PAB-DX8951 | 954.4577 | LVCit-PAB-DX8951 |
| L030-P005 | 53.47 | 1554.7775 | MC-GPRPLVCit-PAB-DX8951 | 954.4623 | LVCit-PAB-DX8951 |
| L031-P005 | 30.06 | 1555.7500 | MC-GPCitPLVCit-PAB-DX8951 | 954.4609 | LVCit-PAB-DX8951 |
| L032-P005 | 96.69 | 1594.8024 | MC-HPRGLVCit-PAB-DX8951 | 954.4828 | LVCit-PAB-DX8951 |
| L033-P005 | 100 | 1635.8166 | MC-HPCitPLVCit-PAB-DX8951 | 954.4837 | LVCit-PAB-DX8951 |
| L034-P005 | 19.49 | 1545.7467 | MC-SPCitGLVCit-PAB-DX8951 | 954.4707 | LVCit-PAB-DX8951 |
| L035-P005 | 11.83 | 1469.7115 | MC-GPAPLVCit-PAB-DX8951 | 954.4684 | LVCit-PAB-DX8951 |
| L037-P005 | 20.52 | 1444.7263 | MC-PLSLVCit-PAB-DX8951 | 954.4720 | LVCit-PAB-DX8951 |
| L038-P002 | 100 | 1859.1205 | MC-SPCitSLVCit-PAB-MMAE | 1236.8321 | LVCit-PAB-MMAE |
| L038-P005 | 18 | 1574.7838 | MC-SPCitSLVCit-PAB-DX8951 | 954.4731 | LVCit-PAB-DX8951 |
| L049-P005 | 28.73 | 1487.7525 | MC-PRSLVCit-PAB-DX8951 | 954.4734 | LVCit-PAB-DX8951 |
| L050-P005 | 18.17 | 1574.7869 | MC-SPRSLVCit-PAB-DX8951 | 954.4747 | LVCit-PAB-DX8951 |
| L051-P005 | 32.86 | 1488.7337 | MC-PCitSLVCit-PAB-DX8951 | 954.4765 | LVCit-PAB-DX8951 |
| L052-P005 | 43.26 | 1498.7554 | MC-PCitPLVCit-PAB-DX8951 | 954.4765 | LVCit-PAB-DX8951 |
| L053-P005 | 27.33 | 1458.7235 | MC-PCitGLVCit-PAB-DX8951 | 954.4761 | LVCit-PAB-DX8951 |
| L054-P005 | 75.98 | 1515.7465 | MC-GPCitGLVCit-PAB-DX8951 | 954.4767 | LVCit-PAB-DX8951 |
| L055-P005 | 51.71 | 1544.7780 | MC-SPRGLVCit-PAB-DX8951 | 954.4768 | LVCit-PAB-DX8951 |
| L056-P005 | 74.36 | 1584.8110 | MC-SPRPLVCit-PAB-DX8951 | 954.4771 | LVCit-PAB-DX8951 |
| L057-P005 | 45.71 | 1585.7904 | MC-SPCitPLVCit-PAB-DX8951 | 954.4770 | LVCit-PAB-DX8951 |
| L058-P005 | 43.85 | 1486.7216 | MC-GPANLVCit-PAB-DX8951 | 954.4771 | LVCit-PAB-DX8951 |
| L059-P005 | 22.29 | 1572.7715 | MC-GPCitNLVCit-PAB-DX8951 | 954.4778 | LVCit-PAB-DX8951 |
| L060-P005 | 84.22 | 1576.7688 | MC-SPCitSDap(Me2)V Cit-PAB-DX8951 | 955.4768 | Dap(Me2)VCit-PAB-DX8951 |
| L060-P002 | 100 | 1860.1213 | MC-SPCitSDap(Me2)V Cit-PAB-MMAE | 1237.8256 | Dap(Me2)VCit-PAB-MMAE |
| L062-P005 | 100 | 1488.7520 | MC-PRSDap(Me2)VCit -PAB-DX8951 | 955.4742 | Dap(Me2)VCit-PAB-DX8951 |
| L062-P002 | 100 | 1772.1054 | MC-PRSDap(Me2)VCit -PAB-MMAE | 1237.8285 | Dap(Me2)VCit-PAB-MMAE |
| L064-P005 | 52.70 | 1515.7050 | MC-PCitNLVCit-PAB-DX8951 | 954.4639 | LVCit-PAB-DX8951 |
| L065-P005 | 27.13 | 1402.7638 | MC-PASLVCit-PAB-DX8951 | 954.4958 | LVCit-PAB-DX8951 |

### Example 4.3 Enzymolysis assay of the linker-payload enzymolysis by MMP2

MMP2 having a final concentration of 200 µg/mL was mixed with 4 mM APMA at a ratio of 1:1 and placed in a 37°C water bath and incubated for 24 h to activate the MMP2 enzyme solution. Subsequently, the linker-payload having a final concentration of 100 µmol was incubated with the activated MMP2 solution having a final concentration of 1 µg/mL in a 37°C water bath for 24 h. After the reaction was complete, LC-MS analysis was performed.

LC-MS results (Table 8) show that all the linker-payloads could be enzymatically cleaved by MMP2 under these conditions, thereby releasing the active molecules LVCit-PAB-MMAE or LVCit-PAB-DX8951.

**Table 8 Enzymolysis results of the linker-payload enzymolysis by MMP2**

| Sample | MMP2 enzymoly sis efficiency (%) | Measured MS molecular weight of the linker-payload before enzymolysis | Structure of the linker-payload before enzymolysis | Measured MS molecular weight of the linker-payload after enzymolysis | Structure of the linker-payload after enzymolysis |
|---|---|---|---|---|---|
| L013-P005 | 29.69 | 1514.7624 | MC-PRNLVCit-PAB-DX8951 | 954.4785 | LVCit-PAB-DX8951 |
| L015-P005 | 100 | 1571.8171 | MC-GPRNLVCit-PAB-DX8951 | 954.4487 | LVCit-PAB-DX8951 |
| L017-P005 | 100 | 1601.7969 | MC-SPRNLVCit-PAB-DX8951 | 954.4799 | LVCit-PAB-DX8951 |
| L027-P005 | 100 | 1457.6980 | MC-PRGLVCit-PAB-DX8951 | 954.4532 | LVCit-PAB-DX8951 |
| L028-P005 | 67.02 | 1497.7393 | MC-PRPLVCit-PAB-DX8951 | 954.4587 | LVCit-PAB-DX8951 |
| L029-P005 | 47.84 | 1544.7489 | MC-GPRSLVCit-PAB-DX8951 | 954.4647 | LVCit-PAB-DX8951 |
| L030-P005 | 42.52 | 1554.7775 | MC-GPRPLVCit-PAB-DX8951 | 954.4620 | LVCit-PAB-DX8951 |
| L031-P005 | 26.49 | 1555.7500 | MC-GPCitPLVCit-PAB-DX8951 | 954.4621 | LVCit-PAB-DX8951 |
| L032-P005 | 100 | 1594.8024 | MC-HPRGLVCit-PAB-DX8951 | 954.4796 | LVCit-PAB-DX8951 |
| L033-P005 | 100 | 1635.8166 | MC-HPCitPLVCit-PAB-DX8951 | 954.4730 | LVCit-PAB-DX8951 |
| L034-P005 | 51.38 | 1545.7467 | MC-SPCitGLVCit-PAB-DX8951 | 954.4673 | LVCit-PAB-DX8951 |
| L035-P005 | 29.56 | 1469.7115 | MC-GPAPLVCit-PAB-DX8951 | 954.4699 | LVCit-PAB-DX8951 |
| L037-P005 | 16.61 | 1444.7263 | MC-PLSLVCit-PAB-DX8951 | 954.4745 | LVCit-PAB-DX8951 |
| L038-P002 | 100 | 1859.1205 | MC-SPCitSLVCit-PAB-MMAE | 1236.8333 | LVCit-PAB-MMAE |
| L03 8-P005 | 44.79 | 1574.7838 | MC-SPCitSLVCit-PAB-DX8951 | 954.4770 | LVCit-PAB-DX8951 |
| L049-P005 | 55.10 | 1487.7525 | MC-PRSLVCit-PAB-DX8951 | 954.4782 | LVCit-PAB-DX8951 |
| L050-P005 | 63.59 | 1574.7869 | MC-SPRSLVCit-PAB-DX8951 | 954.4785 | LVCit-PAB-DX8951 |
| L051-P005 | 47.84 | 1488.7337 | MC-PCitSLVCit-PAB-DX8951 | 954.4786 | LVCit-PAB-DX8951 |
| L052-P005 | 55.57 | 1498.7554 | MC-PCitPLVCit-PAB-DX8951 | 954.4791 | LVCit-PAB-DX8951 |
| L053-P005 | 34.88 | 1458.7235 | MC-PCitGLVCit-PAB-DX8951 | 954.4791 | LVCit-PAB-DX8951 |
| L054-P005 | 95.30 | 1515.7465 | MC-GPCitGLVCit-PAB-DX8951 | 954.4794 | LVCit-PAB-DX8951 |
| L055-P005 | 89.78 | 1544.7780 | MC-SPRGLVCit-PAB-DX8951 | 954.4794 | LVCit-PAB-DX8951 |
| L056-P005 | 86.52 | 1584.8110 | MC-SPRPLVCit-PAB-DX8951 | 954.4794 | LVCit-PAB-DX8951 |
| L057-P005 | 65.18 | 1585.7904 | MC-SPCitPLVCit-PAB-DX8951 | 954.4784 | LVCit-PAB-DX8951 |
| L058-P005 | 67.97 | 1486.7216 | MC-GPANLVCit-PAB-DX8951 | 954.4797 | LVCit-PAB-DX8951 |
| L059-P005 | 25.88 | 1572.7715 | MC-GPCitNLVCit-PAB-DX8951 | 954.4793 | LVCit-PAB-DX8951 |
| L060-P005 | 71.38 | 1576.7688 | MC-SPCitSDap(Me2) VCit-PAB-DX8951 | 955.4743 | Dap(Me2)VCit -PAB-DX8951 |
| L060-P002 | 100 | 1860.1213 | MC-SPCitSDap(Me2) VCit-PAB-MMAE | 1237.8249 | Dap(Me2)VCit -PAB-MMAE |
| L062-P005 | 100 | 1488.7520 | MC-PRSDap(Me2)V Cit-PAB-DX8951 | 955.4750 | Dap(Me2)VCit -PAB-DX8951 |
| L062-P002 | 100 | 1772.1054 | MC-PRSDap(Me2)V Cit-PAB-MMAE | 1237.8273 | Dap(Me2)VCit -PAB-MMAE |
| L064-P005 | 42.89 | 1515.7050 | MC-PCitNLVCit-PAB-DX8951 | 954.4665 | LVCit-PAB-DX8951 |
| L065-P005 | 32.73 | 1402.7638 | MC-PASLVCit-PAB-DX8951 | 954.5214 | LVCit-PAB-DX8951 |

### 4.4 Enzymolysis assay of the linker-payload enzymolysis by Cathepsin B

A Cathepsin B solution having a final concentration of 10 µg/mL was first placed in a 25 mM acetic acid-sodium acetate solution (pH 5.5) containing 15 mM EDTA and 30 mM DTT at room temperature for 15 min, to activate Cathepsin B. Subsequently, the linker-payload having a final concentration of 25 µg/mL and the activated Cathepsin B enzyme solution having a final concentration of 2 µg/mL were mixed in a 25 mM acetic acid-sodium acetate + 1 mM EDTA (pH 6.0) solution, placed at 37°C for reaction at 220 rpm for 4 h. RP-HPLC analysis was performed after completion of the reaction.

RP-HPLC results show that the above linker-payloads could be completely enzymatically cleaved by Cathepsin B under these conditions, thereby releasing the active molecules.

**Table 9 Enzymolysis results of the linker-payload enzymolysis by Cathepsin B**

| Sample | Cathepsin B enzymolysis efficiency % |
|---|---|
| L013-P005 | 100 |
| L015-P005 | 100 |
| L016-P005 | 100 |
| L017-P005 | 100 |
| L027-P005 | 100 |
| L028-P005 | 100 |
| L029-P005 | 100 |
| L030-P005 | 100 |
| L031-P005 | 100 |
| L032-P005 | 100 |
| L033-P005 | 100 |
| L034-P005 | 100 |
| L035-P005 | 100 |
| L037-P005 | 100 |
| L038-P002 | 100 |
| L038-P005 | 100 |
| L049-P005 | 100 |
| L050-P005 | 100 |
| L051-P005 | 100 |
| L052-P005 | 100 |
| L053-P005 | 100 |
| L054-P005 | 100 |
| L055-P005 | 100 |
| L056-P005 | 100 |
| L057-P005 | 100 |
| L058-P005 | 100 |
| L059-P005 | 100 |
| L060-P005 | 100 |
| L060-P002 | 100 |
| L062-P005 | 100 |
| L062-P002 | 100 |

### Example 5 Enzymolysis assay of ADC by matrix metalloproteinase MMP and Cathepsin B

### 5.1 Enzymolysis assay of the anti-Her2-ADC by MMP

MMP9 having a final concentration of 100 µg/mL was mixed with 4 mM APMA at a ratio of 1:1 and incubated at 37°C for 24 h to activate MMP9. Subsequently, the anti-Her2-ADC having a final concentration of 2 mg/mL and the activated MMP9 solution having a final concentration of 5 µg/mL were incubated at 37°C for 4 h. After the reaction was complete, guanidine hydrochloride was added to terminate the reaction, and RP-HPLC analysis was performed to detect the DAR value. Enzymolysis efficiency % = (DAR value before enzymolysis - DAR value after enzymolysis)/DAR value before enzymolysis × 100%.

RP-HPLC results show that the anti-Her2-ADC in the table below could be completely enzymatically cleaved by Cathepsin B under these conditions, thereby releasing the active molecules.

**Table 10 Enzymolysis results of the anti-Her2-ADC enzymolysis by MMP9**

| **Sample name** | **Enzymolysis efficiency (%)** |
|---|---|
| anti-Her2-ADC-01 | 7.8 |
| anti-Her2-ADC-02 | 98.6 |
| anti-Her2-ADC-03 | 5.6 |
| anti-Her2-ADC-04 | 70.3 |
| anti-Her2-ADC-05 | 6.9 |
| anti-Her2-ADC-06 | 100.0 |
| anti-Her2-ADC-07 | 100.0 |

### 5.2 Enzymolysis assay of the anti-TF-ADC by cathepsin B

First, Cathepsin B enzyme solution having a final concentration of 10 µg/mL was placed in a 25 mM acetic acid-sodium acetate (pH 5.5) solution containing 15 mM EDTA and 30 mM DTT at room temperature for 15 min to activate Cathepsin B. Subsequently, h1849-H3L3-ADC-09, h1849-H3L3-ADC-10, h1849-H3L3-ADC-11, h1849-H3L3-ADC-12, or h1849-H3L3-ADC-13 having a final concentration of 90 µg/mL was mixed with the activated Cathepsin B enzyme solution having a final concentration of 2 µg/mL in a 25 mM acetic acid-sodium acetate + 1 mM EDTA (pH 6.0) solution, placed at 40°C, 220 rpm for 18 h. After the reaction was complete, LC-MS analysis was performed. Enzymolysis efficiency % = (DAR value before enzymolysis - DAR value after enzymolysis)/DAR value before enzymolysis × 100%

LC-MS results show that the above ADCs could be completely enzymatically cleaved by Cathepsin B under these conditions, thereby releasing the active molecule. In addition, Cathepsin B could recognize not only the known -V-Cit- site, but also -RG-, -GL-, -RN-, -NL- and other sites in the polypeptide linker of the present application.

**Table 11 Enzymolysis results of the anti-TF-ADC enzymolysis by Cathepsin B**

| **Sample** | **Enzymolysis efficiency (%)** | **MS molecular weight of the ADC light chain before enzymolysis** | **ADC light chain structure before enzymolysis** | **MS molecular weight of the ADC light chain after enzymolysis** | **ADC light chain structure after enzymolysis** |
|---|---|---|---|---|---|
| h1849-H3L3-ADC-09 | 100 | M+1797.11 | LC-GPRGLVCit-PAB-MMAE | M+521.61 | LC-GPR |
| | | | | M+578.61 | LC-GPRG |
| h1849-H3L3-ADC-10 | 100 | M+1796.92 | LC-MC-PRNLVCit-PAB-MMAE | M+464.58 | LC-MC-PR |
| h1849-H3L3-ADC-11 | 100 | M+1854.01 | LC-MC-GPRNLVCit-PAB-MMAE | M+521.61 | LC-MC-GPR |
| | | | | M+636.23 | LC-MC-GPRN |
| h1849-H3L3-ADC-12 | 100 | M+1855.08 | LC-GPRNDap(Me2)VCit-PAB-MMAE | M+521.63 | LC-GPR |
| | | | | M+749.91 | LC-GPRGDap(Me2) |
| h1849-H3L3-ADC-13 | 100 | M+1884.02 | LC-MC-SPRNLVCit-PAB-MMAE | M+551.68 | LC-MC-SPR |
| | | | | M+665.68 | LC-MC-SPRN |

| | | | | | |
|---|---|---|---|---|---|
| Note: M is the abbreviation for the molecular weight of h1849-H3L3 light chain, with a molecular weight of 23271.7. The enzymatically cleaved heavy chains HC-1, HC-2, and HC-3 were in the same situation as the light chain, with corresponding molecular weights after enzymolysis. | | | | | |

### Example 6 Hydrophilicity and stability tests of ADCs

### 6.1 Hydrophilicity of the anti-Her2-ADC, anti-TF-ADC, and anti-GC33-ADC determined by HIC-HPLC method

Referring to the HIC method in 3.1.1, the hydrophilicity conditions of the anti-Her2-ADC-01, anti-Her2-ADC-03, anti-Her2-ADC-04, anti-Her2-ADC-05, and the control ADC anti-Her2-Deruxtecan were analyzed.

The results are shown in Table 12. According to the hydrophobic separation principle of the hydrophobic chromatography column, the retention times of the anti-Her2-ADCs in the analysis spectrum of the hydrophobic chromatography column were all shorter than those of the control ADC, especially anti-Her2-ADC-04. It indicates that the hydrophilicities of ADCs were stronger than that of the anti-Her2-Deruxtecan (control ADC).

**Table 12 Retention times of the anti-Her2-ADCs in HIC-HPLC analysis**

| **ADC** | **HIC-HPLC analysis Retention time (min)** |
|---|---|
| anti-Her2-ADC-01 | 6.89 |
| anti-Her2-ADC-03 | 6.85 |
| anti-Her2-ADC-04 | 6.78 |
| anti-Her2-ADC-05 | 6.86 |
| anti-Her2-Deruxtecan | 7.34 |

Tables 13 and 14 show that as analyzed according to the above method, under the same analysis conditions, after L009-P005, L013-P005, L015-P005, L016-P005, and L017-P005 being linked to the anti-TF antibody and the GC33 antibody, the hydrophilicities of ADCs thereof were better than those of h1849-H3L3-Deruxtecan (control ADC) and GC33-ADC-16 (control ADC: GC33-Deruxtecan).

**Table 13 Retention times of the anti-TF-ADCs in HIC-HPLC analysis**

| **ADC** | **HIC-HPLC analysis Retention time (min)** |
|---|---|
| h1849-H3L3-ADC-03 | 9.689 |
| h1849-H3L3-ADC-04 | 9.500 |
| h1849-H3L3-ADC-05 | 9.454 |
| h1849-H3L3-ADC-06 | 8.599 |
| h1849-H3L3-ADC-07 | 9.484 |
| h1849-H3L3-Deruxtecan | 9.851 |

**Table 14 Retention times of the GC33-ADCs in HIC-HPLC analysis**

| ADC | **HIC-HPLC analysis Retention time (min)** |
|---|---|
| GC33-ADC-02 | 9.581 |
| GC33-ADC-03 | 9.472 |
| GC33-ADC-04 | 9.516 |
| GC33-ADC-05 | 8.660 |
| GC33-ADC-06 | 9.515 |
| GC33-Deruxtecan | 9.856 |

### 6.2 Stability test of ADCs in culture media

### 6.2.1 Stability test of the anti-TF-ADC in culture media

h1849-H3L3-ADC-01, h1849-H3L3-ADC-04, h1849-H3L3-ADC-05, h1849-H3L3-ADC-07, and h1849-H3L3-Deruxtecan (control ADC) were added to DMEM medium at a final concentration of 0.3 mg/mL, respectively, and placed at 37°C for incubation. The day of starting incubation was designated as day 0, and subsequently, samples were respectively collected at 6 h, day 1, day 3, day 5, and day 7 for LC-MS DAR detection.

As shown in Table 15, h1849-H3L3-ADC-01, h1849-H3L3-ADC-04, h1849-H3L3-ADC-05, and h1849-H3L3-ADC-07 were all relatively stable in the medium, and the decrease degree of DAR was less than that of the control ADC.

**Table 15 MS DAR results of anti-TF-ADC stability test in culture media**

| **ADC** | **0h** | **6h** | **d1** | **d3** | **d5** | **d7** | **d0-d7 DAR difference** |
|---|---|---|---|---|---|---|---|
| h1849-H3L3-ADC-01 | 8.7 | 8.8 | 8.8 | 8.5 | 7.8 | 7.0 | 1.7 |
| h1849-H3L3-ADC-04 | 6.0 | NA | 5.8 | 5.6 | 5.1 | 4.5 | 1.5 |
| h1849-H3L3-ADC-05 | 7.3 | NA | 6.9 | 6.7 | 6.0 | 4.3 | 3.0 |
| h1849-H3L3-ADC-07 | 8.4 | NA | 8.1 | 7.6 | 7.2 | 6.8 | 1.6 |
| h1849-H3L3-Deruxtecan | 8.0 | 8.0 | 7.8 | 7.1 | 5.3 | 4.2 | 3.8 |

### 6.2.2 Stability test of GC33-ADC in culture media

GC33-ADC-01, GC33-ADC-02, GC33-ADC-03, and GC33-ADC-16 (control ADC: GC33-Deruxtecan) were added to DMEM medium at a final concentration of 0.5 mg/mL, respectively, and placed at 37°C for incubation. The day of starting incubation was designated as day 0, and subsequently, 120 µL samples were respectively collected at 6 h, day 1, day 3, day 5, and day 7 for LC-MS DAR detection.

The results are shown in Table 16. DAR test results show that after linking L005-P005, L013-P005, and L009-P005 to GC33 antibody, the ADCs thereof were also relatively stable in the culture media.

**Table 16 MS DAR results of GC33-ADC stability test in culture media**

| **ADC** | **0h** | **6h** | **d1** | **d3** | **d5** | **d7** | **d0-d5 DAR difference** |
|---|---|---|---|---|---|---|---|
| GC33-ADC-01 | 8.5 | 8.3 | 7.6 | 7.3 | 6.7 | 6.6 | 1.8 |
| GC33-ADC-02 | 8.5 | 8.2 | 7.8 | 6.7 | 6.4 | 6.4 | 2.1 |
| GC33-ADC-03 | 8.1 | 8.0 | 7.7 | 7.0 | 6.2 | NA | 1.9 |
| GC33-Deruxtecan | 8.0 | 7.5 | 7.2 | 4.5 | 3.6 | 3.6 | 4.4 |

### 6.2.3 Stability test of the anti-Her2-ADC in culture media

The anti-Her2-ADC-01, anti-Her2-ADC-02, anti-Her2-ADC-04, anti-Her2-ADC-06, anti-Her2-ADC-07, and anti-Her2-Deruxtecan (control ADC) were added to DMEM medium at a final concentration of 0.5 mg/mL, respectively, and placed at 37°C for incubation. The day of starting incubation was designated as day 0, and subsequently, 120 µL samples were respectively collected at day 1, day 3, and day 5 for LC-MS DAR detection.

The results are shown in Table 17. DAR test results show that after linking L005-P005, L006-P005, L009-P005, and L012-P005 to anti-Her2 antibody, the ADCs thereof were also relatively stable in the culture media.

**Table 17 MS DAR results of the anti-Her2-ADC stability test in culture media**

| **ADC** | **0h** | **d1** | **d3** | **d5** | **d0-d5** |
|---|---|---|---|---|---|
| | | | | | **DAR difference** |
| anti-Her2-ADC-01 | 8.41 | 8.09 | 6.98 | 5.85 | 2.56 |
| anti-Her2-ADC-02 | 7.99 | 7.2 | 5.34 | 4.63 | 3.36 |
| anti-Her2-ADC-04 | 8 | 7.89 | 7.33 | 6.91 | 1.09 |
| anti-Her2-ADC-06 | 8 | 7.16 | 4.11 | 4.56 | 3.44 |
| anti-Her2-ADC-07 | 8 | 7.45 | 4.38 | 4.64 | 3.36 |
| anti-Her2-Deruxtecan | 7.97 | 6.8 | 3.97 | 3.73 | 4.24 |

### 6.3 Method for stability assay of ADCs in rat plasma

### 6.3.1 Stability assay of GC33-ADC in rat plasma

GC33-ADC-01, GC33-ADC-02, and GC33-Deruxtecan (control ADC) were added to rat plasma at a final concentration of 0.5 mg/mL, respectively, and placed at 37°C for incubation. The day of starting incubation was designated as day 0, and samples were respectively collected at 6 h, 24 h, 72 h, 120 h, and 158 h. After being captured using a protein A affinity filler, MS DAR detection was performed.

The test results show that GC33-ADC-01 and GC33-ADC-02 were both relatively stable in rat plasma, even slightly better than the control ADC.

**Table 18 MS DAR results of the GC33-ADC stability assay in rat plasma**

| **ADC** | **0h** | **6h** | **d1** | **d3** | **d5** | **d6.5** | **d0-d6.5 DAR difference** |
|---|---|---|---|---|---|---|---|
| GC33-ADC-01 | 8.16 | 8.14 | 8.01 | 7.76 | 7.55 | 7.31 | 0.85 |
| GC33-ADC-02 | 8.21 | 8.16 | 7.99 | 7.49 | 7.43 | 6.78 | 1.43 |
| GC33-Deruxtecan | 8 | 8 | 7.8 | 7.28 | 6.86 | 6.13 | 1.87 |

### Example 7 Cell killing ability of ADCs

The inhibitory activity of the bioactive molecules against relevant tumor cells was tested *in vitro.* The sources of tumor cells are shown in Table 19.

**Table 19 Sources of tumor cells**

| Cell name | Tumor type | Source |
|---|---|---|
| JIMT-1 | Human breast cancer cell | Nanjing COBIOER |
| DU145 | Human prostate cancer cell | Nanjing COBIOER |
| Ca Ski | Human cervical epidermoid carcinoma cell | Nanjing COBIOER |
| HepG2 | Human liver cancer cell | Nanjing COBIOER |
| NCI-N87 | Human gastric cancer cell | Nanjing COBIOER |

First, tumor cells DU145, Ca Ski, HepG2, NCI-N87, and JIMT-1 were cultured. The bioactive molecules or fragments to be tested were co-cultured with tumor cells, and then added with a CTG reagent (CellTiter-Glo^{®} Luminescent Cell Viability Assay, Promega, Cat: G7573). ATP in viable cells was detected using a microplate reader (Manufacturer: Tecan, Model: Spark) (chemiluminescence), to evaluate the inhibitory effect of the bioactive molecules on cell proliferation.

### 7.1 In vitro killing assay of the anti-Her2-ADC against JIMT-1 and NCI-N87 tumor cells

### 7.1.1 In vitro killing assay of the anti-Her2-ADC against JIMT-1 tumor cells

The method for testing *in vitro* proliferation inhibition of JIMT-1 cells was taken as an example below, to exemplarily illustrate the method for testing *in vitro* proliferation inhibitory activity of the compounds of the present application against tumor cells. This method is also applicable to, but not limited to, the *in vitro* proliferation inhibition assay of other tumor cells.
1. Cell culture: JIMT-1 cells were cultured in DMEM medium supplemented with 10% FBS.
2. Cell preparation: JIMT-1 cells in the logarithmic growth phase were taken, washed once with PBS, and then digested with the addition of 2-3 mL trypsin for 2-3 min. After completion of the digestion of cells, 10-15 mL cell culture medium was added to elute the digested cells. It was centrifuged at 1500 rpm for 3 min, supernatant was discarded, and cells were resuspended with the addition of 10-20 mL cell culture medium to prepare a single cell suspension.
3. Cell plating: The JIMT-1 single cell suspension was mixed well, and the density of viable cells was adjusted to 3.75×10⁴ cells/mL with the cell culture medium. The cell suspension with the density adjusted was mixed well, and added to a 96-well cell culture plate at 80 µL/well. The culture plate was incubated in an incubator for 18 hours (37°C, 5% CO₂).
4. Preparation of bioactive molecules: The bioactive molecules to be tested were prepared in the above medium to a stock solution having an initial concentration of 10 mM. The bioactive molecule stock solution was diluted with the above medium to obtain a total of 9 concentrations, namely: 500, 100, 20, 4, 0.8, 0.16, 0.032, 0.0064, and 0.00128 nM.
5. Sample addition: 20 µL of the above prepared sample solutions to be tested at different concentrations were added to the culture plate in duplicate. The culture plate was incubated in an incubator for 5 days (37°C, 5% CO₂).
6. Color development: The 96-well cell culture plate was taken out and subjected to standing at room temperature for 10-15 min. 50 µL CTG reagent was added to each well, and the cell culture plate was shaken on a shaker under 1000 rpm for 2 min, and incubated at room temperature in the dark for 10 min.
7. Plate reading: The 96-well cell culture plate was taken out and placed in a microplate reader to measure chemiluminescence.

Result processing: Inhibition rate % = (fluorescence reading of the administration well with cells - fluorescence reading of the medium control well)/(fluorescence reading of the blank well with cells - fluorescence reading of the medium control well) × 100%. A four-parameter logistic curve was plotted with the logarithm of concentration as the abscissa and the killing rate as the ordinate.

Data analysis was performed using Microsoft Excel and GraphPad Prism 8.

Table 20 shows the IC₅₀ values and Imax values for the inhibition of *in vitro* JIMT-1 cell proliferation by the bioactive molecules of the present application.

**Table 20. Killing results of bioactive molecules against JIMT-1 cells**

| Bioactive molecule ID | JIMT-1 | |
|---|---|---|
| | IC₅₀ (nM) | Imax (%) |
| anti-Her2-Deruxtecan | 53.04 | 32.51 |
| anti-Her2-ADC-08 | 2.592 | 71.63 |
| anti-Her2-ADC-09 | 1.417 | 79.28 |
| anti-Her2-ADC-10 | 18.44 | 58.77 |
| anti-Her2-ADC-11 | 3.955 | 68.00 |

According to the results in Table 20, the bioactive molecules of the present application exhibited significant proliferation inhibitory activity against JIMT-1 cells. The bioactive molecules of the present application all may exhibit similar tumor proliferation inhibitory activity.

### 7.1.2 In vitro killing assay of the anti-Her2-ADC against NCI-N87 tumor cells

The method for testing *in vitro* proliferation inhibition of NCI-N87 cells was taken as an example below, to exemplarily illustrate the method for testing *in vitro* proliferation inhibitory activity of the compounds of the present application against tumor cells. This method is also applicable to, but not limited to, the *in vitro* proliferation inhibition assay of other tumor cells.
1. Cell culture: NCI-N87 cells were cultured in DMEM medium supplemented with 10% FBS.
2. Cell preparation: NCI-N87 cells in the logarithmic growth phase were taken, washed once with PBS, and then digested with the addition of 2-3 mL trypsin for 2-3 min. After completion of the digestion of cells, 10-15 mL cell culture medium was added to elute the digested cells. It was centrifuged at 1500 rpm for 3 min, supernatant was discarded, and cells were resuspended with the addition of 10-20 mL cell culture medium to prepare a single cell suspension.
3. Cell plating: The NCI-N87 single cell suspension was mixed well, and the density of viable cells was adjusted to 3.75×10⁴ cells/mL with the cell culture medium. The cell suspension with the density adjusted was mixed well, and added to a 96-well cell culture plate at 80 µL/well. The culture plate was incubated in an incubator for 18 hours (37°C, 5% CO₂).
4. Preparation of bioactive molecules: The bioactive molecules to be tested were prepared in the above medium to a stock solution having an initial concentration of 10 mM. The bioactive molecule stock solution was diluted with the above medium to obtain a total of 9 concentrations, namely: 1000, 200, 40, 8, 1.6, 0.32, 0.064, 0.0128, and 0.00256 nM.
5. Sample addition: 20 µL of the above prepared sample solutions to be tested at different concentrations were added to the culture plate in duplicate. The culture plate was incubated in an incubator for 5 days (37°C, 5% CO₂).
6. Color development: The 96-well cell culture plate was taken out and subjected to standing at room temperature for 10-15 min. 50 µL CTG reagent was added to each well, and the cell culture plate was shaken on a shaker under 1000 rpm for 2 min, and incubated at room temperature in the dark for 10 min.
7. Plate reading: The 96-well cell culture plate was taken out and placed in a microplate reader to measure chemiluminescence.

Result processing: Inhibition rate % = (fluorescence reading of the administration well with cells - fluorescence reading of the medium control well) / (fluorescence reading of the blank well with cells - fluorescence reading of the medium control well) × 100%. A four-parameter logistic curve was then plotted with the logarithm of concentration as the abscissa and the killing rate as the ordinate.

Data analysis was performed using Microsoft Excel and GraphPad Prism 8.

Table 21 shows the IC₅₀ values and Imax values for the inhibition of *in vitro* NCI-N87 cell proliferation by the bioactive molecules of the present application.

**Table 21. Killing results of the bioactive molecules against NCI-N87 cells**

| Bioactive molecule ID | NCI-N87 | |
|---|---|---|
| | IC₅₀ (nM) | Imax (%) |
| anti-Her2-Deruxtecan | 0.589 | 96.95 |
| anti-Her2-ADC-12 | 5.799 | 101.54 |
| anti-Her2-ADC-13 | 3.688 | 102.62 |
| anti-Her2-ADC-14 | 0.472 | 100.91 |
| anti-Her2-ADC-15 | 16.20 | 98.36 |
| anti-Her2-ADC-16 | 0.533 | 102.23 |
| anti-Her2-ADC-17 | 0.607 | 95.20 |

According to the results of Table 21, the bioactive molecules of the present application exhibited significant proliferation inhibitory activity against N87 cells. The bioactive molecules of the present application all may exhibit similar tumor proliferation inhibitory activity.

### 7.2 In vitro killing assay of the anti-TF-ADC against DU145 cells and Ca Ski tumor cells

### 7.2.1 In vitro killing assay of the anti-TF-ADC against tumor cells

The method for testing *in vitro* proliferation inhibition of DU145 cells and Ca Ski cells was taken as an example below, to exemplarily illustrate the method for testing in vitro proliferation inhibitory activity of the compounds of the present application against tumor cells. This method is also applicable to, but not limited to, the *in vitro* proliferation inhibition assay of other tumor cells.
1. Cell culture: DU145 cells and Ca Ski cells were cultured in RPMI-1640 medium supplemented with 10% FBS, respectively.
2. Cell preparation: Cells in the logarithmic growth phase were taken, washed once with PBS, and then digested with the addition of 2-3 mL trypsin for 2-3 min. After completion of the digestion of cells, 10-15 mL cell culture medium was added to elute the digested cells. It was centrifuged at 1500 rpm for 3 min, the supernatant was discarded, and then 10-20 mL cell culture medium was added to resuspend the cells to prepare a single cell suspension.
3. Cell plating: The single cell suspension of DU145 cells and Ca Ski cells was mixed well, respectively, and the density of viable cells was adjusted to 2.5 × 10⁴ cells/mL with the cell culture medium, respectively. The cell suspension with the density adjusted was mixed well, and added to a 96-well cell culture plates at 80 µL/well. The culture plate was incubated in an incubator for 18 hours (37°C, 5% CO₂).
4. Preparation of bioactive molecules: The bioactive molecules to be tested were prepared in the above medium to a stock solution having an initial concentration of 10 mM. The bioactive molecule stock solution was diluted with the above medium to obtain a total of 9 concentrations, namely: 250, 50, 10, 2, 0.4, 0.08, 0.016, 0.0032, and 0.00064 nM.
5. Sample addition: 20 µL of the above prepared sample solutions to be tested at different concentrations were added to the culture plate in duplicate. The culture plate was incubated in an incubator for 5 days (37°C, 5% CO₂).
6. Color development: The 96-well cell culture plate was taken out and subjected to standing at room temperature for 10-15 min. 50 µL CTG reagent was added to each well, and the cell culture plate was shaken on a shaker under 1000 rpm for 2 min, and incubated at room temperature in the dark for 10 min.
7. Plate reading: The 96-well cell culture plate was taken out and placed in a microplate reader to measure chemiluminescence.

Result processing: Inhibition rate % = (fluorescence reading of the administration well with cells - fluorescence reading of the medium control well)/(fluorescence reading of the blank well with cells - fluorescence reading of the medium control well) × 100%. A four-parameter logistic curve was plotted with the logarithm of concentration as the abscissa and the killing rate as the ordinate.

Data analysis was performed using Microsoft Excel and GraphPad Prism 8.

Tables 22 and 23 show the IC₅₀ values and Imax values of the bioactive molecules of the present application for *in vitro* proliferation inhibition against DU145 and Ca Ski cells.

**Table 22. Killing results of the bioactive molecules against DU145 cells**

| Bioactive molecule ID | DU145 | |
|---|---|---|
| | IC50 (nM) | Imax (%) |
| h1849-H3L3-ADC-33 | 1.312 | 102.07 |
| h1849-H3L3-ADC-34 | 1.079 | 100.86 |
| h1849-H3L3-ADC-35 | 3.019 | 99.98 |
| h1849-H3L3-ADC-36 | 1.934 | 100.74 |
| h1849-H3L3-ADC-37 | 2.267 | 87.13 |
| h1849-H3L3-ADC-38 | 11.62 | 84.13 |
| h1849-H3L3-ADC-39 | 2.195 | 77.2 |
| h1849-H3L3-ADC-40 | 0.680 | 102.94 |
| h1849-H3L3-vcMMAE | 11.34 | 83.25 |

**Table 23. Killing results of the bioactive molecules against Ca Ski cells**

| Bioactive molecule ID | Ca Ski | |
|---|---|---|
| | IC50 (nM) | Imax (%) |
| h1849-H3L3-ADC-33 | 0.063 | 88.48 |
| h1849-H3L3-ADC-34 | 0.054 | 90.62 |
| h1849-H3L3-ADC-35 | 0.048 | 88.22 |
| h1849-H3L3-ADC-36 | 0.058 | 89.84 |
| h1849-H3L3-ADC-40 | 0.025 | 87.75 |
| h1849-H3L3-vcMMAE | 0.033 | 97.50 |

According to the results of Tables 22 and 23, the bioactive molecules of the present application exhibited significant proliferation inhibitory activity against DU145 and Ca Ski cells. The bioactive molecules of the present application all can exhibit similar tumor proliferation inhibitory activity.

### 7.2.2 In vitro killing assay of the anti-TF-ADC against tumor cells

The method for testing *in vitro* proliferation inhibition of DU145 cells and Ca Ski cells was taken as an example below, to exemplarily illustrate the method for testing in vitro proliferation inhibitory activity of the compounds of the present application against tumor cells. This method is also applicable to, but not limited to, the *in vitro* proliferation inhibition assay of other tumor cells.
1. Cell culture: DU145 cells and Ca Ski cells were cultured in RPMI-1640 medium supplemented with 10% FBS, respectively.
2. Cell preparation: The cells in the logarithmic growth phase were taken, washed once with PBS, and then digested with the addition of 2-3 mL trypsin for 2-3 min. After completion of the digestion of cells, 10-15 mL cell culture medium was added to elute the digested cells. It was centrifuged at 1500 rpm for 3 min, supernatant was discarded, and cells were resuspended with the addition of 10-20 mL cell culture medium to prepare a single cell suspension.
3. Cell plating: The single cell suspension of DU145 cells and Ca Ski cells was mixed well, respectively, and the density of viable cells was adjusted to 2.5 × 10⁴ cells/mL with the cell culture medium, respectively. The cell suspension with the density adjusted was mixed well, and added to a 96-well cell culture plates at 80 µL/well. The culture plate was incubated in an incubator for 18 hours (37°C, 5% CO₂).
4. Preparation of bioactive molecules: The bioactive molecules to be tested were prepared in the above medium to a stock solution having an initial concentration of 10 mM. The bioactive molecule stock solution was diluted with the above medium to obtain a total of 9 concentrations, namely: 250, 50, 10, 2, 0.4, 0.08, 0.016, 0.0032, and 0.00064 nM.
5. Sample addition: 20 µL of the above prepared sample solutions to be tested at different concentrations were added to the culture plate in duplicate. The culture plate was incubated in an incubator for 5 days (37°C, 5% CO₂).
6. Color development: The 96-well cell culture plate was taken out and subjected to standing at room temperature for 10-15 min. 50 µL CTG reagent was added to each well, and the cell culture plate was shaken on a shaker under 1000 rpm for 2 min, and incubated at room temperature in the dark for 10 min.
7. Plate reading: The 96-well cell culture plate was taken out and placed in a microplate reader to measure chemiluminescence.

Result processing: Inhibition rate % = (fluorescence reading of the administration well with cells - fluorescence reading of the medium control well) / (fluorescence reading of the blank well with cells - fluorescence reading of the medium control well) × 100%. A four-parameter logistic curve was then plotted with the logarithm of concentration as the abscissa and the killing rate as the ordinate.

Data analysis was performed using Microsoft Excel and GraphPad Prism 8.

Tables 24 and 25 show the IC₅₀ values and Imax values of the bioactive molecules of the present application for *in vitro* proliferation inhibition against DU145 and Ca Ski cells.

**Table 24. Killing results of the bioactive molecules against DU145 cells**

| Bioactive molecule ID | DU145 | |
|---|---|---|
| | IC₅₀ (nM) | Imax (%) |
| h1849-H3L3-ADC-04 | 0.095 | 104.04 |
| h1849-H3L3-ADC-05 | 0.075 | 107.02 |
| h1849-H3L3-ADC-07 | 0.100 | 103.63 |
| h1849-H3L3-ADC-06 | 0.925 | 104.66 |
| h1849-H3L3-ADC-08 | 0.377 | 109.9 |
| h1849-H3L3-ADC-09 | 0.425 | 109.6 |
| h1849-H3L3-ADC-10 | 0.378 | 109.9 |
| h1849-H3L3-ADC-11 | 0.151 | 107.5 |
| h1849-H3L3-ADC-12 | 0.136 | 107.4 |
| h1849-H3L3-ADC-13 | 0.1521 | 95.34 |
| h1849-H3L3-ADC-14 | 2.987 | 100 |
| h1849-H3L3-ADC-15 | 0.168 | 100 |
| h1849-H3L3-ADC-16 | 0.103 | 107.7 |
| h1849-H3L3-ADC-17 | 0.6589 | 94.57 |
| h1849-H3L3-ADC-18 | 0.1808 | 95.84 |
| h1849-H3L3-ADC-19 | 0.3032 | 95.95 |
| h1849-H3L3-ADC-20 | 0.0610 | 96.06 |
| h1849-H3L3-ADC-21 | 0.5175 | 96.23 |
| h1849-H3L3-ADC-22 | 0.1096 | 96.76 |
| h1849-H3L3-ADC-23 | 0.5698 | 100 |
| h1849-H3L3-ADC-24 | 0.3575 | 99.87 |
| h1849-H3L3-ADC-25 | 0.1879 | 100 |
| h1849-H3L3-ADC-26 | 0.1474 | 92.97 |
| h1849-H3L3-ADC-27 | 12.83 | 90.93 |
| h1849-H3L3-ADC-28 | 2.277 | 92.98 |
| h1849-H3L3-ADC-29 | 0.237 | 100 |
| h1849-H3L3-ADC-30 | 0.4424 | 100 |
| h1849-H3L3-Deruxtecan | 1.323 | 24.27 |

**Table 25. Killing results of the bioactive molecules against Ca Ski cells**

| Bioactive molecule ID | Ca Ski | |
|---|---|---|
| | IC₅₀ (nM) | Imax (%) |
| h1849-H3L3-ADC-04 | 0.087 | 105.98 |
| h1849-H3L3-ADC-05 | 0.072 | 106.59 |
| h1849-H3L3-ADC-07 | 0.082 | 105.86 |
| h1849-H3L3-ADC-06 | 0.203 | 100.74 |
| h1849-H3L3-ADC-08 | 0.396 | 106.7 |
| h1849-H3L3-ADC-09 | 0.405 | 104.9 |
| h1849-H3L3-ADC-10 | 0.420 | 104.8 |
| h1849-H3L3-ADC-11 | 0.190 | 107.5 |
| h1849-H3L3-ADC-12 | 0.188 | 107.6 |
| h1849-H3L3-ADC-14 | 0.375 | 99.9 |
| h1849-H3L3-ADC-15 | 0.099 | 100 |
| h1849-H3L3-ADC-16 | 0.171 | 107.9 |
| h1849-H3L3-Deruxtecan | 0.092 | 81.62 |

According to the results of Tables 24 and 25, the bioactive molecules of the present application exhibited significant proliferation inhibitory activity against DU145 and Ca Ski cells. The bioactive molecules of the present application all can exhibit similar tumor proliferation inhibitory activity.

### 7.3 In vitro killing assay of the anti-GC33-ADC against HepG2 tumor cells

The method for testing *in vitro* proliferation inhibition of human liver cancer HepG2 cells was taken as an example below, to exemplarily illustrate the method for testing *in vitro* proliferation inhibitory activity of the compounds of the present application against tumor cells. This method is also applicable to, but not limited to, the *in vitro* proliferation inhibition assay of other tumor cells.
1. Cell culture: HepG2 cells were cultured in MEM medium supplemented with 10% FBS.
2. Cell preparation: The cells in the logarithmic growth phase were taken, washed once with PBS, and then digested with the addition of 2-3 mL trypsin for 2-3 min. After completion of the digestion of cells, 10-15 mL cell culture medium was added to elute the digested cells. It was centrifuged at 1500 rpm for 3 min, supernatant was discarded, and cells were resuspended with the addition of 10-20 mL cell culture medium to prepare a single cell suspension.
3. Cell plating: The single cell suspension of HepG2 cells was mixed well, and the density of viable cells was adjusted to 2.5 × 10⁴ cells/mL with the cell culture medium, respectively. The cell suspension with the density adjusted was mixed well, and added to a 96-well cell culture plates at 80 µL/well. The culture plate was incubated in an incubator for 18 hours (37°C, 5% CO₂).
4. Preparation of bioactive molecules: The bioactive molecules to be tested were prepared in the above medium to a stock solution having an initial concentration of 10 mM. The bioactive molecule stock solution was diluted with the above medium to obtain a total of 9 concentrations, namely: 500, 100, 20, 4, 0.8, 0.16, 0.032, 0.0064, and 0.00128 nM.
5. Sample addition: 20 µL of the above prepared sample solutions to be tested at different concentrations were added to the culture plate in duplicate. The culture plate was incubated in an incubator for 5 days (37°C, 5% CO₂).
6. Color development: The 96-well cell culture plate was taken out of the incubator and subjected to standing at room temperature for 10-15 min. 50 µL CTG reagent was added to each well, and the cell culture plate was shaken on a shaker under 1000 rpm for 2 min, and incubated at room temperature in the dark for 10 min.
7. Plate reading: The 96-well cell culture plate was taken out and placed in a microplate reader to measure chemiluminescence.

Result processing: Inhibition rate % = (fluorescence reading of the administration well with cells - fluorescence reading of the medium control well) / (fluorescence reading of the blank well with cells - fluorescence reading of the medium control well) × 100%. A four-parameter logistic curve was then plotted with the logarithm of concentration as the abscissa and the killing rate as the ordinate.

Data analysis was performed using Microsoft Excel and GraphPad Prism 8.

Table 26 shows the IC₅₀ values and Imax values for the inhibition of *in vitro* HepG2 cell proliferation by the bioactive molecules of the present application.

**Table 26. Killing results of the bioactive molecules against target cells**

| Bioactive molecule ID | HepG2 | |
|---|---|---|
| | IC₅₀ (nM) | Imax (%) |
| GC33-ADC-03 | 0.186 | 101.50 |
| GC33-ADC-04 | 0.188 | 100.70 |
| GC33-ADC-06 | 0.169 | 100.32 |
| GC33-ADC-02 | 0.207 | 99.74 |
| GC33-Deruxtecan | 0.218 | 74.99 |

According to the results of Table 26, the bioactive molecules of the present application exhibited significant proliferation inhibitory activity against HepG2 cells. The bioactive molecules of the present application all can exhibit similar tumor proliferation inhibitory activity.

### Example 8 In vivo antitumor activity of ADCs

### 8.1 Test of the efficacy of h1849-H2L1-ADC-01 on a DU145 human prostate cancer xenograft

### Experimental materials

Test compounds: h1849-H2L1-ADC-01, h1849-H2L1-Deruxtecan (control ADC)
Experimental cells: DU145 cells
Experimental animals: BALB/c nude mice, 6-8 weeks old, body weight: 18-22 g, provided by Beijing Charles River Laboratory Animal Technology Co., Ltd. or other qualified suppliers.

### Experimental scheme

**Cell culture:** human prostate cancer DU145 cells (ATCC, Cat. No.: HTB-81) were subjected to *in vitro* adherent culture under culture conditions referring to the technical instruction from the supplier. Cells were passaged by routine digestion with trypsin-EDTA twice a week. When the cell confluence reached 80%-90% and the number met the requirement, cells were harvested, counted, and inoculated.

**Tumor inoculation:** 0.2 mL (10 × 10⁶) DU145 cells (added with matrigel at a volume ratio of 1:1) were subcutaneously inoculated into the right dorsal flank of each mouse. When the average tumor volume reached about 200 mm³, the mice were grouped and administered.

### Animal experimental grouping and administration scheme:

| **Group** | **N¹** | **Compound treatment** | **Dose (mg/kg)** | **Administration volume parameter (µL/g)²** | **Administration route** | **Administration frequency** |
|---|---|---|---|---|---|---|
| 1 | 6 | Vehicle | 3 | 10 | IV | QW*2 |
| 2 | 6 | h1849-H2L1-ADC-01 | 3 | 10 | IV | QW*2 |
| 3 | 6 | h1849-H2L1-Deruxtecan | 3 | 10 | IV | QW*2 |

| | | | | | | |
|---|---|---|---|---|---|---|
| Note: N: number of mice per group; administration volume: 10µL/g based on the mouse body weight. | | | | | | |

**Experimental indexes:** The experimental indexes are used to study whether tumor growth is inhibited, delayed, or cured. Tumor diameter and body weight were measured twice weekly using a vernier caliper. The formula for calculating tumor volume is: V = 0.5a × b², wherein a and b represent the long and short diameters of the tumor, respectively.

Data statistics was performed using Excel statistical software: the mean values were calculated by avg, SD values were calculated by STDEV, SEM values were calculated by ATDEV/SQRT, and T-test was used for comparison between two groups. One-way ANOVA was used for comparison among three or more groups. If the value F is significantly different, multiple comparisons should be performed after ANOVA analysis. p < 0.05 was considered statistically significant.

### Experimental results

The results are shown in FIG. 2. Although both h1849-H2L1-ADC-01 and h1849-H2L1-Deruxtecan are Dxd-ADCs and exhibited the inhibitory effect on DU145 tumor growth, the inhibitory effect of h1849-H2L1-ADC-01 of the present application was superior to that of the control ADC. This demonstrates that the linker used in the present application exhibits certain advantages.

### 8.2 Test of the efficacy of h1849-H3L3-ADC-01 on a DU145 xenograft

### Experimental materials

Test compounds: h1849-H3L3-ADC-01, h1849-H3L3-vcMMAE (control ADC)
Experimental cells: DU145 cells
Experimental animals: BALB/c nude mice, 6-8 weeks old, body weight: 18-22 g, provided by Beijing Charles River Laboratory Animal Technology Co., Ltd. or other qualified suppliers.

### Experimental scheme

**Cell culture:** human prostate cancer DU145 cells (ATCC, Cat. No.: HTB-81) were subjected to *in vitro* adherent culture under culture conditions referring to the technical instruction from the supplier. Cells were passaged by routine digestion with trypsin-EDTA twice a week. When the cell confluence reached 80%-90% and the number met the requirement, cells were harvested, counted, and inoculated.

**Tumor inoculation:** 0.2 mL (10 × 10⁶) DU145 cells (added with matrigel at a volume ratio of 1:1) were subcutaneously inoculated into the right dorsal flank of each mouse. When the average tumor volume reached about 200 mm³, the mice were grouped and administered.

### Animal experimental grouping and administration scheme:

| **Grou p** | **N 1** | **Compound treatment** | **Dose (mg/kg)** | **Administration volume parameter (µL/g)²** | **Administratio n route** | **Administration frequency** |
|---|---|---|---|---|---|---|
| 1 | 6 | Vehicle | 3 | 10 | IV | QW*3 |
| 2 | 6 | h1849-H3L3-ADC-01 | 3 | 10 | IV | QW*3 |
| 3 | 6 | h1849-H3L3-vcMMAE | 3 | 10 | IV | QW*3 |

| | | | | | | |
|---|---|---|---|---|---|---|
| Note: N: number of mice per group; administration volume: 10µL/g based on the mouse body weight. | | | | | | |

**Experimental indexes:** The experimental indexes are used to study whether tumor growth is inhibited, delayed, or cured. Tumor diameter and body weight were measured twice weekly using a vernier caliper. The formula for calculating tumor volume is: V = 0.5a × b², wherein a and b represent the long and short diameters of the tumor, respectively.

Data statistics was performed using Excel statistical software: the mean values were calculated by avg, SD values were calculated by STDEV, SEM values were calculated by ATDEV/SQRT, and T-test was used for comparison between two groups. One-way ANOVA was used for comparison among three or more groups. If the value F is significantly different, multiple comparisons should be performed after ANOVA analysis. p < 0.05 was considered statistically significant.

### Experimental results

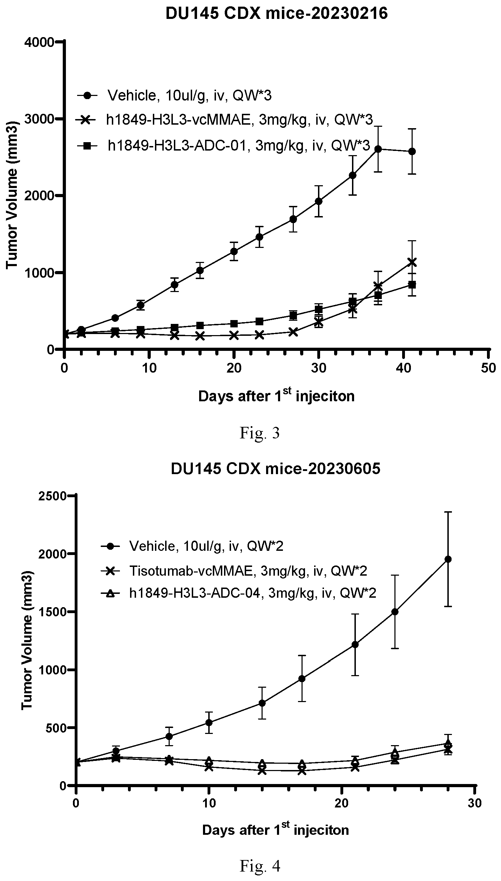

The results are shown in FIG. 3. The experimental results indicate that, as compared to the tumor inhibitory effect of the control ADC (h1849-H3L3-vcMMAE), h1849-H3L3-ADC-01 (anti-TF antibody conjugated with Dx8951) at the same dose maintained comparable long-term efficacy to the h1849-H3L3-vcMMAE with stronger cytotoxicity. This exhibits the advantage of the linker of the present application.

### 8.3 Test of the efficacy of h1849-H3L3-ADC-04 on a DU145 xenograft

### Experimental materials

Test compounds: h1849-H3L3-ADC-04, Tisotumab-vcMMAE (DAR4) (control ADC)
Experimental cells: DU145 cells
Experimental animals: BALB/c nude mice, 6-8 weeks old, body weight: 18-22 g, provided by Beijing Charles River Laboratory Animal Technology Co., Ltd. or other qualified suppliers.

### Experimental scheme

**Cell culture:** human prostate cancer DU145 cells (ATCC, Cat. No.: HTB-81) were subjected to *in vitro* adherent culture under culture conditions referring to the technical instruction from of the supplier. Cells were passaged by routine digestion with trypsin-EDTA twice a week. When the cell confluence reached 80%-90% and the number met the requirement, cells were harvested, counted, and inoculated.

**Tumor inoculation:** 0.2 mL (10 × 10⁶) DU145 cells (added with matrigel at a volume ratio of 1:1) were subcutaneously inoculated into the right dorsal flank of each mouse. When the average tumor volume reached about 200 mm³, the mice were grouped and administered.

### Animal experimental grouping and administration scheme:

| **Group** | **N¹** | **Compound treatment** | **Dose (mg/kg)** | **Administration volume parameter (µL/g)²** | **Administration route** | **Administration frequency** |
|---|---|---|---|---|---|---|
| 1 | 6 | Vehicle | 3 | 10 | IV | QW*2 |
| 2 | 6 | h1849-H3L3-ADC-04 | 3 | 10 | IV | QW*2 |
| 3 | 6 | Tisotumab-vcMMAE | 3 | 10 | IV | QW*2 |

| | | | | | | |
|---|---|---|---|---|---|---|
| Note: N: number of mice per group; administration volume: 10µL/g based on the mouse body weight. | | | | | | |

**Experimental indexes:** The experimental indexes are used to study whether tumor growth is inhibited, delayed, or cured. Tumor diameter and body weight were measured twice weekly using a vernier caliper. The formula for calculating tumor volume is: V = 0.5a × b², wherein a and b represent the long and short diameters of the tumor, respectively.

Data statistics was performed using Excel statistical software: the mean values were calculated by avg, SD values were calculated by STDEV, SEM values were calculated by ATDEV/SQRT, and T-test was used for comparison between two groups. One-way ANOVA was used for comparison among three or more groups. If the value F is significantly different, multiple comparisons should be performed after ANOVA analysis. p < 0.05 was considered statistically significant.

### Experimental results

The results are shown in FIG. 4. Under the circumstance of being administered at the same dose of 3 mg/kg, administered once a week for a total of two times, h1849-H3L3-ADC-04 exhibited tumor inhibitory activity comparable to the control ADC. This indicates that use of the linker of the present application may enhance the inhibitory effect of DX8951 in the DU145 tumor model and provide a basis for increasing the administration dose in the later period.

### 8.4 Test of the efficacy of h1849-H3L3-ADC-05 on a human cervical cancer Ca Ski xenograft

### Experimental materials

Test compounds: h1849-H3L3-ADC-05, Tisotumab-vcMMAE (DAR4) (control ADC)
Experimental cells: Ca Ski cells
Experimental animals: BALB/c nude mice, 6-8 weeks old, body weight: 18-22 g, provided by Beijing Charles River Laboratory Animal Technology Co., Ltd. or other qualified suppliers.

### Experimental scheme

**Cell culture:** human cervical cancer epithelial Ca Ski cells (ATCC, Cat. No.: CRM-CRL-1550) were subjected *in vitro* adherent culture under culture conditions referring to the technical instruction from the supplier. Cells were passaged by routine digestion with trypsin-EDTA twice a week. When the cell confluence reached 80%-90% and the number met the requirement, cells were harvested, counted, and inoculated.

**Tumor inoculation:** 0.1 mL (5 × 10⁶) Ca Ski cells were subcutaneously inoculated into the right dorsal flank of each mouse. When the average tumor volume reached about 200 mm³, the mice were grouped and administered.

### Animal experimental grouping and administration scheme:

| **Gro up** | **N¹** | **Compound treatment** | **Dose (mg/kg)** | **Administration volume parameter (µL/g)²** | **Adminis tration route** | **Administr ation frequency** |
|---|---|---|---|---|---|---|
| 1 | 6 | Vehicle | -- | 10 | IV | QW*2 |
| 2 | 6 | h1849-H3L3-ADC-05 | 3 | 10 | IV | QW*2 |
| 3 | 6 | Tisotumab-vcMMAE | 3 | 10 | IV | QW*2 |

| | | | | | | |
|---|---|---|---|---|---|---|
| Note: N: number of mice per group; administration volume: 10µL/g based on the mouse body weight. | | | | | | |

**Experimental indexes:** The experimental indexes are used to study whether tumor growth is inhibited, delayed, or cured. Tumor diameter and body weight were measured twice weekly using a vernier caliper. The formula for calculating tumor volume is: V = 0.5a × b², wherein a and b represent the long and short diameters of the tumor, respectively.

Data statistics was performed using Excel statistical software: the mean values were calculated by avg, SD values were calculated by STDEV, SEM values were calculated by ATDEV/SQRT, and T-test was used for comparison between two groups. One-way ANOVA was used for comparison among three or more groups. If the value F is significantly different, multiple comparisons should be performed after ANOVA analysis. p < 0.05 was considered statistically significant.

### Experimental results

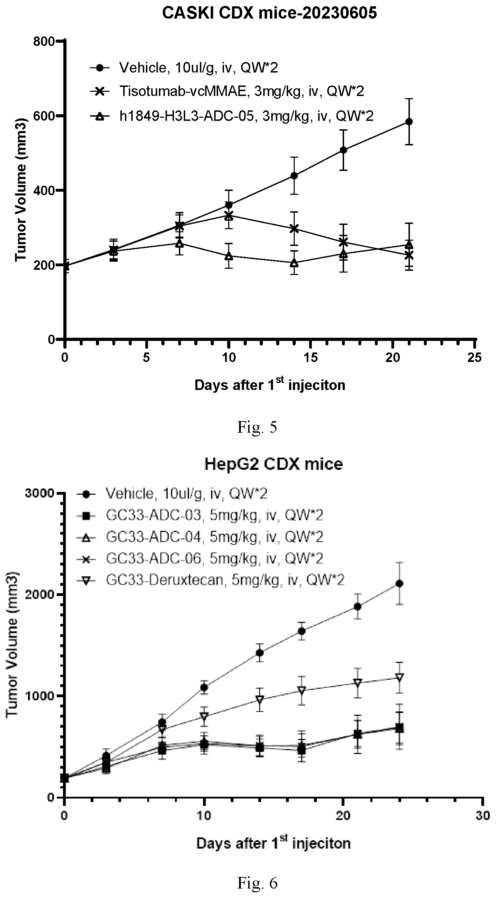

The results are shown in FIG. 5. Under the circumstance of being administered at the same dose of 3 mg/kg, administered once a week for a total of two times, after the anti-TF antibody being linked to DX8951 using the linker L015-P005 of the present application (h1849-H3L3-ADC-05), it also achieved good inhibitory effect on the human cervical cancer Ca Ski mouse model.

### 8.5 Test of the efficacy of GC33-ADC on a human liver cancer HepG2 xenograft

### Experimental materials

Test compounds: GC33-ADC-03, GC33-ADC-04, GC33-ADC-06, and GC33-Deruxtecan (control ADC)
Experimental cells: HepG2 cells
Experimental animals: BALB/c Nude mice, body weight: about 18-22 g, purchased from Shanghai Lingchang Biotechnology Co., Ltd.

### Experimental scheme

Cell culture: HepG2 cells were cultured in MEM culture solution containing 10% fetal bovine serum (FBS), 1% glutamine, 1% non-essential amino acids, and 1% sodium pyruvate. Cells in the logarithmic growth phase were collected, resuspended in HBSS, and used for subcutaneous tumor inoculation in BALB/c Nude mice.

**Tumor inoculation:** HepG2 cells in the logarithmic growth phase were collected, resuspended with the addition of HBSS, and counted, and the density thereof was adjusted to 1×10⁸ cells/mL. It was placed on ice for further use. Prior to inoculation, the cell suspension was mixed with Matrigel at a ratio of 1:1, and 0.2 mL of tumor cell suspension was inoculated subcutaneously into the right forelimb dorsum of mice. The day of inoculation was designated as D0. After inoculation, when the average tumor volume reached 180-220 mm³, the mice were grouped and administered.

### Animal experimental grouping and administration scheme:

| **Grou p** | **N¹** | **Compound treatment** | **Dose (mg/kg)** | **Administrati on volume parameter (µL/g)²** | **Administratio n route** | **Administratio n frequency** |
|---|---|---|---|---|---|---|
| 1 | 6 | Vehicle | -- | 10 | IV | QW*2 |
| 2 | 6 | GC33-ADC-03 | 5 | 10 | IV | QW*2 |
| 3 | 6 | GC33-ADC-04 | 5 | 10 | IV | QW*2 |
| 4 | 6 | GC33-ADC-06 | 5 | 10 | IV | QW*2 |
| 5 | 6 | GC33-Deruxtecan | 5 | 10 | IV | QW*2 |

| | | | | | | |
|---|---|---|---|---|---|---|
| Note: N: number of mice per group; administration volume: 10µL/g based on the mouse body weight. | | | | | | |

**Experimental indexes:** The experimental indexes are used to study whether tumor growth is inhibited, delayed, or cured. Tumor diameter and body weight were measured twice weekly using a vernier caliper. The formula for calculating tumor volume is: V = 0.5a × b², wherein a and b represent the long and short diameters of the tumor, respectively.

All data were analyzed using Graphpad and expressed as Mean ± SEM. All data were analyzed using the One-way ANOVA LSD(L) test in Graphpad to compare statistical differences between the test drug group and the control group. P < 0.05 was considered statistically significant.

### Experimental results

The results are shown in FIG. 6. Compared with the vehicle group, GC33-ADC exhibited the inhibitory effect on human liver cancer HepG2, and the inhibitory effect was significantly superior to the control ADC GC33-Deruxtecan.

For the purposes of description and disclosure, all the patents, patent applications, and other publications are expressly incorporated herein by reference. These publications are provided merely for their disclosures prior to the filing date of the present application. All the statements regarding the dates of these documents or the representations of the contents of these documents are based on information available to the applicant and do not constitute any admission to the correctness of the dates of these documents or the contents of these documents. Moreover, any reference to these publications herein does not constitute an admission that the publications form a part of the common general knowledge in the art in any country.

Those skilled in the art will recognize that the scope of the present application is not limited to the various specific embodiments and examples described above, but can be subjected to various modifications, substitutions, or recombinations without departing from the spirit of the present application, all of which fall within the protection scope of the present application.

## Claims

1. A polypeptide or polypeptide fragment having a structure represented by -P1-P2-, wherein P1 comprises a polypeptide responsive to matrix metalloproteinase 2, matrix metalloproteinase 9, or cathepsin B, and P2 comprises a polypeptide responsive to cathepsin B, matrix metalloproteinase 2, or matrix metalloproteinase 9.

2. The polypeptide or polypeptide fragment according to claim 1, wherein P1 comprises a polypeptide structure of -X1-P-X2-X3-Z-, and P2 comprises a polypeptide structure of -X1'-X2'-X3'-X4'-; wherein P is a proline residue, X1 represents absence, a single amino acid, or a single amino acid derivative, X2, X3, and Z each represent a single amino acid or a single amino acid derivative, X1' and X2' each represent a single amino acid or a single amino acid derivative, and X3' and X4' each represent absence or a single amino acid or a single amino acid derivative.

3. The polypeptide or polypeptide fragment according to claim 2, wherein X1 is selected from absence, Gly (G), Val (V), His (H), Ser (S), and Ile (I);
X2 is selected from Ala (A), Ser (S), Leu (L), Arg (R), Gln (Q), Val (V), and Cit;
X3 is selected from Gly (G), Ser (S), Pro (P), Asn (N), and Ala (A); and
Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylomithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylornithine (Orn(Et2)), and N,N-diethyllysine (Lys(Et2)).

4. The polypeptide or polypeptide fragment according to claim 2 or 3, wherein X1' is selected from Val (V), Als (A), Leu (L), Ile (I), Met (M), Phe (F), Trp (W), Pro (P), Tyr (Y), Gly (G), and Glu (E);
X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Om(Et2);
X3' is selected from absence, Phe (F), Gly (G), Lys (K), Lys(Me2), Lys(Et2), His (H), Ala (A), Arg (R), Cit, Orn, Orn(Me2), and Orn(Et2); and
X4' is selected from absence, Gly (G), and Phe (F).

5. The polypeptide or polypeptide fragment according to any one of claims 2 to 4, wherein X1 is selected from absence, Gly (G), Val (V), His (H), Ser (S), and Ile (I);
X2 is selected from Ala (A), Ser (S), Leu (L), Arg (R), Gln (Q), Val (V), and Cit;
X3 is selected from Gly (G), Ser (S), Pro (P), Asn (N), and Ala (A);
Z is selected from Leu (L), Ile (I), Val (V), Phe (F), Met (M), Ala (A), Trp (W), Gln (Q), N,N-dimethyl-3-aminopropionic acid (Dap(Me2)), N,N-dimethyl-4-aminobutyric acid (Dab(Me2)), N,N-dimethylornithine (Orn(Me2)), N,N-dimethyllysine (Lys(Me2)), N,N-diethyl-3-aminopropionic acid (Dap(Et2)), N,N-diethyl-4-aminobutyric acid (Dab(Et2)), N,N-diethylomithine (Orn(Et2)), and N,N-diethyllysine (Lys(Et2));
X1' is selected from Val (V), Als (A), Leu (L), Ile (I), Met (M), Phe (F), Trp (W), Pro (P), Tyr (Y), Gly (G), and Glu (E);
X2' is selected from Val (V), Leu (L), Ile (I), Met (M), Trp (W), Pro (P), Lys (K), Lys(Me2), Lys(Et2), His (H), Ala (A), Arg (R), Gly (G), Phe (F), Cit, Orn, Orn(Me2), and Om(Et2);
X3' is selected from absence, Phe (F), Gly (G), Lys (K), Lys(Me2), Lys(Et2), His (H), Ala (A), Arg (R), Cit, Orn, Orn(Me2), and Orn(Et2);
X4' is selected from absence, Gly (G), and Phe (F); and
preferably, X1 is absent, or is Gly (G), Ser (S), or His (H); X2 is Ala (A), Leu (L), Arg (R), Ser (S), Leu (L), or Cit; X3 is Gly (G), Ser (S), Ala (A), Asn (N), or Pro (P); Z is Leu (L) or Dap(Me2); X1' is Val (V) or Gly (G), X2' is Gly (G) or Cit, X3' and X4' are both absent, or X3' is Phe (F), and X4' is Gly (G).

6. The polypeptide or polypeptide fragment according to any one of claims 1 to 5, wherein P2 is -Val-Cit-, -Ala-Lys-, or -Gly-Gly-Phe-Gly-.

7. The polypeptide or polypeptide fragment according to any one of claims 1 to 6, which is selected from:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly-( SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50);
-Gly-Pro-Arg-Gly-Dap(Me2)-Val-Cit- (SEQ ID NO: 51);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67); and
-Pro-Ala-Ser-Leu-Val-Cit- (SEQ ID NO: 68);
or a variant having one or two mismatches relative to any sequence of SEQ ID NOs:1-51 and 60-68;
preferably, the polypeptide or polypeptide fragment is selected from:
-GPLSLVCit- (SEQ ID NO: 4);
-GPASLVCit- (SEQ ID NO: 5);
-GPAGLVCit- (SEQ ID NO: 6);
-GPRGLVCit- (SEQ ID NO: 10);
-GPSALVCit- (SEQ ID NO: 12);
-GPLGLGGFG- (SEQ ID NO: 20);
-GPRGLGGFG- (SEQ ID NO: 22);
-GPAALGGFG- (SEQ ID NO: 23);
-PRGLVCit- (SEQ ID NO: 25);
-PRSLVCit- (SEQ ID NO: 26);
-PRNLVCit- (SEQ ID NO: 27);
-PRPLVCit- (SEQ ID NO: 28);
-PCitGLVCit- (SEQ ID NO: 29);
-PCitPLVCit- (SEQ ID NO: 30);
-GPRSLVCit- (SEQ ID NO: 31);
-GPRPLVCit- (SEQ ID NO: 32);
-GPRNLVCit- (SEQ ID NO: 33);
-GPCitGLVCit- (SEQ ID NO: 34);
-GPCitPLVCit- (SEQ ID NO: 35);
-HPRGLVCit- (SEQ ID NO: 36);
-HPCitPLVCit- (SEQ ID NO: 41);
-SPRGLVCit- (SEQ ID NO: 42);
-SPRSLVCit- (SEQ ID NO: 43);
-SPRPLVCit- (SEQ ID NO: 44);
-SPRNLVCit- (SEQ ID NO: 45);
-SPCitGLVCit- (SEQ ID NO: 46);
-SPCitSLVCit- (SEQ ID NO: 47);
-SPCitPLVCit- (SEQ ID NO: 48);
-GPAPLVCit- (SEQ ID NO: 49);
-GPANLVCit- (SEQ ID NO: 50);
-GPRNDap(Me2)VCit- (SEQ ID NO: 60);
-PLSLVCit- (SEQ ID NO: 61);
-GPRNLGGFG- (SEQ ID NO: 62);
-PCitSLVCit- (SEQ ID NO: 63);
-GPCitNLVCit- (SEQ ID NO: 64);
-SPCitSDap(Me2)VCit- (SEQ ID NO: 65);
-PRSDap(Me2)VCit- (SEQ ID NO: 66);
-PCitNLVCit- (SEQ ID NO: 67); and
-PASLVCit- (SEQ ID NO: 68).

8. The polypeptide or polypeptide fragment according to any one of claims 1 to 7, which is selected from:
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Arg-Gly-Leu-Val-Cit- (SEQ ID NO: 10);
-Gly-Pro-Ser-Ala-Leu-Val-Cit- (SEQ ID NO: 12);
-Gly-Pro-Leu-Gly-Leu-Gly-Gly-Phe-Gly- (SEQ ID NO: 20);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Asn-Leu-Val-Cit- (SEQ ID NO: 50);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67); and
-Pro-Ala-Ser-Leu-Val-Cit- (SEQ ID NO: 68);
preferably, the polypeptide or polypeptide fragment is one or more selected from:
-GPASLVCit- (SEQ ID NO: 5);
-GPAGLVCit- (SEQ ID NO: 6);
-GPRGLVCit- (SEQ ID NO: 10);
-GPSALVCit- (SEQ ID NO: 12);
-GPLGLGGFG- (SEQ ID NO: 20);
-GPRGLGGFG- (SEQ ID NO: 22);
-GPAALGGFG- (SEQ ID NO: 23);
-PRGLVCit- (SEQ ID NO: 25);
-PRSLVCit- (SEQ ID NO: 26);
-PRNLVCit- (SEQ ID NO: 27);
-PRPLVCit- (SEQ ID NO: 28);
-PCitGLVCit- (SEQ ID NO: 29);
-PCitPLVCit- (SEQ ID NO: 30);
-GPRSLVCit- (SEQ ID NO: 31);
-GPRPLVCit- (SEQ ID NO: 32);
-GPRNLVCit- (SEQ ID NO: 33);
-GPCitGLVCit- (SEQ ID NO: 34);
-GPCitPLVCit- (SEQ ID NO: 35);
-HPRGLVCit- (SEQ ID NO: 36);
-HPCitPLVCit- (SEQ ID NO: 41);
-SPRGLVCit- (SEQ ID NO: 42);
-SPRSLVCit- (SEQ ID NO: 43);
-SPRPLVCit- (SEQ ID NO: 44);
-SPRNLVCit- (SEQ ID NO: 45);
-SPCitGLVCit- (SEQ ID NO: 46);
-SPCitSLVCit- (SEQ ID NO: 47);
-SPCitPLVCit- (SEQ ID NO: 48);
-GPAPLVCit- (SEQ ID NO: 49);
-GPANLVCit- (SEQ ID NO: 50);
-GPRNDap(Me2)VCit- (SEQ ID NO: 60);
-PLSLVCit- (SEQ ID NO: 61);
-GPRNLGGFG- (SEQ ID NO: 62);
-PCitSLVCit- (SEQ ID NO: 63);
-GPCitNLVCit- (SEQ ID NO: 64);
-SPCitSDap(Me2)VCit- (SEQ ID NO: 65);
-PRSDap(Me2)VCit- (SEQ ID NO: 66);
-PCitNLVCit- (SEQ ID NO: 67); and
-PASLVCit- (SEQ ID NO: 68).

9. A linker comprising the polypeptide or polypeptide fragment having a structure represented by -P1-P2- according to any one of claims 1 to 8, wherein the linker has a general formula of C1-La-L1-P1-P2-L2;
wherein C1 is an adapter;
La is absent or a tuning spacer;
L1 is absent or a spacer;
L2 is absent or a spacer; and
positions of La and L1 are interchangeable.

10. The linker according to claim 9, wherein C1 is selected from the following structures: 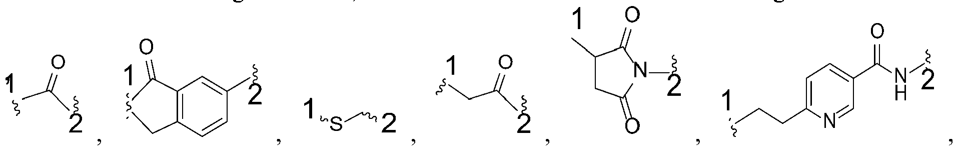 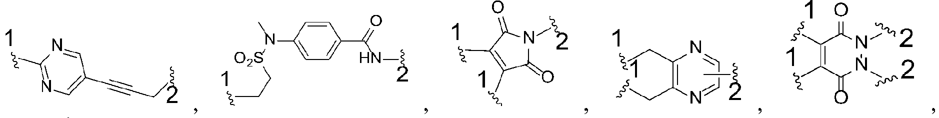 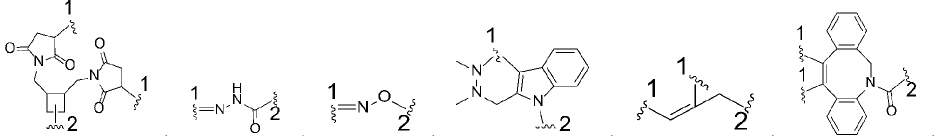 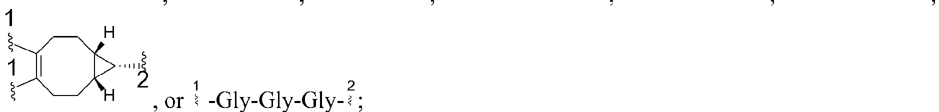 wherein 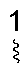 indicates a position for connection to a bioligand unit, and 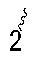 indicates a position for connection to La.

11. The linker according to claim 9 or 10, wherein C1 is selected from a structure of - (succinimid-3-yl-N)-, -CH₂-C(=O)-, -C(=O)-, or 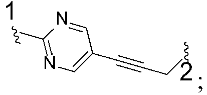 wherein -(succinimid-3-yl-N)-has a structure of 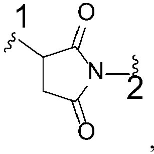 wherein 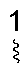 indicates a position for connection to a bioligand unit, and 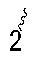 indicates a position for connection to La.

12. The linker according to any one of claims 9 to 11, wherein La is selected from the following structures or combinations thereof:
R1-S-S-R2,
R1-(CH₂CH₂O)ₘ-R2,
R1-(CH₂CH₂O)ₘ-CH₂CH₂S-(CH₂CH₂O)ₙ-R2,
R1-(CH₂CH₂O)ₘ-CH₂CH₂NH-(CH₂CH₂O)ₙ-R2,
R1-(CH₂CH₂O)ₘ-CH₂CH₂(C=O)NH-(CH₂CH₂O)ₙ-R2, 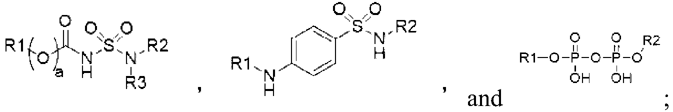
a is 0 or 1;
m is any integer from 1 to 20;
n is any integer from 1 to 20;
R3 is H, -C₁-C₁₀ alkylene-, -C₃-C₈ carbocyclyl-, -C₂-C₁₀ heteroaryl-, or -C₃-C₂₄ alkylheteroaryl-, wherein -C₁-C₁₀ alkylene-, -C₃-C₈ carbocyclyl-, -C₂-C₁₀ heteroaryl-, and -C₃-C₂₄ alkylheteroaryl- can be substituted by one or more heteroatoms, and the heteroatoms can be O, S, or NR³, wherein R³ is H or -C₁-C₄ alkylene;
R1 is a structure for connection to C1, and R2 is a structure for connection to L1;
R1 is a single bond or selected from a structure of -X¹-C₁-C₁₀ alkylene-X²-, -X¹-C₁-C₁₀ heteroalkylene-X²-, -X¹-C₃-C₈ carbocyclyl-X²-, -X¹-arylene-X²-, -X¹-C₁-C₁₀ alkylene-arylene-X²-, - X¹-arylene-C₁-C₁₀ alkylene-X²-, -X¹-C₁-C₁₀ alkylene-(C₃-C₈ carbocyclyl)-X²-, -X¹-(C₃-C₈ carbocyclyl)-C₁-C₁₀ alkylene-X²-, -X¹-C₃-C₈ heterocyclyl-X²-, -X¹-C₁-C₁₀ alkylene-(C₃-C₈ heterocyclyl)-X²-, and -X¹-(C₃-C₈ heterocyclyl)-C₁-C₁₀ alkylene-X²-;
wherein X¹ and X² are each independently selected from absence or O, S, NH, C(=O), NHC(=O), C(=O)NH, and NHC(=O)NH; wherein when R1 is not a single bond, R1 is optionally substituted with a basic unit (BU), and the basic unit is -(CH₂)ₓNH₂, -(CH₂)ₓNHRₘ, or -(CH₂)ₓN(Rₘ)₂; wherein x is any integer from 1 to 4; and each Rₘ is independently selected from C₁-C₆ alkyl and C₁-C₆ haloalkyl, or two Rₘ groups, in combination with the nitrogen to which they are attached, form a 4-to 6-membered heterocycloalkyl ring, an azetidinyl, pyrrolidinyl, or piperidinyl group;
R2 is a single bond or is selected from a structure of -X³-C₁-C₁₀ alkylene-X⁴-, -X³-C₁-C₁₀ heteroalkylene-X⁴-, -X³-C₃-C₈ carbocyclyl-X⁴-, -X³-arylene-X⁴-, -X³-C₁-C₁₀ alkylene-arylene-X⁴-, - X³-arylene-C₁-C₁₀ alkylene-X⁴-, -X³-C₁-C₁₀ alkylene-(C₃-C₈ carbocyclyl)-X⁴-, -X³-(C₃-C₈ carbocyclyl)-C₁-C₁₀ alkylene-X⁴-, -X³-C₃-C₈ heterocyclyl-X⁴-, -X³-C₁-C₁₀ alkylene-(C₃-C₈ heterocyclyl)-X⁴-, and -X³-(C₃-C₈ heterocyclyl)-C₁-C₁₀ alkylene-X⁴-; and
wherein X³ is absent or selected from O, S, NH, C(=O), NHC(=O), C(=O)NH, and NHC(=O)NH, and X⁴ is absent or selected from O, NH, S, C(=O), C(=O)NH, NHC(=O), and NHC(=O)NH; wherein when R2 is not a single bond, R2 is optionally substituted with a basic unit (BU), and the basic unit is -(CH₂)ₓNH₂, -(CH₂)ₓNHRₘ, or -(CH₂)ₓN(Rₘ)₂; wherein x is any integer from 1 to 4; and each Rₘ is independently selected from C₁-C₆ alkyl and C₁-C₆ haloalkyl, or two Rₘ groups, in combination with the nitrogen to which they are attached, form a 4- to 6-membered heterocycloalkyl ring, an azetidinyl, pyrrolidinyl, or piperidinyl group.

13. The linker according to any one of claims 9 to 12, wherein L1 is selected from a structure of -X⁵-C₁-C₁₀ alkylene-X⁶-, -X⁵-C₁-C₁₀ heteroalkylene-X⁶-, -X⁵-C₃-C₈ carbocyclyl-X⁶-, -X⁵-arylene-X⁶-, -X⁵-heteroarylene-X⁶-, -X⁵-C₁-C₁₀ alkylene-arylene-X⁶-, -X⁵-C₁-C₁₀ alkylene-heteroarylene-X⁶-, -X³-arylene-C₁-C₁₀ alkylene-X⁶-, -X⁵-heteroarylene-C₁-C₁₀ alkylene-X⁶-, -X⁵-C₁-C₁₀ alkylene-(C₃-C₈ carbocyclyl)-X⁶-, -X⁵-(C₃-C₈ carbocyclyl)-C₁-C₁₀ alkylene-X⁶-, -X⁵-C₃-C₈ heterocyclyl-X⁶-, -X⁵-C₁-C₁₀ alkylene-(C₃-C₈ heterocyclyl)-X⁶-, -X⁵-(C₃-C₈ heterocyclyl)-C₁-C₁₀ alkylene-X⁶-, -X⁵-C₃-C₈ heterocyclyl-arylene-X⁶-, -X⁵-arylene-C₃-C₈ heterocyclyl-X⁶-, -X⁵-C₃-C₈ heterocyclyl-heteroarylene-X⁶-, and -X⁵-heteroarylene-C₃-C₈ heterocyclyl-X⁶-;
wherein X⁵ is absent or selected from O, S, NH, C(=O), NHC(=O), C(=O)NH, and NHC(=O)NH, and X⁶ is absent or selected from O, NH, S, C(=O), NHC(=O), C(=O)NH, and NHC(=O)NH; and
wherein any methylene unit of L1 can be independently substituted with -O-, -S-, -N(Rₙ)-, - CH(N(Rₙ)₂)-, -CH(P(Rₙ)₂)-, -C(N(Rₙ)₂)₂-, -C(ORₙ)(N(Rₙ)₂)-, -C(=O)-, -OC(=O)-, -C(=O)O-, - N(Rₙ)C(=O)-, -C(=O)N(Rₙ)-, -SO-, -SO₂-, -N(Rₙ)SO₂-, -SO₂N(Rₙ)-, -P(Rₙ)-, -P(=O)(Rₙ)-, - P(=O)(O)-, -C(=S)-, -C(=N-Rₙ)-, -S-S-, -N=N-, -N(Rₙ)-N=, =N-N(Rₙ)-, -C=N-, or -N=C-; wherein each Rₙ is independently selected from H, C₁ -C₆ alkyl, C₃-C₈ carbocyclyl, and C₁-C₆ haloalkyl, or two Rₙ groups, in combination with the nitrogen or phosphorus to which they are attached, form a 4-to 6-membered heterocycloalkyl, or azetidinyl, pyrrolidinyl, or piperidinyl group.

14. The linker according to any one of claims 9 to 13, wherein C1-La-L1- is

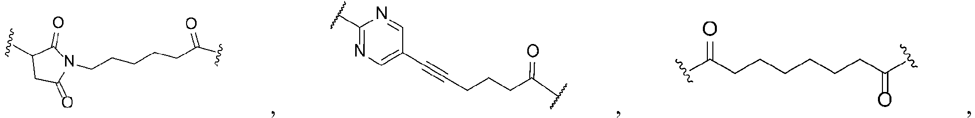

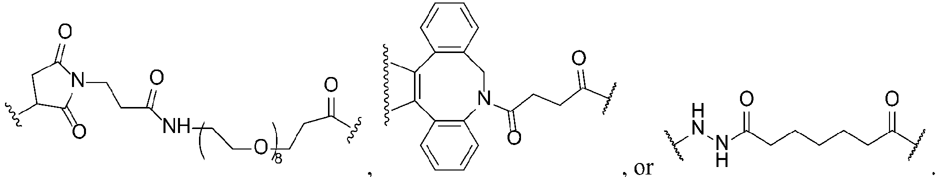

15. The linker according to any one of claims 9 to 14, wherein L2 is absent or is selected from the following structures: 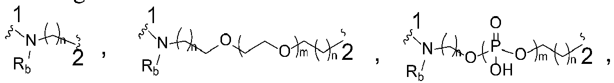 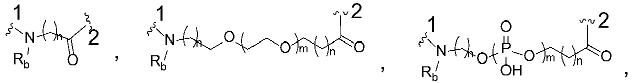 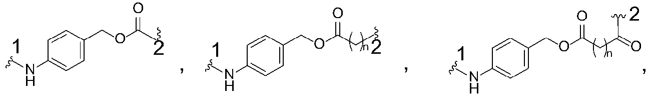 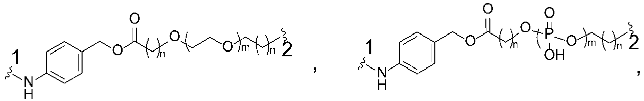 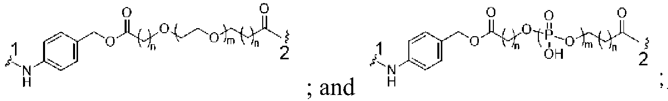
wherein 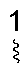 indicates a position for connection to the polypeptide, and 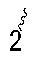 indicates a position for connection to a payload with a specific biological function;
m is any integer from 1 to 20;
n is any integer from 1 to 6;
R_{b} is independently selected from H, C₁-C₆ alkyl, C₃-C₈ carbocyclyl, C₁-C₆ haloalkyl, C₃-C₈ heterocyclyl, C₁-C₆ alkyl-C₃-C₈ heterocyclyl, substitutable aryl, substitutable heteroaryl, C₁-C₆ alkyl-substitutable aryl, and C₁-C₆ alkyl-substitutable heteroaryl; and
wherein any methylene unit of L2 can be independently substituted with -CHX-, -C(X₂)-, -C₃-C₈ carbocyclyl-, -C₃-C₈ heterocyclyl-, -arylene-, -heteroarylene-, -O-, -S-, -N(Rₙ)-, -CH(N(Rₙ)₂)-, - CH(P(Rₙ)₂)-, -C(N(Rₙ)₂)₂-, -C(ORₙ)(N(Rₙ)₂)-, -C(=O)-, -OC(=O)-, -C(=O)O-, -N(Rₙ)C(=O)-, - C(=O)N(Rₙ)-, -SO-, -SO₂-, -N(Rₙ)SO₂-, -SO₂N(Rₙ)-, -P(Rₙ)-, -P(=O)(Rₙ)-, -P(=O)(O)-, -C(=S)-, - C(=N-Rₙ)-, -S-S-, -N=N-, -N(Rₙ)-N=, =N-N(Rₙ)-, -C=N-, or -N=C-, wherein X represents a halogen or deuterium, and each Rₙ is independently selected from H, C₁-C₆ alkyl, C₃-C₈ carbocyclyl, and C₁-C₆ haloalkyl, or two Rₙ groups, in combination with the nitrogen or phosphorus to which they are attached, form a 4- to 6-membered heterocycloalkyl ring, or an azetidinyl, pyrrolidinyl, or piperidinyl group.

16. The linker according to any one of claims 9 to 15, wherein L2 is absent or is selected from the following structures: 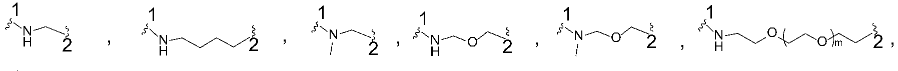 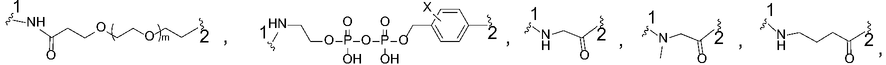 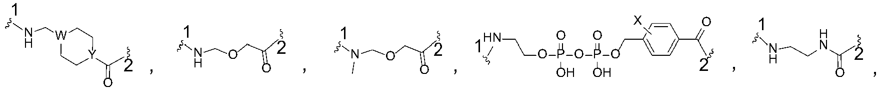 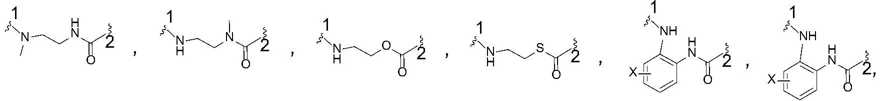 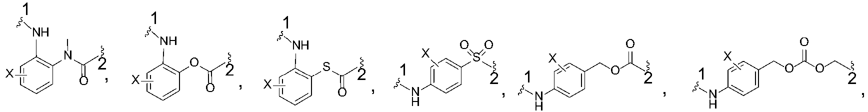 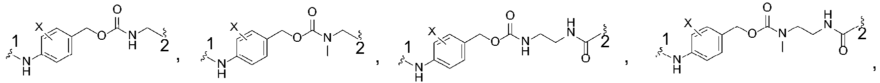 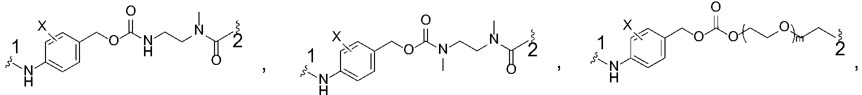 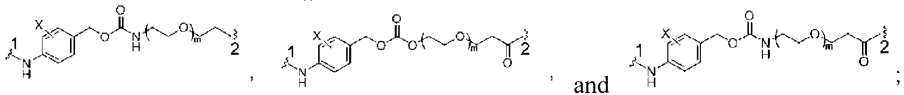
wherein 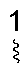 indicates a position for connection to the polypeptide, and 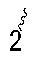 indicates a position for connection to a payload with a specific biological function;
W and Y are each independently N or CR^{Y}, and each R^{Y} is independently H or C₁ -C₁₀ alkyl;
m is any integer from 0 to 20; and
X is a halogen or deuterium.

17. The linker according to any one of claims 9 to 16, wherein L2 is absent or is selected from: 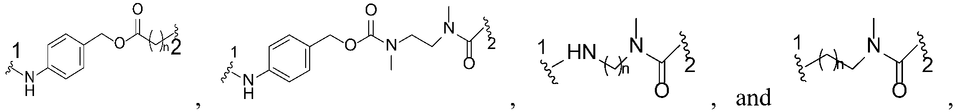 wherein n is any integer selected from 1-6, 1-5, 1-4, or 1-3; and
preferably, L2 is absent or is selected from -PAB-, -PAB-DMEDA-, and -NMEDA-.

18. The linker according to any one of claims 9 to 17, wherein the linker is selected from:
-(succinimid-3-yl-N)-CH₂-C(=O)-P1-P2-;
-(succinimid-3-yl-N)-CH₂CH₂-C(=O)-P1-P2-;
-(succinimid-3-yl-N)-(CH₂CH₂O)₄-CH₂CH₂-C(=O)-P1-P2-;
-(succinimid-3-yl-N)-(CH₂CH₂O)₈-CH₂CH₂-C(=O)-P1-P2-;
-(succinimid-3-yl-N)-CH₂CH₂C(O)NH-(CH₂CH₂O)₄-CH₂CH₂-C(=O)-P1-P2-;
-(succinimid-3-yl-N)-CH₂CH₂C(O)NH-(CH₂CH₂O)₈-CH₂CH₂-C(=O)-P1-P2-;
-(succinimid-3-yl-N)-OC(O)-(CH₂CH₂O)₄-CH₂CH₂-C(=O)-P1-P2-;
-(succinimid-3-yl-N)-OC(O)-(CH₂CH₂O)₈-CH₂CH₂-C(=O)-P1-P2-;
-(succinimid-3-yl-N)-CH₂CH₂CH₂CH₂CH₂-C(=O)-P1-P2-;
-(succinimid-3-yl-N)-CH₂-C(=O)-P1-P2-PAB-;
-(succinimid-3-yl-N)-CH₂CH₂-C(=O)-P1-P2-PAB-;
-(succinimid-3-yl-N)-(CH₂CH₂O)₄-CH₂CH₂-C(=O)-P1-P2-PAB-;
-(succinimid-3-yl-N)-(CH₂CH₂O)₈-CH₂CH₂-C(=O)-P1-P2-PAB-;
-(succinimid-3-yl-N)-CH₂CH₂C(O)NH-(CH₂CH₂O)₄-CH₂CH₂-C(=O)-P1-P2-PAB-;
-(succinimid-3-yl-N)-CH₂CH2C(O)NH-(CH₂CH₂O)₈-CH₂CH₂-C(=O)-P1-P2-PAB-;
-(succinimid-3-yl-N)-OC(O)-(CH₂CH₂O)₄-CH₂CH₂-C(=O)-P1-P2-PAB-;
-(succinimid-3-yl-N)-OC(O)-(CH₂CH₂O)₈-CH₂CH₂-C(=O)-P1-P2-PAB-;
-(succinimid-3-yl-N)-CH₂CH₂CH₂CH₂CH₂-C(=O)-P1-P2-PAB-; 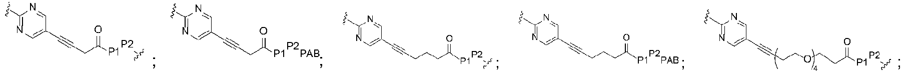 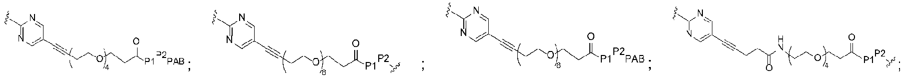 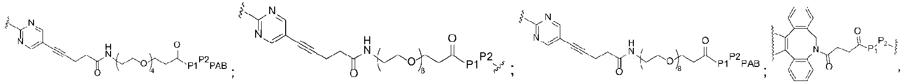 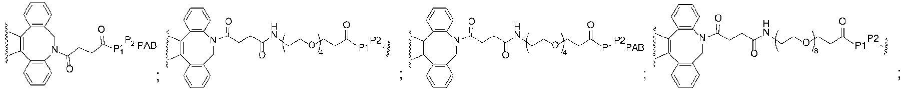 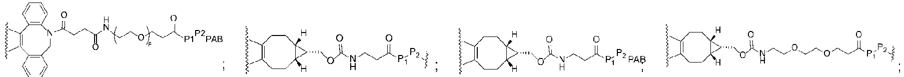 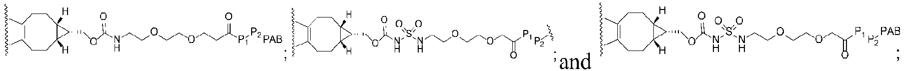
wherein 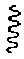 indicates a position for connection to a bioligand unit.

19. The linker according to any one of claims 9 to 18, wherein the linker is selected from:
-(succinimid-3-yl-N)-C1-C10 alkylene-C(=O)-P1-P2-;
-(succinimid-3-yl-N)-C1-C10 alkylene-C(=O)NH-P1-P2-;
-(succinimid-3-yl-N)-C1-C10 alkylene-C(=O)NH-(CH₂CH₂O)ₘ-P1-P2-;
-(succinimid-3-yl-N)-C1-C10 alkylene-C(=O)NH-(CH₂CH₂O)ₘ-C1-C10 alkylene-C(=O)-P1-P2-;
-(succinimid-3-yl-N)-C1-C10 alkylene-C(=O)-P1-P2-PAB-;
-(succinimid-3-yl-N)-C1-C10 alkylene-C(=O)NH-P1-P2-PAB-;
-(succinimid-3-yl-N)-C1-C10 alkylene-C(=O)NH-(CH₂CH₂O)ₘ-P1-P2-PAB-;
-(succinimid-3-yl-N)-C1-C10 alkylene-C(=O)NH-(CH₂CH₂O)ₘ-C1-C10 alkylene-C(=O)-P1-P2-PAB-;
-(succinimid-3-yl-N)-C1-C10 alkylene-C(=O)-P1-P2-PAB-DMEDA-;
-(succinimid-3-yl-N)-C1-C10 alkylene-C(=O)NH-P1-P2-PAB-DMEDA-;
-(succinimid-3-yl-N)-C1-C10 alkylene-C(=O)NH-(CH₂CH₂O)ₘ-P1-P2-PAB-DMEDA-;
-(succinimid-3-yl-N)-C1-C10 alkylene-C(=O)NH-(CH₂CH₂O)ₘ-C1-C10 alkylene-C(=O)-P1-P2-PAB-DMEDA-;
-(succinimid-3-yl-N)-C1-C10 alkylene-C(=O)-P1-P2-NMEDA-;
-(succinimid-3-yl-N)-C1-C10 alkylene-C(=O)NH-P1-P2-NMEDA-;
-(succinimid-3-yl-N)-C1-C10 alkylene-C(=O)NH-(CH₂CH₂O)ₘ-P1-P2-NMEDA-;
-(succinimid-3-yl-N)-C1-C10 alkylene-C(=O)NH-(CH₂CH₂O)ₘ-C1-C10 alkylene-C(=O)-P1-P2-NMEDA-;
-(C=O)-C1-C10 alkylene-C(=O)-P1-P2-;
-(C=O)-C1-C10 alkylene-C(=O)-P1-P2-PAB-;
-(C=O)-C1-C10 alkylene-C(=O)NH-P1-P2-PAB-;
-(C=O)-C1-C10 alkylene-C(=O)-P1-P2-PAB-DMEDA-;
-(C=O)-C1-C10 alkylene-C(=O)NH-P1-P2-PAB-DMEDA-;
-(C=O)-C1-C10 alkylene-C(=O)-P1-P2-NMEDA-;
-(C=O)-C1-C10 alkylene-C(=O)NH-P1-P2-NMEDA-; 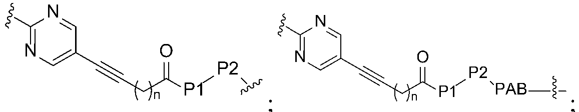 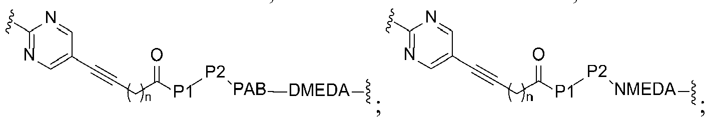 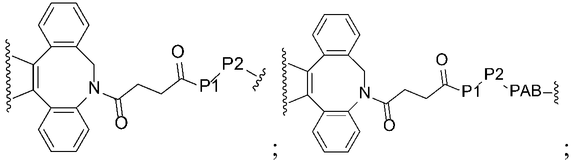 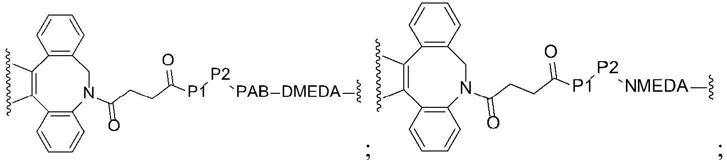 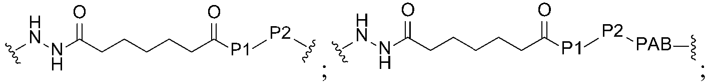 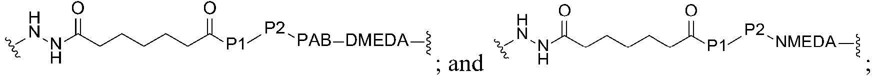
m is any integer selected from 3 to 15, preferably 5-10, 6-9, or 7-8,
n is any integer selected from 1 to 10, preferably 2-6, 2-5, or 2-4;
preferably, the linker is selected from:
-(succinimid-3-yl-N)-C5 alkylene-C(=O)-P1-P2-;
-(succinimid-3-yl-N)-C5 alkylene-C(=O)-P1-P2-PAB-;
-(succinimid-3-yl-N)-C5 alkylene-C(=O)-P1-P2-PAB-DMEDA-;
-(succinimid-3-yl-N)-C5 alkylene-C(=O)-P1-P2-NMEDA-; 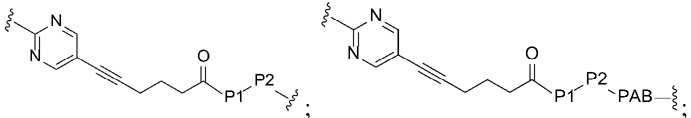 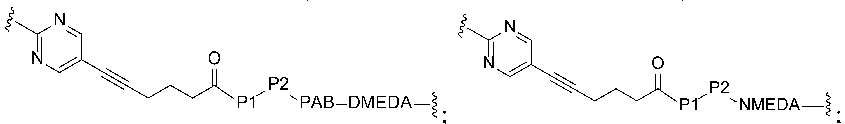
-(C=O)-C6 alkylene-C(=O)-P1-P2-;
-(C=O)-C6 alkylene-C(=O)-P1-P2-PAB-;
-(C=O)-C6 alkylene-C(=O)-P1-P2-PAB-DMEDA-;
-(C=O)-C6 alkylene-C(=O)-P1-P2-NMEDA-;
-(succinimid-3-yl-N)-C2 alkylene-C(=O)NH-(CH₂CH₂O)₈-C2 alkylene-C(=O)-P1-P2-;
-(succinimid-3-yl-N)-C2 alkylene-C(=O)NH-(CH₂CH₂O)₈-C2 alkylene-C(=O)-P1-P2-PAB-;
-(succinimid-3-yl-N)-C2 alkylene-C(=O)NH-(CH₂CH₂O)₈-C2 alkylene-C(=O)-P1-P2-PAB-DMEDA-;
-(succinimid-3-yl-N)-C2 alkylene-C(=O)NH-(CH₂CH₂O)₈-C2 alkylene-C(=O)-P1-P2-NMEDA-; 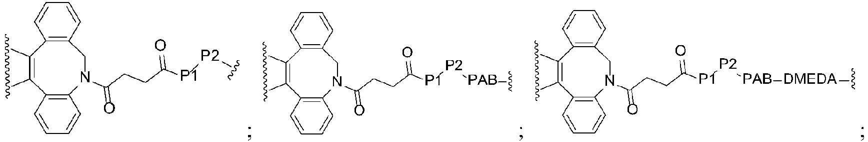 and

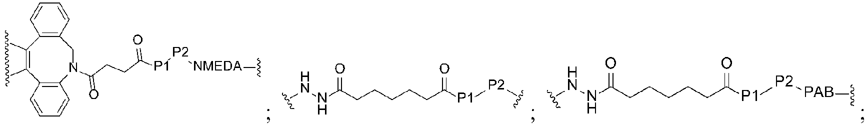

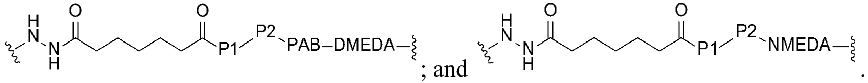

20. The linker according to any one of claims 9 to 19, wherein the linker is selected from:
-(succinimid-3-yl-N)-C5 alkylene-C(=O)-GPAGLVCit-PAB-;
-(succinimid-3-yl-N)-C5 alkylene-C(=O)-GPLGLGGFG-;
-(succinimid-3-yl-N)-C5 alkylene-C(=O)-GPASLVCit-PAB-;
-(succinimid-3-yl-N)-C5 alkylene-C(=O)-GPRGLVCit-PAB-;
-(succinimid-3-yl-N)-C5 alkylene-C(=O)-GPSALVCit-PAB-;
-(succinimid-3-yl-N)-C5 alkylene-C(=O)-GPRGLGGFG-;
-(succinimid-3-yl-N)-C5 alkylene-C(=O)-GPAALGGFG-;
-(succinimid-3-yl-N)-C5 alkylene-C(=O)-PRNLVCit-PAB-;
-(succinimid-3-yl-N)-C5 alkylene-C(=O)-GPRNLVCit-PAB-;
-(succinimid-3-yl-N)-C5 alkylene-C(=O)-GPRNDap(Me2)VCit-PAB-;
-(succinimid-3-yl-N)-C5 alkylene-C(=O)-SPRNLVCit-PAB-;
-(succinimid-3-yl-N)-C5 alkylene-C(=O)-GPLSLVCit-PAB-;
-(succinimid-3-yl-N)-C5 alkylene-C(=O)-PRGLVCit-PAB-;
-(succinimid-3-yl-N)-C5 alkylene-C(=O)-PRPLVCit-PAB-;
-(succinimid-3-yl-N)-C5 alkylene-C(=O)-GPRSLVCit-PAB-;
-(succinimid-3-yl-N)-C5 alkylene-C(=O)-GPRPLVCit-PAB-;
-(succinimid-3-yl-N)-C5 alkylene-C(=O)-GPCitPLVCit-PAB-;
-(succinimid-3-yl-N)-C5 alkylene-C(=O)-HPRGLVCit-PAB-;
-(succinimid-3-yl-N)-C5 alkylene-C(=O)-HPCitPLVCit-PAB-;
-(succinimid-3-yl-N)-C5 alkylene-C(=O)-SPCitGLVCit-PAB-;
-(succinimid-3-yl-N)-C5 alkylene-C(=O)-GPAPLVCit-PAB-;
-(succinimid-3-yl-N)-C5 alkylene-C(=O)-PLSLVCit-PAB-;
-(succinimid-3-yl-N)-C5 alkylene-C(=O)-SPCitSLVCit-PAB-; 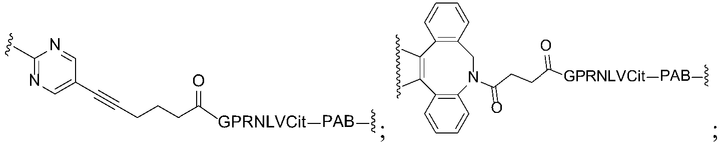
-(C=O)-C6 alkylene-C(=O)-GPRNLVCit-PAB-; 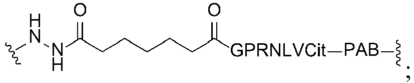
-(succinimid-3-yl-N)-C2 alkylene-C(=O)NH-(CH₂CH₂O)₈-C2 alkylene-C(=O)-GPRNLVCit-PAB-;
-(succinimid-3-yl-N)-C5 alkylene-C(=O)-GPRNLVCit-PAB-DMEDA-;
-(succinimid-3-yl-N)-C5 alkylene-C(=O)-GPRNLGGFG-;
-(succinimid-3-yl-N)-C5 alkylene-C(=O)-GPRNLGGFG-NMEDA-;
-(succinimid-3-yl-N)-C5 alkylene-C(=O)-PRSLVCit-PAB-;
-(succinimid-3-yl-N)-C5 alkylene-C(=O)- SPRSLVCit-PAB-;
-(succinimid-3-yl-N)-C5 alkylene-C(=O)-PCitSLVCit-PAB-;
-(succinimid-3-yl-N)-C5 alkylene-C(=O)-PCitPLVCit-PAB-;
-(succinimid-3-yl-N)-C5 alkylene-C(=O)-PCitGLVCit-PAB-;
-(succinimid-3-yl-N)-C5 alkylene-C(=O)-GPCitGLVCit-PAB-;
-(succinimid-3-yl-N)-C5 alkylene-C(=O)-SPRGLVCit-PAB-;
-(succinimid-3-yl-N)-C5 alkylene-C(=O)- SPRPLVCit-PAB-;
-(succinimid-3-yl-N)-C5 alkylene-C(=O)-SPCitPLVCit-PAB-;
-(succinimid-3-yl-N)-C5 alkylene-C(=O)-GPANLVCit-PAB-;
-(succinimid-3-yl-N)-C5 alkylene-C(=O)-GPCitNLVCit-PAB-;
-(succinimid-3-yl-N)-C5 alkylene-C(=O)-SPCitSDap(Me2)VCit-PAB-;
-(succinimid-3-yl-N)-C5 alkylene-C(=O)-PRSDap(Me2)VCit-PAB-;
-(succinimid-3-yl-N)-C5 alkylene-C(=O)- PCitNLVCit-PAB-; and
-(succinimid-3-yl-N)-C5-alkylene-C(=O)- PASLVCit-PAB-;
preferably, the linker is selected from:
-(succinimid-3-yl-N)- CH₂CH₂CH₂CH₂CH₂-C(=O)-GPLGLGGFG-;
-(succinimid-3-yl-N)-CH₂CH₂CH₂CH₂CH₂-C(=O)-GPRGLGGFG-;
-(succinimid-3-yl-N)-CH₂CH₂CH₂CH₂CH₂-C(=O)-GPAALGGFG-;
-(succinimid-3-yl-N)-CH₂CH₂CH₂CH₂CH₂-C(=O)-GPAGLVCit-PAB-;
-(succinimid-3-yl-N)-CH₂CH₂CH₂CH₂CH₂-C(=O)-GPASLVCit-PAB-;
-(succinimid-3-yl-N)-CH₂CH₂CH₂CH₂CH₂-C(=O)-GPRGLVCit-PAB-;
-(succinimid-3-yl-N)-CH₂CH₂CH₂CH₂CH₂-C(=O)-GPSALVCit-PAB-;
-(succinimid-3-yl-N)-CH₂CH₂CH₂CH₂CH₂-C(=O)-PRNLVCit-PAB-;
-(succinimid-3-yl-N)-CH₂CH₂CH₂CH₂CH₂-C(=O)-GPRNLVCit-PAB-;
-(succinimid-3-yl-N)-CH₂CH₂CH₂CH₂CH₂-C(=O)-GPRGDap(Me2)VCit-PAB-;
-(succinimid-3-yl-N)-CH₂CH₂CH₂CH₂CH₂-C(=O)-SPRNLVCit-PAB-; and
-(succinimid-3-yl-N)-CH₂CH₂CH₂CH₂CH₂-C(=O)-GPLSLVCit-PAB-.

21. A linker drug comprising the polypeptide or polypeptide fragment having a structure represented by -P1-P2- according to any one of claims 1 to 8, wherein the linker drug has a structure of C1'-La-L1-P1-P2-L2-D,
wherein C1' is a functional group that reacts with a bioligand;
La is absent or a tuning spacer;
L1 is absent or a spacer;
L2 is absent or a spacer; and
D is a drug unit; and
positions of La and L1 are interchangeable.

22. The linker drug according to claim 21, wherein La is as defined in claim 12, L1 is as defined in claim 13, and L2 is as defined in any one of claims 15 to 17.

23. The linker drug according to claim 21 or 22, wherein C1' is selected from a functional group that reacts with amino, sulfhydryl, keto, azido, or alkynyl.

24. The linker drug according to any one of claims 21 to 23, wherein C1' is selected from the following structures: 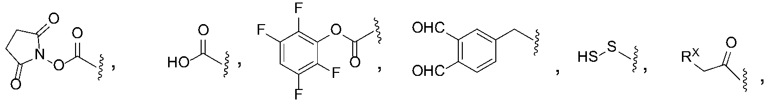 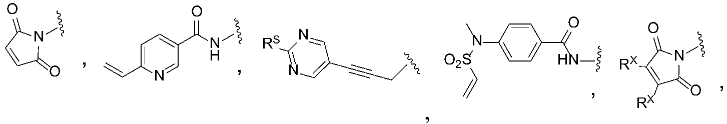 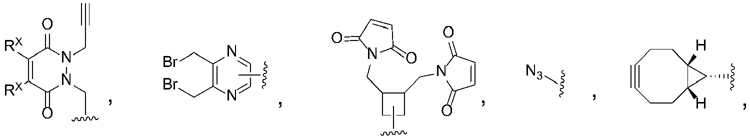 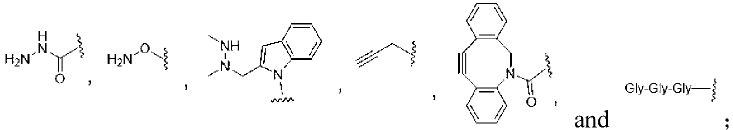
wherein R^{X} is selected from a halogen and -SPh; R^{S} is selected from a halogen, sulfonyl, a tertiary-amine salt group, a diazonium salt group, -OMs, MeSO₂-, and CF₃SO₃-; 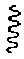 indicates a position for connection to La; and
preferably, C1' is selected from a structure of 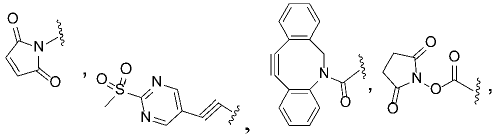 and

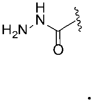

25. The linker drug according to any one of claims 21 to 24, wherein C1'-La-L1- is MC-, MeO2S-Pym-, NHS-, Mal-PEG8-, DBCO-, or Hydrazide-.

26. The linker drug according to any one of claims 21 to 25, wherein the drug unit D is selected from immunomodulators, protein degrading agents, and cytotoxic agents.

27. The linker drug according to any one of claims 21 to 26, wherein the drug unit D comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2, 1-c][1, 4]benzodi-azepines (PBDs), puromycins, rhizoxins, SN-38, taxanes, tubulysins, vincaalkaloids, tetrahydroisoquinoline alkaloids or derivatives thereof, protein degrading agents or derivatives thereof.

28. The linker drug according to any one of claims 21 to 27, wherein the drug unit D comprises a maytansine alkaloid or derivatives thereof, camptothecin or derivatives thereof, auristatin or derivatives thereof, a tetrahydroisoquinoline alkaloid or derivatives thereof, a BTK protein degrading agent and derivatives thereof, or a GSPT1 protein degrading agent and derivatives thereof.

29. The linker drug according to any one of claims 21 to 28, wherein the drug unit D comprises DM1, DM4, DX8951, MMAE, Dxd, SN38, Trabectedin (ET743), Lurbinectedin, CC885, or CC-90009.

30. The linker drug according to any one of claims 21 to 29, wherein the linker drug is selected from:
MC-P1-P2-D;
MC-P1-P2-PAB-D;
MC-P1-P2-PAB-DMEDA-D;
MC-P1-P2-NMEDA-D;
MeO2S-Pym-P1-P2-D;
MeO2S-Pym-P1-P2-PAB-D;
MeO2S-Pym-P1-P2-PAB-DMEDA-D;
MeO2S-Pym-P1-P2-NMEDA-D;
NHS-P1-P2-D;
NHS-P1-P2-PAB-D;
NHS-P1-P2-PAB-DMEDA-D;
NHS-P1-P2-NMEDA-D;
Mal-PEG8-P1-P2-D;
Mal-PEG8-P1-P2-PAB-D;
Mal-PEG8-P1-P2-PAB-DMEDA-D;
Mal-PEG8-P1-P2-NMEDA-D;
DBCO-P1-P2-D;
DBCO-P1-P2-PAB-D;
DBCO-P1-P2-PAB-DMEDA-D;
DBCO-P1-P2-NMEDA-D;
Hydrazide-P1-P2-D;
Hydrazide-P1-P2-PAB-D;
Hydrazide-P1-P2-PAB-DMEDA-D; and
Hydrazide-P1-P2-NMEDA-D;
and preferably, the drug unit D comprises DX8951, MMAE, Dxd, SN38, or ET743.

31. The linker drug according to any one of claims 21 to 30, wherein the linker drug has the following structure:
(maleimid-N-yl)-CH2-C(=O)-P1-P2-D;
(maleimid-N-yl)-CH2CH2-C(=O)-P1-P2-D;
(maleimid-N-yl)-(CH2CH2O)₄-CH2CH2-C(=O)-P1-P2-D;
(maleimid-N-yl)-(CH2CH2O)₈-CH2CH2-C(=O)-P1-P2-D;
(maleimid-N-yl)-CH2CH2C(O)NH-(CH2CH2O)₄-CH2CH2-C(=O)-P1-P2-D;
(maleimid-N-yl)-CH2CH2C(O)NH-(CH2CH2O)₈-CH2CH2-C(=O)-P1-P2-D;
(maleimid-N-yl)-OC(O)-(CH2CH2O)₄-CH2CH2-C(=O)-P1-P2-D;
(maleimid-N-yl)-OC(O)-(CH2CH2O)₈-CH2CH2-C(=O)-P1-P2-D;
(maleimid-N-yl)-CH2CH2CH2CH2CH2-C(=O)-P1-P2-D;
(maleimid-N-yl)-CH2-C(=O)-P1-P2-PAB-D;
(maleimid-N-yl)-CH2CH2-C(=O)-P1-P2-PAB-D;
(maleimid-N-yl)-(CH2CH2O)₄-CH2CH2-C(=O)-P1-P2-PAB-D;
(maleimid-N-yl)-(CH2CH2O)₈-CH2CH2-C(=O)-P1-P2-PAB-D;
(maleimid-N-yl)-CH2CH2C(O)NH-(CH2CH2O)₄-CH2CH2-C(=O)-P1-P2-PAB-D;
(maleimid-N-yl)-CH2CH2C(O)NH-(CH2CH2O)₈-CH2CH2-C(=O)-P1-P2-PAB-D;
(maleimid-N-yl)-OC(O)-(CH2CH2O)₄-CH2CH2-C(=O)-P1-P2-PAB-D;
(maleimid-N-yl)-OC(O)-(CH2CH2O)₈-CH2CH2-C(=O)-P1-P2-PAB-D;
(maleimid-N-yl)-CH2CH2CH2CH2CH2-C(=O)-P1-P2-PAB-D;
(N-hydroxysuccinimide ester-1-yl)-CH2-C(=O)-P1-P2-D;
(N-hydroxysuccinimide ester-1-yl)-CH2CH2-C(=O)-P1-P2-D;
(N-hydroxysuccinimide ester-1-yl)-(CH2CH2O)₄-CH2CH2-C(=O)-P1-P2-D;
(N-hydroxysuccinimide ester-1-yl)-(CH2CH2O)₈-CH2CH2-C(=O)-P1-P2-D;
(N-hydroxysuccinimide ester-1-yl)-CH2CH2C(O)NH-(CH2CH2O)₄-CH2CH2-C(=O)-P1-P2-D;
(N-hydroxysuccinimide ester-1-yl)-CH2CH2C(O)NH-(CH2CH2O)₈-CH2CH2-C(=O)-P1-P2-D;
(N-hydroxysuccinimide ester-1-yl)-(1,4-cyclohexylene)-CH2-C(=O)-P1-P2-D;
(N-hydroxysuccinimide ester-1-yl)-(1,4-cyclohexylene)-CH2NH-(CH2CH2O)₄-CH2CH2-C(=O)-P1-P2-D;
(N-hydroxysuccinimide ester-1-yl)-(1,4-cyclohexylene)-CH2NH-(CH2CH2O)₈-CH2CH2-C(=O)-P1-P2-D;
(N-hydroxysuccinimide ester-1-yl)-(1,4-cyclohexylene)-CH2C(=O)NH-(CH2CH2O)₄-CH2CH2-C(=O)-P1-P2-D;
(N-hydroxysuccinimide ester-1-yl)-(1,4-cyclohexylene)-CH2C(=O)NH-(CH2CH2O)₈-CH2CH2-C(=O)-P1-P2-D;
(N-hydroxysuccinimide ester-1-yl)-(1,4-cyclohexylene)-CH2O-(CH2CH2O)₄-CH2CH2-C(= O)-P1-P2-D;
(N-hydroxysuccinimide ester-1-yl)-(1,4-cyclohexylene)-CH2O-(CH2CH2O)₈-CH2CH2-C(= O)-P1-P2-D;
(N-hydroxysuccinimide ester-1-yl)-CH2CH2CH2CH2CH2-C(=O)-P1-P2-D;
(N-hydroxysuccinimide ester-1-yl)-CH2-C(=O)-P1-P2-PAB-D;
(N-hydroxysuccinimide ester-1-yl)-CH2CH2-C(=O)-P1-P2-PAB-D;
(N-hydroxysuccinimide ester-1-yl)-(CH2CH2O)₄-CH2CH2-C(=O)-P1-P2-PAB-D;
(N-hydroxysuccinimide ester-1-yl)-(CH2CH2O)₈-CH2CH2-C(=O)-P1-P2-PAB-D;
(N-hydroxysuccinimide ester-1-yl)-CH2CH2C(O)NH-(CH2CH2O)₄-CH2CH2-C(=O)-P1-P2-PAB-D;
(N-hydroxysuccinimide ester-1-yl)-CH2CH2C(O)NH-(CH2CH2O)₈-CH2CH2-C(=O)-P1-P2-PAB-D;
(N-hydroxysuccinimide ester-1-yl)-(1,4-cyclohexylene)-CH2-C(=O)-P1-P2-PAB-D;
(N-hydroxysuccinimide ester-1-yl)-(1,4-cyclohexylene)-CH2NH-(CH2CH2O)₄-CH2CH2-C(=O)-P1-P2-PAB-D;
(N-hydroxysuccinimide ester-1-yl)-(1,4-cyclohexylene)-CH2NH-(CH2CH2O)₈-CH2CH2-C(=O)-P1-P2-PAB-D;
(N-hydroxysuccinimide ester-1-yl)-(1,4-cyclohexylene)-CH2C(=O)NH-(CH2CH2O)₄-CH2CH2-C(=O)-P1-P2-PAB-D;
(N-hydroxysuccinimide ester-1-yl)-(1,4-cyclohexylene)-CH2C(=O)NH-(CH2CH2O)₈-CH2CH2-C(=O)-P1-P2-PAB-D;
(N-hydroxysuccinimide ester-1-yl)-(1,4-cyclohexylene)-CH2O-(CH2CH2O)₄-CH2CH2-C(= O)-P1-P2-PAB-D;
(N-hydroxysuccinimide ester-1-yl)-(1,4-cyclohexylene)-CH2O-(CH2CH2O)₈-CH2CH2-C(= O)-P1-P2-PAB-D;
(N-hydroxysuccinimide ester-1-yl)-CH2CH2CH2CH2CH2-C(=O)-P1-P2-PAB-D;
Gly-Gly-Gly-NH-CH2CH2CH2CH2CH2-C(=O)-P1-P2-D;
Gly-Gly-Gly-NH-CH2CH2CH2CH2CH2-C(=O)-P1-P2-PAB-D; and 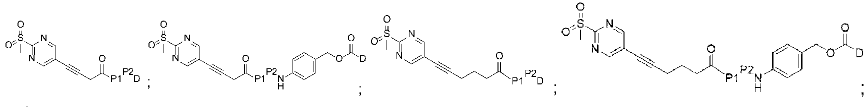 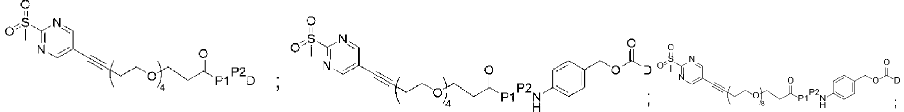 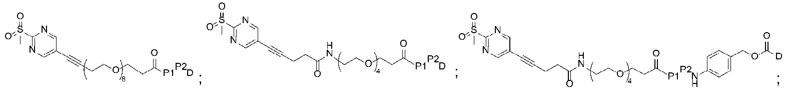 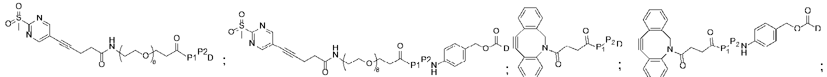 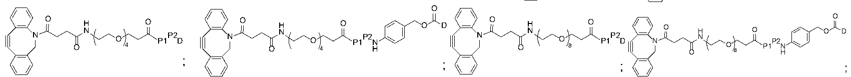 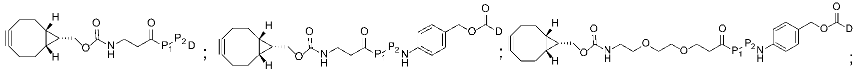 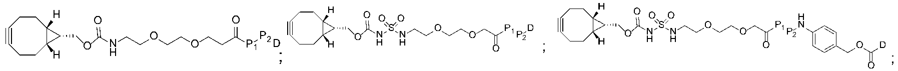
D is camptothecin or a derivative thereof, auristatin or a derivative thereof, a tetrahydroisoquinoline alkaloid or a derivative thereof, a protein degrading agent and a derivative thereof, preferably DX8951, MMAE, Dxd, SN38, ET743, Lurbinectedin, CC885, or CC-90009, and more preferably DX8951, MMAE, Dxd, SN38, or ET743.

32. The linker drug according to any one of claims 21 to 31, wherein the linker drug has the following structure:
MC-GPAGLVCit-PAB-DX8951;
MC-GPAGLVCit-PAB-MMAE;
MC-GPAGLVCit-PAB-ET743;
MC-GPLGLGGFG-DX8951;
MC-GPASLVCit-PAB-DX8951;
MC-GPRGLVCit-PAB-DX8951;
MC-GPRGLVCit-PAB-MMAE;
MC-GPSALVCit-PAB-DX8951;
MC-GPRGLGGFG-DX8951;
MC-GPAALGGFG-DX8951;
MC-PRNLVCit-PAB-DX8951;
MC-PRNLVCit-PAB-MMAE;
MC-GPRNLVCit-PAB-DX8951;
MC-GPRNLVCit-PAB-MMAE;
MC-GPRNLVCit-PAB-ET743;
MC-GPRNDap(Me2)VCit-PAB-DX8951;
MC-GPRNDap(Me2)VCit-PAB-MMAE;
MC-SPRNLVCit-PAB-DX8951;
MC-SPRNLVCit-PAB-MMAE;
MC-GPLSLVCit-PAB-DX8951;
MC-GPLSLVCit-PAB-MMAE;
MC-PRGLVCit-PAB-DX8951;
MC-PRPLVCit-PAB-DX8951;
MC-GPRSLVCit-PAB-DX8951;
MC-GPRPLVCit-PAB-DX8951;
MC-GPCitPLVCit-PAB-DX8951;
MC-HPRGLVCit-PAB-DX8951;
MC-HPCitPLVCit-PAB-DX8951;
MC-SPCitGLVCit-PAB-DX8951;
MC-GPAPLVCit-PAB-DX8951;
MC-PLSLVCit-PAB-DX8951;
MC-SPCitSLVCit-PAB-DX8951;
MC-SPCitSLVCit-PAB-MMAE;
MC-SPCitSLVCit-PAB-ET743;
MeO2S-Pym-GPRNLVCit-PAB-DX8951;
DBCO-GPRNLVCit-PAB-DX8951;
NHS-GPRNLVCit-PAB-DX8951;
Hydrazide-GPRNLVCit-PAB-DX8951;
Mal-PEG8-GPRNLVCit-PAB-DX8951;
MC-GPRNLVCit-PAB-DMEDA-SN38;
MC-GPRNLGGFG-Dxd;
MC-GPRNLGGFG-NMEDA-Dxd;
MC-PRSLVCit-PAB-DX8951;
MC-SPRSLVCit-PAB-DX8951
MC-PCitSLVCit-PAB-DX8951;
MC-PCitPLVCit-PAB-DX8951;
MC-PCitGLVCit-PAB-DX8951;
MC-GPCitGLVCit-PAB-DX8951;
MC-SPRGLVCit-PAB-DX8951;
MC-SPRPLVCit-PAB-DX8951;
MC-SPCitPLVCit-PAB-DX8951;
MC-GPANLVCit-PAB-DX8951;
MC-GPCitNLVCit-PAB-DX8951;
MC-SPCitSDap(Me2)VCit-PAB-DX8951;
MC-SPCitSDap(Me2)VCit-PAB-MMAE;
MC-PRSDap(Me2)VCit-PAB-DX8951;
MC-PRSDap(Me2)VCit-PAB-MMAE;
MC-PCitNLVCit-PAB-DX8951; and
MC-PASLVCit-PAB-DX8951;
preferably, the linker drug has the following structure:
MC-GPAGLVCit-PAB-DX8951;
MC-GPLGLGGFG-DX8951;
MC-GPASLVCit-PAB-DX8951;
MC-GPRGLVCit-PAB-DX8951;
MC-GPSALVCit-PAB-DX8951;
MC-GPRGLGGFG-DX8951;
MC-GPAALGGFG-DX8951;
MC-PRNLVCit-PAB-DX8951;
MC-GPRNLVCit-PAB-DX8951;
MC-GPRGDap(Me2)LVCit-PAB-DX8951;
MC-SPRNLVCit-PAB-DX8951;
MC-GPLSLVCit-PAB-DX8951;
MC-PRGLVCit-PAB-DX8951;
MC-PRPLVCit-PAB-DX8951;
MC-GPRSLVCit-PAB-DX8951;
MC-GPRPLVCit-PAB-DX8951;
MC-GPCitPLVCit-PAB-DX8951;
MC-HPRGLYCit-PAB-DX8951;
MC-HPCitPLVCit-PAB-DX8951;
MC-SPCitGLVCit-PAB-DX8951;
MC-GPAPLVCit-PAB-DX8951;
MC-PLSLVCit-PAB-DX8951;
MC-SPCitSLVCit-PAB-DX8951;
MC-PRSLYCit-PAB-DX8951;
MC-SPRSLVCit-PAB-DX8951;
MC-PCitSLVCit-PAB-DX8951;
MC-PCitPLVCit-PAB-DX8951;
MC-PCitGLYCit-PAB-DX8951;
MC-GPCitGLVCit-PAB-DX8951;
MC-SPRGLVCit-PAB-DX8951;
MC-SPRPLVCit-PAB-DX8951;
MC-SPCitPLVCit-PAB-DX8951;
MC-GPANLVCit-PAB-DX8951;
MC-GPCitNLYCit-PAB-DX8951;
MC-SPCitSDap(Me2)VCit-PAB-DX8951;
MC-PRSDap(Me2)VCit-PAB-DX8951;
MC-GPAGLVCit-PAB-MMAE;
MC-GPRGLVCit-PAB-MMAE;
MC-PRNLVCit-PAB-MMAE;
MC-GPRNLVCit-PAB-MMAE;
MC-GPRGDap(Me2)LVCit-PAB-MMAE;
MC-SPRNLVCit-PAB-MMAE;
MC-GPLSLVCit-PAB-MMAE;
MC-SPCitSLVCit-PAB-MMAE
MC-SPCitSDap(Me2)VCit-PAB-MMAE
MC-SPCitSDap(Me2)VCit-PAB-MMAE
MC-GPAGLVCit-PAB-ET743;
MC-GPRNLVCit-PAB-ET743; or
MC-SPCitSLVCit-PAB-ET743.

33. A bioligand-drug conjugate comprising the linker according to any one of claims 9 to 20, wherein the conjugate has a general formula of 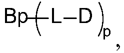 wherein:
Bp is a bioligand;
L is the linker;
D is a drug unit; and
p is any integer from 1 to 20.

34. The conjugate according to claim 33, wherein the conjugate has a general formula: 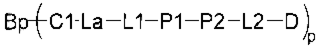 wherein:
Bp is a bioligand;
C1-La-L1-P1-P2-L2 is the linker;
D is a drug unit; and
p is any integer from 1 to 20.

35. The conjugate according to claim 33 or 34, wherein the drug unit D is as defined in any one of claims 26 to 31.

36. The conjugate according to any one of claims 33 to 35, wherein the bioligand targets a cell surface receptor or a tumor-associated antigen, wherein the cell surface receptor or the tumor-associated antigen comprises: EGFR, HER2, HER3, c-Met, claudin 18.2, TROP-2, folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, IGF-1R, TNFR1, or GLP-1.

37. The conjugate according to any one of claims 33 to 36, wherein the bioligand comprises an antibody or an antigen-binding fragment thereof, a modified antibody or an antigen-binding fragment thereof, an oligonucleotide, a DNA fragment, a nucleic acid aptamer, a polypeptide, a nanoparticle with targeting ability, or a chemically-synthesized small molecule.

38. The conjugate according to claim 37, wherein the antigen-binding fragment comprises a Fab, a Fab', a Fv fragment, a F(ab')2, a scFv, a di-scFv, and/or a dAb.

39. The conjugate according to claim 37 or 38, wherein the antibody or the antigen-binding fragment thereof, the modified antibody or the modified antigen-binding fragment thereof is an anti-EGFR, anti-HER2, anti-HER3, anti-c-Met, anti-claudin18.2, anti-TROP-2, anti-FRα, anti-ROR1, anti-ROR2, anti-BCMA, anti-PSMA, anti-CD19, anti-CD20, anti-CD22, anti-CD30, anti-CD33, anti-CD37, anti-CD46, anti-CD48, anti-CD56, anti-CD79b, anti-CD123, anti-CD138, anti-CD166, anti-CD276, anti-CEACAM5, anti-SDC1, anti-CD74, anti-CD70, anti-MUC1, anti-MUC16, anti-GUCY2C, anti-MSLN, anti-SCL34A2, anti-FOLH1, anti-TPBC, anti-PVRL4, anti-STEAP1, anti-SCL44A4, anti-NACM1, anti-EDNRB, anti-GPNMB, anti-FGFR, anti-SEZ6, anti-Nectin-4, anti-PD-L1, anti-TF, anti-B7-H3, anti-B7-H4, anti-LY6E, anti-LIV-1, anti-CDH4, anti-CDH6, anti-ITGB6, anti-GPC1, anti-GPC3, anti-ENPP3, anti-KAAG1, anti-FZD7, anti-SSTR2, anti-DLK1, anti-TMEFF1, anti-TM4SF1, anti-SLAMF7, anti-DLL3, anti-FLT3, anti-TNFRSF10B, anti-EPCAM, anti-AXL, anti-IL2RA, anti-5T4, anti-FAP, anti-ICAM-1, anti-IGF-1R, anti-TNFR1 or anti-GLP-1 antibody, or an antigen-binding fragment thereof.

40. The conjugate according to any one of claims 37 to 39, wherein the antibody or the antigen-binding fragment thereof, the modified antibody or the modified antigen-binding fragment thereof is an anti-Her2, anti-TF, anti-ROR1, or anti-GPC3 antibody or an antigen-binding fragment thereof.

41. The conjugate according to any one of claims 37 to 40, wherein the antibody is Trastuzumab, Tisotumab, Zilovertamab, or Codrituzumab.

42. The conjugate according to any one of claims 33 to 41, wherein the conjugate is an antibody-drug conjugate, and the antibody-drug conjugate is selected from: 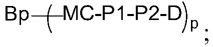 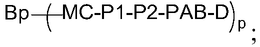 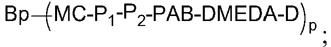 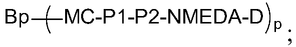 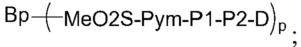 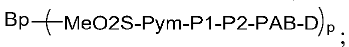 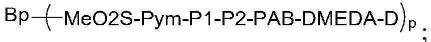 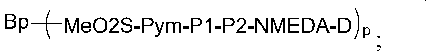 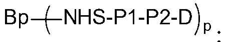 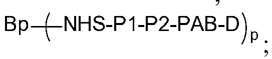 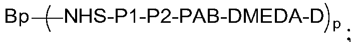 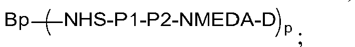 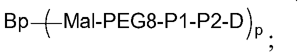 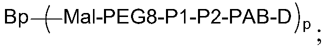 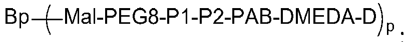 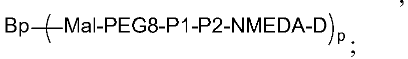 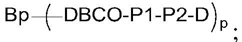 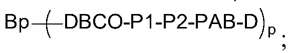 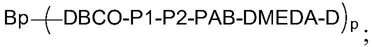 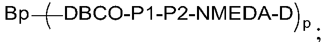 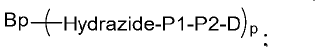 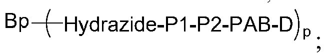 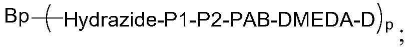 and 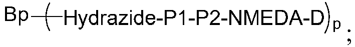 preferably, the antibody-drug conjugate is selected from the following structures:
Ab-((succinimid-3-yl-N)-C5 alkylene-C(=O)-P1-P2-D)ₙ;
Ab-((succinimid-3-yl-N)-C5 alkylene-C(=O)-P1-P2-PAB-D)ₙ;
Ab-((succinimid-3-yl-N)-C5 alkylene-C(=O)-P1-P2-PAB-DMEDA-D)ₙ;
Ab-((succinimid-3-yl-N)-C5 alkylene-C(=O)-P1-P2-NMEDA-D)ₙ; 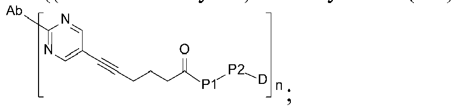 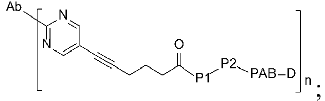 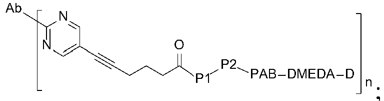 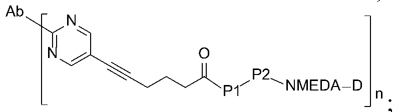
Ab-((C=O)-C6 alkylene-C(=O)-D)ₙ ;
Ab-((C=O)-C6 alkylene-C(=O)-P1-P2-PAB-D)ₙ;
Ab-((C=O)-C6 alkylene-C(=O)-P1-P2-PAB-DMEDA-D)ₙ;
Ab-((C=O)-C6 alkylene-C(=O)-P1-P2-NMEDA-D)ₙ;
Ab-((succinimid-3-yl-N)-C2 alkylene-C(=O)NH-(CH₂CH₂O)₈-C2 alkylene-C(=O)-P1-P2-D)ₙ;
Ab-((succinimid-3-yl-N)-C2 alkylene-C(=O)NH-(CH₂CH₂O)₈-C2 alkylene-C(=O)-P1-P2-PAB-D)ₙ;
Ab-((succinimid-3-yl-N)-C2 alkylene-C(=O)NH-(CH₂CH₂O)₈-C2 alkylene-C(=O)-P1-P2-PAB-DMEDA-D)ₙ; and
Ab-((succinimid-3-yl-N)-C2 alkylene-C(=O)NH-(CH₂CH₂O)₈-C2 alkylene-C(=O)-P1-P2-NMEDA-D)ₙ; 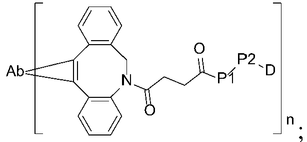 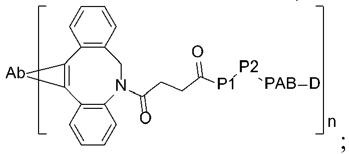 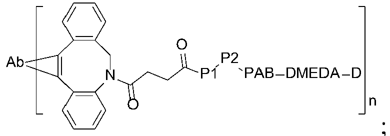 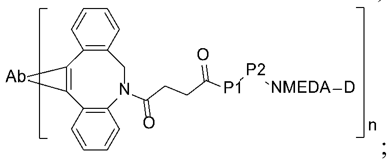 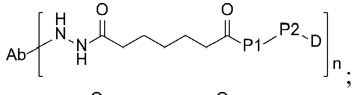 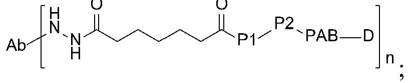 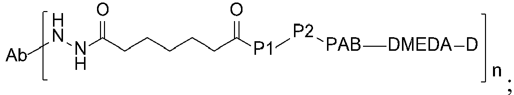
and 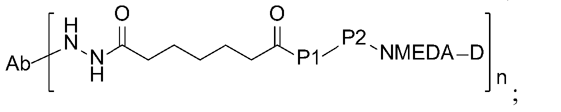
Ab represents a monoclonal antibody or an antigen-binding fragment thereof, or a modified monoclonal antibody or an antigen-binding fragment thereof, preferably an anti-Her2, anti-TF, anti-ROR1 or anti-GPC3 antibody or an antigen-binding fragment thereof; D is camptothecin or a derivative thereof, auristatin or a derivative thereof, a tetrahydroisoquinoline alkaloid or a derivative thereof, or a protein degrading agent and a derivative thereof, and preferably DX8951, MMAE, Dxd, SN38, ET743, Lurbinectedin, CC885, or CC-90009; and n represents any value from 1 to 10.

43. The conjugate according to any one of claims 33 to 42, wherein the conjugate is an antibody-drug conjugate, and the antibody-drug conjugate is selected from the following structures:
Ab-((succinimid-3-yl-N)-CH2-C(=O)-P1-P2-D)ₙ;
Ab-((succinimid-3-yl-N)-CH2CH2-C(=O)-P1-P2-D)ₙ;
Ab-((succinimid-3-yl-N)-(CH2CH2O)₄-CH2CH2-C(=O)-P1-P2-D)ₙ;
Ab-((succinimid-3-yl-N)-(CH2CH2O)₈-CH2CH2-C(=O)-P1-P2-D)ₙ;
Ab-((succinimid-3-yl-N)-CH2CH2C(O)NH-(CH2CH2O)₄-CH2CH2-C(=O)-P1-P2-D)ₙ;
Ab-((succinimid-3-yl-N)-CH2CH2C(O)NH-(CH2CH2O)₈-CH2CH2-C(=O)-P1-P2-D)ₙ;
Ab-((succinimid-3-yl-N)-OC(O)-(CH2CH2O)₄-CH2CH2-C(=O)-P1-P2-D)ₙ;
Ab-((succinimid-3-yl-N)-OC(O)-(CH2CH2O)₈-CH2CH2-C(=O)-P1-P2-D)ₙ;
Ab-((succinimid-3-yl-N)-CH2CH2CH2CH2CH2-C(=O)-P1-P2-D)ₙ;
Ab-((succinimid-3-yl-N)-CH2-C(=O)-P1-P2-PAB-D)ₙ;
Ab-((succinimid-3-yl-N)-CH2CH2-C(=O)-P1-P2-PAB-D)ₙ;
Ab-((succinimid-3-yl-N)-(CH2CH2O)₄-CH2CH2-C(=O)-P1-P2-PAB-D)ₙ;
Ab-((succinimid-3-yl-N)-(CH2CH2O)₈-CH2CH2-C(=O)-P1-P2-PAB-D)ₙ;
Ab-((succinimid-3-yl-N)-CH2CH2C(O)NH-(CH2CH2O)₄-CH2CH2-C(=O)-P1-P2-PAB-D)ₙ;
Ab-((succinimid-3-yl-N)-CH2CH2C(O)NH-(CH2CH2O)₈-CH2CH2-C(=O)-P1-P2-PAB-D)ₙ;
Ab-((succinimid-3-yl-N)-OC(O)-(CH2CH2O)₄-CH2CH2-C(=O)-P1-P2-PAB-D)ₙ;
Ab-((succinimid-3-yl-N)-OC(O)-(CH2CH2O)₈-CH2CH2-C(=O)-P1-P2-PAB-D)ₙ;
Ab-((succinimid-3-yl-N)-CH2CH2CH2CH2CH2-C(=O)-P1-P2-PAB-D)ₙ; 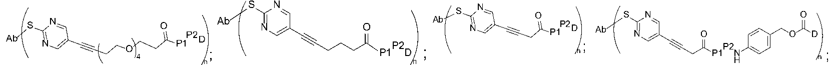 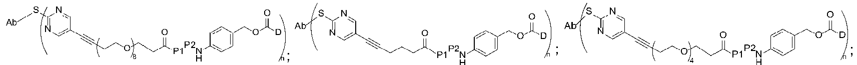 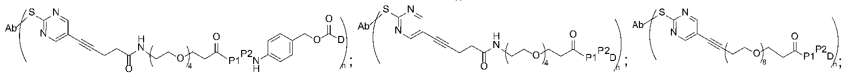 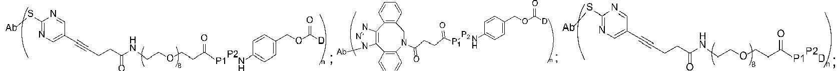 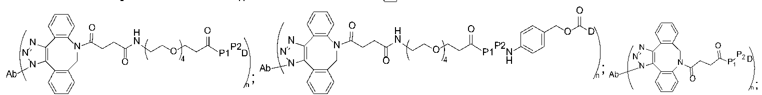 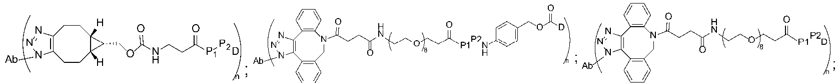 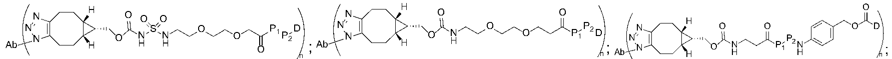 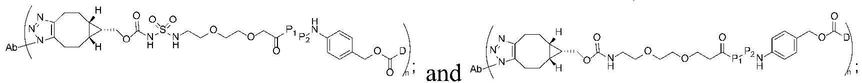
Ab represents a monoclonal antibody or an antigen-binding fragment thereof, or a modified monoclonal antibody or an antigen-binding fragment thereof, and preferably an anti-Her2, anti-TF, anti-ROR1, or anti-GPC3 antibody or an antigen-binding fragment thereof;
-P1-P2- represents the following structure:
-His-Val-Leu-Asn-Leu-Val-Cit- (SEQ ID NO: 1);
-Val-Pro-Leu-Ser-Leu-Val-Cit- (SEQ ID NO: 2);
-Ile-Pro-Val-Ser-Leu-Val-Cit- (SEQ ID NO: 3);
-Gly-Pro-Leu-Ser-Leu-Val-Cit- (SEQ ID NO: 4);
-Gly-Pro-Ala-Ser-Leu-Val-Cit- (SEQ ID NO: 5);
-Gly-Pro-Ala-Gly-Leu-Val-Cit- (SEQ ID NO: 6);
-Gly-Pro-Gln-Gly-Leu-Val-Cit- (SEQ ID NO: 7);
-Gly-Pro-Leu-Gly-Leu-Val-Cit- (SEQ ID NO: 8);
-Gly-Pro-Ser-Gly-Leu-Val-Cit- (SEQ ID NO: 9);
-Gly-Pro-Arg-Gly-Leu-Val-Cit- (SEQ ID NO: 10);
-Gly-Pro-Ala-Ala-Leu-Val-Cit- (SEQ ID NO: 11);
-Gly-Pro-Ser-Ala-Leu-Val-Cit- (SEQ ID NO: 12);
-His-Val-Leu-Asn-Leu-Gly-Gly-Phe-Gly- (SEQ ID NO: 13);
-Val-Pro-Leu-Ser-Leu-Gly-Gly-Phe-Gly- (SEQ ID NO: 14);
- Ile-Pro-Val-Ser-Leu-Gly-Gly-Phe-Gly- (SEQ ID NO: 15);
-Gly-Pro-Leu-Ser-Leu-Gly-Gly-Phe-Gly- (SEQ ID NO: 16);
-Gly-Pro-Ala-Ser-Leu-Gly-Gly-Phe-Gly- (SEQ ID NO: 17);
-Gly-Pro-Ala-Gly-Leu-Gly-Gly-Phe-Gly- (SEQ ID NO: 18);
-Gly-Pro-Gln-Gly-Leu-Gly-Gly-Phe-Gly- (SEQ ID NO: 19);
-Gly-Pro-Leu-Gly-Leu-Gly-Gly-Phe-Gly- (SEQ ID NO: 20);
-Gly-Pro-Ser-Gly-Leu-Gly-Gly-Phe-Gly- (SEQ ID NO: 21);
-Gly-Pro-Arg-Gly-Leu-Gly-Gly-Phe-Gly- (SEQ ID NO: 22);
-Gly-Pro-Ala-Ala-Leu-Gly-Gly-Phe-Gly- (SEQ ID NO: 23);
-Gly-Pro-Ser-Ala-Leu-Gly-Gly-Phe-Gly- (SEQ ID NO: 24);
-Pro-Arg-Gly-Leu-Val-Cit- (SEQ ID NO: 25);
-Pro-Arg-Ser-Leu-Val-Cit- (SEQ ID NO: 26);
-Pro-Arg-Asn-Leu-Val-Cit- (SEQ ID NO: 27);
-Pro-Arg-Pro-Leu-Val-Cit- (SEQ ID NO: 28);
-Pro-Cit-Gly-Leu-Val-Cit- (SEQ ID NO: 29);
-Pro-Cit-Pro-Leu-Val-Cit- (SEQ ID NO: 30);
-Gly-Pro-Arg-Ser-Leu-Val-Cit- (SEQ ID NO: 31);
-Gly-Pro-Arg-Pro-Leu-Val-Cit- (SEQ ID NO: 32);
-Gly-Pro-Arg-Asn-Leu-Val-Cit- (SEQ ID NO: 33);
-Gly-Pro-Cit-Gly-Leu-Val-Cit- (SEQ ID NO: 34);
-Gly-Pro-Cit-Pro-Leu-Val-Cit- (SEQ ID NO: 35);
-His-Pro-Arg-Gly-Leu-Val-Cit- (SEQ ID NO: 36);
-His-Pro-Arg-Ser-Leu-Val-Cit- (SEQ ID NO: 37);
-His-Pro-Arg-Pro-Leu-Val-Cit- (SEQ ID NO: 38);
-His-Pro-Arg-Asn-Leu-Val-Cit- (SEQ ID NO: 39);
-His-Pro-Cit-Gly-Leu-Val-Cit- (SEQ ID NO: 40);
-His-Pro-Cit-Pro-Leu-Val-Cit- (SEQ ID NO: 41);
-Ser-Pro-Arg-Gly-Leu-Val-Cit- (SEQ ID NO: 42);
-Ser-Pro-Arg-Ser-Leu-Val-Cit- (SEQ ID NO: 43);
-Ser-Pro-Arg-Pro-Leu-Val-Cit- (SEQ ID NO: 44);
-Ser-Pro-Arg-Asn-Leu-Val-Cit- (SEQ ID NO: 45);
-Ser-Pro-Cit-Gly-Leu-Val-Cit- (SEQ ID NO: 46);
-Ser-Pro-Cit-Ser-Leu-Val-Cit- (SEQ ID NO: 47);
-Ser-Pro-Cit-Pro-Leu-Val-Cit- (SEQ ID NO: 48);
-Gly-Pro-Ala-Pro-Leu-Val-Cit- (SEQ ID NO: 49);
-Gly-Pro-Ala-Asn-Leu-Val-Cit- (SEQ ID NO: 50);
-Gly-Pro-Arg-Gly-Dap(Me2)-Val-Cit- (SEQ ID NO: 51);
-Gly-Pro-Arg-Asn-Dap(Me2)-Val-Cit- (SEQ ID NO: 60);
-Pro-Leu-Ser-Leu-Val-Cit- (SEQ ID NO: 61);
-Gly-Pro-Arg-Asn-Leu-Gly-Gly-Phe-Gly- (SEQ ID NO: 62);
-Pro-Cit-Ser-Leu-Val-Cit- (SEQ ID NO: 63);
-Gly-Pro-Cit-Asn-Leu-Val-Cit- (SEQ ID NO: 64);
-Ser-Pro-Cit-Ser-Dap(Me2)-Val-Cit- (SEQ ID NO: 65);
-Pro-Arg-Ser-Dap(Me2)-Val-Cit- (SEQ ID NO: 66);
-Pro-Cit-Asn-Leu-Val-Cit- (SEQ ID NO: 67); or
-Pro-Ala-Ser-Leu-Val-Cit- (SEQ ID NO: 68);
D is camptothecin or derivatives thereof, auristatin or a derivative thereof, a tetrahydroisoquinoline alkaloid or a derivative thereof, a protein degrading agent or derivatives thereof, and preferably DX8951, MMAE, Dxd, SN38, ET743, Lurbinectedin, CC885, or CC-90009; and
n represents an integer from 1 to 10.

44. The conjugate according to any one of claims 33 to 43, wherein the conjugate is an antibody-drug conjugate, and the antibody-drug conjugate is selected from the following structures:
Ab-((succinimid-3-yl-N)-C5 alkylene-C(=O)-GPAGLVCit-PAB-D)ₙ;
Ab-((succinimid-3-yl-N)-C5 alkylene-C(=O)-GPLGLGGFG-D)ₙ;
Ab-((succinimid-3-yl-N)-C5 alkylene-C(=O)-GPASLVCit-PAB-D)ₙ;
Ab-((succinimid-3-yl-N)-C5 alkylene-C(=O)-GPRGLVCit-PAB-D)ₙ;
Ab-((succinimid-3-yl-N)-C5 alkylene-C(=O)-GPSALVCit-PAB-D)ₙ;
Ab-((succinimid-3-yl-N)-C5 alkylene-C(=O)-GPRGLGGFG-D)ₙ;
Ab-((succinimid-3-yl-N)-C5 alkylene-C(=O)-GPAALGGFG-D)ₙ;
Ab-((succinimid-3-yl-N)-C5 alkylene-C(=O)-PRNLVCit-PAB-D)ₙ;
Ab-((succinimid-3-yl-N)-C5 alkylene-C(=O)-GPRNLVCit-PAB-D)ₙ;
Ab-((succinimid-3-yl-N)-C5 alkylene-C(=O)-GPRNDap(Me2)VCit-PAB-D)ₙ;
Ab-((succinimid-3-yl-N)-C5 alkylene-C(=O)-SPRNLVCit-PAB- D)ₙ;
Ab-((succinimid-3-yl-N)-C5 alkylene-C(=O)-GPLSLVCit-PAB-D)ₙ;
Ab-((succinimid-3-yl-N)-C5 alkylene-C(=O)-PRGLVCit-PAB-D)ₙ;
Ab-((succinimid-3-yl-N)-C5 alkylene-C(=O)-PRPLVCit-PAB-D)ₙ;
Ab-((succinimid-3-yl-N)-C5 alkylene-C(=O)-GPRSLVCit-PAB-D)ₙ;
Ab-((succinimid-3-yl-N)-C5 alkylene-C(=O)-GPRPLVCit-PAB-D)ₙ;
Ab-((succinimid-3-yl-N)-C5 alkylene-C(=O)-GPCitPLVCit-PAB-D)ₙ;
Ab-((succinimid-3-yl-N)-C5 alkylene-C(=O)-HPRGLVCit-PAB-D)ₙ;
Ab-((succinimid-3-yl-N)-C5 alkylene-C(=O)-HPCitPLVCit-PAB-D)ₙ;
Ab-((succinimid-3-yl-N)-C5 alkylene-C(=O)-SPCitGLVCit-PAB-D)ₙ;
Ab-((succinimid-3-yl-N)-C5 alkylene-C(=O)-GPAPLVCit-PAB-D)ₙ;
Ab-((succinimid-3-yl-N)-C5 alkylene-C(=O)-PLSLVCit-PAB-D)ₙ;
Ab-((succinimid-3-yl-N)-C5 alkylene-C(=O)-SPCitSLVCit-PAB-D)ₙ; 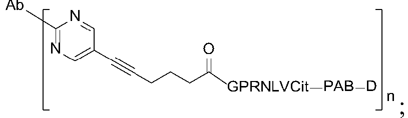 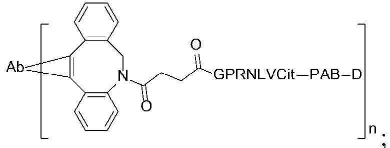
Ab-((C=O)-C6 alkylene-C(=O)-GPRNLVCit-PAB-D)ₙ; 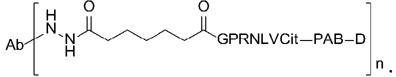
Ab-((succinimid-3-yl-N)-C2 alkylene-C(=O)NH-(CH₂CH₂O)₈-C2 alkylene-C(=O)-GPRNLVCit-PAB-D)ₙ;
Ab-((succinimid-3-yl-N)-C5 alkylene-C(=O)-GPRNLVCit-PAB-DMEDA-D)ₙ;
Ab-((succinimid-3-yl-N)-C5 alkylene-C(=O)-GPRNLGGFG-D)ₙ;
Ab-((succinimid-3-yl-N)-C5 alkylene-C(=O)-GPRNLGGFG-NMEDA-D)ₙ;
Ab-((succinimid-3-yl-N)-C5 alkylene-C(=O)-PRSLUCit-PAB-D)ₙ;
Ab-((succinimid-3-yl-N)-C5 alkylene-C(=O)-SPRSLVCit-PAB-D)ₙ;
Ab-((succinimid-3-yl-N)-C5 alkylene-C(=O)-PCitSLVCit-PAB-D)ₙ;
Ab-((succinimid-3-yl-N)-C5 alkylene-C(=O)-PCitPLVCit-PAB-D)ₙ;
Ab-((succinimid-3-yl-N)-C5 alkylene-C(=O)-PCitGLVCit-PAB-D)ₙ;
Ab-((succinimid-3-yl-N)-C5 alkylene-C(=O)-GPCitGLVCit-PAB-D)ₙ;
Ab-((succinimid-3-yl-N)-C5 alkylene-C(=O)-SPRGLVCit-PAB-D)ₙ;
Ab-((succinimid-3-yl-N)-C5 alkylene-C(=O)-SPRPLVCit-PAB-D)ₙ;
Ab-((succinimid-3-yl-N)-C5-alkylene-C(=O)-SPCitPLVCit-PAB-D)ₙ;
Ab-((succinimid-3-yl-N)-C5-alkylene-C(=O)-GPANLVCit-PAB-D)ₙ;
Ab-((succinimid-3-yl-N)-C5-alkylene-C(=O)-GPCitNLVCit-PAB-D)ₙ;
Ab-((succinimid-3-yl-N)-C5-alkylene-C(=O)-SPCitSDap(Me2)VCit-PAB-D)ₙ;
Ab-((succinimid-3-yl-N)-C5-alkylene-C(=O)-PRSDap(Me2)VCit-PAB-D)ₙ;
Ab-((succinimid-3-yl-N)-C5-alkylene-C(=O)-PCitNLVCit-PAB-D)ₙ; and
Ab-((succinimid-3-yl-N)-C5-alkylene-C(=O)-PASLVCit-PAB-D)ₙ;
Ab is an anti-Her2, anti-TF, anti-ROR1, or anti-GPC3 antibody or an antigen-binding fragment thereof; D is DX8951, MMAE, Dxd, SN38, or ET743; and n represents any value selected from 2 to 9, and preferably from 2 to 8;
preferably, the antibody-drug conjugate is selected from the following structures:
Ab-((succinimid-3-yl-N)-C5-alkylene-C(=O)-GPAGLVCit-PAB-DX8951)ₙ;
Ab-((succinimid-3-yl-N)-C5-alkylene-C(=O)-GPAGLVCit-PAB-MMAE)ₙ;
Ab-((succinimid-3-yl-N)-C5-alkylene-C(=O)-GPAGLVCit-PAB-ET743)ₙ;
Ab-((succinimid-3-yl-N)-C5-alkylene-C(=O)-GPLGLGGFG-DX8951)ₙ;
Ab-((succinimid-3-yl-N)-C5-alkylene-C(=O)-GPASLVCit-PAB-DX8951)ₙ;
Ab-((succinimid-3-yl-N)-C5-alkylene-C(=O)-GPRGLVCit-PAB-DX8951)ₙ;
Ab-((succinimid-3-yl-N)-C5-alkylene-C(=O)-GPRGLVCit-PAB-MMAE)ₙ;
Ab-((succinimid-3-yl-N)-C5-alkylene-C(=O)- GPSALVCit-PAB-DX8951)ₙ;
Ab-((succinimid-3-yl-N)-C5-alkylene-C(=O)- GPRGLGGFG-DX8951)ₙ;
Ab-((succinimid-3-yl-N)-C5 alkylene-C(=O)-GPAALGGFG-DX8951)ₙ;
Ab-((succinimid-3-yl-N)-C5-alkylene-C(=O)-PRNLVCit-PAB-DX8951)ₙ;
Ab-((succinimid-3-yl-N)-C5-alkylene-C(=O)-PRNLVCit-PAB-MMAE)ₙ;
Ab-((succinimid-3-yl-N)-C5-alkylene-C(=O)-GPRNLVCit-PAB-DX8951)ₙ;
Ab-((succinimid-3-yl-N)-C5-alkylene-C(=O)-GPRNLVCit-PAB-MMAE)ₙ;
Ab-((succinimid-3-yl-N)-C5-alkylene-C(=O)-GPRNLVCit-PAB-ET743)ₙ;
Ab-((succinimid-3-yl-N)-C5-alkylene-C(=O)-GPRNDap(Me2)VCit-PAB-DX8951)ₙ;
Ab-((succinimid-3-yl-N)-C5-alkylene-C(=O)-GPRNDap(Me2)VCit-PAB-MMAE)ₙ;
Ab-((succinimid-3-yl-N)-C5-alkylene-C(=O)-SPRNLVCit-PAB-DX8951)ₙ;
Ab-((succinimid-3-yl-N)-C5-alkylene-C(=O)-SPRNLVCit-PAB-MMAE)ₙ;
Ab-((succinimid-3-yl-N)-C5-alkylene-C(=O)-GPLSLYCit-PAB-DX8951)ₙ;
Ab-((succinimid-3-yl-N)-C5-alkylene-C(=O)-GPLSLVCit-PAB-MMAE)ₙ;
Ab-((succinimid-3-yl-N)-C5-alkylene-C(=O)-PRGLVCit-PAB-DX8951)ₙ;
Ab-((succinimid-3-yl-N)-C5-alkylene-C(=O)-PRPLVCit-PAB-DX8951)ₙ;
Ab-((succinimid-3-yl-N)-C5-alkylene-C(=O)-GPRSLVCit-PAB-DX8951)ₙ;
Ab-((succinimid-3-yl-N)-C5-alkylene-C(=O)-GPRPLVCit-PAB-DX8951)ₙ;
Ab-((succinimid-3-yl-N)-C5-alkylene-C(=O)-GPCitPLVCit-PAB-DX8951)ₙ;
Ab-((succinimid-3-yl-N)-C5-alkylene-C(=O)-HPRGLVCit-PAB-DX8951)ₙ;
Ab-((succinimid-3-yl-N)-C5-alkylene-C(=O)-HPCitPLVCit-PAB-DX8951)ₙ;
Ab-((succinimid-3-yl-N)-C5-alkylene-C(=O)-SPCitGLVCit-PAB-DX8951)ₙ;
Ab-((succinimid-3-yl-N)-C5-alkylene-C(=O)-GPAPLVCit-PAB-DX8951)ₙ;
Ab-((succinimid-3-yl-N)-C5-alkylene-C(=O)-PLSLVCit-PAB-DX8951)ₙ;
Ab-((succinimid-3-yl-N)-C5-alkylene-C(=O)-SPCitSLVCit-PAB-DX8951)ₙ;
Ab-((succinimid-3-yl-N)-C5-alkylene-C(=O)-SPCitSLVCit-PAB-MMAE)ₙ;
Ab-((succinimid-3-yl-N)-C5-alkylene-C(=O)-SPCitSLVCit-PAB-ET743)ₙ; 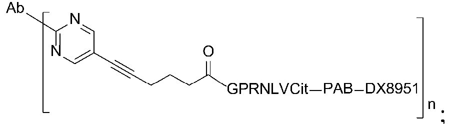 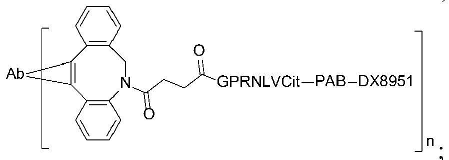
Ab-((C=O)-C6-alkylene-C(=O)-GPRNLVCit-PAB-DX8951)ₙ; 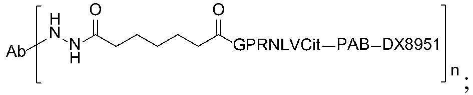
Ab-((succinimid-3-yl-N)-C2-alkylene-C(=O)NH-(CH₂CH₂O)₈-C2-alkylene-C(=O)-GPRNLVCit-PAB-DX8951)ₙ;
Ab-((succinimid-3-yl-N)-C5-alkylene-C(=O)-GPRNLVCit-PAB-DMEDA-SN38)ₙ;
Ab-((succinimid-3-yl-N)-C5-alkylene-C(=O)-GPRNLGGFG-Dxd)ₙ;
Ab-((succinimid-3-yl-N)-C5-alkylene-C(=O)-GPRNLGGFG-NMEDA-Dxd)ₙ;
Ab-((succinimid-3-yl-N)-C5-alkylene-C(=O)-PRSLVCit-PAB-DX8951)ₙ;
Ab-((succinimid-3-yl-N)-C5-alkylene-C(=O)-SPRSLVCit-PAB-DX8951)ₙ;
Ab-((succinimid-3-yl-N)-C5-alkylene-C(=O)-PCitSLVCit-PAB-DX8951)ₙ;
Ab-((succinimid-3-yl-N)-C5-alkylene-C(=O)-PCitPLVCit-PAB-DX8951)ₙ;
Ab-((succinimid-3-yl-N)-C5-alkylene-C(=O)-PCitGLVCit-PAB-DX8951)ₙ;
Ab-((succinimid-3-yl-N)-C5-alkylene-C(=O)-GPCitGLVCit-PAB-DX8951)ₙ;
Ab-((succinimid-3-yl-N)-C5-alkylene-C(=O)-SPRGLVCit-PAB-DX8951)ₙ;
Ab-((succinimid-3-yl-N)-C5-alkylene-C(=O)-SPRPLVCit-PAB-DX8951)ₙ;
Ab-((succinimid-3-yl-N)-C5-alkylene-C(=O)-SPCitPLVCit-PAB-DX8951)ₙ;
Ab-((succinimid-3-yl-N)-C5-alkylene-C(=O)-GPANLVCit-PAB-DX8951)ₙ;
Ab-((succinimid-3-yl-N)-C5-alkylene-C(=O)-GPCitNLVCit-PAB-DX8951)ₙ;
Ab-((succinimid-3-yl-N)-C5-alkylene-C(=O)-SPCitSDap(Me2)VCit-PAB-DX8951)ₙ;
Ab-((succinimid-3-yl-N)-C5-alkylene-C(=O)-SPCitSDap(Me2)VCit-PAB-MMAE)ₙ;
Ab-((succinimid-3-yl-N)-C5-alkylene-C(=O)-PRSDap(Me2)VCit-PAB-DX8951)ₙ;
Ab-((succinimid-3-yl-N)-C5-alkylene-C(=O)-PRSDap(Me2)VCit-PAB-MMAE)ₙ;
Ab-((succinimid-3-yl-N)-C5-alkylene-C(=O)-PCitNLVCit-PAB-DX8951)ₙ; and
Ab-((succinimid-3-yl-N)-C5-alkylene-C(=O)-PASLVCit-PAB-DX8951)ₙ;
Ab is an anti-Her2, anti-TF, anti-ROR1, or anti-GPC3 antibody or an antigen-binding fragment thereof; and n represents any value selected from 2 to 9.

45. The conjugate according to any one of claims 33 to 44, wherein the conjugate is an antibody-drug conjugate, and the antibody-drug conjugate is selected from the following structures:
Ab-((succinimid-3-yl-N)-CH2CH2CH2CH2CH2-C(=O)-GPAGLVCit-PAB-DX8951)ₙ;
Ab-((succinimid-3-yl-N)-CH2CH2CH2CH2CH2-C(=O)-GPLGLGGFG-DX8951)ₙ;
Ab-((succinimid-3-yl-N)-CH2CH2CH2CH2CH2-C(=O)-GPASLVCit-PAB-DX8951)ₙ;
Ab-((succinimid-3-yl-N)-CH2CH2CH2CH2CH2-C(=O)-GPRGLVCit-PAB-DX8951)ₙ;
Ab-((succinimid-3-yl-N)-CH2CH2CH2CH2CH2-C(=O)-GPSALVCit-PAB-DX8951)ₙ;
Ab-((succinimid-3-yl-N)-CH2CH2CH2CH2CH2-C(=O)-GPRGLGGFG-DX8951)ₙ;
Ab-((succinimid-3-yl-N)-CH2CH2CH2CH2CH2-C(=O)-GPAALGGFG-DX8951)ₙ;
Ab-((succinimid-3-yl-N)-CH2CH2CH2CH2CH2-C(=O)-PRNLVCit-PAB-DX8951)ₙ;
Ab-((succinimid-3-yl-N)-CH2CH2CH2CH2CH2-C(=O)-GPRNLVCit-PAB-DX8951)ₙ;
Ab-((succinimid-3-yl-N)-CH2CH2CH2CH2CH2-C(=O)-GPRGDap(Me2)LVCit-PAB-DX8951)ₙ;
Ab-((succinimid-3-yl-N)-CH2CH2CH2CH2CH2-C(=O)-SPRNLVCit-PAB-DX8951)ₙ;
Ab-((succinimid-3-yl-N)-CH2CH2CH2CH2CH2-C(=O)-GPLSLVCit-PAB-DX8951)ₙ;
Ab-((succinimid-3-yl-N)-CH2CH2CH2CH2CH2-C(=O)-GPAGLVCit-PAB-MMAE)ₙ;
Ab-((succinimid-3-yl-N)-CH2CH2CH2CH2CH2-C(=O)-GPRGLVCit-PAB-MMAE)ₙ;
Ab-((succinimid-3-yl-N)-CH2CH2CH2CH2CH2-C(=O)-PRNLVCit-PAB-MMAE)ₙ;
Ab-((succinimid-3-yl-N)-CH2CH2CH2CH2CH2-C(=O)-GPRNLVCit-PAB-MMAE)ₙ;
Ab-((succinimid-3-yl-N)-CH2CH2CH2CH2CH2-C(=O)-GPRGDap(Me2)LVCit-PAB-MMAE)ₙ;
Ab-((succinimid-3-yl-N)-CH2CH2CH2CH2CH2-C(=O)-SPRNLVCit-PAB-MMAE)ₙ;
Ab-((succinimid-3-yl-N)-CH2CH2CH2CH2CH2-C(=O)-GPLSLVCit-PAB-MMAE)ₙ;
Ab-((succinimid-3-yl-N)-CH2CH2CH2CH2CH2-C(=O)-GPAGLVCit-PAB-ET743)ₙ; and
Ab-((succinimid-3-yl-N)-CH2CH2CH2CH2CH2-C(=O)-GPRNLVCit-PAB-ET743)ₙ;
Ab represents a monoclonal antibody or an antigen-binding fragment thereof, or a modified monoclonal antibody or an antigen-binding fragment thereof, and preferably an anti-Her2, anti-TF, anti-ROR1, or anti-GPC3 antibody, or an antigen-binding fragment thereof; and
n is an integer from 1 to 10, and preferably, 2, 4, 6, or 8.

46. An anti-tissue factor (TF) antibody or an antigen-binding fragment thereof, comprising:
(a) a VH comprising an amino acid sequence set forth in SEQ ID NO: 52 or 54; and/or
(b) a VL comprising an amino acid sequence set forth in SEQ ID NO: 53 or 55.

47. The anti-TF antibody or the antigen-binding fragment thereof according to claim 46, wherein the anti-TF antibody or the antigen-binding fragment thereof comprises:
(1) a VH having an amino acid sequence set forth in SEQ ID NO: 52 and a VL having an amino acid sequence set forth in SEQ ID NO: 53; or
(2) a VH having an amino acid sequence set forth in SEQ ID NO: 54, and a VL having an amino acid sequence set forth in SEQ ID NO: 53; or
(3) a VH having an amino acid sequence set forth in SEQ ID NO: 54, and a VL having an amino acid sequence set forth in SEQ ID NO: 55; or
(4) a VH having an amino acid sequenceset forth in SEQ ID NO: 52, and a VL having an amino acid sequence set forth in SEQ ID NO: 55.

48. The anti-TF antibody or the antigen-binding fragment thereof according to claim 46 or 47, wherein the anti-TF antibody or the antigen-binding fragment thereof comprises a light chain having an amino acid sequence set forth in SEQ ID NO: 56, or an amino acid sequence having at least 80% identity to the amino acid sequence set forth in SEQ ID NO: 56; a heavy chain having an amino acid sequence set forth in SEQ ID NO: 57, or an amino acid sequence having at least 80% identity to the amino acid sequence set forth in SEQ ID NO: 57.

49. The anti-TF antibody or the antigen-binding fragment thereof according to claim 46 or 47, wherein the anti-TF antibody or the antigen-binding fragment thereof comprises a light chain having an amino acid sequence set forth in SEQ ID NO: 56, or an amino acid sequence having at least 80% identity to the amino acid sequence set forth in SEQ ID NO: 56; and a heavy chain having an amino acid sequence set forth in SEQ ID NO: 59, or an amino acid sequence having at least 80% identity to the amino acid sequence set forth in SEQ ID NO: 59.

50. The anti-TF antibody or the antigen-binding fragment thereof according to claim 46 or 47, wherein the anti-TF antibody or the antigen-binding fragment thereof comprises a light chain having an amino acid sequence set forth in SEQ ID NO: 58, or an amino acid sequence having at least 80% identity to the amino acid sequence set forth in SEQ ID NO: 58; and a heavy chain having an amino acid sequence set forth in SEQ ID NO: 59, or an amino acid sequence having at least 80% identity to the amino acid sequence set forth in SEQ ID NO: 59.

51. A nucleic acid molecule, encoding the anti-TF antibody or the antigen-binding fragment thereof according to any one of claims 46 to 50, or a heavy chain variable region and/or a light chain variable region thereof.

52. A recombinant vector, comprising the nucleic acid molecule of claim 51.

53. A recombinant cell, comprising the nucleic acid molecule of claim 51 or the recombinant vector of claim 52.

54. A method of preparing an anti-TF antibody or an antigen-binding fragment thereof, comprising culturing the recombinant cell of claim 53.

55. Use of the anti-TF antibody or the antigen-binding fragment thereof according to any one of claims 46 to 50, the nucleic acid molecule of claim 51, the recombinant vector of claim 52, or the recombinant cell of claim 53 in preparation of a medicament; preferably, the medicament is a medicament for prevention and/or treatment of a cancer; and more preferably, the cancer is a TF-expressing cancer.

56. Use of the anti-TF antibody or the antigen-binding fragment thereof according to any one of claims 46 to 50, the nucleic acid molecule of claim 51, the recombinant vector of claim 52, or the recombinant cell of claim 53 in preparation of a reagent for diagnosing a cell proliferation-related disease; preferably, the disease is a cancer; and more preferably, the cancer is a TF-expressing cancer.

57. A pharmaceutical composition, comprising the polypeptide or the polypeptide fragment according to any one of claims 1 to 8, the linker according to any one of claims 9 to 20, the linker drug according to any one of claims 21 to 32, the conjugate according to any one of claims 33 to 45, the anti-TF antibody or the antigen-binding fragment thereof according to any one of claims 46 to 50, the nucleic acid molecule of claim 51, the recombinant vector of claim 52, the recombinant cell of claim 53, and optionally one or more pharmaceuticalexcipients; and preferably, further comprising a pharmaceutically-acceptable carrier and/or excipient.

58. Use of the polypeptide or polypeptide fragment according to any one of claims 1 to 8, the linker according to any one of claims 9 to 20, the linker drug according to any one of claims 21 to 32, the conjugate according to any one of claims 33 to 45, the anti-TF antibody or the antigen-binding fragment thereof according to any one of claims 46 to 50, the nucleic acid molecule of claim 51, the recombinant vector of claim 52, the recombinant cell of claim 53, or the pharmaceutical composition of claim 57 in preparation of a medicament for treatment or prevention of a disease or a disorder.

59. Use of the polypeptide or polypeptide fragment according to any one of claims 1 to 8, the linker according to any one of claims 9 to 20, the linker drug according to any one of claims 21 to 32, the conjugate according to any one of claims 33 to 45, the anti-TF antibody or the antigen-binding fragment thereof according to any one of claims 46 to 50, the nucleic acid molecule of claim 51, the recombinant vector of claim 52, the recombinant cell of claim 53, or the pharmaceutical composition of claim 57 in preparation of a medicament for treatment/prevention of a disease or a disorder associated with abnormal cellular activity.

60. The use according to claim 58 or 59, wherein the disease or the disorder comprises a tumor.

61. The use according to claim 60, wherein the disease or the disorder comprises a solid tumor and a hematological malignancy.

62. A method of treating a disease or a disorder, preferably a tumor, in a subject in need thereof, comprising administering to the subject an effective amount of the conjugate according to any one of claims 33 to 45.

63. The use according to any one of claims 58 to 61 or the method according to claim 62, wherein the disease or the disorder is selected from the following protein expression-associated tumors:
EGFR, HER2, HER3, c-Met, claudin18.2, TROP-2, folate receptor α (FRα), ROR1, ROR2, BCMA, PSMA, CD19, CD20, CD22, CD30, CD33, CD37, CD46, CD48, CD56, CD79b, CD123, CD138, CD166, CD276, CEACAM5, SDC1, CD74, CD70, MUC1, MUC16, GUCY2C, MSLN, SCL34A2, FOLH1, TPBC, PVRL4, STEAP1, SCL44A4, NACM1, EDNRB, GPNMB, FGFR, SEZ6, Nectin-4, PD-L1, Tissue Factor (TF), B7-H3, B7-H4, LY6E, LIV-1, CDH4, CDH6, ITGB6, GPC1, GPC3, ENPP3, KAAG1, FZD7, SSTR2, DLK1, TMEFF1, TM4SF1, SLAMF7, DLL3, FLT3, TNFRSF10B, EPCAM, AXL, IL2RA, 5T4, FAP, ICAM-1, and IGF-1R; and
preferably, selected from tumors associated with expression of Her2, TF, ROR1, or GPC3.

64. The use according to any one of claims 58 to 61 or the method according to claim 62, wherein the disease or the disorder is selected from esophageal cancer, brain cancer, lung cancer, epithelial cell carcinoma, bladder cancer, gastric cancer, ovarian cancer, pancreatic cancer, head and neck cancer, urothelial carcinoma, colon cancer, colorectal cancer, cervical cancer, endometrial cancer, rectal cancer, renal cancer, prostate cancer, melanoma, liver cancer, gallbladder cancer, cholangiocarcinoma, thyroid cancer, non-Hodgkin's lymphoma, multiple myeloma, and B-cell lymphoma; and
preferably, the tumor is selected from esophageal cancer, lung cancer, breast cancer, gastric cancer, urothelial carcinoma, cholangiocarcinoma, prostate cancer, colorectal cancer, cervical cancer, and ovarian cancer.

65. Use of the polypeptide or polypeptide fragment according to any one of claims 1 to 8, the linker according to any one of claims 9 to 20, or the linker drug according to any one of claims 21 to 32 in preparation of an antibody-drug conjugate for treating a tumor.
